# EUROPEAN PATENT SPECIFICATION

(11) **EP 3 000 889 B1**
(45) Date of publication and mention of the grant of the patent: **05.12.2018**
(21) Application number: 15188570.4
(22) Date of filing: 01.03.2010
(51) Int. Cl.: C12N 15/82, C07K 14/415

(54) **ISOLATED POLYNUCLEOTIDES AND POLYPEPTIDES, AND METHODS OF USING SAME FOR INCREASING PLANT YIELD AND/OR AGRICULTURAL CHARACTERISTICS**
ISOLIERTE POLYNUKLEOTIDE UND POLYPEPTIDE SOWIE VERFAHREN ZU IHRER VERWENDUNG ZUR ERHÖHUNG DES ERTRAGS ODER ZUR VERBESSERUNG DER LANDWIRTSCHAFTLICHEN EIGENSCHAFTEN VON PFLANZEN
POLYNUCLÉOTIDES ET POLYPEPTIDES ISOLÉS, ET PROCÉDÉS D'UTILISATION DE CEUX-CI POUR AUGMENTER UN RENDEMENT VÉGÉTAL ET/OU DES CARACTÉRISTIQUES AGRICOLES

(30) Priority: 02.03.2009 US 202459 P; 05.08.2009 US 231349 P; 28.12.2009 US 282183 P
(43) Date of publication of application: 30.03.2016
(62) Divisional of application: 10748403.2
(73) Proprietor: Evogene Ltd., 76121 Rechovot (IL)
(72) Inventor: EMMANUEL, Eyal, 76302 Rechovot (IL); DIBER, Alex, 75502 Rishon-LeZion (IL); POLLOCK, Sarah Rachel, 63506 Tel-Aviv (IL); KARCHI, Hagai, 76834 Moshav Sitriya (IL)
(74) Representative: Becker Kurig Straus

(56) References cited:
- WO-A2-2004/058963
- US-A1- 2007 044 171
- DATABASE EMBL [Online] 19 November 2000 (2000-11-19), "HVSMEf0009N22f Hordeum vulgare seedling root EST library HVcDNA0007 (Etiolated and unstressed) Hordeum vulgare cDNA clone HVSMEf0009N22f, mRNA sequence.", XP002752784, retrieved from EBI accession no. EM_EST:BF256321 Database accession no. BF256321
- DATABASE EMBL [Online] 27 August 2004 (2004-08-27), "BNEL25c1 Barley EST endosperm library Hordeum vulgare subsp. vulgare cDNA clone BNEL25c1 5' similar to Unknown Function, mRNA sequence.", XP002752785, retrieved from EBI accession no. EM_EST:CV057209 Database accession no. CV057209

## Description

### FIELD AND BACKGROUND OF THE INVENTION

The present invention, in some embodiments thereof, relates to isolated polynucleotides and polypeptides which can increase biomass, growth rate, oil content and/or nitrogen use efficiency of a plant.

The ever-increasing world population and the decreasing availability in arable land for agriculture affect the yield of plants and plant-related products. The global shortage of water supply, desertification, abiotic stress (ABS) conditions (e.g., salinity, drought, flood, suboptimal temperature and toxic chemical pollution), and/or limited nitrogen and fertilizer sources cause substantial damage to agricultural plants such as major alterations in the plant metabolism, cell death, and decreases in plant growth and crop productivity.

Drought is a gradual phenomenon, which involves periods of abnormally dry weather that persists long enough to produce serious hydrologic imbalances such as crop damage, water supply shortage and increased susceptibility to various diseases.

Salinity, high salt levels, affects one in five hectares of irrigated land. None of the top five food crops, *i.e.,* wheat, corn, rice, potatoes, and soybean, can tolerate excessive salt. Detrimental effects of salt on plants result from both water deficit, which leads to osmotic stress (similar to drought stress), and the effect of excess sodium ions on critical biochemical processes. As with freezing and drought, high salt causes water deficit; and the presence of high salt makes it difficult for plant roots to extract water from their environment. Thus, salination of soils that are used for agricultural production is a significant and increasing problem in regions that rely heavily on agriculture, and is worsen by over-utilization, over-fertilization and water shortage, typically caused by climatic change and the demands of increasing population.

Suboptimal temperatures affect plant growth and development through the whole plant life cycle. Thus, low temperatures reduce germination rate and high temperatures result in leaf necrosis. In addition, mature plants that are exposed to excess heat may experience heat shock, which may arise in various organs, including leaves and particularly fruit, when transpiration is insufficient to overcome heat stress. Heat also damages cellular structures, including organelles and cytoskeleton, and impairs membrane function. Heat shock may produce a decrease in overall protein synthesis, accompanied by expression of heat shock proteins, e.g., chaperones, which are involved in refolding proteins denatured by heat. High-temperature damage to pollen almost always occurs in conjunction with drought stress, and rarely occurs under well-watered conditions. Combined stress can alter plant metabolism in novel ways. Excessive chilling conditions, e.g., low, but above freezing, temperatures affect crops of tropical origins, such as soybean, rice, maize, and cotton. Typical chilling damage includes wilting, necrosis, chlorosis or leakage of ions from cell membranes. Excessive light conditions, which occur under clear atmospheric conditions subsequent to cold late summer/autumn night's, can lead to photoinhibition of photosynthesis (disruption of photosynthesis). In addition, chilling may lead to yield losses and lower product quality through the delayed ripening of maize.

Suboptimal nutrient (macro and micro nutrient) affect plant growth and development through the whole plant life cycle. One of the essential macronutrients for the plant is Nitrogen. Nitrogen is responsible for biosynthesis of amino acids and nucleic acids, prosthetic groups, plant hormones, plant chemical defenses, and the like. Nitrogen is often the rate-limiting element in plant growth and all field crops have a fundamental dependence on inorganic nitrogenous fertilizer. Since fertilizer is rapidly depleted from most soil types, it must be supplied to growing crops two or three times during the growing season. Additional important macronutrients are Phosphorous (P) and Potassium (K), which have a direct correlation to yield and general plant tolerance.

Yield is affected by various factors, such as, the number and size of the plant organs, plant architecture (for example, the number of branches), grains set length, number of filled grains, vigor (e.g. seedling), growth rate, root development, utilization of water, nutrients (e.g., nitrogen) and fertilizers, and stress tolerance.

Crops such as, corn, rice, wheat, canola and soybean account for over half of total human caloric intake, whether through direct consumption of the seeds themselves or through consumption of meat products raised on processed seeds or forage. Seeds are also a source of sugars, oils and metabolites used in industrial processes. The ability to increase plant yield, whether through increase dry matter accumulation rate, modifying cellulose or lignin composition, increase stalk strength, enlarge meristem size, change of plant branching pattern, erectness of levees, increase in fertilization efficiency, enhanced seed dry matter accumulation rate, modification of seed development, enhanced seed filling or by increasing the content of oil, starch or protein in the seeds would have many applications in agricultural and non-agricultural uses such as in the biotechnological production of pharmaceuticals, antibodies or vaccines.

Studies have shown that plant adaptations to adverse environmental conditions are complex genetic traits with polygenic nature. Conventional means for crop and horticultural improvements utilize selective breeding techniques to identify plants having desirable characteristics. However, selective breeding is tedious, time consuming and has an unpredictable outcome. Furthermore, limited germplasm resources for yield improvement and incompatibility in crosses between distantly related plant species represent significant problems encountered in conventional breeding. Advances in genetic engineering have allowed mankind to modify the germplasm of plants by expression of genes-of-interest in plants. Such a technology has the capacity to generate crops or plants with improved economic, agronomic or horticultural traits.

WO publication No. 2009/013750 discloses genes, constructs and methods of increasing abiotic stress tolerance, biomass and/or yield in plants generated thereby.

WO publication No. 2008/122980 discloses genes constructs and methods for increasing oil content, growth rate and biomass of plants.

WO publication No. 2008/075364 discloses polynucleotides involved in plant fiber development and methods of using same.

WO publication No. 2007/049275 discloses isolated polypeptides, polynucleotides encoding same, transgenic plants expressing same and methods of using same for increasing plant abiotic stress tolerance and biomass.

WO publication No. 2004/104162 discloses methods of increasing abiotic stress tolerance and/or biomass in plants and plants generated thereby.

WO publication No. 2005/121364 discloses polynucleotides and polypeptides involved in plant fiber development and methods of using same for improving fiber quality, yield and/or biomass of a fiber producing plant.

WO publication No. 2007/020638 discloses methods of increasing abiotic stress tolerance and/or biomass in plants and plants generated thereby.

### SUMMARY OF THE INVENTION

According to an aspect of some embodiments of the present invention there is provided a method of increasing biomass, growth rate, oil content, and/or nitrogen use efficiency of a plant, comprising expressing within the plant an exogenous polynucleotide comprising a nucleic acid sequence encoding a polypeptide at least 80 % identical to SEQ ID NO: 333, thereby increasing the biomass, growth rate, oil content, and/or nitrogen use efficiency of the plant.

According to an aspect of some embodiments of the present invention there is provided a plant cell exogenously expressing a nucleic acid construct comprising an isolated polynucleotide comprising a nucleic acid sequence encoding a polypeptide at least 80 % identical to SEQ ID NO: 333, and a promoter for directing transcription of said nucleic acid sequence in a host cell, wherein said nucleic acid sequence is capable of increasing biomass, growth rate, oil content, and/or nitrogen use efficiency of a plant.

According to an aspect of some embodiments of the present invention there is provided an isolated polypeptide comprising an amino acid sequence at least 80 % homologous to SEQ ID NO: 333, wherein said amino acid sequence is capable of increasing biomass, growth rate, oil content, and/or nitrogen use efficiency of a plant.

According to an aspect of some embodiments of the present invention there is provided a plant cell exogenously expressing the polypeptide of the present invention.

According to some embodiments of the invention, the plant cell forms a part of a plant.

Further aspects and embodiments of the present invention are specified in the accompanying claims.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

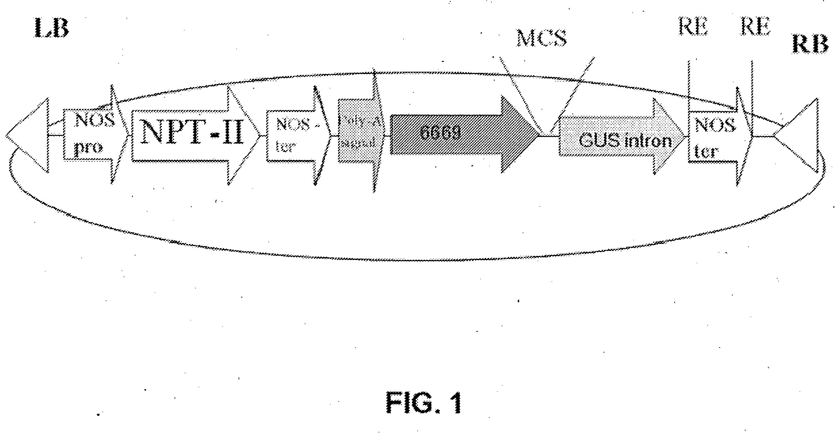

FIG. 1 is a schematic illustration of the modified pGI binary plasmid containing the new At6669 promoter (SEQ ID NO:4198) and the GUSintron (pQYN_6669) used for expressing the isolated polynucleotide sequences. RB - T-DNA right border; LB - T-DNA left border; MCS - Multiple cloning site; RE - any restriction enzyme; NOS pro = nopaline synthase promoter; NPT-II = neomycin phosphotransferase gene; NOS ter = nopaline synthase terminator; Poly-A signal (polyadenylation signal); GUSintron - the GUS reporter gene (coding sequence and intron). The isolated polynucleotide sequences were cloned into the vector while replacing the GUSintron reporter gene.

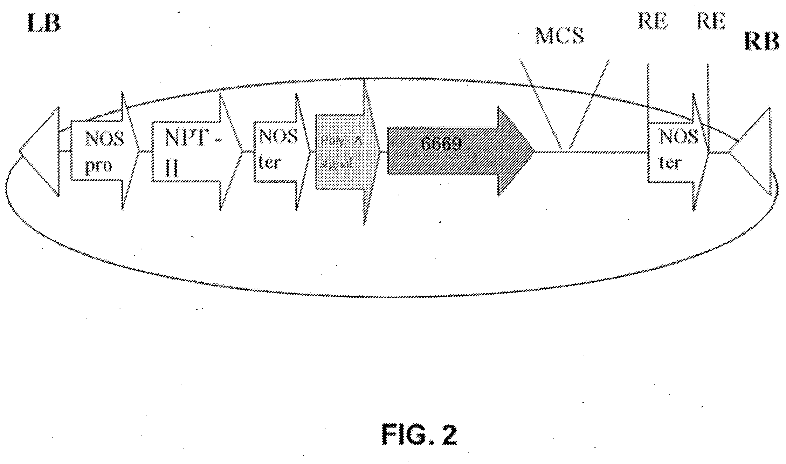

FIG. 2 is a schematic illustration of the modified pGI binary plasmid containing the new At6669 promoter (SEQ ID NO:4198) (pQFN) used for expressing the isolated polynucleotide sequences. RB - T-DNA right border; LB - T-DNA left border; MCS - Multiple cloning site; RE - any restriction enzyme; NOS pro = nopaline synthase promoter; NPT-II = neomycin phosphotransferase gene; NOS ter = nopaline synthase terminator; Poly-A signal (polyadenylation signal); GUSintron - the GUS reporter gene (coding sequence and intron). The isolated polynucleotide sequences were cloned into the MCS of the vector.

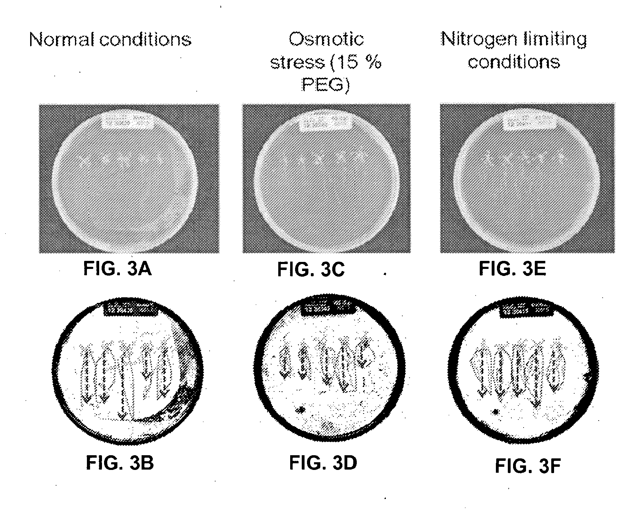

FIGs. 3A-F are images depicting visualization of root development of transgenic plants exogenously expressing the polynucleotide when grown in transparent agar plates under normal (FIGs. 3A-B), osmotic stress (15 % PEG; FIGs. 3C-D) or nitrogen-limiting (FIGs. 3E-F) conditions. The different transgenes were grown in transparent agar plates for 17 days (7 days nursery and 10 days after transplanting). The plates were photographed every 3-4 days starting at day 1 after transplanting. FIG. 3A - An image of a photograph of plants taken following 10 after transplanting days on agar plates when grown under normal (standard) conditions. FIG. 3B - An image of root analysis of the plants shown in FIG. 3A in which the lengths of the roots measured are represented by arrows. FIG. 3C - An image of a photograph of plants taken following 10 days after transplanting on agar plates, grown under high osmotic (PEG 15 %) conditions. FIG. 3D - An image of root analysis of the plants shown in FIG. 3C in which the lengths of the roots measured are represented by arrows. FIG. 3E - An image of a photograph of plants taken following 10 days after transplanting on agar plates, grown under low nitrogen conditions. FIG. 3F - An image of root analysis of the plants shown in FIG. 3E in which the lengths of the roots measured are represented by arrows.

### DESCRIPTION OF SPECIFIC EMBODIMENTS OF THE INVENTION

The present invention, in some embodiments thereof, relates to isolated polynucleotides and polypeptides which can increase biomass, growth rate, oil content, and/or nitrogen use efficiency of a plant.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

The present inventors have identified novel polynucleotides and polypeptides which can be used in increasing yield, biomass, growth rate, vigor, oil content, fiber yield, fiber quality abiotic stress tolerance, and/or fertilizer use efficiency (e.g., nitrogen use efficiency) of a plant.

As shown in the Examples section which follows, the present inventors have employed a bioinformatic approach which combines clustering and assembly of sequences from databases of arabidopsis, rice, poplar, brachypodium, soybean, grape, castobean, sorghum and maize and other publicly available plant genomes, expressed sequence tags (ESTs), mRNA sequences, properitary ESTs sequences (Barley, Sorghum), protein and pathway databases, quantitative trait loci (QTL), single nucleotide polymorphism (SNPs) information with a digital expression profile ("electronic Northern Blot") and identified polynucleotides and polypeptides which can increase yield, growth rate, biomass, vigor, tolerance to abiotic stress, nitrogen use efficiency, water use efficiency and fertilizer use efficiency (SEQ ID NOs:1-239 for polynucleotides; SEQ ID NOs:240-465 for polypeptides; Table 1, Example 1). Orthologs from plant species which exhibit at least 80 % homology to the identified polypeptides and polynucleotides were also identified (SEQ ID NO:467-1973 for polynucleotides; SEQ ID NOs:1974-3480 for polypeptides; Table 2, Example 1). Selected genes were cloned (Example 7, Tables 26-28), transformed into agrobacterium tumefaciens cells (Example 8) and further into arabidopsis plants (Example 9). Transgenic plants over-expressing the identified polynucleotides were found to exhibit increased seed yield, oil yield, dry weight, fresh weight, root coverage, root length, harvest index, growth rate, rosette area, biomass, oil percentage in seed and weight of 1000 seeds (Examples 10-11; Tables 29-36), and increased tolerance to abiotic stress conditions such as limiting nitrogen conditions (Example 11, Tables 37-38). Thus, the identified polynucleotides and polypeptides can be used to increase plant's yield, biomass (e.g., of grain or any harvestable plant part with economical value), vigor, growth rate, oil content, fiber yield, fiber quality, tolerance to abiotic stress, nitrogen use efficiency, water use efficiency and/or fertilizer use efficiency.

Thus, according to an aspect of some embodiments of the invention there is provided a method of increasing biomass, growth rate, oil content, and/or nitrogen use efficiency of a plant.

The method is effected by expressing within the plant an exogenous polynucleotide comprising a nucleic acid sequence at least 80 % identical to SEQ ID NO: 333, thereby increasing biomass, growth rate, oil content, and/or nitrogen use efficiency of the plant.

As used herein the phrase "plant yield" refers to the amount (e.g., as determined by weight or size) or quantity (numbers) of tissues or organs produced per plant or per growing season. Hence increased yield could affect the economic benefit one can obtain from the plant in a certain growing area and/or growing time.

It should be noted that a plant yield can be affected by various parameters including, but not limited to, plant biomass; plant vigor; growth rate; seed yield; seed or grain quantity; seed or grain quality; oil yield; content of oil, starch and/or protein in harvested organs (e.g., seeds or vegetative parts of the plant); number of flowers (florets) per panicle (expressed as a ratio of number of filled seeds over number of primary panicles); harvest index; number of plants grown per area; number and size of harvested organs per plant and per area; number of plants per growing area (density); number of harvested organs in field; total leaf area; carbon assimilation and carbon partitioning (the distribution/allocation of carbon within the plant); resistance to shade; number of harvestable organs (e.g. seeds), seeds per pod, weight per seed; and modified architecture [such as increase stalk diameter, thickness or improvement of physical properties (e.g. elasticity)] .

As used herein the phrase "seed yield" refers to the number or weight of the seeds per plant, seeds per pod, or per growing area or to the weight of a single seed, or to the oil extracted per seed. Hence seed yield can be affected by seed dimensions (e.g., length, width, perimeter, area and/or volume), number of (filled) seeds and seed filling rate and by seed oil content. Hence increase seed yield per plant could affect the economic benefit one can obtain from the plant in a certain growing area and/or growing time; and increase seed yield per growing area could be achieved by increasing seed yield per plant, and/or by increasing number of plants grown on the same given area.

The term "seed" (also referred to as "grain" or "kernel") as used herein refers to a small embryonic plant enclosed in a covering called the seed coat (usually with some stored food), the product of the ripened ovule of gymnosperm and angiosperm plants which occurs after fertilization and some growth within the mother plant.

The phrase "oil content" as used herein refers to the amount of lipids in a given plant organ, either the seeds (seed oil content) or the vegetative portion of the plant (vegetative oil content) and is typically expressed as percentage of dry weight (10 % humidity of seeds) or wet weight (for vegetative portion).

It should be noted that oil content is affected by intrinsic oil production of a tissue (e.g., seed, vegetative portion), as well as the mass or size of the oil-producing tissue per plant or per growth period.

An increase in oil content of the plant can be achieved by increasing the size/mass of a plant's tissue(s) which comprise oil per growth period. Thus, increased oil content of a plant can be achieved by increasing the yield, growth rate, biomass and vigor of the plant.

As used herein the phrase "plant biomass" refers to the amount (e.g., measured in grams of air-dry tissue) of a tissue produced from the plant in a growing season, which could also determine or affect the plant yield or the yield per growing area. An increase in plant biomass can be in the whole plant or in parts thereof such as aboveground (harvestable) parts, vegetative biomass, roots and seeds.

As used herein the phrase "growth rate" refers to the increase in plant organ/tissue size per time (can be measured in cm² per day).

As used herein the phrase "plant vigor" refers to the amount (measured by weight) of tissue produced by the plant in a given time. Hence increased vigor could determine or affect the plant yield or the yield per growing time or growing area. In addition, early vigor (seed and/or seedling) results in improved field stand.

It should be noted that a plant yield can be determined under stress (e.g., abiotic stress, nitrogen-limiting conditions) and/or non-stress (normal) conditions.

As used herein, the phrase "non-stress conditions" refers to the growth conditions (e.g., water, temperature, light-dark cycles, humidity, salt concentration, fertilizer concentration in soil, nutrient supply such as nitrogen, phosphorous and/or potassium), that do not significantly go beyond the everyday climatic and other abiotic conditions that plants may encounter, and which allow optimal growth, metabolism, reproduction and/or viability of a plant at any stage in its life cycle (e.g., in a crop plant from seed to a mature plant and back to seed again). Persons skilled in the art are aware of normal soil conditions and climatic conditions for a given plant in a given geographic location. It should be noted that while the non-stress conditions may include some mild variations from the optimal conditions (which vary from one type/species of a plant to another), such variations do not cause the plant to cease growing without the capacity to resume growth.

The phrase "abiotic stress" as used herein refers to any adverse effect on metabolism, growth, reproduction and/or viability of a plant. Accordingly, abiotic stress can be induced by suboptimal environmental growth conditions such as, for example, salinity, water deprivation, flooding, freezing, low or high temperature, heavy metal toxicity, anaerobiosis, nutrient deficiency, atmospheric pollution or UV irradiation. The implications of abiotic stress are discussed in the Background section.

The phrase "abiotic stress tolerance" as used herein refers to the ability of a plant to endure an abiotic stress without suffering a substantial alteration in metabolism, growth, productivity and/or viability.

As used herein the phrase "water use efficiency (WUE)" refers to the level of organic matter produced per unit of water consumed by the plant, i.e., the dry weight of a plant in relation to the plant's water use, e.g., the biomass produced per unit transpiration.

As used herein the phrase "fertilizer use efficiency" refers to the metabolic process(es) which lead to an increase in the plant's yield, biomass, vigor, and growth rate per fertilizer unit applied. The metabolic process can be the uptake, spread, absorbent, accumulation, relocation (within the plant) and use of one or more of the minerals and organic moieties absorbed by the plant, such as nitrogen, phosphates and/or potassium.

As used herein the phrase "fertilizer-limiting conditions" refers to growth conditions which include a level (e.g., concentration) of a fertilizer applied which is below the level needed for normal plant metabolism, growth, reproduction and/or viability.

As used herein the phrase "nitrogen use efficiency (NUE)" refers to the metabolic process(es) which lead to an increase in the plant's yield, biomass, vigor, and growth rate per nitrogen unit applied. The metabolic process can be the uptake, spread, absorbent, accumulation, relocation (within the plant) and use of nitrogen absorbed by the plant.

As used herein the phrase "nitrogen-limiting conditions" refers to growth conditions which include a level (e.g., concentration) of nitrogen (e.g., ammonium or nitrate) applied which is below the level needed for normal plant metabolism, growth, reproduction and/or viability.

Improved plant NUE and FUE is translated in the field into either harvesting similar quantities of yield, while implementing less fertilizers, or increased yields gained by implementing the same levels of fertilizers. Thus, improved NUE or FUE has a direct effect on plant yield in the field. Thus, the polynucleotides and polypeptides of some embodiments of the invention can positively affect plant yield, seed yield, and plant biomass. In addition, the benefit of improved plant NUE will certainly improve crop quality and biochemical constituents of the seed such as protein yield and oil yield.

It should be noted that improved ABST will confer plants with improved vigor also under non-stress conditions, resulting in crops having improved biomass and/or yield e.g., elongated fibers for the cotton industry, higher oil content.

The term "fiber" is usually inclusive of thick-walled conducting cells such as vessels and tracheids and to fibrillar aggregates of many individual fiber cells. Hence, the term "fiber" refers to (a) thick-walled conducting and non-conducting cells of the xylem; (b) fibers of extraxylary origin, including those from phloem, bark, ground tissue, and epidermis; and (c) fibers from stems, leaves, roots, seeds, and flowers or inflorescences (such as those of Sorghum vulgare used in the manufacture of brushes and brooms).

As used herein the phrase "fiber producing plant" refers to plants that share the common feature of having an elongated shape and abundant cellulose in thick cell walls, typically termed as secondary walls. Such walls may or may not be lignified, and the protoplast of such cells may or may be viable at maturity. Such fibers have many industrial uses, for example in lumber and manufactured wood products, paper, textiles, sacking and boxing material, cordage, brushes and brooms, filling and stuffing, caulking, reinforcement of other materials, and manufacture of cellulose derivatives.

Example of fiber producing plants, include, but are not limited to, agricultural crops such as cotton, silk cotton tree (Kapok, Ceiba pentandra), desert willow, creosote bush, winterfat, balsa, kenaf, roselle, jute, sisal abaca, flax, corn, sugar cane, hemp, ramie, kapok, coir, bamboo, spanish moss and Agave spp. (e.g. sisal).

The fiber producing plant can be cotton.

As used herein the phrase "fiber quality" refers to at least one fiber parameter which is agriculturally desired, or required in the fiber industry (further described hereinbelow). Examples of such parameters, include but are not limited to, fiber length, fiber strength, fiber fitness, fiber weight per unit length, maturity ratio and uniformity.

Cotton fiber (lint) quality is typically measured according to fiber length, strength and fineness. Accordingly, the lint quality is considered higher when the fiber is longer, stronger and finer.

As used herein the phrase "fiber yield" refers to the amount or quantity of fibers produced from the fiber producing plant.

As used herein the term "increasing" refers to at least about 2 %, at least about 3 %, at least about 4 %, at least about 5 %, at least about 10 %, at least about 15 %, at least about 20 %, at least about 30 %, at least about 40 %, at least about 50 %, at least about 60 %, at least about 70 %, at least about 80 % or greater increase in plant yield, biomass, growth rate, vigor, oil content, fiber yield, fiber quality, water use efficiency, nitrogen use efficiency, fertilizer use efficiency and/or abiotic stress tolerance as compared to a native plant [*i.e.,* a plant not modified with the biomolecules (polynucleotide or polypeptides), e.g., a non-transformed plant of the same species which is grown under the same growth conditions as the transformed plant].

The phrase "expressing within the plant an exogenous polynucleotide" as used herein refers to upregulating the expression level of an exogenous polynucleotide within the plant by introducing the exogenous polynucleotide into a plant cell or plant and expressing by recombinant means, as further described herein below.

As used herein "expressing" refers to expression at the mRNA and optionally polypeptide level.

As used herein, the phrase "exogenous polynucleotide" refers to a heterologous nucleic acid sequence which may not be naturally expressed within the plant or which overexpression in the plant is desired. The exogenous polynucleotide may be introduced into the plant in a stable or transient manner, so as to produce a ribonucleic acid (RNA) molecule and/or a polypeptide molecule. It should be noted that the exogenous polynucleotide may comprise a nucleic acid sequence which is identical or partially homologous to an endogenous nucleic acid sequence of the plant.

The term "endogenous" as used herein refers to any polynucleotide or polypeptide which is present and/or naturally expressed within a plant or a cell thereof.

Identity (e.g., percent homology) can be determined using any homology comparison software, including for example, the BlastN software of the National Center of Biotechnology Information (NCBI) such as by using default parameters.

As used herein the term "polynucleotide" refers to a single or double stranded nucleic acid sequence which is isolated and provided in the form of an RNA sequence, a complementary polynucleotide sequence (cDNA), a genomic polynucleotide sequence and/or a composite polynucleotide sequences (e.g., a combination of the above).

The term "isolated" refers to at least partially separated from the natural environment e.g., from a plant cell.

As used herein the phrase "complementary polynucleotide sequence" refers to a sequence, which results from reverse transcription of messenger RNA using a reverse transcriptase or any other RNA dependent DNA polymerase. Such a sequence can be subsequently amplified *in vivo* or *in vitro* using a DNA dependent DNA polymerase.

As used herein the phrase "genomic polynucleotide sequence" refers to a sequence derived (isolated) from a chromosome and thus it represents a contiguous portion of a chromosome.

As used herein the phrase "composite polynucleotide sequence" refers to a sequence, which is at least partially complementary and at least partially genomic. A composite sequence can include some exonal sequences required to encode the polypeptide of the present invention, as well as some intronic sequences interposing therebetween. The intronic sequences can be of any source, including of other genes, and typically will include conserved splicing signal sequences. Such intronic sequences may further include cis acting expression regulatory elements.

The exogenous polynucleotide encodes a polypeptide which comprises an amino acid sequence encoding a polypeptide at least about 80 %, at least about 81 %, at least about 82 %, at least about 83 %, at least about 84 %, at least about 85 %, at least about 86 %, at least about 87 %, at least about 88 %, at least about 89 %, at least about 90 %, at least about 91 %, at least about 92 %, at least about 93 %, at least about 94 %, at least about 95 %, at least about 96 %, at least about 97 %, at least about 98 %, at least about 99 %, or more say 100 % homologous to the amino acid sequence of SEQ ID NO: 333.

Homology (e.g., percent homology) can be determined using any homology comparison software, including for example, the BlastP or TBLASTN software of the National Center of Biotechnology Information (NCBI) such as by using default parameters, when starting from a polypeptide sequence; or the tBLASTX algorithm (available via the NCBI) such as by using default parameters, which compares the six-frame conceptual translation products of a nucleotide query sequence (both strands) against a protein sequence database.

Homologous sequences include both orthologous and paralogous sequences. The term "paralogous" relates to gene-duplications within the genome of a species leading to paralogous genes. The term "orthologous" relates to homologous genes in different organisms due to ancestral relationship.

One option to identify orthologues in plant species is by performing a reciprocal blast search. This may be done by a first blast involving blasting the sequence-of-interest against any sequence database, such as the publicly available NCBI database which may be found at: Hypertext Transfer Protocol://World Wide Web (dot) ncbi (dot) nlm (dot) nih (dot) gov. If orthologues in rice were sought, the sequence-of-interest would be blasted against, for example, the 28,469 full-length cDNA clones from Oryza sativa Nipponbare available at NCBI. The blast results may be filtered. The full-length sequences of either the filtered results or the non-filtered results are then blasted back (second blast) against the sequences of the organism from which the sequence-of-interest is derived. The results of the first and second blasts are then compared. An orthologue is identified when the sequence resulting in the highest score (best hit) in the first blast identifies in the second blast the query sequence (the original sequence-of-interest) as the best hit. Using the same rational a paralogue (homolog to a gene in the same organism) is found. In case of large sequence families, the ClustalW program may be used [Hypertext Transfer Protocol://World Wide Web (dot) ebi (dot) ac (dot) uk/Tools/clustalw2/index (dot) html], followed by a neighbor-joining tree (Hypertext Transfer Protocol://en (dot) wikipedia (dot) org/wiki/Neighbor-joining) which helps visualizing the clustering.

Nucleic acid sequences encoding the polypeptides of the present invention may be optimized for expression. Examples of such sequence modifications include, but are not limited to, an altered G/C content to more closely approach that typically found in the plant species of interest, and the removal of codons atypically found in the plant species commonly referred to as codon optimization.

The phrase "codon optimization" refers to the selection of appropriate DNA nucleotides for use within a structural gene or fragment thereof that approaches codon usage within the plant of interest. Therefore, an optimized gene or nucleic acid sequence refers to a gene in which the nucleotide sequence of a native or naturally occurring gene has been modified in order to utilize statistically-preferred or statistically-favored codons within the plant. The nucleotide sequence typically is examined at the DNA level and the coding region optimized for expression in the plant species determined using any suitable procedure, for example as described in Sardana et al. (1996, Plant Cell Reports 15:677-681). In this method, the standard deviation of codon usage, a measure of codon usage bias, may be calculated by first finding the squared proportional deviation of usage of each codon of the native gene relative to that of highly expressed plant genes, followed by a calculation of the average squared deviation. The formula used is: $1 SDCU = n = 1 N \left[\left(Xn - Yn\right) / Yn\right] 2 / N ,$ where Xn refers to the frequency of usage of codon n in highly expressed plant genes, where Yn to the frequency of usage of codon n in the gene of interest and N refers to the total number of codons in the gene of interest. A Table of codon usage from highly expressed genes of dicotyledonous plants is compiled using the data of Murray et al. (1989, Nuc Acids Res. 17:477-498).

One method of optimizing the nucleic acid sequence in accordance with the preferred codon usage for a particular plant cell type is based on the direct use, without performing any extra statistical calculations, of codon optimization Tables such as those provided on-line at the Codon Usage Database through the NIAS (National Institute of Agrobiological Sciences) DNA bank in Japan (Hypertext Transfer Protocol://World Wide Web (dot) kazusa (dot) or (dot) jp/codon/). The Codon Usage Database contains codon usage tables for a number of different species, with each codon usage Table having been statistically determined based on the data present in Genbank.

By using the above Tables to determine the most preferred or most favored codons for each amino acid in a particular species (for example, rice), a naturally-occurring nucleotide sequence encoding a protein of interest can be codon optimized for that particular plant species. This is effected by replacing codons that may have a low statistical incidence in the particular species genome with corresponding codons, in regard to an amino acid, that are statistically more favored. However, one or more less-favored codons may be selected to delete existing restriction sites, to create new ones at potentially useful junctions (5' and 3' ends to add signal peptide or termination cassettes, internal sites that might be used to cut and splice segments together to produce a correct full-length sequence), or to eliminate nucleotide sequences that may negatively effect mRNA stability or expression.

The naturally-occurring encoding nucleotide sequence may already, in advance of any modification, contain a number of codons that correspond to a statistically-favored codon in a particular plant species. Therefore, codon optimization of the native nucleotide sequence may comprise determining which codons, within the native nucleotide sequence, are not statistically-favored with regards to a particular plant, and modifying these codons in accordance with a codon usage table of the particular plant to produce a codon optimized derivative. A modified nucleotide sequence may be fully or partially optimized for plant codon usage provided that the protein encoded by the modified nucleotide sequence is produced at a level higher than the protein encoded by the corresponding naturally occurring or native gene. Construction of synthetic genes by altering the codon usage is described in for example PCT Patent Application 93/07278.

The exogenous polynucleotide can be a non-coding RNA.

As used herein the phrase 'non-coding RNA" refers to an RNA molecule which does not encode an amino acid sequence (a polypeptide). Examples of such non-coding RNA molecules include, but are not limited to, an antisense RNA, a pre-miRNA (precursor of a microRNA), or a precursor of a Piwi-interacting RNA (piRNA).

Non-limiting examples of non-coding RNA polynucleotides are provided in SEQ ID NOs:37 and 43.

Thus, disclosed are the nucleic acid sequences described hereinabove; fragments thereof, sequences hybridizable therewith, sequences homologous thereto, sequences encoding similar polypeptides with different codon usage, altered sequences characterized by mutations, such as deletion, insertion or substitution of one or more nucleotides, either naturally occurring or man induced, either randomly or in a targeted fashion.

The nucleic acid sequence can be capable of increasing yield, biomass, growth rate, vigor, oil content, fiber yield, fiber quality, water use efficiency, nitrogen use efficiency, fertilizer use efficiency and/or abiotic stress tolerance of a plant.

Also disclosed is an isolated polynucleotide comprising a nucleic acid sequence encoding a polypeptide which comprises an amino acid sequence at least about 80 %, at least about 81 %, at least about 82 %, at least about 83 %, at least about 84 %, at least about 85 %, at least about 86 %, at least about 87 %, at least about 88 %, at least about 89 %, at least about 90 %, at least about 91 %, at least about 92 %, at least about 93 %, at least about 93 %, at least about 94 %, at least about 95 %, at least about 96 %, at least about 97 %, at least about 98 %, at least about 99 %, or more say 100 % homologous to the amino acid sequence selected from the group consisting of SEQ ID NO: 246, 240-245, 247-465, 1974-3480, and 3675-3737.

The amino acid sequence can be capable of increasing yield, biomass, growth rate, vigor, oil content, fiber yield, fiber quality, water use efficiency, nitrogen use efficiency, fertilizer use efficiency and/or abiotic stress tolerance of a plant.

Also disclosed are fragments of the above described polypeptides and polypeptides having mutations, such as deletions, insertions or substitutions of one or more amino acids, either naturally occurring or man induced, either randomly or in a targeted fashion.

The term '"plant" as used herein encompasses whole plants, ancestors and progeny of the plants and plant parts, including seeds, shoots, stems, roots (including tubers), and plant cells, tissues and organs. The plant may be in any form including suspension cultures, embryos, meristematic regions, callus tissue, leaves, gametophytes, sporophytes, pollen, and microspores. Plants that are particularly useful in the methods of the invention include all plants which belong to the superfamily Viridiplantae, in particular monocotyledonous and dicotyledonous plants including a fodder or forage legume, ornamental plant, food crop, tree, or shrub selected from the list comprising Acacia spp., Acer spp., Actinidia spp., Aesculus spp., Agathis australis, Albizia amara, Alsophila tricolor, Andropogon spp., Arachis spp, Areca catechu, Astelia fragrans, Astragalus cicer, Baikiaea plurijuga, Betula spp., Brassica spp., Bruguiera gymnorrhiza, Burkea africana, Butea frondosa, Cadaba farinosa, Calliandra spp, Camellia sinensis, Canna indica, Capsicum spp., Cassia spp., Centroema pubescens, Chacoomeles spp., Cinnamomum cassia, Coffea arabica, Colophospermum mopane, Coronillia varia, Cotoneaster serotina, Crataegus spp., Cucumis spp., Cupressus spp., Cyathea dealbata, Cydonia oblonga, Cryptomeria japonica, Cymbopogon spp., Cynthea dealbata, Cydonia oblonga, Dalbergia monetaria, Davallia divaricata, Desmodium spp., Dicksonia squarosa, Dibeteropogon amplectens, Dioclea spp, Dolichos spp., Dorycnium rectum, Echinochloa pyramidalis, Ehraffia spp., Eleusine coracana, Eragrestis spp., Erythrina spp., Eucalypfus spp., Euclea schimperi, Eulalia vi/losa, Pagopyrum spp., Feijoa sellowlana, Fragaria spp., Flemingia spp, Freycinetia banksli, Geranium thunbergii, GinAgo biloba, Glycine javanica, Gliricidia spp, Gossypium hirsutum, Grevillea spp., Guibourtia coleosperma, Hedysarum spp., Hemaffhia altissima, Heteropogon contoffus, Hordeum vulgare, Hyparrhenia rufa, Hypericum erectum, Hypeffhelia dissolute, Indigo incamata, Iris spp., Leptarrhena pyrolifolia, Lespediza spp., Lettuca spp., Leucaena leucocephala, Loudetia simplex, Lotonus bainesli, Lotus spp., Macrotyloma axillare, Malus spp., Manihot esculenta, Medicago saliva, Metasequoia glyptostroboides, Musa sapientum, Nicotianum spp., Onobrychis spp., Ornithopus spp., Oryza spp., Peltophorum africanum, Pennisetum spp., Persea gratissima, Petunia spp., Phaseolus spp., Phoenix canariensis, Phormium cookianum, Photinia spp., Picea glauca, Pinus spp., Pisum sativam, Podocarpus totara, Pogonarthria fleckii, Pogonaffhria squarrosa, Populus spp., Prosopis cineraria, Pseudotsuga menziesii, Pterolobium stellatum, Pyrus communis, Quercus spp., Rhaphiolepsis umbellata, Rhopalostylis sapida, Rhus natalensis, Ribes grossularia, Ribes spp., Robinia pseudoacacia, Rosa spp., Rubus spp., Salix spp., Schyzachyrium sanguineum, Sciadopitys vefficillata, Sequoia sempervirens, Sequoiadendron giganteum, Sorghum bicolor, Spinacia spp., Sporobolus fimbriatus, Stiburus alopecuroides, Stylosanthos humilis, Tadehagi spp, Taxodium distichum, Themeda triandra, Trifolium spp., Triticum spp., Tsuga heterophylla, Vaccinium spp., Vicia spp., Vitis vinifera, Watsonia pyramidata, Zantedeschia aethiopica, Zea mays, amaranth, artichoke, asparagus, broccoli, Brussels sprouts, cabbage, canola, carrot, cauliflower, celery, collard greens, flax, kale, lentil, oilseed rape, okra, onion, potato, rice, soybean, straw, sugar beet, sugar cane, sunflower, tomato, squash tea, maize, wheat, barely, rye, oat, peanut, pea, lentil and alfalfa, cotton, rapeseed, canola, pepper, sunflower, tobacco, eggplant, eucalyptus, a tree, an ornamental plant, a perennial grass and a forage crop. Alternatively algae and other non-Viridiplantae can be used for the methods of the present invention.

The plant used in the method of the invention can be a crop plant such as rice, maize, wheat, barley, peanut, potato, sesame, olive tree, palm oil, banana, soybean, sunflower, canola, sugarcane, alfalfa, millet, leguminosae (bean, pea), flax, lupinus, rapeseed, tobacco, poplar, cotton and sorghum.

According to some embodiments of the invention, there is provided a plant cell exogenously expressing the nucleic acid construct of some embodiments of the invention and/or the polypeptide of some embodiments of the invention.

Expressing the exogenous polynucleotide of the invention within the plant can be effected by transforming one or more cells of the plant with the exogenous polynucleotide, followed by generating a mature plant from the transformed cells and cultivating the mature plant under conditions suitable for expressing the exogenous polynucleotide within the mature plant.

The transformation can be effected by introducing to the plant cell a nucleic acid construct which includes the exogenous polynucleotide of some embodiments of the invention and at least one promoter capable of directing transcription of the exogenous polynucleotide in the plant cell. Further details of suitable transformation approaches are provided herein below.

A nucleic acid construct is disclosed comprising the isolated polynucleotide of the invention, and a promoter for directing transcription of the nucleic acid sequence of the isolated polynucleotide in a host cell.

The isolated polynucleotide can be operably linked to the promoter sequence.

A coding nucleic acid sequence is "operably linked" to a regulatory sequence (e.g., promoter) if the regulatory sequence is capable of exerting a regulatory effect on the coding sequence linked thereto.

As used herein, the term "promoter" refers to a region of DNA which lies upstream of the transcriptional initiation site of a gene to which RNA polymerase binds to initiate transcription of RNA. The promoter controls where (e.g., which portion of a plant) and/or when (e.g., at which stage or condition in the lifetime of an organism) the gene is expressed.

Any suitable promoter sequence can be used by the nucleic acid construct of the present invention. The promoter can be a constitutive promoter, a tissue-specific, or an abiotic stress-inducible promoter.

Suitable constitutive promoters include, for example, CaMV 35S promoter (SEQ ID NO:4196; Odell et al., Nature 313:810-812, 1985); Arabidopsis At6669 promoter (SEQ ID NO:4195; see PCT Publication No. WO04081173A2); Arabidopsis new At6669 promoter (SEQ ID NO:4198); maize Ubi 1 (Christensen et al., Plant Sol. Biol. 18:675-689, 1992); rice actin (McElroy et al., Plant Cell 2:163-171, 1990); pEMU (Last et al., Theor. Appl. Genet. 81:581-588, 1991); CaMV 19S (Nilsson et al., Physiol. Plant 100:456-462, 1997); GOS2 (de Pater et al, Plant J Nov;2(6):837-44, 1992); ubiquitin (Christensen et al, Plant Mol. Biol. 18: 675-689, 1992); Rice cyclophilin (Bucholz et al, Plant Mol Biol. 25(5):837-43, 1994); Maize H3 histone (Lepetit et al, Mol. Gen. Genet. 231: 276-285, 1992); Actin 2 (An et al, Plant J. 10(1);107-121, 1996) and Synthetic Super MAS (Ni et al., The Plant Journal 7: 661-76, 1995). Other constitutive promoters include those in U.S. Pat. Nos. 5,659,026, 5,608,149; 5.608,144; 5,604,121; 5.569,597: 5.466,785; 5,399,680; 5,268,463; and 5,608,142.

Suitable tissue-specific promoters include, but not limited to, leaf-specific promoters [such as described, for example, by Yamamoto et al., Plant J. 12:255-265, 1997; Kwon et al., Plant Physiol. 105:357-67, 1994; Yamamoto et al., Plant Cell Physiol. 35:773-778, 1994; Gotor et al., Plant J. 3:509-18, 1993; Orozco et al., Plant Mol. Biol. 23:1129-1138, 1993; and Matsuoka et al., Proc. Natl. Acad. Sci. USA 90:9586-9590, 1993], seed-preferred promoters [e.g., from seed specific genes (Simon, et al., Plant Mol. Biol. 5. 191, 1985; Scofield, et al., J. Biol. Chem. 262: 12202, 1987; Baszczynski, et al., Plant Mol. Biol. 14: 633, 1990), Brazil Nut albumin (Pearson' et al., Plant Mol. Biol. 18: 235- 245, 1992), legumin (Ellis, et al. Plant Mol. Biol. 10: 203-214, 1988), Glutelin (rice) (Takaiwa, et al., Mol. Gen. Genet. 208: 15-22, 1986; Takaiwa, et al., FEBS Letts. 221: 43-47, 1987), Zein (Matzke et al., Plant Mol Biol, 143).323-32 1990), napA (Stalberg, et al., Planta 199: 515-519, 1996), Wheat SPA (Albanietal, Plant Cell, 9: 171- 184, 1997), sunflower oleosin (Cummins, etal., Plant Mol. Biol. 19: 873-876, 1992)], endosperm specific promoters [e.g., wheat LMW and HMW, glutenin-1 (Mol Gen Genet 216:81-90, 1989; NAR 17:461-2), wheat a, b and g gliadins (EMBO3:1409-15, 1984), Barley ltrl promoter, barley B1, C, D hordein (Theor Appl Gen 98:1253-62, 1999; Plant J 4:343-55, 1993; Mol Gen Genet 250:750- 60, 1996), Barley DOF (Mena et al., The Plant Journal, 116(1): 53- 62, 1998), Biz2 (EP99106056.7), Synthetic promoter (Vicente-Carbajosa et al., Plant J. 13: 629-640, 1998), rice prolamin NRP33, rice -globulin Glb-1 (Wu et al., Plant Cell Physiology 39(8) 885- 889, 1998), rice alpha-globulin REB/OHP-1 (Nakase et al. Plant Mol. Biol. 33: 513-S22, 1997), rice ADP-glucose PP (Trans Res 6:157-68, 1997), maize ESR gene family (Plant J 12:235-46, 1997), sorghum gamma- kafirin (PMB 32:1029-35, 1996); e.g., the Napin promoter (SEQ ID NO:4197)], embryo specific promoters [e.g., rice OSH1 (Sato et al., Proc. Natl. Acad. Sci. USA, 93: 8117-8122), KNOX (Postma-Haarsma et al, Plant Mol. Biol. 39:257-71, 1999), rice oleosin (Wu et at, J. Biochem., 123:386, 1998)], and flower-specific promoters [e.g., AtPRP4, chalene synthase (chsA) (Van der Meer, et al., Plant Mol. Biol. 15, 95-109, 1990), LAT52 (Twell et al., Mol. Gen Genet. 217:240-245; 1989), apetala- 3].

Suitable abiotic stress-inducible promoters include, but not limited to, salt-inducible promoters such as RD29A (Yamaguchi-Shinozalei et al., Mol. Gen. Genet. 236:331-340, 1993); drought-inducible promoters such as maize rab17 gene promoter (Pla et al., Plant Mol. Biol. 21:259-266, 1993), maize rab28 gene promoter (Busk et al., Plant J. 11:1285-1295, 1997) and maize Ivr2 gene promoter (Pelleschi et al., Plant Mol. Biol. 39:373-380, 1999); heat-inducible promoters such as heat tomato hsp80-promoter from tomato (U.S. Pat. No. 5,187,267).

The nucleic acid construct of some embodiments of the invention can further include an appropriate selectable marker and/or an origin of replication. The nucleic acid construct utilized can be a shuttle vector, which can propagate both in *E. coli* (wherein the construct comprises an appropriate selectable marker and origin of replication) and be compatible with propagation in cells. The construct according to some embodiments of the invention can be, for example, a plasmid, a bacmid, a phagemid, a cosmid, a phage, a virus or an artificial chromosome.

The nucleic acid construct of some embodiments of the invention can be utilized to stably or transiently transform plant cells. In stable transformation, the exogenous polynucleotide is integrated into the plant genome and as such it represents a stable and inherited trait. In transient transformation, the exogenous polynucleotide is expressed by the cell transformed but it is not integrated into the genome and as such it represents a transient trait.

There are various methods of introducing foreign genes into both monocotyledonous and dicotyledonous plants (Potrykus, I., Annu. Rev. Plant. Physiol., Plant. Mol. Biol. (1991) 42:205-225; Shimamoto et al., Nature (1989) 338:274-276).

The principle methods of causing stable integration of exogenous DNA into plant genomic DNA include two main approaches:
(i) Agrobacterium-mediated gene transfer (e.g., T-DNA using Agrobacterium tumefaciens or Agrobacterium rhizogenes); see for example, Klee et al. (1987) Annu. Rev. Plant Physiol. 38:467-486; Klee and Rogers in Cell Culture and Somatic Cell Genetics of Plants, Vol. 6, Molecular Biology of Plant Nuclear Genes, eds. Schell, J., and Vasil, L. K., Academic Publishers, San Diego, Calif. (1989) p. 2-25; Gatenby, in Plant Biotechnology, eds. Kung, S, and Arntzen, C. J., Butterworth Publishers, Boston, Mass. (1989) p. 93-112.
(ii) Direct DNA uptake: Paszkowski et al., in Cell Culture and Somatic Cell Genetics of Plants, Vol. 6, Molecular Biology of Plant Nuclear Genes eds. Schell, J., and Vasil, L. K., Academic Publishers, San Diego, Calif. (1989) p. 52-68; including methods for direct uptake of DNA into protoplasts, Toriyama, K. et al. (1988) Bio/Technology 6:1072-1074. DNA uptake induced by brief electric shock of plant cells: Zhang et al. Plant Cell Rep. (1988) 7:379-384. Fromm et al. Nature (1986) 319:791-793. DNA injection into plant cells or tissues by particle bombardment, Klein et al. Bio/Technology (1988) 6:559-563; McCabe et al. Bio/Technology (1988) 6:923-926; Sanford, Physiol. Plant. (1990) 79:206-209; by the use of micropipette systems: Neuhaus et al., Theor. Appl. Genet. (1987) 75:30-36; Neuhaus and Spangenberg, Physiol. Plant. (1990) 79:213-217; glass fibers or silicon carbide whisker transformation of cell cultures, embryos or callus tissue, U.S. Pat. No. 5,464,765 or by the direct incubation of DNA with germinating pollen, DeWet et al. in Experimental Manipulation of Ovule Tissue, eds. Chapman, G. P. and Mantell, S. H. and Daniels, W. Longman, London, (1985) p. 197-209; and Ohta, Proc. Natl. Acad. Sci. USA (1986) 83:715-719.

The Agrobacterium system includes the use of plasmid vectors that contain defined DNA segments that integrate into the plant genomic DNA. Methods of inoculation of the plant tissue vary depending upon the plant species and the Agrobacterium delivery system. A widely used approach is the leaf disc procedure which can be performed with any tissue explant that provides a good source for initiation of whole plant differentiation. See, e.g., Horsch et al. in Plant Molecular Biology Manual A5, Kluwer Academic Publishers, Dordrecht (1988) p. 1-9. A supplementary approach employs the Agrobacterium delivery system in combination with vacuum infiltration. The Agrobacterium system is especially viable in the creation of transgenic dicotyledonous plants.

There are various methods of direct DNA transfer into plant cells. In electroporation, the protoplasts are briefly exposed to a strong electric field. In microinjection, the DNA is mechanically injected directly into the cells using very small micropipettes. In microparticle bombardment, the DNA is adsorbed on microprojectiles such as magnesium sulfate crystals or tungsten particles, and the microprojectiles are physically accelerated into cells or plant tissues.

Following stable transformation plant propagation is exercised. The most common method of plant propagation is by seed. Regeneration by seed propagation, however, has the deficiency that due to heterozygosity there is a lack of uniformity in the crop, since seeds are produced by plants according to the genetic variances governed by Mendelian rules. Basically, each seed is genetically different and each will grow with its own specific traits. Therefore, it is preferred that the transformed plant be produced such that the regenerated plant has the identical traits and characteristics of the parent transgenic plant. For this reason it is preferred that the transformed plant be regenerated by micropropagation which provides a rapid, consistent reproduction of the transformed plants.

Micropropagation is a process of growing new generation plants from a single piece of tissue that has been excised from a selected parent plant or cultivar. This process permits the mass reproduction of plants having the preferred tissue expressing the fusion protein. The new generation plants which are produced are genetically identical to, and have all of the characteristics of, the original plant. Micropropagation allows mass production of quality plant material in a short period of time and offers a rapid multiplication of selected cultivars in the preservation of the characteristics of the original transgenic or transformed plant. The advantages of cloning plants are the speed of plant multiplication and the quality and uniformity of plants produced.

Micropropagation is a multi-stage procedure that requires alteration of culture medium or growth conditions between stages. Thus, the micropropagation process involves four basic stages: Stage one, initial tissue culturing; stage two, tissue culture multiplication; stage three, differentiation and plant formation; and stage four, greenhouse culturing and hardening. During stage one, initial tissue culturing, the tissue culture is established and certified contaminant-free. During stage two, the initial tissue culture is multiplied until a sufficient number of tissue samples are produced to meet production goals. During stage three, the tissue samples grown in stage two are divided and grown into individual plantlets. At stage four, the transformed plantlets are transferred to a greenhouse for hardening where the plants' tolerance to light is gradually increased so that it can be grown in the natural environment.

The transgenic plants can be generated by transient transformation of leaf cells, meristematic cells or the whole plant.

Transient transformation can be effected by any of the direct DNA transfer methods described above or by viral infection using modified plant viruses.

Viruses that have been shown to be useful for the transformation of plant hosts include CaMV, Tobacco mosaic virus (TMV), brome mosaic virus (BMV) and Bean Common Mosaic Virus (BV or BCMV). Transformation of plants using plant viruses is described in U.S. Pat. No. 4,855,237 (bean golden mosaic virus; BGV), EP-A 67,553 (TMV), Japanese Published Application No. 63-14693 (TMV), EPA 194,809 (BV), EPA 278,667 (BV); and Gluzman, Y. et al., Communications in Molecular Biology: Viral Vectors, Cold Spring Harbor Laboratory, New York, pp. 172-189 (1988). Pseudovirus particles for use in expressing foreign DNA in many hosts, including plants are described in WO 87/06261.

The virus used for transient transformations can be avirulent and thus incapable of causing severe symptoms such as reduced growth rate, mosaic, ring spots, leaf roll, yellowing, streaking, pox formation, tumor formation and pitting. A suitable avirulent virus may be a naturally occurring avirulent virus or an artificially attenuated virus. Virus attenuation may be effected by using methods well known in the art including, but not limited to, sub-lethal heating, chemical treatment or by directed mutagenesis techniques such as described, for example, by Kurihara and Watanabe (Molecular Plant Pathology 4:259-269, 2003), Gal-on et al. (1992), Atreya et al. (1992) and Huet et al. (1994).

Suitable virus strains can be obtained from available sources such as, for example, the American Type culture Collection (ATCC) or by isolation from infected plants. Isolation of viruses from infected plant tissues can be effected by techniques well known in the art such as described, for example by Foster and Tatlor, Eds. "Plant Virology Protocols: From Virus Isolation to Transgenic Resistance (Methods in Molecular Biology (Humana Pr), Vol 81)", Humana Press, 1998. Briefly, tissues of an infected plant believed to contain a high concentration of a suitable virus, preferably young leaves and flower petals, are ground in a buffer solution (e.g., phosphate buffer solution) to produce a virus infected sap which can be used in subsequent inoculations.

Construction of plant RNA viruses for the introduction and expression of non-viral exogenous polynucleotide sequences in plants is demonstrated by the above references as well as by Dawson, W. O. et al., Virology (1989) 172:285-292; Takamatsu et al. EMBO J. (1987) 6:307-311; French et al. Science (1986) 231:1294-1297; Takamatsu et al. FEBS Letters (1990) 269:73-76; and U.S. Pat. No. 5,316,931.

When the virus is a DNA virus, suitable modifications can be made to the virus itself. Alternatively, the virus can first be cloned into a bacterial plasmid for ease of constructing the desired viral vector with the foreign DNA. The virus can then be excised from the plasmid. If the virus is a DNA virus, a bacterial origin of replication can be attached to the viral DNA, which is then replicated by the bacteria. Transcription and translation of this DNA will produce the coat protein which will encapsidate the viral DNA. If the virus is an RNA virus, the virus is generally cloned as a cDNA and inserted into a plasmid. The plasmid is then used to make all of the constructions. The RNA virus is then produced by transcribing the viral sequence of the plasmid and translation of the viral genes to produce the coat protein(s) which encapsidate the viral RNA.

A plant viral polynucleotide is also disclosed in which the native coat protein coding sequence has been deleted from a viral polynucleotide, a non-native plant viral coat protein coding sequence and a non-native promoter, preferably the subgenomic promoter of the non-native coat protein coding sequence, capable of expression in the plant host, packaging of the recombinant plant viral polynucleotide, and ensuring a systemic infection of the host by the recombinant plant viral polynucleotide, has been inserted. Alternatively, the coat protein gene may be inactivated by insertion of the non-native polynucleotide sequence within it, such that a protein is produced. The recombinant plant viral polynucleotide may contain one or more additional non-native subgenomic promoters. Each non-native subgenomic promoter is capable of transcribing or expressing adjacent genes or polynucleotide sequences in the plant host and incapable of recombination with each other and with native subgenomic promoters. Non-native (foreign) polynucleotide sequences may be inserted adjacent the native plant viral subgenomic promoter or the native and a non-native plant viral subgenomic promoters if more than one polynucleotide sequence is included. The non-native polynucleotide sequences are transcribed or expressed in the host plant under control of the subgenomic promoter to produce the desired products.

A recombinant plant viral polynucleotide is also disclosed as the one mentioned above except that the native coat protein coding sequence is placed adjacent one of the non-native coat protein subgenomic promoters instead of a non-native coat protein coding sequence.

A recombinant plant viral polynucleotide is also disclosed in which the native coat protein gene is adjacent its subgenomic promoter and one or more non-native subgenomic promoters have been inserted into the viral polynucleotide. The inserted non-native subgenomic promoters are capable of transcribing or expressing adjacent genes in a plant host and are incapable of recombination with each other and with native subgenomic promoters. Non-native polynucleotide sequences may be inserted adjacent the non-native subgenomic plant viral promoters such that the sequences are transcribed or expressed in the host plant under control of the subgenomic promoters to produce the desired product.

A recombinant plant viral polynucleotide is also disclosed as the one mentioned above except that the native coat protein coding sequence is replaced by a non-native coat protein coding sequence.

The viral vectors are encapsidated by the coat proteins encoded by the recombinant plant viral polynucleotide to produce a recombinant plant virus. The recombinant plant viral polynucleotide or recombinant plant virus is used to infect appropriate host plants. The recombinant plant viral polynucleotide is capable of replication in the host, systemic spread in the host, and transcription or expression of foreign gene(s) (exogenous polynucleotide) in the host to produce the desired protein.

Techniques for inoculation of viruses to plants may be found in Foster and Taylor, eds. "Plant Virology Protocols: From Virus Isolation to Transgenic Resistance (Methods in Molecular Biology (Humana Pr), Vol 81)", Humana Press, 1998; Maramorosh and Koprowski, eds. "Methods in Virology" 7 vols, Academic Press, New York 1967-1984; Hill, S.A. "Methods in Plant Virology", Blackwell, Oxford, 1984; Walkey, D.G.A. "Applied Plant Virology", Wiley, New York, 1985; and Kado and Agrawa, eds. "Principles and Techniques in Plant Virology", Van Nostrand-Reinhold, New York.

In addition to the above, the polynucleotide of the present invention can also be introduced into a chloroplast genome thereby enabling chloroplast expression.

A technique for introducing exogenous polynucleotide sequences to the genome of the chloroplasts is known. This technique involves the following procedures. First, plant cells are chemically treated so as to reduce the number of chloroplasts per cell to about one. Then, the exogenous polynucleotide is introduced via particle bombardment into the cells with the aim of introducing at least one exogenous polynucleotide molecule into the chloroplasts. The exogenous polynucleotide is selected such that it is integratable into the chloroplast's genome via homologous recombination which is readily effected by enzymes inherent to the chloroplast. To this end, the exogenous polynucleotide includes, in addition to a gene of interest, at least one polynucleotide stretch which is derived from the chloroplast's genome. In addition, the exogenous polynucleotide includes a selectable marker, which serves by sequential selection procedures to ascertain that all or substantially all of the copies of the chloroplast genomes following such selection will include the exogenous polynucleotide. Further details relating to this technique are found in U.S. Pat. Nos. 4,945,050; and 5,693,507. A polypeptide can thus be produced by the protein expression system of the chloroplast and become integrated into the chloroplast's inner membrane.

Since yield (or other parameters affecting yield such as growth rate, biomass, vigor, content of seeds, oil content and the like), fiber yield and/or quality, water use efficiency, fertilizer use efficiency, nitrogen use efficiency and/or abiotic stress tolerance in plants can involve multiple genes acting additively or in synergy (see, for example, in Quesda et al., Plant Physiol. 130:951-063, 2002), the invention also envisages expressing a plurality of exogenous polynucleotides in a single host plant to thereby achieve superior effect on yield, fiber yield and/or quality, water use efficiency, fertilizer use efficiency, nitrogen use efficiency and/or abiotic stress tolerance.

Expressing a plurality of exogenous polynucleotides in a single host plant can be effected by co-introducing multiple nucleic acid constructs, each including a different exogenous polynucleotide, into a single plant cell. The transformed cell can then be regenerated into a mature plant using the methods described hereinabove.

Alternatively, expressing a plurality of exogenous polynucleotides in a single host plant can be effected by co-introducing into a single plant-cell a single nucleic-acid construct including a plurality of different exogenous polynucleotides. Such a construct can be designed with a single promoter sequence which can transcribe a polycistronic messenger RNA including all the different exogenous polynucleotide sequences. To enable co-translation of the different polypeptides encoded by the polycistronic messenger RNA, the polynucleotide sequences can be inter-linked via an internal ribosome entry site (IRES) sequence which facilitates translation of polynucleotide sequences positioned downstream of the IRES sequence. In this case, a transcribed polycistronic RNA molecule encoding the different polypeptides described above will be translated from both the capped 5' end and the two internal IRES sequences of the polycistronic RNA molecule to thereby produce in the cell all different polypeptides. Alternatively, the construct can include several promoter sequences each linked to a different exogenous polynucleotide sequence.

The plant cell transformed with the construct including a plurality of different exogenous polynucleotides can be regenerated into a mature plant, using the methods described hereinabove.

Alternatively, expressing a plurality of exogenous polynucleotides can be effected by introducing different nucleic acid constructs, including different exogenous polynucleotides, into a plurality of plants. The regenerated transformed plants can then be cross-bred and resultant progeny selected for superior yield (e.g., growth rate, biomass, vigor, oil content), fiber yield and/or quality, water use efficiency, fertilizer use efficiency, nitrogen use efficiency and/or abiotic stress tolerance traits, using conventional plant breeding techniques.

The plant expressing the exogenous polynucleotide(s) can be grown under non-stress or normal conditions (e.g., biotic conditions and/or conditions with sufficient water, nutrients such as nitrogen and fertilizer). Such conditions, which depend on the plant being grown, are known to those skilled in the art of agriculture, and are further, described hereinbelow.

The method may further comprise growing the plant expressing the exogenous polynucleotide under the abiotic stress.

Non-limiting examples of abiotic stress conditions include, salinity, drought, water deprivation, excess of water (e.g., flood, waterlogging), etiolation, low temperature, high temperature, heavy metal toxicity, anaerobiosis, nutrient deficiency, nutrient excess, atmospheric pollution and UV irradiation.

Thus, the invention encompasses plants exogenously expressing the polynucleotide(s), the nucleic acid constructs and/or polypeptide(s) of the invention. Once expressed within the plant cell or the entire plant, the level of the polypeptide encoded by the exogenous polynucleotide can be determined by methods well known in the art such as, activity assays, Western blots using antibodies capable of specifically binding the polypeptide, Enzyme-Linked Immuno Sorbent Assay (ELISA), radio-immuno-assays (RIA), immunohistochemistry, immunocytochemistry, immunofluorescence and the like.

Methods of determining the level in the plant of the RNA transcribed from the exogenous polynucleotide are well known in the art and include, for example, Northern blot analysis, reverse transcription polymerase chain reaction (RT-PCR) analysis (including quantitative, semi-quantitative or real-time RT-PCR) and RNA-*in situ* hybridization.

The sequence information and annotations uncovered by the present teachings can be harnessed in favor of classical breeding. Thus, sub-sequence data of those polynucleotides described above, can be used as markers for marker assisted selection (MAS), in which a marker is used for indirect selection of a genetic determinant or determinants of a trait of interest (e.g., biomass, growth rate, oil content, fiber yield and/or quality, yield, abiotic stress tolerance, water use efficiency, nitrogen use efficiency and/or fertilizer use efficiency). Nucleic acid data of the present teachings (DNA or RNA sequence) may contain or be linked to polymorphic sites or genetic markers on the genome such as restriction fragment length polymorphism (RFLP), microsatellites and single nucleotide polymorphism (SNP), DNA fingerprinting (DFP), amplified fragment length polymorphism (AFLP), expression level polymorphism, polymorphism of the encoded polypeptide and any other polymorphism at the DNA or RNA sequence.

Examples of marker assisted selections include, but are not limited to, selection for a morphological trait (e.g., a gene that affects form, coloration, male sterility or resistance such as the presence or absence of awn, leaf sheath coloration, height, grain color, aroma of rice); selection for a biochemical trait (e.g., a gene that encodes a protein that can be extracted and observed; for example, isozymes and storage proteins); selection for a biological trait (e.g., pathogen races or insect biotypes based on host pathogen or host parasite interaction can be used as a marker since the genetic constitution of an organism can affect its susceptibility to pathogens or parasites).

The polynucleotides and polypeptides described hereinabove can be used in a wide range of economical plants, in a safe and cost effective manner.

Plant lines exogenously expressing the polynucleotide or the polypeptide of the invention can be screened to identify those that show the greatest increase of the desired plant trait.

The effect of the transgene (the exogenous polynucleotide encoding the polypeptide) on abiotic stress tolerance can be determined using known methods such as detailed below and in the Examples section which follows.

***Plant's growth rate, biomass, yield and*/*or vigor* -** Plant vigor can be calculated by the increase in growth parameters such as leaf area, fiber length, rosette diameter, plant fresh weight and the like per time.

The growth rate can be measured using digital analysis of growing plants. For example, images of plants growing in greenhouse on plot basis can be captured every 3 days and the rosette area can be calculated by digital analysis. Rosette area growth is calculated using the difference of rosette area between days of sampling divided by the difference in days between samples.

Evaluation of growth rate can be also done by measuring plant biomass produced, rosette area, leaf size or root length per time (can be measured in cm² per day of leaf area).

***Relative growth area*** can be calculated using Formula II. $Relative growth rate area = Regression coefficient of area along time course$

Thus, the relative growth area rate is in units of 1/day and length growth rate is in units of 1/day.

***Seed yield*** - Evaluation of the seed yield per plant can be done by measuring the amount (weight or size) or quantity (*i.e.,* number) of dry seeds produced and harvested from 8-16 plants and divided by the number of plants.

For example, the total seeds from 8-16 plants can be collected, weighted using e.g., an analytical balance and the total weight can be divided by the number of plants. Seed yield per growing area can be calculated in the same manner while taking into account the growing area given to a single plant. Increase seed yield per growing area could be achieved by increasing seed yield per plant, and/or by increasing number of plants capable of growing in a given area.

Seed yield can be expressed as thousand kernel weight (1000-weight), which is extrapolated from the number of filled seeds counted and their total weight. Hence, an increased 1000-weight may result from an increased seed size and/or seed weight (e.g., increase in embryo size and/or endosperm size). For example, the weight of 1000 seeds can be determined as follows: seeds are scattered on a glass tray and a picture is taken. Each sample is weighted and then using the digital analysis, the number of seeds in each sample is calculated.

The 1000 seeds weight can be calculated using formula III: $1000 Seed Weight = number of seed in sample/ sample weight \times 1000$

The Harvest Index can be calculated using Formula IV $Harvest Index = Average seed yield per plant/ Average dry weight$

Since the transgenic plants of the invention have increased yield, it is likely that these plants exhibit an increased growth rate (during at least part of their life cycle), relative to the growth rate of corresponding wild type plants at a corresponding stage in their life cycle. The increased growth rate may be specific to one or more parts of a plant (including seeds), or may be throughout substantially the whole plant. A plant having an increased growth rate may also exhibit early flowering. Increased growth rate during the early stages in the life cycle of a plant may reflect enhanced vigor. The increase in growth rate may alter the harvest cycle (early maturing) of a plant allowing plants to be sown later and/or harvested sooner than would otherwise be possible. If the growth rate is sufficiently increased, it may allow for the sowing of further seeds of the same plant species (for example sowing and harvesting of rice plants followed by sowing and harvesting of further rice plants all within one conventional growing period). Similarly, if the growth rate is sufficiently increased, it may allow for the sowing of further seeds of different plants species (for example the sowing and harvesting of rice plants followed by, for example, the sowing and optional harvesting of soybean, potato or any other suitable plant). Harvesting additional times from the same rootstock in the case of some plants may also be possible. Altering the harvest cycle of a plant may lead to an increase in annual biomass production per area (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size).

Increased yield of corn may be manifested as one or more of the following: increase in the number of plants per growing area, increase in the number of ears per plant, increase in the number of rows per ear, number of kernels per ear row, kernel weight, thousand kernel weight (1000-weight), ear length/diameter, increase oil content per kernel and increase starch content per kernel.

As mentioned, the increase of plant yield can be determined by various parameters. For example, increased yield of rice may be manifested by an increase in one or more of the following: number of plants per growing area, number of panicles per plant, number of spikelets per panicle, number of flowers per panicle, increase in the seed filling rate, increase in thousand kernel weight (1000-weight), increase oil content per seed, increase starch content per seed, among others. An increase in yield may also result in modified architecture, or may occur because of modified architecture.

Similarly, increased yield of soybean may be manifested by an increase in one or more of the following: number of plants per growing area, number of pods per plant, number of seeds per pod, increase in the seed filling rate, increase in thousand seed weight (1000-weight), reduce pod shattering, increase oil content per seed, increase protein content per seed, among others. An increase in yield may also result in modified architecture, or may occur because of modified architecture.

Increased yield of canola may be manifested by an increase in one or more of the following: number of plants per growing area, number of pods per plant, number of seeds per pod, increase in the seed filling rate, increase in thousand seed weight (1000-weight), reduce pod shattering, increase oil content per seed, among others. An increase in yield may also result in modified architecture, or may occur because of modified architecture.

Increased yield of cotton may be manifested by an increase in one or more of the following: number of plants per growing area, number of bolls per plant, number of seeds per boll, increase in the seed filling rate, increase in thousand seed weight (1000-weight), increase oil content per seed, improve fiber length, fiber strength, among others. An increase in yield may also result in modified architecture, or may occur because of modified architecture.

***Oil content*** - The oil content of a plant can be determined by extraction of the oil from the seed or the vegetative portion of the plant. Briefly, lipids (oil) can be removed from the plant (e.g., seed) by grinding the plant tissue in the presence of specific solvents (e.g., hexane or petroleum ether) and extracting the oil in a continuous extractor. Indirect oil content analysis can be carried out using various known methods such as Nuclear Magnetic Resonance (NMR) Spectroscopy, which measures the resonance energy absorbed by hydrogen atoms in the liquid state of the sample [See for example, Conway TF. and Earle FR., 1963, Journal of the American Oil Chemists' Society; Springer Berlin / Heidelberg, ISSN: 0003-021X (Print) 1558-9331 (Online)]; the Near Infrared (NI) Spectroscopy, which utilizes the absorption of near infrared energy (1100-2500 nm) by the sample; and a method described in WO/2001/023884, which is based on extracting oil a solvent, evaporating the solvent in a gas stream which forms oil particles, and directing a light into the gas stream and oil particles which forms a detectable reflected light.

Fiber length can be measured using fibrograph. The fibrograph system was used to compute length in terms of "Upper Half Mean" length. The upper half mean (UHM) is the average length of longer half of the fiber distribution. The fibrograph measures length in span lengths at a given percentage point (Hypertext Transfer Protocol://World Wide Web (dot) cottoninc (dot) com/ClassificationofCotton/?Pg=4#Length).

***Abiotic stress tolerance* -** Transformed (*i.e.,* expressing the transgene) and non-transformed (wild type) plants are exposed to an abiotic stress condition, such as water deprivation, suboptimal temperature (low temperature, high temperature), nutrient deficiency, nutrient excess, a salt stress condition, osmotic stress, high or low light conditions, heavy metal toxicity, anaerobiosis, atmospheric pollution and UV irradiation.

***Salinity tolerance assay*** - Transgenic plants with tolerance to high salt concentrations are expected to exhibit better germination, seedling vigor or growth in high salt. Salt stress can be effected in many ways such as, for example, by irrigating the plants with a hyperosmotic solution, by cultivating the plants hydroponically in a hyperosmotic growth solution (e.g., Hoagland solution with added salt), or by culturing the plants in a hyperosmotic growth medium [e.g., 50 % Murashige-Skoog medium (MS medium) with added salt]. Since different plants vary considerably in their tolerance to salinity, the salt concentration in the irrigation water, growth solution, or growth medium can be adjusted according to the specific characteristics of the specific plant cultivar or variety, so as to inflict a mild or moderate effect on the physiology and/or morphology of the plants (for guidelines as to appropriate concentration see, Bernstein and Kafkafi, Root Growth Under Salinity Stress In: Plant Roots, The Hidden Half 3rd ed. Waisel Y, Eshel A and Kafkafi U. (editors) Marcel Dekker Inc., New York, 2002, and reference therein).

For example, a salinity tolerance test can be performed by irrigating plants at different developmental stages with increasing concentrations of sodium chloride (for example 50 mM, 100 mM, 200 mM, 400 mM NaCl) applied from the bottom and from above to ensure even dispersal of salt. Following exposure to the stress condition the plants are frequently monitored until substantial physiological and/or morphological effects appear in wild type plants. Thus, the external phenotypic appearance, degree of chlorosis and overall success to reach maturity and yield progeny are compared between control and transgenic plants. Quantitative parameters of tolerance measured include, but are not limited to, the average wet and dry weight, growth rate, leaf size, leaf coverage (overall leaf area), the weight of the seeds yielded, the average seed size and the number of seeds produced per plant. Transformed plants not exhibiting substantial physiological and/or morphological effects, or exhibiting higher biomass than wild-type plants, are identified as abiotic stress tolerant plants.

***Osmotic tolerance test* -** Osmotic stress assays (including sodium chloride and PEG assays) are conducted to determine if an osmotic stress phenotype was sodium chloride-specific or if it was a general osmotic stress related phenotype. Plants which are tolerant to osmotic stress may have more tolerance to drought and/or freezing. For salt and osmotic stress experiments, the medium is supplemented for example with 50 mM, 100 mM, 200 mM NaCl or 15 %, 20 % or 25 % PEG.

***Drought tolerance assay*** - Soil-based drought screens are performed with plants overexpressing the polynucleotides detailed above. Seeds from control Arabidopsis plants, or other transgenic plants overexpressing the polypeptide of the invention are germinated and transferred to pots. Drought stress is obtained after irrigation is ceased. Transgenic and control plants are compared to each other when the majority of the control plants develop severe wilting. Plants are re-watered after obtaining a significant fraction of the control plants displaying a severe wilting. Plants are ranked comparing to controls for each of two criteria: tolerance to the drought conditions and recovery (survival) following re-watering.

Quantitative parameters of tolerance measured include, but are not limited to, the average wet and dry weight, growth rate, leaf size, leaf coverage (overall leaf area), the weight of the seeds yielded, the average seed size and the number of seeds produced per plant. Transformed plants not exhibiting substantial physiological and/or morphological effects, or exhibiting higher biomass than wild-type plants, are identified as drought stress tolerant plants

***Cold stress tolerance*** - One way to analyze cold stress is as follows. Mature (25 day old) plants are transferred to 4 °C chambers for 1 or 2 weeks, with constitutive light. Later on plants are moved back to greenhouse. Two weeks later damages from chilling period, resulting in growth retardation and other phenotypes, are compared between control and transgenic plants, by measuring plant weight (wet and dry), and by comparing growth rates measured as time to flowering, plant size, yield, and the like.

***Heat stress tolerance*** - One way to measure heat stress tolerance is by exposing the plants to temperatures above 34 °C for a certain period. Plant tolerance is examined after transferring the plants back to 22 °C for recovery and evaluation after 5 days relative to internal controls (non-transgenic plants) or plants not exposed to neither cold or heat stress.

***Germination tests* -** Germination tests compare the percentage of seeds from transgenic plants that could complete the germination process to the percentage of seeds from control plants that are treated in the same manner. Normal conditions are considered for example, incubations at 22 °C under 22-hour light 2-hour dark daily cycles. Evaluation of germination and seedling vigor is conducted between 4 and 14 days after planting. The basal media is 50 % MS medium (Murashige and Skoog, 1962 Plant Physiology 15, 473-497).

Germination is checked also at unfavorable conditions such as cold (incubating at temperatures lower than 10 °C instead of 22 °C) or using seed inhibition solutions that contain high concentrations of an osmolyte such as sorbitol (at concentrations of 50 mM, 100 mM, 200 mM, 300 mM, 500 mM, and up to 1000 mM) or applying increasing concentrations of salt (of 50 mM, 100 mM, 200 mM, 300 mM, 500 mM NaCl).

***Water use efficiency (WUE)*** - can be determined as the biomass produced per unit transpiration. To analyze WUE, leaf relative water content can be measured in control and transgenic plants. Fresh weight (FW) is immediately recorded; then leaves are soaked for 8 hours in distilled water at room temperature in the dark, and the turgid weight (TW) is recorded. Total dry weight (DW) is recorded after drying the leaves at 60 °C to a constant weight. Relative water content (RWC) is calculated according to the following Formula V: $RWC = \left(FW - DW/TW - DW\right) \times 100$

Plants that maintain high relative water content (RWC) compared to control lines are considered more tolerant to drought than those exhibiting reduced relative water content. A non limiting example in Arabidopsis is when water uptake by roots matches water loss by transpiration from leaves. Under these circumstances the plant is determined to be under equilibrium and the RWC is about 0.9. When the RWC of transgenic plants decreases significantly less as compared to wild type plants, the transgenic plants are considered more tolerant to drought [Gaxiola et al. PNAS September 25, 2001 vol. 98 no. 20 11444-11449].

***Fertilizer use efficiency*** - To analyze whether the transgenic plants are more responsive to fertilizers, plants are grown in agar plates or pots containing growth media with a limited amount of fertilizer (e.g., nitrogen, phosphate, potassium), essentially as described in Yanagisawa et al (Proc Natl Acad Sci U S A. 2004; 101:7833-8). The plants are analyzed for their overall size, time to flowering, yield, protein content of shoot, grain and/or seed production. The parameters checked are the overall size of the mature plant, its wet and dry weight, the weight of the seeds yielded, the average seed size and the number of seeds produced per plant. Other parameters that may be tested are: the chlorophyll content of leaves (as nitrogen plant status and the degree of leaf greenness is highly correlated), amino acid and the total protein content of the seeds or other plant parts such as leaves or shoots, oil content, etc. In this way, nitrogen use efficiency (NUE), phosphate use efficiency (PUE) and potassium use efficiency (KUE) are assessed, checking the ability of the transgenic plants which express the exogenous polynucleotide of the invention to thrive under nutrient restraining conditions. For example, to analyze whether the transgenic Arabidopsis plants are more responsive to phosphate, plants are grown in 250 mM (phosphate deficient conditions) or 1 mM (optimal phosphate concentration). To test the potassium use efficiency, Arabidopsis plants which express the exogenous polynucleotide of the invention are grown in 0.03 mM potassium (potassium deficient conditions) or 3 mM potassium (optimal potassium concentration) essentially as described by Watson et al. Plant Physiol. (1996) 11 1 : 1077-1 083.

***Nitrogen determination*** - The procedure for N (nitrogen) concentration determination in the structural parts of the plants involves the potassium persulfate digestion method to convert organic N to NO₃⁻ (Purcell and King 1996 Argon. J. 88:111-113, the modified Cd⁻ mediated reduction of NO₃⁻ to NO₂⁻ (Vodovotz 1996 Biotechniques 20:390-394) and the measurement of nitrite by the Griess assay (Vodovotz 1996, supra). The absorbance values are measured at 550 nm against a standard curve of NaNO₂. The procedure is described in details in Samonte et al. 2006 Agron. J. 98:168-176.

***Nitrogen use efficiency*** - To analyze whether the transgenic Arabidopsis plants are more responsive to nitrogen plant are grown in 0.75- 1.5 mM (nitrogen deficient conditions) or 6-10 mM (optimal nitrogen concentration). Plants are allowed to grow for additional 20 days or until seed production. The plants are then analyzed for their overall size, time to flowering, yield, protein content of shoot and/or grain/ seed production. The parameters checked can be the overall size of the plant, wet and dry weight, the weight of the seeds yielded, the average seed size and the number of seeds produced per plant. Other parameters that may be tested are: the chlorophyll content of leaves (as nitrogen plant status and the degree of leaf greenness is highly correlated), amino acid and the total protein content of the seeds or other plant parts such as leaves or shoots and oil content. Transformed plants not exhibiting substantial physiological and/or morphological effects, or exhibiting higher measured parameters levels than wild-type plants, are identified as nitrogen use efficient plants.

***Nitrogen use efficiency assay using plantlets* -** The assay is done according to Yanagisawa-S. et al. with minor modifications ("Metabolic engineering with Dof1 transcription factor in plants: Improved nitrogen assimilation and growth under low-nitrogen conditions" Proc. Natl. Acad. Sci. USA 101, 7833-7838). Briefly, transgenic plants which are grown for 7-10 days in 0.5 x MS [Murashige-Skoog] supplemented with a selection agent are transferred to two nitrogen-limiting conditions: MS media in which the combined nitrogen concentration (NH₄NO₃ and KNO₃) was 0.2 mM or 0.05 mM. Plants are allowed to grow for additional 30-40 days and then photographed, individually removed from the Agar (the shoot without the roots) and immediately weighed (fresh weight) for later statistical analysis. Constructs for which only T1 seeds are available are sown on selective media and at least 25 seedlings (each one representing an independent transformation event) are carefully transferred to the nitrogen-limiting media. For constructs for which T2 seeds are available, different transformation events are analyzed. Usually, 25 randomly selected plants from each event are transferred to the nitrogen-limiting media allowed to grow for 3-4 additional weeks and individually weighed at the end of that period. Transgenic plants are compared to control plants grown in parallel under the same conditions. Mock-transgenic plants expressing the uidA reporter gene (GUS) under the same promoter are used as control.

***Grain protein concentration*** - Grain protein content (g grain protein m⁻²) is estimated as the product of the mass of grain N (g grain N m⁻²) multiplied by the N/protein conversion ratio of k-5.13 (Mosse 1990, supra). The grain protein concentration is estimated as the ratio of grain protein content per unit mass of the grain (g grain protein kg⁻¹ grain).

Thus, the present invention is of high agricultural value for promoting the yield, biomass, growth rate, vigor, water use efficiency, fertilizer use efficiency, nitrogen use efficiency and abiotic stress tolerance of commercially desired crops (e.g., biomass of vegetative organ such as poplar wood, or reproductive organ such as number of seeds or seed biomass).

As used herein the term "about" refers to ± 10 %.

The terms "comprises", "comprising", "includes", "including", "having" and their conjugates mean "including but not limited to".

The term "consisting of means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader..

### EXAMPLE 1

### IDENTIFYING GENES WHICH IMPROVE YIELD AND AGRONOMICAL IMPORTANT TRAITS IN PLANTS

The present inventors have identified polynucleotides which expression thereof in plants can increase yield, fiber yield, fiber quality, growth rate, vigor, biomass, growth rate, oil content, abiotic stress tolerance (ABST), nitrogen use efficiency (NUE), water use efficiency (WUE) and fertilizer use efficiency (FUE) of a plant, as follows.

All nucleotide sequence datasets used here were originated from publicly available databases or from performing sequencing using the Solexa technology (e.g. Barley and Sorghum). Sequence data from 100 different plant species was introduced into a single, comprehensive database. Other information on gene expression, protein annotation, enzymes and pathways were also incorporated. Major databases used include:
- ***Genomes***
   ∘ Arabidopsis genome [TAIR genome version 6 (Hypertext Transfer Protocol://World Wide Web (dot) arabidopsis (dot) org/)]
   ∘ Rice genome [IRGSP build 4.0 (Hypertext Transfer Protocol://rgp (dot) dna (dot) affrc (dot) go (dot) jp/IRGSP/)].
   ∘ Poplar [Populus trichocarpa release 1.1 from JGI (assembly release v1.0) (Hypertext Transfer Protocol://World Wide Web (dot) genome (dot) jgi-psf (dot) org/)]
   ∘ Brachypodium [JGI 4x assembly, Hypertext Transfer Protocol://World Wide Web (dot) brachpodium (dot) org)]
   ∘ Soybean [DOE-JGI SCP, version Glyma0 (Hypertext Transfer Protocol://World Wide Web (dot) phytozome (dot) net/)]
   ∘ Grape [French-Italian Public Consortium for Grapevine Genome Characterization grapevine genome (Hypertext Transfer Protocol:// World Wide Web (dot) genoscope (dot) cns (dot) fr /)]
   ∘ Castobean [TIGR/J Craig Venter Institute 4x assembly [(Hypertext Transfer Protocol://msc (dot) jcvi (dot) org/r communis]
   ∘ Sorghum [DOE-JGI SCP, version Sbi1 [Hypertext Transfer Protocol://World Wide Web (dot) phytozome (dot) net/)].
   ∘ Partially assembled genome of Maize [Hypertext Transfer Protocol://maizesequence (dot) org/]
- ***Expressed EST and mRNA sequences were extracted from the following databases:***
   ∘ GeneBank versions 154, 157, 160, 161, 164, 165, 166 and 168 (Hypertext Transfer Protocol://World Wide Web (dot) ncbi (dot) nlm (dot) nih (dot) gov/dbEST/)
   ∘ RefSeq (Hypertext Transfer Protocol://World Wide Web (dot) ncbi (dot) nlm (dot) nih (dot) gov/RefSeq/).
   ∘ TAIR (Hypertext Transfer Protocol://World Wide Web (dot) arabidopsis (dot) org/).
- ***Protein and pathway databases***
   ∘ Uniprot [Hypertext Transfer Protocol://World Wide Web (dot) uniprot (dot) org/].
   ∘ AraCyc [Hypertext Transfer Protocol://World Wide Web (dot) arabidopsis (dot) org/biocyc/index (dot) jsp].
   ∘ ENZYME [Hypertext Transfer Protocol://expasy (dot) org/enzyme/].
- ***Microarray datasets were downloaded from:***
   ∘ GEO (Hypertext Transfer Protocol://World Wide Web.ncbi.nlm.nih.gov/geo/)
   ∘ TAIR (Hypertext Transfer Protocol://World Wide Web.arabidopsis.org/).
   ∘ Proprietary microarray data (WO2008/122980).
- ***QTL and SNPs information***
   ∘ Gramene [Hypertext Transfer Protocol://World Wide Web (dot) gramene (dot) org/qtl/].
   ∘ Panzea [Hypertext Transfer Protocol://World Wide Web (dot) panzea (dot) org/index (dot) html].

***Database Assembly*** - was performed to build a wide, rich, reliable annotated and easy to analyze database comprised of publicly available genomic mRNA, ESTs DNA sequences, data from various crops as well as gene expression, protein annotation and pathway data QTLs, and other relevant information.

Database assembly is comprised of a toolbox of gene refining, structuring, annotation and analysis tools enabling to construct a tailored database for each gene discovery project. Gene refining and structuring tools enable to reliably detect splice variants and antisense transcripts, generating understanding of various potential phenotypic outcomes of a single gene. The capabilities of the "LEADS" platform of Compugen LTD for analyzing human genome have been confirmed and accepted by the scientific community [see e.g., "Widespread Antisense Transcription", Yelin, et al. (2003) Nature Biotechnology 21, 379-85; "Splicing of Alu Sequences", Lev-Maor, et al. (2003) Science 300 (5623), 1288-91; "Computational analysis of alternative splicing using EST tissue information", Xie H et al. Genomics 2002], and have been proven most efficient in plant genomics as well.

***EST clustering and gene assembly* -** For gene clustering and assembly of organisms with available genome sequence data (arabidopsis, rice, castorbean, grape, brachypodium, poplar, soybean, sorghum) the genomic LEADS version (GANG) was employed. This tool allows most accurate clustering of ESTs and mRNA sequences on genome, and predicts gene structure as well as alternative splicing events and anti-sense transcription.

For organisms with no available full genome sequence data, "expressed LEADS" clustering software was applied.

***Gene annotation*** - Predicted genes and proteins were annotated as follows:
Blast search [Hypertext Transfer Protocol://blast (dot) ncbi (dot) nlm (dot) nih (dot) gov /Blast (dot) cgi] against all plant UniProt [Hypertext Transfer Protocol://World Wide Web (dot) uniprot (dot) org/] sequences was performed. Open reading frames of each putative transcript were analyzed and longest ORF with higher number of homologues was selected as predicted protein of the transcript. The predicted proteins were analyzed by InterPro [Hypertext Transfer Protocol://World Wide Web (dot) ebi (dot) ac (dot) uk/interpro/].

Blast against proteins from AraCyc and ENZYME databases was used to map the predicted transcripts to AraCyc pathways.

Predicted proteins from different species were compared using blast algorithm [Hypertext Transfer Protocol://World Wide Web (dot) ncbi (dot) nlm (dot) nih (dot) gov /Blast (dot) cgi] to validate the accuracy of the predicted protein sequence, and for efficient detection of orthologs.

***Gene expression profiling*** - Several data sources were exploited for gene expression profiling, namely microarray data and digital expression profile (see below). According to gene expression profile, a correlation analysis was performed to identify genes which are co-regulated under different development stages and environmental conditions and associated with different phenotypes.

Publicly available microarray datasets were downloaded from TAIR and NCBI GEO sites, renormalized, and integrated into the database. Expression profiling is one of the most important resource data for identifying genes important for yield.

A digital expression profile summary was compiled for each cluster according to all keywords included in the sequence records comprising the cluster. Digital expression, also known as electronic Northern Blot, is a tool that displays virtual expression profile based on the EST sequences forming the gene cluster. The tool provides the expression profile of a cluster in terms of plant anatomy (e.g., the tissue/organ in which the gene is expressed), developmental stage (the developmental stages at which a gene can be found) and profile of treatment (provides the physiological conditions under which a gene is expressed such as drought, cold, pathogen infection, etc). Given a random distribution of ESTs in the different clusters, the digital expression provides a probability value that describes the probability of a cluster having a total of N ESTs to contain X ESTs from a certain collection of libraries. For the probability calculations, the following is taken into consideration: a) the number of ESTs in the cluster, b) the number of ESTs of the implicated and related libraries, c) the overall number of ESTs available representing the species. Thereby clusters with low probability values are highly enriched with ESTs from the group of libraries of interest indicating a specialized expression.

Recently, the accuracy of this system was demonstrated by Portnoy et al., 2009 (Analysis Of The Melon Fruit Transcriptome Based On 454 Pyrosequencing) in: Plant & Animal Genomes XVII Conference, San Diego, CA. Transcriptomic analysis, based on relative EST abundance in data was performed by 454 pyrosequencing of cDNA representing mRNA of the melon fruit. Fourteen double strand cDNA samples obtained from two genotypes, two fruit tissues (flesh and rind) and four developmental stages were sequenced. GS FLX pyrosequencing (Roche/454 Life Sciences) of non-normalized and purified cDNA samples yielded 1,150,657 expressed sequence tags, that assembled into 67,477 unigenes (32,357 singletons and 35,120 contigs). Analysis of the data obtained against the Cucurbit Genomics Database [Hypertext Transfer Protocol://World Wide Web (dot) icugi (dot) org/] confirmed the accuracy of the sequencing and assembly. Expression patterns of selected genes fitted well their qRT-PCR data.

To further investigate and identify putative orthologs of the yield, growth rate, vigor, biomass, growth rate, abiotic stress tolerance (ABST), nitrogen use efficiency (NUE) and fertilizer use efficiency (FUE) genes from other plant species, expression data was analyzed and the EST libraries were classified using a fixed vocabulary of custom terms such as developmental stages (e.g., genes showing similar expression profile through development with up regulation at specific stage, such as at the seed filling stage) and/or plant organ (e.g., genes showing similar expression profile across their organs with up regulation at specific organs such as seed). The annotations from all the ESTs clustered to a gene were analyzed statistically by comparing their frequency in the cluster versus their abundance in the database, allowing to construct a numeric and graphic expression profile of that gene, which is termed "digital expression". The rationale of using these two complementary methods with methods of phenotypic association studies of QTLs, SNPs and phenotype expression correlation is based on the assumption that true orthologs are likely to retain identical function over evolutionary time. These methods provide different sets of indications on function similarities between two homologous genes, similarities in the sequence level - identical amino acids in the protein domains and similarity in expression profiles.

Overall, 239 genes were identified to have a major impact on plant yield, growth rate, vigor, biomass, growth rate, oil content, abiotic stress tolerance, nitrogen use efficiency, water use efficiency and fertilizer use efficiency when expression thereof is increased in plants. The identified genes, their curated polynucleotide and polypeptide sequences, as well as their updated sequences according to Genbank database are summarized in Table 1, hereinbelow.

**Table 1**

| ***Identified genes for increasing yield, growth rate, vigor, biomass, growth rate, oil content, abiotic stress tolerance, nitrogen use efficiency, water use efficiency and fertilizer use efficiency of a plant*** | | | | |
|---|---|---|---|---|
| ***Gene Name*** | ***Cluster Name*** | ***Organism*** | ***Polynucleotide SEQ ID NO:*** | ***Polypeptide SEQ ID NO:*** |
| LYM1 | rice\|gb 157.2\|AU058137 | rice | 1 | 240 |
| LYM2 | rice\|pb157.2\|AA750140 | rice | 2 | 241 |
| LYM3 | rice\|gb 157.2\|AU032158 | rice | 3 | 242 |
| LYM4 | rice\|gb157.2\|AU082697 | rice | 4 | 243 |
| LYM5 | rice\|gb157.2\|AW155107 | rice | 5 | 244 |
| LYM6 | rice\|gb157.2\|AW155114 | rice | 6 | 245 |
| LYM7 | rice\|gb157.2\|BE039635 | rice | 7 | 246 |
| LYM8 | rice\|gb157.2\|BE040233 | rice | 8 | 247 |
| LYM9 | rice\|gb157.2\|BE040806 | rice | 9 | 248 |
| LYM10 | rice\|gb157.2\|BE230434 | rice | 10 | 249 |
| LYM12 | rice\|gb157.2\|B1807331 | rice | 11 | 250 |
| LYM13 | rice\|gb157.2\|BM037844 | rice | 12 | 251 |
| LYM14 | rice\|gb157.2\|BM038118 | rice | 13 | 252 |
| LYM15 | rice\|pb 157.2\|CA761603 | rice | 14 | 253 |
| LYM16 | rice\|gb157.2\|U38074 | rice | 15 | 254 |
| LYM17 | rice\|gb157.2\|AU033038 | rice | 16 | 255 |
| LYM19 | rice\|gb157.2\|BE040457 | rice | 17 | 256 |
| LYM20 | rice\|gb157.2\|BF430570 | rice | 18 | 257 |
| LYM21 | rice\|gb157.2\|BI805660 | rice | 19 | 258 |
| LYM22 | rice\|gb157.2\|BI808357 | rice | 20 | 259 |
| LYM23 | rice\|gb157.2\|AA749984 | rice | 21 | 260 |
| LYM24 | rice\|gb157.2\|AF050674 | rice | 22 | 261 |
| LYM26 | barley\|gb157.3\|AJ431915 | barley | 23 | 262 |
| LYM30 | rice\|gb157.2\|AK100743 | rice | 24 | 263 |
| LYM31 | rice\|gb 157.2\|AK101734 | rice | 25 | 264 |
| LYM32 | rice\|gb157.2\|AK106380 | rice | 26 | 265 |
| LYM34 | rice\|gb157.2\|AK107902 | rice | 27 | 266 |
| LYM35 | rice\|gb 157.2\|AK107934 | rice | 28 | 267 |
| LYM36 | rice\|gb 157.2\|AK108674 | rice | 29 | 268 |
| LYM37 | rice\|gb157.2\|AK111353 | rice | 30 | 269 |
| LYM38 | barley\|pb157.3\|AL508889 | barley | 31 | 270 |
| LYM40 | rice\|pb157.2\|AU082329 | rice | 32 | 271 |
| LYM41 | rice\|gb157.2\|AU096202 | rice | 33 | 272 |
| LYM42 | rice\|gb157.2\|AU097348 | rice | 34 | 273 |
| LYM43 | rice\|gb157.2\|AU101198 | rice | 35 | 274 |
| LYM44 | rice\|gb157.2\|AU172519 | rice | 36 | 275 |
| LYM49 | maize\|gb164\|AW331061 | maize | 37 | |
| LYM51 | barley\|gb157.3\|BE412472 | barley | 38 | 276 |
| LYM52 | barley\|gb157.3\|BE422132 | barley | 39 | 277 |
| LYM53 | maize\|gb 164\|BE511332 | maize | 40 | 278 |
| LYM56 | barley\|gb157.3\|BF625411 | barley | 41 | 279 |
| LYM57 | rice\|gb157.2\|BI809626 | rice | 42 | 280 |
| LYM59 | barley\|gb157.3\|BI952737 | barley | 43 | |
| LYM61 | maize\|gb164\|BM079029 | maize | 44 | 281 |
| LYM62 | maize\|gb164\|BM348041 | maize | 45 | 282 |
| LYM66 | barley\|gb157.3\|BU974981 | barley | 46 | 283 |
| LYM67 | rice\|gb157.2\|CA763759 | rice | 47 | 284 |
| LYM68 | rice\|gb 157.2\|CA767240 | rice | 48 | 285 |
| LYM69 | rice\|pb157.2\|CA997856 | rice | 49 | 286 |
| LYM73 | rice\|gb157.2\|CB683204 | rice | 50 | 287 |
| LYM74 | maize\|pb164\|CF075309 | maize | 51 | 288 |
| LYM79 | maize\|gb164\|AW191191 | maize | 52 | 289 |
| LYM82 | barley\|gb157.3\|AL507706 | barley | 53 | 290 |
| LYM83 | barley\|gb157.3\|BI952401 | barley | 54 | 291 |
| LYM84 | barley\|gb157.3\|BF622069 | barley | 55 | 292 |
| LYM86 | rice\|gb157.2\|AU031857 | rice | 56 | 293 |
| LYM88 | arabidopsis\|gb165\|AT2G3775 0 | arabidopsis | 57 | 294 |
| LYM89 | arabidopsis\|gb165\|AT5G6749 0 | arabidopsis | 58 | 295 |
| LYM90 | barley\|gb157.3\|AV927104 | barley | 59 | 296 |
| LYM91 | barley\|gb157.3\|BE060518 | barley | 60 | 297 |
| LYM93 | barley\|gb157.3\|BI955752 | barley | 61 | 298 |
| LYM99 | barley\|gb157.3\|BI947870 | barley | 62 | 299 |
| LYM95 | barley\|gb157.3\|B1959932 | barley | 63 | 300 |
| LYM 100 | barley\|gb157.3\|AV912944 | barley | 64 | 301 |
| LYM102 | rice\|gb157.2\|CA760613 | rice | 65 | 302 |
| LYM103 | maize\|gb164\|CD963970 | maize | 66 | 303 |
| LYM105 | barley\|gb 157.31AL507901 | barley | 67 | 304 |
| LYM106 | barley\|gb157.3\|B1954225 | barley | 68 | 305 |
| LYM110 | maize\|gb164\|BE552618 | maize | 69 | 306 |
| LYM111 | maize\|gb164\|AW053159 | maize | 70 | 307 |
| LYM119 | maize\|gb164\|AW498426 | maize | 71 | 308 |
| LYM 120 | rice\|gb 157.3\|BI795677 | rice | 72 | 309 |
| LYM122 | rice\|gb157.3\|BI118816 | rice | 73 | 310 |
| LYM125 | rice\|gb157.2\|AK108452 | rice | 74 | 311 |
| LYM 126 | rice\|gb157.2\|AK108969 | rice | 75 | 312 |
| LYM 127 | rice\|gb157.2\|AU172589 | rice | 76 | 313 |
| LYM128 | rice\|gb157.2\|AU172667 | rice | 77 | 314 |
| LYM129 | rice\|gb157.3\|BE230206 | rice | 78 | 315 |
| LYM 130 | rice\|gb157.3\|BF430580 | rice | 79 | 316 |
| LYM131 | rice\|gb157.3\|CF309827 | rice | 80 | 317 |
| LYM132 | rice\|gb157.3\|BE229876 | rice | 81 | 318 |
| LYM 134 | rice\|gb157.2\|BI809462 | rice | 82 | 319 |
| LYM136 | rice\|gb157.2\|AU093861 | rice | 83 | 320 |
| LYM 137 | barley\|gb157.3\|AL501911 | barley | 84 | 321 |
| LYM140 | barley\|gb157.3\|BF623993 | barley | 85 | 322 |
| LYM141 | rice\|gb157.2\|CA761074 | rice | 86 | 323 |
| LYM142 | barley\|gb157.3\|CB866504 | barley | 87 | 324 |
| LYM143 | rice\|gb157.3\|BI306405 | rice | 88 | 325 |
| LYM144 | rice\|gb157.2\|BM420331 | rice | 89 | 326 |
| LYM145 | rice\|gb157.2\|AK073109 | rice | 90 | 327 |
| LYM148 | barley\|gb157.3\|AL500574 | barley | 91 | 328 |
| LYM149 | barley\|gb 157.3\|AL509762 | barley | 92 | 329 |
| LYM152 | arabidopsis\|gb165\|AT5G5729 0 | arabidopsis | 93 | 330 |
| LYM153 | rice\|gb157.3\|AU066244 | rice | 94 | 331 |
| LYM 156 | barley\|gb157.3\|BE421631 | barley | 95 | 332 |
| LYM157 | barley\|gb157.3\|BE454937 | barley | 96 | 333 |
| LYM 159 | barley\|gb157.3\|BF259387 | barley | 97 | 334 |
| LYM160 | barley\|gb157.3\|BG300909 | barley | 98 | 335 |
| LYM161 | barley\|gb 157.3\|BG344928 | barley | 99 | 336 |
| LYM 162 | maize\|gb164\|BG462213 | maize | 100 | 337 |
| LYM164 | rice\|gb157.3\|BI805693 | rice | 101 | 338 |
| LYM165 | maize\|gb164\|CD439546 | maize | 102 | 339 |
| LYM166 | wheat\|gb164\|CJ547519 | wheat | 103 | 340 |
| LYM 170 | rice\|gb157.2\|AU057403 | rice | 104 | 341 |
| LYM172 | rice\|gb157.2\|BE229411 | rice | 105 | 342 |
| LYM173 | rice\|gb157.3\|AA751564 | rice | 106 | 343 |
| LYM 174 | sorghum\|gb161.crp\|AW28430 3 | sorghum | 107 | 344 |
| LYM 175 | rice\|gb157.2\|AK060073 | rice | 108 | 345 |
| LYM 176 | rice\|gb157.2\|BI305434 | rice | 109 | 346 |
| LYM178 | barley\|gb157.3\|BE421520 | barley | 110 | 347 |
| LYM 179 | maize\|gb164\|BE051631 | maize | 111 | 348 |
| LYM 107 | maize\|gb164\|AW497895 | maize | 112 | 349 |
| LYM 109 | maize\|gb 169.2\|CD984002 | maize | 113 | 350 |
| LYM112 | maize\|gb164\|CF038223 | maize | 114 | 351 |
| LYM113 | maize\|gb164\|AW257902 | maize | 115 | 352 |
| LYM115 | maize\|gb164\|CF646135 | maize | 116 | 353 |
| LYM116 | maize\|gb164\|AI964572 | maize | 117 | 354 |
| LYM117 | maize\|gb164\|AI739834 | maize | 118 | 355 |
| LYM118 | maize\|gb164\|CO518843 | maize | 119 | 356 |
| LYM121 | rice\|gb157.2\|AK103124 | rice | 120 | 357 |
| LYM123 | rice\|gb157.2\|AI978352 | rice | 121 | 358 |
| LYM 135 | rice\|gb 157.2\|AU101278 | rice | 122 | 359 |
| LYM 138 | rice\|gb157.2\|BI905497 | rice | 123 | 360 |
| LYM 146 | maize\|gb164\|AI770878 | maize | 124 | 361 |
| LYM147 | maize\|gb 164\|AI901828 | maize | 125 | 362 |
| LYM 154 | barley\|gb 157.3\|AV836282 | barley | 126 | 363 |
| LYM155 | barley\|gb 157.3\|BE412535 | barley | 127 | 364 |
| LYM180 | barley\|gb 157.3\|AJ476822 | barley | 128 | 365 |
| LYM181 | barley\|gb157.3\|AL450622 | barley | 129 | 366 |
| LYM182 | barley\|gb 157.3\|AL507048 | barley | 130 | 367 |
| LYM184 | barley\|gb157.3\|AV833284 | barley | 131 | 368 |
| LYM185 | barley\|gb157.3\|AV933969 | barley | 132 | 369 |
| LYM186 | barley\|gb157.3\|AV834971 | barley | 133 | 370 |
| LYM 188 | barley\|gb157.3\|BE438660 | barley | 134 | 371 |
| LYM189 | barley\|gb157.3\|BF256192 | barley | 135 | 372 |
| LYM192 | barley\|gb157.3\|BF627356 | barley | 136 | 373 |
| LYM 193 | barley\|gb 157.3\|CB858276 | barley | 137 | 374 |
| LYM 194 | barley\|gb 157.3\|CB860975 | barley | 138 | 375 |
| LYM196 | maize\|gb 164\|AI372352 | maize | 139 | 376 |
| LYM197 | maize\|gb 164\|AI444704 | maize | 140 | 377 |
| LYM 198 | maize\|gb 164\|AI491323 | maize | 141 | 378 |
| LYM201 | maize\|gb164\|AI600670 | maize | 142 | 379 |
| LYM203 | maize\|gb164\|AI629486 | maize | 143 | 380 |
| LYM204 | maize\|gbl64\|AI649791 | maize | 144 | 381 |
| LYM206 | maize\|gb164\|AI691210 | maize | 145 | 382 |
| LYM207 | maize\|gb164\|AI920398 | maize | 146 | 383 |
| LYM208 | maize\|gb164\|AI941717 | maize | 147 | 384 |
| LYM212 | maize\|gb 164\|AW000408 | maize | 148 | 385 |
| LYM213 | maize\|gb 164\|AW000438 | maize | 149 | 386 |
| LYM215 | maize\|gb 164\|AW498464 | maize | 150 | 387 |
| LYM217 | maize\|gb164\|BE129928 | maize | 151 | 388 |
| LYM219 | maize\|gb164\|BE238495 | maize | 152 | 389 |
| LYM220 | maize\|gb164\|BG842756 | maize | 153 | 390 |
| LYM221 | maize\|gb164\|BI502603 | maize | 154 | 391 |
| LYM223 | maize\|gb 164\|BM338985 | maize | 155 | 392 |
| LYM224 | maize\|gb164\|CA401086 | maize | 156 | 393 |
| LYM227 | maize\|gb 164\|EC877515 | maize | 157 | 394 |
| LYM228 | maize\|gb164\|EC892599 | maize | 158 | 395 |
| LYM232 | rice\|gb 157.3\|AA750121 | rice | 159 | 396 |
| LYM233 | rice\|gb157.3\|AA750182 | rice | 160 | 397 |
| LYM234 | rice\|gb157.3\|AA752388 | rice | 161 | 398 |
| LYM236 | rice\|gb157.3\|AF155334 | rice | 162 | 399 |
| LYM238 | rice\|gb157.3\|AK066551 | rice | 163 | 400 |
| LYM239 | rice\|gb157.3\|AU068651 | rice | 164 | 401 |
| LYM240 | rice\|gb157.3\|AU069131 | rice | 165 | 402 |
| LYM241 | rice\|gb 157.3\|AU162998 | rice | 166 | 403 |
| LYM242 | rice\|gb157.3\|BE039711 | rice | 167 | 404 |
| LYM243 | rice\|gb157.3\|BE228686 | rice | 168 | 405 |
| LYM245 | rice\|gb 157.3\|BF430828 | rice | 169 | 406 |
| LYM248 | rice\|gb 157.3\|BQ906571 | rice | 170 | 407 |
| LYM249 | rice\|gb157.3\|C25903 | rice | 171 | 408 |
| LYM250 | rice\|gb 157.3\|CA759158 | rice | 172 | 409 |
| LYM251 | rice\|gb157.3\|CA759241 | rice | 173 | 410 |
| LYM252 | rice\|gb157.3\|CA759659 | rice | 174 | 411 |
| LYM254 | rice\|gb157.3\|CB657978 | rice | 175 | 412 |
| LYM255 | rice\|gb157.3\|CF330913 | rice | 176 | 413 |
| LYM260 | rice\|gb157.3\|CI581223 | rice | 177 | 414 |
| LYM261 | rice\|gb157.3\|D41406 | rice | 178 | 415 |
| LYM263 | sorghum\|gb161.crp\|AI622410 | sorghum | 179 | 416 |
| LYM 183 | barley\|gb157.3\|AL509795 | barley | 180 | 417 |
| LYM256 | rice\|gb157.3\|CI004090 | rice | 181 | 418 |
| LYM200 | maize\|gb164\|AI586731 | maize | 182 | 419 |
| LYM267 | maize\|gb164\|AW231521 | maize | 183 | 420 |
| LYM268 | rice\|gb157.2\|BI800054 | rice | 184 | 421 |
| LYM270 | maize\|gb164\|AI670268 | maize | 185 | 422 |
| LYM271 | maize\|gb164\|CF637107 | maize | 186 | 423 |
| LYM272 | rice\|gb157.2\|CA761620 | rice | 187 | 424 |
| LYM273 | rice\|gb157.2\|BM418692 | rice | 188 | 425 |
| LYM274 | rice\|gb157.2\|AK073201 | rice | 189 | 426 |
| LYM277 | rice\|pb 157.2\|BM038097 | rice | 190 | 427 |
| LYM278 | barley\|gb157.3\|BLYTRAA | barley | 191 | 428 |
| LYM283 | rice\|gb157.2\|D23167 | rice | 192 | 429 |
| LYM284 | rice\|gb157.2\|BI306331 | rice | 193 | 430 |
| LYM285 | rice\|gb157.2\|CB631346 | rice | 194 | 431 |
| LYM287 | rice\|gb157.2\|AK102063 | rice | 195 | 432 |
| LYM288 | rice\|gb157.2\|BE040927 | rice | 196 | 433 |
| LYM289 | barley\|gb157.3\|AV925962 | barley | 197 | 434 |
| LYM290 | maize\|gb164\|AA979729 | maize | 198 | 435 |
| LYM291 | rice\|gb157.2\|BM037976 | rice | 199 | 436 |
| LYM293 | rice\|gb157.2\|AK059161 | rice | 200 | 437 |
| LYM38 | barley\|gb157.3\|AL508889 | barley | 201 | 438 |
| LYM42 | rice\|gb157.2\|AU097348 | rice | 202 | 439 |
| LYM51 | barley\|gb 157.3\|BE412472 | barley | 203 | 276 |
| LYM52 | barley\|gb157.3\|BE422132 | barley | 204 | 277 |
| LYM56 | barley\|gb157.3\|BF625411 | barley | 205 | 279 |
| LYM59 | barley\|gb157.3\|BI952737 | barley | 206 | |
| LYM66 | barley\|gb157.3\|BU974981 | barley | 207 | 440 |
| LYM79 | maize\|gb164\|AW191191 | maize | 208 | 441 |
| LYM83 | barley\|gb157.3\|BI952401 | barley | 209 | 442 |
| LYM90 | barley\|gb157.3\|AV927104 | barley | 210 | 296 |
| LYM99 | barley\|gb157.3\|BI947870 | barley | 211 | 299 |
| LYM95 | barley\|gb157.3\|BI959932 | barley | 212 | 443 |
| LYM148 | barley\|gb 157.3\|AL500574 | barley | 213 | 328 |
| LYM159 | barley\|gb157.3\|BF259387 | barley | 214 | 334 |
| LYM161 | barley\|gb157.3\|BG344928 | barley | 215 | 444 |
| LYM166 | wheat\|gb164\|CJ547519 | wheat | 216 | 445 |
| LYM175 | rice\|gb157.2\|AK060073 | rice | 217 | 446 |
| LYM109 | maize\|gb164\|CD984002 | maize | 218 | 447 |
| LYM112 | maize\|gb 164\|CF038223 | maize | 219 | 448 |
| LYM116 | maize\|gb164\|AI964572 | maize | 220 | 354 |
| LYM117 | maize\|gb164\|AI739834 | maize | 221 | 449 |
| LYM154 | barley\|gb157.3\|AV836282 | barley | 222 | 450 |
| LYM 155 | barley\|gb157.3\|BE412535 | barley | 223 | 451 |
| LYM180 | barley\|gb 157.3\|AJ476822 | barley | 224 | 452 |
| LYM181 | barley\|gb 157.3\|AL450622 | barley | 225 | 453 |
| LYM 184 | barley\|gb157.3\|AV833284 | barley | 226 | 454 |
| LYM185 | barley\|gb157.3\|AV833969 | barley | 227 | 455 |
| LYM186 | barley\|gb157.3\|AV834971 | barley | 228 | 370 |
| LYM 188 | barley\|gb157.3\|BE438660 | barley | 229 | 456 |
| LYM 189 | barley\|gb157.3\|BF256192 | barley | 230 | 457 |
| LYM192 | barley\|gb 157.3\|BF627356 | barley | 231 | 458 |
| LYM 193 | barley\|gb157.3\|CB858276 | barley | 232 | 459 |
| LYM194 | barley\|gb 157.3\|CB860975 | barley | 233 | 460 |
| LYM219 | maize\|gb 164\|BE238495 | maize | 234 | 389 |
| LYM221 | maize\|gb164\|BI502603 | maize | 235 | 461 |
| LYM228 | maize\|gb 164\|EC892599 | maize | 236 | 462 |
| LYM250 | rice\|gb157.3\|CA759158 | rice | 237 | 463 |
| LYM183 | barley\|gb157.3\|AL509795 | barley | 238 | 464 |
| LYM272 | rice\|gb 157.2\|CA761620 | rice | 239 | 465 |

Table 1: Provided are the identified genes, their annotation, organism and polynucleotide and polypeptide sequence identifiers.

### EXAMPLE 2

### IDENTIFICATION OF HOMOLOGOUS SEQUENCES THAT INCREASE YIELD, FIBER YIELD, FIBER QUALITY, GROWTH RATE, BIOMASS, OIL CONTENT, VIGOR, ABST, AND/OR NUE OF A PLANT

The concepts of orthology and paralogy have recently been applied to functional characterizations and classifications on the scale of whole-genome comparisons. Orthologs and paralogs constitute two major types of homologs: The first evolved from a common ancestor by specialization, and the latter are related by duplication events. It is assumed that paralogs arising from ancient duplication events are likely to have diverged in function while true orthologs are more likely to retain identical function over evolutionary time.

To identify putative orthologs of the genes affecting plant yield, oil yield, oil content, seed yield, growth rate, vigor, biomass, abiotic stress tolerance and/or nitrogen use efficiency, all sequences were aligned using the BLAST (*Basic Local Alignment Search Tool*). Sequences sufficiently similar were tentatively grouped. These putative orthologs were further organized under a Phylogram - a branching diagram (tree) assumed to be a representation of the evolutionary relationships among the biological taxa. Putative ortholog groups were analyzed as to their agreement with the phylogram and in cases of disagreements these ortholog groups were broken accordingly.

Expression data was analyzed and the EST libraries were classified using a fixed vocabulary of custom terms such as developmental stages (e.g., genes showing similar expression profile through development with up regulation at specific stage, such as at the seed filling stage) and/or plant organ (e.g., genes showing similar expression profile across their organs with up regulation at specific organs such as seed). The annotations from all the ESTs clustered to a gene were analyzed statistically by comparing their frequency in the cluster versus their abundance in the database, allowing the construction of a numeric and graphic expression profile of that gene, which is termed "digital expression". The rationale of using these two complementary methods with methods of phenotypic association studies of QTLs, SNPs and phenotype expression correlation is based on the assumption that true orthologs are likely to retain identical function over evolutionary time. These methods provide different sets of indications on function similarities between two homologous genes, similarities in the sequence level - identical amino acids in the protein domains and similarity in expression profiles.

The search and identification of homologous genes involves the screening of sequence information available, for example, in public databases such as the DNA Database of Japan (DDBJ), Genbank, and the European Molecular Biology Laboratory Nucleic Acid Sequence Database (EMBL) or versions thereof or the MIPS database. A number of different search algorithms have been developed, including but not limited to the suite of programs referred to as BLAST programs. There are five implementations of BLAST, three designed for nucleotide sequence queries (BLASTN, BLASTX, and TBLASTX) and two designed for protein sequence queries (BLASTP and TBLASTN) (Coulson, Trends in Biotechnology: 76-80, 1994; Birren et al., Genome Analysis, I: 543, 1997). Such methods involve alignment and comparison of sequences. The BLAST algorithm calculates percent sequence identity and performs a statistical analysis of the similarity between the two sequences. The software for performing BLAST analysis is publicly available through the National Centre for Biotechnology Information. Other such software or algorithms are GAP, BESTFIT, FASTA and TFASTA. GAP uses the algorithm of Needleman and Wunsch (J. Mol. Biol. 48: 443-453, 1970) to find the alignment of two complete sequences that maximizes the number of matches and minimizes the number of gaps.

The homologous genes may belong to the same gene family. The analysis of a gene family may be carried out using sequence similarity analysis. To perform this analysis one may use standard programs for multiple alignments e.g. Clustal W. A neighbour-joining tree of the proteins homologous to the genes in this invention may be used to provide an overview of structural and ancestral relationships. Sequence identity may be calculated using an alignment program as described above. It is expected that other plants will carry a similar functional gene (ortholog) or a family of similar genes and those genes will provide the same preferred phenotype as the genes presented here. Advantageously, these family members may be useful in the methods of the invention. Example of other plants are included here but not limited to, barley (Hordeum vulgare), Arabidopsis (Arabidopsis thaliana), maize (Zea mays), cotton (Gossypium), Oilseed rape (Brassica napus), Rice (Oryza sativa), Sugar cane (Saccharum officinarum), Sorghum (Sorghum bicolor), Soybean (Glycine max), Sunflower (Helianthus annuus), Tomato (Lycopersicon esculentum), Wheat (Triticum aestivum).

The above-mentioned analyses for sequence homology can be carried out on a full-length sequence, but may also be based on a comparison of certain regions such as conserved domains. The identification of such domains, would also be well within the realm of the person skilled in the art and would involve, for example, a computer readable format of the nucleic acids of the present invention, the use of alignment software programs and the use of publicly available information on protein domains, conserved motifs and boxes. This information is available in the PRODOM (Hypertext Transfer Protocol://World Wide Web (dot) biochem (dot) ucl (dot) ac (dot) uk/bsm/dbbrowser/protocol/prodomqry (dot) html), PIR (Hypertext Transfer Protocol://pir (dot) Georgetown (dot) edu/) or Pfam (Hypertext Transfer Protocol://World Wide Web (dot) sanger (dot) ac (dot) uk/Software/Pfam/) database. Sequence analysis programs designed for motif searching may be used for identification of fragments, regions and conserved domains as mentioned above. Preferred computer programs include, but are not limited to, MEME, SIGNALSCAN, and GENESCAN.

A person skilled in the art may use the homologous sequences provided herein to find similar sequences in other species and other organisms. Homologues of a protein encompass, peptides, oligopeptides, polypeptides, proteins and enzymes having amino acid substitutions, deletions and/or insertions relative to the unmodified protein in question and having similar biological and functional activity as the unmodified protein from which they are derived. To produce such homologues, amino acids of the protein may be replaced by other amino acids having similar properties (conservative changes, such as similar hydrophobicity, hydrophilicity, antigenicity, propensity to form or break a-helical structures or 3-sheet structures). Conservative substitution tables are well known in the art (see for example Creighton (1984) Proteins. W.H. Freeman and Company). Homologues of a nucleic acid encompass nucleic acids having nucleotide substitutions, deletions and/or insertions relative to the unmodified nucleic acid in question and having similar biological and functional activity as the unmodified nucleic acid from which they are derived.

Table 2, hereinbelow, lists a summary of orthologous and homologous sequences of the polynucleotide sequences (SEQ ID NOs:1-239) and polypeptide sequences (SEQ ID NOs:240-465) presented in Table 1 above, which were identified from the databases using the NCBI BLAST software (e.g., using the Blastp and tBlastn algorithms) and needle (EMBOSS package) as being at least 80% homologous to the selected polynucleotides and polypeptides, and which are expected to increase plant yield, seed yield, oil yield, oil content, growth rate, fiber yield, fiber quality, biomass, vigor, ABST and/or NUE of a plant.

**Table 2**

| ***Homologues of the identified genes*/*polypeptides for increasing yield, fiber yield, fiber quality, growth rate, vigor, biomass, growth rate, abiotic stress tolerance, nitrogen use efficiency, water use efficiency and fertilizer use efficiency of a plant*** | | | | | | |
|---|---|---|---|---|---|---|
| ***Nucl. SEQ ID NO:*** | ***Gene Name*** | ***cluster name*** | ***Polyp. SEQ ID NO:*** | ***Homolog. to SEQ ID NO:*** | ***% global identity*** | ***Algor.*** |
| 467 | LYM2 H5 | brachypodium\|09v1\|D V480246 | 1974 | 241 | 89.1 | blastp |
| 468 | LYM2 H6 | maize\|gb170\|AW224918 | 1975 | 241 | 86.6 | blastp |
| 469 | LYM2 H7 | millet\|09v1\|EVO454P M002089 | 1976 | 241 | 87.6 | blastp |
| 470 | LYM2 H8 | sorghum\|09v1\|SB07G 004285 | 1977 | 241 | 86.6 | blastp |
| 471 | LYM2 H4 | switchgrass\|gb167\|FE6 06998 | 1978 | 241 | 89.9 | blastp |
| 472 | LYM2 H5 | wheat\|gb164\|BM1368 11 | 1979 | 241 | 80.53 | tblastn |
| 473 | LYM4 H6 | barlcy\|gb157SOLEXA \|BE438934 | 1980 | 243 | 81.5 | blastp |
| 474 | LYM4 H7 | brachypodium\|09v 1\|D V469575 | 1981 | 243 | 81.5 | blastp |
| 475 | LYM4 H2 | cenchrus\|gb166\|BM08 4020 | 1982 | 243 | 83 | blastp |
| 476 | LYM4 H8 | maize\|gb170\|AI600994 | 1983 | 243 | 82.2 | blastp |
| 477 | LYM4 H9 | maize\|gb170\|AW054478 | 1984 | 243 | 82.4 | blastp |
| 478 | LYM4 H10 | rice\|gb170\|OS05G13950 | 1985 | 243 | 94.7 | blastp |
| 479 | LYM4 H11 | sorghum\|09v1\|SB03G 000920 | 1986 | 243 | 83.2 | blastp |
| 480 | LYM4 H5 | switchgrass\|gb167\|FL7 03533 | 1987 | 243 | 83 | blastp |
| 481 | LYM4 H6 | wheat\|gb164\|BE44499 1 | 1988 | 243 | 81.3 | blastp |
| 482 | LYM5 H16 | barley\|gb157SOLEXA \|BI953887 | 1989 | 244 | 90.9 | blastp |
| 483 | LYM5 H17 | brachypodium\|09v1\|D V474010 | 1990 | 244 | 91.3 | blastp |
| 484 | LYM5 H3 | cenchrus\|gb166\|EB655978 | 1991 | 244 | 88.19 | tblastn |
| 485 | LYM5 H4 | fescue\|gb161\|DT688132 | 1992 | 244 | 85.8 | blastp |
| 486 | LYM5 H5 | leymus\|gb166\|EG3949 68 | 1993 | 244 | 90.9 | blastp |
| 487 | LYM5 H18 | maize\|gb170\|AI793290 | 1994 | 244 | 92.1 | blastp |
| 488 | LYM5 H19 | maize\|gb170\|BG265158 | 1995 | 244 | 92.5 | blastp |
| 489 | LYM5 H20 | rice\|gb170\|OS02G466 60 | 1996 | 244 | 87.8 | blastp |
| 490 | LYM5 H21 | sorghum\|09v1\|SB04G 031180 | 1997 | 244 | 80.3 | blastp |
| 491 | LYM5 H22 | sorghum\|09v1\|SB06G 027060 | 1998 | 244 | 90.9 | blastp |
| 492 | LYM5 H23 | sugarcane\|gb157.3\|CA 118359 | 1999 | 244 | 91.7 | blastp |
| 493 | LYM5 H12 | switchgrass\|gb167\|FE6 41223 | 2000 | 244 | 93.3 | blastp |
| 494 | LYM5 H13 | switchgrass\|gb167\|FL7 08642 | 2001 | 244 | 92.5 | blastp |
| 495 | LYM5 H14 | wheat\|gb164\|BE414733 | 2002 | 244 | 90.6 | blastp |
| 496 | LYM5 H15 | wheat\|gb164\|BE431026 | 2003 | 244 | 91.3 | blastp |
| 497 | LYM5 H16 | wheat\|gb164\|CA613380 | 2004 | 244 | 90.9 | blastp |
| 498 | LYM7 H35 | b oleracea\|gb161\|AM05 7184 | 2005 | 246 | 81.2 | blastp |
| 499 | LYM7 H36 | barley\|gb 157SOLEXA \|BE413128 | 2006 | 246 | 82.6 | blastp |
| 500 | LYM7 H37 | barley\|gb157SOLEXA \|BF627706 | 2007 | 246 | 95.7 | blastp |
| 501 | LYM7 H38 | brachypodium\|09v1\|D V468966 | 2008 | 246 | 94.2 | blastp |
| 502 | LYM7 H39 | brachypodium\|09v1\|D V474806 | 2009 | 246 | 82.6 | blastp |
| 503 | LYM7 H6 | bruguiera\|gb166\|BP94 5554 | 2010 | 246 | 81.2 | blastp |
| 504 | LYM7 H40 | canola\|gb161\|CD811653 | 2011 | 246 | 81.2 | blastp |
| 505 | LYM7 H41 | canola\|gb161\|CD838423 | 2012 | 246 | 81.2 | blastp |
| 506 | LYM7 H42 | cassava\|09v1\|CK652348 | 2013 | 246 | 82.6 | blastp |
| 507 | LYM7 H43 | castorbean\|09v1\|XM0 02532394 | 2014 | 246 | 82.6 | blastp |
| 508 | LYM7 H44 | cucumber\|09v1\|AM71 7859 | 2015 | 246 | 82.6 | blastp |
| 509 | LYM7 H9 | eucalyptus\|gb166\|CB9 67858 | 2016 | 246 | 81.2 | blastp |
| 510 | LYM7 H10 | kiwi\|gb166\|FG431017 | 2017 | 246 | 81.2 | blastp |
| 511 | LYM7 H11 | kiwi\|gb166\|FG521634 | 2018 | 246 | 82.6 | blastp |
| 512 | LYM7 H12 | liriodendron\|gb166\|FD 494835 | 2019 | 246 | 82.6 | blastp |
| 513 | LYM7 H45 | maize\|gb170\|AI943908 | 2020 | 246 | 82.6 | blastp |
| 514 | LYM7 H46 | maize\|gb170\|AW282244 | 2021 | 246 | 88.4 | blastp |
| 515 | LYM7 H47 | maize\|gb170\|LLA1855 232 | 2022 | 246 | 82.61 | tblastn |
| 516 | LYM7 H48 | maize\|gb 170\|LLDN20 9190 | 2023 | 246 | 82.61 | tblastn |
| 517 | LYM7 H49 | millet\|09v1\|EV0454P M003641 | 2024 | 246 | 91.3 | blastp |
| 518 | LYM7 H50 | millet\|09v1\|EV0454P M019125 | 2025 | 246 | 81.2 | blastp |
| 519 | LYM7 H16 | oat\|gb164\|CN817490 | 2026 | 246 | 88.4 | blastp |
| 520 | LYM7 H17 | oat\|gb164\|CN819643 | 2027 | 246 | 82.6 | blastp |
| 521 | LYM7 H51 | poplar\|gb 170\|BI06944 6 | 2028 | 246 | 81.2 | blastp |
| 522 | LYD97 H18 | poplar\|gb170\|BI12366 2 | 2029 | 246 | 81.2 | blastp |
| 523 | LYM7 H20 | rye\|gb164\|BE496021 | 2030 | 246 | 94.2 | blastp |
| 524 | LYM7 H21 | rye\|gb164\|BE587226 | 2031 | 246 | 81.2 | blastp |
| 525 | LYM7 H52 | sorghum\|09v1\|SB05G 003875 | 2032 | 246 | 88.4 | blastp |
| 526 | LYM7 H53 | sugarcane\|gb157.3\|CA 079082 | 2033 | 246 | 89.9 | blastp |
| 527 | LYM7 H54 | sugarcane\|gb157.3\|CA 158782 | 2034 | 246 | 81.2 | blastp |
| 528 | LYM7 H25 | switchgrass\|gb167\|DN 149707 | 2035 | 246 | 82.6 | blastp |
| 529 | LYM7 H26 | switchgrass\|gb167\|FE6 44021 | 2036 | 246 | 84.1 | blastp |
| 530 | LYM7 H27 | switchgrass\|gb167\|FE6 57215 | 2037 | 246 | 80 | blastp |
| 531 | LYM7 H28 | switchgrass\|167\|FE6 58413 | 2038 | 246 | 88.4 | blastp |
| 532 | LYM7 H29 | switchgrass\|gb167\|FL6 89692 | 2039 | 246 | 88.4 | blastp |
| 533 | LYM7 H30 | wheat\|gb164\|BE404350 | 2040 | 246 | 81.2 | blastp |
| 534 | LYM7 H31 | wheat\|gb164\|BE414371 | 2041 | 246 | 84.1 | blastp |
| 535 | LYM7 H32 | wheat\|gb164\|BE430017 | 2042 | 246 | 94.2 | blastp |
| 536 | LYM7 H33 | wheat\|gb164\|BE444058 | 2043 | 246 | 95.7 | blastp |
| 537 | LYM7 H34 | wheat\|gb164\|BE44478 9 | 2044 | 246 | 82.6 | blastp |
| 538 | LYM7 H35 | wheat\|gb164\|CA598363 | 2045 | 246 | 95.7 | blastp |
| 539 | LYM8 H7 | arabidopsis lyrata\|09v 1\|JGIAL008 627 | 2046 | 247 | 80.2 | blastp |
| 540 | LYM8 H8 | arabidopsis tyrata\|09v1\|JGIAL021 400 | 2047 | 247 | 83.3 | blastp |
| 541 | LYM8 H1 | arabidopsis\|gb165\|AT 3G03110 | 2048 | 247 | 80.6 | blastp |
| 542 | LYM8 H2 | arabidopsis\|gb165\|AT 5G17020 | 2049 | 247 | 82.9 | blastp |
| 543 | LYM8 H9 | brachypodium\|09v1\|G T774368 | 2050 | 247 | 95.6 | blastp |
| 544 | LYM8 H10 | castorbean\|09v1\|EE25 5045 | 2051 | 247 | 84.2 | blastp |
| 545 | LYM8 H11 | chestnut\|gb170\|SRR00 629550059698 | 2052 | 247 | 85.7 | blastp |
| 546 | LYM8 H12 | cucumber\|09v1\|GD17 4631 | 2053 | 247 | 84.5 | blastp |
| 547 | LYM8 H13 | lotus\|09v1\|BP043858 | 2054 | 247 | 83.8 | blastp |
| 548 | LYM8 H14 | lotus\|09v1\|BP071708 | 2055 | 247 | 83.6 | blastp |
| 549 | LYM8 H15 | maize\|gb170\|AA030709 | 2056 | 247 | 92.1 | blastp |
| 550 | LYM8 H16 | maize\|gb170\|A1621522 | 2057 | 247 | 92.2 | blastp |
| 551 | LYM8 H17 | medicago\|09v1\|BE205102 | 2058 | 247 | 83.5 | blastp |
| 552 | LYM8 H18 | medicago\|09v1\|BM77 9128 | 2059 | 247 | 83.9 | blastp |
| 553 | LYM8 H19 | poplar\|gb170\|BI127444 | 2060 | 247 | 84.8 | blastp |
| 554 | LYM8 H20 | poplar\|gb170\|BU837911 | 2061 | 247 | 85 | blastp |
| 555 | LYM8 H21 | rice\|gb170\|OS03G64080 | 2062 | 247 | 99.72 | tblastn |
| 556 | LYM8 H22 | solanum phureja\|09v1\|SPHBG1 28228 | 2063 | 247 | 83.2 | blastp |
| 557 | LYM8 H23 | sorghum\|09v1\|SB01G 000490 | 2064 | 247 | 93.9 | blastp |
| 558 | LYM8 H24 | sorghum\|09v1\|SB02G 009800 | 2065 | 247 | 89 | blastp |
| 559 | LYM8 H6 | soybean\|gb168\|BE205102 | 2066 | 247 | 82.98 | tblastn |
| 560 | LYM8 H7 | soybean\|gb168\|BE823 809 | 2067 | 247 | 81.6 | blastp |
| 561 | LYM8 H25 | tomato\|09v1\|BG128228 | 2068 | 247 | 83.33 | tblastn |
| 562 | LYM9 H0 | lolium\|09v1\|AU245599 | 2069 | 248 | 80.1 | blastp |
| 563 | LYM10 H1 | antirrhinum\|gb166\|AJ7 86992 | 2070 | 249 | 89.9 | blastp |
| 564 | LYM10 H207 | apple\|gb171\|CN44392 9 | 2071 | 249 | 91.3 | blastp |
| 565 | LYM10 H208 | apple\|gb171\|CN48976 3 | 2072 | 249 | 91.3 | blastp |
| 566 | LYM10 H209 | apple\|gb171\|CN87419 2 | 2073 | 249 | 87 | blastp |
| 567 | LYM10 H210 | arabidopsis lyrata\|09v1\|JGIAL017 989 | 2074 | 249 | 85.5 | blastp |
| 568 | LYM10 H211 | arabidopsis lyrata\|09v1\|JGIAL025 614 | 2075 | 249 | 91.3 | blastp |
| 569 | LYM10 H212 | arabidopsis lyrata\|09v1\|JGIAL029 470 | 2076 | 249 | 91.3 | blastp |
| 570 | LYM10 H7 | arabidopsis\|gb165\|AT 3G48570 | 2077 | 249 | 85.5 | blastp |
| 571 | LYM10 H8 | arabidopsis\|gb165\|AT 4G24920 | 2078 | 249 | 91.3 | blastp |
| 572 | LYM10 H9 | arabidopsis\|gb165\|AT 5G50460 | 2079 | 249 | 91.3 | blastp |
| 573 | LYM10 H10 | artemisia\|gb164\|EX98 0216 | 2080 | 249 | 88.4 | blastp |
| 574 | LYM10 H11 | b juncea\|gb164\|EVGN0 0323614690486 | 2081 | 249 | 85.5 | blastp |
| 575 | LYM10 H12 | b juncea\|gb164\|EVGN0 0357611620134 | 2082 | 249 | 81.16 | tblastn |
| 576 | LYMIO H13 | b juncea\|gb164\|EVGN0 0407015981886 | 2083 | 249 | 91.3 | blastp |
| 577 | LYM10 H14 | b juncea\|gb 164\|EVGN0 1046711722157 | 2084 | 249 | 91.3 | blastp |
| 578 | LYM10 H15 | b juncea\|gb164\|EVGN0 1350404310247 | 2085 | 249 | 91.3 | tblastn |
| 579 | LYM10 H16 | b juncea\|gb 164\|EVGN0 1826229072660 | 2086 | 249 | 91.3 | blastp |
| 580 | LYM10 H17 | b juncea\|gb164\|EVGN1 0412810992898 | 2087 | 249 | 86.3 | blastp |
| 581 | LYM10 H18 | b juncea\|gb164\|EVGN1 9578802581818 | 2088 | 249 | 81.2 | blastp |
| 582 | LYM10 H19 | b oleracea\|gb161\|AM05 9639 | 2089 | 249 | 91.3 | blastp |
| 583 | LYM10 H20 | b oleracea\|gb161\|EH414 574 | 2090 | 249 | 91.3 | blastp |
| 584 | LYM10 H21 | b oleracea\|gb161\|EH427 198 | 2091 | 249 | 81.7 | blastp |
| 585 | LYM10H22 | b rapa\|gb 162\|BG544908 | 2092 | 249 | 91.3 | blastp |
| 586 | LYM10H23 | b rapa\|gb 162\|DY010003 | 2093 | 249 | 91.3 | blastp |
| 587 | LYM10 H24 | b rapa\|gb 162\|EE524434 | 2094 | 249 | 91.3 | tblastn |
| 588 | LYM10 H25 | b rapa\|gb162\|L35825 | 2095 | 249 | 91.3 | blastp |
| 589 | LYM10 H26 | banana\|gb167\|DN2397 48 | 2096 | 249 | 94.2 | blastp |
| 590 | LYM10 H27 | banana\|gb167\|ES4325 17 | 2097 | 249 | 95.7 | blastp |
| 591 | LYM10 H28 | banana\|gb167\|FL6497 89 | 2098 | 249 | 95.7 | blastp |
| 592 | LYM10 H29 | banana\|gb167\|FL6581 61 | 2099 | 249 | 94.2 | blastp |
| 593 | LYM10 H213 | barley\|gb 157SOLEXA \|AJ433765 | 2100 | 249 | 100 | blastp |
| 594 | LYM10 H214 | barley\|gb157SOLEXA \|BE412470 | 2101 | 249 | 100 | blastp |
| 595 | LYM10 H215 | barley\|gb157SOLEXA \|BF254576 | 2102 | 249 | 98.6 | blastp |
| 596 | LYM10 H216 | barley\|gb 157SOLEXA \|BF257015 | 2103 | 249 | 100 | blastp |
| 597 | LYM10 H34 | bean\|gb167\|CA907476 | 2104 | 249 | 92.75 | tblastn |
| 598 | LYM10 H35 | bean\|gb167\|CA907483 | 2105 | 249 | 94.2 | blastp |
| 599 | LYM10 H217 | beech\|gb170\|SRR0062 93S0011456 | 2106 | 249 | 92.8 | blastp |
| 600 | LYM10 H218 | beech\|gb170\|SRR0062 94S0008365 | 2107 | 249 | 88.4 | blastp |
| 601 | LYM10 H219 | brachypodium\|09v 1\|D V469126 | 2108 | 249 | 100 | blastp |
| 602 | LYM10 H220 | brachypodium\|09v1\|G T803631 | 2109 | 249 | 92.8 | blastp |
| 603 | LYM10 H38 | bruguiera\|gb166\|BP94 1922 | 2110 | 249 | 95.7 | blastp |
| 604 | LYM10 H39 | bruguiera\|gb166\|BP94 4773 | 2111 | 249 | 95.7 | blastp |
| 605 | LYM10 H40 | cacao\|gb167\|CU47152 9 | 2112 | 249 | 94.2 | blastp |
| 606 | LYM10 H41 | cacao\|gb167\|CU48059 7 | 2113 | 249 | 84.1 | blastp |
| 607 | LYM10 H42 | cacao\|gb167\|CU49329 8 | 2114 | 249 | 89.9 | blastp |
| 608 | LYM10 H43 | canola\|gb161\|CD8132 31 | 2115 | 249 | 91.3 | tblastn |
| 609 | LYMIO H44 | canola\|gb161\|CD8175 28 | 2116 | 249 | 91.3 | tblastn |
| 610 | LYM10 H45 | canola\|gb161\|CD8200 75 | 2117 | 249 | 91.3 | tblastn |
| 611 | LYM10 H46 | canola\|gb161\|CD8242 39 | 2118 | 249 | 91.3 | tblastn |
| 612 | LYM10 H47 | canola\|gb 161\| CD8380 62 | 2119 | 249 | 86.3 | blastp |
| 613 | LYM10 H48 | canola\|gb161\|CD8408 08 | 2120 | 249 | 91.3 | blastp |
| 614 | LYM10 H49 | canola\|gb161\|CN7324 34 | 2121 | 249 | 91.3 | tblastn |
| 615 | LYMIO H50 | canola\|gb161\|DW9992 88 | 2122 | 249 | 91.3 | blastp |
| 616 | LYM10 H51 | canola\|gb161\|EE43417 6 | 2123 | 249 | 84.1 | blastp |
| 617 | LYM10 H52 | canola\|gb161 \|EE46403 6 | 2124 | 249 | 87 | blastp |
| 618 | LYM10 H221 | cassava\|09v1\|CK6422 25 | 2125 | 249 | 94.2 | blastp |
| 619 | LYM10 H222 | cassava\|09v1\|DV4557 17 | 2126 | 249 | 94.2 | blastp |
| 620 | LYM10 H223 | cassava\|09v 1 \|FF38038 9 | 2127 | 249 | 94.2 | blastp |
| 621 | LYM10 H224 | castorbean\|09v1\|EG66 4279 | 2128 | 249 | 92.8 | blastp |
| 622 | LYM10 H225 | castorbean\|09v1\|XM0 02509459 | 2129 | 249 | 91.3 | blastp |
| 623 | LYM10 H58 | catharanthus\|gb166\|E G560643 | 2130 | 249 | 91.3 | blastp |
| 624 | LYM10 H59 | catharanthus\|gb166\|FD 416462 | 2131 | 249 | 91.3 | blastp |
| 625 | LYM10 H60 | catharanthus\|gb166\|FD 420164 | 2132 | 249 | 92.8 | blastp |
| 626 | LYM10 H61 | centaurea\|gb166\|EH74 7070 | 2133 | 249 | 87 | blastp |
| 627 | LYM10 H62 | centaurea\|gb166\|EH78 8831 | 2134 | 249 | 89.9 | blastp |
| 628 | LYM10 H226 | chestnut\|gb170\|SRR00 6295S0002470 | 2135 | 249 | 95.7 | blastp |
| 629 | LYM10 H227 | chestnut\|gb170\|SRR00 6295S0013318 | 2136 | 249 | 92.8 | blastp |
| 630 | LYM10 H228 | cichorium\|gb171\|FL67 3304 | 2137 | 249 | 85.51 | tblastn |
| 631 | LYM10 H63 | citrus\|gb166\|CF41752 0 | 2138 | 249 | 91.3 | blastp |
| 632 | LYM10 H64 | coffea\|gb157.2\|DV666 460 | 2139 | 249 | 91.3 | blastp |
| 633 | LYM10 H65 | coffea\|gb157.2\|DV676 797 | 2140 | 249 | 89.86 | tblastn |
| 634 | LYMIO H66 | cotton\|gb164\|BE05219 8 | 2141 | 249 | 94.2 | blastp |
| 635 | LYM10 H67 | cotton\|gb164\|BQ4048 33 | 2142 | 249 | 95.7 | blastp |
| 636 | LYM10 H68 | cotton\|gb164\|BQ4074 07 | 2143 | 249 | 92.8 | blastp |
| 637 | LYM10 H69 | cotton\|gb164\|CK6405 93 | 2144 | 249 | 95.7 | blastp |
| 638 | LYM10 H70 | cotton\|gb164\|DT05275 9 | 2145 | 249 | 88 | blastp |
| 639 | LYM10 H71 | cotton\|gb164\|DT57406 1 | 2146 | 249 | 80.7 | blastp |
| 640 | LYM10 H72 | cowpea\|gb166\|FF3865 43 | 2147 | 249 | 94.2 | blastp |
| 641 | LYM10 H73 | cowpea\|gb166\|FF3893 57 | 2148 | 249 | 94.2 | tblastn |
| 642 | LYM10 H74 | cryptomeria\|gb166\|BP 174192 | 2149 | 249 | 89.9 | blastp |
| 643 | LYM10 H75 | cryptomeria\|gb166\|BP 174931 | 2150 | 249 | 88.6 | blastp |
| 644 | LYM10 H229 | cucumber\|09v1\|AM71 5093 | 2151 | 249 | 88.41 | tblastn |
| 645 | LYM10 H230 | cucumber\|09v1\|AM72 0495 | 2152 | 249 | 98.6 | blastp |
| 646 | LYM10 H231 | cucumber\|09v1\|DN90 9507 | 2153 | 249 | 95.7 | blastp |
| 647 | LYM10 H76 | cycas\|gb166\|CB09149 9 | 2154 | 249 | 88.4 | blastp |
| 648 | LYM10 H77 | cynara\|gb167\|GE5892 84 | 2155 | 249 | 88.4 | blastp |
| 649 | LYM10 H78 | dandelion\|gb161\|DY8 11008 | 2156 | 249 | 88.41 | tblastn |
| 650 | LYM10 H79 | dandelion\|gb161\|DY8 39599 | 2157 | 249 | 89.86 | tblastn |
| 651 | LYM10 H80 | eucalyptus\|gb166\|CD6 69252 | 2158 | 249 | 92.8 | blastp |
| 652 | LYM10 H232 | fern\|gb171\|DK961389 | 2159 | 249 | 88.4 | blastp |
| 653 | LYM10 H81 | fescue\|gb 161\|DT7022 92 | 2160 | 249 | 100 | tblastn |
| 654 | LYM10 H82 | fescue\|gb161\|DT7044 58 | 2161 | 249 | 100 | tblastn |
| 655 | LYM10 H233 | flax\|09v1\|EU829193 | 2162 | 249 | 92.8 | blastp |
| 656 | LYM10 H234 | gerbera\|09v1\|AJ76119 3 | 2163 | 249 | 89.9 | blastp |
| 657 | LYM10 H235 | gerbera\|09v1\|AJ76591 3 | 2164 | 249 | 84.1 | blastp |
| 658 | LYM10 H83 | ginger\|gb164\|DY3684 24 | 2165 | 249 | 95.65 | tblastn |
| 659 | LYM10 H84 | ginger\|gb164\|DY3821 25 | 2166 | 249 | 94.2 | blastp |
| 660 | LYM10 H85 | grape\|gb160\|BQ79355 2 | 2167 | 249 | 91.3 | blastp |
| 661 | LYM10 H86 | grape\|gb160\|CA80999 7 | 2168 | 249 | 91.3 | blastp |
| 662 | LYM10 H87 | iceplant\|gb164\|A19434 23 | 2169 | 249 | 88.4 | blastp |
| 663 | LYM10 H88 | ipomoea\|gb157.2\|BJ55 3479 | 2170 | 249 | 89.86 | tblastn |
| 664 | LYM10 H89 | ipomoea\|gb157.2\|CB3 29955 | 2171 | 249 | 88.41 | tblastn |
| 665 | LYM10 H90 | ipomoea\|gb157.2\|CJ75 1960 | 2172 | 249 | 88.4 | blastp |
| 666 | LYM10 H236 | jatropha\|09v 1\|GO2476 49 | 2173 | 249 | 94.2 | blastp |
| 667 | LYM10 | kiwi\|gb166\|FG397070 | 2174 | 249 | 89.9 | blastp |
| 668 | LYM10 | kiwi\|gb166\|FG477805 | 2175 | 249 | 95.7 | blastp |
| 669 | LYM10 H93 | lettuce\|gb157.2\|DW04 7717 | 2176 | 249 | 88.4 | blastp |
| 670 | LYM10 H94 | lettuce\|gb157.2\|DW05 1281 | 2177 | 249 | 89.9 | blastp |
| 671 | LYM10 H95 | lettuce\|gb157.2\|DW08 0235 | 2178 | 249 | 82.61 | tblastn |
| 672 | LYM10 H96 | lettuce\|gb157.2\|DW10 1958 | 2179 | 249 | 88.4 | blastp |
| 673 | LYM10 H97 | lettuce\|gb157.2\|DW12 3456 | 2180 | 249 | 88.41 | tblastn |
| 674 | LYM10 H237 | liquorice\|gb171\|FS242 287 | 2181 | 249 | 94.2 | blastp |
| 675 | LYM10 H98 | liriodendroa\|gb166\|FD 495465 | 2182 | 249 | 95.7 | blastp |
| 676 | LYM10 H99 | liriodendron\|gb 166\|FD 500844 | 2183 | 249 | 94.2 | blastp |
| 677 | LYM10 H238 | lolium\|09v1\|AU24781 9 | 2184 | 249 | 98.6 | blastp |
| 678 | LYM10 H239 | lotus\|09v1\|CB827059 | 2185 | 249 | 92.8 | blastp |
| 679 | LYM10 H240 | lotus\|09v1\|DN6S2280 | 2186 | 249 | 89.9 | blastp |
| 680 | LYM10 H102 | lovegrass\|gb167\|DN48 2980 | 2187 | 249 | 98.6 | blastp |
| 681 | LYM10 H241 | maize\|gb170\|AI00134 0 | 2188 | 249 | 97.1 | blastp |
| 682 | LYM10 H242 | maize\|gb 170\|AI66551 2 | 2189 | 249 | 98.6 | blastp |
| 683 | LYM10 H243 | maize\|gb170\|AI67719 5 | 2190 | 249 | 97.1 | blastp |
| 684 | LYM10 H244 | maize\|gb170\|LLAI619 401 | 2191 | 249 | 97.1 | blastp |
| 685 | LYM10 H245 | maize\|gb170\|LLCF003 156 | 2192 | 249 | 81.16 | tblastn |
| 686 | LYM10 H246 | maize\|gb 170\|LLDQ24 5943 | 2193 | 249 | 98.6 | blastp |
| 687 | LYM10 H247 | maize\|gb170\|W21637 | 2194 | 249 | 98.6 | blastp |
| 688 | LYM10 H109 | marchantia\|gb166\|BJ8 44102 | 2195 | 249 | 87 | blastp |
| 689 | LYM10 H248 | medicago\|09v1 \|AA660 461 | 2196 | 249 | 94.2 | blastp |
| 690 | LYM10 H249 | medicago\|09v1\|AW28 7868 | 2197 | 249 | 94.2 | blastp |
| 691 | LYM10 H250 | medicago\|09v1\|LLBQ 138650 | 2198 | 249 | 85.5 | blastp |
| 692 | LYM10 H112 | melon\|gb165\|AM7150 93 | 2199 | 249 | 94.2 | tblastn |
| 693 | LYM10 H113 | melon\|gb165\|AM7204 95 | 2200 | 249 | 98.55 | tblastn |
| 694 | LYM10 H114 | melon\|gb165lDV6317 10 | 2201 | 249 | 95.7 | blastp |
| 695 | LYM10 H251 | millet\|09v 1CD724432 | 2202 | 249 | 98.6 | blastp |
| 696 | LYM10 H252 | monkeyflower\|09v1\|D V208117 | 2203 | 249 | 91.3 | blastp |
| 697 | LYM10 H116 | nuphar\|gb 166\|CD4725 02 | 2204 | 249 | 97.1 | blastp |
| 698 | LYM10 H253 | oak\|gb170\|SRR006307 S0013335 | 2205 | 249 | 94.2 | blastp |
| 699 | LYM10 H254 | oak\|gb170\|SRR006307 S0023745 | 2206 | 249 | 92.75 | tblastn |
| 700 | LYM10 H117 | oil palm\|gb 166\|EL684180 | 2207 | 249 | 92.8 | blastp |
| 701 | LYM10 H118 | onion\|gb162\|BQ58014 8 | 2208 | 249 | 97.1 | blastp |
| 702 | LYM10 H119 | papayalgb165\|EX2792 51 | 2209 | 249 | 89.9 | blastp |
| 703 | LYM 10 H255 | peanut\|gb171\|EE1245 30 | 2210 | 249 | 94.2 | blastp |
| 704 | LYM10 H256 | peanut\|gb171\|EE1266 80 | 2211 | 249 | 94.2 | blastp |
| 705 | LYM10 H257 | peanut\|gb171\|EG0288 25 | 2212 | 249 | 81.2 | blastp |
| 706 | LYM10 H258 | peanut\|gb 171\|EG3739 93 | 2213 | 249 | 94.2 | blastp |
| 707 | LYM10 H259 | pepper\|gb171\|CA5166 79 | 2214 | 249 | 91.3 | blastp |
| 708 | LYM10 H260 | pepper\|gb171\|GD0537 70 | 2215 | 249 | 89.9 | blastp |
| 709 | LYM10 H261 | petunia\|gb 171\|AF0499 33 | 2216 | 249 | 87 | blastp |
| 710 | LYM10 H262 | petunia\|gb171\|EB1743 81 | 2217 | 249 | 87 | blastp |
| 711 | LYM10 H263 | physcomitrella\| 10v1\|A W145358 | 2218 | 249 | 81.2 | blastp |
| 712 | LYM10 H264 | physcomitrella\|10v1\|B G361572 | 2219 | 249 | 81.2 | blastp |
| 713 | LYMIO H133 | pine\|gb157.2\|AL75081 3 | 2220 | 249 | 91.3 | blastp |
| 714 | LYM10 H134 | pine\|gb157.2\|AW0648 95 | 2221 | 249 | 91.3 | blastp |
| 715 | LYM10 H135 | pine\|gb157.2\|AW2264 88 | 2222 | 249 | 89.9 | blastp |
| 716 | LYM10 H265 | poplar\|gb170\|BI12774 5 | 2223 | 249 | 92.8 | blastp |
| 717 | LYM10 H266 | poplar\|gb170\|BU8241 90 | 2224 | 249 | 92.8 | blastp |
| 718 | LYM10 H267 | poplar\|gb170\|BU8626 32 | 2225 | 249 | 92.8 | blastp |
| 719 | LYM10 H139 | poppy\|gb 166\|FE96500 9 | 2226 | 249 | 88.4 | blastp |
| 720 | LYM10 H140 | poppy\|gb166\|FE96643 0 | 2227 | 249 | 92.8 | blastp |
| 721 | LYM10 H141 | potato\|gb157.2\|BG350 890 | 2228 | 249 | 91.3 | blastp |
| 722 | LYM10 H142 | potato\|gb157.2\|BG589 211 | 2229 | 249 | 89.86 | tblastn |
| 723 | LYM10 H143 | potato\|gb157.2\|BG592 598 | 2230 | 249 | 89.86 | tblastn |
| 724 | LYM10 H144 | potato\|gb157.2\|BQ516 058 | 2231 | 249 | 91.3 | blastp |
| 725 | LYM10 H145 | prunus\|gb167\|BU0395 66 | 2232 | 249 | 92.8 | blastp |
| 726 | LYM10 H146 | prunus\|gb167\|BU0467 83 | 2233 | 249 | 87 | blastp |
| 727 | LYM10 H147 | radish\|gb164\|EV52635 4 | 2234 | 249 | 91.3 | tblastn |
| 728 | LYM10 H148 | radish\|gb164\|EV52839 0 | 2235 | 249 | 91.3 | blastp |
| 729 | LYM10 H 149 | radish\|gb164\|EV53627 3 | 2236 | 249 | 91.3 | blastp |
| 730 | LYM10 H150 | radish\|gb164\|EV54575 1 | 2237 | 249 | 91.3 | tblastn |
| 731 | LYM10 H151 | radish\|gb 164\|EV54872 1 | 2238 | 249 | 91.3 | blastp |
| 732 | LYM10 H152 | radish\|gb164\|EV55048 8 | 2239 | 249 | 91.3 | blastp |
| 733 | LYM10 H153 | radish\|gb164\|EV56739 7 | 2240 | 249 | 85.5 | blastp |
| 734 | LYM10 H154 | radish\|gb164\|EW7134 25 | 2241 | 249 | 89.9 | blastp |
| 735 | LYM10 H155 | radish\|gb164\|FD57055 9 | 2242 | 249 | 89.9 | blastp |
| 736 | LYM10 H268 | rice\|gb170\|OS06G443 74 | 2243 | 249 | 95.7 | blastp |
| 737 | LYM10 H151 | rose\|gb157.2\|BI97819 8 | 2244 | 249 | 91.3 | tblastn |
| 738 | LYM10 H158 | rose\|gb157.2\|EC58984 2 | 2245 | 249 | 92.75 | tblastn |
| 739 | LYM10 H159 | safflower\|gb162\|EL39 3855 | 2246 | 249 | 88.41 | tblastn |
| 740 | LYM10 H160 | safflower\|gb162\|EL51 1136 | 2247 | 249 | 89.9 | blastp |
| 741 | LYM10 H269 | senecio\|gb170\|CO553 399 | 2248 | 249 | 88.4 | blastp |
| 742 | LYM10 H161 | sesame\|gb157.2\|BU66 9069 | 2249 | 249 | 91.3 | tblastn |
| 743 | LYM10 H270 | solanum phureja\|09v 1\|SPHAI4 83617 | 2250 | 249 | 89.9 | blastp |
| 744 | LYM10 H271 | solanum phureja\|09v1\|SPHBG1 27130 | 2251 | 249 | 91.3 | blastp |
| 745 | LYM10 H272 | sorghum\|09v1\|SB04G 005280 | 2252 | 249 | 98.6 | blastp |
| 746 | LYM10 H273 | sorghum\|09v1\|SB10G 026000 | 2253 | 249 | 97.1 | blastp |
| 747 | LYM10 H164 | soybean\|gb168\|AA660 461 | 2254 | 249 | 94.2 | blastp |
| 748 | LYM10 H165 | soybean\|gb168\|AW47 2512 | 2255 | 249 | 92.8 | blastp |
| 749 | LYM10 H166 | soybean\|gb168\|BU544 187 | 2256 | 249 | 94.2 | blastp |
| 750 | LYM10 H167 | spikemoss\|gb165\|DN8 38422 | 2257 | 249 | 85.51 | tblastn |
| 751 | LYM10 H168 | spruce\|gb162\|CO2169 79 | 2258 | 249 | 91.3 | blastp |
| 752 | LYM10 H169 | spruce\|gb162\|CO2170 20 | 2259 | 249 | 91.3 | blastp |
| 753 | LYM10 H170 | spurge\|gb161\|DV1126 55 | 2260 | 249 | 84.1 | blastp |
| 754 | LYM10 H171 | spurge\|gb161\|DV1202 63 | 2261 | 249 | 86.5 | blastp |
| 755 | LYM10 H172 | strawberry\|gb164\|CO3 80171 | 2262 | 249 | 91.3 | tblastn |
| 756 | LYMIO H173 | strawberry\|gb164\|EX6 64646 | 2263 | 249 | 92.8 | blastp |
| 757 | LYM10 H274 | sugarcane\|gb157.3\|CA 072778 | 2264 | 249 | 97.1 | blastp |
| 758 | LYM10 H275 | sugarcane\|gb157.3\|CA 087927 | 2265 | 249 | 98.6 | blastp |
| 759 | LYMIO H276 | sugarcane\|gb157.3\|CA 103056 | 2266 | 249 | 98.6 | blastp |
| 760 | LYM10 H177 | sunflower\|gb162\|BU0 15373 | 2267 | 249 | 89.86 | tblastn |
| 761 | LYM10 H178 | sunflower\|gb162\|CD8 49625 | 2268 | 249 | 88.4 | blastp |
| 762 | LYMIO H179 | sunflower\|gb162\|CD8 51122 | 2269 | 249 | 88.41 | tblastn |
| 763 | LYM10 H180 | switchgrass\|gb167\|DN 145554 | 2270 | 249 | 98.6 | blastp |
| 764 | LYM10 H181 | switchgrass\|gb167\|FE6 33347 | 2271 | 249 | 97.1 | blastp |
| 765 | LYM10 H182 | switchgrass\|gb167\|FE6 39131 | 2272 | 249 | 98.6 | blastp |
| 766 | LYM10 H183 | switchgrass\|gb167\|FL7 20694 | 2273 | 249 | 95.7 | blastp |
| 767 | LYM10 H184 | tamarix\|gb166\|EG968 743 | 2274 | 249 | 85.5 | blastp |
| 768 | LYM10 H185 | tamarix\|gb166\|EG969 152 | 2275 | 249 | 88.4 | blastp |
| 769 | LYM10 H277 | tea\|gb171\|FF682807 | 2276 | 249 | 95.7 | blastp |
| 770 | LYM10 H186 | thellungiella\|gb167\|BI 698898 | 2277 | 249 | 91.3 | blastp |
| 771 | LYM10 H187 | thellungiella\|gb167\|EC 599088 | 2278 | 249 | 91.3 | blastp |
| 772 | LYM10 H188 | tobacco\|gb162\|CV020 564 | 2279 | 249 | 81.8 | blastp |
| 773 | LYM10 H189 | tobacco\|gb162\|CV021 149 | 2280 | 249 | 80.8 | blastp |
| 774 | LYM10 H190 | tobacco\|gb162\|CV021 577 | 2281 | 249 | 91.3 | tblastn |
| 775 | LYM10 H191 | tobacco\|gb162\|EB426 093 | 2282 | 249 | 91.3 | tblastn |
| 776 | LYM10 H192 | tobacco\|gb1 62\|EB447 225 | 2283 | 249 | 89.9 | blastp |
| 777 | LYM10 H278 | tomato\|09v1\|AI483617 | 2284 | 249 | 89.9 | blastp |
| 778 | LYM10 H279 | tomato\|09v1\|BG12713 0 | 2285 | 249 | 91.3 | tblastn |
| 779 | LYM10 H195 | triphysaria\|gb164\|EX9 88147 | 2286 | 249 | 81.6 | blastp |
| 780 | LYMIO H 196 | walnuts\|gb166\|CB304 079 | 2287 | 249 | 98.6 | blastp |
| 781 | LYM10 H197 | wheat\|gb164\|BE42322 6 | 2288 | 249 | 100 | tblastn |
| 782 | LYM10 H198 | wheat\|gb164\|BE42385 8 | 2289 | 249 | 100 | tblastn |
| 783 | LYM10 H199 | wheat\|gb164\|BE44513 9 | 2290 | 249 | 98.55 | tblastn |
| 784 | LYM10 H200 | wheat\|gb164\|BE60483 4 | 2291 | 249 | 98.55 | tblastn |
| 785 | LYM10 H201 | wheat\|gb164\|BF47348 2 | 2292 | 249 | 100 | tblastn |
| 786 | LYM10 H202 | wheat\|gb164\|BF47481 4 | 2293 | 249 | 98.55 | tblastn |
| 787 | LYM10 H203 | wheat\|gb 164\|BI47989 5 | 2294 | 249 | 98.55 | tblastn |
| 788 | LYM10 H204 | wheat\|gb164\|CA61935 7 | 2295 | 249 | 86.96 | tblastn |
| 789 | LYM10 H205 | wheat\|gb164\|CA61996 5 | 2296 | 249 | 91.3 | tblastn |
| 790 | LYM10 H206 | wheat\|gb164\|CA62731 5 | 2297 | 249 | 83.8 | blastp |
| 791 | LYM10 H207 | wheat\|gb164\|DR73720 5 | 2298 | 249 | 85.51 | tblastn |
| 792 | LYM13 H3 | brachypodium\|09v1\|G T794488 | 2299 | 251 | 80.6 | blastp |
| 793 | LYM13 H4 | maize\|gb170\|T12684 | 2300 | 251 | 84.5 | blastp |
| 794 | LYM13 H5 | sorghum\|09v1\|SB01G 049950 | 2301 | 251 | 84.5 | blastp |
| 795 | LYM13 H3 | switchgrass\|gb167\|FE6 34401 | 2302 | 251 | 84.53 | tblastn |
| 796 | LYM14 H1 | aquilegia\|gb157.3\|DR9 27713 | 2303 | 252 | 81.1 | blastp |
| 797 | LYM14 H31 | arabidopsis lyrata\|09v1\|JGIAL009 556 | 2304 | 252 | 80.7 | blastp |
| 798 | LYM14 H32 | arabidopsis lyrata\|09v1\|JGIAL020 254 | 2305 | 252 | 80.4 | blastp |
| 799 | LYM14 H2 | arabidopsis\|gb165\|AT 3G11320 | 2306 | 252 | 80.4 | blastp |
| 800 | LYM14 H3 | arabidopsis\|gb165\|AT 5G05820 | 2307 | 252 | 80.4 | blastp |
| 801 | LYM14 H4 | artemisia\|gb164\|EY03 4514 | 2308 | 252 | 81.7 | blastp |
| 802 | LYM14 H33 | brachypodium\|09v1\|G T762944 | 2309 | 252 | 96 | blastp |
| 803 | LYM14 H7 | canola\|gb161\|DY0246 85 | 2310 | 252 | 81.1 | blastp |
| 804 | LYM14 H34 | cassava\|09v1\|CK6500 18 | 2311 | 252 | 80.1 | blastp |
| 805 | LYM14 H35 | castorbean\|09v1\|EG65 9029 | 2312 | 252 | 80.43 | tblastn |
| 806 | LYM14 H8 | centaurea\|gb166\|EH71 2821 | 2313 | 252 | 80.1 | blastp |
| 807 | LYM14 H36 | cichorium\|gb171\|EH6 88253 | 2314 | 252 | 80.7 | blastp |
| 808 | LYM14 H11 | cotton\|gb164\|AA6599 84 | 2315 | 252 | 80.43 | tblastn |
| 809 | LYM14 H37 | cucumber\|09v1\|DN91 0737 | 2316 | 252 | 80.75 | tblastn |
| 810 | LYM14 H12 | ginger\|gb164\|DY3589 76 | 2317 | 252 | 83.3 | blastp |
| 811 | LYM14 H13 | iceplant\|gb164\|AI8228 35 | 2318 | 252 | 80.75 | tblastn |
| 812 | LYM14 H14 | lettuce\|gb157.2\|DW15 8376 | 2319 | 252 | 82.3 | tblastn |
| 813 | LYM14 H15 | leymus\|gb166\|CD8091 80 | 2320 | 252 | 95 | blastp |
| 814 | LYM14 H38 | maize\|gb 170\|AI78326 0 | 2321 | 252 | 93.8 | blastp |
| 815 | LYM14 H39 | maize\|gb 170\|AI94167 5 | 2322 | 252 | 95.1 | blastp |
| 816 | LYM14 H18 | melon\|gb165\|AM7137 63 | 2323 | 252 | 80.5 | tblastn |
| 817 | LYM14 H40 | monkeyflower\|09v1\|G O959633 | 2324 | 252 | 80.12 | tblastn |
| 818 | LYM14 H41 | monkeyflower\|09v1\|G R111000 | 2325 | 252 | 80.12 | tblastn |
| 819 | LYM14 H19 | papaya\|gb165\|EX2611 25 | 2326 | 252 | 81.1 | blastp |
| 820 | LYM14 H20 | radish\|gb164\|EW7236 81 | 2327 | 252 | 81.37 | tblastn |
| 821 | LYM14 H42 | solanum phureja\|09v 1\|SPHBG6 28013 | 2328 | 252 | 80.1 | blastp |
| 822 | LYM14 H43 | sorghum\|09v1\|SB01G 038730 | 2329 | 252 | 96 | blastp |
| 823 | LYM14 H44 | sorghum\|09v1\|SB02G 044050 | 2330 | 252 | 86 | blastp |
| 824 | LYM14 H23 | soybean\|gb168\|AW56 0935 | 2331 | 252 | 80.1 | blastp |
| 825 | LYM14 H25 | spikemoss\|gb165\|FE43 4307 | 2332 | 252 | 81.06 | tblastn |
| 826 | LYM14 H45 | sugarcane\|gb157.3\|CA 079818 | 2333 | 252 | 84.2 | blastp |
| 827 | LYM14 H46 | sugarcane\|gb157.3\|CA 150518 | 2334 | 252 | 93.85 | tblastn |
| 828 | LYM14 H29 | sunflower\|gb162\|EL48 4937 | 2335 | 252 | 80.75 | tblastn |
| 829 | LYM14 H30 | switchgrass\|gb167\|DN 143407 | 2336 | 252 | 95.4 | blastp |
| 830 | LYM14 H47 | tomato\|09v1\|BG62801 3 | 2337 | 252 | 80.1 | blastp |
| 831 | LYM14 H31 | wheat\|gb164\|BE41600 3 | 2338 | 252 | 83.6 | blastp |
| 832 | LYM15 H4 | brachypodium\|09v 1\|D V476162 | 2339 | 253 | 80.2 | blastp |
| 833 | LYM15 H2 | pseudoroegneria\|gb16 7\|FF343970 | 2340 | 253 | 82 | blastp |
| 834 | LYM15 H3 | wheat\|gb164\|BE213295 | 2341 | 253 | 81.4 | blastp |
| 835 | LYM15 H4 | wheat\|gb164\|BE49683 3 | 2342 | 253 | 81.4 | blastp |
| 836 | LYM16 H9 | barley\|gb157SOLEXA \|BE421507 | 2343 | 254 | 90.9 | blastp |
| 837 | LYM16 H10 | brachypodium\|09v1\|D V475217 | 2344 | 254 | 92.7 | blastp |
| 838 | LYM16 H3 | fescue\|gb161\|DT691110 | 2345 | 254 | 93.9 | blastp |
| 839 | LYM16 H11 | lolium\|09v1\|AU24687 6 | 2346 | 254 | 84.1 | blastp |
| 840 | LYD 199 | maize\|gb 170\|BI42368 7 | 2347 | 254 | 82.9 | blastp |
| 841 | LYM16 H12 | maize\|gb170\|LLFL254 633 | 2348 | 254 | 81.1 | tblastn |
| 842 | LYM16 H5 | pseudoroegneria\|gb16 7\|FF355494 | 2349 | 254 | 92.1 | blastp |
| 843 | LYM16 H6 | rye\|gb164\|BE494944 | 2350 | 254 | 90.24 | tblastn |
| 844 | LYM16 H7 | wheat\|gb164\|BE21698 I | 2351 | 254 | 91.5 | blastp |
| 845 | LYM16 H8 | wheat\|gb164\|BE416071 | 2352 | 254 | 90.9 | blastp |
| 846 | LYM16 H9 | wheat\|gb164\|BE418113 | 2353 | 254 | 91.5 | blastp |
| 847 | LYM17 H6 | barley\|gb157SOLEXA \|BE602651 | 2354 | 255 | 83.3 | blastp |
| 848 | LYM17 H7 | sugarcane\|gb157.3\|CA 152022 | 2355 | 255 | 80.3 | blastp |
| 849 | LYM17 H3 | wheat\|gb164\|BE406565 | 2356 | 255 | 85.6 | blastp |
| 850 | LYM17 H4 | wheat\|gb164\|BE429209 | 2357 | 255 | 85.6 | blastp |
| 851 | LYM17 H5 | wheat\|gb164\|BE490714 | 2358 | 255 | 86.4 | blastp |
| 852 | LYM17 H6 | wheat\|gb164\|BQ803190 | 2359 | 255 | 85.6 | blastp |
| 853 | LYM19 H10 | barley\|gb 157SOLEXA \|AL506367 | 2360 | 256 | 86 | blastp |
| 854 | LYM19 H11 | brachypodium\|09v1\|D V476339 | 2361 | 256 | 87.2 | blastp |
| 855 | LYM19 H3 | leymus\|gb166\|EG387247 | 2362 | 256 | 84.8 | blastp |
| 856 | LYM19 H5 | pseudoroegneria\|gb16 7\|FF352256 | 2363 | 256 | 86.9 | blastp |
| 857 | LYM19 H12 | sorghum\|09v1\|SB05G 009990 | 2364 | 256 | 82.6 | blastp |
| 858 | LYM19 H7 | switchgrass\|gb167\|FE6 03507 | 2365 | 256 | 83.6 | blastp |
| 859 | LYM19 H8 | wheat\|gb164\|BE398692 | 2366 | 256 | 82.7 | blastp |
| 860 | LYM 19 H9 | wheat\|gb164\|BE58597 9 | 2367 | 256 | 86.28 | tblastn |
| 861 | LYM19 H10 | wheat\|gb164\|BU67232 5 | 2368 | 256 | 81.4 | blastp |
| 862 | LYM20 H9 | barley\|gb157SOLEXA \|AL450927 | 2369 | 257 | 86.3 | blastp |
| 863 | LYM20 H10 | brachypodium\|09v1\|D V479896 | 2370 | 257 | 89.1 | blastp |
| 864 | LYM20 H11 | castorbean\|09v1\|XM0 02519056 | 2371 | 257 | 80 | blastp |
| 865 | LYM20 H12 | maize\|gb170\|AI85723 6 | 2372 | 257 | 90.1 | blastp |
| 866 | LYM20 H5 | pseudoroegneria\|gb 16 7\|FF343142 | 2373 | 257 | 89 | blastp |
| 867 | LYM20 H13 | sorghum\|09v1\|SB01G 009140 | 2374 | 257 | 89.7 | blastp |
| 868 | LYM20 H14 | sugarcane\|gb157.3\|CA 072511 | 2375 | 257 | 83.9 | blastp |
| 869 | LYM20 H8 | switchgrass\|gb167\|FE6 54910 | 2376 | 257 | 84.5 | blastp |
| 870 | LYM20 H9 | wheat\|gb164\|BE411982 | 2377 | 257 | 88.6 | blastp |
| 871 | LYM21 H1 | banana\|gb167\|FF557436 | 2378 | 258 | 80.9 | blastp |
| 872 | LYM21 H2 | banana\|gb167\|FF559448 | 2379 | 258 | 80.9 | blastp |
| 873 | LYM21 H27 | barley\|gb157SOLEXA \|BE437461 | 2380 | 258 | 88.2 | blastp |
| 874 | LYM21 H28 | brachypodium\|09v 1\|D V488150 | 2381 | 258 | 95.5 | blastp |
| 875 | LYM21 H29 | brachypodium\|09v1\|G T760558 | 2382 | 258 | 97.3 | blastp |
| 876 | LYM21 H5 | cenchrus\|gb166\|EB655 | 2383 | 258 | 91.8 | blastp |
| 877 | LYM21 H6 | fescue\|gb161\|DT680631 | 2384 | 258 | 90 | blastp |
| 878 | LYM21 H7 | kiwi\|gb166\|FG405276 | 2385 | 258 | 81.8 | blastp |
| 879 | LYM21 H8 | leymus\|gb166\|CN465770 | 2386 | 258 | 88.2 | blastp |
| 880 | LYM21 H30 | lolium\|09v1\|AU25028 8 | 2387 | 258 | 90 | blastp |
| 881 | LYM21 H9 | lovegrass\|gb167\|DN48 0337 | 2388 | 258 | 92.7 | blastp |
| 882 | LYM21 H31 | maize\|gb 170\|AI58645 9 | 2389 | 258 | 93.6 | blastp |
| 883 | LYM21 H32 | millet\|09v 1\|EVO454P M000432 | 2390 | 258 | 95.5 | blastp |
| 884 | LYM21 H33 | millet\|09v1\|EVO454P M000947 | 2391 | 258 | 91.8 | blastp |
| 885 | LYM21 H12 | pineapple\|gb157.2\|CO 731607 | 2392 | 258 | 82.73 | tblastn |
| 886 | LYM21 H34 | rice\|gb170\|OS02G473 20 | 2393 | 258 | 87.27 | tblastn |
| 887 | LYM21 H35 | sorghum\|09v1\|SB02G 006170 | 2394 | 258 | 93.6 | blastp |
| 888 | LYM21 H36 | sorghum\|09v1\|SB06G 027500 | 2395 | 258 | 95.5 | blastp |
| 889 | LYM21 H37 | sugarcane\|gb157.3\|BQ 529660 | 2396 | 258 | 93.6 | blastp |
| 890 | LYM21 H38 | sugarcane\|gb157.3\|BQ 535381 | 2397 | 258 | 92.7 | blastp |
| 891 | LYM21 H39 | sugarcane\|gb157.3\|CA 118830 | 2398 | 258 | 87.3 | blastp |
| 892 | LYM21 H19 | switchgrass\|gb167\|DN 151016 | 2399 | 258 | 93.6 | blastp |
| 893 | LYM21 H20 | swilchgrass\|gb167\|FL7 22429 | 2400 | 258 | 94.5 | blastp |
| 894 | LYM21 H21 | switchgrass\|gb167\|FL9 36988 | 2401 | 258 | 95.5 | blastp |
| 895 | LYM21 H22 | tobacco\|gb162\|AM791 579 | 2402 | 258 | 88.2 | blastp |
| 896 | LYM21 H23 | wheat\|gb164\|BE35263 2 | 2403 | 258 | 89.1 | blastp |
| 897 | LYM21 H24 | whent\|gb164\|BE40279 2 | 2404 | 258 | 89.1 | blastp |
| 898 | LYM21 H25 | wheat\|gb164\|BE49257 5 | 2405 | 258 | 89.09 | tblastn |
| 899 | LYM21 H26 | wheatlgb164\|CA48457 5 | 2406 | 258 | 94.5 | blastp |
| 900 | LYM21 H27 | wheat\|gb164\|CA61660 9 | 2407 | 258 | 92.73 | tblastn |
| 901 | LYM24 H1 | fescue\|gb 161\|DT6811 71 | 2408 | 261 | 80.61 | tblastn |
| 902 | LYM24 H2 | leymus\|gb166\|CD8086 | 2409 | 261 | 80.5 | blastp |
| 903 | LYM24 H8 | maize\|gb170\|AI62144 0 | 2410 | 261 | 81 | blastp |
| 904 | LYM24 H9 | pseudoroegneria\|gb16 7\|FF349814 | 2411 | 261 | 80 | blastp |
| 905 | LYM24 H10 | sorghum\|09v1\|SB03G 044280 | 2412 | 261 | 83.1 | blastp |
| 906 | LYM24 H11 | sugarcane\|gb157.3\|CA 072633 | 2413 | 261 | 82.6 | blastp |
| 907 | LYM24 H6 | switchgrass\|gb167\|DN 144637 | 2414 | 261 | 84.6 | blastp |
| 908 | LYM24 H7 | switchgrass\|gb167\|DN 145452 | 2415 | 261 | 85.6 | blastp |
| 909 | LYM24 H8 | wheat\|gb164\|BE42590 0 | 2416 | 261 | 80 | tblastn |
| 910 | LYM26 H1 | wheat\|gb164\|BE39890 3 | 2417 | 262 | 88.6 | blastp |
| 911 | LYM30 H5 | brachypodium\|09v1\|S RR031799S0073966 | 2418 | 263 | 86.5 | blastp |
| 912 | LYM30 H6 | maize\|gb170\|AW5201 | 2419 | 263 | 85.8 | blastp |
| 913 | LYM30 H7 | maize\|gb170\|AW9276 | 2420 | 263 | 85 | blastp |
| 914 | LYM30 H8 | rice\|gb170\|OS11G025 80 | 2421 | 263 | 99.2 | blastp |
| 915 | LYM30 H9 | rice\|gb170\|OS12G025 | 2422 | 263 | 87.7 | blastp |
| 916 | LYM30 H10 | sorghum\|09v1\|SB05G 001250 | 2423 | 263 | 86.1 | blastp |
| 917 | LYM30 H5 | switchgrass\|gb167\|FL7 96240 | 2424 | 263 | 80. 16 | tblastn |
| 918 | LYM31 H1 | rice\|gb170\|OS12G027 10 | 2425 | 264 | 97.9 | blastp |
| 919 | LYM35 H5 | brachypodium\|09v1\|S RR031798S0189278 | 2426 | 267 | 80 | blastp |
| 920 | LYM35 H6 | maize\|gb170\|BM4168 80 | 2427 | 267 | 89.7 | blastp |
| 921 | LYM35 H7 | sorghum\|09v1\|SB06G 031730 | 2428 | 267 | 86.7 | blastp |
| 922 | LYM35 H8 | sugarcane\|gb157.3\|CA 105471 | 2429 | 267 | 87.5 | blastp |
| 923 | LYM35 H4 | switchgrass\|gb167\|FL9 39819 | 2430 | 267 | 89.9 | blastp |
| 924 | LYM35 H5 | wheat\|gb164\|BE50050 4 | 2431 | 267 | 86.1 | blastp |
| 925 | LYM42 H0 | rice\|gb170\|OS01G411 20 | 2432 | 273 | 96.7 | blastp |
| 925 | LYM42 H0 | rice\|gb170\|OS01G411 20 | 2432 | 439 | 99.83 | tblastn |
| 926 | LYM43 H 1 | rice\|gb170\|OS12G028 | 2433 | 274 | 91.4 | blastp |
| 927 | LYM52 H1 | b rapa\|gb 162\|EX068270 | 2434 | 277 | 94.5 | blastp |
| 928 | LYM52 H2 | fescue\|gb161\|CK8028 23 | 2435 | 277 | 84.4 | blastp |
| 929 | LYM52 H3 | leymus\|gb166\|EG3794 66 | 2436 | 277 | 96.3 | blastp |
| 930 | LYM52 H10 | maize\|gb170\|BE13009 4 | 2437 | 277 | 80 | blastp |
| 931 | LYM52 H11 | maize\|gb 170\|LLBE05 6010 | 2438 | 277 | 81 | blastp |
| 932 | LYM52 H12 | rice\|gb170\|OS04G517 92 | 2439 | 277 | 81.4 | blastp |
| 933 | LYM52 H13 | sorghum\|09v1\|SB06G 027870 | 2440 | 277 | 82.5 | blastp |
| 934 | LYM52 H14 | sugarcane\|gb157.3\|AA 577629 | 2441 | 277 | 84.47 | tblastn |
| 935 | LYM52 H8 | switchgrass\|gb167\|DN 147335 | 2442 | 277 | 82.65 | tblastn |
| 936 | LYM52 H9 | wheat\|gb164\|BG909259 | 2443 | 277 | 94.5 | blastp |
| 937 | LYM52 H10 | wheat\|gb164\|BG90949 3 | 2444 | 277 | 95.1 | blastp |
| 938 | LYM56 H9 | brachypodium\|09v 1\|S RR031795S0049724 | 2445 | 279 | 85.9 | blastp |
| 939 | LYM56 H10 | maize\|gb170\|AI71195 4 | 2446 | 279 | 80.14 | tblastn |
| 940 | LYM56 H2 | pseudoroegneria\|gb 16 7\|FF341776 | 2447 | 279 | 87.9 | blastp |
| 941 | LYM56 H11 | rice\|gb170\|OS03G457 20 | 2448 | 279 | 80.1 | blastp |
| 942 | LYM56 H12 | sorghum\|09v1\|SB01G 012840 | 2449 | 279 | 81 | blastp |
| 943 | LYM56 H13 | sugarcane\|gb157.3\|BQ 533995 | 2450 | 279 | 80.14 | tblastn |
| 944 | LYM56 H6 | switchgrass\|gb167\|FE6 31693 | 2451 | 279 | 84.5 | blastp |
| 945 | LYM56 H7 | switchgrass\|gb1671\|FL7 82747 | 2452 | 279 | 83.1 | blastp |
| 946 | LYM56 H8 | wheat\|gb164\|BE404207 | 2453 | 279 | 88.7 | blastp |
| 947 | LYM56 H9 | wheat\|gb164\|CD912963 | 2454 | 279 | 87.8 | blastp |
| 948 | LYM57 H0 | brachypodium\|09v1\|D V475724 | 2455 | 280 | 81.1 | blastp |
| 949 | LYM62 H1 | sorghum\|09v1\|SB10G 012150 | 2456 | 282 | 88.9 | blastp |
| 950 | LYM66 H1 | wheat\|gb164\|BE403932 | 2457 | 283 | 83 | blastp |
| 950 | LYM66 H 1 | wheat\|gb164\|BE403932 | 2457 | 440 | 83 | blastp |
| 951 | LYM66 H2 | wheat\|gb164\|BE405409 | 2458 | 283 | 90.4 | blastp |
| 951 | LYM66 H2 | wheat\|gb164\|BE405409 | 2458 | 440 | 90.4 | blastp |
| 952 | LYM66 H3 | wheat\|gb164\|CA600263 | 2459 | 283 | 90.1 | blastp |
| 952 | LYM66 H3 | wheat\|gb164\|CA60026 3 | 2459 | 440 | 90.1 | blastp |
| 953 | LYM69 HO | rice\|gb170\|OS07G42520 | 2460 | 286 | 98.3 | blastp |
| 954 | LYM73 H6 | brachypodium\|09v1\|D V481090 | 2461 | 287 | 95.8 | blastp |
| 955 | LYM73 H7 | maize\|gb170\|AW256155 | 2462 | 287 | 93.7 | blastp |
| 956 | LYM73 H8 | sorghum\|09v1\|SB07G 004300 | 2463 | 287 | 94.3 | blastp |
| 957 | LYM73 H9 | sugarcane\|gb157.3\|CA 117425 | 2464 | 287 | 94.3 | blastp |
| 958 | LYM73 H5 | switcbgrass\|gb167\|DN 145973 | 2465 | 287 | 94.6 | blastp |
| 959 | LYM73 H6 | wheat\|gb164\|AF289257S1 | 2466 | 287 | 92.67 | tblastn |
| 960 | LYM79 H3 | brachypodium\|09v1\|S RR031800S0005207 | 2467 | 289 | 83.8 | blastp |
| 961 | LYM79 H4 | millet\|09v 1\|EVO454P M011117 | 2468 | 441 | 82.72 | tblastn |
| 962 | LYM79 H5 | sorghum\|09v1\|SB10G 012140 | 2469 | 289 | 93 | blastp |
| 962 | LYM79 H5 | sorghum\|09v1\|SB10G 012140 | 2469 | 441 | 93.75 | tblastn |
| 963 | LYM79 H 1 | switchgrass\|gb167\|FE5 98528 | 2470 | 289 | 90.6 | blastp |
| 963 | LYM79 H1 | switchgrass\|gb167\|FE5 98528 | 2470 | 441 | 91.1 | tblastn |
| 964 | LYM79 H2 | switchgrass\|gb167\|FL9 57870 | 2471 | 441 | 86.6 | tblastn |
| 965 | LYM79 H3 | wheat\|gb164\|BE590518 | 2472 | 289 | 80 | blastp |
| 965 | LYM79 H3 | wheat\|gb164\|BE590518 | 2472 | 441 | 83.33 | tblastn |
| 966 | LYM82 H1 | banana\|gb167\|FF561962 | 2473 | 290 | 82.1 | blastp |
| 967 | LYM82 H12 | brachypodium\|09v1\|G T816645 | 2474 | 290 | 94.1 | blastp |
| 968 | LYM82 H2 | leymus\|gb166\|EG384073 | 2475 | 290 | 97.9 | blastp |
| 969 | LYM82 H13 | maize\|gb170\|AW1811 52 | 2476 | 290 | 90.6 | blastp |
| 970 | LYM82 H4 | melon\|gb165\|AM7182 13 | 2477 | 290 | 80.21 | tblastn |
| 971 | LYM82 H 14 | millet\|09v1\|EVO454P M002754 | 2478 | 290 | 82.99 | tblastn |
| 972 | LYM82 H5 | pseudoroegneria\|gb 16 7\|FF352234 | 2479 | 290 | 99 | blastp |
| 973 | LYM82 H15 | rice\|gb170\|OS06G044 60 | 2480 | 290 | 91 | blastp |
| 974 | LYM82 H16 | sorghum\|09v1\|SB10G 002420 | 2481 | 290 | 90.3 | blastp |
| 975 | LYM82 H8 | soybean\|gb168\|CA921 223 | 2482 | 290 | 80.21 | tblastn |
| 976 | LYM82 H17 | sugarcane\|gb157.3\|BU 102729 | 2483 | 290 | 90.3 | blastp |
| 977 | LYM82 H10 | switchgrass\|gb167\|FL7 44837 | 2484 | 290 | 89.6 | blastp |
| 978 | LYM82 H11 | wheat\|gb164\|BE40384 2 | 2485 | 290 | 99 | blastp |
| 979 | LYM82 H12 | wheat\|gb164\|CA66078 8 | 2486 | 290 | 99 | blastp |
| 980 | LYM83 H8 | brachypodium\|09v1\|S RR031797S0009670 | 2487 | 291 | 86.5 | blastp |
| 980 | LYM83 H8 | brachypodium\|09v 1\|S RR031797S0009670 | 2487 | 442 | 86.1 | blastp |
| 981 | LYM83 H9 | lolium\|09v1\|ES699086 | 2488 | 291 | 84.84 | tblastn |
| 981 | LYM83 H9 | lolium\|09v1\|ES699086 | 2488 | 442 | 84.43 | tblastn |
| 982 | LYM83 H10 | maize\|gb 170\|AI66534 7 | 2489 | 291 | 82.4 | blastp |
| 982 | LYM83 H10 | maize\|gb170\|AI66534 7 | 2489 | 442 | 82.4 | blastp |
| 983 | LYM83 H3 | pseudoroegneria\|gb16 7\|FF354990 | 2490 | 291 | 97.5 | blastp |
| 983 | LYM83 H3 | pseudoroegneria\|gb 16 7\|FF354990 | 2490 | 442 | 97.1 | blastp |
| 984 | LYM83 H11 | rice\|gb 170\|OS05G453 00 | 2491 | 291 | 81.6 | blastp |
| 984 | LYM83 H11 | rice\|gb 170\|OS05G453 00 | 2491 | 442 | 81.1 | blastp |
| 985 | LYM83 H12 | sorghum\|09v1\|SB09G 026370 | 2492 | 291 | 82 | blastp |
| 985 | LYM83 H12 | sorghum\|09v1\|SB09G 026370 | 2492 | 442 | 81.6 | blastp |
| 986 | LYM83 H6 | switchgrass\|gb167\|DN 149383 | 2493 | 291 | 84 | blastp |
| 986 | LYM83 H6 | switchgrass\|gb167\|DN 149383 | 2493 | 442 | 83.6 | blastp |
| 987 | LYM83 H7 | wheat\|gb164\|BE51671 5 | 2494 | 291 | 94.7 | blastp |
| 987 | LYM83 H7 | wheat\|gb164\|BE516715 | 2494 | 442 | 94.3 | blastp |
| 988 | LYM83 H8 | wheat\|gb164\|BF428688 | 2495 | 291 | 95.1 | blastp |
| 988 | LYM83 H8 | wheat\|gb164\|BF42868 8 | 2495 | 442 | 94.7 | blastp |
| 989 | LYM84 H8 | brachypodium\|09v 1\|S RR031795S0021840 | 2496 | 292 | 92.8 | blastp |
| 990 | LYM84 H9 | maize\|gb170\|AW282161 | 2497 | 292 | 82.8 | blastp |
| 991 | LYM84 H10 | maize\|gb170\|LLDQ24 5778 | 2498 | 292 | 98.7 | blastp |
| 992 | LYM84 H4 | pseudoroegneria\|gb 16 7\|FF355744 | 2499 | 292 | 98.3 | blastp |
| 993 | LYM84 H11 | rice\|gb170\|OS03G416 12 | 2500 | 292 | 84.58 | tblastn |
| 994 | LYM84 H12 | sorghum\|09v1\|SB01G 014640 | 2501 | 292 | 82.9 | blastp |
| 995 | LYM84 H7 | switchgrass\|gb167\|FE6 52995 | 2502 | 292 | 81.3 | blastp |
| 996 | LYM84 H8 | wheat\|gb164\|BE41769 7 | 2503 | 292 | 95.1 | blastp |
| 997 | LYM88 H0 | arabidopsis lyrata\|09v1\|JGIAL014 996 | 2504 | 294 | 91.5 | blastp |
| 998 | LYM89 H5 | arabidopsis lyrata\|09v1\|JGIAL031 299 | 2505 | 295 | 86.36 | tblastn |
| 999 | LYM89 H2 | canola\|gb 161\|CD8120 18 | 2506 | 295 | 81.8 | blastp |
| 1000 | LYM89 H3 | canola\|gb161\|CD8218 97 | 2507 | 295 | 81.8 | blastp |
| 1001 | LYM89 H4 | radish\|gb164\|EW7327 98 | 2508 | 295 | 81.65 | tblastn |
| 1002 | LYM89 H5 | radish\|gb164\|EX74970 2 | 2509 | 295 | 80.7 | blastp |
| 1003 | LYM90 H3 | brachypodium\|09v1\|D V488904 | 2510 | 296 | 83.2 | blastp |
| 1004 | LYM90 H2 | wheat\|gb164\|BQ24215 | 2511 | 296 | 94.6 | blastp |
| 1005 | LYM90 H3 | wheat\|gb164\|BQ244922 | 2512 | 296 | 94.6 | blastp |
| 1006 | LYM91 H1 | rye\|gb164\|BE494176 | 2513 | 297 | 82.5 | blastp |
| 1007 | LYM91 H2 | wheat\|gb164\|BE400659 | 2514 | 297 | 85.4 | blastp |
| 1008 | LYM91 H3 | wheat\|gb164\|CA593112 | 2515 | 297 | 86.27 | tblastn |
| 1009 | LYM93 H1 | wheat\|gb164\|BE401535 | 2516 | 298 | 93.6 | blastp |
| 1010 | LYM93 H2 | wheat\|gb164\|BE418047 | 2517 | 298 | 93.6 | blastp |
| 1011 | LYM93 H3 | wheat\|gb164\|CA624071 | 2518 | 298 | 84.6 | blastp |
| 1012 | LYM93 H4 | wheat\|gb164\|CA678405 | 2519 | 298 | 94.55 | tblastn |
| 1013 | LYM93 H5 | wheat\|gb164\|CJ920171 | 2520 | 298 | 88.7 | blastp |
| 1014 | LYM99 H2 | brachypodium\|09v1\|D V482533 | 2521 | 299 | 86.8 | blastp |
| 1015 | LYM99 H2 | wheat\|gb164\|AL81899 | 2522 | 299 | 94.12 | tblastn |
| 1016 | LYM100 | wheat\|gb164\|BE399036 | 2523 | 301 | 89.5 | blastp |
| 1017 | LYM103 H2 | sorghum\|09v1\|SB03G 004410 | 2524 | 303 | 89.69 | tblastn |
| 1018 | LYM103 H3 | sugarcane\|gb157.3\|CA 078686 | 2525 | 303 | 89.69 | tblastn |
| 1019 | LYM 103 H2 | switchgrass\|gb167\|FL8 77864 | 2526 | 303 | 87 | blastp |
| 1020 | LYM105 H5 | barley\|gb157SOLEXA\|BQ461657 | 2527 | 304 | 90.9 | blastp |
| 1021 | LYM 105 H2 | pseudoroegneria\|gb16 7\|FF366339 | 2528 | 304 | 90.5 | blastp |
| 1022 | LYM 105 H3 | wheat\|gb164\|BE40533 0 | 2529 | 304 | 90 | blastp |
| 1023 | LYM105 H4 | wheal\|gb164\|BE63793 6 | 2530 | 304 | 91.8 | blastp |
| 1024 | LYM 105 H5 | wheat\|gb164\|BQ74387 5 | 2531 | 304 | 89.4 | blastp |
| 1025 | LYM106 H8 | brachypodium\|09v1\|D V476632 | 2532 | 305 | 80.7 | blastp |
| 1026 | LYM 106 H9 | maize\|gb170\|LLDQ24 5927 | 2533 | 305 | 97.9 | blastp |
| 1027 | LYM 106 H3 | pseudoroegneria\|gb 16 7\|FF347837 | 2534 | 305 | 96.5 | blastp |
| 1028 | LYM 106 H4 | rye\|gb164\|BE586535 | 2535 | 305 | 90.3 | blastp |
| 1029 | LYM106 H5 | spruce\|gb162\|DR5435 63 | 2536 | 305 | 84.72 | tblastn |
| 1030 | LYM 106 H6 | wheat\|gb164\|BE44319 5 | 2537 | 305 | 96.5 | blastp |
| 1031 | LYM 106 H7 | wheat\|gb164\|BE44526 4 | 2538 | 305 | 97.9 | blastp |
| 1032 | LYM106 H8 | wheat\|gb164\|BF48509 8 | 2539 | 305 | 97.2 | blastp |
| 1033 | LYM110 H1 | sugarcane\|gb157.3\|CA 204413 | 2540 | 306 | 84.3 | tblastn |
| 1034 | LYM111 H7 | brachypodium\|09v 1\|G T765731 | 2541 | 307 | 80.96 | tblastn |
| 1035 | LYM111 H1 | cenchrus\|gb166\|EB657 665 | 2542 | 307 | 87.6 | blastp |
| 1036 | LYM111 H8 | maize\|gb170\|AI94154 5 | 2543 | 307 | 89.9 | blastp |
| 1037 | LYM111 H9 | rice\|gb170\|OS01G570 66 | 2544 | 307 | 81.5 | blastp |
| 1038 | LYM111 H10 | sorghum\|09v1\|SB03G 036350 | 2545 | 307 | 91.5 | blastp |
| 1039 | LYM111 H11 | sorghum\|09v1\|SB05G 023720 | 2546 | 307 | 93.1 | tblastn |
| 1040 | LYM111 H12 | sugarcane\|gb157.3\|CA 072460 | 2547 | 307 | 89.38 | tblastn |
| 1041 | LYM111 H7 | switchgrass\|gb167\|FL7 11377 | 2548 | 307 | 90.8 | blastp |
| 1042 | LYM119 H1 | sorghum\|09v1\|SB05G 003680 | 2549 | 308 | 93.8 | blastp |
| 1043 | LYM122 H1 | brachypodium\|09v1\|D V469739 | 2550 | 310 | 84.5 | blastp |
| 1044 | LYM122 H1 | pseudoroegneria\|gb16 7\|FF350527 | 2551 | 310 | 82.53 | tblastn |
| 1045 | LYM129 H2 | brachypodium\|09v1\|S RR031795S0005798 | 2552 | 315 | 80.4 | blastp |
| 1046 | LYM129H3 | maize\|gb170\|BQ486269 | 2553 | 315 | 82.8 | blastp |
| 1047 | LYM129 H4 | sorghum\|09v1\|SB03G 044510 | 2554 | 315 | 81.6 | blastp |
| 1048 | LYM129 H2 | switchgrass\|gb167\|FE6 43628 | 2555 | 315 | 80.6 | blastp |
| 1049 | LYM1 30 H1 | leymus\|gb166\|EG3779 85 | 2556 | 316 | 80.2 | blastp |
| 1050 | LYM130 H2 | rice\|gb170\|OS05G043 80 | 2557 | 316 | 99.4 | blastp |
| 1051 | LYM130 H2 | wheat\|gb164\|BE41476 7 | 2558 | 316 | 80.8 | blastp |
| 1052 | LYM131 H1 | aquilegia\|gb157.3\|DR9 17618 | 2559 | 317 | 81.2 | blastp |
| 1053 | LYM131 H13 | barley\|gb 157SOLEXA \|AL450948 | 2560 | 317 | 83 | blastp |
| 1054 | LYM131 H14 | brachypodium\|09v l\|D V479902 | 2561 | 317 | 85.3 | blastp |
| 1055 | LYM131 H15 | maize\|gb 170\|AI86132 7 | 2562 | 317 | 90.8 | blastp |
| 1056 | LYM131 H16 | maize\|gb 170\|AW1298 26 | 2563 | 317 | 82.8 | blastp |
| 1057 | LYM131 H17 | maize\|gb170\|AW4531 72 | 2564 | 317 | 91.7 | blastp |
| 1058 | LYM131 H18 | rice\|gb170\|OS08G127 50 | 2565 | 317 | 85.1 | blastp |
| 1059 | LYM131 H19 | sorghum\|09v1\|SB06G 027970 | 2566 | 317 | 91.5 | blastp |
| 1060 | LYM131 H20 | sorghum\|09v1\|SB07G 006320 | 2567 | 317 | 81 | blastp |
| 1061 | LYM131 H21 | sugarcane\|gb157.3\|CA 068895 | 2568 | 317 | 91.5 | blastp |
| 1062 | LYM131 H11 | switchgrass\|gb167\|FE6 07026 | 2569 | 317 | 81.7 | blastp |
| 1063 | LYM131 H12 | switchgrass\|gb167\|FL7 08944 | 2570 | 317 | 91.49 | tblastn |
| 1064 | LYM131 H13 | wheat\|gb164\|BE41225 7 | 2571 | 317 | 83.7 | blastp |
| 1065 | LYM134 H0 | rice\|gb170\|OS04G569 90 | 2572 | 319 | 98.8 | blastp |
| 1066 | LYM137 H2 | amborella\|gb166\|CK7 58151 | 2573 | 321 | 85.1 | blastp |
| 1067 | LYM137 H3 | antirrhinum\|gb166\|AJ7 88115 | 2574 | 321 | 83.7 | blastp |
| 1068 | LYM 137 H4 | antirrhinum\|gb166\|AJ7 91024 | 2575 | 321 | 83 | blastp |
| 1069 | LYM137 H5 | antirrhinum\|gb166\|AJ7 93144 | 2576 | 321 | 83.01 | tblastn |
| 1070 | LYM 137 H217 | apple\|gb171\|CN44533 3 | 2577 | 321 | 81 | blastp |
| 1071 | LYM 137 H218 | apple\|gb171\|CN48901 9 | 2578 | 321 | 82.4 | blastp |
| 1072 | LYM 137 H219 | apple\|gb171\|CN49188 8 | 2579 | 321 | 81.8 | blastp |
| 1073 | LYM 137 H10 | aquilegia\|gb157.3\|DT7 29599 | 2580 | 321 | 82.7 | blastp |
| 1074 | LYM137 H220 | arabidopsis lyrata\|09vl\|BQ834082 | 2581 | 321 | 81.8 | blastp |
| 1075 | LYM137 H221 | arabidopsis lyrata\|09vl\|BQ834260 | 2582 | 321 | 80.5 | blastp |
| 1076 | L YM137 H222 | arabidopsis lyrata\|09vl\|JGIAL015 196 | 2583 | 321 | 80.5 | blastp |
| 1077 | LYM137 H11 | arabidopsis\|gb165\|AT 3G55280 | 2584 | 321 | 81.2 | blastp |
| 1078 | LYM137 H12 | artemisia\|gb164\|EY03 5831 | 2585 | 321 | 85 | blast p |
| 1079 | LYM137 H13 | avocado\|gb164\|CO995 706 | 2586 | 321 | 85.3 | blastp |
| 1080 | LYM137 H14 | avocado\|gb164\|CV460 574 | 2587 | 321 | 84.6 | blastp |
| 1081 | LYM 137 H15 | b juncea\|gb164\|EVGN0 0185312102498 | 2588 | 321 | 83.8 | blastp |
| 1082 | LYM 137 H16 | b juncea\|gb164\|EVGN0 0317014862029 | 2589 | 321 | 81.2 | blastp |
| 1083 | LYM137 H17 | b juncea\|gb164\|EVGN0 1375409582897 | 2590 | 321 | 81.2 | blastp |
| 1084 | LYM137 H223 | b nigra\|09v1\|GT069407 | 2591 | 321 | 81.2 | blastp |
| 1085 | LYM137 H18 | oleracca\|gb161\|AM39 2244 | 2592 | 321 | 81.2 | blastp |
| 1086 | LYM137 H19 | b oleracca\|gb161\|DY014 383 | 2593 | 321 | 83.8 | blastp |
| 1087 | LYM137 H20 | b oleracea\|gb161\|DY023 491 | 2594 | 321 | 83.8 | blastp |
| 1088 | LYM137 H21 | b oleracea\|gb161\|DY023 494 | 2595 | 321 | 81.2 | blastp |
| 1089 | LYM137 H22 | b oleracea\|gb161\|DY027 443 | 2596 | 321 | 81.2 | blastp |
| 1090 | LYM 137 H23 | b oleracea\|gb161\|EE535 717 | 2597 | 321 | 80.5 | blastp |
| 1091 | LYM137 H24 | b rapa\|gb162\|BG543640 | 2598 | 321 | 83.77 | tblastn |
| 1092 | LYM137 | b rapa\|gb162\|BQ791808 | 2599 | 321 | 80.5 | blastp |
| 1093 | LYM137 H26 | b rapa\|gb162\|CA991997 | 2600 | 321 | 81.2 | blastp |
| 1094 | LYM137 H27 | b rapa\|gb162\|CV432555 | 2601 | 321 | 81.2 | blastp |
| 1095 | LYM137 H28 | b rapa\|gb162\|CV432912 | 2602 | 321 | 81.2 | blastp |
| 1096 | LYM137 H29 | b rapa\|gb162\|CV432918 | 2603 | 321 | 83.8 | blastp |
| 1097 | LYM137 H30 | b rapa\|gb162\|CX267185 | 2604 | 321 | 81.2 | blastp |
| 1098 | LYM137 H37 | b rapa\|pb1621\|CX271342 | 2605 | 321 | 81.2 | blastp |
| 1099 | LYM137 H32 | banana\|gb167\|DN2395 14 | 2606 | 321 | 85.7 | blastp |
| 1100 | LYM137 H33 | banana\|gb167\|ES4316 62 | 2607 | 321 | 86.4 | blastp |
| 1101 | LYM137 H34 | banana\|gb167\|FF5581 02 | 2608 | 321 | 85.7 | blastp |
| 1102 | LYM 137 H35 | banana\|gb167\|FF5587 29 | 2609 | 321 | 86.4 | blastp |
| 1103 | LYM 137 H36 | banana\|gb167\|FF5608 01 | 2610 | 321 | 85.7 | blastp |
| 1104 | LYM137 H37 | banana\|gb167\|FL6573 44 | 2611 | 321 | 85.8 | blastp |
| 1105 | LYM137 H38 | basilicum\|gb157.3\|DY 342616 | 2612 | 321 | 80.5 | blastp |
| 1106 | LYM137 H39 | bean\|gb167\|CA897728 | 2613 | 321 | 85 | blastp |
| 1107 | LYM137 H40 | bean\|gb167\|CA897730 | 2614 | 321 | 83.1 | blastp |
| 1108 | LYM137 H41 | beet\|gb162\|BF011189 | 2615 | 321 | 82.5 | blastp |
| 1109 | LYM137 H42 | beet\|gb162\|BI096284 | 2616 | 321 | 84.4 | blastp |
| 1110 | LYM137 H224 | brachypodium\|09v1\|D V476457 | 2617 | 321 | 96.1 | blastp |
| 1111 | LYM137 H225 | brachypodium\|09v1\|D V489152 | 2618 | 321 | 96.1 | blastp |
| 1112 | LYM 137 H45 | cacao\|gb167\|CA79579 8 | 2619 | 321 | 83.7 | blastp |
| 1113 | LYM137 H46 | cacao\|gb167\|CU47679 8 | 2620 | 321 | 83.8 | blastp |
| 1114 | LYM137 H47 | canola\|gb161\|CD8116 40 | 2621 | 321 | 83.8 | blastp |
| 1115 | LYM137 H48 | canola\|gb161\|CD8122 85 | 2622 | 321 | 81.2 | blastp |
| 1116 | LYM 137 H49 | canola\|gb1611\|CD8123 12 | 2623 | 321 | 81.2 | blastp |
| 1117 | LYM 137 H50 | canola\|gb161\|CD8125 01 | 2624 | 321 | 81.2 | blastp |
| 1118 | LYM137 H51 | canola\|gb161\|CD8154 20 | 2625 | 321 | 81.2 | blastp |
| 1119 | LYM 137 H52 | canola\|gb161\|CD8 169 02 | 2626 | 321 | 81.2 | blastp |
| 1120 | LYM137 H53 | canola\|gb161\|CD8175 91 | 2627 | 321 | 81.2 | blastp |
| 1121 | LYM137 H54 | canola\|gb161\|CD8216 63 | 2628 | 321 | 83.8 | blastp |
| 1122 | LYM 137 H55 | canola\|gb161\|CN7260 29 | 2629 | 321 | 83.8 | blastp |
| 1123 | LYM 137 H56 | canola\|gb161\|CN7310 28 | 2630 | 321 | 81.2 | blastp |
| 1124 | LYM137 H57 | canola\|gb161\|EE47936 8 | 2631 | 321 | 83.8 | blastp |
| 1125 | LYM137 H58 | canola\|gb161\|H07535 | 2632 | 321 | 83.8 | blastp |
| 1126 | LYM137 H226 | cassava\|09v1\|CK6437 71 | 2633 | 321 | 83.1 | blastp |
| 1127 | LYM137 H227 | cassava\|09v1\|CK6474 92 | 2634 | 321 | 83.8 | blastp |
| 1128 | LYM137 H228 | cassava\|09v1\|DV4553 55 | 2635 | 321 | 81.8 | blastp |
| 1129 | LYM137 | castorbean\|09v 1\|EG70 0188 | 2636 | 321 | 85 | blastp |
| 1130 | LYM137 H230 | castorbean\|09v1\|XM0 02531924 | 2637 | 321 | 84.9 | blastp |
| 1131 | LYM137 H63 | catharanthus\|gb166\|E G556131 | 2638 | 321 | 82.6 | blastp |
| 1132 | LYM137 H64 | catharanthus\|gb166\|E G557604 | 2639 | 321 | 82.6 | blastp |
| 1133 | LYM137 H65 | cenchrus\|gb166\|EB652 612 | 2640 | 321 | 93.4 | blastp |
| 1134 | LYM137 H66 | centaurea\|gb166\|EH71 5158 | 2641 | 321 | 83 | blastp |
| 1135 | LYM137 H67 | centaurea\|gb166\|EH73 7696 | 2642 | 321 | 84.3 | blastp |
| 1136 | LYM137 H68 | centaurea\|gb166\|EH74 6709 | 2643 | 321 | 82.5 | blastp |
| 1137 | LYM137 H231 | chestnut\|gb170\|SRR00 6295S0004667 | 2644 | 321 | 83.8 | blastp |
| 1138 | LYM137 H232 | chestnut\|gb170\|SRR00 6295S0010167 | 2645 | 321 | 83.1 | blastp |
| 1139 | LYM137 H233 | chickpea\|09v2\|FE6692 44 | 2646 | 321 | 85.6 | blastp |
| 1140 | LYM137 H234 | chickpea\|09v2\|FE6716 15 | 2647 | 321 | 85.6 | blastp |
| 1141 | LYM137 H235 | cichorium\|gb171\|DT2 10912 | 2648 | 321 | 84.3 | blastp |
| 1142 | LYM 137 H236 | cichorium\|gb171\|EH6 81883 | 2649 | 321 | 84.4 | blastp |
| 1143 | LYM137 H237 | cichorium\|gb171\|EH6 96050 | 2650 | 321 | 83.8 | blastp |
| 1144 | LYM137 H72 | citrus\|gb166\|CB61057 8 | 2651 | 321 | 83.7 | blastp |
| 1145 | LYM137 H73 | citrus\|gb166\|CN18341 5 | 2652 | 321 | 82.9 | blastp |
| 1146 | LYM137 H74 | coffea\|gb157.2\|DV663 668 | 2653 | 321 | 85 | blastp |
| 1147 | LYM 137 H75 | cotton\|gb164\|AI72852 2 | 2654 | 321 | 84.3 | blastp |
| 1148 | LYM137 H76 | cotion\|gb164\|AI72953 1 | 2655 | 321 | 85 | blastp |
| 1149 | LYM137 H77 | cotton\|gb164\|BE05571 9 | 2656 | 321 | 83 | blastp |
| 1150 | LYM137 H78 | cotton\|gb164\|BF26964 1 | 2657 | 321 | 84.3 | btastp |
| 1151 | LYM137 H79 | cotton\|gb164\|BG4414 96 | 2658 | 321 | 81.8 | blastp |
| 1152 | LYM 137 H80 | cowpea\|gb166\|BE3362 50 | 2659 | 321 | 83.9 | blastp |
| 1153 | LYM 137 H81 | cowpea\|gb166\|FF3823 48 | 2660 | 321 | 85.1 | blastp |
| 1154 | LYM 137 H82 | cowpea\|gb166\|FF3912 67 | 2661 | 321 | 86.9 | blastp |
| 1155 | LYM137 H83 | cryptomeria\|gb166\|BP 176442 | 2662 | 321 | 80.5 | blastp |
| 1156 | LYM137 H84 | cryptomeria\|gb166\|B W996322 | 2663 | 321 | 81.2 | blastp |
| 1157 | LYM137 H238 | cucumber\|09v1\|BGI45 4H0165031 | 2664 | 321 | 81.6 | blastp |
| 1158 | LYM137 H239 | cucumber\|09v1\|CK085 893 | 2665 | 321 | 83.7 | blastp |
| 1159 | LYM 137 H240 | cucumber\|09v1lDV63 2825 | 2666 | 321 | 85 | blastp |
| 1160 | LYM 137 H85 | cynara\|gb167\|GE5881 38 | 2667 | 321 | 84.4 | blastp |
| 1161 | LYM 137 H86 | dandelion\|gb161\|DY8 04086 | 2668 | 321 | 85.1 | blastp |
| 1162 | LYM137 H87 | dandelion\|gb161\|DY8 13115 | 2669 | 321 | 83.8 | blastp |
| 1163 | LYM137 H88 | eucalyptus\|gb166\|CT9 80143 | 2670 | 321 | 83.1 | blastp |
| 1164 | LYM 137 H89 | eucalyptus\|gb1661\|CT9 81000 | 2671 | 321 | 81.3 | blastp |
| 1165 | LYM 137 H90 | fescue\|gb161\|DT6864 72 | 2672 | 321 | 80.9 | blastp |
| 1166 | LYM137 H241 | flax\|09v1\|EU829592 | 2673 | 321 | 81 | blastp |
| 1167 | LYM137 H242 | gerbera\|09v1\|AJ75070 7 | 2674 | 321 | 85.6 | blastp |
| 1168 | LYM137 H243 | gerbera\|09v1\|AJ75312 7 | 2675 | 321 | 84.31 | tblastn |
| 1169 | LYM137 H244 | gerbera\|09v1\|AJ75544 0 | 2676 | 321 | 81.7 | blastp |
| 1170 | LYM137 H91 | ginger\|gb164\|DY3520 00 | 2677 | 321 | 85.5 | blastp |
| 1171 | LYM137 H92 | ginger\|gb164\|DY3569 13 | 2678 | 321 | 83.7 | blastp |
| 1172 | LYM 137 H93 | grape\|gb160\|CA81633 5 | 2679 | 321 | 80.6 | blastp |
| 1173 | LYM137 H94 | grape\|gb160\|CB34828 9 | 2680 | 321 | 81.3 | blastp |
| 1174 | LYM137 H95 | grape\|gb160\|CB97964 1 | 2681 | 321 | 84.5 | blastp |
| 1175 | LYM137 H96 | iceplant\|gb164\|CA834 927 | 2682 | 321 | 82.5 | blastp |
| 1176 | LYM137 H97 | ipomoea\|gb157.2\|BU6 90174 | 2683 | 321 | 82.2 | blastp |
| 1177 | LYM137 H98 | ipomoea\|gb157.2\|CJ73 8553 | 2684 | 321 | 82.9 | blastp |
| 1178 | LYM137 H245 | jatropha\|09v 1\|GO2473 33 | 2685 | 321 | 85.6 | blastp |
| 1179 | LYM137 H99 | kiwi\|gb166\|FG413271 | 2686 | 321 | 83.1 | blastp |
| 1180 | LYM137 H100 | kiwi\|gb166\|FG421995 | 2687 | 321 | 84.2 | blastp |
| 1181 | LYM137 H101 | kiwi\|gb 166\|FG429490 | 2688 | 321 | 83.9 | blastp |
| 1182 | LYM137 H102 | kiwi\|gb166\|FG461535 | 2689 | 321 | 84.2 | blastp |
| 1183 | LYM137 H103 | kiwi\|gb166\|FG501757 | 2690 | 321 | 83.6 | blastp |
| 1184 | LYM 137 H104 | lettuce\|gb157.2\|CV700 088 | 2691 | 321 | 83.8 | blastp |
| 1185 | LYM137 H105 | lettuce\|gb157.2\|DW04 6053 | 2692 | 321 | 83 | blastp |
| 1186 | LYM137 H106 | lettuce\|gb157.2\|DW04 9273 | 2693 | 321 | 83.8 | blastp |
| 1187 | LYM137 H107 | lettuce\|gb157.2\|DW07 7894 | 2694 | 321 | 83.8 | blastp |
| 1188 | LYM137 H108 | lettuce\|gb157.2\|DW08 0256 | 2695 | 321 | 83.7 | blastp |
| 1189 | LYM137 H109 | lettuce\|gb157.2\|DW08 0360 | 2696 | 321 | 83.1 | blastp |
| 1190 | LYM137 H110 | lettuce\|gb157.2\|DW11 2293 | 2697 | 321 | 83.8 | blastp |
| 1191 | LYM137 H111 | lettuce\|gb157.2\|DW14 5178 | 2698 | 321 | 83.8 | blastp |
| 1192 | LYM137 H112 | leymus\|gb166\|CD8092 09 | 2699 | 321 | 98.7 | blastp |
| 1193 | LYM137 H246 | liquorice\|gb171\|FS239 986 | 2700 | 321 | 83.9 | blastp |
| 1194 | LYM137 H113 | liriodendron\|gb166\|CK 743367 | 2701 | 321 | 85.3 | blastp |
| 1195 | LYM 137 H247 | lolium\|09v1\|AU25101 2 | 2702 | 321 | 97.4 | blastp |
| 1196 | LYM137 H248 | lotus\|09v1\|LLBG6622 85 | 2703 | 321 | 85 | blastp |
| 1197 | LYM137 H249 | lotus\|09v1\|LLB141868 7 | 2704 | 321 | 83.9 | blastp |
| 1198 | LYM 137 H250 | lotus\|09v1\|LLGO0069 21 | 2705 | 321 | 83.7 | blastp |
| 1199 | LYM137 H115 | lovegrass\|gb167\|EH18 3574 | 2706 | 321 | 92.8 | blastp |
| 1200 | LYM137 H116 | lovegrass\|gb167\|EH18 5967 | 2707 | 321 | 90.1 | blastp |
| 1201 | LYM137 H251 | maize\|gb170\|AA97982 2 | 2708 | 321 | 93.4 | blastp |
| 1202 | LYM137 H252 | maize\|gb 170\|AI61234 9 | 2709 | 321 | 94.7 | blastp |
| 1203 | LYM137 H253 | maize\|gb170\|EY96202 7 | 2710 | 321 | 82.89 | tblastn |
| 1204 | LYM137 H254 | maize\|gb170\|LLBG32 0994 | 2711 | 32 1 | 94.1 | blastp |
| 1205 | LYM 137 H255 | maize\|gb170\|LLDQ24 5209 | 2712 | 321 | 99.3 | blastp |
| 1206 | LYM137 H256 | maize\|gb170\|LLDQ24 5775 | 2713 | 321 | 81.2 | blastp |
| 1207 | LYM 137 H257 | maize\|gb170\|T18742 | 2714 | 321 | 93.4 | blastp |
| 1208 | LYM137 H258 | medicago\|09v 1\|AW20 8139 | 2715 | 321 | 85.1 | blastp |
| 1209 | LYM137 H259 | medicago\|09v 1\|AW28 7975 | 2716 | 321 | 83.6 | blastp |
| 1210 | LYM137 H260 | medicago\|09v1\|LLAJ8 46422 | 2717 | 321 | 82.47 | tblastn |
| 1211 | LYM137 H127 | melon\|gb165\|DV6328 25 | 2718 | 321 | 85 | blastp |
| 1212 | LYM137 H128 | melon\|gb165\|DV6329 19 | 2719 | 321 | 83.7 | blastp |
| 1213 | LYM137 H261 | millet\|09v1\|CD725778 | 2720 | 321 | 94.7 | blastp |
| 1214 | LYM137 H262 | millet\|09v1\|EVO454P M001999 | 2721 | 321 | 91.4 | blastp |
| 1215 | LYM 137 H263 | monkeyflower\|09v1\|D V211090 | 2722 | 321 | 81.3 | blastp |
| 1216 | LYM 137 H264 | monkeyflower\|09v 1\|G 0948368 | 2723 | 321 | 81.3 | blastp |
| 1217 | LYM 137 H129 | nicotiana benthamiana\|gb162\|ES 885186 | 2724 | 321 | 85.7 | blastp |
| 1218 | LYM137 H130 | nuphar\|gb 166\|FD3861 60 | 2725 | 321 | 85.8 | blastp |
| 1219 | LYM 137 H265 | oak\|gb170\|CU656727 | 2726 | 321 | 83.1 | blastp |
| 1220 | LYM137 H266 | oak\|gb170\|SRR006307 S0003551 | 2727 | 321 | 83.8 | blastp |
| 1221 | LYM 137 HI31 | oat\|gb164\|CN814837 | 2728 | 321 | 98 | blastp |
| 1222 | LYM137 H132 | oil palm\|gb166\|CN59945 7 | 2729 | 321 | 87.2 | blastp |
| 1223 | LYM137H133 | oil palm\|gb166\|EL688664 | 2730 | 321 | 84.5 | blastp |
| 1224 | LYM137 H134 | onion\|gb162\|CF45144 2 | 2731 | 321 | 85.2 | blastp |
| 1225 | LYM137 H135 | papaya\|gb165\|EX2389 83 | 2732 | 321 | 83. 1 | blastp |
| 1226 | LYM137 H136 | papaya\|gb 165\|EX2817 27 | 2733 | 321 | 84.3 | blastp |
| 1227 | LYM137 H267 | peanut\|gb171\|CD0380 36 | 2734 | 321 | 84.4 | blastp |
| 1228 | LYM137 H268 | peanut\|gb 171\|CD0380 42 | 2735 | 321 | 83.2 | blastp |
| 1229 | LYM137 H269 | peanut\|gb 171\|EH0429 12 | 2736 | 321 | 85.1 | blastp |
| 1230 | LYM137 H270 | pea\|09v 1\|EX570565 | 2737 | 321 | 83.7 | blastp |
| 1231 | LYM137 H271 | pepper\|gb171\|BM0631 92 | 2738 | 321 | 83 | blastp |
| 1232 | LYM137 H272 | pepper\|gb 171\|CA5184 36 | 2739 | 321 | 81.8 | blastp |
| 1233 | LYM137 H273 | petunia\|gb171\|CV2988 26 | 2740 | 321 | 83 | blastp |
| 1234 | LYM137 H274 | petunia\|gb171\|CV2990 96 | 2741 | 321 | 83.7 | blastp |
| 1235 | LYM137 H275 | petunia\|gb171\|FN0076 57 | 2742 | 321 | 83.1 | blastp |
| 1236 | LYM 137 H276 | poplar\|gb170\|AI16182 2 | 2743 | 321 | 81.8 | blastp |
| 1237 | LYM137 H277 | poplar\|gb170\|AI16481 2 | 2744 | 321 | 80.4 | blastp |
| 1238 | LYM137 H278 | poplar\|gb170\|CN5176 15 | 2745 | 321 | 81.58 | tblastn |
| 1239 | LYM137 H150 | poppy\|gb 166\|FE96448 2 | 2746 | 321 | 82.6 | blastp |
| 1240 | LYM137 H151 | poppy\|gb166\|FE96848 9 | 2747 | 321 | 83.9 | blastp |
| 1241 | LYM 137 H152 | potato\|gb157.2\|BE923 747 | 2748 | 321 | 83.7 | blastp |
| 1242 | LYM137 H153 | potato\|gb157.2\|BF459 639 | 2749 | 321 | 83.7 | blastp |
| 1243 | LYM137 H155 | potato\|gb157.2\|BI4070 78 | 2750 | 321 | 82.5 | blastp |
| 1244 | L YM137 H156 | potato\|gb 157.2\|BQ046 658 | 2751 | 321 | 82.5 | blastp |
| 1245 | LYM137 H157 | prunus\|gb167\|BU0480 09 | 2752 | 321 | 83.8 | blastp |
| 1246 | LYM137 H158 | prunus\|gb167\|BU5726 74 | 2753 | 321 | 83.8 | blastp |
| 1247 | LYM137 H159 | pseudoroegneria\|gb 16 7\|FF344271 | 2754 | 321 | 98.7 | blastp |
| 1248 | LYM 137 H160 | radish\|gb 164\|EV52441 2 | 2755 | 321 | 82.5 | blastp |
| 1249 | LYM137 H161 | radish\|gb164\|EV52673 2 | 2756 | 321 | 81.2 | blastp |
| 1250 | LYM137 H162 | radish\|gb164\|EV52780 6 | 2757 | 321 | 81.2 | blastp |
| 1251 | LYM 137 H163 | radish\|gb164\|EV53694 9 | 2758 | 321 | 81.2 | blastp |
| 1252 | LYM 137 H164 | radish\|gb164\|EV53908 7 | 2759 | 321 | 83.1 | blastp |
| 1253 | LYM137 H165 | radish\|gb164\|EV54524 8 | 2760 | 321 | 81.2 | blastp |
| 1254 | LYM 137 H166 | radish\|gb164\|EV57049 2 | 2761 | 321 | 81.2 | blastp |
| 1255 | LYM137 H167 | radish\|gb164\|EW7244 65 | 2762 | 321 | 81.2 | blastp |
| 1256 | LYM137 H168 | radish\|gb164\|EW7247 37 | 2763 | 321 | 83.1 | blastp |
| 1257 | LYM137 H169 | radish\|gb164\|EW7319 70 | 2764 | 321 | 81.2 | blastp |
| 1258 | LYM137 H170 | radish\|gb164\|EW7327 28 | 2765 | 321 | 81.2 | blastp |
| 1259 | LYM 137 H171 | radish\|gb164\|EX75564 1 | 2766 | 321 | 80.4 | blastp |
| 1260 | LYM137 H172 | radish\|gb164\|EX75678 4 | 2767 | 321 | 83.8 | blastp |
| 1261 | LYM 137 H173 | radish\|gb164\|EX75782 4 | 2768 | 321 | 81.2 | blastp |
| 1262 | LYM137 H279 | rice\|gb170\|OS01G246 90 | 2769 | 321 | 92.2 | blastp |
| 1263 | LYM 137 H280 | rice\|gb 170\|OS04G422 70 | 2770 | 321 | 94.1 | blastp |
| 1264 | LYM 137 H176 | ryc\|gb164\|BE493987 | 2771 | 321 | 100 | blastp |
| 1265 | LYM137 H177 | safflower\|gb162\|EL37 8228 | 2772 | 321 | 82.4 | blastp |
| 1266 | LYM 137 H178 | safflower\|gb162\|EL39 9454 | 2773 | 321 | 83.8 | blastp |
| 1267 | LYM137 H179 | safflower\|gb162\|EL41 1711 | 2774 | 321 | 81.7 | blastp |
| 1268 | LYM137 H281 | senecio\|gb170\|DY666 439 | 2775 | 321 | 83.01 | tblastn |
| 1269 | LYM137 H282 | solanum phureja\|09v1\|SPHAA8 24956 | 2776 | 321 | 82.5 | blastp |
| 1270 | LYM137 H283 | solanum phureja\|09v1\|SPHBQ1 15070 | 2777 | 321 | 82.69 | tblastn |
| 1271 | LYM137 H284 | solanum phureja\|09v 1\|SPHTO M289A | 2778 | 321 | 83.7 | blastp |
| 1272 | LYM 137 H285 | sorghun1\|09v1\|SB06G 021660 | 2779 | 321 | 94.7 | blastp |
| 1273 | LYM137 H286 | sorghum\|09v1\|SB10G 005240 | 2780 | 321 | 94.1 | blastp |
| 1274 | LYM137 H182 | soybean\|gb168\|AL365 737 | 2781 | 321 | 85.6 | blastp |
| 1275 | LYM137 H183 | soybean\|gb168\|AW20 8139 | 2782 | 321 | 84.3 | blastp |
| 1276 | LYM137 H184 | soybean\|gb168\|AW28 7975 | 2783 | 321 | 85.6 | blastp |
| 1277 | LYM137 H185 | soybean\|gb168\|BE336 250 | 2784 | 321 | 81.9 | blastp |
| 1278 | LYM 137 H186 | soybean\|gb168\|BE336 251 | 2785 | 321 | 84.5 | blastp |
| 1279 | LYM137 H187 | soybean\|gb168\|BI9675 38 | 2786 | 321 | 85 | blastp |
| 1280 | LYM137 H188 | spurge\|gb161\|BI99357 4 | 2787 | 321 | 83.8 | blastp |
| 1281 | LYM137 H189 | strawberry\|gb164\|CO3 78565 | 2788 | 321 | 83.7 | blastp |
| 1282 | LYM137 H190 | strawberry\|gb164\|EX6 85316 | 2789 | 321 | 82.5 | blastp |
| 1283 | LYM137 H287 | sugarcane\|gb157.3\|AI2 16927 | 2790 | 321 | 94.7 | blastp |
| 1284 | LYM137 H288 | sugarcane\|gb157.3\|BQ 535570 | 2791 | 321 | 92.8 | blastp |
| 1285 | LYM137 H289 | sugarcane\|gb157.3\|BQ 535956 | 2792 | 321 | 94.1 | blastp |
| 1286 | LYM137 H290 | sugarcane\|gb157.3\|BQ 537453 | 2793 | 321 | 93.4 | blastp |
| 1287 | LYM137 H291 | sugarcane\|gb157.3\|CA 106878 | 2794 | 321 | 94.7 | blastp |
| 1288 | LYM137 H292 | sugarcane\|gb157.3\|CA 111839 | 2795 | 321 | 93.4 | blastp |
| 1289 | LYM 137 H293 | sugarcane\|gb157.3\|CA 113465 | 2796 | 321 | 92.11 | tblastn |
| 1290 | LYM137 H198 | sunflower\|gb162\|CD8 46067 | 2797 | 321 | 84.3 | blastp |
| 1291 | LYM 137 H199 | sunflower\|gb162\|CD8 48213 | 2798 | 321 | 84.3 | blastp |
| 1292 | LYM137 H200 | sunflower\|gb162\|CD8 51130 | 2799 | 321 | 85.6 | blastp |
| 1293 | LYM 137 H201 | sunflower\|gb162\|CD8 53875 | 2800 | 321 | 83.8 | blastp |
| 1294 | LYM137 H202 | switchgrass\|gb167\|DN 140751 | 2801 | 321 | 92.8 | blastp |
| 1295 | LYM137 H203 | switchgrass\|gb167\|DN 143966 | 2802 | 321 | 90.8 | blastp |
| 1296 | LYM137 H204 | switchgrass\|gb167\|FE6 03312 | 2803 | 321 | 91.4 | blastp |
| 1297 | LYM137 H205 | switchgrass\|gb1 67\|FE6 45253 | 2804 | 321 | 92.8 | blastp |
| 1298 | LYM137 H294 | tca\|gb171\|GE652357 | 2805 | 321 | 84.52 | tblastn |
| 1299 | LYM137 H295 | tea\|gb171\|GH613259 | 2806 | 321 | 81.2 | blastp |
| 1300 | LYM137 H206 | thellungiella\|gb167\|D N773656 | 2807 | 321 | 81.2 | blastp |
| 1301 | LYM137 H207 | tobacco\|gb162\|DV157 653 | 2808 | 321 | 82.5 | blastp |
| 1302 | LYM 137 H208 | tobacco\|gb162\|EB444 171 | 2809 | 321 | 85.1 | blastp |
| 1303 | LYM137 H209 | tobacco\|gb162\|EB445 443 | 2810 | 321 | 85.6 | blastp |
| 1304 | LYM137 H210 | tobacco\|gb162\|EB679 214 | 2811 | 321 | 83.8 | blastp |
| 1305 | LYM137 H211 | tobacco\|gb162\|TOBRP L25A | 2812 | 321 | 85.7 | blastp |
| 1306 | LYM 137 H296 | tomato\|09v1\|AA82495 6 | 2813 | 321 | 81.8 | blastp |
| 1307 | LYM137 H297 | tomato\|09v1\|BQ11507 0 | 2814 | 321 | 83.8 | blastp |
| 1308 | LYM 137 H298 | tomato\|09v1\|TOM289 A | 2815 | 321 | 83.7 | blastp |
| 1309 | LYM 137 H214 | wheat\|gb1604\|BE40186 0 | 2816 | 321 | 99.3 | blastp |
| 1310 | LYM137 H215 | wheat\|gb164\|BE40351 6 | 2817 | 321 | 99.3 | blastp |
| 1311 | LYM137 H216 | wheat\|gb164\|BE40448 8 | 2818 | 321 | 99.3 | blastp |
| 1312 | LYM137 H217 | wheat\|gb164\|CA61289 8 | 2819 | 321 | 82.89 | tblastn |
| 1313 | LYM137 H299 | zinnia\|gb171\|AU3044 73 | 2820 | 321 | 83 | blastp |
| 1314 | LYM140 H18 | apple\|gb171\|CN87400 7 | 2821 | 322 | 80.1 | blastp |
| 1315 | LYM140 H1 | banana\|gb167\|ES4337 90 | 2822 | 322 | 80 | tblastn |
| 1316 | LYM140 H19 | brachypodium\|09v1\|D V472528 | 2823 | 322 | 90.3 | blastp |
| 1317 | LYM 140 H20 | cassava\|09v1\|BM2597 38 | 2824 | 322 | 80.3 | blastp |
| 1318 | LYM 140 H21 | cassava\|09v1\|CK6408 86 | 2825 | 322 | 80.3 | blastp |
| 1319 | LYM140 H22 | castorbean\|09v1\|EG65 6528 | 2826 | 322 | 80.4 | blastp |
| 1320 | LYM140 H23 | chestnut\|gb170\|SRR00 6295S0060343 | 2827 | 322 | 80 | blastp |
| 1321 | LYM140 H24 | chestnut\|gb170\|SRR00 6296S0039724 | 2828 | 322 | 80.49 | tblastn |
| 1322 | LYM 140 H4 | citrus\|gb166\|CB29047 9 | 2829 | 322 | 80.3 | blastp |
| 1323 | LYM140 | cotton\|gb164\|BF27194 2 | 2830 | 322 | 81.4 | blastp |
| 1324 | LYM140 H6 | cotton\|gb164\|CA9926 95 | 2831 | 322 | 80.1 | blastp |
| 1325 | LYM140 H7 | cowpea\|gb166\|FC4597 91 | 2832 | 322 | 80.48 | tblastn |
| 1326 | LYM140 H25 | maize\|sb170\|AW3312 87 | 2833 | 322 | 89.2 | blastp |
| 1327 | LYM140 H9 | palm\|gb166\|ES414711 | 2834 | 322 | 80.8 | blastp |
| 1328 | LYM140 H10 | papaya\|gb165\|EX2277 99 | 2835 | 322 | 80 | blastp |
| 1329 | LYM 140 H11 | radish\|gb164\|EV52827 2 | 2836 | 322 | 80.3 | blastp |
| 1330 | LYM140 H26 | rice\|gb170\|OS04G532 10 | 2837 | 322 | 88.6 | blastp |
| 1331 | LYM140 H27 | sorghum\|09v1\|SB06G 028990 | 2838 | 322 | 88.9 | blastp |
| 1332 | LYM140 H13 | soybean\|gb168\|AW12 6193 | 2839 | 322 | 80 | blastp |
| 1333 | LYM 140 H28 | sugarcane\|gb157.3\|CA 071893 | 2840 | 322 | 88.1 | blastp |
| 1334 | LYM140 H15 | sunflower\|gb162\|BU6 71805 | 2841 | 322 | 80.1 | blastp |
| 1335 | LYM140 H16 | switchgrass\|gb167\|FE6 24047 | 2842 | 322 | 90.5 | blastp |
| 1336 | LYM140 H17 | wheat\|gb164\|BE41572 6 | 2843 | 322 | 98.1 | blastp |
| 1337 | LYM140 H18 | wheat\|gb164\|3F20061 3 | 2844 | 322 | 86.7 | blastp |
| 1338 | LYM141 H0 | rice\|gb170\|OS 12G029 10 | 2845 | 323 | 86.6 | blastp |
| 1339 | LYM142H7 | barley\|gb157SOLEXA\|BQ761869 | 2846 | 324 | 86.6 | blastp |
| 1340 | LYM142 H8 | brachypodium\|09v1\|D V478753 | 2847 | 324 | 86.8 | blastp |
| 1341 | LYM 142 H3 | leymus\|gb166\|EG4015 96 | 2848 | 324 | 97.7 | blastp |
| 1342 | LYM142 H4 | pseudoroegneria\|gb16 7\|FF362922 | 2849 | 324 | 97.7 | blastp |
| 1343 | LYM142 H9 | rice\|gb170\|OS02G335 50 | 2850 | 324 | 83.9 | blastp |
| 1344 | LYM142 H6 | wheat\|gb164\|BE40484 3 | 2851 | 324 | 94.4 | blastp |
| 1345 | LYM142H7 | wheat\|gb164\|CA63146 7 | 2852 | 324 | 83.9 | blastp |
| 1346 | LYM144 H0 | brachypodium\|09v1\|G T775853 | 2853 | 326 | 80.6 | blastp |
| 1347 | LYM148 H10 | brachypodium\|09v 1\|D V478384 | 2854 | 328 | 91.1 | blastp |
| 1348 | LYM148 H2 | leymus\|gb166\|EG3989 61 | 2855 | 328 | 92.73 | tblastn |
| 1349 | LYM148 H11 | maize\|gb170\|AW0600 86 | 2856 | 328 | 84.5 | blastp |
| 1350 | LYM 148 H12 | millet\|09v1\|EVO454P M023692 | 2857 | 328 | 82.9 | blastp |
| 1351 | LYM148 H13 | rice\|gb 170\|OS06G454 40 | 2858 | 328 | 82.4 | blastp |
| 1352 | LYM148 H14 | sorghum\|09v1\|SB10G 026570 | 2859 | 328 | 83.3 | blastp |
| 1353 | LYM148 H6 | switchgrass\|gb167\|FE6 04043 | 2860 | 328 | 82.5 | blastp |
| 1354 | LYM148 H7 | switchgrass\|gb167\|FE6 41627 | 2861 | 328 | 81.1 | blastp |
| 1355 | LYM148 H8 | wheat\|gb164\|BE44289 | 2862 | 328 | 96.3 | blastp |
| 1356 | LYM148 H9 | wheat\|gb164\|BQ29525 | 2863 | 328 | 97 | blastp |
| 1357 | LYM148 H10 | wheat\|gb164\|BQ78864 1 | 2864 | 328 | 97 | blastp |
| 1358 | LYM149 H5 | brachypodium\|09v1\|D V488199 | 2865 | 329 | 83.2 | blastp |
| 1359 | LYM149 H2 | pseudoroegneria\|gb 16 7\|FF346387 | 2866 | 329 | 87 | blastp |
| 1360 | LYM149 H3 | wheat\|gb164\|B E42905 2 | 2867 | 329 | 87.2 | blastp |
| 1361 | LYM149 H4 | wheat\|gb164\|BQ620138 | 2868 | 329 | 87.5 | blastp |
| 1362 | LYM149 H5 | wheat\|gb164\|CA64142 4 | 2869 | 329 | 89.69 | tblastn |
| 1363 | LYM152 H14 | arabidopsis lyrata\|09v1\|BQ834051 | 2870 | 330 | 86.7 | blastp |
| 1364 | LYM 152 H15 | arabidopsis lyrata\|09v1\|JGIAL030 307 | 2871 | 330 | 96.7 | blastp |
| 1365 | LYM 152 H1 | arabidopsis\|gb165\|AT 4G25890 | 2872 | 330 | 82.5 | tblastn |
| 1366 | LYM152 H2 | b oleracea\|gb161\|DY027 305 | 2873 | 330 | 95 | blastp |
| 1367 | LYM152 H3 | b oleracea\|gb161\|DY028 937 | 2874 | 330 | 93.4 | blastp |
| 1368 | LYM152 H4 | b rapa\|gb162\|BG544013 | 2875 | 330 | 94.17 | tblastn |
| 1369 | LYM152 H5 | b rapa\|gb162\|L35776 | 2876 | 330 | 92.6 | blastp |
| 1370 | LYM152 H6 | b rapa\|gb162\|L35823 | 2877 | 330 | 95 | blastp |
| 1371 | LYM152 H7 | canola\|gb161\|CD8120 96 | 2878 | 330 | 94.2 | blastp |
| 1372 | LYM152 H8 | canola\|gb161\|CD8125 52 | 2879 | 330 | 95 | blastp |
| 1373 | LYM152 H9 | canola\|gb161\|CD8170 37 | 2880 | 330 | 95 | blastp |
| 1374 | LYM152 H10 | canola\|gb161\|CD8303 47 | 2881 | 330 | 92.6 | blastp |
| 1375 | LYM152 H12 | radish\|gb164\|EV54823 5 | 2882 | 330 | 92.5 | blastp |
| 1376 | LYM 152 H13 | radish\|gb164\|EW7181 96 | 2883 | 330 | 92.5 | blastp |
| 1377 | LYM152 H14 | thellungiella\|gb167\|B M985697 | 2884 | 330 | 91.7 | blastp |
| 1378 | LYM153 H6 | barley\|gb157SOLEXA\|BF625242 | 2885 | 331 | 81.5 | blastp |
| 1379 | LYM153 H7 | brachypodium\|09v1\|S RR031796S0027091 | 2886 | 331 | 81.5 | blastp |
| 1380 | LYM153 H2 | cenchrus\|gb166\|EB654 758 | 2887 | 331 | 80 | blastp |
| 1381 | LYM153 H8 | millet\|09v1\|EVO454P M017552 | 2888 | 331 | 83.1 | blastp |
| 1382 | LYM153 H9 | sorghum\|09v1\|SB10G 003440 | 2889 | 331 | 81.5 | blastp |
| 1383 | LYM153 H4 | switchgrass\|gb167\|FE6 34744 | 2890 | 331 | 83.1 | blastp |
| 1384 | LYM153 H5 | wheat\|gb164\|CD49095 1 | 2891 | 331 | 94.03 | tblastn |
| 1385 | LYM153 H6 | wheat\|gb164\|CK21566 0 | 2892 | 331 | 80.3 | tblastn |
| 1386 | LYM156 H6 | barley\|gb157SOLEXA \|AL506124 | 2893 | 332 | 96.1 | blastp |
| 1387 | LYM 156 H7 | barley\|gb 157SOLEXA \|BE 195142 | 2894 | 332 | 93.1 | blastp |
| 1388 | LYM156 H3 | pseudoroegneria\|gb16 7\|FF346665 | 2895 | 332 | 92.8 | blastp |
| 1389 | LYM 156 H4 | pseudoroegneria\|gb16 7\|FF354463 | 2896 | 332 | 86.3 | blastp |
| 1390 | LYM156 H8 | rice\|gb170\|OS07G468 30 | 2897 | 332 | 80 | blastp |
| 1391 | LYM156H6 | wheat\|gb164\|BE637888 | 2898 | 332 | 91.8 | blastp |
| 1392 | LYM 157 H1 | barley\|gb157SOLEXA \|BG299283 | 2899 | 333 | 94.9 | blastp |
| 1393 | LYM159 H1 | wheat\|gb164\|CA598148 | 2900 | 334 | 81.01 | tblastn |
| 1394 | LYM159 H2 | wheat\|gb164\|CD86603 7 | 2901 | 334 | 87.7 | blastp |
| 1395 | LYM159 H3 | wheat\|gb164\|CD867356 | 2902 | 334 | 86.4 | blastp |
| 1396 | LYM160 H1 | wheat\|gb164\|BE39999 7 | 2903 | 335 | 87.6 | tblastn |
| 1397 | LYM160 H2 | wheat\|gb164\|BE50020 0 | 2904 | 335 | 80 | blastp |
| 1398 | LYM161 H0 | brachypodium\|09v 1\|D V471902 | 2905 | 336 | 81.9 | blastp |
| 1398 | LYM161 H0 | brachypodium\|09v1\|D V471902 | 2905 | 444 | 81.02 | tblastn |
| 1399 | LYM162 H5 | maize\|gb170\|AW3311 05 | 2906 | 337 | 84.8 | blastp |
| 1400 | LYM162 H6 | millet\|09v1\|EVO454P M02675I | 2907 | 337 | 85.7 | blastp |
| 1401 | LYM162 H7 | sorghum\|09v1\|SB03G 043995 | 2908 | 337 | 87.5 | blastp |
| 1402 | LYM162 H8 | sugarcane\|gb157.3\|BQ 536349 | 2909 | 337 | 87.5 | blastp |
| 1403 | LYM162 H4 | switchgrass\|gb167\|FL7 38992 | 2910 | 337 | 83.9 | blastp |
| 1404 | LYM162 H5 | switchgrass\|gb167\|FL8 29126 | 2911 | 337 | 85.7 | blastp |
| 1405 | LYM165 H5 | maize\|gb170\|LLCO45 2769 | 2912 | 339 | 99.2 | blastp |
| 1406 | LYM165 H6 | sorghum\|09v1\|SB03G 030690 | 2913 | 339 | 89.2 | blastp |
| 1407 | LYM165 H7 | sugarcane\|gb157.3\|BQ 529806 | 2914 | 339 | 81.1 | blastp |
| 1408 | LYM165 H8 | sugarcane\|gb157.3\|CA 084294 | 2915 | 339 | 90.4 | blastp |
| 1409 | LYM165 H4 | switchgrass\|gb167\|DN 143471 | 2916 | 339 | 85.9 | blastp |
| 1410 | LYM165 H5 | switchgrass\|gb167\|DN 144101 | 2917 | 339 | 85.7 | blastp |
| 1411 | LYM 166 H1 | brachypodium\|09v1\|D V486893 | 2918 | 340 | 85.4 | tblastn |
| 1411 | LYM 166 H1 | brachypodium\|09v 1\|D V486893 | 2918 | 445 | 85.36 | tblastn |
| 1412 | LYM170 H7 | barley\|gb157SOLEXA\|AV915706 | 2919 | 341 | 91.6 | blastp |
| 1413 | LYM170 H8 | brachypodium\|09v1 \|S RR031797S0049196 | 2920 | 341 | 94.8 | blastp |
| 1414 | LYM 170 H9 | maize\|gb170\|AI665902 | 2921 | 341 | 87.3 | blastp |
| 1415 | LYM 170 H10 | maize\|gb 170\|BE05062 8 | 2922 | 341 | 86.4 | blastp |
| 1416 | LYM170 H11 | sorghum\|09v 1 \|SB03G 036440 | 2923 | 341 | 87.3 | blastp |
| 1417 | LYM 170 H12 | sugarcane\|gb157.3\|CA 067017 | 2924 | 341 | 88.3 | blastp |
| 1418 | LYM170 H6 | switchgrass\|gb167\|DN 142710 | 2925 | 341 | 87.3 | blastp |
| 1419 | LYM170 H7 | wheat\|gb164\|BE41537 1 | 2926 | 341 | 93.5 | blastp |
| 1420 | LYM172 H11 | brachypodium\|09v1\|D V489358 | 2927 | 342 | 87.6 | blastp |
| 1421 | LYM172 H12 | maize\|gb170\|AA97977 0 | 2928 | 342 | 81.7 | blastp |
| 1422 | LYM172 H13 | maize\|gb170\|AI71196 6 | 2929 | 342 | 81.2 | blastp |
| 1423 | LYM172 H14 | maize\|gb 170\|AI94752 1 | 2930 | 342 | 80.71 | tblastn |
| 1424 | LYM 172 H15 | maize\|gb170\|AW1201 45 | 2931 | 342 | 82.9 | blastp |
| 1425 | LYM172 H16 | millet\|09v 1\|EVO454P M002481 | 2932 | 342 | 85.6 | tblastn |
| 1426 | LYM 172 H17 | rice\|gb170\|OS02G083 64 | 2933 | 342 | 81.8 | blastp |
| 1427 | LYM 172 H18 | sorghum\|09v1\|SB04G 005430 | 2934 | 342 | 80.43 | tblastn |
| 1428 | LYM172 H19 | sorghum\|09v1\|SB10G 025800 | 2935 | 342 | 83.9 | blastp |
| 1429 | LYM 172 H20 | sugarcane\|gb157.3\|CA 071007 | 2936 | 342 | 80.1 | blastp |
| 1430 | LYM172 H9 | switchgrass\|gb167\|FL6 92588 | 2937 | 342 | 80.7 | blastp |
| 1431 | LYM 172 H10 | wheat\|gb164\|BE40076 1 | 2938 | 342 | 81.25 | tblastn |
| 1432 | LYM 172 H11 | wheat\|gb164\|BE49886 8 | 2939 | 342 | 87.77 | tblastn |
| 1433 | LYM213 H1 | switchgrass\|gb167\|FE6 20008 | 2940 | 343 | 80.4 | blastp |
| 1433 | LYM213 H1 | switchgrass\|gb167\|FE6 20008 | 2940 | 386 | 88.7 | blastp |
| 1434 | LYM174 H3 | maize\|gb170\|AW1449 17 | 2941 | 344 | 89.4 | blastp |
| 1435 | LYM174 H4 | maize\|gb170\|AW2673 79 | 2942 | 344 | 86.6 | blastp |
| 1436 | LYM174 H5 | sugarcane\|gb157.3\|CA 089309 | 2943 | 344 | 92.31 | tblastn |
| 1437 | LYM174 H3 | switchgrass\|gb167\|DN 150662 | 2944 | 344 | 80.9 | blastp |
| 1438 | LYM 175 H0 | maize\|gb170\|AW4984 64 | 2945 | 345 | 81.2 | blastp |
| 1439 | LYM175 H1 | rice\|gb170\|OS01G690 90 | 2946 | 345 | 95 | blastp |
| 1439 | LYM175 H1 | rice\|gb170\|OS01G690 90 | 2946 | 446 | 100 | blastp |
| 1440 | LYM215 H2 | sorghum\|09v1\|SB03G 043980 | 2947 | 345 | 81.2 | blastp |
| 1440 | LYM215 H2 | sorghum\|09v1\|SB03G 043980 | 2947 | 387 | 94.6 | blastp |
| 1441 | LYM 176 H2 | maize\|gb170\|CA45271 3 | 2948 | 346 | 82.2 | blastp |
| 1442 | LYM 176 H3 | sorghum\|09v1\|SB03G 036470 | 2949 | 346 | 83 | blastp |
| 1443 | LYM176 H2 | switchgrass\|gb167\|FE6 06366 | 2950 | 346 | 82 | blastp |
| 1444 | LYM178 H9 | brachypodium\|09v1\|D V472161 | 2951 | 347 | 84.9 | blastp |
| 1445 | LYM178 H10 | brachypodium\|09v1\|S RR031797S0177787 | 2952 | 347 | 84.6 | blastp |
| 1446 | LYM178 H1 | fescue\|gb161\|DT6744 27 | 2953 | 347 | 89.4 | blastp |
| 1447 | LYM 178 H2 | leymus\|gb166\|EG3945 91 | 2954 | 347 | 84.08 | tblastn |
| 1448 | LYM178 H11 | maize\|gb 170\|W21746 | 2955 | 347 | 83.4 | blastp |
| 1449 | LYM 178 H12 | millet\|09v1\|EVO454P M001531 | 2956 | 347 | 83.1 | blastp |
| 1450 | LYM178 H3 | pseudoroegneria\|gb16 7\|FF358503 | 2957 | 347 | 93.6 | blastp |
| 1451 | LYM178 H13 | sorghum\|09v1\|SB03G 008890 | 2958 | 347 | 83.1 | blastp |
| 1452 | LYM178 H14 | sorghum\|09v1\|SB07G 001060 | 2959 | 347 | 81.9 | blastp |
| 1453 | LYM178 H15 | sugarcane\|gb157.3\|CA 066169 | 2960 | 347 | 82.4 | blastp |
| 1454 | LYM 178 H16 | sugarcane\|gb157.3\|CA 079726 | 2961 | 347 | 83.1 | blastp |
| 1455 | LYM178 H8 | switchgrass\|gb167\|FE6 36508 | 2962 | 347 | 82.4 | blastp |
| 1456 | LYM 178 H9 | wheat\|gb 164\|BQ62075 2 | 2963 | 347 | 94.7 | blastp |
| 1457 | LYM179 H0 | sorghum\|09v1\|SB08G 006470 | 2964 | 348 | 86.5 | blastp |
| 1458 | LYM107 H2 | brachypodium\|09v1\|G T808738 | 2965 | 349 | 86.9 | blastp |
| 1459 | LYM107 H3 | maize\|gb 170\|CF07558 7 | 2966 | 349 | 95.2 | blastp |
| 1460 | LYM107 H4 | rice\|gb 170\|OS05G266 60 | 2967 | 349 | 86.4 | blastp |
| 1461 | LYM107 H5 | sorghum\|09v1\|SB03G 036480 | 2968 | 349 | 95 | blastp |
| 1462 | LYM109 H1 | maize\|gb 170\|BG51716 3 | 2969 | 350 | 82.62 | tblastn |
| 1462 | LYM109 H1 | maize\|gb 170\|BG51716 3 | 2969 | 447 | 82.6 | tblastn |
| 1463 | LYM109 H2 | sorghum\|09v1\|SB05G 003660 | 2970 | 350 | 88.75 | tblastn |
| 1463 | LYM 109 H2 | sorghum\|09v1\|SB05G 003660 | 2970 | 447 | 88.87 | tblastn |
| 1464 | LYM112 H1 | maize\|gb170\|CF037322 | 2971 | 351 | 95.4 | blastp |
| 1464 | LYM112 H1 | maize\|gb 170\|CF03732 2 | 2971 | 448 | 93.96 | tblastn |
| 1465 | LYM112 H2 | sorghum\|09v1\|SB02G 039985 | 2972 | 351 | 92.43 | tblastn |
| 1465 | LYM112 H2 | sorghum\|09v1\|SB02G 039985 | 2972 | 448 | 84.8 | blastp |
| 1466 | LYM115 H0 | sorghum\|09v1\|SB01G 043900 | 2973 | 353 | 90.19 | tblastn |
| 1467 | LYM116 H3 | sorghum\|09v1\|SB06G 031340 | 2974 | 354 | 88.2 | blastp |
| 1468 | LYM116 H2 | switchgrass\|gb1 67\|FE6 53493 | 2975 | 354 | 80.88 | tblastn |
| 1469 | LYM116 H3 | switchgrass\|gb167\|FL7 90906 | 2976 | 354 | 80.88 | tblastn |
| 1470 | LYM117 H2 | maize\|gb170\|GFXAF2 43041X1 | 2977 | 355 | 84.4 | blastp |
| 1470 | LYM117 H2 | maize\|gb170\|GFXAF2 43041X1 | 2977 | 449 | 84.5 | blastp |
| 1471 | LYM117 H3 | sorghum\|09v1\|SB07G 027220 | 2978 | 355 | 82.3 | blastp |
| 1471 | LYM117 H3 | sorghum\|09v1\|SB07G 027220 | 2978 | 449 | 82.3 | blastp |
| 1472 | LYM121 H1 | rice\|gb170\|OS12G026 20 | 2979 | 357 | 90.6 | blastp |
| 1473 | LYM123 H4 | barley\|gb 157SOLEXA \|AF268595 | 2980 | 358 | 85.5 | blastp |
| 1474 | LYM123 H5 | brachypodium\|09v1\|G T761722 | 2981 | 358 | 84 | blastp |
| 1475 | LYM123 H6 | maize\|gb 170\|AI79555 8 | 2982 | 358 | 85.2 | blastp |
| 1476 | LYM123 H7 | sorghum\|09v1\|SB06G 031680 | 2983 | 358 | 86.3 | blastp |
| 1477 | LYM123 H4 | wheat\|gb164\|BE40187 1 | 2984 | 358 | 85.6 | blastp |
| 1478 | LYM 135 H1 | brachypodium\|09v1\|D V480514 | 2985 | 359 | 93.1 | blastp |
| 1479 | LYM135 H2 | maize\|gb170\|CB88566 7 | 2986 | 359 | 80.2 | blastp |
| 1480 | LYM135 H3 | sorghum\|09v1\|SB10G 007850 | 2987 | 359 | 84.1 | blastp |
| 1481 | LYM138 H2 | brachypodium\|09v1\|G T810825 | 2988 | 360 | 84 | blastp |
| 1482 | LYM138 H3 | maize\|gb170\|AI612333 | 2989 | 360 | 86.17 | tblastn |
| 1483 | LYM138 H4 | sorghum\|09v1\|SB06G 031570 | 2990 | 360 | 86.33 | tblastn |
| 1484 | LYM146 H2 | brachypodium\|09v1\|S RR031798S0143459 | 2991 | 361 | 84.3 | blastp |
| 1485 | LYM146 H3 | rice\|gb170\|OS03G217 30 | 2992 | 361 | 86.4 | blastp |
| 1486 | LYM146 H4 | sorghum\|09v1\|SB01G 036160 | 2993 | 361 | 96 | blastp |
| 1487 | LYM147 H0 | sorghum\|09v1\|SB03G 003200 | 2994 | 362 | 82 | blastp |
| 1488 | LYM 154 H0 | wheat\|gb164\|BE50057 1 | 2995 | 363 | 82.5 | blastp |
| 1489 | LYM155 H3 | brachypodium\|09v1\|D V479969 | 2996 | 364 | 84.7 | blastp |
| 1489 | LYM155 H3 | brachypodium\|09v1\|D V479969 | 2996 | 451 | 83.5 | blastp |
| 1490 | LYM155 H4 | rice\|gb170\|OS03G588 90 | 2997 | 364 | 81.3 | blastp |
| 1490 | LYM155 H4 | rice\|gb170\|OS03G588 90 | 2997 | 451 | 80 | blastp |
| 1491 | LYM155 H3 | wheat\|gb164[BQ80101 9 | 2998 | 364 | 92.3 | blastp |
| 1491 | LYM155 H3 | wheat\|gb164\|BQ801019 | 2998 | 451 | 91.6 | blastp |
| 1492 | LYM180 H0 | brachypodium\|09v1\|D V473436 | 2999 | 365 | 82.9 | blastp |
| 1492 | LYM180 H0 | brachypodium\|09v 1\|D V473436 | 2999 | 452 | 83.54 | tblastn |
| 1493 | LYM181 H3 | brachypodium\|09v1\|D V485149 | 3000 | 366 | 85.3 | blastp |
| 1493 | LYM181 H3 | brachypodium\|09v1\|D V485149 | 3000 | 453 | 84.68 | tblastn |
| 1494 | LYM181 H4 | maize\|gb170\|DR452216 | 3001 | 366 | 80.8 | blastp |
| 1494 | LYM181 | maize\|gb 170\|DR45221 6 | 3001 | 453 | 81.25 | tblastn |
| 1495 | LYM181 H5 | rice\|gb170\|OS07G320 10 | 3002 | 366 | 82.8 | blastp |
| 1496 | LYM181 H6 | sorghum\|09v1\|SB02G 034110 | 3003 | 366 | 83.5 | blastp |
| 1497 | LYM181 H2 | wheat\|gb164\|BE42537 7 | 3004 | 453 | 87.16 | tblastn |
| 1498 | LYM181 H3 | wheat\|gb164\|BG90502 8 | 3005 | 453 | 93.58 | tblastn |
| 1499 | LYM 182 H8 | brachypodium\|09v1\|G T780326 | 3006 | 367 | 87.64 | tblastn |
| 1500 | LYM182 H9 | maize\|gb170\|AI79573 7 | 3007 | 367 | 84.27 | tblastn |
| 1501 | LYM182 H10 | millet\|09v1\|EVO454P M084568 | 3008 | 367 | 82.02 | tblastn |
| 1502 | LYM 182 H11 | rice\|gb170\|OS04G333 00 | 3009 | 367 | 82.02 | tblastn |
| 1503 | LYM 182 H12 | sorghum\|09v1\|SB06G 015280 | 3010 | 367 | 83.15 | tblastn |
| 1504 | LYM 182 H13 | sugarcane\|gb157.3\|CA 066047 | 3011 | 367 | 83.15 | tblastn |
| 1505 | LYM182 H6 | switchgrass\|gb167\|FE6 10729 | 3012 | 367 | 82.02 | tblastn |
| 1506 | LYM182 H7 | switchgrass\|gb167\|FL6 99214 | 3013 | 367 | 83.15 | tblastn |
| 1507 | LYM182 H8 | wheat\|gb164\|BF20013 | 3014 | 367 | 95.51 | tblastn |
| 1508 | LYM184 H5 | brachypodium\|09v1\|D V476770 | 3015 | 454 | 82.68 | tblastn |
| 1509 | LYM184 H6 | brachypodium\|09v1\|G T762544 | 3016 | 454 | 82.61 | tblastn |
| 1510 | LYM184 H7 | brachypodium\|09v1\|S RR031799S0153720 | 3017 | 454 | 82.68 | tblastn |
| 1511 | LYM184 H3 | leymus\|gb166\|EG3851 50 | 3018 | 454 | 84.8 | blastp |
| 1512 | LYM184 H8 | maize\|gb170\|AI69121 | 3019 | 382 | 100 | blastp |
| 1512 | LYM184 H8 | maize\|gb170\|AI69121 | 3019 | 454 | 80.87 | tblastn |
| 1513 | LYM206 H2 | sorghum\|09v1\|SB07G 021090 | 3020 | 382 | 84.5 | blastp |
| 1513 | LYM206 H2 | sorghum\|09v1\|SB07G 021090 | 3020 | 454 | 80.52 | tblastn |
| 1514 | LYM184 H4 | switchgrass\|gb167\|FL7 04827 | 3021 | 454 | 80.5 | blastp |
| 1515 | LYM184 H5 | wheat\|gb164\|AL82125 | 3022 | 368 | 82.66 | tblastn |
| 1515 | LYM184 H5 | wheat\|gb164\|AL82125 4 | 3022 | 454 | 86.96 | tblastn |
| 1516 | LYM185 H1 | pseudoroegneria\|gb16 7\|FF360278 | 3023 | 455 | 91.53 | tblastn |
| 1517 | LYM185 H2 | wheat\|gb164\|BG90607 7 | 3024 | 455 | 85.31 | tblastn |
| 1518 | LYM 186 H4 | brachypodium\|09v1\|G T761126 | 3025 | 370 | 90.7 | blastp |
| 1519 | LYM186 H5 | maize\|gb170\|BE55288 7 | 3026 | 370 | 82.5 | tblastn |
| 1520 | LYM186 H6 | rice\|gb170\|OS10G399 30 | 3027 | 370 | 86.3 | blastp |
| 1521 | LYM186 H7 | sorghum\|09v1\|SB01G 030050 | 3028 | 370 | 86.5 | blastp |
| 1522 | LYM186 H4 | wheat\|gb164\|BE42942 5 | 3029 | 370 | 97.6 | blastp |
| 1523 | LYM188 H8 | brachypodium\|09v1\|D V475481 | 3030 | 371 | 92.4 | blastp |
| 1523 | LYM188 H8 | brachypodium\|09v1\|D V475481 | 3030 | 456 | 89.41 | tblastn |
| 1524 | LYM188 H9 | maize\|gb170\|AI62272 6 | 3031 | 371 | 87.2 | blastp |
| 1524 | LYM 188 H9 | maize\|gb 170\|AI62272 6 | 3031 | 456 | 83.47 | tblastn |
| 1525 | LYM188 H10 | maize\|gb170\|AW4248 65 | 3032 | 371 | 86 | blastp |
| 1525 | LYM 188 H10 | maize\|gb170\|AW4248 65 | 3032 | 456 | 83.9 | tblastn |
| 1526 | LYM188 H11 | millet\|09v1\|EVO454P M011140 | 3033 | 456 | 85.17 | tblastn |
| 1527 | LYM188 H3 | pseudoroegneria\|gb 16 7\|FF341644 | 3034 | 456 | 80.5 | blastp |
| 1528 | LYM188 H12 | rice\|gb170\|OS03G539 60 | 3035 | 371 | 88.2 | blastp |
| 1528 | LYM 188 H12 | rice\|gb170\|OS03GS39 60 | 3035 | 456 | 84.75 | tblastn |
| 1529 | LYM 188 H13 | sorghum\|09v1\|SB01G 007950 | 3036 | 371 | 87.4 | blastp |
| 1529 | LYM188 H13 | sorghum\|09v1\|SB01G 007950 | 3036 | 456 | 84.32 | tblastn |
| 1530 | LYM 188 H14 | sugarcane\|gb157.3\|BQ 530106 | 3037 | 371 | 87.4 | blastp |
| 1530 | LYM188 H14 | sugarcane\|gb157.3\|BQ 530106 | 3037 | 456 | 84.32 | tblastn |
| 1531 | LYM 188 H7 | switchgrass\|gb167\|FL7 09807 | 3038 | 456 | 84.32 | tblastn |
| 1532 | LYM188 H8 | wheat\|gb164\|CA742940 | 3039 | 456 | 81.78 | tblastn |
| 1533 | LYM193 H1 | leymus\|gb166\|EG3819 14 | 3040 | 459 | 87.1 | tblastn |
| 1534 | LYM193 H2 | wheat\|gb164\|BQ23667 | 3041 | 459 | 85.26 | tblastn |
| 1535 | LYM194 H0 | brachypodium\|09v1\|S RR031796S0004815 | 3042 | 375 | 84.8 | blastp |
| 1536 | LYM194 H1 | maize\|gb170\|BQ539102 | 3043 | 375 | 82.1 | blastp |
| 1537 | LYM194 H2 | sorghum\|09v1\|SB06G 015320 | 3044 | 375 | 84.9 | blastp |
| 1538 | LYM194 H3 | switchgrass\|gb167\|FE6 45079 | 3045 | 375 | 82.9 | blastp |
| 1539 | LYM194 H4 | wheat\|gb164\|BE518078 | 3046 | 375 | 96.3 | blastp |
| 1540 | LYM197 H2 | sugarcane\|gb157.3\|BQ 536804 | 3047 | 377 | 88.57 | tblastn |
| 1541 | LYM198 H1 | sorghum\|09v1\|SB01G045460 045460 | 3048 | 378 | 85.5 | blastp |
| 1542 | LYM201 H1 | aquitegia\|gb157.3\|DR9 15888 | 3049 | 379 | 82.4 | blastp |
| 1543 | LYM201 H19 | arabidopsis lyrata\|09v1\|JGIAL022 871 | 3050 | 379 | 80.07 | tblastn |
| 1544 | LYM201 H2 | artemisia\|gb164\|EY03 3288 | 3051 | 379 | 80.5 | blastp |
| 1545 | LYM201 H3 | b rapa\|gb 162\|CV433700 | 3052 | 379 | 80.3 | blastp |
| 1546 | LYM201 H20 | brachypodium\|09v 1\|D V471345 | 3053 | 379 | 93.8 | blastp |
| 1547 | LYM201 H21 | brachypodium\|09v1\|G T759255 | 3054 | 379 | 81.2 | blastp |
| 1548 | LYM201 H22 | cassava\|09v 1\|DQ 1383 70 | 3055 | 379 | 82.2 | blastp |
| 1549 | LYM201 H23 | cassava\|09v1\|FF38053 9 | 3056 | 379 | 81.3 | blastp |
| 1550 | LYM201 H24 | castorbean\|09v1\|EE25 6048 | 3057 | 379 | 81.4 | blastp |
| 1551 | LYM201 H25 | chestnut\|gb170\|SRR00 6295S0006313 | 3058 | 379 | 80.6 | blastp |
| 1552 | LYM201 H7 | cotton\|gb164\|AI728378 | 3059 | 379 | 81.7 | blastp |
| 1553 | LYM201 H8 | cotton\|gb164\|CO0709 70 | 3060 | 379 | 82 | blastp |
| 1554 | LYM201 H26 | cucumber\|09v1\|AM73 1598 | 3061 | 379 | 81.7 | blastp |
| 1555 | LYM201 H27 | lotus\|09v1\|BI419437 | 3062 | 379 | 80.5 | blastp |
| 1556 | LYM201 H28 | maize\|gb 170\|AI97825 4 | 3063 | 379 | 98.2 | blastp |
| 1557 | LYM201 H29 | maize\|gb170\|DR97111 8 | 3064 | 379 | 80.1 | blastp |
| 1558 | LYM201 H30 | medicago\|09v 1\|AW68 4099 | 3065 | 379 | 80.7 | blastp |
| 1559 | LYM201 H31 | oak\|gb 170\|CU656181 | 3066 | 379 | 82.56 | tblastn |
| 1560 | LYM201 H32 | poplar\|gb170\|BI06963 7 | 3067 | 379 | 81.3 | blastp |
| 1561 | LYM201 H33 | poplar\|gb170\|BU887151 51 | 3068 | 379 | 80.7 | blastp |
| 1562 | LYM201 H34 | rice\|gb 170\|OS02G345 60 | 3069 | 379 | 95.2 | blastp |
| 1563 | LYM201 H35 | solanum phureja\|09v1\|SPHBG1 23984 | 3070 | 379 | 81.4 | blastp |
| 1564 | LYM201 H36 | solanum phureja\|09v 1\|SPHBG1 29477 | 3071 | 379 | 81.2 | blastp |
| 1565 | LYM201 H37 | sorghum\|09v1\|SB04G 022350 | 3072 | 379 | 98.4 | blastp |
| 1566 | LYM201 H15 | soybean\|gb168\|AL367 670 | 3073 | 379 | 80.7 | blastp |
| 1567 | LYM201 H16 | soybean\|gb168\|AW68 4099 | 3074 | 379 | 80.5 | blastp |
| 1568 | LYM201 H38 | sugarcane\|gb157.3\|CA 065291 | 3075 | 379 | 98.6 | blastp |
| 1569 | LYM201 H18 | switchgrass\|gb167\|FE6 41755 | 3076 | 379 | 98.2 | blastp |
| 1570 | LYM201 H39 | tomato\|09v 1\|BG 12398 4 | 3077 | 379 | 81.4 | blastp |
| 1571 | LYM201 H40 | tomato\|09v 1\|BG 12947 7 | 3078 | 379 | 81.2 | blastp |
| 1572 | LYM201 H19 | wheat\|gb164\|BE40316 8 | 3079 | 379 | 93.2 | blastp |
| 1573 | LYM203 H9 | barley\|gb157SOLEXA\|BI949918 | 3080 | 380 | 84.7 | blastp |
| 1574 | LYM203 H10 | lolium\|09v1\|AU24700 9 | 3081 | 380 | 85.2 | blastp |
| 1575 | LYM203 H11 | maize\|gb 170\|AI62967 5 | 3082 | 380 | 95.7 | blastp |
| 1576 | LYM203 H12 | millet\|09v1\|EVO454P M058394 | 3083 | 380 | 81.7 | blastp |
| 1577 | LYM203 H13 | rice\|gb 170\|OS02G083 80 | 3084 | 380 | 89.2 | blastp |
| 1578 | LYM203 H14 | sorghum\|09v1\|SB04G 005460 | 3085 | 380 | 96.8 | blastp |
| 1579 | LYM203 H15 | sugarcane\|gb 157.3\|CA 119568 | 3086 | 380 | 94.1 | blastp |
| 1580 | LYM203 H6 | switchgrass\|gb167\|DN 142920 | 3087 | 380 | 95.2 | blastp |
| 1581 | LYM203 H7 | switchgrass\|gb167\|FE6 17741 | 3088 | 380 | 95.7 | blastp |
| 1582 | LYM203 H8 | wheat\|gb164\|BQ801966 | 3089 | 380 | 85.7 | blastp |
| 1583 | LYM203 H9 | wheat\|gb164\|BQ90323 0 | 3090 | 380 | 86.2 | blastp |
| 1584 | LYM206 H3 | maize\|gb170\|AW0559 97 | 3091 | 382 | 84.1 | blastp |
| 1585 | LYM207 H2 | maize\|gb170\|BM3402 89 | 3092 | 383 | 86.5 | blastp |
| 1586 | LYM207 H3 | sorghum\|09v1\|SB06G 023870 | 3093 | 383 | 94.3 | blastp |
| 1587 | LYM207 H2 | switchgrass\|gb167\|FE6 01320 | 3094 | 383 | 82.3 | blastp |
| 1588 | LYM208 H6 | maize\|gb170\|AI691368 | 3095 | 384 | 94.4 | blastp |
| 1589 | LYM208 H7 | rice\|gb170\|OS09G016 90 | 3096 | 384 | 82 | blastp |
| 1590 | LYM208 H8 | sorghum\|09v1\|SB01G 032070 | 3097 | 384 | 92.1 | blastp |
| 1591 | LYM208 H9 | sorghum\|09v1\|SB08G 002510 | 3098 | 384 | 86.6 | blastp |
| 1592 | LYM208 H5 | switchgrass\|gb167\|FE6 12629 | 3099 | 384 | 90.4 | blastp |
| 1593 | LYM208 H6 | switchgrass\|gb167\|FL7 29765 | 3100 | 384 | 90.4 | blastp |
| 1594 | LYM212 H6 | barley\|gb 157SOLEXA \|BF623682 | 3101 | 385 | 80.3 | blastp |
| 1595 | LYM212 | maize \|gb 170\|AI67747 4 | 3102 | 385 | 88.5 | blastp |
| 1596 | LYM212 H8 | rice\|gb 170\|OS03G079 10 | 3103 | 385 | 80.11 | tblastn |
| 1597 | LYM212 H9 | sorghum\|09v1\|SB01G 045480 | 3104 | 385 | 92.5 | blastp |
| 1598 | LYM212 H10 | sugarcane\|gb157.3\|CA 088789 | 3105 | 385 | 92 | blastp |
| 1599 | LYM212 H6 | wheat\|gb164\|BE40224 2 | 3106 | 385 | 80.38 | tblastn |
| 1600 | LYM213 H1 | maize\|gb170\|AW2822 49 | 3107 | 386 | 90.9 | blastp |
| 1601 | LYM213 H2 | sorghum\|09v1\|SB06G 018770 | 3108 | 386 | 91.7 | blastp |
| 1602 | LYM215 H2 | switchgrass\|gb167\|FE6 18086 | 3109 | 387 | 90.42 | tblastn |
| 1603 | LYM217 H3 | sorghum\|09v1\|SB01G 043910 | 3110 | 388 | 88.2 | blastp |
| 1604 | LYM217 H4 | sugarcane\|gb157.3\|CA 136491 | 3111 | 388 | 87.7 | blastp |
| 1605 | LYM217 H3 | switchgrass\|gb167\|FE6 06442 | 3112 | 388 | 86.7 | blastp |
| 1606 | LYM22 1 H1 | brachypodium\|09v1\|G T792319 | 3113 | 391 | 83.8 | blastp |
| 1606 | LYM22 1 H1 | brachypodium\|09v1\|G T792319 | 3113 | 461 | 81.3 | blastp |
| 1607 | LYM221 H2 | rice\|gb170\|OS03G248 70 | 3114 | 391 | 82.9 | blastp |
| 1607 | LYM221 H2 | rice\|gb170\|OS03G248 70 | 3114 | 461 | 80.5 | blastp |
| 1608 | LYM221 H3 | sorghum\|09v1\|SB01G 034610 | 3115 | 391 | 90.7 | blastp |
| 1608 | LYM221 H3 | sorghum\|09v1\|SB01G 034610 | 3115 | 461 | 88.7 | blastp |
| 1609 | LYM224 H2 | brachypodium\|09v1\|G T762108 | 3116 | 393 | 82.4 | blastp |
| 1610 | LYM224 H3 | sorghum\|09v1\|SB02G 040000 | 3117 | 393 | 92.9 | blastp |
| 1611 | LYM224 H2 | switchgrass\|gb167\|FE6 17506 | 3118 | 393 | 83.19 | tblastn |
| 1612 | LYM227 H1 | sorghum\|09v1\|SB01G 043140 | 3119 | 394 | 88.3 | blastp |
| 1613 | LYM228 H1 | sorghum\|09v1\|SB09G 006910 | 3120 | 395 | 85 | blastp |
| 1613 | LYM228 H1 | sorghum\|09v1\|SB09G 006910 | 3120 | 462 | 83.7 | blastp |
| 1614 | LYM232 H3 | sorghum\|09v1\|SB02G 000450 | 3121 | 396 | 80.4 | blastp |
| 1615 | LYM232 H4 | sugarcane\|gb157.3\|CA 073189 | 3122 | 396 | 80.8 | blastp |
| 1616 | LYM232 H3 | switchgrass\|gb167\|FL8 49399 | 3123 | 396 | 80.7 | blastp |
| 1617 | LYM233 H0 | brachypodium\|09v1\|T MPLOS01G70020T1 | 3124 | 397 | 99.6 | blastp |
| 1618 | LYM234 H0 | rice\|gb170\|OS07G382 90 | 3125 | 398 | 81.3 | blastp |
| 1619 | LYM236 H99 | apple\|gb171\|CN48856 8 | 3126 | 399 | 89.1 | blastp |
| 1620 | LYM236 H100 | apple\|gb171\|CN88310 0 | 3127 | 399 | 89.6 | blastp |
| 1621 | LYM236 H3 | aquilegia\|gb157.3\|DR9 19774 | 3128 | 399 | 87.4 | blastp |
| 1622 | LYM236 H101 | arabidopsis lyrata\|09v1\|JGIAL005 113 | 3129 | 399 | 83.9 | blastp |
| 1623 | LYM236 H102 | arabidopsis lyrata\|09v1\|JGIAL006 646 | 3130 | 399 | 86.89 | tblastn |
| 1624 | LYM236 H4 | arabidopsis\|gb165\|AT 1G54340 | 3131 | 399 | 83.5 | blastp |
| 1625 | LYM236 H5 | arabidopsis\|gb 165\|AT 1G65930 | 3132 | 399 | 87.1 | blastp |
| 1626 | LYM236 H6 | artemisia\|gb164\|EY03 4635 | 3133 | 399 | 87.9 | blastp |
| 1627 | LYM236 H7 | avocado\|gb164\|CO995 120 | 3134 | 399 | 91.3 | blastp |
| 1628 | LYM236 H8 | b oleracea\|gb161\|DY028 218 | 3135 | 399 | 87.2 | blastp |
| 1629 | LYM236 H9 | b rapa\|gb 162\|CX269094 | 3136 | 399 | 87.2 | blastp |
| 1630 | LYM236 H10 | b rapa\|gb162\|GFXAF25 8246X 1 | 3137 | 399 | 84.3 | tblastn |
| 1631 | LYM236 H11 | b rapa\|gb162\|L47856 | 3138 | 399 | 87.2 | blastp |
| 1632 | LYM236 H103 | barley\|gb157SOLEXA \|AL502504 | 3139 | 399 | 89.3 | blastp |
| 1633 | LYM236 H13 | basilicum\|gb157.3\|DY 322368 | 3140 | 399 | 87.7 | blastp |
| 1634 | LYM236 H14 | bean\|gb167\|CA896841 | 3141 | 399 | 87.9 | blastp |
| 1635 | LYM236 H104 | brachypodium\|09v1\|D V478973 | 3142 | 399 | 85.3 | blastp |
| 1636 | LYM236 H105 | brachypodium\|09v1\|D V482439 | 3143 | 399 | 90.5 | blastp |
| 1637 | LYM236 H17 | cacao\|gb167\|DQ44887 5 | 3144 | 399 | 89.2 | blastp |
| 1638 | LYM236 H18 | canola\|gb161\|BQ7049 58 | 3145 | 399 | 87.2 | blastp |
| 1639 | LYM236 H19 | canola\|gb161\|CD8173 40 | 3146 | 399 | 87.2 | blastp |
| 1640 | LYM236 H20 | canola\|gb161\|CD8331 08 | 3147 | 399 | 83.9 | blastp |
| 1641 | LYM236 H21 | canola\|gb161\|CX1894 42 | 3148 | 399 | 86.5 | blastp |
| 1642 | LYM236 H22 | canola\|gb161\|DY0113 90 | 3149 | 399 | 87.2 | blastp |
| 1643 | LYM236 H106 | cassava\|09v1\|CK6426 10 | 3150 | 399 | 90.1 | blastp |
| 1644 | LYM236 H107 | cassava\|09v1\|DV4579 42 | 3151 | 399 | 87.5 | blastp |
| 1645 | LYM236 H108 | castorbean\|09v1\|EE25 6632 | 3152 | 399 | 86.1 | blastp |
| 1646 | LYM236 H109 | castorbean\|09v1\|EE25 9479 | 3153 | 399 | 90.6 | blastp |
| 1647 | LYM236 H26 | centaurea\|gb166\|EH73 1203 | 3154 | 399 | 81.2 | blastp |
| 1648 | LYM236 H27 | centaurea\|gb166\|EL93 2337 | 3155 | 399 | 86.6 | blastp |
| 1649 | LYM236 H110 | chestnut\|gb170\|SRR00 6295S0002580 | 3156 | 399 | 82.27 | tblastn |
| 1650 | LYM236 H111 | chestnut\|gb170\|SRR00 6295S0002701 | 3157 | 399 | 89.6 | blastp |
| 1651 | LYM236 H112 | cichorium\|gb171\|EH6 75189 | 3158 | 399 | 87.3 | blastp |
| 1652 | LYM236 H113 | cichorium\|gb171\|EH6 83726 | 3159 | 399 | 89.1 | blastp |
| 1653 | LYM236 H30 | citrus\|gb166\|AF17666 9 | 3160 | 399 | 90.6 | blastp |
| 1654 | LYM236 H31 | citrus\|gb166\|CD57391 1 | 3161 | 399 | 85.1 | blastp |
| 1655 | LYM236 H32 | coffea\|gb157.2\|DV663 279 | 3162 | 399 | 87.8 | blastp |
| 1656 | LYM236 H33 | cotton\|gb164\|AI72726 0 | 3163 | 399 | 87.2 | blastp |
| 1657 | LYM236 H34 | cotton\|gb164\|BF27858 8 | 3164 | 399 | 83.5 | blastp |
| 1658 | LYM236 H35 | cowpea\|gb166\|FC4581 36 | 3165 | 399 | 89.1 | blastp |
| 1659 | LYM236 H36 | cynara\|gb167\|GE5772 43 | 3166 | 399 | 86.47 | tblastn |
| 1660 | LYM236 H37 | cynara\|gb167\|GE5796 63 | 3167 | 399 | 88.6 | blastp |
| 1661 | LYM236 H38 | dandelion\|gb161\|DY8 04243 | 3168 | 399 | 86.96 | tblastn |
| 1662 | LYM236 H39 | eucalyptus\|gb166\|X97 063 | 3169 | 399 | 88 | blastp |
| 1663 | LYM236 H40 | fescue\|gb161\|CK8010 45 | 3170 | 399 | 89.8 | blastp |
| 1664 | LYM236 H41 | fescue\|gb161\|DT6747 24 | 3171 | 399 | 90 | blastp |
| 1665 | LYM236 H42 | ginger\|gb164\|DY3602 89 | 3172 | 399 | 91.3 | blastp |
| 1666 | LYM236 H43 | grape\|gb160\|BM43675 5 | 3173 | 399 | 89.9 | blastp |
| 1667 | LYM236 H44 | kiwi\|gb166\|FG396670 | 3174 | 399 | 88.2 | blastp |
| 1668 | LYM236 H45 | kiwi\|gb166\|FG397107 | 3175 | 399 | 88.7 | blastp |
| 1669 | LYM236 H46 | kiwi\|gb166\|FG397291 | 3176 | 399 | 89.4 | blastp |
| 1670 | LYM236 H47 | lettuce\|gb157.2\|DW08 8948 | 3177 | 399 | 87.4 | blastp |
| 1671 | LYM236 H48 | leymus\|gb166\|CD8091 60 | 3178 | 399 | 89.6 | blastp |
| 1672 | LYM236 H49 | liriodendron\|gb166\|CK 762229 | 3179 | 399 | 88.2 | blastp |
| 1673 | LYM236 H114 | lotus\|09v1\|AW428686 | 3180 | 399 | 89.4 | blastp |
| 1674 | LYM236 H115 | lotus\|09v1\|BP033879 | 3181 | 399 | 83.7 | blastp |
| 1675 | LYM236 H51 | lovegrass\|gb167\|DN48 0596 | 3182 | 399 | 90.8 | blastp |
| 1676 | LYM236 H116 | maize\|gb170\|AI60081 5 | 3183 | 399 | 93.7 | blastp |
| 1677 | LYM236 H117 | maize\|gb170\|AW3132 97 | 3184 | 399 | 92.2 | blastp |
| 1678 | LYM236 H118 | maize\|gb170\|W21690 | 3185 | 399 | 91.3 | blastp |
| 1679 | LYM236 H119 | medicago[09v 1\|AW19 1201 | 3186 | 399 | 88.2 | blastp |
| 1680 | LYM236 H120 | medicago\|09v1\|AW68 9032 | 3187 | 399 | 84.9 | blastp |
| 1681 | LYM236 H121 | millet\|09v 1\|CD725798 | 3188 | 399 | 92.5 | blastp |
| 1682 | LYM236 H122 | millet\|09v1\|EVO454P M002708 | 3189 | 399 | 94.2 | blastp |
| 1683 | LYM236 H123 | monkeyflowerl09v1\|D V206036 | 3190 | 399 | 87.4 | blastp |
| 1684 | LYM236 H56 | nicotiana benthamiana\|gb162\|C K291144 | 3191 | 399 | 86.5 | blastp |
| 1685 | LYM236 H57 | palm\|gb166\|EL684429 | 3192 | 399 | 87.3 | blastp |
| 1686 | LYM236 H124 | peanut\|gb 171\|CD0386 82 | 3193 | 399 | 87.9 | blastp |
| 1687 | LYM236 H125 | pea\|09v1\|CD860585 | 3194 | 399 | 89.6 | blastp |
| 1688 | LYM236 H126 | pepper\|gb171\|BM0617 61 | 3195 | 399 | 88 | blastp |
| 1689 | LYM236 H127 | pepper\|gb171\|CA5165 82 | 3196 | 399 | 85.3 | blastp |
| 1690 | LYM236 H128 | petunia\|gb171\|DC2402 08 | 3197 | 399 | 88.2 | blastp |
| 1691 | LYM236 H129 | physcomitrella\|10v1\|A W497149 | 3198 | 399 | 82.2 | blastp |
| 1692 | LYM236 H62 | pine\|gb157.2\|AW0102 92 | 3199 | 399 | 85.6 | blastp |
| 1693 | LYM236 H63 | pine\|gb157.2\|BX2498 26 | 3200 | 399 | 85.6 | blastp |
| 1694 | LYM236 H130 | poplar\|gb 170\|AI116195 6 | 3201 | 399 | 89.6 | blastp |
| 1695 | LYM236 H131 | poplar\|gb170\|BI12770 6 | 3202 | 399 | 86.6 | blastp |
| 1696 | LYM236 H132 | poplar\|gbl70\|BI13161 0 | 3203 | 399 | 86.8 | blastp |
| 1697 | LYM236 H133 | poplar\|gb170\|BU8210 63 | 3204 | 399 | 90.4 | blastp |
| 1698 | LYM236 H66 | potato\|gb157.2\|BG591 093 | 3205 | 399 | 88 | blastp |
| 1699 | LYM236 H67 | prunus\|gb167\|AF3674 43 | 3206 | 399 | 89.4 | blastp |
| 1700 | LYM236 H68 | radish\|gb164\|EV52684 7 | 3207 | 399 | 85.8 | blastp |
| 1701 | LYM236 H69 | radish\|gb164\|EV53122 5 | 3208 | 399 | 86.9 | blastp |
| 1702 | LYM236 H134 | rice\|gb170\|OS01G145 80 | 3209 | 399 | 84.8 | blastp |
| 1703 | LYM236 H135 | ric\|gb170\|OS01G466 10 | 3210 | 399 | 93 | blastp |
| 1704 | LYM236 H72 | rye\|gb164\|BE494776 | 3211 | 399 | 86.17 | tblastn |
| 1705 | LYM236 H73 | safflower\|gb162\|EL37 2795 | 3212 | 399 | 87.3 | blastp |
| 1706 | LYM236 H74 | safflower\|gb162\|EL37 4725 | 3213 | 399 | 89.1 | blastp |
| 1707 | LYM236 H136 | solanum phureja\|09v 1\|SPHBG1 31802 | 3214 | 399 | 86.3 | blastp |
| 1708 | LYM236 H137 | solanum phureja\|09v1\|SPHBG6 29432 | 3215 | 399 | 87.7 | blastp |
| 1709 | LYM236 H138 | sorghum\|09v1\|SB03G 029840 | 3216 | 399 | 92 | blastp |
| 1710 | LYM236 H139 | sorghum\|09v1\|SB09G 029110 | 3217 | 399 | 94.7 | blastp |
| 1711 | LYM236 H77 | soybean\|gb168\|AW25 7518 | 3218 | 399 | 88.7 | blastp |
| 1712 | LYM236 H78 | soybean\|gb168\|AW68 9032 | 3219 | 399 | 85.6 | blastp |
| 1713 | LYM236 H79 | soybean\|gb168\|FF554 826 | 3220 | 399 | 84.7 | blastp |
| 1714 | LYM236 H80 | soybean\|gb168\|SOYID H | 3221 | 399 | 89.4 | blastp |
| 1715 | LYM236 H81 | spikemoss\|gb165\|FE44 1231 | 3222 | 399 | 84.2 | blastp |
| 1716 | LYM236 H82 | spikemoss\|gb165\|FE44 3181 | 3223 | 399 | 82.3 | blastp |
| 1717 | LYM236 H83 | spruce\|gb162\|CO2258 56 | 3224 | 399 | 85 | blastp |
| 1718 | LYM236 H84 | strawberry\|gb164\|DV4 38767 | 3225 | 399 | 82.6 | blastp |
| 1719 | LYM236 H140 | sugarcane\|gb157.3\|BU 103347 | 3226 | 399 | 94.5 | blastp |
| 1720 | LYM236 H141 | sugarcane\|gb157.3\|CA 070718 | 3227 | 399 | 92.5 | blastp |
| 1721 | LYM236 H87 | sunflower\|gb162\|CD8 46861 | 3228 | 399 | 86.6 | blastp |
| 1722 | LYM236 H88 | sunflower\|gb162\|CD8 54278 | 3229 | 399 | 87.9 | blastp |
| 1723 | LYM236 H89 | switchgrass\|gb167\|DN 142415 | 3230 | 399 | 92 | blastp |
| 1724 | LYM236 H90 | switchgrass\|gb167\|DN 143508 | 3231 | 399 | 95.4 | blastp |
| 1725 | LYM236 H91 | switchgrass\|gb167\|DN 150237 | 3232 | 399 | 95.2 | blastp |
| 1726 | LYM236 H92 | switchgrass\|gb167\|DN 151575 | 3233 | 399 | 92.2 | blastp |
| 1727 | LYM236 H93 | thellungiella\|gb167\|D N774112 | 3234 | 399 | 87.14 | tblastn |
| 1728 | LYM236 H94 | tobacco\|gb162\|DV158 343 | 3235 | 399 | 83.3 | blastp |
| 1729 | LYM236 H95 | tobacco\|gb162\|X77944 | 3236 | 399 | 88.5 | blastp |
| 1730 | LYM236 H142 | tomato\|09v1\|BG13180 2 | 3237 | 399 | 85.6 | blastp |
| 1731 | LYM236 H143 | tomato\|09v1\|BG62943 2 | 3238 | 399 | 87.3 | blastp |
| 1732 | LYM236 H98 | triphysaria\|gb164\|DR1 71747 | 3239 | 399 | 88.4 | blastp |
| 1733 | LYM236 H99 | wheat\|gb164\|BE44254 0 | 3240 | 399 | 84.1 | blastp |
| 1734 | LYM238 H0 | rice\|gb 170\|OS05G450 80 | 3241 | 400 | 95.4 | blastp |
| 1735 | LYM240 | barley\|gb157SOLEXA \|AL508021 | 3242 | 402 | 91.9 | blastp |
| 1736 | LYM240 H10 | brachypodium\|09v1\|G T810642 | 3243 | 402 | 91.9 | blastp |
| 1737 | LYM240 H11 | maize\|gb170\|DR97279 0 | 3244 | 402 | 84.2 | blastp |
| 1738 | LYM240 H12 | sorghum\|09v1\|SB02G 038240 | 3245 | 402 | 84.2 | blastp |
| 1739 | LYM240 H13 | sugarcane\|gb157.3\|CA 075929 | 3246 | 402 | 82.3 | blastp |
| 1740 | LYM240 H6 | switchgrass\|gb167\|FE5 97498 | 3247 | 402 | 81.6 | blastp |
| 1741 | LYM240 H7 | switchgrass\|gb167\|FE6 10847 | 3248 | 402 | 80 | blastp |
| 1742 | LYM240 H8 | switchgrass\|gb167\|FL9 60804 | 3249 | 402 | 81.6 | blastp |
| 1743 | LYM240 H9 | wheat\|gb164\|CA67449 1 | 3250 | 402 | 89.93 | tblastn |
| 1744 | LYM242 H7 | barley\|gb157SOLEXA \|BE412570 | 3251 | 404 | 82.2 | blastp |
| 1745 | LYM242 H8 | brachypodium\|09v1\|G T764573 | 3252 | 404 | 86.1 | blastp |
| 1746 | LYM242 H9 | maize\|gb 170\|A174605 3 | 3253 | 404 | 83.4 | blastp |
| 1747 | LYM242 H10 | millet\|09v1\|EVO454P M008771 | 3254 | 404 | 85 | blastp |
| 1748 | LYM242 H11 | sorghum\|09v1\|SB03G 003160 | 3255 | 404 | 82.6 | blastp |
| 1749 | LYM242 H12 | sugarcane\|gb157.3\|BQ 534251 | 3256 | 404 | 83.8 | blastp |
| 1750 | LYM242 H5 | switchgrass\|gb167\|DN 143169 | 3257 | 404 | 85.1 | blastp |
| 1751 | LYM242 H6 | switchgrass\|gb167\|FE6 54179 | 3258 | 404 | 85 | blastp |
| 1752 | LYM242 H7 | wheat\|gb164\|BE40328 5 | 3259 | 404 | 82.6 | blastp |
| 1753 | LYM248 H3 | brachypodium\|09v1\|G T773582 | 3260 | 407 | 84.5 | blastp |
| 1754 | LYM248 H4 | maize\|gb170\|AI96695 7 | 3261 | 407 | 88.5 | blastp |
| 1755 | LYM248 H5 | sorghum\|09v1\|SB04G 000645 | 3262 | 407 | 88.2 | blastp |
| 1756 | LYM248 H2 | switchgrass\|gb167\|DN 149511 | 3263 | 407 | 81.1 | blastp |
| 1757 | LYM248 H3 | wheat\|gb164\|BE41803 | 3264 | 407 | 84.89 | tblastn |
| 1758 | LYM250 H0 | rice\|gb170\|OS09G387 | 3265 | 409 | 100 | tblastn |
| 1758 | LYM250 H0 | rice\|gb 170\|OS09G387 00 | 3265 | 463 | 97.78 | tblastn |
| 1759 | LYM251 H1 | antirrhinum\|gb166\|AJ5 60114 | 3266 | 410 | 82 | blastp |
| 1760 | LYM251 H76 | apple\|gb171\|CN49264 3 | 3267 | 410 | 80.1 | blastp |
| 1761 | LYM251 H77 | arabidopsis lyrata\|09v1\|JGIAL012 198 | 3268 | 410 | 81.4 | blastp |
| 1762 | LYM251 H3 | arabidopsis\|gb165\|AT 2G17380 | 3269 | 410 | 80.7 | blastp |
| 1763 | LYM251 H4 | arabidopsis\|gb165\|AT 4G35410 | 3270 | 410 | 80.12 | tblastn |
| 1764 | LYM251 H5 | b oteracea\|b161\|AM38 5265 | 3271 | 410 | 81.4 | blastp |
| 1765 | LYM251 H6 | b rapa\|gb162\|L33527 | 3272 | 410 | 81.4 | blastp |
| 1766 | LYM251 H7 | banana\|gb167\|FF5583 00 | 3273 | 410 | 81.37 | tblastn |
| 1767 | LYM251 H78 | barleylgb157SOLEXA \|BE421807 | 3274 | 410 | 81.4 | blastp |
| 1768 | LYM251 H9 | basilicum\|gb157.3\|DY 343154 | 3275 | 410 | 80.12 | tblastn |
| 1769 | LYM251 | bean\|gb167\|CV536707 | 3276 | 410 | 80.1 | blastp |
| 1770 | LYM251 H79 | brachypodium\|09v 1\|D V469153 | 3277 | 410 | 82.6 | blastp |
| 1771 | LYM25 1 H13 | canola\|gb161\|CN7260 86 | 3278 | 410 | 81.4 | blastp |
| 1772 | LYM251 H80 | cassava\|09v1\|CK6504 27 | 3279 | 410 | 80.12 | tblastn |
| 1773 | LYM251 H81 | cassava\|09v1\|DV4488 85 | 3280 | 410 | 81.4 | blastp |
| 1774 | LYM25 1 H82 | castorbean\|09v1\|XM0 02514342 | 3281 | 410 | 81.4 | blastp |
| 1775 | LYM251 H16 | catharanthus\|gb166\|E G554670 | 3282 | 410 | 81.4 | blastp |
| 1776 | LYM251 H17 | centaurea\|gb166\|EH73 7707 | 3283 | 410 | 81.4 | blastp |
| 1777 | LYM25 1 H18 | centaurea\|gb166\|EH78 2010 | 3284 | 410 | 81.4 | blastp |
| 1778 | LYM251 H83 | chestnut\|gb170\|SRR00 6295S0001854 | 3285 | 410 | 80.1 | blastp |
| 1779 | LYM251 H84 | chickpea\|09v2\|DY475 463 | 3286 | 410 | 80.12 | tblastn |
| 1780 | LYM251 H85 | cichoriumlgb171\|EH6 77909 | 3287 | 410 | 81.4 | blastp |
| 1781 | LYM251 H86 | cichorium\|gb171\|EH6 81849 | 3288 | 410 | 80.7 | blastp |
| 1782 | LYM251 H21 | citruslgb166\|CF41901 5 | 3289 | 410 | 82 | blastp |
| 1783 | LYM251 H22 | clover\|gb162\|BB93513 6 | 3290 | 410 | 81.4 | blastp |
| 1784 | LYM251 H23 | cowpea\|gb166\|FF3837 63 | 3291 | 410 | 80.1 | blastp |
| 1785 | LYM251 H87 | cucumber\|09v1\|CK085 508 | 3292 | 410 | 80.1 | blastp |
| 1786 | LYM251 H24 | dandelion\|gb161\|DY8 22878 | 3293 | 410 | 80.1 | blastp |
| 1787 | LYM251 H25 | eucalyptus\|gb166\|CT9 81708 | 3294 | 410 | 80.7 | blastp |
| 1788 | LYM251 | fern\|gb171\|DK949355 | 3295 | 410 | 80.1 | blastp |
| 1789 | LYM251 H26 | fescue\|gb161\|DT7028 20 | 3296 | 410 | 82.6 | blastp |
| 1790 | LYM251 H89 | gerbera\|09v1\|AJ75631 9 | 3297 | 410 | 80.7 | blastp |
| 1791 | LYM251 H27 | grape\|gb160\|CB00819 1 | 3298 | 410 | 82 | blastp |
| 1792 | LYM251 H28 | grape\|gb160\|CD79981 9 | 3299 | 410 | 82 | blastp |
| 1793 | LYM251 H29 | ipomoea\|gb157.2\|CJ75 1066 | 3300 | 410 | 81.4 | blastp |
| 1794 | LYM251 H90 | jatropha\|09v1\|GO2471 40 | 3301 | 410 | 80.7 | blastp |
| 1795 | LYM25 1 H30 | lettuce\|gb157.2\|DW04 5452 | 3302 | 410 | 81.4 | blastp |
| 1796 | LYM251 H31 | liriodendron\|gb166\|CK 762515 | 3303 | 410 | 83.2 | blastp |
| 1797 | LYM251 H91 | maize\|gb 170\|AA97985 6 | 3304 | 410 | 83.23 | tblastn |
| 1798 | LYM251 H92 | maize\|gb170\|AI61934 2 | 3305 | 410 | 83.2 | blastp |
| 1799 | LYM251 H93 | maize\|gb 170\|AW1344 57 | 3306 | 410 | 91.3 | tblastn |
| 1800 | LYM251 H94 | medicago\|09v1\|MSU9 3094 | 3307 | 410 | 82 | blastp |
| 1801 | LYM251 H36 | melon\|gb165\|DV6335 83 | 3308 | 410 | 80.1 | blastp |
| 1802 | LYM251 H95 | millet\|09v1\|EVO454P M010186 | 3309 | 410 | 83.2 | blastp |
| 1803 | LYM251 H96 | millet\|09v 1\|EVO454P M104784 | 3310 | 410 | 91.93 | tblastn |
| 1804 | LYM251 H97 | monkeyflower\|09v1\|G R007448 | 3311 | 410 | 82.6 | blastp |
| 1805 | LYM251 H37 | nuphar\|gb166\|DT5885 73 | 3312 | 410 | 82 | blastp |
| 1806 | LYM251 H98 | oak\|gb170\|DN949793 | 3313 | 410 | 80.1 | blastp |
| 1807 | LYM251 H38 | papaya\|gb165\|EX2615 60 | 3314 | 410 | 81.4 | blastp |
| 1808 | LYM251 H99 | peanut\|gb 171\|EH0468 45 | 3315 | 410 | 82 | blastp |
| 1809 | LYM251 H100 | pepper\|gb171\|BM0682 91 | 3316 | 410 | 83.2 | blastp |
| 1810 | LYM251 H101 | petunia\|gb171\|FN0050 56 | 3317 | 410 | 82.6 | blastp |
| 1811 | LYM251 H42 | pine\|gb157.2\|AA5568 57 | 3318 | 410 | 81.4 | blastp |
| 1812 | LYM251 H43 | pine\|gb157.2\|AW2898 37 | 3319 | 410 | 81.4 | blastp |
| 1813 | LYM251 H102 | poplar\|gb170\|AI16513 0 | 3320 | 410 | 80.1 | blastp |
| 1814 | LYM251 H45 | poppy\|gb166\|FG61376 3 | 3321 | 410 | 80.1 | blastp |
| 1815 | LYM251 H46 | potato\|gb157.2\|BF054 079 | 3322 | 410 | 82.6 | blastp |
| 1816 | LYM251 H47 | pseudoroegneria\|gb 16 7\|FF342572 | 3323 | 410 | 80.7 | blastp |
| 1817 | LYM251 H48 | radish\|gb164\|EV52520 6 | 3324 | 410 | 80.7 | blastp |
| 1818 | LYM251 H49 | radish\|gb164\|EV52859 3 | 3325 | 410 | 80.7 | blastp |
| 1819 | LYM25 1 H50 | radish\|gb164\|EV53499 6 | 3326 | 410 | 81.4 | blastp |
| 1820 | LYM251 H51 | radish\|gb164\|EV56567 7 | 3327 | 410 | 81.4 | blastp |
| 1821 | LYM251 H103 | rice\|gb170\|OS03G570 40 | 3328 | 410 | 82.6 | blastp |
| 1822 | LYM251 H53 | rye\|gb164\|BE637009 | 3329 | 410 | 80.7 | blastp |
| 1823 | LYM251 H54 | safflower\|gb162\|EL40 2398 | 3330 | 410 | 81.4 | blastp |
| 1824 | LYM251 H104 | solanum phureja\|09v 1\|SPHBG1 26373 | 3331 | 410 | 82.6 | blastp |
| 1825 | LYM251 H105 | sorghum\|09v1\|SB01G 003840 | 3332 | 410 | 91.3 | tblastn |
| 1826 | LYM25 1 H106 | sorghum\|09v1\|SB01G 006180 | 3333 | 410 | 83.2 | blastp |
| 1827 | LYM251 H57 | soybean\|gb168\|AW68 5285 | 3334 | 410 | 81.4 | blastp |
| 1828 | LYM251 H58 | soybean\|gb168\|BQ154 723 | 3335 | 410 | 80.1 | blastp |
| 1829 | LYM251 H59 | spruce\|gb162\|Z93754 | 3336 | 410 | 80.7 | blastp |
| 1830 | LYM251 H107 | sugarcane\|gb157.3\|BQ 533200 | 3337 | 410 | 83.2 | blastp |
| 1831 | LYM251 H108 | sugarcanelgb157.3\|BU 103624 | 3338 | 410 | 83.2 | blastp |
| 1832 | LYM251 H62 | sunflower\|gb162\|CD8 54801 | 3339 | 410 | 81.4 | blastp |
| 1833 | LYM251 H63 | sunflower\|gb162\|DY9 17387 | 3340 | 410 | 81.4 | blastp |
| 1834 | LYM251 H64 | sunflower\|gb162\|EL48 5634 | 3341 | 410 | 81.4 | blastp |
| 1835 | LYM251 H65 | switchgrass\|gb167\|DN 152225 | 3342 | 410 | 83.2 | blastp |
| 1836 | LYM251 H66 | switchgrass\|gb167\|DN 152580 | 3343 | 410 | 82 | blastp |
| 1837 | LYM251 H67 | switchgrass\|gb167\|FL8 27716 | 3344 | 410 | 92.5 | blastp |
| 1838 | LYM251 H68 | thellungiella\|gb167\|D N776639 | 3345 | 410 | 80.7 | blastp |
| 1839 | LYM251 H69 | tobacco\|g162\|CV020 782 | 3346 | 410 | 82 | blastp |
| 1840 | LYM251 H109 | tomato\|09v1\|BG12637 3 | 3347 | 410 | 83.2 | blastp |
| 1841 | LYM25 1 H71 | triphysaria\|gb164\|EY0 17996 | 3348 | 410 | 80.7 | blastp |
| 1842 | LYM251 H72 | walnuts\|gb166\|EL8929 83 | 3349 | 410 | 80.1 | blastp |
| 1843 | LYM251 H73 | walnuts\|gb166\|EL9034 96 | 3350 | 410 | 80.1 | blastp |
| 1844 | LYM251 H74 | wheat\|gb164\|BE44435 6 | 3351 | 410 | 81.4 | blastp |
| 1845 | LYM251 H75 | wheat\|gb164\|BE49857 9 | 3352 | 410 | 81.4 | blastp |
| 1846 | LYM251 H76 | wheat\|gb164\|BQ90530 8 | 3353 | 410 | 81.4 | blastp |
| 1847 | LYM255 H3 | brachypodium\|09v1\|D V486276 | 3354 | 413 | 83.9 | blastp |
| 1848 | LYM255 H4 | maize\|gb170\|AA072446 | 3355 | 413 | 80.8 | blastp |
| 1849 | LYM255 H5 | sorghum\|09v1\|SB04G 034000 | 3356 | 413 | 82 | blastp |
| 1850 | LYM255 H3 | switchgrass\|gb167\|FE6 06184 | 3357 | 413 | 83.8 | blastp |
| 1851 | LYM260 H0 | rice\|gb170\|OSMG388 00 | 3358 | 414 | 80.5 | blastp |
| 1852 | LYM263 | maize\|gb170\|AI67385 | 3359 | 416 | 87.47 | tblastn |
| 1853 | LYM183 H8 | brachypodium\|09v1\|D V474476 | 3360 | 417 | 90.5 | blastp |
| 1853 | LYM 183 H8 | brachypodium\|09v1\|D V474476 | 3360 | 464 | 90.5 | blastp |
| 1854 | LYM 183 H1 | cenchrus\|gb166\|EB655 853 | 3361 | 417 | 83.5 | blastp |
| 1854 | LYM 183 H1 | cenchrus\|gb166\|EB655 853 | 3361 | 464 | 83.5 | blastp |
| 1855 | LYM183 H2 | leymus\|gb166\|EG3757 19 | 3362 | 417 | 94.2 | blastp |
| 1855 | LYM183 H2 | leymus\|gb166\|EG3757 19 | 3362 | 464 | 94.2 | blastp |
| 1856 | LYM183 H9 | maize\|gb 170\|AI96467 3 | 3363 | 417 | 85.2 | blastp |
| 1856 | LYM183 H9 | maize\|gb170\|AI96467 3 | 3363 | 464 | 85.2 | blastp |
| 1857 | LYM183 H10 | rice\|gb170\|OS03G607 80 | 3364 | 417 | 84.8 | blastp |
| 1857 | LYM183 H10 | rice\|gb170\|OS03G607 80 | 3364 | 464 | 84.8 | blastp |
| 1858 | LYM183 H11 | sorghum\|09v1\|SB01G 003070 | 3365 | 417 | 84.4 | blastp |
| 1858 | LYM183 H11 | sorghum\|09v1\|SB01G 003070 | 3365 | 464 | 84.4 | blastp |
| 1859 | LYM183 H12 | sugarcane\|gb157.3\|CA 111823 | 3366 | 417 | 83.6 | blastp |
| 1859 | LYM 183 H12 | sugarcane\|gb157.3\|CA 111823 | 3366 | 464 | 83.6 | blastp |
| 1860 | LYM183 H6 | switchgrass\|gb167\|DN 151763 | 3367 | 417 | 83.4 | blastp |
| 1860 | LYM183 H6 | switchgrass\|gb167\|DN 151763 | 3367 | 464 | 83.4 | blastp |
| 1861 | LYM183 H7 | wheat\|gb164\|BE51561 6 | 3368 | 417 | 93.9 | tblastn |
| 1861 | LYM183 H7 | wheat\|gb 164\|BE51561 6 | 3368 | 464 | 93.63 | tblastn |
| 1862 | LYM183 H8 | wheat\|gb164\|BQ16080 | 3369 | 417 | 94.7 | blastp |
| 1862 | LYM183 H8 | wheat\|gb164\|BQ16080 3 | 3369 | 464 | 94.7 | blastp |
| 1863 | LYM256 H2 | rice\|gb170\|OS05G451 10 | 3370 | 418 | 87.3 | blastp |
| 1864 | LYM256 H3 | rice\|gb170\|OS10G079 70 | 3371 | 418 | 81.09 | tblastn |
| 1865 | LYM200 H0 | sorghum\|09v1\|SB04G 005600 | 3372 | 419 | 86.5 | btastp |
| 1866 | LYM267 H1 | sorghum\|09v1\|SB01G 044240 | 3373 | 420 | 88.3 | blastp |
| 1867 | LYM268 H1 | sugarcane\|gb157.3\|CA 079076 | 3374 | 421 | 81.6 | blastp |
| 1868 | LYM270 H0 | sorghum\|09v1\|SB02G 040045 | 3375 | 422 | 82.3 | blastp |
| 1869 | LYM271 H7 | barley\|gb157SOLEXA\|BG344953 | 3376 | 423 | 89.9 | blastp |
| 1870 | LYM271 H8 | brachypodium\|09v1\|G T838823 | 3377 | 423 | 89.9 | blastp |
| 1871 | LYM271 H9 | rice\|gb170\|OS07G434 60 | 3378 | 423 | 91.1 | blastp |
| 1872 | LYM271 H10 | sorghum\|09v1\|SB02G 040020 | 3379 | 423 | 96.5 | blastp |
| 1873 | LYM271 H11 | sugarcane\|gb157.3\|CA 118167 | 3380 | 423 | 89.53 | tblastn |
| 1874 | LYM271 H6 | switchgrass\|gb167\|FL6 91032 | 3381 | 423 | 94.2 | blastp |
| 1875 | LYM271 H7 | wheat\|gb164\|CJ66420 9 | 3382 | 423 | 91.5 | blastp |
| 1876 | LYM273 H4 | brachypodium\|09v1\|D V469687 | 3383 | 425 | 83.3 | blastp |
| 1877 | LYM273 H5 | maize\|gb170\|AW3559 78 | 3384 | 425 | 85.2 | blastp |
| 1878 | LYM273 H6 | sorghum\|09v1\|SB03G 044410 | 3385 | 425 | 84 | blastp |
| 1879 | LYM273 H4 | wheat\|gb164\|BE51837 7 | 3386 | 425 | 85 | blastp |
| 1880 | LYM274 H0 | rice\|gb170\|OS01G702 40 | 3387 | 426 | 100 | blastp |
| 1880 | LYM274 H0 | rice\|gb170\|OS01G702 40 | 3387 | 466 | 89.2 | blastp |
| 1881 | LYM278 H9 | maize\|gb170\|LLDQ24 4681 | 3388 | 428 | 95.2 | blastp |
| 1882 | LYM278 H2 | rye\|gb164\|Z23257 | 3389 | 428 | 92.86 | tblastn |
| 1883 | LYM278 H3 | wheat\|gb164\|AL82126 4 | 3390 | 428 | 95.3 | blastp |
| 1884 | LYM278 H4 | wheat\|gb164\|BE51672 3 | 3391 | 428 | 94 | blastp |
| 1885 | LYM278 H5 | wheat\|gb164\|BF20040 2 | 3392 | 428 | 86.9 | tblastn |
| 1886 | LYM278 H6 | wheat\|gb164\|BF47442 3 | 3393 | 428 | 92.86 | tblastn |
| 1887 | LYM278 H7 | wheat\|gb164\|BG90946 2 | 3394 | 428 | 89.3 | blastp |
| 1888 | LYM278 H8 | wheat\|gb164\|BQ57909 7 | 3395 | 428 | 95.24 | tblastn |
| 1889 | LYM278 H9 | wheat\|gb164\|CA65034 9 | 3396 | 428 | 83.3 | blastp |
| 1890 | LYM284 H16 | barley\|gb 157SOLEXA \|BE438857 | 3397 | 430 | 86.4 | blastp |
| 1891 | LYM284 H17 | brachypodium\|09v1\|D V474685 | 3398 | 430 | 84.3 | blastp |
| 1892 | LYM284 H18 | brachypodium\|09v1\|D V488161 | 3399 | | 88.2 | blastp |
| 1893 | LYM284 H3 | fescue\|gb161\|DT6744 46 | 3400 | | 86.91 | tblastn |
| 1894 | LYM284 H19 | maize\|gb 170\|AI63725 6 | 3401 | | 88.5 | blastp |
| 1895 | LYM284 H20 | maize\|gb170\|AI86113 1 | 3402 | 430 | 84.3 | blastp |
| 1896 | LYM284 H21 | maize\|gb170\|BM5012 76 | 3403 | | 83.8 | blastp |
| 1897 | LYM284 H22 | rice\|gb170\|OS01G430 20 | 3404 | | 81.4 | blastp |
| 1898 | LYM284 H23 | rice\|gb 170\|OS01G465 70 | 3405 | 430 | 84.6 | blastp |
| 1899 | LYM284 H24 | sorghum\|09v1\|SB03G 027960 | 3406 | 430 | 80.6 | blastp |
| 1900 | LYM284 H25 | sorghum\|09v1\|SB03G 029790 | 3407 | | 85.1 | blastp |
| 1901 | LYM284 H26 | sorghum\|09v1\|SB09G 029130 | 3408 | 430 | 87.2 | blastp |
| 1902 | LYM284 H27 | sugarcane\|gb157.3\|BQ 533889 | 3409 | 430 | 84.5 | blastp |
| 1903 | LYM284 H12 | switchgrass\|gb167\|DN 151636 | 3410 | 430 | 81 | blastp |
| 1904 | LYM284 H13 | switchgrass\|gb1671FE6 34580 | 3411 | | 85.4 | blastp |
| 1905 | LYM284 H14 | switchgrass\|gb167\|FL7 12120 | 3412 | | 89.53 | tblastn |
| 1906 | LYM284 H15 | wheat\|gb164\|BE40078 9 | 3413 | | 88.1 | blastp |
| 1907 | LYM284 H16 | wheat\|gb164\|BF20080 4 | 3414 | 430 | 80.4 | blastp |
| 1908 | LYM285 H3 | brachypodium\|09v1\|D V476342 | 3415 | 431 | 89.2 | blastp |
| 1909 | LYM285 H4 | maize\|gb 170\|AI37229 8 | 3416 | 431 | 88.1 | blastp |
| 1910 | LYM285 H5 | rice\|gb170\|OS01G699 00 | 3417 | 431 | 99.82 | tblastn |
| 1911 | LYM285 H6 | sorghum\|09v1\|SB03G 044210 | 3418 | 431 | 89.3 | blastp |
| 1912 | LYM285 H3 | switchgrass\|gb167\|DN 142770 | 3419 | 431 | 89.75 | tblastn |
| 1913 | LYM288 H6 | brachypodium\|09v1\|D V471350 | 3420 | 433 | 83.3 | blastp |
| 1914 | LYM288 H1 | leymus\|gb166\|EG3779 96 | 3421 | 433 | 84.7 | blastp |
| 1915 | LYM288 H7 | maize\|gb 170\|AI97792 4 | 3422 | 433 | 84.5 | blastp |
| 1916 | LYM288 H8 | sorghum\|09v1\|SB02G 039240 | 3423 | 433 | 83.9 | blastp |
| 1917 | LYM288 H9 | sugarcane\|gb157.3\|CA 067537 | 3424 | 433 | 85.8 | blastp |
| 1918 | LYM288 H5 | switchgrass\|gb167\|DN 151710 | 3425 | 433 | 85.3 | blastp |
| 1919 | LYM288 H6 | switchgrass\|gb167\|FE6 10990 | 3426 | 433 | 85.3 | blastp |
| 1920 | LYM289 H39 | barley\|gb157SOLEXA \|AL500321 | 3427 | 434 | 91.1 | blastp |
| 1921 | LYM289 H40 | barley\|gb157SOLEXA \|AV915627 | 3428 | 434 | 92.7 | blastp |
| 1922 | LYM289 H41 | barley\|gb157SOLEXA \|BF624195 | 3429 | 434 | 98.2 | blastp |
| 1923 | LYM289 H42 | barley\|gb157SOLEXA \|BF627133 | 3430 | 434 | 92.7 | blastp |
| 1924 | LYM289 H43 | barley\|gb 157SOLEXA \|BQ739963 | 3431 | 434 | 92.7 | blastp |
| 1925 | LYM289 H44 | brachypodium\|09v1\|G FXEF059989X41 | 3432 | 434 | 81.8 | blastp |
| 1926 | LYM289 H7 | cacao\|gb167\|CU56893 3 | 3433 | 434 | 85.5 | blastp |
| 1927 | LYM289 H8 | fescue\|gb161\|CK8015 01 | 3434 | 434 | 90.9 | blastp |
| 1928 | LYM289 H9 | fescue\|gb161\|DT6836 63 | 3435 | 434 | 90.9 | blastp |
| 1929 | LYM289 H10 | leymus\|gb166\|EG3762 87 | 3436 | 434 | 96.4 | blastp |
| 1930 | LYM289 H45 | lolium\|09v1\|AU24668 3 | 3437 | 434 | 89.1 | blastp |
| 1931 | LYM289 H11 | lovegrass\|gb167\|DN48 0351 | 3438 | 434 | 87.3 | blastp |
| 1932 | LYM289 H46 | maize\|gb170\|AI63724 2 | 3439 | 434 | 81.8 | blastp |
| 1933 | LYM289 H47 | maize\|gb170\|LLEC86 5320 | 3440 | 434 | 81.8 | blastp |
| 1934 | LYM289 H48 | maize\|gb170\|T12715 | 3441 | 434 | 87.3 | blastp |
| 1935 | LYM289 H49 | millet\|09v1\|CD724594 | 3442 | 434 | 80 | blastp |
| 1936 | LYM289 H50 | millet\|09v 1\|EB410971 | 3443 | 434 | 87.3 | blastp |
| 1937 | LYM289 H15 | oat\|gb164\|CN818354 | 3444 | 434 | 90.9 | blastp |
| 1938 | LYM289 H16 | pineapple\|gb 157.2\|DT 337702 | 3445 | 434 | 80 | blastp |
| 1939 | LYM289 H51 | rice\|19b170\|OS06G051 20 | 3446 | 434 | 83.6 | blastp |
| 1940 | LYM289 H52 | sorghum\|09v1\|SB09G 005410 | 3447 | 434 | 80 | blastp |
| 1941 | LYM289 H53 | sorghum\|09v1\|SB10G 002980 | 3448 | 434 | 87.3 | blastp |
| 1942 | LYM289 H54 | sugarcane\|gb157.3\|CA 117495 | 3449 | 434 | 87.3 | blastp |
| 1943 | LYM289 H55 | sugarcane\|gb157.3\|CA 149658 | 3450 | 434 | 81.8 | blastp |
| 1944 | LYM289 H56 | sugarcane\|gb157.3\|CF 575668 | 3451 | 434 | 87.3 | blastp |
| 1945 | LYM289 H23 | switchgrass\|gb167\|DN 144776 | 3452 | 434 | 80 | blastp |
| 1946 | LYM289 H24 | switchgrass\|gb167\|DN 144989 | 3453 | 434 | 89.1 | blastp |
| 1947 | LYM289 H25 | switchgrass\|gb167\|FE6 07508 | 3454 | 434 | 89.1 | blastp |
| 1948 | LYM289 H26 | switchgrass\|gb167\|FL7 36037 | 3455 | 434 | 81.8 | blastp |
| 1949 | LYM289 H27 | wheal\|gb164\|AL81111 9 | 3456 | 434 | 98.2 | blastp |
| 1950 | LYM289 H28 | wheat\|gb164\|BE44378 6 | 3457 | 434 | 96.4 | blastp |
| 1951 | LYM289 H29 | wheat\|gb164\|BG90709 8 | 3458 | 434 | 98.2 | blastp |
| 1952 | LYM289 H30 | wheat\|gb164\|BM1365 71 | 3459 | 434 | 96.4 | blastp |
| 1953 | LYM289 H31 | wheat\|gb164\|BQ80426 1 | 3460 | 434 | 96.4 | blastp |
| 1954 | LYM289 H32 | wheat\|gb164\|CA61658 6 | 3461 | 434 | 81.8 | blastp |
| 1955 | LYM289 H33 | wheat\|gb164\|CA64017 8 | 3462 | 434 | 98.2 | blastp |
| 1956 | LYM289 H34 | wheat\|gb164\|CA64378 4 | 3463 | 434 | 98.2 | blastp |
| 1957 | LYM289 H35 | wheat\|gb164\|CA73397 2 | 3464 | 434 | 80.36 | tblastn |
| 1958 | LYM289 H36 | wheat\|gb164\|CD89687 3 | 3465 | 434 | 82.1 | blastp |
| 1959 | LYM289 H37 | wheat\|gb164\|CJ58670 9 | 3466 | 434 | 89.1 | blastp |
| 1960 | LYM289 H38 | wheat\|gb164\|CJ64697 0 | 3467 | 434 | 80 | blastp |
| 1961 | LYM289 H39 | wheat\|gb164\|CJ9O337 8 | 3468 | 434 | 96.4 | blastp |
| 1962 | LYM290 H10 | barley\|gb 157SOLEXA \|BE412989 | 3469 | 435 | 82.9 | blastp |
| 1963 | LYM290 H11 | brachypodium\|09v1\|G T759831 | 3470 | 435 | 81.9 | blastp |
| 1964 | LYM290 H12 | rice\|gb170\|OS04G202 30 | 3471 | 435 | 82.9 | blastp |
| 1965 | LYM290 H3 | rye\|gb164\|BE495099 | 3472 | 435 | 81.9 | blastp |
| 1966 | LYM290 H13 | sorghum\|09v1\|SB01G 009390 | 3473 | 435 | 95.2 | blastp |
| 1967 | LYM290 H14 | sorghum\|09v1\|SB06G 004500 | 3474 | 435 | 94.8 | blastp |
| 1968 | LYM290 H15 | sugarcane\|gb157.3\|BQ 535968 | 3475 | 435 | 95.7 | blastp |
| 1969 | LYM290 H16 | sugarcane\|gb157.3\|CA 136629 | 3476 | 435 | 80.3 | blastp |
| 1970 | LYM290 H8 | switchgrass\|gb167\|FE6 22978 | 3477 | 435 | 91.4 | blastp |
| 1971 | LYM290 H9 | wheat\|gb164\|BE43009 0 | 3478 | 435 | 83.3 | blastp |
| 1972 | LYM290 H10 | wheat\|gb164\|BF19952 4 | 3479 | 435 | 82.4 | blastp |
| 1973 | LYM293 H0 | rice\|gb170\|OS09G385 10 | 3480 | 437 | 91.8 | blastp |

Table 2: Provided are the homologous polypeptides and polynucleotides of the genes for increasing yield (e.g., oil yield, seed yield, fiber yield and/or quality), growth rate, vigor, biomass, abiotic stress tolerance, nitrogen use efficiency, water use efficiency and fertilizer use efficiency genes of a plant which are listed in Table 1 above. Homology was calculated as % of identity over the aligned sequences. The query sequences were polynucleotide sequences SEQ ID NOs: 1-239 and polypeptide SEQ ID NOs:240-465 and the subject sequences are protein sequences identified in the database based on greater than 80 % identity to the predicted translated sequences of the query nucleotide sequences or to the polypeptide sequences. "Nucl." = polynucleotide; "polyp." = polypeptide; "Algor." = algorithm (used for sequence alignment and determination of percent homology).

The following sequences were found to be 100 % identical: SEQ ID NO: 4 is identical to SEQ ID NO: 478; SEQ ID NO: 24 is identical to SEQ ID NO: 914; SEQ ID NO: 79 is identical to SEQ ID NO: 1050; SEQ ID NO: 132 is identical to SEQ ID NO: 3608; SEQ ID NO: 163 is identical to SEQ ID NO: 1734; SEQ ID NO: 249 is identical to SEQ ID NO: 2100, 2101, 2103, and 2108; SEQ ID NO: 321 is identical to SEQ ID NO: 2771; SEQ ID NO: 382 is identical to SEQ ID NO: 3019; SEQ ID NO: 387 is identical to SEQ ID NO: 2945; SEQ ID NO: 426 is identical to SEQ ID NO: 3387; SEQ ID NO: 446 is identical to SEQ ID NO: 2946; SEQ ID NO: 449 is identical to SEQ ID NO: 3705; SEQ ID NO: 2005 is identical to SEQ ID NO: 2011 and 2012; SEQ ID NO: 2007 is identical to SEQ ID NO: 2043 and 2045; SEQ ID NO: 2038 is identical to SEQ ID NO: 2039; SEQ ID NO: 2071 is identical to SEQ ID NO: 2072; SEQ ID NO: 2075 is identical to SEQ ID NO:2076, 2078, 2079, 2083, 2084, 2086, 2089, 2090, 2092, 2093, 2095, 2120, 2122, 2235, 2236, 2238, 2239, 2277, and 2278; SEQ ID NO: 2080 is identical to SEQ ID NO: 2155, 2176, 2179, 2248, and 2268; SEQ ID NO: 2102 is identical to SEQ ID NO: 2193; SEQ ID NO: 2105 is identical to SEQ ID NO: 2147, 2181, 2196, 2197, 2210, 2211, 2213, 2254, and 2256; SEQ ID NO: 2125 is identical to SEQ ID NO: 2126 and 2127; SEQ ID NO: 2130 is identical to SEQ ID NO: 2131, 2214, 2228, 2231, and 2251; SEQ ID NO: 2134 is identical to SEQ ID NO: 2247; SEQ ID NO: 2144 is identical to SEQ ID NO: 2153 and 2201; SEQ ID NO: 2188 is identical to SEQ ID NO: 2191, 2253, 2264, and 2271; SEQ ID NO: 2189 is identical to SEQ ID NO: 2202, 2252, 2265, 2266, 2270, and 2272; SEQ ID NO: 2215 is identical to SEQ ID NO: 2250 and 2284; SEQ ID NO: 2218 is identical to SEQ ID NO: 2219; SEQ ID NO: 2220 is identical to SEQ ID NO: 2221 and 2258; SEQ ID NO: 2356 is identical to SEQ ID NO: 2357 and 2359; SEQ ID NO: 2380 is identical to SEQ ID NO: 2402; SEQ ID NO: 2384 is identical to SEQ ID NO: 2387; SEQ ID NO: 2463 is identical to SEQ ID NO:2464; SEQ ID NO: 2481 is identical to SEQ ID NO: 2483; SEQ ID NO: 2485 is identical to SEQ ID NO: 2486; SEQ ID NO: 2533 is identical to SEQ ID NO: 2538; SEQ ID NO: 2582 is identical to SEQ ID NO: 2583; SEQ ID NO: 2588 is identical to SEQ ID NO: 2594, 2603, 2621, and 2629; SEQ ID NO: 2589 is identical to SEQ ID NO:2590, 2591, 2592, 2595, 2596, 2601, 2604, 2605, 2623, 2624, 2626, 2627, 2630, 2756, 2757, 2758, 2760, 2761, 2762, 2764, 2765, and 2807; SEQ ID NO:2593 is identical to SEQ ID NO:2632, and 2767; SEQ ID NO: 2600 is identical to SEQ ID NO: 2622; SEQ ID NO: 2606 is identical to SEQ ID NO: 2610; SEQ ID NO: 2607 is identical to SEQ ID NO: 2609; SEQ ID NO: 2625 is identical to SEQ ID NO: 2713; SEQ ID NO: 2638 is identical to SEQ ID NO: 2639; SEQ ID NO: 2644 is identical to SEQ ID NO: 2727; SEQ ID NO: 2645 is identical to SEQ ID NO: 2726; SEQ ID NO: 2665 is identical to SEQ ID NO: 2719; SEQ ID NO: 2687 is identical to SEQ ID NO: 2689; SEQ ID NO: 2691 is identical to SEQ ID NO: 2693 and 2698; SEQ ID NO: 2694 is identical to SEQ ID NO: 2697; SEQ ID NO: 2712 is identical to SEQ ID NO: 2816, 2817 and 2818; SEQ ID NO: 2748 is identical to SEQ ID NO: 2749, 2778 and 2815; SEQ ID NO: 2750 is identical to SEQ ID NO: 2751 and 2776; SEQ ID NO: 2779 is identical to SEQ ID NO: 2790 and 2794; SEQ ID NO: 2801 is identical to SEQ ID NO: 2804; SEQ ID NO: 2873 is identical to SEQ ID NO: 2880; SEQ ID NO: 2876 is identical to SEQ ID NO: 2881; SEQ ID NO: 2877 is identical to SEQ ID NO: 2879; SEQ ID NO: 2908 is identical to SEQ ID NO: 2909; SEQ ID NO: 3135 is identical to SEQ ID NO: 3136; SEQ ID NO: 3138 is identical to SEQ ID NO: 3145; SEQ ID NO: 3268 is identical to SEQ ID NO: 3271, 3272, 3278, 3326, and 3327; SEQ ID NO: 3274 is identical to SEQ ID NO: 3351, 3352, and 3353; SEQ ID NO: 3277 is identical to SEQ ID NO: 3296; SEQ ID NO: 3285 is identical to SEQ ID NO: 3313; SEQ ID NO: 3287 is identical to SEQ ID NO: 3302; SEQ ID NO: 3309 is identical to SEQ ID NO: 3333, 3337, 3338, and 3342; SEQ ID NO: 3318 is identical to SEQ ID NO: 3319; SEQ ID NO: 3322 is identical to SEQ ID NO: 3331; SEQ ID NO: 3428 is identical to SEQ ID NO: 3430 3431; SEQ ID NO: 3434 is identical to SEQ ID NO: 3435; SEQ ID NO: 3439 is identical to SEQ ID NO: 3440 and 3461; SEQ ID NO: 3448 is identical to SEQ ID NO: 3449 and 3451; SEQ ID NO: 3453 is identical to SEQ ID NO: 3454; SEQ ID NO: 3456 is identical to SEQ ID NO: 3458, 3462 and 3463; SEQ ID NO: 3457 is identical to SEQ ID NO: 3459 and 3468;

The output of the functional genomics approach described herein is a set of genes highly predicted to improve yield and/or other agronomic important traits such as growth rate, vigor, oil content, fiber yield and/or quality, biomass, growth rate, abiotic stress tolerance, nitrogen use efficiency, water use efficiency and fertilizer use efficiency of a plant by increasing their expression. Although each gene is predicted to have its own impact, modifying the mode of expression of more than one gene is expected to provide an additive or synergistic effect on the plant yield and/or other agronomic important yields performance. Altering the expression of each gene described here alone or set of genes together increases the overall yield and/or other agronomic important traits, hence expects to increase agricultural productivity.

### EXAMPLE 3

### PRODUCTION OF BARLEY TRANSCRIPTOM AND HIGH THROUGHPUT CORRELATION ANALYSIS USING 44K BARLEY OLIGONUCLEOTIDE MICRO-ARRAY

In order to produce a high throughput correlation analysis, the present inventors utilized a Barley oligonucleotide micro-array, produced by Agilent Technologies [Hypertext Transfer Protocol://World Wide Web (dot) chem. (dot) agilent (dot) com/Scripts/PDS (dot) asp?lPage=50879]. The array oligonucleotide represents about 47,500 Barley genes and transcripts. In order to define correlations between the levels of RNA expression and yield or vigor related parameters, various plant characteristics of 25 different Barley accessions were analyzed. Among them, 13 accessions encompassing the observed variance were selected for RNA expression analysis. The correlation between the RNA levels and the characterized parameters was analyzed using Pearson correlation test [Hypertext Transfer Protocol://World Wide Web (dot) davidmlane (dot) com/hyperstat/A34739 (dot) html].

### Experimental procedures

***RNA extraction*** - Five tissues at different developmental stages [meristem, flower, booting spike, stem, flag leaf], representing different plant characteristics, were sampled and RNA was extracted using TRIzol Reagent from Invitrogen [Hypertext Transfer Protocol://World Wide Web (dot) invitrogen (dot) com/content (dot)cfm?pageid=469]. Approximately 30-50 mg of tissue was taken from samples. The weighed tissues were ground using pestle and mortar in liquid nitrogen and resuspended in 500 µl of TRIzol Reagent. To the homogenized lysate, 100 µl of chloroform was added followed by precipitation using isopropanol and two washes with 75 % ethanol. The RNA was eluted in 30 µl of RNase-free water. RNA samples were cleaned up using Qiagen's RNeasy minikit clean-up protocol as per the manufacturer's protocol (QIAGEN Inc, CA USA).

For convenience, each micro-array expression information tissue type has received a Set ID as summarized in Table 3 below.

**Table 3**

| ***Barley transcriptom expression sets*** | |
|---|---|
| ***Expression Set*** | ***Set ID*** |
| Meristem | A |
| Flower | B |
| Booting spike | C |
| Stem | D |
| Flag leaf | E |

Table 3

***Barley yield components and vigor related parameters assessment* -** 25 Barley accessions in 4 repetitive blocks (named A, B, C, and D), each containing 4 plants per plot were grown at net house. Plants were phenotyped on a daily basis following the standard descriptor of barley (Table 4, below). Harvest was conducted while 50 % of the spikes were dry to avoid spontaneous release of the seeds. Plants were separated to the vegetative part and spikes, of them, 5 spikes were threshed (grains were separated from the glumes) for additional grain analysis such as size measurement, grain count per spike and grain yield per spike. All material was oven dried and the seeds were threshed manually from the spikes prior to measurement of the seed characteristics (weight and size) using scanning and image analysis. The image analysis system included a personal desktop computer (Intel P4 3.0 GHz processor) and a public domain program - ImageJ 1.37 (Java based image processing program, which was developed at the U.S. National Institutes of Health and freely available on the internet [Hypertext Transfer Protocol://rsbweb (dot) nih (dot) gov/]. Next, analyzed data was saved to text files and processed using the JMP statistical analysis software (SAS institute).

**Table 4**

| ***Barley standard descriptors*** | | | |
|---|---|---|---|
| ***Trait*** | ***Parameter*** | ***Range*** | ***Description*** |
| Growth habit | Scoring | 1-9 | Prostrate (1) or Erect (9) |
| Hairiness of basal leaves | Scoring | P (Presence)/A (Absence) | Absence (1) or Presence (2) |
| Stem pigmentation | Scoring | 1-5 | Green (1), Basal only or Half or more (5) |
| Days to Flowering | Days | | Days from sowing to emergence of awns |
| Plant height | Centimeter (cm) | | Height from ground level to top of the longest spike excluding awns |
| Spikes per plant | Number | | Terminal Counting |
| Spike length | Centimeter (cm) | | Terminal Counting 5 spikes per plant |
| Grains per spike | Number | | Terminal Counting 5 spikes per plant |
| Vegetative dry weight | Gram | | Oven-dried for 48 hours at 70°C |
| Spikes dry weight | Gram | | Oven-dried for 48 hours at 30°C |

Table 4.

***Grains per spike* -** At the end of the experiment (50 % of the spikes were dry) all spikes from plots within blocks A-D are collected. The total number of grains from 5 spikes that were manually threshed was counted. The average grain per spike is calculated by dividing the total grain number by the number of spikes.

***Grain average size (cm)*** - At the end of the experiment (50 % of the spikes were dry) all spikes from plots within blocks A-D are collected. The total grains from 5 spikes that were manually threshed were scanned and images were analyzed using the digital imaging system. Grain scanning was done using Brother scanner (model DCP-135), at the 200 dpi resolution and analyzed with Image J software. The average grain size was calculated by dividing the total grain size by the total grain number.

***Grain average weight* (*mgr*)** - At the end of the experiment (50 % of the spikes were dry) all spikes from plots within blocks A-D are collected. The total grains from 5 spikes that were manually threshed were counted and weight. The average weight was calculated by dividing the total weight by the total grain number.

***Grain yield per spike* (*gr*)** - At the end of the experiment (50 % of the spikes were dry) all spikes from plots within blocks A-D are collected. The total grains from 5 spikes that were manually threshed were weight. The grain yield was calculated by dividing the total weight by the spike number.

***Spike length analysis*** - At the end of the experiment (50 % of the spikes were dry) all spikes from plots within blocks A-D are collected. The five chosen spikes per plant were measured using measuring tape excluding the awns.

***Spike number analysis*** - At the end of the experiment (50 % of the spikes were dry) all spikes from plots within blocks A-D are collected. The spikes per plant were counted.

***Growth habit scoring*** - At the growth stage 10 (booting), each of the plants was scored for its growth habit nature. The scale that was used was 1for prostate nature till 9 for erect.

***Hairiness of basal leaves* -** At the growth stage 5 (leaf sheath strongly erect; end of tillering), each of the plants was scored for its hairiness nature of the leaf before the last. The scale that was used was 1for prostate nature till 9 for erect.

***Plant height* -** At the harvest stage (50 % of spikes were dry) each of the plants was measured for its height using measuring tape. Height was measured from ground level to top of the longest spike excluding awns.

***Days to flowering*** - Each of the plants was monitored for flowering date. Days of flowering was calculated from sowing date till flowering date.

***Stem pigmentation*** - At the growth stage 10 (booting), each of the plants was scored for its stem color. The scale that was used was 1for green till 5 for full purple.

***Vegetative dry weight and spike yield*** - At the end of the experiment (50 % of the spikes were dry) all spikes and vegetative material from plots within blocks A-D are collected. The biomass and spikes weight of each plot was separated, measured and divided by the number of plants.

***Dry weight*** = total weight of the vegetative portion above ground (excluding roots) after drying at 70 °C in oven for 48 hours;
***Spike yield per plant*** = total spike weight per plant (gr) after drying at 30 °C in oven for 48 hours.

***Harvest Index (for barley)*** - The harvest index is calculated using Formula VI. $Harvest Index = Average spike dry weight per plant / \left(Average vegetative dry weight per plant + Average spike dry weight per plant\right)$

**Table 5**

| ***Barley correlated parameters (vectors)*** | |
|---|---|
| ***Correlated parameter with (units)*** | ***Correlation Id*** |
| Grains per spike (numbers) | 1 |
| Grains size (mm²) | 2 |
| Grain weight (miligrams) | 3 |
| Grain Yield per spike (gr/spike) | 4 |
| Spike length (cm) | 5 |
| Spikes per plant (numbers) | 6 |
| Growth habit (scores 1-9) | 7 |
| Hairiness of basal leaves (scoring 1-2) | 8 |
| Plant height (cm) | 9 |
| Days to flowering (days) | 10 |
| Stem pigmentation (scoring 1-5) | 11 |
| Vegetative dry weight (gram) | 12 |
| Harvest Index (ratio) | 13 |

Table 5.

### Experimental Results

13 different Barley accessions were grown and characterized for 13 parameters as described above. The average for each of the measured parameter was calculated using the JMP software and values are summarized in Tables 6 and 7 below. Subsequent correlation analysis between the various transcriptom sets (Table 3) and the average parameters, was conducted. Follow, results were integrated to the database.

**Table 6**

| | | | | | | | |
|---|---|---|---|---|---|---|---|
| ***Measured parameters of correlation Ids in Barley accessions*** | | | | | | | |

| ***Accession* /*Parameter*** | ***6*** | ***10*** | ***3*** | ***5*** | ***2*** | ***1*** | ***7*** |
|---|---|---|---|---|---|---|---|
| Amatzya | 48.85 | 62.40 | 35.05 | 12.04 | 0.27 | 20.23 | 2.60 |
| Ashqelon | 48.27 | 64.08 | 28.06 | 10.93 | 0.23 | 17.98 | 2.00 |
| Canada park | 37.42 | 65.15 | 28.76 | 11.83 | 0.24 | 17.27 | 1.92 |
| Havarim stream | 61.92 | 58.92 | 17.87 | 9.90 | 0.17 | 17.73 | 3.17 |
| Jordan est | 33.27 | 63.00 | 41.22 | 11.68 | 0.29 | 14.47 | 4.33 |
| Klil | 41.69 | 70.54 | 29.73 | 11.53 | 0.28 | 16.78 | 2.69 |
| Maale Efraim | ND | 52.80 | 25.22 | 8.86 | 0.22 | 13.47 | 3.60 |
| Mt Arbel | 40.63 | 60.88 | 34.99 | 11.22 | 0.28 | 14.07 | 3.50 |
| Mt Harif | 62.00 | 58.10 | 20.58 | 11.11 | 0.19 | 21.54 | 3.00 |
| Neomi | 49.33 | 53.00 | 27.50 | 8.58 | 0.22 | 12.10 | 3.67 |
| Neot Kdumim | 50.60 | 60.40 | 37.13 | 10.18 | 0.27 | 14.36 | 2.47 |
| Oren canyon | 43.09 | 64.58 | 29.56 | 10.51 | 0.27 | 15.28 | 3.50 |
| Yeruham | 51.40 | 56.00 | 19.58 | 9.80 | 0.18 | 17.07 | 3.00 |

Table 6. Provided are the values of each of the parameters measured in Barley accessions according to the following correlation identifications (Correlation Ids): 6 = Spikes per plant; 10 = Days to flowering; 3 = Grain weight; 5 = Spike length; 2 = Grains Size; 1 = Grains per spike; 7 = Growth habit.

**Table 7**

| ***Barley accessions, additional measured parameters*** | | | | | | |
|---|---|---|---|---|---|---|
| ***Accession* /*Parameter*** | ***8*** | ***9*** | ***4*** | ***11*** | ***12*** | ***13*** |
| Amatzya | 1.53 | 134.27 | 3.56 | 1.13 | 78.87 | 0.45 |
| Ashqelon | 1.33 | 130.50 | 2.54 | 2.50 | 66.14 | 0.42 |
| Canada park | 1.69 | 138.77 | 2.58 | 1.69 | 68.49 | 0.40 |
| Havarim stream | 1.08 | 114.58 | 1.57 | 1.75 | 53.39 | 0.44 |
| Jordan est | 1.42 | 127.75 | 3.03 | 2.33 | 68.30 | 0.43 |
| Klil | 1.69 | 129.38 | 2.52 | 2.31 | 74.17 | 0.40 |
| Maale Efraim | 1.30 | 103.89 | 1.55 | 1.70 | 35.35 | 0.52 |
| Mt Arbel | 1.19 | 121.63 | 2.62 | 2.19 | 58.33 | 0.48 |
| Mt Harif | 1.00 | 126.80 | 2.30 | 2.30 | 62.23 | 0.44 |
| Neomi | 1.17 | 99.83 | 1.68 | 1.83 | 38.32 | 0.49 |
| Neot Kdumim | 1.60 | 121.40 | 2.68 | 3.07 | 68.31 | 0.45 |
| Oren canyon | 1.08 | 118.42 | 2.35 | 1.58 | 56.15 | ND |
| Yeruham | 1.17 | 117.17 | 1.67 | 2.17 | 42.68 | ND |

Table 7. Provided are the values of each of the parameters measured in Barley accessions according to the following correlation identifications (Correlation Ids): 8 = Hairiness of basal leaves; 9 = Plant height; 4 = Grain yield per spike; 11 = Stem pigmentation; 12 = Vegetative dry weight; 13 = Harvest Index.

**Table 8**

| ***Correlation between the expression level of the selected polynucleotides and their homologues in specific tissues or developmental stages and the phenotypic performance across Barley ecotypes*** | | | | |
|---|---|---|---|---|
| **Gene Name** | **Expression Set** | **Correlation Vector** | **R** | **P** |
| LYM26 | A | 12 | 0.87938 | 0.00178 |
| LYM26 | A | 4 | 0.86421 | 0.00265 |
| LYM26 | A | 12 | 0.85977 | 0.00069 |
| LYM26 | A | 4 | 0.84991 | 0.00092 |
| LYM26 | A | 9 | 0.83315 | 0.00145 |
| LYM26 | A | 9 | 0.81930 | 0.00689 |
| LYM26 | A | 5 | 0.81231 | 0.00238 |
| LYM26 | A | 5 | 0.80624 | 0.00867 |
| LYM26 | C | 1 | 0.74897 | 0.00799 |
| LYM26 | C | 1 | 0.73316 | 0.02461 |
| LYM26 | A | 10 | 0.72560 | 0.02691 |
| LYM5 1 | A | 10 | 0.93629 | 0.00020 |
| LYM51 | A | 12 | 0.92036 | 0.00043 |
| LYM51 | A | 5 | 0.88679 | 0.00144 |
| LYM51 | A | 9 | 0.87500 | 0.00201 |
| LYM51 | A | 10 | 0.85681 | 0.00075 |
| LYM51 | A | 4 | 0.82050 | 0.00674 |
| LYM51 | A | 5 | 0.78204 | 0.00445 |
| LYM51 | A | 9 | 0.77050 | 0.00552 |
| LYM51 | A | 12 | 0.74603 | 0.00838 |
| LYM51 | A | 4 | 0.71036 | 0.01430 |
| LYM59 | A | 4 | 0.88939 | 0.00133 |
| LYM59 | A | 3 | 0.80853 | 0.00834 |
| LYM59 | A | 2 | 0.80307 | 0.00915 |
| LYM59 | A | 12 | 0.79319 | 0.01075 |
| LYM59 | A | 5 | 0.73433 | 0.02426 |
| LYM59 | A | 10 | 0.72556 | 0.02693 |
| LYM66 | A | 1 | 0.77697 | 0.01376 |
| LYM66 | A | 1 | 0.75508 | 0.00722 |
| LYM82 | A | 10 | 0.88760 | 0.00140 |
| LYM82 | A | 12 | 0.86378 | 0.00061 |
| LYM82 | A | 12 | 0.85529 | 0.00329 |
| LYM82 | A | 10 | 0.84623 | 0.00102 |
| LYM82 | A | 9 | 0.83000 | 0.00562 |
| LYM82 | A | 9 | 0.82602 | 0.00173 |
| LYM82 | A | 4 | 0.81534 | 0.00222 |
| LYM82 | A | 4 | 0.79017 | 0.01127 |
| LYM82 | A | 5 | 0.78467 | 0.00424 |
| LYM82 | A | 5 | 0.76164 | 0.01709 |
| LYM84 | C | 2 | 0.79418 | 0.01058 |
| LYM84 | B | 2 | 0.79145 | 0.01928 |
| LYM84 | C | 3 | 0.77694 | 0.01377 |
| LYM84 | B | 3 | 0.75502 | 0.03033 |
| LYM84 | A | 2 | 0.70823 | 0.01473 |
| LYM99 | C | 7 | 0.75021 | 0.01989 |
| LYM99 | A | 7 | 0.72294 | 0.02776 |
| LYM95 | B | 2 | 0.81158 | 0.00436 |
| LYM95 | B | 3 | 0.77615 | 0.00830 |
| LYM95 | B | 10 | 0.73186 | 0.01612 |
| LYM95 | C | 2 | 0.72471 | 0.02719 |
| LYM95 | B | 5 | 0.71170 | 0.02097 |
| LYM100 | A | 3 | 0.77258 | 0.01467 |
| LYM100 | A | 4 | 0.76559 | 0.01619 |
| LYM100 | A | 2 | 0.72628 | 0.02670 |
| LYM105 | A | 4 | 0.80126 | 0.00943 |
| LYM105 | A | 2 | 0.80060 | 0.00953 |
| LYM105 | A | 3 | 0.78770 | 0.01171 |
| LYM 105 | A | 8 | 0.71795 | 0.02939 |
| LYM105 | B | 1 | 0.71160 | 0.04775 |
| LYM 137 | C | 5 | 0.91789 | 0.00007 |
| LYM 137 | C | 4 | 0.87211 | 0.00046 |
| LYM137 | C | 10 | 0.86290 | 0.00063 |
| LYM137 | C | 12 | 0.85934 | 0.00070 |
| LYM137 | C | 9 | 0.82372 | 0.00183 |
| LYM137 | C | 3 | 0.73788 | 0.02323 |
| LYM137 | C | 2 | 0.71562 | 0.03017 |
| LYM140 | C | 6 | 0.83623 | 0.00968 |
| LYM142 | B | 6 | 0.85850 | 0.01338 |
| LYM142 | A | 4 | 0.80126 | 0.00943 |
| LYM142 | A | 2 | 0.80060 | 0.00953 |
| LYM142 | A | 3 | 0.78770 | 0.01171 |
| LYM142 | A | 8 | 0.73208 | 0.01042 |
| LYM142 | A | 8 | 0.71795 | 0.02939 |
| LYM142 | B | 1 | 0.71160 | 0.04775 |
| LYM148 | A | 4 | 0.79887 | 0.00318 |
| LYM148 | A | 12 | 0.76743 | 0.00583 |
| LYM148 | A | 4 | 0.76342 | 0.01668 |
| LYM148 | A | 9 | 0.74349 | 0.00873 |
| LYM148 | A | 5 | 0.70612 | 0.01516 |
| LYM149 | B | 7 | 0.75092 | 0.03177 |
| LYM156 | C | 2 | 0.79406 | 0.00352 |
| LYM156 | C | 2 | 0.77724 | 0.01371 |
| LYM 156 | A | 2 | 0.76712 | 0.00586 |
| LYM 156 | A | 3 | 0.73707 | 0.00965 |
| LYM156 | C | 3 | 0.71152 | 0.01407 |
| LYM 156 | B | 3 | 0.70014 | 0.05315 |
| LYM157 | A | 4 | 0.78681 | 0.01187 |
| LYM 157 | A | 3 | 0.73234 | 0.02485 |
| LYM157 | A | 12 | 0.72729 | 0.02639 |
| LYM160 | A | 12 | 0.87825 | 0.00184 |
| LYM 160 | A | 10 | 0.82699 | 0.00596 |
| LYM 160 | A | 12 | 0.82567 | 0.00174 |
| LYM 160 | A | 9 | 0.80855 | 0.00834 |
| LYM160 | A | 9 | 0.80222 | 0.00297 |
| LYM160 | A | 10 | 0.74770 | 0.00815 |
| LYM160 | A | 5 | 0.72266 | 0.02785 |
| LYM160 | A | 5 | 0.71752 | 0.01292 |
| LYM154 | C | 2 | 0.92810 | 0.00004 |
| LYM154 | C | 3 | 0.90313 | 0.00014 |
| LYM154 | C | 4 | 0.85169 | 0.00088 |
| LYM154 | C | 5 | 0.73538 | 0.00991 |
| LYM 154 | C | 10 | 0.71818 | 0.01280 |
| LYM154 | C | 12 | 0.70985 | 0.01440 |
| LYM155 | C | 6 | 0.72312 | 0.04266 |
| LYM155 | C | 1 | 0.70598 | 0.01519 |
| LYM 180 | C | 1 | 0.76320 | 0.00628 |
| LYM180 | B | 6 | 0.75133 | 0.05153 |
| LYM181 | C | 6 | 0.78450 | 0.02115 |
| LYM184 | B | 6 | 0.82395 | 0.02266 |
| LYM 184 | B | 6 | 0.73704 | 0.01501 |
| LYM 189 | A | 10 | 0.81585 | 0.00733 |
| LYM189 | A | 12 | 0.81256 | 0.00777 |
| LYM189 | A | 9 | 0.72388 | 0.02746 |
| LYM189 | A | 5 | 0.71589 | 0.03008 |
| LYM 192 | A | 2 | 0.86386 | 0.00061 |
| LYM 192 | A | 3 1 | 0.80919 | 0.00255 |
| LYM192 | A | 3 | 0.77612 | 0.01393 |
| LYM278 | A | 4 | 0.90217 | 0.00088 |
| LYM278 | A | 4 | 0.87108 | 0.00048 |
| LYM278 | A | 12 | 0.81518 | 0.00742 |
| LYM278 | A | 3 | 0.79925 | 0.00975 |
| LYM278 | A | 3 | 0.78102 | 0.00454 |
| LYM278 | A | 12 | 0.76569 | 0.00601 |
| LYM278 | A | 2 | 0.73983 | 0.00925 |
| LYM38 | A | 4 | 0.97624 | 0.00001 |
| LYM38 | A | 12 | 0.82191 | 0.00191 |
| LYM38 | A | 8 | 0.82190 | 0.00656 |
| LYM38 | A | 3 | 0.82153 | 0.00193 |
| LYM38 | A | 5 | 0.81079 | 0.00246 |
| LYM52 | B | 8 | 0.88462 | 0.00352 |
| LYM52 | A | 2 | 0.80812 | 0.00261 |
| LYM52 | A | 4 | 0.80802 | 0.00841 |
| LYM52 | A | 3 | 0.78340 | 0.01251 |
| LYM52 | A | 12 | 0.77364 | 0.01444 |
| LYM52 | A | 9 | 0.75221 | 0.01938 |
| LYM52 | A | 5 | 0.74914 | 0.02016 |
| LYM52 | A | 8 | 0.71086 | 0.03181 |
| LYM56 | A | 3 | 0.85797 | 0.00073 |
| LYM56 | A | 4 | 0.85130 | 0.00089 |
| LYM56 | A | 2 | 0.82071 | 0.00196 |
| LYM56 | A | 8 | 0.76236 | 0.01692 |
| LYM56 | A | 12 | 0.72510 | 0.01157 |
| LYM83 | A | 9 | 0.87556 | 0.00198 |
| LYM83 | A | 5 | 0.87005 | 0.00050 |
| LYM83 | A | 12 | 0.82187 | 0.00191 |
| LYM90 | C | 4 | 0.86848 | 0.00238 |
| LYM90 | C | 3 | 0.81261 | 0.00777 |
| LYM90 | C | 12 | 0.77921 | 0.01332 |
| LYM90 | C | 2 | 0.76512 | 0.01629 |
| LYM93 | A | 9 | 0.86630 | 0.00056 |
| LYM93 | A | 12 | 0.78696 | 0.00405 |
| LYM93 | A | 5 | 0.76542 | 0.00604 |
| LYM 106 | A | 3 | 0.79483 | 0.01047 |
| LYM106 | A | 2 | 0.75674 | 0.01825 |
| LYM106 | C | 6 | 0.73962 | 0.03596 |
| LYM159 | C | 1 | 0.82807 | 0.00584 |
| LYM159 | B | 8 | 0.79356 | 0.01873 |
| LYM161 | C | 3 | 0.89287 | 0.00119 |
| LYM161 | C | 2 | 0.88206 | 0.00165 |
| LYM161 | A | 6 | 0.85373 | 0.00699 |
| LYM161 | C | 4 | 0.74623 | 0.02093 |
| LYM 178 | C | 6 | 0.83756 | 0.00945 |
| LYM182 | A | 6 | 0.93567 | 0.00063 |
| LYM185 | C | 4 | 0.76455 | 0.01642 |
| LYM185 | A | 1 | 0.75952 | 0.01759 |
| LYM 185 | A | 6 | 0.70292 | 0.01584 |
| LYM 186 | A | 6 | 0.86003 | 0.00616 |
| LYM188 | A | 6 | 0.82774 | 0.00166 |
| LYM188 | C | 2 | 0.73602 | 0.02377 |
| LYM188 | C | 3 | 0.71072 | 0.03186 |
| LYM194 | A | 2 | 0.89053 | 0.00024 |
| LYM194 | A | 3 | 0.82982 | 0.00157 |
| LYM289 | A | 9 | 0.77609 | 0.01394 |
| LYM289 | A | 5 | 0.77182 | 0.01483 |

Table 8. Provided are the correlations (R) and p-values (P) between the expression levels of selected genes in various tissues or developmental stages (Expression sets) and the phenotypic performance in various yield (seed yield, oil yield, oil content), biomass, growth rate and/or vigor components [Correlation (Corr.) vector (Vec.)] Corr. Vec. = correlation vector specified in Tables 5, 6 and 7; Exp. Set = expression set specified in Table 3.

### EXAMPLE 4

### PRODUCTION OF ARABIDOPSIS TRANSCRIPTOM AND HIGH THROUGHPUT CORRELATION ANALYSIS OF YIELD, BIOMASS AND/OR VIGOR RELATED PARAMETERS USING 44K ARABIDOPSIS FULL GENOME OLIGONUCLEOTIDE MICRO-ARRAY

To produce a high throughput correlation analysis, the present inventors utilized an Arabidopsis thaliana oligonucleotide micro-array, produced by Agilent Technologies [Hypertext Transfer Protocol://World Wide Web (dot) chem. (dot) agilent (dot) com/Scripts/PDS (dot) asp?1Page=50879]. The array oligonucleotide represents about 40,000 *A. thaliana* genes and transcripts designed based on data from the TIGR ATH1 v.5 database and Arabidopsis MPSS (University of Delaware) databases. To define correlations between the levels of RNA expression and yield, biomass components or vigor related parameters, various plant characteristics of 15 different Arabidopsis ecotypes were analyzed. Among them, nine ecotypes encompassing the observed variance were selected for RNA expression analysis. The correlation between the RNA levels and the characterized parameters was analyzed using Pearson correlation test [Hypertext Transfer Protocol://World Wide Web (dot) davidmlane (dot) com/hyperstat/A34739 (dot) html].

### Experimental procedures

***RNA extraction*** - Five tissues at different developmental stages including root, leaf, flower at anthesis, seed at 5 days after flowering (DAF) and seed at 12 DAF, representing different plant characteristics, were sampled and RNA was extracted as described in Example 3 above. For convenience, each micro-array expression information tissue type has received a Set ID as summarized in Table 9 below.

**Table 9**

| ***Tissues used for Arabidopsis transcriptom expression sets*** | |
|---|---|
| ***Expression Set*** | ***Set ID*** |
| Root | A |
| Leaf | B |
| Flower | C |
| Seed 5 DAF | D |
| Seed 12 DAF | E |

Table 9: Provided are the identification (ID) letters of each of the *Arabidopsis* expression sets (A-E). DAF = days after flowering.

***Yield components and vigor related parameters assessment*** - eight out of the nine Arabidopsis ecotypes were used in each of 5 repetitive blocks (named A, B, C, D and E), each containing 20 plants per plot. The plants were grown in a greenhouse at controlled conditions in 22 °C, and the N:P:K fertilizer (20:20:20; weight ratios) [nitrogen (N), phosphorus (P) and potassium (K)] was added. During this time data was collected, documented and analyzed. Additional data was collected through the seedling stage of plants grown in a tissue culture in vertical grown transparent agar plates. Most of chosen parameters were analyzed by digital imaging.

***Digital imaging in Tissue culture*** - A laboratory image acquisition system was used for capturing images of plantlets sawn in square agar plates. The image acquisition system consists of a digital reflex camera (Canon EOS 300D) attached to a 55 mm focal length lens (Canon EF-S series), mounted on a reproduction device (Kaiser RS), which included 4 light units (4x150 Watts light bulb) and located in a darkroom.

***Digital imaging in Greenhouse*** - The image capturing process was repeated every 3-4 days starting at day 7 till day 30. The same camera attached to a 24 mm focal length lens (Canon EF series), placed in a custom made iron mount, was used for capturing images of larger plants sawn in white tubs in an environmental controlled greenhouse. The white tubs were square shape with measurements of 36 x 26.2 cm and 7.5 cm deep. During the capture process, the tubs were placed beneath the iron mount, while avoiding direct sun light and casting of shadows. This process was repeated every 3-4 days for up to 30 days.

An image analysis system was used, which consists of a personal desktop computer (Intel P4 3.0 GHz processor) and a public domain program - ImageJ 1.37, Java based image processing program, which was developed at the U.S National Institutes of Health and is freely available on the internet at Hypertext Transfer Protocol://rsbweb (dot) nih (dot) gov/. Images were captured in resolution of 6 Mega Pixels (3072 x 2048 pixels) and stored in a low compression JPEG (Joint Photographic Experts Group standard) format. Next, analyzed data was saved to text files and processed using the JMP statistical analysis software (SAS institute).

***Leaf analysis*** - Using the digital analysis leaves data was calculated, including leaf number, area, perimeter, length and width. On day 30, 3-4 representative plants were chosen from each plot of blocks A, B and C. The plants were dissected, each leaf was separated and was introduced between two glass trays, a photo of each plant was taken and the various parameters (such as leaf total area, laminar length etc.) were calculated from the images. The blade circularity was calculated as laminar width divided by laminar length.

***Root analysis*** - During 17 days, the different ecotypes were grown in transparent agar plates. The plates were photographed every 3 days starting at day 7 in the photography room and the roots development was documented (see examples in Figures 3A-F). The growth rate of roots was calculated according to Formula VII. $Relative growth rate of root coverage = Regression coefficient of root coverage along time course.$

***Vegetative growth rate analysis*** - was calculated according to Formula VIII. The analysis was ended with the appearance of overlapping plants. $Relative vegetative growth ratet area = Regression coefficient of vegetative area along time course.$

For comparison between ecotypes the calculated rate was normalized using plant developmental stage as represented by the number of true leaves. In cases where plants with 8 leaves had been sampled twice (for example at day 10 and day 13), only the largest sample was chosen and added to the Anova comparison.

***Seeds in siliques analysis*** - On day 70, 15-17 siliques were collected from each plot in blocks D and E. The chosen siliques were light brown color but still intact. The siliques were opened in the photography room and the seeds were scatter on a glass tray, a high resolution digital picture was taken for each plot. Using the images the number of seeds per silique was determined.

***Seeds average weight*** - At the end of the experiment all seeds from plots of blocks A-C were collected. An average weight of 0.02 grams was measured from each sample, the seeds were scattered on a glass tray and a picture was taken. Using the digital analysis, the number of seeds in each sample was calculated.

***Oil percentage in seeds*** - At the end of the experiment all seeds from plots of blocks A-C were collected. Columbia seeds from 3 plots were mixed grounded and then mounted onto the extraction chamber. 210 ml of n-Hexane (Cat No. 080951 Biolab Ltd.) were used as the solvent. The extraction was performed for 30 hours at medium heat 50 °C. Once the extraction has ended the n-Hexane was evaporated using the evaporator at 35 °C and vacuum conditions. The process was repeated twice. The information gained from the Soxhlet extractor (Soxhlet, F. Die gewichtsanalytische Bestimmung des Milchfettes, Polytechnisches J. (Dingler's) 1879,232,461) was used to create a calibration curve for the Low Resonance NMR. The content of oil of all seed samples was determined using the Low Resonance NMR (MARAN Ultra- Oxford Instrument) and its MultiQuant sowftware package.

***Silique length analysis*** - On day 50 from sowing, 30 siliques from different plants in each plot were sampled in block A. The chosen siliques were green-yellow in color and were collected from the bottom parts of a grown plant's stem. A digital photograph was taken to determine silique's length.

***Dry weight and seed yield*** - On day 80 from sowing, the plants from blocks A-C were harvested and left to dry at 30 °C in a drying chamber. The biomass and seed weight of each plot was separated, measured and divided by the number of plants. Dry weight = total weight of the vegetative portion above ground (excluding roots) after drying at 30 °C in a drying chamber; Seed yield per plant = total seed weight per plant (gr).

***Oil yield*** - The oil yield was calculated using Formula IX. $Seed Oil yield = Seed yield per plant \left(gr\right) * Oil % in seed$

***Harvest Index (seed)*** - The harvest index was calculated using Formula IV (described above): Harvest Index = Average seed yield per plant/ Average dry weight.

### Experimental Results

Nine different *Arabidopsis* ecotypes were grown and characterized for 18 parameters (named as vectors).

**Table 10**

| ***Arabidopsis correlated parameters (vectors)*** | |
|---|---|
| ***Correlated parameter with*** | ***Correlation ID*** |
| Root length day 13 (cm) | 1 |
| Root length day 7 (cm) | 2 |
| Relative root growth (cm /day) day 13 | 3 |
| Fresh weight per plant (gr) at bolting stage | 4 |
| Dry matter per plant (gr) | 5 |
| Vegetative growth rate (cm² / day) till 8 true leaves | 6 |
| Blade circularity | 7 |
| Lamina width (cm) | 8 |
| Lamina length (cm) | 9 |
| Total leaf area per plant (cm) | 10 |
| 1000 Seed weight (gr) | 11 |
| Oil % per seed | 12 |
| Seeds per silique | 13 |
| Silique length (cm) | 14 |
| Seed yield per plant (gr) | 15 |
| Oil yield per plant (mg) | 16 |
| Harvest Index | 17 |
| Leaf width/length | 18 |

Table 10. Provided are the Arabidopsis correlated parameters (correlation ID Nos. 1-18). Abbreviations: Cm = centimeter(s); gr = gram(s); mg = milligram(s).

The characterized values are summarized in Tables 11 and 12 below.

**Table 11**

| ***Measured parameters in Arabidopsis ecotypes*** | | | | | | | | | |
|---|---|---|---|---|---|---|---|---|---|
| ***Ecotype*** | ***15*** | ***16*** | ***12*** | ***11*** | ***5*** | ***17*** | ***10*** | ***13*** | ***14*** |
| An-1 | 0.34 | 118.63 | 34.42 | 0.0203 | 0.64 | 0.53 | 46.86 | 45.44 | 1.06 |
| Col-0 | 0.44 | 138.73 | 31.19 | 0.0230 | 1.27 | 0.35 | 109.89 | 53.47 | 1.26 |
| Ct-1 | 0.59 | 224.06 | 38.05 | 0.0252 | 1.05 | 0.56 | 58.36 | 58.47 | 1.31 |
| Cvi (N8580) | 0.42 | 116.26 | 27.76 | 0.0344 | 1.28 | 0.33 | 56.80 | 35.27 | 1.47 |
| Gr-6 | 0.61 | 218.27 | 35.49 | 0.0202 | 1.69 | 0.37 | 114.66 | 48.56 | 1.24 |
| Kondara | 0.43 | 142.11 | 32.91 | 0.0263 | 1.34 | 0.32 | 110.82 | 37.00 | 1.09 |
| Ler-1 | 0.36 | 114.15 | 31.56 | 0.0205 | 0.81 | 0.45 | 88.49 | 39.38 | 1.18 |

| ***Ecotype*** | ***15*** | ***16*** | ***12*** | ***11*** | ***5*** | ***17*** | ***10*** | ***13*** | ***14*** |
|---|---|---|---|---|---|---|---|---|---|
| Mt-0 | 0.62 | 190.06 | 30.79 | 0.0226 | 1.21 | 0.51 | 121.79 | 40.53 | 1.18 |
| Shakdara | 0.55 | 187.62 | 34.02 | 0.0235 | 1.35 | 0.41 | 93.04 | 25.53 | 1.00 |

Table 11. Provided are the values of each of the parameters measured in Arabidopsis ecotypes: 15 = Seed yield per plant (gram); 16 = oil yield per plant (mg); 12 = oil % per seed; 11 = 1000 seed weight (gr); 5 = dry matter per plant (gr); 17 = harvest index; 10 = total leaf area per plant (cm); 13 = seeds per silique; 14 = Silique length (cm).

**Table 12**

| ***Additional measured parameters in Arabidopsis ecotypes*** | | | | | | | | | |
|---|---|---|---|---|---|---|---|---|---|
| **Ecotype** | **6** | **3** | **2** | **1** | **4** | **9** | **8** | **18** | **7** |
| An-1 | 0.313 | 0.631 | 0.937 | 4.419 | 1.510 | 2.767 | 1.385 | 0.353 | 0.509 |
| Col-0 | 0.378 | 0.664 | 1.759 | 8.530 | 3.607 | 3.544 | 1.697 | 0.288 | 0.481 |
| Ct-1 | 0.484 | 1.176 | 0.701 | 5.621 | 1.935 | 3.274 | 1.460 | 0.316 | 0.450 |
| Cvi (N8580) | 0.474 | 1.089 | 0.728 | 4.834 | 2.082 | 3.785 | 1.374 | 0.258 | 0.370 |
| Gr-6 | 0.425 | 0.907 | 0.991 | 5.957 | 3.556 | 3.690 | 1.828 | 0.356 | 0.501 |
| Kondara | 0.645 | 0.774 | 1.163 | 6.372 | 4.338 | 4.597 | 1.650 | 0.273 | 0.376 |
| Ler-1 | 0.430 | 0.606 | 1.284 | 5.649 | 3.467 | 3.877 | 1.510 | 0.305 | 0.394 |
| Mt-0 | 0.384 | 0.701 | 1.414 | 7.060 | 3.479 | 3.717 | 1.817 | 0.335 | 0.491 |
| Shakdar a | 0.471 | 0.782 | 1.251 | 7.041 | 3.710 | 4.149 | 1.668 | 0.307 | 0.409 |

Table 12. Provided are the values of each of the parameters measured in Arabidopsis ecotypes: 6 = Vegetative growth rate (cm²/day) until 8 true leaves; 3 = relative root growth (cm/day) (day 13); 2 = Root length day 7 (cm); 1 = Root length day 13 (cm); 4 = fresh weight per plant (gr) at bolting stage; 9. = Lamima length (cm); 8 = Lamina width (cm); 18 = Leaf width/length; 7 = Blade circularity.

Table 13, below, provides selected genes, the characterized parameters (which are used as x axis for correlation) and the correlated tissue transcriptom along with the correlation value (R, calculated using Pearson correlation). When the correlation coefficient (R) between the levels of a gene's expression in a certain tissue and a phenotypic performance across ecotypes is high in absolute value (between 0.5-1), there is an association between the gene (specifically the expression level of this gene) and the phenotypic character.

**Table 13**

| ***Correlation between the expression level of selected genes in specific tissues or developmental stages and the phenotypic performance across Arabidopsis ecotypes*** | | | | |
|---|---|---|---|---|
| **Gene Name** | **Expression Set** | **Correlation Vector** | **R** | **P** |
| LYM88 | E | 13 | 0.75035 | 0.03198 |
| LYM89 | D | 10 | 0.88062 | 0.00886 |
| LYM89 | D | 4 | 0.84712 | 0.01614 |
| LYM89 | A | 6 | 0.84690 | 0.00797 |
| LYM89 | D | 5 | 0.83715 | 0.01879 |
| LYM89 | D | 6 | 0.70174 | 0.07884 |
| LYM152 | E | 14 | 0.85500 | 0.00682 |

Table 13. Provided are the correlations between the expression level of yield improving genes and their homologs in specific tissues or developmental stages (expression sets) and the phenotypic performance (correlation vector) across Arabidopsis ecotypes. The phenotypic characters [correlation (Corr.) vector (Vec.)] include yield (seed yield, oil yield, oil content), biomass, growth rate and/or vigor components as described in Tables 10-12. Exp. Set = expression set according to Table 9 hereinabove.

### EXAMPLE 5

### PRODUCTION OF ARABIDOPSIS TRANSCRIPTOM AND HIGH THROUGHPUT CORRELATION ANALYSIS OF NORMAL AND NITROGEN LIMITING CONDITIONS USING 44K ARABIDOPSIS OLIGONUCLEOTIDE MICRO-ARRAY

In order to produce a high throughput correlation analysis, the present inventors utilized a Arabidopsis oligonucleotide micro-array, produced by Agilent Technologies [Hypertext Transfer Protocol://World Wide Web (dot) chem (dot) agilent (dot) com/Scripts/PDS (dot) asp?1Page=50879]. The array oligonucleotide represents about 44,000 Arabidopsis genes and transcripts. To define correlations between the levels of RNA expression with NUE, yield components or vigor related parameters various plant characteristics of 14 different Arabidopsis ecotypes were analyzed. Among them, ten ecotypes encompassing the observed variance were selected for RNA expression analysis. The correlation between the RNA levels and the characterized parameters was analyzed using Pearson correlation test [Hypertext Transfer Protocol://World Wide Web (dot) davidmlane (dot) com/hyperstat/A34739 (dot) html].

### Experimental procedures

***RNA extraction*** - Two tissues of plants [leaves and stems] growing at two different nitrogen fertilization levels (1.5 mM Nitrogen or 6 mM Nitrogen) were sampled and RNA was extracted as described in Examples 3 above. For convenience, each micro-array expression information tissue type has received a Set ID as summarized in Table 14 below.

**Table 14**

| ***Tissues used for Arabidopsis transcriptom expression sets*** | |
|---|---|
| ***Expression Set*** | ***Set ID*** |
| Leaves at 1.5 mM Nitrogen fertilization | A |
| Leaves at 6 mM Nitrogen fertilization | B |
| Stems at 1.5 mM Nitrogen fertilization | C |
| Stem at 6 mM Nitrogen fertilization | D |

Table 14: Provided are the identification (ID) letters of each of the Arabidopsis expression sets.

***Assessment of Arabidopsis yield components and vigor related parameters under different nitrogen fertilization levels* -** 10 Arabidopsis accessions in 2 repetitive plots each containing 8 plants per plot were grown at greenhouse. The growing protocol used was as follows: surface sterilized seeds were sown in Eppendorf tubes containing 0.5 x Murashige-Skoog basal salt medium and grown at 23 °C under 12-hour light and 12-hour dark daily cycles for 10 days. Then, seedlings of similar size were carefully transferred to pots filled with a mix of perlite and peat in a 1:1 ratio. Constant nitrogen limiting conditions were achieved by irrigating the plants with a solution containing 1.5 mM inorganic nitrogen in the form of KNO₃, supplemented with 2 mM CaCl₂, 1.25 mM KH₂PO₄, 1.50 mM MgSO₄, 5 mM KCl, 0.01 mM H₃BO₃ and microelements, while normal irrigation conditions (Normal Nitrogen conditions) was achieved by applying a solution of 6 mM inorganic nitrogen also in the form of KNO₃, supplemented with 2 mM CaCl₂, 1.25 mM KH₂PO₄, 1.50 mM MgSO₄, 0.01 mM H₃BO₃ and microelements. To follow plant growth, trays were photographed the day nitrogen limiting conditions were initiated and subsequently every 3 days for about 15 additional days. Rosette plant area was then determined from the digital pictures. ImageJ software was used for quantifying the plant size from the digital pictures [Hypertext Transfer Protocol://rsb (dot) info (dot) nih (dot) gov/ij/] utilizing proprietary scripts designed to analyze the size of rosette area from individual plants as a function of time. The image analysis system included a personal desktop computer (Intel P4 3.0 GHz processor) and a public domain program - ImageJ 1.37 (Java based image processing program, which was developed at the U.S. National Institutes of Health and freely available on the internet [Hypertext Transfer Protocol://rsbweb (dot) nih (dot) gov/]. Next, analyzed data was saved to text files and processed using the JMP statistical analysis software (SAS institute).

Data parameters collected are summarized in Table 15, hereinbelow.

**Table 15**

| ***Arabidopsis correlated parameters (vectors)*** | |
|---|---|
| ***Correlated parameter with*** | ***Correlation Id*** |
| N 1.5 mM; Rosette Area at day 8 | 1 |
| N 1.5 mM; Rosette Area at day 10 [cm²] | 2 |
| N 1.5 mM; Plot Coverage at day 8 [%] | 3 |
| N 1.5 mM; Plot Coverage at day 10 [%] | 4 |
| N 1.5 mM; Leaf Number at day 10 | 5 |
| N 1.5 mM; Leaf Blade Area at day 10 [cm²] | 6 |
| N 1.5 mM; RGR of Rosette Area at day 3 [cm²/day] | 7 |
| N 1.5 mM; t50 Flowering [day] | 8 |
| N 1.5 mM; Dry Weight [gr/plant] | 9 |
| N 1.5 mM; Seed Yield [gr/plant] | 10 |
| N 1.5 mM; Harvest Index | 11 |
| N 1.5 mM; 1000 Seeds weight [gr] | 12 |
| N 1.5 mM; seed yield/ rosette area at day 10 [gr/cm²] | 13 |
| N 1.5 mM; seed yield/leaf blade [gr/cm²] | 14 |
| N 1.5 mM; % Seed yield reduction compared to N 6 mM | 15 |
| N 1.5 mM; % Biomass reduction compared to N 6 mM | 16 |
| N 1.5 mM; N level /DW [SPAD unit/gr] | 17 |
| N 1.5 mM; DW/ N level [gr/ SPAD unit] | 18 |
| N 1.5 mM; seed yield/ N level [gr/ SPAD unit] | 19 |
| N 6 mM; Rosette Area at day 8 [cm²] | 20 |
| N 6 mM; Rosette Area at day 10 [cm²] | 21 |
| N 6 mM; Plot Coverage at day 8 [%] | 22 |
| N 6 mM; Plot Coverage at day 10 [%] | 23 |
| N 6 mM; Leaf Number at day 10 | 24 |
| N 6 mM; Leaf Blade Area at day 10 | 25 |
| N 6 mM; RGR of Rosette Area at day 3 [cm²/gr] | 26 |
| N 6 mM; t50 Flowering [day] | 27 |
| N 6 mM; Dry Weight [gr/plant] | 28 |
| N 6 mM; Seed Yield [gr/plant] | 29 |
| N 6 mM; Harvest Index | 30 |
| N 6 mM; 1000 Seeds weight [gr] | 31 |
| N 6 mM; seed yield/ rosette area day at day 10 [gr/cm²] | 32 |
| N 6 mM; seed yield/leaf blade [gr/cm²] | 33 |
| N 6 mM; N level / FW | 34 |
| N 6 mM; DW/ N level [gr/ SPAD unit] | 35 |
| N 6 mM; N level /DW (SPAD unit/gr plant) | 36 |
| N 6 mM; Seed yield/N unit [gr/ SPAD unit] | 37 |

Table 15. Provided are the Arabidopsis correlated parameters (vectors). "N" = Nitrogen at the noted concentrations; "gr." = grams; "SPAD" = chlorophyll levels; "t50" = time where 50% of plants flowered; "gr/ SPAD unit" = plant biomass expressed in grams per unit of nitrogen in plant measured by SPAD. "DW" = Plant Dry Weight; "FW" = Plant Fresh weight; "N level /DW" = plant Nitrogen level measured in SPAD unit per plant biomass [gr]; "DW/ N level" = plant biomass per plant [gr]/SPAD unit; Rosette Area (measured using digital analysis); Plot Coverage at the indicated day [%] (calculated by the dividing the total plant area with the total plot area); Leaf Blade Area at the indicated day [cm²] (measured using digital analysis); RGR (relative growth rate) of Rosette Area at the indicated day [cm²/day]; t50 Flowering [day] (the day in which 50% of plant flower); seed yield/ rosette area at day 10 [gr/cm²] (calculated); seed yield/leaf blade [gr/cm²] (calculated); seed yield/ N level [gr/ SPAD unit] (calculated).

***Assessment of NUE, yield components and vigor-related parameters*** - Ten Arabidopsis ecotypes were grown in trays, each containing 8 plants per plot, in a greenhouse with controlled temperature conditions for about 12 weeks. Plants were irrigated with different nitrogen concentration as described above depending on the treatment applied. During this time, data was collected documented and analyzed. Most of chosen parameters were analyzed by digital imaging.

### Digital imaging - Greenhouse assay

An image acquisition system, which consists of a digital reflex camera (Canon EOS 400D) attached with a 55 mm focal length lens (Canon EF-S series) placed in a custom made Aluminum mount, was used for capturing images of plants planted in containers within an environmental controlled greenhouse. The image capturing process is repeated every 2-3 days starting at day 9-12 till day 16-19 (respectively) from transplanting.

An image processing system was used, which consists of a personal desktop computer (Intel P4 3.0 GHz processor) and a public domain program - ImageJ 1.37, Java based image processing software, which was developed at the U.S. National Institutes of Health and is freely available on the internet at Hypertext Transfer Protocol://rsbweb (dot) nih (dot) gov/. Images were captured in resolution of 10 Mega Pixels (3888x2592 pixels) and stored in a low compression JPEG (Joint Photographic Experts Group standard) format. Next, image processing output data was saved to text files and analyzed using the JMP statistical analysis software (SAS institute).

***Leaf analysis* -** Using the digital analysis leaves data was calculated, including leaf number, leaf blade area, plot coverage, Rosette diameter and Rosette area.

***Relative growth rate area:*** The relative growth rate of the rosette and the leaves was calculated according to Formulas XIV and XVII, respectively.

***Seed yield and 1000 seeds weight*** - At the end of the experiment all seeds from all plots were collected and weighed in order to measure seed yield per plant in terms of total seed weight per plant (gr). For the calculation of 1000 seed weight, an average weight of 0.02 grams was measured from each sample, the seeds were scattered on a glass tray and a picture was taken. Using the digital analysis, the number of seeds in each sample was calculated.

***Dry weight and seed yield*** - At the end of the experiment, plant were harvested and left to dry at 30 °C in a drying chamber. The biomass was separated from the seeds, weighed and divided by the number of plants. Dry weight = total weight of the vegetative portion above ground (excluding roots) after drying at 30 °C in a drying chamber.

***Harvest Index (seed)*** - The harvest index was calculated using Formula IV as described above [Harvest Index = Average seed yield per plant/ Average dry weight].

***T₅₀ days to flowering* -** Each of the repeats was monitored for flowering date. Days of flowering was calculated from sowing date till 50 % of the plots flowered.

***Plant nitrogen level*** - The chlorophyll content of leaves is a good indicator of the nitrogen plant status since the degree of leaf greenness is highly correlated to this parameter. Chlorophyll content was determined using a Minolta SPAD 502 chlorophyll meter and measurement was performed at time of flowering. SPAD meter readings were done on young fully developed leaf. Three measurements per leaf were taken per plot. Based on this measurement, parameters such as the ratio between seed yield per nitrogen unit [seed yield/N level = seed yield per plant [gr]/SPAD unit], plant DW per nitrogen unit [DW/ N level= plant biomass per plant [g]/SPAD unit], and nitrogen level per gram of biomass [N level/DW= SPAD unit/ plant biomass per plant (gr)] were calculated.

***Percent of seed yield reduction-*** measures the amount of seeds obtained in plants when grown under nitrogen-limiting conditions compared to seed yield produced at normal nitrogen levels expressed in %.

### Experimental Results

10 different Arabidopsis accessions (ecotypes) were grown and characterized for 37 parameters as described above. The average for each of the measured parameters was calculated using the JMP software. Subsequent correlation analysis between the various transcriptom sets (Table 14) was conducted. Following are the results integrated to the database.

**Table 16**

| ***Correlation between the expression level of selected genes and their homologs in tissues under limiting or normal nitrogen fertilization and the phenotypic performance across Arabidopsis ecotypes*** | | | | |
|---|---|---|---|---|
| **Gene Name** | **Expression Set** | **Correlation Vector** | **R** | **P** |
| LYM88 | C | 17 | 0.93533 | 0.01955 |
| LYM88 | D | 16 | 0.79502 | 0.01044 |
| LYM8_H1 | D | 31 | 0.745282 | 0.021184 |
| LYM 10_H7 | C | 24 | 0.895562 | 0.000458 |
| LYM10_H7 | C | 5 | 0.77817 | 0.008026 |
| LYM 10_H7 | C | 21 | 0.725922 | 0.017459 |
| LYM10_H7 | C | 2 | 0.717752 | 0.019423 |
| LYM10_H8 | C | 8 | 0.850748 | 0.001806 |
| LYM10_H8 | C | 27 | 0.7752 | 0.008432 |
| LYM 10_H8 | C | 15 | 0.77175 | 0.008921 |
| LYM10_H9 | A | 1 | 0.844351 | 0.002119 |
| LYM10_H9 | A | 2 | 0.755723 | 0.01146 |
| LYM10_H9 | A | 20 | 0.733447 | 0.015776 |
| LYM10_H9 | A | 21 | 0.722114 | 0.018356 |
| LYM14_H2 | B | 27 | 0.892136 | 0.000519 |
| LYM14_H2 | B | 8 | 0.830273 | 0.002942 |
| LYM14_H2 | C | 8 | 0.816286 | 0.003967 |
| LYM14_H2 | C | 8 | 0.794197 | 0.006071 |
| LYM14_H2 | C | 27 | 0.767463 | 0.009557 |
| LYM14_H2 | C | 27 | 0.733255 | 0.015818 |
| LYM14_H2 | D | 1 | 0.725116 | 0.027066 |
| LYM14_H3 | B | 15 | 0.855842 | 0.001582 |
| LYM14_H3 | C | 8 | 0.802977 | 0.005158 |
| LYM14_H3 | B | 8 | 0.79811 | 0.005651 |
| LYM14_H3 | B | 12 | 0.792747 | 0.006233 |
| LYM14_H3 | B | 27 | 0.785721 | 0.007057 |
| LYM14_H3 | C | 27 | 0.734206 | 0.015613 |
| LYM14_H3 | A | 9 | 0.720081 | 0.018848 |
| LYM137_H11 | C | 20 | 0.815207 | 0.004055 |
| LYM137_H11 | C | 21 | 0.79814 | 0.005648 |
| LYM137_H11 | D | 5 | 0.754601 | 0.018779 |
| LYM137_H11 | C | 24 | 0.701843 | 0.023676 |
| LYM152_H1 | D | 5 | 0.714383 | 0.030592 |
| LYM152_H1 | D | 5 | 0.713442 | 0.030914 |
| LYM236_H4 | B | 27 | 0.937557 | 6.17E-05 |
| LYM236_H4 | B | 8 | 0.921247 | 0.000153 |
| LYM236_H4 | B | 15 | 0.865782 | 0.001204 |
| LYM236_H4 | B | 28 | 0.709595 | 0.02153 |
| LYM236_H5 | A | 10 | 0.737484 | 0.014922 |
| LYM236_H5 | B | 10 | 0.71158 | 0.021004 |

Table 16. Provided are the correlations (R) between the expression levels of yield improving genes and their homologs in tissues (leaves or stems) under limiting (1.5 mM Nitrogen) or normal (6 mM Nitrogen) conditions (Expression sets) and the phenotypic performance in various yield (seed yield, oil yield, oil content), biomass, growth rate and/or vigor components [Correlation (Corr.) vector (Vec.)] under limiting or normal Nitrogen conditions. Corr. Vec. = correlation vector according to Table 15 hereinabove; Exp. Set = expression set according to Table 14 hereinabove.

### EXAMPLE 6

### PRODUCTION OF SORGHUM TRANSCRIPTOM AND HIGH THROUGHPUT CORRELATION ANALYSIS WITH ABST RELATED PARAMETRERS USING 44K SORGUHM OLIGONUCLEOTIDE MICRO-ARRAYS

In order to produce a high throughput correlation analysis, the present inventors utilized a Sorghum oligonucleotide micro-array, produced by Agilent Technologies [Hypertext Transfer Protocol://World Wide Web (dot) chem. (dot) agilent (dot) com/Scripts/PDS (dot) asp?1Page=50879]. The array oligonucleotide represents about 44,000 Sorghum genes and transcripts. In order to define correlations between the levels of RNA expression with ABST and yield components or vigor related parameters, various plant characteristics of 17 different sorghum varieties were analyzed. Among them, 10 varieties encompassing the observed variance were selected for RNA expression analysis. The correlation between the RNA levels and the characterized parameters was analyzed using Pearson correlation test [Hypertext Transfer Protocol://World Wide Web (dot) davidmlane (dot) com/hyperstat/A34739 (dot) html].

### Correlation of Sorghum varieties across ecotype grown under severe drought conditions

### Experimental procedures

17 Sorghum varieties were grown in 3 repetitive plots, in field. Briefly, the growing protocol was as follows: sorghum seeds were sown in soil and grown under normal condition until around 35 days from sowing, around V8 (Last leaf visible, but still rolled up, ear beginning to swell). At this point, irrigation was stopped, and severe drought stress was developed. In order to define correlations between the levels of RNA expression with drought, yield components or vigor related parameters, the 17 different sorghum varieties were analyzed. Among them, 10 varieties encompassing the observed variance were selected for RNA expression analysis. The correlation between the RNA levels and the characterized parameters was analyzed using Pearson correlation test [Hypertext Transfer Protocol://World Wide Web (dot) davidmlane (dot) com/hyperstat/A34739 (dot) html].

***RNA extraction*** - All 10 selected Sorghum varieties were sample per each treatment. Plant tissues [Flag leaf, Flower meristem and Flower] growing under severe drought stress and plants grown under Normal conditions were sampled and RNA was extracted as described in Examples 3 above. For convenience, each micro-array expression information tissue type has received a Set ID as summarized in Table 17 below.

**Table 17**

| ***Sorghum transcriptom expression sets*** | |
|---|---|
| ***Expression Set*** | ***Set ID*** |
| Sorghum field/Normal/flower meristem | 1 |
| Sorghum field/Normal/flower | 2 |
| Sorghum field/Normal/flag leaf | 3 |
| Drought Stress: Flag leaf | 4 |

Table 17: Provided are the sorghum transcriptom expression sets 1, 2, 3 and 4. Flag leaf = the leaf below the flower; Flower meristem = Apical meristem following panicle initiation; Flower = the flower at the anthesis day. Expression sets 1, 2 and 3 are from plants grown under normal conditions. Expression set 4 derived from plants grown under drought conditions.

The following parameters were collected using digital imaging system:
***Average Grain Area (cm²)*** - At the end of the growing period the grains were separated from the Plant 'Head'. A sample of -200 grains were weight, photographed and images were processed using the below described image processing system. The grain area was measured from those images and was divided by the number of grains.

***Average Grain Length (cm)*** - At the end of the growing period the grains were separated from the Plant 'Head'. A sample of -200 grains were weight, photographed and images were processed using the below described image processing system. The sum of grain lengths (longest axis) was measured from those images and was divided by the number of grains.

***Head Average Area (cm²)*** At the end of the growing period 5 'Heads' were, photographed and images were processed using the below described image processing system. The 'Head' area was measured from those images and was divided by the number of 'Heads'.

***Head Average Length (cm)*** At the end of the growing period 5 'Heads' were, photographed and images were processed using the below described image processing system. The 'Head' length (longest axis) was measured from those images and was divided by the number of 'Heads'.

The image processing system was used, which consists of a personal desktop computer (Intel P4 3.0 GHz processor) and a public domain program - ImageJ 1.37, Java based image processing software, which was developed at the U.S. National Institutes of Health and is freely available on the internet at Hypertext Transfer Protocol://rsbweb (dot) nih (dot) gov/. Images were captured in resolution of 10 Mega Pixels (3888x2592 pixels) and stored in a low compression JPEG (Joint Photographic Experts Group standard) format. Next, image processing output data for seed area and seed length was saved to text files and analyzed using the JMP statistical analysis software (SAS institute).

Additional parameters were collected either by sampling 5 plants per plot or by measuring the parameter across all the plants within the plot.

***Total Seed Weight*/*Head (gr.)*** - At the end of the experiment (plant 'Heads') heads from plots within blocks A-C were collected. 5 heads were separately threshed and grains were weighted, all additional heads were threshed together and weighted as well. The average grain weight per head was calculated by dividing the total grain weight by number of total heads per plot (based on plot). In case of 5 heads, the total grains weight of 5 heads was divided by 5.

***FW Head*/*Plant gr*** - At the end of the experiment (when heads were harvested) total and 5 selected heads per plots within blocks A-C were collected separetaly. The heads (total and 5) were weighted (gr.) separately and the average fresh weight per plant was calculated for total (FW Head/Plant gr based on plot) and for 5 (FW Head/Plant gr based on 5 plants).

***Plant height*** - Plants were characterized for height during growing period at 5 time points. In each measure, plants were measured for their height using a measuring tape. Height was measured from ground level to top of the longest leaf.

***Plant leaf number*** - Plants were characterized for leaf number during growing period at 5 time points. In each measure, plants were measured for their leaf number by counting all the leaves of 3 selected plants per plot.

***Relative Growth Rate*** was calculated using Formulas X and XI. $Relative growth rate of plant height = Regression coefficient of plant height along time course.$ $Relative growth rate of plant leaf number = Regression coefficient of plant leaf number along time course.$

***SPAD*** - Chlorophyll content was determined using a Minolta SPAD 502 chlorophyll meter and measurement was performed 64 days post sowing. SPAD meter readings were done on young fully developed leaf. Three measurements per leaf were taken per plot.

***Vegetative dry weight and Heads*** - At the end of the experiment (when Inflorescence were dry) all Inflorescence and vegetative material from plots within blocks A-C were collected. The biomass and Heads weight of each plot was separated, measured and divided by the number of Heads.

***Dry weight*** = total weight of the vegetative portion above ground (excluding roots) after drying at 70 °C in oven for 48 hours;
***Harvest Index (HI) (Sorghum)-*** The harvest index was calculated using Formula XII. $Harvest Index = Average grain dry weight per Head / \left(Average vegetative dry weight per Head + Average Head dry weight\right)$

***FW Headsl(FW Heads* + *FW Plants)*** - The total fresh weight of heads and their respective plant biomass were measured at the harvest day. The heads weight was divided by the sum of weights of heads and plants.

### Experimental Results

16 different sorghum varieties were grown and characterized for different parameters: The average for each of the measured parameter was calculated using the JMP software (Tables 19-20) and a subsequent correlation analysis between the various transcriptom sets (Table 17) and the average parameters, was conducted (Tables 21). Results were then integrated to the database.

**Table 18**

| ***Sorghum correlated parameters (vectors)*** | |
|---|---|
| ***Correlation Vector*** | ***Correlation Id*** |
| Average Seed Area cm2-normal | A |
| Average Seed Length cm-normal | B |
| FW/Plant gr based on plot-normal | C |
| FW Head/Plant gr based on 5 plants-normal | D |
| FW Head/Plant gr based on plot-normal | E |
| FW Heads/(FW Heads + FW Plants) based on plot-normal | F |
| Head Average Area cm2-normal | G |
| Head Average Length cm-normat | H |
| HI-normal | J |
| Leaf SPAD 64 Days Post Sowing-normal | K |
| Relative Growth Rate of Leaf Num-normal | L |
| Relative Growth Rate of Plant Heiht-normal | M |
| Total Seed Weight/Head gr based on plot-normal | N |
| Total Seed Weight /Head gr based on 5 heads-normal | O |

Table 18. Provided are the Sorghum correlated parameters (vectors). "gr." = grams; "SPAD" = chlorophyll levels; "FW" = Plant Fresh weight;"normal" = standard growth conditions.

**Table 19**

| ***Measured parameters in Sorghum accessions*** | | | | | | | | | |
|---|---|---|---|---|---|---|---|---|---|
| ***Seed Id*** | ***A*** | ***B*** | ***C*** | ***D*** | ***E*** | ***F*** | ***G*** | ***H*** | ***J*** |
| 20 | 0.1047 | 0.3856 | 162.6 | 406.5 | 175.2 | 0.51 | 120.1 | 25.58 | 200.7 |
| 21 | 0.1124 | 0.4017 | 212.6 | 518 | 223.5 | 0.5101 | 167.6 | 26.84 | 127 |
| 22 | 0.1313 | 0.4446 | 334.8 | 148 | 56.4 | 0.1154 | 85.14 | 21.02 | 51.8 |
| 24 | 0.1293 | 0.4496 | 313.5 | 423 | 111.6 | 0.2626 | 157.3 | 26.84 | 122.4 |
| 25 | | | | | | 0.1204 | | | 54.53 |
| 26 | | | | | | 0.177 | | | 93.92 |
| 27 | 0.1098 | 0.3999 | 151.1 | 423.5 | 126.2 | 0.4591 | 168.5 | 31.33 | 327.3 |
| 28 | 0.1134 | 0.4054 | 137.6 | 386.5 | 107.7 | 0.4316 | 109.3 | 23.18 | 231.5 |
| 29 | 0.1022 | 0.3837 | 168 | 409.5 | 123.9 | 0.4249 | 135.1 | 25.7 | 241.4 |
| 30 | 0.118 | 0.4186 | 129 | 328.9 | 102.8 | 0.4416 | 169 | 28.82 | 304.1 |
| 31 | 0.1205 | 0.4302 | 97.62 | 391 | 82.33 | 0.4581 | 156.1 | 28.13 | 335.6 |
| 32 | 0.1106 | 0.4003 | 99.32 | 435.8 | 77.59 | 0.4473 | 112.1 | 22.97 | 349.6 |
| 33 | 0.1165 | 0.4094 | 112.2 | 429.5 | 91.17 | 0.4474 | 154.7 | 28.09 | 293.2 |
| 34 | 0.108 | 0.4008 | 157.4 | 441 | 150.4 | 0.5134 | 171.7 | 30 | 410.9 |
| 35 | 0.1048 | 0.3947 | 130.5 | 415.8 | 109.1 | 0.4595 | 168.5 | 30.54 | 285.1 |
| 36 | 0.1097 | 0.3953 | 135.7 | 429.5 | 107.6 | 0.4425 | 162.5 | 27.17 | 282.7 |
| 37 | 0.1053 | 0.3924 | 209.2 | 428.5 | 130.9 | 0.3856 | 170.5 | 29.26 | 204 |

Table 19: Provided are the values of each of the parameters (as described above) measured in Sorghum accessions (Seed ID) under normal and drought conditions. Growth conditions are specified in the experimental procedure section.

**Table 20**

| ***Additional measured parameters in Sorghum accessions*** | | | | |
|---|---|---|---|---|
| ***Seed Id*** | ***L*** | ***M*** | ***N*** | ***O*** |
| 20 | 0.1032 | 1.891 | 31.12 | 47.4 |
| 21 | | 1.622 | 26.35 | 46.3 |
| 22 | 0.2128 | 3.418 | 18.72 | 28.37 |
| 24 | 0.1862 | 2.425 | 38.38 | 70.4 |
| 25 | 0.1898 | 3.118 | | |
| 26 | 0.1599 | 3.323 | | |
| 27 | 0.1957 | 2.178 | 47.67 | 63.45 |

| ***Seed Id*** | ***L*** | ***M*** | ***N*** | ***O*** |
|---|---|---|---|---|
| 28 | 0.1694 | 2.188 | 31 | 44.45 |
| 29 | 0.1821 | 2.572 | 39.99 | 56.65 |
| 30 | | 2.046 | 38.36 | 60 |
| 31 | | 2.069 | 32.1 | 45.45 |
| 32 | 0.1754 | 2.547 | 32.69 | 58.19 |
| 33 | 0.117 | 2.327 | 32.79 | 70.6 |
| 34 | 0.207 | 3.039 | 51.53 | 70.1 |
| 35 | 0.1859 | 2.335 | 35.71 | 53.95 |
| 36 | 0.151 | 2.516 | 38.31 | 59.87 |
| 37 | 0.24 | 2.81 | 42.44 | 52.65 |

Table 20: Provided are the values of each of the parameters (as described above) measured in Sorghum accessions (Seed ID) under normal and drought conditions. Growth conditions are specified in the experimental procedure section.

**Table 21**

| ***Correlation between the expression level of selected genes in various tissues and the phenotypic performance under normal or abiotic stress conditions across Sorghum accessions*** | | | | | |
|---|---|---|---|---|---|
| **Gene Name** | **Cluster Name** | **Exp. Set** | **Corr. Vec.** | **R** | **P** |
| LYM174 | sorghum\|gb161.crp\|AW284303 | 1 | J | 0.746 | 0.013251 |
| LYM263 | sorghum\|gb161crp\|AI622410 | 2 | O | 0.860 | 0.00292 |
| LYM263 | sorghum\|gb161.crp\|AI622410 | 2 | J | 0.847 | 0.00196 |
| LYM5 H21 | sorghum\|09v1\|SB04G031180 | 1 | B | 0.898 | 0.00101 |
| LYM5 H21 | sorghum\|09v1\|SB04G031180 | 1 | A | 0.896 | 0.00107 |
| LYM8 H23 | sorghum\|09v1\|SB01G000490 | 1 | O | 0.755 | 0.018718 |
| LYM8 H23 | sorghum\|09v1\|SB01G000490 | 2 | O | 0.714 | 0.030884 |
| LYM10 H272 | sorghum\|09v1\|SB04G005280 | 3 | L | 0.756 | 0.029952 |
| LYM14 H44 | sorghum\|09v1\|SB02G044050 | 2 | E | 0.769 | 0.015504 |
| LYM24 H10 | sorghum\|09v1 \|SB03G044280 | 2 | C | 0.774 | 0.014343 |
| LYM24 H10 | sorghum\|09v1\|SB03G044280 | 3 | B | 0.743 | 0.021898 |
| LYM24 H10 | sorghum\|09v1 \|SB03G044280 | 3 | A | 0.728 | 0.026226 |
| LYM35 H7 | sorghum\|09v1\|SB06G031730 | 2 | E | 0.823 | 0.006385 |
| LYM73 H8 | sorghum\|09v1\|SB07G004300 | 1 | E | 0.748 | 0.02039 |
| LYM82 H16 | sorghum\|09v1\|SB10G002420 | 3 | J | 0.783 | 0.007439 |
| LYM82 H16 | sorghum\|09v1\|SB10G002420 | 3 | N | 0.780 | 0.013111 |
| LYM82 H16 | sorghum\|09v10G002420 | 3 | O | 0.726 | 0.02673 |
| LYM82 H16 | sorghum\|09v1 \|SB 10G002420 | 3 | G | 0.723 | 0.027818 |
| LYM111 H10 | sorghum\|09v1\|SB03G036350 | 3 | C | 0.892 | 0.00122 |
| LYM111 H10 | sorghum\|09v1\|SB03G036350 | 3 | A | 0.753 | 0.019062 |
| LYM111 H10 | sorghum\|09v1\|SB03G036350 | 3 | B | 0.702 | 0.035192 |
| LYM119 H1 | sorghum\|09v1\|SB05G003680 | 2 | C | 0.759 | 0.017691 |
| LYM131 H19 | sorghum\|09v1\|SB06G027970 | 1 | E | 0.841 | 0.004488 |
| LYM131 H19 | sorghum\|09v1\|SB06G027970 | 3 | B | 0.787 | 0.011775 |
| LYM131 H19 | sorghum\|09v1\|SB06G027970 | 3 | A | 0.762 | 0.017013 |
| LYM131 H19 | sorghum\|09v1\|SB06G027970 | 1 | F | 0.700 | 0.024184 |
| LYM131 H20 | sorghum\|09v1\|SB07G006320 | 1 | E | 0.854 | 0.003353 |
| LYM137 H285 | sorghum\|09v1\|SB06G021660 | 2 | F | 0.747 | 0.013056 |
| LYM137 H285 | sorghum\|09v1\|SB06G021660 | 1 | E | 0.716 | 0.030132 |
| LYM137 H286 | sorghum\|09v1 \|SB 10G005240 | 1 | E | 0.786 | 0.012037 |
| LYM 140 H27 | sorghum\|09v1\|SB06G028990 | 1 | C | 0.772 | 0.014832 |
| LYM148 H14 | sorghum\|09v1\|SB10G026570 | 1 | B | 0.812 | 0.007885 |
| LYM148 H14 | sorghum\|09v1\|SB10G026570 | 1 | A | 0.770 | 0.015311 |
| LYM148 H14 | sorghum\|09v1\|SB10G026570 | 1 | C | 0.768 | 0.015705 |
| LYM162 H7 | sorghum\|09v1\|SB03G043995 | 1 | N | 0.837 | 0.004911 |
| LYM215 H2 | sorghum\|09v1\|SB03G043980 | 1 | N | 0.718 | 0.029262 |
| LYM178 H13 | sorghum\|09v1\|SB03G008890 | 3 | B | 0.862 | 0.002833 |
| LYM178 H13 | sorghum\|09v1\|SB03G008890 | 3 | A | 0.792 | 0.010892 |
| LYM178 H14 | sorghum\|09v1\|SB07G001060 | 2 | A | 0.768 | 0.01572 |
| LYM179 H0 | sorghum\|09v1\|SB08G006470 | 1 | O | 0.818 | 0.006992 |
| LYM109 H2 | sorghum\|09v1\|SB05G003660 | 1 | B | 0.762 | 0.017077 |
| LYM109 H2 | sorghum\|09v1\|SB05G003660 | 3 | A | 0.751 | 0.019579 |
| LYM109 H2 | sorghum\|09v 1 \|SB05G003660 | 1 | A | 0.732 | 0.025074 |
| LYM112 H2 | sorghum\|09v1\|SB02G039985 | 1 | B | 0.827 | 0.005944 |
| LYM112 H2 | sorghum\|09v1\|SB02G039985 | 3 | B | 0.790 | 0.011246 |
| LYM112 H2 | sorghum\|09v1\|SB02G039985 | 1 | A | 0.789 | 0.011426 |
| LYM112 H2 | sorghum\|09v1\|SB02G039985 | 3 | A | 0.701 | 0.035452 |
| LYM123 H7 | sorghum\|09v1\|SB06G031680 | 1 | B | 0.769 | 0.015524 |
| LYM181 H6 | sorghum\|09v1\|SB02G034110 | 1 | B | 0.740 | 0.022556 |
| LYM181 H6 | sorghum\|09v1\|SB02G034110 | 3 | N | 0.724 | 0.027264 |
| LYM181 H6 | sorhum\|09v1\|SB02G034110 | 3 | O | 0.702 | 0.035114 |
| LYM182 H12 | sorghum\|09v1\|SB06G015280 | 1 | E | 0.767 | 0.015834 |
| LYM206 H2 | sorghum\|09v1\|SB07G021090 | 3 | N | 0.795 | 0.010488 |
| LYM188 H13 | sorghum\|09v1\|SB01G007950 | 1 | E | 0.788 | 0.011658 |
| LYM198 H1 | sorghum\|09v1\|SB01G045460 | 1 | E | 0.829 | 0.005689 |
| LYM201 H37 | sorghum\|09v1\|SB04G022350 | 2 | N | 0.741 | 0.022246 |
| LYM201 H37 | sorghum\|09v1\|SB04G022350 | 2 | D | 0.709 | 0.032326 |
| LYM201 H37 | sorghum\|09v1\|SB04G022350 | 2 | H | 0.701 | 0.035468 |
| LYM207 H3 | sorghum\|09v1\|SB06G023870 | 3 | K | 0.781 | 0.007669 |
| LYM207 H3 | sorghum\|09v1\|SB06G023870 | 1 | E | 0.768 | 0.015595 |
| LYM207 H3 | sorghum\|09v1\|SB06G023870 | 3 | O | 0.718 | 0.029344 |
| LYM207 H3 | sorghum\|09v1\|SB06G023870 | 1 | N | 0.716 | 0.02988 |
| LYM208 H8 | sorghum\|09v1\|SB01G032070 | I | N | 0.734 | 0.024302 |
| LYM208 H8 | sorghum\|09v1\|SB01G032070 | 1 | E | 0.701 | 0.03525 |
| LYM212 H9 | sorghum\|09v1\|SB01G045480 | 1 | E | 0.841 | 0.004537 |
| LYM221 H3 | sorghum\|09v1\|SB01G03461 0 | 2 | A | 0.728 | 0.02604 |
| LYM221 H3 | sorghum\|09v1\|SB01G034610 | 2 | C | 0.721 | 0.028264 |
| LYM224 H3 | sorghum\|09v1\|SB02G040000 | 3 | C | 0.835 | 0.005096 |
| LYM224 H3 | sorghum\|09v1\|SB02G040000 | 3 | L | 0.827 | 0.011397 |
| LYM224 H3 | sorghum\|09v1\|SB02G040000 | 3 | M | 0.708 | 0.021888 |
| LYM232 H3 | sorghum\|09v 1 \|SB02G000450 | 1 | O | 0.808 | 0.00839 |
| LYM232 H3 | sorghum\|09v1\|SB02G000450 | 1 | N | 0.759 | 0.017814 |
| LYM236 H139 | sorghum\|09v1\|SB09G0291 10 | 1 | A | 0.859 | 0.00303 |
| LYM236 H139 | sorghum\|09v1\|SB09G029110 | 1 | B | 0.836 | 0.004971 |
| LYM248 H5 | sorghum\|09v1\|SB04G000645 | 3 | L | 0.878 | 0.004118 |
| LYM248 H5 | sorghum\|09v1\|SB04G000645 | 1 | N | 0.868 | 0.002424 |
| LYM183 H11 | sorghum\|09v1\|SB01G003070 | 1 | E | 0.832 | 0.005437 |
| LYM183 H11 | sorghum\|09v1\|SB01G003070 | 1 | N | 0.749 | 0.020124 |
| LYM267 H1 | sorghum\|09v 1\|SB0 1G044240 | 2 | A | 0.797 | 0.010102 |
| LYM267 H1 | sorghum\|09v1\|SB01G044240 | 2 | B | 0.753 | 0.019206 |
| LYM267 H 1 | sorghum\|09v1\|SB01G044240 | 1 | H | 0.707 | 0.033248 |
| LYM267 H1 | sorghum\|09v\| \|SB0 1G044240 | 1 | O | 0.705 | 0.033872 |
| LYM267 H1 | sorghum\|09v1 \|SB0 1G044240 | 1 | N | 0.705 | 0.033976 |
| LYM270 H0 | sorghum\|09v1\|SB02G040045 | 1 | E | 0.820 | 0.006742 |
| LYM271 H10 | sorghum\|09v1\|SB02G040020 | 1 | N | 0.780 | 0.013152 |
| LYM273 H6 | sorghum\|09v1\|SB03G044410 | 3 | B | 0.955 | 5.9 1E-05 |
| LYM273 H6 | sorghum\|09v1\|SB03G044410 | 3 | A | 0.950 | 8.6E-05 |
| LYM284 H24 | sorghum\|09v1\|SB03G027960 | 1 | E | 0.867 | 0.00247 |
| LYM289 H52 | sorghum\|09v1\|SB09G005410 | 3 | O | 0.928 | 0.000307 |
| LYM289 H52 | sorghum\|09v1\|SB09G005410 | 3 | N | 0.830 | 0.005588 |
| LYM289 H52 | sorghum\|09v1\|SB09G005410 | 3 | H | 0.781 | 0.012924 |
| LYM289 H52 | sorghum\|09v1\|SB09G005410 | 3 | G | 0.714 | 0.03066 |
| LYM289 H52 | sorghum\|09v1\|SB09G005410 | 3 | D | 0.704 | 0.03412 |
| LYM290 H13 | sorghum\|09v1\|SB01G009390 | I | E | 0.766 | 0.01617 |

Table 21. Provided are the correlations (R) between the expression levels of yield improving genes and their homologs in tissues (Flag leaf, Flower meristem and Flower; Expression (Exp.) sets) and the phenotypic performance in various yield, biomass, growth rate and/or vigor components [Correlation (Corr.) vector (Vec.)] under stress conditions or normal conditions across Sorghum accessions.

***Sorghum vigor related parameters under 100 mM NaCl and low temperature*** (10 ± 2 °C) - Ten Sorghum varieties were grown in 3 repetitive plots, each containing 17 plants, at a net house under semi-hydroponics conditions. Briefly, the growing protocol was as follows: Sorghum seeds were sown in trays filled with a mix of vermiculite and peat in a 1:1 ratio. Following germination, the trays were transferred to the high salinity solution (100 mM NaCl in addition to the Full Hogland solution), low temperature (10 ± 2 °C in the presence of Full Hogland solution) or at Normal growth solution [Full Hogland solution at 28 ± 2 °C].

Full Hogland solution consists of: KNO₃ - 0.808 grams/liter, MgSO₄ - 0.12 grams/liter, KH₂PO₄ - 0.172 grams/liter and 0.01 % (volume/volume) of 'Super coratin' micro elements (Iron-EDDHA [ethylenediamine-N,N'-bis(2-hydroxyphenylacetic acid)]- 40.5 grams/liter; Mn - 20.2 grams/liter; Zn 10.1 grams/liter; Co 1.5 grams/liter; and Mo 1.1 grams/liter), solution's pH should be 6.5 - 6.8].

***RNA extraction*** - All 10 selected Sorghum varieties were sampled per each treatment. Two tissues [leaves and roots] growing at 100 mM NaCl, low temperature (10 ± 2 °C) or under Normal conditions (full Hogland at a temperature between 28 ± 2 °C) were sampled and RNA was extracted as described in Example 3 above.

**Table 22**

| ***Sorghum transcriptom expression sets*** | |
|---|---|
| ***Expression Set*** | ***Set ID*** |
| Sorghum roots under cold | 1 |
| Sorghum vegetative meristem NaCl | 2 |
| Sorghum vegetative meristem under low nitrogen | 3 |
| Sorghum vegetative meristem under cold conditions | 4 |
| Sorghum roots under NaCl | 5 |

| ***Expression Set*** | ***Set ID*** |
|---|---|
| Sorghum vegetative meristem under normal conditions | 6 |
| Sorghum roots under low nitrogen | 7 |
| Sorghum roots under normal | 8 |

Table 22: Provided are the Sorghum transcriptom expression sets. Cold conditions = 10 ± 2 °C; NaCl = 100 mM NaCl; low nitrogen =1.2 mM Nitrogen; Normal conditions = 16 mM Nitrogen.

### Experimental Results

10 different Sorghum varieties were grown and characterized for the following parameters: "Leaf number Normal" = leaf number per plant under normal conditions (average of five plants); "Plant Height Normal" = plant height under normal conditions (average of five plants); "Root DW 100 mM NaCl" - root dry weight per plant under salinity conditions (average of five plants); The average for each of the measured parameter was calculated using the JMP software and values are summarized in Table 24 below. Subsequent correlation analysis between the various transcriptom sets and the average parameters were conducted (Table 25). Results were then integrated to the database.

**Table 23**

| ***Sorghum correlated parameters (vectors)*** | |
|---|---|
| **Correlation Vector** | Corr. Id |
| DW Root/Plant - Cold | A |
| DW Root/Plant - 100 mM NaCl | B |
| DW Shoot/Plant - Low Nitrogen | C |
| DW Root/Plant - Low Nitrogen | D |
| Leaf number TP-3* - Cold | E |
| Leaf number TP-3*- 100 mM NaCl | F |
| Plant Height TP-3*- 100 mM NaCl | G |
| DW Shoot/Plant - Cold | H |
| DW Shoot/Plant - Normal | I |
| Plant Height TP-3* - Low Nitrogen | J |
| Leaf number TP-3* - Low Nitrogen | K |
| DW Shoot/Plant - 100 mM NaCl | L |
| Leaf number TP-3* - Normal | M |

Table 23: Provided are the Sorghum correlated parameters. Cold conditions = 10 ± 2 °C; NaCl = 100 mM NaCl; low nitrogen = 1.2 mM Nitrogen; Normal conditions = 16 mM Nitrogen * TP-3 refers to time point 3.

**Table 24**

| ***Sorghum accessions, measured parameters*** | | | | | | | | | |
|---|---|---|---|---|---|---|---|---|---|
| ***Seed ID*** | ***F*** | ***B*** | ***L*** | ***G*** | ***E*** | ***A*** | ***H*** | ***M*** | ***I*** |
| 20 | 3.67 | 0.35 | 0.66 | 14.63 | 3.88 | 0.83 | 1.03 | 4.17 | 0.81 |
| 22 | 3.88 | 1.45 | 2.43 | 16.31 | 4.16 | 0.95 | 1.34 | 4.48 | 1.89 |
| 26 | 4.28 | 1.49 | 2.40 | 20.56 | 4.52 | 1.47 | 1.71 | 4.93 | 2.51 |
| 27 | 4.03 | 0.81 | 1.61 | 14.70 | 4.28 | 1.06 | 1.28 | 4.53 | 1.26 |
| 28 | 3.97 | 1.03 | 1.77 | 16.43 | 4.33 | 0.71 | 1.12 | 4.52 | 1.55 |
| 29 | 3.98 | 0.95 | 1.66 | 16.12 | 4.17 | 1.38 | 1.69 | 4.64 | 1.50 |
| 30 | 3.90 | 2.00 | 2.23 | 15.61 | 3.94 | 2.04 | 2.24 | 4.49 | 1.93 |
| 31 | 4.18 | 1.39 | 2.76 | 18.71 | 4.26 | 1.03 | 1.26 | 4.79 | 1.95 |
| 34 | 3.70 | 1.29 | 1.29 | 13.65 | 4.20 | 1.01 | 1.08 | 4.37 | 1.48 |
| 37 | 3.82 | 1.76 | 1.55 | 15.72 | 4.04 | 1.01 | 1.02 | 4.54 | 1.85 |

Table 24: Provided are the measured parameters under 100 mM NaCl and low temperature (8-10 °C) conditions of Sorghum accessions (Seed ID) according to the Correlation ID numbers (described in Table 23 above) as follows: F [100 mM NaCl: leaf Number]; B [100 mM NaCl: Root DW]; L [100 mM NaCl: Shoot DW]; G [100 mM NaCl: Plant height]; E [low temperature: leaf Number]; A [low temperature: Root DW]; H [low temperature: Shoot DW];; M [Normal: leaf Number]; I [Normal: Shoot DW].

**Table 25**

| ***Correlation between the expression level of selected genes in roots and the phenotypic performance under normal or abiotic stress conditions across Sorghum accessions*** | | | | | |
|---|---|---|---|---|---|
| ***Gene Name*** | ***Cluster Id*** | ***Exp. Set*** | ***Corr. Vec.*** | ***R*** | ***P*** |
| LYM263 | sorghum\|gb161.crp\|AI622410 | 5 | G | 0.705635 | 0.183016 |
| LYM263 | sorphum\|gb161.crp\|AI622410 | 5 | F | 0.908761 | 0.032626 |
| LYM263 | sorghum\|gb161.crp\|AI622410 | 8 | I | 0.836212 | 0.004969 |
| LYM2 H8 | sorghum\|09v1\|SB7G004285 | 1 | A | 0.73969 | 0.01447 |
| LYM4 H11 | sorghum\|09v1\|SB03G000920 | 2 | B | 0.83675 | 0.00491 |
| LYM4 H11 | sorghum\|09v1\|SB03G000920 | 2 | B | 0.73187 | 0.02499 |
| LYM4 H11 | sorghum\|09v1\|SB03G000920 | 4 | E | 0.70634 | 0.03342 |
| LYM14 H43 | sorghum\|09v1\|SB01G038730 | 1 | A | 0.71433 | 0.02029 |
| LYM19 H12 | sorghum\|09v1\|SB05G009990 | 5 | F | 0.97628 | 0.00437 |
| LYM19 H12 | sorphum\|09v1\|SB05G009990 | 5 | G | 0.88580 | 0.04552 |
| LYM24 H10 | sorghum\|09v1\|SB03G044280 | 4 | H | 0.75256 | 0.01929 |
| LYM24 H10 | sorghum\|09v1\|SB03G044280 | 4 | H | 0.74948 | 0.02008 |
| LYM73 H8 | sorghum\|09v1\|SB07G004300 | 5 | F | 0.97233 | 0.00550 |
| LYM73 H8 | sorghum\|09v1\|SB07G004300 | 5 | F | 0.92449 | 0.02462 |
| LYM73 H8 | sorghum\|09v1\|SB07G004300 | 4 | E | 0.73646 | 0.02364 |
| LYM83 H12 | sorghum\|09v1\|SB09G026370 | 2 | F | 0.70736 | 0.03305 |
| LYM129 H4 | sorghum\|09v1\|SB03G044510 | 1 | E | 0.72429 | 0.01784 |
| LYM129 H4 | sorghum\|09v1\|SB03G044510 | 2 | F | 0.72339 | 0.02761 |
| LYM129 H4 | sorghum\|09v1\|SB03G044510 | 6 | I | 0.71891 | 0.02907 |
| LYM129 H4 | sorghum\|09v1\|SB03G044510 | 6 | I | 0.71123 | 0.03168 |
| LYM129 H4 | sorghum\|09v1\|SB03G044510 | 2 | F | 0.70792 | 0.03285 |
| LYM140 H27 | sorghum\|09v1\|SB06G028990 | 2 | G | 0.80589 | 0.00873 |
| LYM140 H27 | sorghum\|09v1\|SB06G028990 | 2 | F | 0.78965 | 0.01136 |
| LYM140 H27 | sorghum\|09v1\|SB06G028990 | 6 | I | 0.71625 | 0.02996 |
| LYM153 H9 | sorghum\|09v1\|SB10G0U3440 | 4 | H | 0.78966 | 0.01136 |
| LYM153 H9 | sorghum\|09v1\|SB10G003440 | 4 | H | 0.73143 | 0.02512 |
| LYM153 H9 | sorghum\|09v1\|SB10G003440 | 4 | A | 0.71026 | 0.03202 |
| LYM115 H0 | sorghum\|09v1\|SB01G043900 | 2 | F | 0.74903 | 0.02019 |
| LYM188 H13 | sorghum\|09v1\|SB01G007950 | 5 | F | 0.87882 | 0.04971 |
| LYM203 H14 | sorghum\|09v1\|SB04G005460 | 2 | B | 0.78001 | 0.01316 |
| LYM217 H3 | sorghum\|09v1\|SB01G043910 | 5 | B | 0.94269 | 0.01633 |
| LYM217 H3 | sorghum\|09v1\|SB01G043910 | 5 | B | 0.93691 | 0.01884 |
| LYM228 H1 | sorghum\|09v1\|SB09G006910 | 1 | A | 0.72334 | 0.01806 |
| LYM232 H3 | sorghum\|09v1\|SB02G000450 | 2 | L | 0.77653 | 0.01385 |
| LYM232 H3 | sorghum\|09v1\|SB02G000450 | 2 | F | 0.72326 | 0.02766 |
| LYM240 H12 | sohum\|09v1\|SB02G038240 | 4 | H | 0.76382 | 0.01659 |
| LYM240 H12 | sorghum\|09v1\|SB02G038240 | 2 | L | 0.73895 | 0.02293 |
| LYM251 H106 | sorghum\|09v1\|SB01G006180 | 5 | F | 0.95275 | 0.01224 |
| LYM284 H24 | sorghum\|09v1\|SB03G027960 | 6 | M | 0.76300 | 0.01677 |
| LYM289 H53 | sorghum\|09v1\|SB10G002980 | 5 | G | 0.91500 | 0.02936 |

Table 25. Provided are the correlations (R) between the expression levels yield improving genes and their homologs in various tissues [Expression (Exp.) sets] and the phenotypic performance [yield, biomass, growth rate and/or vigor components (Correlation vector)] under abiotic stress conditions (salinity) or normal conditions across Sorghum accessions. Corr. Vec. = correlation vector as described hereinabove (Table 23).

### EXAMPLE 7

### GENE CLONING AND GENERATION OF BINARY VECTORS FOR PLANT EXPRESSION

To validate their role in improving oil content, plant yield, seed yield, biomass, fiber yield and/or quality, growth rate, ABST, NUE and/or vigor, selected genes were over-expressed in plants, as follows.

### Cloning strategy

Genes listed in Examples 1-6 hereinabove were cloned into binary vectors for the generation of transgenic plants. For cloning, the full-length open reading frame (ORF) was first identified. In case of ORF-EST clusters and in some cases already published mRNA sequences were analyzed to identify the entire open reading frame by comparing the results of several translation algorithms to known proteins from other plant species. To clone the full-length cDNAs, reverse transcription (RT) followed by polymerase chain reaction (PCR; RT-PCR) was performed on total RNA extracted from leaves, flowers, siliques or other plant tissues, growing under normal conditions. Total RNA was extracted as described in Example 3 above. Production of cDNA and PCR amplification was performed using standard protocols described elsewhere (Sambrook J., E.F. Fritsch, and T. Maniatis. 1989. Molecular Cloning. A Laboratory Manual., 2nd Ed. Cold Spring Harbor Laboratory Press, New York.) which are well known to those skilled in the art. PCR products were purified using PCR purification kit (Qiagen). In case where the entire coding sequence was not found, RACE kit from Invitrogen (RACE = R apid A ccess to cDNA E nds) was used to access the full cDNA transcript of the gene from the RNA samples described above.

In case genomic DNA was cloned, as in the case of LYM122 (SEQ ID NO:3739) and LYM273 (SEQ ID NO:3738), the genes were amplified by direct PCR on genomic DNA extracted from leaf tissue using the DNAeasy kit (Qiagen Cat. No. 69104).

Usually, 2 sets of primers were synthesized for the amplification of each gene from a cDNA or a genomic sequence; an external set of primers and an internal set (nested PCR primers). When needed (e.g., when the first PCR reaction did not result in a satisfactory product for sequencing), an additional primer (or two) of the nested PCR primers were used. Table 26 below provides primers used for cloning of selected genes.

**Table 26**

| ***The PCR primers used for cloning the genes into high copy vectors*** | | |
|---|---|---|
| ***Gene Name*** | ***Restriction Enzymes used for cloning*** | ***Primers used for amplification*** |
| LYM1 | SalI,XbaI | LYM1_NF_SalI (SEQ ID NO: 3740) AAAGTCGACAGTAGGCAATCATGTGTGAGG |
| | | LYM 1_NR_XbaI (SEQ ID NO: 3741) AATTCTAGACTAAGCTAGAGAGCTCGACTAATGC |
| LYM10 | XhoI,KpnI | LYM10 NF XhoI (SEQ ID NO: 3742) ATACTCGAGTCTCCAACCTTGCGAAGG |
| | | LYM 10 EF Xhol (SEQ ID NO: 3743) ATACTCGAGAACCCGATCTCTCCAACC |
| | | LYM 10 NR KpnI (SEQ ID NO: 3744) TATGGTACCCCTGCGAATTCTTGCCTTAG |
| | | LYM 10 ER KpnI (SEQ ID NO: 3745) TATGGTACCTGACGCCACCCTCAACTC |
| LYM 100 | SalI,XbaI | LYM 100_NF_SalI (SEQ ID NO: 3746) AAAGTCGACAGGAAACCCTAACGAAGATACC |
| | | LYM 100_EF_SalI (SEQ ID NO: 3747) AAAGTCGACGGAAACATACAGGTCGATTGAG |
| | | LYM 100_NR_XbaI (SEQ ID NO: 3748) AAATCTAGAGGGAAAGTTTAGTAGCACCAAC |
| | | LYM 100_ER_XbaI (SEQ ID NO: 3749) AAATCTAGAATATAACGTTAGAGCGGAGTGG |
| LYM 102 | BamHI,XhoI | LYM102_NF_BamHI (SEQ ID NO: 3750) AAAGGATCCGAGCTGCTGATTGTGAGTCAAG |
| | | LYM102_NR_XhoI (SEQ ID NO: 3751) AAACTCGAGCTAGGACAGCACTTCAGATAAGACC |
| LYM 103 | BamHI,XhoI | LYM 103_NF_BamHI (SEQ ID NO: 3752) AAAGGATCCCGACCGAGTCAATCAATCC |
| | | LYM 103_NR_XhoI (SEQ ID NO: 3753) AAACTCGAGAACAAGTATGACAGGCCAACTC |
| LYM 105 | BamHI,XhoI | LYM 105_NF_BamHI (SEQ ID NO: 3754) AAAGGATCCTAGCTAGCTTACTCCACAGTGC |
| | | LYM 105_ EF_BamHI (SEQ ID NO: 3755) AAAGGATCCAGCCACACGCTTAGCTTAGC |
| | | LYM 105_NR_XhoI (SEQ ID NO: 3756) AAACTCGAGCGAGCAGAAATTAACAGCTAAC |
| | | LYM 105_ER_XhoI (SEQ ID NO: 3757) AAACTCGAGACTACAGATCCAAAGCACGAAC |
| LYM 106 | SalI,XbaI | LYM 106_NF_SalI (SEQ ID NO: 3758) AAAGTCGACACTCAACGTAGTTCCTCACCTG |
| | | LYM 106_NR_XbaI (SEQ ID NO: 3759) AAATCTAGAAAGCTTTAGTTCTAGCACACGAC |
| LYM 107 | BamHI,XhoI | LYM107_NF_BamHI (SEQ ID NO: 3760) AAAGGATCCGTACTCCTATATTAGGCTCGCTC |
| | | LYM 107_EF_BamHI (SEQ ID NO: 3761) AAAGGATCCCTGCGTACTCCTATATTAGGCTC |
| | | LYM 107_NR_XhoI (SEQ ID NO: 3762) AAACTCGAGAATTTGGTATCAGAAACCTTGC |
| | | LYM 107_ER_XhoI (SEQ ID NO: 3763) AATCTCGAGTGAATCACTCAGTGTGCATGAC |
| LYM109 | XhoI,StuI | LYM 109_F2_XhoI (SEQ ID NO: 3764) AAACTCGAGCCCAGCGGACTCCTACTCTG |
| | | LYM109_F2_ XhoI (SEQ ID NO: 3764) AAACTCGAGCCCAGCGGACTCCTACTCTG |
| | | LYM 109_R2_StuI (SEQ ID NO: 3765) TTTAGGCCTTCACAGTCTTACAAGTCCGATTGCC |
| | | LYM 109_R2_StuI (SEQ ID NO: 3765) TTTAGGCCTTCACAGTCTTACAAGTCCGATTGCC |
| LYM110 | BamHI,XhoI | LYM 110_NF_BamHI (SEQ ID NO: 3766) AAAGGATCCGAACCAAACCTCGGAGAAAC |
| | | LYM110_NR_XhoI (SEQ ID NO: 3767) AAACTCGAGACCATCACCTGTAATACAACTACC |
| LYM111 | XhoI,SacI | LYM111_ _NF_XhoI (SEQ ID NO: 3768) AAACTCGAGGAATCTGGTTGCTCATCTCATC |
| | | LYM11_ EF_XhoI (SEQ ID NO: 3769) AAACTCGAGCTTCACAACGGACGAGAGG |
| | | LYM1 11_NR_SacI (SEQ ID NO: 3770) AAAGAGCTCATAATCGTTGGAACTTGGAATC |
| | | LYM111_ER_SacI (SEQ ID NO: 3771) AAAGAGCTCACAGCTTATCCCTACATGCTTC |
| LYM112 | BamHI,XhoI | LYM1 12_NF_BamHI (SEQ ID NO: 3772) AAAGGATCCTCAATTGAATCAGATGCTCCAC |
| | | LYM112_EF_BamHI (SEQ ID NO: 3773) AAAGGATCCATTCCTTTGACCGATTTCTTG |
| | | LYM112_NR_XhoI (SEQ ID NO: 3774) AAACTCGAGCTAATTAAGACAAATCAGTGGCACC |
| | | LYM112_ER_XhoI (SEQ ID NO: 3775) AAACTCGAGACAGAAGGTCGATGTTGATCTG |
| LYM113 | SalI,XbaI | LYM113_NF_SalI (SEQ ID NO: 3776) AAAGTCGACTTCTTGATCTAAATTTGGGTGG |
| | | LYM113_EF_SalI (SEQ ID NO: 3777) AAAGTCGACACTAGCTCTGCACTTTCCCTG |
| | | LYM113_NR_XbaI (SEQ ID NO: 3778) AAATCTAGAGATTCAAGTGCGTTGTCTGTC |
| | | LYM113_ER_XbaI (SEQ ID NO: 3779) AAATCTAGACTTGGTATTTACAGGACAATCG |
| LYM115 | BamHI,XhoI | LYM115_F_BamHI (SEQ ID NO: 3780) AAAGGATCCTCGCCGCAGATGGAAGTCT |
| | | LYM115_ER_XhoI (SEQ ID NO: 3781) TTTCTCGAGCAAACTCGTCTGGAGATGGG |
| LYM116 | SalI,XbaI | LYM116_EF_SatI (SEQ ID NO: 3782) AAAGTCGACTTGGCTCCGGATATCGCA |
| | | LYM116_ER_XbaI (SEQ ID NO: 3783) AAATCTAGAAGGCAGATGTTCATAACCACAC |
| LYM117 | | LYM117_F2_BamHI (SEQ ID NO: 3784) AAAGGATCCCGTCGTCAAGTGCTGGC |
| | | LYM117_R2_EcoRV (SEQ ID NO: 3785) AGTGATATCTCAATGTTTAGGGTCTCGGCATG |
| LYM119 | SalI,XbaI | LYM119_NF_SalI (SEQ ID NO: 3786) AAAGTCGACATCGAGTTGTTCGTCCGTC |
| | | LYM119_NR_XbaI (SEQ ID NO: 3787) AAATCTAGAACACCAAGCGTACATCTCAGAC |
| LYM12 | XhoI,KpnI | LYM12 EF XhoI (SEQ ID NO: 3788) TTACTCGAGTGCTTCTCTTCTTTCCTCTCTG |
| | | LYM12 ER KpnI (SEQ ID NO: 3789) ATAGGTACCTCACAGCAAACTAACATGAACCG |
| LYM120 | BamHI,XhoI | LYM120_NF_BamHI (SEQ ID NO: 3790) AAAGGATCCGGAAGTCCGGAGTTGGAAG |
| | | LYM120_NR_XhoI (SEQ ID NO: 3791) AAACTCGAGCAGTCACTCACACGCTACTACG |
| LYM121 | BamHI,XhoI | LYM121_NF_BamHI (SEQ ID NO: 3792) AAAGGATCCACTGCTGACCAACTTCAGTGTC |
| | | LYM121_EF_BamHI (SEQ ID NO: 3793) AAAGGATCCGACAAGGCTATCACATCCAATC |
| | | LYM121_NR_XhoI (SEQ ID NO: 3794) AAACTCGAGTTCTAAAGAAACAATCACGCAC |
| | | LYM121_ER_XhoI (SEQ ID NO: 3795) AAACTCGAGAGCAGAAGAAACTAGGCATGTG |
| LYM122_G | | LYM122_EF_BamHI (SEQ ID NO: 3796) AAAGGATCCTGCAGCCCTGACACACAAC |
| | | LYM122_ER_XhoI (SEQ ID NO: 3797) AAACTCGAGACCATCATGTAATACCCACCTC |
| LYM125 | | LYM125_EF_BamHI (SEQ ID NO: 3798) AAAGGATCCCTGTGCTTGGAGTAGACACGAG |
| | | LYM125_ER_KpnI (SEQ ID NO: 3799) AAAGGTACCGGAGAATTTGGATCAGTGCAG |
| LYM127 | | LYM 127_F2_BamHI (SEQ ID NO: 3800) TTTGGATCCCTTCTTGCTGTCGAACACCAG |
| | | LYM 127_R2_XhoI (SEQ ID NO: 3801) TTTCTCGAGGTCATGGGATTCTTGTCAGATACTAG |
| LYM 128 | BamHI,XhoI | LYM 128_NF_BamHI (SEQ ID NO: 3802) TTTGGATCCTTCACACCTCACCGAGCG |
| | | LYM 128_EF_BamHI (SEQ ID NO: 3803) AAAGGATCCAACCCGTTCACACCTCACC |
| | | LYM 128_NR_XhoI (SEQ ID NO: 3804) AAACTCGAGGATCACTTGACAATTACCGTGC |
| | | LYM 128_ER_XhoI (SEQ ID NO: 3805) AAACTCGAGTATGCTGATATGCCAGGTTTAC |
| LYM 129 | SalI,XbaI | LYM129 _NF_SalI (SEQ ID NO: 3806) AAAGTCGACATTCAGTCTTGTCGGCTACATC |
| | | LYM 129_EF_SalI (SEQ ID NO: 3807) AAAGTCGACTAGATCAGCCTCGATTCATCTC |
| | | LYM 129_NR_XbaI (SEQ ID NO: 3808) AAATCTAGAGCTTAATCAGAAGAAACGAACC |
| | | LYM 129_ER_XbaI (SEQ ID NO: 3809) AAATCTAGAAATTGCACAATACATGAACACG |
| LYM 13 | SalI,BamHI | LYM 13_NF_SalI (SEQ ID NO: 3810) AAAGTCGACCAAGCGGTAGGAGATGAGG |
| | | LYM 13_NR_BamHI (SEQ ID NO: 3811) AAAGGATCCTTATAACAACTATTCCCGGTAAGC |
| LYM130 | SalI,XbaI | LYM 130_NF_SalI (SEQ ID NO: 3812) AAAGTCGACAGAAATTAAGTTGCCGGAGAG |
| | | LYM 130_NR_XbaI (SEQ ID NO: 3813) AAATCTAGAATGCAGATGAGAGCTCAAGATG |
| LYM131 | SalI,XhoI | LYM131_NF_SalI (SEQ ID NO: 3814) AAAGTCGACTCCCTACCCTAGTCGATCTCC |
| | | LYM 13 1_EF_SalI (SEQ ID NO: 3815) AAAGTCGACGACTCGTCTCCTCGTTGCTC |
| | | LYM131_NF_SalI (SEQ ID NO: 3814) AAAGTCGACTCCCTACCCTAGTCGATCTCC |
| | | LYM131_ ER_XhoI (SEQ ID NO: 3816) AAACTCGAGTATAACACAGGCATAAAGCAGC |
| LYM132 | BamHI,XhoI | LYM 132_EF_BamHI (SEQ ID NO: 3817) AAAGGATCCATATTGGAATGCTTCTGTCGTC |
| | | LYM 132_ER_XhoI (SEQ ID NO: 3818) AAACTCGAGTACACGATAATCACAAACCACG |
| LYM 134 | BamHI,XhoI | LYM134_NF_BamHI (SEQ ID NO: 3819) AAAGGATCCATGGTGATTCGGTTGTTGTTAG |
| | | LYM 134_EF_BamHI (SEQ ID NO: 3820) AAAGGATCCATCGTTGAATTGATGGTGATTC |
| | | LYM 134_NR_XhoI (SEQ ID NO: 3821) AAACTCGAGTCATACGTCGAAGAACCAGAAC |
| | | LYM 134_ER_XhoI (SEQ ID NO: 3822) AAACTCGAGTGAAACTTTCGCCAACTACAC |
| LYM 135 | | LYM135_NF_SalI (SEQ ID NO: 3823) AAAGTCGACTTCTGATCTGCTCAGCTAAAGG |
| | | LYM 135_NR_SacI (SEQ ID NO: 3824) AAAGAGCTCCTGATGCACAAATATGGTAACG |
| LYM 136 | BamHI,KpnI | LYM 136_NF_BamHI (SEQ ID NO: 3825) AAAGGATCCCCGGTTCTATGTGTAGGAAGAG |
| | | LYM 136_EF_BamHI (SEQ ID NO: 3826) AAAGGATCCCAGGATGAGTGTTGATCCATTC |
| | | LYM 136_NR_KpnI (SEQ ID NO: 3827) AAAGGTACCGTCACAAACGCCTCAACATATC |
| | | LYM 136_ER_KpnI (SEQ ID NO: 3828) AAAGGTACCTTCACCATATTGCTACGAAATC |
| LYM 137 | SalI,XbaI | LYM 137_NF_SalI (SEQ ID NO: 3829) AAAGTCGACAGTTCAAGAGGCTGTCCTGAG |
| | | LYM 137_NR_XbaI (SEQ ID NO: 3830) AAATCTAGATCCAATAACATAAGAAACCACG |
| LYM138 | SalI,SacI | LYM 138_EF_SalI (SEQ ID NO: 3831) AAAGTCGACAACGAACCACTCTTCTGCATC |
| | | LYM 138_ER_SacI (SEQ ID NO: 3832) AAAGAGCTCGAAGCAACCTGGAAATAAACTC |
| LYM14 | EcoRV,PstI | LYM 14_NF_EcoRV (SEQ ID NO: 3833) AAAGATATCCTCCTCAGATCCACCACCAC |
| | | LYM 14_NR_PstI (SEQ ID NO: 3834) AATCTGCAGCTAAAATATTCAGGGCTTGTTG |
| LYM140 | XhoI,SacI | LYM 140_F_XhoI (SEQ ID NO: 3835) AAACTCGAGCTCCAGCACACGGACGAG |
| | | LYM140_ER_SacI (SEQ ID NO: 3836) AAAGAGCTCTACGAGTACGAATTATTGCCAG |
| LYM141 | | LYM141_NF_BamHI (SEQ ID NO: 3837) AAAGGATCCACAAGCGTCTTCTTCGTCTTC |
| | | LYM141_NR_KpnI (SEQ ID NO: 3838) AAAGGTACCCCATGCCACCCTTACTATACTC |
| LYM 142 | SalI,SacI | LYM 142_NF_SaIB (SEQ ID NO: 3839) TAAGTCGACCACACAGAGCACAGCACAGAG |
| | | LYM 142_NR_SacB (SEQ ID NO: 3840) TGAGCTCTGAACATGCGACCGTATGC |
| LYM143 | SalI,XbaI | LYM143_NF_SalI (SEQ ID NO: 3841) AAAGTCGACCACTAGCGCACAGATCTCCTAC |
| | | LYM 143_NR_XbaI (SEQ ID NO: 3842) AAATCTAGAAATAGTGTCCATGAGACGAACG |
| LYM 144 | SalI,EcoRV | LYM 144_NF_SalI (SEQ ID NO: 3843) AAAGTCGACACGACGAGGAGGAGGATG |
| | | LYM144_NR_EcoRV (SEQ ID NO: 3844) AATGATATCACGCATGGATTTCTTTAAGTTG |
| LYM 145 | BamHI,XhoI | LYM 145_F2_BamHI (SEQ ID NO: 3845) ATCGGATCCTAGCTTTGCCCAGTTTTGCT |
| | | LYM 145_F2_BamHI (SEQ ID NO: 3845) ATCGGATCCTAGCTTTGCCCAGTTTTGCT |
| | | LYM 145_R2_XhoI (SEQ ID NO: 3846) TTTCTCGAGCTATGCAGTTTTAGCCTAAGGCAAG |
| | | LYM145_R2_XhoI (SEQ ID NO: 3846) TTTCTCGAGCTATGCAGTTTTAGCCTAAGGCAAG |
| LYM 146 | | LYM 146_F2_KpnI (SEQ ID NO: 3847) AAAGGTACCCGAGGTCGTCACGCACAG |
| | | LYM146_R2_KpnI (SEQ ID NO: 3848) AATGGTACCTGGGTGGTTAGACAGCAAGG |
| LYM147 | SalI,XbaI | LYM147_ _NF_SalI (SEQ ID NO: 3849) AAAGTCGACCTCTGGCGCTCTCCTATACTC |
| | | LYM 147_EF_SalI (SEQ ID NO: 3850) AAAGTCGACAGTACGTGTACGTTTCAGGGAG |
| | | LYM147_NR_XbaI (SEQ ID NO: 3851) AAATCTAGAAGTACCACTAGCAGAAAGGCAG |
| | | LYM147_ER_XbaI (SEQ ID NO: 3852) AAATCTAGATGGCACCCAATACTAGTACCAC |
| LYM148 | BamHI,XhoI | LYM 148_NF_BamHI (SEQ ID NO: 3853) AAAGGATCCCTTACCCTTCCCTGAGATCC |
| | | LYM 148_NR_XhoI (SEQ ID NO: 3854) AAACTCGAGCTAACTACCAAAGTTCAAGCAGCTC |
| LYM 149 | SalI,XbaI | LYM 149_NF_SalI (SEQ ID NO: 3855) AAAGTCGACACCATGAGTTCATAACAAGAAGG |
| | | LYM149_NR_XbaI (SEQ ID NO: 3856) AAATCTAGACTAATACATGGAAGTGCAGACATGC |
| LYM15 | SalI,XbaI | LYM15_NF_SalI (SEQ ID NO: 3857) AAAGTCGACAGGTACAGTATAGTATGACACCGAC |
| | | LYM 15_NR_XbaI (SEQ ID NO: 3858) AATTCTAGACTACTGTTAACCGCTGATTATATCC |
| LYM 152 | SalI,XbaI | LYM 152_NF_SalI (SEQ ID NO: 3859) TTTGTCGACGAAGAAGAGATGGGAGTTTTCTC |
| | | LYM152_NR_XbaI (SEQ ID NO: 3860) AAATCTAGAATTTCTGACATTACATTATAGTCTCG |
| LYM153 | SalI,XbaI | LYM153_NF_SalI (SEQ ID NO: 3861) AAAGTCGACTTCTCCTCCTACGTTCTACTGG |
| | | LYM 153_NR_XbaI (SEQ ID NO: 3862) AAATCTAGACTAACAGGGTTTCTCCACTAAGTAAG |
| LYM155 | SalI,XbaI | LYM 155_NF_SalI (SEQ ID NO: 3863) AAAGTCGACTCCACTATAAGCAACGCACC |
| | | LYM 155_EF_SalI (SEQ ID NO: 3864) AAAGTCGACGAAGGAAACTCGGTGACACG |
| | | LYM155_NR_XbaI (SEQ ID NO: 3865) AAATCTAGAATGCCATGCTACTAAGAACCTAC |
| | | LYM 155_ER_XbaI (SEQ ID NO: 3866) AAATCTAGATAAACATCTCATGCCATGCTAC |
| LYM156 | StuI,StuI | LYM 156_NF_StuI (SEQ ID NO: 3867) TTTAGGCCTCAAGATCCGCAGAGATGATC |
| | | LYM 156 NR StuI_2 (SEQ ID NO: 3868) AAAAGGCCTTTAAGTGCTTGCGTCGTTTACAG |
| LYM157_G | XbaI,SacI | LYM157_EF_Xba_B (SEQ ID NO: 3869) AA TCT AGACCTCGAGCCACCCACTTTC |
| | | LYM 157_ER_Sac_B (SEQ ID NO: 3870) TGAGCTCTCACCTTCATCTTGTCTTCACTGGT |
| LYM159 | SalI,XbaI | LYM 159_NF_SalI (SEQ ID NO: 3871) AAAGTCGACCTCTACCTTCTTCTTCGGTCAG |
| | | LYM 159_NR_XbaI (SEQ ID NO: 3872) AAATCTAGAAGCTTAGCTAGGCCAACAATAC |
| LYM 16 | SalI,XbaI | LYM16 NF SalI (SEQ ID NO: 3873) CTAGTCGACAAGAAATTGGCACAGAAATGG |
| | | LYM16 NR XbaI (SEQ ID NO: 3874) TATTCTAGATCAAAGAGCCTAGTGAGCGTCTTC |
| LYM160 | SalI,XbaI | LYM160_F2_SalI (SEQ ID NO: 3875) AAAGTCGACAGGCCAGACCAAAACCATG |
| | | LYM 160_F2_SalI (SEQ ID NO: 3875) AAAGTCGACAGGCCAGACCAAAACCATG |
| | | LYM160_NR_XbaI (SEQ ID NO: 3876) AAATCTAGAAGAGTAACATGGACACACGACC |
| | | LYM 160_R2_XbaI (SEQ ID NO: 3877) AATTCTAGATCAGTACAAGAGCCAGATGTCTGA |
| LYM161 | BamHI,XhoI | LYM 161_EF_BamHI (SEQ ID NO: 3878) AAAGGATCCGAGAGAGGAGCAAAGATTCACC |
| | | LYM161_ER_XhoI (SEQ ID NO: 3879) AAACTCGAGTACAGGATGGTTGGTCTTCTTC |
| LYM 162 | BamHI,XhoI | LYM 162_NF_BamHI (SEQ ID NO: 3880) TTTGGATCCGCATCTAAGCCGAATTGAAG |
| | | LYM 162_NR_XhoI (SEQ ID NO: 3881) AAACTCGAGCTATTTCATGCTCAGTACCTGCAC |
| LYM164 | SalI,XbaI | LYM 164_NF_SalI (SEQ ID NO: 3882) AAAGTCGACATCCAGATGCTTCACATTCTTG |
| | | LYM 164_NR_XbaI (SEQ ID NO: 3883) AAATCTAGATCGAGTTTGACACGAACTTATG |
| LYM165 | | LYM 165_F2_XhoI (SEQ ID NO: 3884) AAACTCGAGCTACTCCGATCGGATCCTGAC |
| | | LYM165_R2_SacI (SEQ ID NO: 3885) AAAGAGCTCAAACGACGCACGGTCTCAC |
| LYM17 | SmaI,KpnI | LYM 17 NF X/SmaI (SEQ ID NO: 3886) ATACCCGGGTCTCTCAAGATGGTGGTGCTG |
| | | LYM17 NR Kpnl (SEQ ID NO: 3887) TATGGTACCAAGGGCTTAGCAAATTCTTTC |
| LYM 170 | SalI,XbaI | LYM170_NF_SalI (SEQ ID NO: 3888) AAAGTCGACATTCTTCGACCTCCTAAACTCC |
| | | LYM170_EF_SalI (SEQ ID NO: 3889) AAAGTCGACAGTCTCACACAGATCGCTTCAC |
| | | LYM170_NR_XbaI (SEQ ID NO: 3890) AAATCTAGACTACCAACTCAGAACCAGGATGAG |
| | | LYM 170_ER_XbaI (SEQ ID NO: 3891) AAATCTAGACATACCTATAAGGCTATAACACTGC |
| LYM 172 | BamHI,XhoI | LYM 172_NF_BamHI (SEQ ID NO: 3892) AAAGGATCCCTCGTCTTCGTCTACTCCACC |
| | | LYM172_EF_BamHI (SEQ ID NO: 3893) AAAGGATCCCCTCACTCGTAGTCTCGTCTTC |
| | | LYM 172_NR_XhoI (SEQ ID NO: 3894) AAACTCGAGGGAGCTTTGGAGAATAACAAAC |
| | | LYM 172_ER_XhoI (SEQ ID NO: 3895) AAACTCGAGCAACAGGTAACTCATTTCCACC |
| LYM173 | BamHI,XhoI | LYM 173_NF_BamHI (SEQ ID NO: 3896) AAAGGATCCTCATCAGTTCCCTGTTCTTCAG |
| | | LYM 173_NR_XhoI (SEQ ID NO: 3897) AAACTCGAGATGACTGGACTAAAGCAACCAC |
| LYM 174 | BamHI,KpnI | LYM 174_NF_BamHI (SEQ ID NO: 3898) AAAGGATCCCTCTTGCTAGGAGTAGCCTGC |
| | | LYM 174_NR_KpnI (SEQ ID NO: 3899) AAAGGTACCTATTATCCTACATGCCACATGC |
| LYM 175 | SalI,XbaI | LYM 175_NF_SalI (SEQ ID NO: 3900) AAAGTCGACCACTCCCTCTTATAGCCCACC |
| | | LYM175_NR_XbaI (SEQ ID NO: 3901) AAATCTAGACTAAGTGTACAGTTCACGGCACG |
| LYM 176 | SalI,XbaI | LYM1 76_NF_SalI (SEQ ID NO: 3902) AAAGTCGACTCTCGTTTCTCCTACCCTACAG |
| | | LYM 176_NR_XbaI (SEQ ID NO: 3903) AAATCTAGACTAACAGTTTCCAGTCAAAGCTACAG |
| LYM178 | SalI,XbaI | LYM 178_NF-SalI (SEQ ID NO: 3904) AAAGTCGACCTATCCATCCGCCACAAGAC |
| | | LYM 178_NR_XbaI (SEQ ID NO: 3905) AAATCTAGAACACAAGACACCATTTCTGGAG |
| LYM 179 | SalI,XhoI | LYM 179_NF_SalI (SEQ ID NO: 3906) AAAGTCGACAGGATTTCTCTAGGATAGCAGC |
| | | LYM 179_EF_SalI (SEQ ID NO: 3907) AAAGTCGACCTCAGTCGAGCGAGGATTTC |
| | | LYM179_NR_XhoI (SEQ ID NO: 3908) AAACTCGAGAAACAGAGCCTAACAGACATGG |
| | | LYM 179_ER_XhoI (SEQ ID NO: 3909) AAACTCGAGGGGATGTTTAGACTGCTACAGG |
| LYM 180 | BamHI,XhoI | LYM 180_NF_BamHI (SEQ ID NO: 3910) TATGGATCCCGACCTTTGATACCAAGCAAG |
| | | LYM 180_NR_XhoI (SEQ ID NO: 3911) TTACTCGAGCACGGATTAGTTTGTAGTAGCATGG |
| LYM181 | | LYM181_F2_BamHI (SEQ ID NO: 3912) AATGGATCCTAAAAATGGCGGCTGCTACTC |
| | | LYM 181 _R2_EcoRV (SEQ ID NO: 3913) TTTGATATCTCATACACGGTTTCATATGGTCGG |
| LYM 183 | | LYM 183_EF_SalI (SEQ ID NO: 3914) AAAGTCGACATCAAACCAACGAGAGCACTAC |
| | | LYM 183_ER_XbaI (SEQ ID NO: 3915) AAATCTAGAACTTCAGTGTACTTTCCCTTGC |
| LYM184 | | LYM184_NF_BamHI (SEQ ID NO: 3916) AAAGGATCCAACACGACTTGTGAGTGAGAGC |
| | | LYM 184_EF_BamHI (SEQ ID NO: 3917) AAAGGATCCATATGAGTAACGCCATCAGGAG |
| | | LYM 184_NR_XhoI (SEQ ID NO: 3918) AAACTCGAGTGCCTCATTTAATCTTGGGTC |
| | | LYM184_ER_XhoI (SEQ ID NO: 3919) |
| | | AAACTCGAGGAAATTGCCTCATTTAATCTTG |
| LYM 185 | BamHI,KpnI | LYM185_NF_BamHI (SEQ ID NO: 3920) AAAGGATCCAATTCGAGATATTTGGCTGTTC |
| | | LYM 185_EF_BamHI (SEQ ID NO: 3921) AAAGGATCCAGATAGCAAGATAGTCCGGTTG |
| | | LYM 185_NR_KpnI (SEQ ID NO: 3922) AAAGGTACCGGTCTATCACAAGCATCCTCAC |
| | | LYM 185_ER_KpnI (SEQ ID NO: 3923) AAAGGTACCACCACCTTTGTGATTGTTTCTC |
| LYM 186 | SalI,XbaI | LYM 186_NF_SalI (SEQ ID NO: 3924) AAAGTCGACCGACCCAAATTGACATAACTC |
| | | LYM 186_NR_XbaI (SEQ ID NO: 3925) AAATCTAGAATAGCTGGAACCTGGTATTGAC |
| LYM 188 | BamHI,XhoI | LYM 188_EF_BamHI (SEQ ID NO: 3926) AAAGGATCCCGAGCTAGGGTTAGGGTTTC |
| | | LYM188_ER_XhoI (SEQ ID NO: 3927) AAACTCGAGCAACAACTCACGCTACACATTC |
| LYM 189 | SalI,XbaI | LYM189_NF_SalI (SEQ ID NO: 3928) AAAGTCGACCCACGTCCTAGAATGAAAGAG |
| | | LYM 189_EF_SalI (SEQ ID NO: 3929) AAAGTCGACTTCCTCTGCTTCCCACAGC |
| | | LYM 189_NR_XbaI (SEQ ID NO: 3930) AAATCTAGACTGTTCATTCACGGTTGCAC |
| | | LYM 189_ER_XbaI (SEQ ID NO: 3931) AAATCTAGAGCAAATCTGTCGCTTTATTAGG |
| LYM19 | SalI,XbaI | LYM 19_NF_SalI (SEQ ID NO: 3932) AAAGTCGACGAGAGAAGAGAGATGGTCCTCC |
| | | LYM19_NR_XbaI (SEQ ID NO: 3933) AAATCTAGATTATCATGCTGACTTCTTGCCAC |
| LYM192 | XhoI,EcoRV | LYM 192_EF_XhoI (SEQ ID NO: 3934) AAACTCGAGTGAGCAGCGAGCCCTAAC |
| | | LYM192_R_EcoRV (SEQ ID NO: 3935) |
| | | TTTGATATCTCACACTACTAGGGAGTGGAGTAGTAA CTTGA |
| LYM193 | BamHI,XhoI | LYM 193_NF _BamHI (SEQ ID NO: 3936) AAAGGATCCCTAGTAGTGTTCTTCCCATTCG |
| | | LYM193_EF_BamHI (SEQ ID NO: 3937) AAAGGATCCAACAATCCGTCCTTTCATTTG |
| | | LYM 193_NR_XhoI (SEQ ID NO: 3938) AAACTCGAGTAAACGACAGCGGTACACATAC |
| | | LYM 193_ER_XhoI (SEQ ID NO: 3939) AAACTCGAGTACATCTCTAGGCAGCAAACAG |
| LYM196 | | LYM196_NF_BamHI (SEQ ID NO: 3940) AAAGGATCCGAGGACACCGCTTGCTTTC |
| | | LYM 196_NR_XhoI (SEQ ID NO: 3941) AAACTCGAGAACCTTGGATATGACCAATCAG |
| LYM 197 | BamHI,XhoI | LYM 197_EF_BamHI (SEQ ID NO: 3942) AAAGGATCCCTGTTGCCACATCTAGTGGTTC |
| | | LYM 197_ER_XhoI (SEQ ID NO: 3943) AAACTCGAGCACAATTCAGCGATTATTTCAG |
| LYM 198 | BamHI,XhoI | LYM198_F2_BamHI (SEQ ID NO: 3944) ATTGGATCCTTCATTTCCGCCATCCGT |
| | | LYM 198_R2_XhoI (SEQ ID NO: 3945) AAACTCGAGCACCATCTCTTGCAGAAGGC |
| LYM2 | EcoRV,KpnI | LYM2_NF_EcoRV (SEQ ID NO: 3946) AAAGATATCCGGTAGGTAGATGAAATTAAGG |
| | | LYM2_NR_KpnI (SEQ ID NO: 3947) CGAGGTACCCTAATATGCAGGTCAGCACACAAG |
| LYM20 | EcoRV,KpnI | LYM20 NF EcoRV (SEQ ID NO: 3948) ATAGATATCACTCCGAATCCGACGCAC |
| | | LYM20 EF EcoRV (SEQ ID NO: 3949) ATAGATATCGAGATCCCAACTCCGAATCC |
| | | LYM20 NR KpnI (SEQ ID NO: 3950) TATGGTACCCTACGTAAATCTCAGCACATGC |
| | | LYM20 ER KpnI (SEQ ID NO: 3951) TATGGTACCCTTCTGCAACGTTATTTGAGG |
| LYM200 | BamHI,XhoI | LYM200_NF_BamHI (SEQ ID NO: 3952) AAAGGATCCACTTTACCGGGCTACCATTC |
| | | LYM200_EF_BamHI (SEQ ID NO: 3953) AAAGGATCCTTACAAGAGCCTGTGAGCTGAG |
| | | LYM200_NR_XhoI (SEQ ID NO: 3954) AAACTCGAGCTTATCTGGACCACACTTGGAC |
| | | LYM200_ER_XhoI (SEQ ID NO: 3955) AAACTCGAGAAGAAATACATAGCCCTCCTCC |
| LYM201 | BamHI,XhoI | LYM201_NF_BamHI (SEQ ID NO: 3956) AAAGGATCCGCCTCATCTCGGTTTACTATAAG |
| | | LYM201_NR_XhoI (SEQ ID NO: 3957) AAACTCGAGAAGTAGACACAAACCATCCTGG |
| LYM203 | BamHI,XhoI | LYM203_EF_BamHI (SEQ ID NO: 3958) AAAGGATCCTCTATCAAATCAGCCACCTGTC |
| | | LYM203_ER_XhoI (SEQ ID NO: 3959) AAACTCGAGCTAGCAACTTTGTAGACCAGACGTG |
| LYM204 | | LYM204_NF_BamHI (SEQ ID NO: 3960) AAAGGATCCCTACTACCAGACAGAGAGGACAGG |
| | | LYM204_EF_BamHI (SEQ ID NO: 3961) TTTGGATCCGCTTTCTGGCATCGCTACTAC |
| | | LYM204_NR_XhoI (SEQ ID NO: 3962) TGTCTCGAGTCAGTAGGAGTTTATGAGATGAACC |
| | | LYM204_ER_Xho (SEQ ID NO: 3963) AAACTCGAGTCAACTCATCATCCGGAACATGGTAC |
| LYM206 | XhoI,EcoRV | LYM206_EF_XhoI (SEQ ID NO: 3964) AAACTCGAGAATTCTAGCAAGGCAGCTCAG |
| | | LYM206_ER_EcoRV (SEQ ID NO: 4199) AAAGATATCTAAAGGAGTCGTAGCCCTCTC |
| LYM207 | | LYM207_EF_BamHI (SEQ ID NO: 3966) AAAGGATCCACTCTTCCAACCGCTCCTC |
| | | LYM207_ER_KpnI (SEQ ID NO: 4200) AAAGGTACCCTAGTCTTGCGAAGTGCGAG |
| LYM208 | BamHI,XhoI | LYM208_F2_BamHI (SEQ ID NO: 3968) AAAGGATCCTGCGGCTGAGTACAGACGAC |
| | | LYM208_R2_KpnI (SEQ ID NO: 3969) AAAGGTACCCATCAATCCATGCTAATGTAGAGC |
| LYM21 | EcoRV,KpnI | LYM21_NF_EcoRV (SEQ ID NO: 3970) AAAGATATCTCTCGCAGCACAAAGATGG |
| | | LYM21 NR Kpnl (SEQ ID NO: 3971) ATAGGTACCTCACCCTTAGTTCTTCACAGTGGTG |
| LYM212 | SalI,XbaI | LYM212_NF_SalI (SEQ ID NO: 3972) AAAGTCGACCTGATACCCATCCATCCACC |
| | | LYM212_EF_SalI (SEQ ID NO: 3973) AAAGTCGACACTGACAAACCGGACCCAC |
| | | LYM212_NR_XbaI (SEQ ID NO: 3974) AAATCTAGACTAGCAGAGCCGAAGTAGTACGAG |
| | | LYM212_ER_XbaI (SEQ ID NO: 3975) AAATCTAGACTAGAACGAAGTAGTACGAGCAAGC |
| LYM213 | BamHI,XhoI | LYM213_EF_BamHI (SEQ ID NO: 3976) AAAGGATCCCAGCTCATCAGAACACAGAAGG |
| | | LYM213_ER_XhoI (SEQ ID NO: 3977) AAACTCGAGTTCGACAATTTGCAATAGAAAG |
| LYM215 | BamHI,XhoI | LYM215_F2_BamHI (SEQ ID NO: 3978) AATGGATCCTTCCCTCCCACCGAAATG |
| | | LYM215_F2_BamHI (SEQ ID NO: 3978) AATGGATCCTTCCCTCCCACCGAAATG |
| | | LYM215_R2_XhoI (SEQ ID NO: 3979) AAACTCGAGGAGCATGCAAAATGGACTAGACT |
| | | LYM215-R2-XhoI (SEQ ID NO: 3979) AAACTCGAGGAGCATGCAAAATGGACTAGACT |
| LYM217 | SalI,XbaI | LYM217_F2_SalI (SEQ ID NO:4201 ) AAAGTCGACCGACCGATCCAAGTAGTGAGC |
| | | LYM217_R2_XbaI (SEQ ID NO:4202 ) AAATCTAGAAGCTGATAGGCCAGTCAATCC |
| LYM219 | BamHI,KpnI | LYM219_F_BamHI (SEQ ID NO: 3980) AAAGGATCCTAGCAGTCTCGATGGCCG |
| | | LYM219_F_BamHI (SEQ ID NO: 3980) AAAGGATCCTAGCAGTCTCGATGGCCG |
| | | LYM219_R_KpnI (SEQ ID NO: 3981) TTTGGTACCCGAGTCAGCTTTTGTAATGATAG |
| | | LYM219_R_KpnI (SEQ ID NO: 3981) TTTGGTACCCGAGTCAGCTTTTGTAATGATAG |
| LYM22 | SalI,XbaI | LYM22_NF_SalI (SEQ ID NO: 3982) AAAGTCGACTTAGCACACATGGCGTCTTC |
| | | LYM22_EF_SalI (SEQ ID NO: 3983) AAAGTCGACCATCGGCATCTTCCTAACTG |
| | | LYM22_NR_XbaI (SEQ ID NO: 3984) AATTCTAGATAATCTGTAGATGGCTGCCG |
| | | LYM22_ER_SmaI (SEQ ID NO: 3985) AATCCCGGGTAACAACGTACATGCAAGTCATC |
| LYM220 | BamHI,EcoRV | LYM220_NF_BamHI (SEQ ID NO: 3986) AAAGGATCCCGACTTCAAGCATCAGACTACC |
| | | LYM220_EF_BamHI (SEQ ID NO: 3987) AAAGGATCCAGCACACACATCCTCTAAGTGC |
| | | LYM220_NR_EcoRV (SEQ ID NO: 3988) AAAGATATCAACAGCAGTCACTTCACTCGTC |
| | | LYM220_ER_EcoRV (SEQ ID NO: 3989) AAAGATATCAAGTGGTACGGCTGAGTGTAAC |
| LYM221 | BamHI,XhoI | LYM221 _NF_BamHI (SEQ ID NO: 3990) AAAGGATCCACTGTCCACTGCGTCTGTCTC |
| | | LYM221_EF_BamHI (SEQ ID NO: 3991) AAAGGA TCCATCGTT AGAGGCTCAGAGTCAG |
| | | LYM221_NR_XhoI (SEQ ID NO: 3992) AAACTCGAGACTACGTATTACACGGAGGTGG |
| | | LYM221 _ER_XhoI (SEQ ID NO: 3993) AAACTCGAGTCTGCAGCATTCCTTAACCTAC |
| LYM223 | XhoI,SacI | LYM223_NF_XhoI (SEQ ID NO: 3994) AAACTCGAGACCTGCCTGCCACTATACTATC |
| | | LYM223_EF_XhoI (SEQ ID NO: 3995) AAACTCGAGAGACCCGTCTTAACTCTACCTG |
| | | LYM223_NR_SacI (SEQ ID NO: 3996) AAAGAGCTCAGCACCGGTTGATCTAGAATAC |
| | | LYM223_ER_SacI (SEQ ID NO: 3997) AAAGAGCTCATTTATCCACGAACCCATATTC |
| LYM224 | BamHI,XhoI | LYM224_EF_BamHI (SEQ ID NO: 3998) AAAGGATCCCAGGCCTCACGTGTCATTC |
| | | LYM224_EF_BamHI (SEQ ID NO: 3998) AAAGGATCCCAGGCCTCACGTGTCATTC |
| | | LYM224_R2_XhoI (SEQ ID NO: 3999) AAACTCGAGGTTTCCAGCCAACCAGAACAC |
| | | LYM224_ER_ XhoI (SEQ ID NO: 4000) AAACTCGAGGATCCAAATTGGTAATGCTTTG |
| LYM228 | | LYM228_NF_BamHI (SEQ ID NO: 4001) AAAGGATCCGCAAGCACTCCACTTCAAGC |
| | | LYM228_F2_BamHI (SEQ ID NO: 4002) AAAGGATCCCTCGAAGTGTCCAAGAAGAACACA |
| | | LYM228_R2_KpnI (SEQ ID NO: 4003) TAAGGTACCGAGCTGCAAACATAACGTCGAG |
| | | LYM228_R2_KpnI (SEQ ID NO: 4003) TAAGGTACCGAGCTGCAAACATAACGTCGAG |
| LYM23 | BamHI,KpnI | LYM23_NF_BamHI (SEQ ID NO: 4004) AAAGGATCCTCATCTCTCTCCCTCTCATCG |
| | | LYM23_NR_KpnI (SEQ ID NO: 4005) AAAGGTACCGTGCTGCTTCAACTATCCTCTC |
| LYM232 | | LYM232_EF_BamHI (SEQ ID NO: 4006) AAAGGATCCAAATTCCCAATTTCTTCGGTC |
| | | LYM232_ER_XhoI (SEQ ID NO: 4007) AAACTCGAGAGCACACACAGGTTCCTAAGAG |
| LYM236 | SalI,XbaI | LYM236_F_SalI (SEQ ID NO: 4008) AAAGTCGACGACTACCAATCCAATCTCCTCC |
| | | LYM236_ER_XbaI (SEQ ID NO: 4009) AAATCTAGAAGAAATGTATAATCGAAGTGCATC |
| LYM238 | | LYM238_EF_SmaI (SEQ ID NO: 4010) AAACCCGGGTAGTGGTGGAGAGACGAAACAC |
| | | LYM238_ER_SacI (SEQ ID NO: 4011) AAAGAGCTCCTACAAGTGCTGACTGCTGAAG |
| LYM239 | BamHI,XhoI | LYM239_EF_BamHI (SEQ ID NO: 4012) AAAGGATCCCTTGGTCCGTCTCCACTCTC |
| | | LYM239_R_XhoI (SEQ ID NO: 4013) AAACTCGAGCTAGGATTGGTACTCATTTCTTTGTG |
| LYM24 | SalI,XbaI | LYM24_NF_SalI (SEQ ID NO: 4014) AACGTCGACTCTTCTCTTTCTCTTCTCCTCG |
| | | LYM24_NR_XbaI (SEQ ID NO: 4015) ATATCTAGACATTCCAAACATTGTTATCAAAC |
| LYM240 | BamHI,KpnI | LYM240_NF_BamHI (SEQ ID NO: 4016) AAAGGATCCTACTGTAAGCAGTTTCCCACC |
| | | LYM240_EF_BamHI (SEQ ID NO: 4017) AAAGGATCCAACAACGCTCGTACTGTAAGC |
| | | LYM240_NR_KpnI (SEQ ID NO: 4018) AAAGGTACCACAAGTCATTCTACCAAGCACC |
| | | LYM240_ER_KpnI (SEQ ID NO: 4019) AAAGGTACCATACTTTCCTTGCTCTGCTGTC |
| LYM241 | , | LYM241_NF_BamHI (SEQ ID NO: 4020) AAAGGATCCAAACGGTTGGGAGGTTAGC |
| | | LYM241_NR_XhoI (SEQ ID NO: 4021) AAACTCGAGACTGGATCAGATTGTGAAGGTG |
| LYM242 | BamHI,XhoI | LYM242_NF_BamHI (SEQ ID NO: 4022) AAAGGATCCACGACTCCGACGAGCGAC |
| | | LYM242_NR_XhoI (SEQ ID NO: 4023) AAACTCGAGAACTCAAGTGGACAAATGTTGC |
| LYM243 | BamHI,XhoI | LYM243_EF_BamHI (SEQ ID NO: 4024) AAAGGATCCAGAAGCGTAGAGCGGTCAAG |
| | | LYM243_ER_XhoI (SEQ ID NO: 4025) AAACTCGAGCATTAAGCGAATTAACCATGTG |
| LYM245 | BamHI,KpnI | LYM245_F_BamHI (SEQ ID NO: 4026) AAAGGATCCGCTAGCTACTAGCAAATTGAAGC |
| | | LYM245_F_BamHI (SEQ ID NO: 4026) AAAGGATCCGCTAGCTACTAGCAAATTGAAGC |
| | | LYM245_NR_KpnI (SEQ ID NO: 4027) AAAGGTACCGGTCACCCGTTAGACTTATGC |
| | | LYM245_ER_KpnI (SEQ ID NO: 4028) AAAGGTACCTGGTAAATTATGGGTATTCAGC |
| LYM248 | BamHI,EcoRV | LYM248_F_BamHI (SEQ ID NO: 4029) AAAGGATCCACCACCGCTCGTCTCCAC |
| | | LYM248_NR_EcoRV (SEQ ID NO: 4030) AAAGATATCACAAGAGAGATGGTGTGTCAGC |
| LYM249 | | LYM249_EF_BamHI (SEQ ID NO: 4031) AAAGGATCCGGGTGTCATCAAACGGACTAC |
| | | LYM249_ER_KpnI (SEQ ID NO: 4032) AAAGGTACCCTAAACGAGGTTACGGAATGTGTC |
| LYM250 | SalI,XbaI | LYM250_EF_SalI (SEQ ID NO: 4033) AAAGTCGACGGAATTGGTGAGGTGATGC |
| | | LYM250_ER_XbaI (SEQ ID NO: 4034) AAATCTAGACAGATAAACCTCAATCAAAGTCG |
| LYM25 1 | | LYM251_NF_SalI (SEQ ID NO: 4035) AAAGTCGACCTGTCCTCTACTACGCATCTCTC |
| | | LYM251_NR_XbaI (SEQ ID NO: 4036) AAATCTAGATAATCATCATTGTAGCAGGCAC |
| LYM252 | BamHI,KpnI | LYM252_NF_BamHI (SEQ ID NO: 4037) AAAGGATCCTAGGAAGGATGGTACTGGCTG |
| | | LYM252_EF_BamHI (SEQ ID NO: 4038) AAAGGATCCGCGATAGGAAGGATGGTACTG |
| | | LYM252_NR_KpnI (SEQ ID NO: 4039) AAAGGTACCAGGCAAACACAATGATTTCAAC |
| | | LYM252_ER_KpnI (SEQ ID NO: 4040) AAAGGTACCTGTAACATAAGTACCGGGCAG |
| LYM254 | | LYM254_EF_SalI (SEQ ID NO: 4041) AAAGTCGACAATCTCCCACGCTCCAAAG |
| | | LYM254_ER_XbaI (SEQ ID NO: 4042) AAATCTAGAAGTTACATTCTTGACCAGCAGC |
| LYM255 | BamHI,XhoI | LYM255_NF_BamHI (SEQ ID NO: 4043) AAAGGATCCCTTCTAGTAGCACAGTAGTAGCAGC |
| | | LYM255_NR_XhoI (SEQ ID NO: 4044) AAACTCGAGAACGAGGAAGAATCGGTATATG |
| LYM256 | BamHI,XhoI | LYM256_NF_BamHI (SEQ ID NO: 4045) AAAGGATCCGGAACAACTCGTAGCCATGAC |
| | | LYM256_EF_BamHI (SEQ ID NO: 4046) TATGGATCCCAATTTGAGAGCATTTGCTACG |
| | | LYM256_ NR_XhoI (SEQ ID NO: 4047) TAACTCGAGCTGAACTTAATAGCAATTCCGTAGC |
| | | LYM256_ER_XhoI (SEQ ID NO: 4048) AAACTCGAGCGCACTACTGTGCTTCTGAAC |
| LYM26 | SalI,X6aI | LYM26_EF_SalI (SEQ ID NO: 4049) AAAGTCGACTTGCTCCCTCTCTCTCTCTTG |
| | | LYM26_ER_XbaI (SEQ ID NO: 4050) AAATCTAGATGTATTCACGAGGTAAACAACG |
| LYM260 | | LYM260_NF_BamHI (SEQ ID NO: 4051) AAAGGATCCGAGAGATTAATTAAGTGGCAGG |
| | | LYM260_EF_BamHI (SEQ ID NO: 4052) AAAGGATCCAGAAGAGAGATTAATTAAGTGGCAG |
| | | LYM260_NR_KpnI (SEQ ID NO: 4053) AAAGGTACCCTAATATCGATCCAAACTCACACAAG |
| | | LYM260_ER_KpnI (SEQ ID NO: 3965) AAAGGTACCTACGTGCGTATCATACATGGAG |
| LYM261 | | LYM261_EF_SmaI (SEQ ID NO: 4054) AATCCCGGGTCGAGAGGTTTCATTCAGTGC |
| | | LYM261_ER_KpnI (SEQ ID NO: 4055) TTTGGTACCTTATTACATTTGGATGGGCTGT |
| LYM267 | SalI,EcoRV | LYM267_F_SalI (SEQ ID NO: 4056) AAAGTCGACGAGCACAGGTAGGGTTTCG |
| | | LYM261_ER_EcoRV (SEQ ID NO: 4057) AAAGATATCCACTACCGAAGACTCACACGAC |
| LYM268 | | LYM268_EF_XhoI (SEQ ID NO: 4058) AAACTCGAGAACCCTCGCGAATCTGAG |
| | | LYM268_ER_EcoRV (SEQ ID NO: 4059) AAAGATATCTAGTTCTCCATTCAGCATCTCC |
| LYM270 | BamHI,XhoI | LYM270_NF_BamHI (SEQ ID NO: 4060) AAAGGATCCAAAGCAGTTCCAGCCTTCC |
| | | LYM270_EF_BamHI (SEQ ID NO: 4061) AAAGGATCCACCAATGGCTGCCTGAGAC |
| | | LYM270_NF_BamHI (SEQ ID NO: 4060) AAAGGATCCAAAGCAGTTCCAGCCTTCC |
| | | LYM270_ER_XhoI (SEQ ID NO: 4062) AAACTCGAGGATTGGATATGCCACTTGATTG |
| LYM271 | BamHI,XhoI | LYM271_EF_BamHI (SEQ ID NO: 4063) AAAGGATCCCACCTTCTTCCCAGATCAATAG |
| | | LYM271_ER_XhoI (SEQ ID NO: 4064) AAACTCGAGGAAACAAAGCACAGTCAGTAGTAG |
| LYM273_S | BamHI,XhoI | LYM273_EF_BamHI (SEQ ID NO: 4065) AAAGGATCCTACTAACAAACAGATAATCTCCACG |
| | | LYM273_R2_XhoI (SEQ ID NO: 4066) ATACTCGAGAACATGTTGGAGATCTTTGATGC |
| LYM274 | BamHI,XhoI | LYM274_EF_BamHI (SEQ ID NO: 4067) AAAGGATCCGAGAAGCTCCACTCTTCTCCAC |
| | | LYM274_ER_XhoI (SEQ ID NO: 4068) AAACTCGAGTATAATGCACAGTTATGGGCAG |
| LYM277 | | LYM277_NF_SalI (SEQ ID NO: 4069) AAAGTCGACTCAACGCCCAAGCTAGATTAC |
| | | LYM277_NR_SacI (SEQ ID NO: 4070) AAAGAGCTCCTCAACATTGCAACAACTATGG |
| LYM278 | SalI,SacI | LYM278_EF_SalI (SEQ ID NO: 4071) AAAGTCGACGCAGCCACACAACACTATCTC |
| | | LYM278_ER_SacI (SEQ ID NO: 4072) AAAGAGCTCTTGACGATACATAGCACATAAGG |
| LYM283 | | LYM283_NF_SmaI (SEQ ID NO: 4073) TTTCCCGGGTGCCACTTGTGCGAGGAG |
| | | LYM283_R_KpnI (SEQ ID NO: 4074) AACGGTACCTCACCAATCAAAATGTACAATCATGT |
| LYM284 | BamHI,KpnI | LYM284_EF_BamHI (SEQ ID NO: 4075) AAAGGATCCGAGCAACCACCCGTAGTCAG |
| | | LYM284_ER_KpnI (SEQ ID NO: 4076) AAAGGTACCACAGCTCAAGTGCTCATTTCTC |
| LYM285 | XhoI,EcoRV | LYM285_NF_XhoI (SEQ ID NO: 4077) AAACTCGAGCCGCCATCTACTCGGAGC |
| | | LYM285_EF_XhoI (SEQ ID NO: 4078) AAACTCGAGCCTCCTCCGCCATCTACTC |
| | | LYM285_NR_EcoRV (SEQ ID NO: 4079) AAAGATATCAGAATTCACACTGTCCCAACAC |
| | | LYM285_ER_EcoRV (SEQ ID NO: 4080) AAAGATATCCAGTTATTATAGGCCTCGTTCC |
| LYM287 | XhoI,EcoRV | LYM287_EF_XhoI (SEQ ID NO: 4081) AAACTCGAGTGATTGCGTTTCCTTAAATATG |
| | | LYM287_ER_EcoRV (SEQ ID NO: 4082) AAAGATATCCAATCAATCCTACAAACACAGC |
| LYM288 | XhoI,SacI | LYM288_EF_XhoI (SEQ ID NO: 4083) AAACTCGAGTGTTAGGAAGTGAGGACTGAGC |
| | | LYM288_ER_SacI (SEQ ID NO: 4084) AAAGAGCTCGCTCAATTATTCACCATTTCATC |
| LYM289 | SalI,XbaI | LYM289_EF_SalI (SEQ ID NO: 4085) AAAGTCGACGCACAACCCTTGGAGACTTC |
| | | LYM289_ER_XbaI (SEQ ID NO: 4086) AAATCTAGATCCTCTCATCGAGCTAAGACAC |
| LYM290 | BamHI,KpnI | LYM290_EF_BamHI (SEQ ID NO: 4087) AAAGGATCCATCCGGATCTCCACATTCC |
| | | LYM290_ER_KpnI (SEQ ID NO: 4088) |
| | | AAAGGTACCGAAACAATCTCATGGTCTCTGC |
| LYM291 | SalI,BamHI | LYM291_EF_SalI (SEQ ID NO: 4089) AAAGTCGACACTGAGCTCTCTGCTAAGTTGG |
| | | LYM291_ER_BamHI (SEQ ID NO: 4090) AAAGGATCCTCCTAGCAACAGAAGATCCAAG |
| LYM293 | XhoI,SacI | LYM293_NF_XhoI (SEQ ID NO: 4091) AAACTCGAGAGCTTCCTCCCTAGCTGTCC |
| | | LYM293_EF_XhoI (SEQ ID NO: 4092) AAACTCGAGGTGTAGCTTCCTCCCTAGCTG |
| | | LYM293_NR_SacI (SEQ ID NO: 4093) AAAGAGCTCCTATTCCAGGAGAAGAACAATAAGAG |
| | | LYM293_ER_SacI (SEQ ID NO: 4094) AAAGAGCTCCTATTCATGTTCCAGGAGAAGAAC |
| LYM3 | XhoI,KpnI | LYM3_EF_XhoI (SEQ ID NO: 4095) AATCTCGAGATTTATCTGCTTCAATGGCAAC |
| | | LYM3_ER_KpnI (SEQ ID NO: 4096) ATAGGTACCCTAAGCATCATTCTGCCTACC |
| LYM30 | SalI,XhoI | LYM30_NF_SalI (SEQ ID NO: 4097) AAAGTCGACCCTCCATCCTTCAGTAATTGG |
| | | LYM30_NR_XhoI (SEQ ID NO: 4098) TTTCTCGAGTCAGTCTCCTTGGATGTTTGAGTTG |
| LYM31 | SalI,XhoI | LYM31_NF_SalI (SEQ ID NO: 4099) AAGGTCGACACTCCCAACGTCTACTCTTCC |
| | | LYM31_EF_SalI (SEQ ID NO: 4100) AATGTCGACCTCACCACTCCCAACGTCTAC |
| | | LYM31_NR_XhoI (SEQ ID NO: 4101) AAACTCGAGATGTAAGAATGAAATCTTGTAGCTC |
| | | LYM3 1_ER_XhoI (SEQ ID NO: 4102) AATCTCGAGTGCAAGGATGTAAGAATGAAATC |
| LYM34 | BamHI,KpnI | LYM34_NF_BamHI (SEQ ID NO: 4103) AAAGGATCCGAGATAATTAGCTCACTCCATGG |
| | | LYM34_NR_KpnI (SEQ ID NO: 4104) TATGGTACCGAATTGGGCCTATGAGACG |
| LYM35 | SalI,X6aI | LYM35_NF_SalI (SEQ ID NO: 4105) AAAGTCGACAACACCTCTCTGGCTCTCTCC |
| | | LYM35_NR_SacI (SEQ ID NO: 4106) AAAGAGCTCTCCTAAGACTTTCTCAGCCATC |
| LYM37 | SalI,X6aI | LYM37_NF_SalI (SEQ ID NO: 4203) AAAGTCGACAAAGTTAGCGACCAAGAAACC |
| | | LYM37_NR_XbaI (SEQ ID NO: 4204) AAATCTAGACATTTCTTTTGGATGGATGAAC |
| LYM4 | EcoRV,KpnI | LYM4_NF_EcoRV (SEQ ID NO: 4107) AAAGATATCACCTCGAAACCCTAGATCG |
| | | LYM4_EF_EcoRV (SEQ ID NO: 4108) AAAGATATCATTCCTCGACCAGCTCACG |
| | | LYM4_NR_Kpn (SEQ ID NO: 4109) TTAGGTACCACTCAAAGGAGAGCTTCAGCC |
| | | LYM4 ER Kpnl (SEQ ID NO: 4110) TAAGGTACCGTTGGCATTCTTCAAACCAG |
| LYM40 | | LYM40_NF_SalI (SEQ ID NO: 4111) AAAGTCGACCTCGAGAGCTCAATGATTCG |
| | | LYM40_NR_XbaI (SEQ ID NO: 4112) |
| | | AAATCTAGAACCAACCAATTAAAGGCTAATG |
| LYM41 | SalI,X6aI | LYM41_NF_SalI (SEQ ID NO: 4113) AAAGTCGACGATTGGTTGCTTGGGTTTG |
| | | LYM41_NR_XbaI (SEQ ID NO: 4114) AAATCTAGATGCTTTCTTTCAGAACATCTCC |
| LYM42 | SalI,XbaI | LYM42_NF_SalI (SEQ ID NO: 4115) AAAGTCGACAACCTCTCCTCCTCGTCACAC |
| | | LYM42_EF_SalI (SEQ ID NO: 4116) AAAGTCGACATCAAACCTCTCCTCCTCGTC |
| | | LYM42_NR_XbaI (SEQ ID NO: 4117) AATTCTAGATCACAGGAAGGAGGGGTAGTAACAG |
| | | LYM42_ER_XbaI (SEQ ID NO: 4118) AAATCTAGAATTTCCTGCTGTTCATTCAAAG |
| LYM43 | SalI,XbaI | LYM43_NF_SalI (SEQ ID NO: 4119) AAAGTCGACTCAGTGTTCTTCCATTCTTTCC |
| | | LYM43_NR_XbaI (SEQ ID NO: 4120) AAATCTAGATTGAATTAGCAGCAGCAAGAG |
| LYM44 | SalI,XbaI | LYM44_NF_SalI (SEQ ID NO: 4121) AAAGTCGACCGAACTAACTAACCATCTCATCC |
| | | LYM44_NR_XbaI (SEQ ID NO: 4122) AAATCTAGAATCGTTCGATTATTATTGCTCC |
| LYM5 | EcoRV,PstI | LYM5_EF_EcoRV (SEQ ID NO: 4123) AAAGATATCTCCTCTTCTCAAACTCCATCTC |
| | | LYM5_ER_PstI (SEQ ID NO: 4124) AATCTGCAGGGTCCTGTCATGCTGTGTAGTC |
| LYM51 | SalI,X6aI | LYM5 1_EF_SalI (SEQ ID NO: 4125) AAAGTCGACAATTCACCTCCCAAGCAGAG |
| | | LYM51_ER_XbaI (SEQ ID NO: 4126) AAATCTAGAATACAAGGCCTGCACTACCTAC |
| LYM52 | EcoRV,XhoI | LYM52_F_XhoI (SEQ ID NO: 4127) AAACTCGAGAAACCCGATAAGAAAATGGC |
| | | LYM52_ER_EcoRV (SEQ ID NO: 4128) TTTGATATCCTAGTGCCATACGTGCCTAACCT |
| LYM53 | SalI,X6aI | LYM53_NF_SalI (SEQ ID NO: 4129) AAAGTCGACATCCTCTCTTTCCACTCCTAGC |
| | | LYM53_NR_XbaI (SEQ ID NO: 4130) AAATCTAGATAGCACTCAGCTTAATTGGATG |
| LYM56 | SalI,XbaI | LYM56_F_SalI (SEQ ID NO: 4131) AAAGTCGACCTCGCTTGCCCACTCCTT |
| | | LYM56_F_SalI (SEQ ID NO: 4131) AAAGTCGACCTCGCTTGCCCACTCCTT |
| | | LYM56_NR_XbaI (SEQ ID NO: 4132) AAATCTAGACTAGCATGATCCTGGATGTTTACTC |
| | | LYM56_ER_XbaI (SEQ ID NO: 4133) AAATCTAGAAGCAGAGATAGGCATAAGTCCA |
| LYM57 | EcoRV,XhoI | LYM57_NF_EcoRV (SEQ ID NO: 4134) AAAGATATCACCACTAGGACTCAACGAGAAG |
| | | LYM57_NR_XhoI (SEQ ID NO: 4135) AACCTCGAGAGTAACATCCGAACGTATACACC |
| LYM6 | SmaI,KpnI | LYM6_NF_X/SmaI (SEQ ID NO: 4136) ATACCCGGGAACCACGCGAAGACATGG |
| | | LYM6_NR_KpnI (SEQ ID NO: 4137) TATGGTACCGGATCAGGTTATACTTCTTATTGAC |
| LYM61 | BamHI,XhoI | LYM61_NF_BamHI (SEQ ID NO: 4138) AAAGGATCCAAGCCTGTTCTCTGTCGATTG |
| | | LYM61_NR_XhoI (SEQ ID NO: 4139) AAACTCGAGAATGCATGTCCTAGTCTTTACG |
| LYM62 | BamHI,KpnI | LYM62_NF_BamHI (SEQ ID NO: 4140) TTAGGATCCAACATTTACGCGATCCATTG |
| | | LYM62_EF_BamHI (SEQ ID NO: 4141) TTAGGATCCATCATCTGCTTTGTCTACCTCG |
| | | LYM62_NR_KpnI (SEQ ID NO: 4142) ATCGGTACCTCAACTGAATTCGCTGAAACTTGTC |
| | | LYM62_ER_KpnI (SEQ ID NO: 4143) AAAGGTACCGAAAACAAATGGAAGCAATCTG |
| LYM66 | EcoRV,XhoI | LYM66_NF_EcoRV (SEQ ID NO: 4144) AAAGATATCGAGACGCAAGAAACATAGCTC |
| | | LYM66_NR_XhoI (SEQ ID NO: 4145) AAACTCGAGCAATCACTGCTACAAATCCGT |
| LYM67 | SalI,XbaI | LYM67_NF_SalI (SEQ ID NO: 4146) TATGTCGACTCTTCTTCACTGAGGCAAGTTC |
| | | LYM67_NR_XbaI (SEQ ID NO: 4147) AAGTCTAGATCAAAGATCCATAACATTCCATGC |
| LYM68 | SalI,XhoI | LYM68_NF_SalI (SEQ ID NO: 4148) ATTGTCGACTTGAGATAAAGGCAAAATTACG |
| | | LYM68_EF_SalI (SEQ ID NO: 4149) TTTGTCGACGTCTCGTTTCAGATTCTTCTGC |
| | | LYM68_NR_XhoI (SEQ ID NO: 4150) TTCCTCGAGTCTCTAGAGTTGCATTCCTTCC |
| | | LYM68_ER_XhoI (SEQ ID NO: 4151) TGACTCGAGCATCGTTTACACTGAACCACTG |
| LYM69 | SalI,XbaI | LYM69_NF_SalI (SEQ ID NO: 4152) AAAGTCGACACCCAGGAACACATCATCATC |
| | | LYM69_NR_XbaI (SEQ ID NO: 4153) AAATCTAGAAGGACACGTCAAATGAGAAAAC |
| LYM7 | SalI,X6aI | LYM7_NF_SalI (SEQ ID NO: 4154) AAAGTCGACAGTCAGATCCATTCCTCCTCC |
| | | LYM7_NR_XbaI (SEQ ID NO: 4155) AATTCTAGAAAAAGTAGCAGCCGGTCATC |
| LYM73 | | LYM73_EF_SalI (SEQ ID NO: 4156) AACGTCGACAATCTTGACACCATCTCGCTC |
| | | LYM73_ER_StuI (SEQ ID NO: 4157) TTTAGGCCTCTCGCACATTATTTTGTACAGC |
| LYM79 | SalI,XbaI | LYM79_F_SalI (SEQ ID NO: 4158) AAAGTCGACGCGACAGAGAATCCATGGC |
| | | LYM79_F_SalI (SEQ ID NO: 4158) AAAGTCGACGCGACAGAGAATCCATGGC |
| | | LYM79_NR_XbaI (SEQ ID NO: 4159) AATTCTAGATCAAACTCCTCTTATATGCACCTGC |
| | | LYM79_ER_XbaI (SEQ ID NO: 4160) |
| | | AAATCTAGATCAGAAACTAACTCCTCTTATATGCAC C |
| LYM8 | XhoI,KpnI | LYM8 NF Xhol (SEQ ID NO: 4161) ATACTCGAGCTTCCCCGATAGAAATCCATC |
| | | LYM8 NR KpnI (SEQ ID NO: 4162) TAGGGTACCACCAAACAGCACATATGCGG |
| LYM82 | SalI,XbaI | LYM82_EF_SalI (SEQ ID NO: 4163) AAAGTCGACCGCAACCGGAGAGAAATC |
| | | LYM82_ER_XbaI (SEQ ID NO: 4164) AAATCTAGATCGACAATCTTCATACACAACG |
| LYM83 | | LYM83_NF_BamHI (SEQ ID NO: 4165) AAAGGATCCCGACAGTCACCACTCACCAAC |
| | | LYM83_F2_BamHI (SEQ ID NO: 4166) AAAGGATCCTCCGCACGCAACTCAGTG |
| | | LYM83-R2-XhoI (SEQ ID NO: 4167) AAACTCGAGCAACGGTAACACACAAGCATTC |
| | | LYM83_R2_XhoI (SEQ ID NO: 4167) AAACTCGAGCAACGGTAACACACAAGCATTC |
| LYM84 | BamHI,XhoI | LYM84_NF_BamHI (SEQ ID NO: 4168) AAAGGATCCACCCAGAACCCGAAGAATG |
| | | LYM84_F2_BamHI (SEQ ID NO: 4169) AATGGATCCTAAACCCAGAACCCGAAGAATG |
| | | LYM84_R2_XhoI (SEQ ID NO: 4170) AAACTCGAGCAAACTGGAGCATAGCAACTAGG |
| | | LYM84_R2_XhoI (SEQ ID NO: 4170) AAACTCGAGCAAACTGGAGCATAGCAACTAGG |
| LYM86 | BamHI,XhoI | LYM86_EF_BamHI (SEQ ID NO: 4171) AAAGGATCCCACACACCACAGTCGCAATC |
| | | LYM86_ER_XhoI (SEQ ID NO: 4172) AAACTCGAGAGAATCGATGCAGGTAACTACG |
| LYM88 | BamHI,XhoI | LYM88_F_BamHI (SEQ ID NO: 4173) AAAGGATCCACAATAAACAAGATAAATGGAGG |
| | | LYM88_F_BamHI (SEQ ID NO: 4173) AAAGGATCCACAATAAACAAGATAAATGGAGG |
| | | LYM88_NR_XhoI (SEQ ID NO: 4174) AAACTCGAGTCACACGCAACTTCAGGTTC |
| | | LYM88_ER_XhoI (SEQ ID NO: 4175) AAACTCGAGCAAACCGAATTATTACATCAGG |
| LYM89 | SalI,SacI | LYM89_NF_SalI (SEQ ID NO: 4176) AAAGTCGACGGCCGACACATCTGATCTAAC |
| | | LYM89_NR_SacI (SEQ ID NO: 4177) AAAGAGCTCTCCCAGAAATATATAAGAACAAGC |
| LYM9 | SalI,XbaI | LYM9_NF_SalI (SEQ ID NO: 4178) AAAGTCGACAACTCCCCAACCAAGCAG |
| | | LYM9_NR_XbaI (SEQ ID NO: 4179) AAATCTAGATTAGTACTAAGAGTCGGCTTTGGC |
| LYM90 | SalI,X6aI | LYM90_NF_SalI (SEQ ID NO: 4180) AAAGTCGACCTAAACCCTAACCCTAGATTGG |
| | | LYM90_NR_XbaI (SEQ ID NO: 4181) AAATCTAGAAGACTTGGCTAATGCTAACCTG |
| LYM91 | SalI,X6aI | LYM91_F2_SalI (SEQ ID NO: 4182) TAAGTCGACCGTCTCTCAAGCTCGCAGC |
| | | LYM91_F2_SalI (SEQ ID NO: 4182) TAAGTCGACCGTCTCTCAAGCTCGCAGC |
| | | LYM9 1_R2_XbaI (SEQ ID NO: 4183) ATTTCTAGACGAGAGCCTCTAATGGATCACAG |
| | | LYM91_R2_XbaI (SEQ ID NO: 4183) ATTTCTAGACGAGAGCCTCTAATGGATCACAG |
| LYM93 | | LYM93_EF_SalI (SEQ ID NO: 4184) AAAGTCGACATTGCACTGCATAGGGCTG |
| | | LYM93_ER_XbaI (SEQ ID NO: 4185) |
| | | AAATCTAGACTAAGAGTTGAGCATGATAAATACGA C |
| LYM95 | SalI,XbaI | LYM95_NF_SalI (SEQ ID NO: 4186) ATAGTCGACGAGAAAGTGGAAGAGAACATGG |
| | | LYM95_EF_SalI (SEQ ID NO: 4187) AAAGTCGACCCGCTGGAGAAAGTGGAAG |
| | | LYM95_NR_XbaI (SEQ ID NO: 4188) AAATCTAGAGTCCACAGATCCATGTCAAATC |
| | | LYM95_ER_XbaI (SEQ ID NO: 4189) AAATCTAGAGTGAATTTGATTTATTGCCAAC |
| LYM99 | BamHI,KpnI | LYM99_NF_BamHI (SEQ ID NO: 4190) AAAGGATCCCCGACCACGGATTGATTC |
| | | LYM99_EF_BamHI (SEQ ID NO: 4191) AAAGGATCCTTGACTTGGGTGTCTGGTCC |
| | | LYM99_NR_KpnI (SEQ ID NO: 4192) AAAGGTACCGTGCCTATGTCTTCCTAGCATC |
| | | LYM99_ER_KpnI (SEQ ID NO: 4193) AAAGGTACCATATTTAGGCGCCAGTAAAGAC |

Table 26. Provided are the PCR primers used for cloning the genes. Fwd = forward primer; Rev = reverse primer; Nested = nested primer for PCR (internal primer); External = external primer for PCR.

To facilitate cloning of the cDNAs/ genomic sequences, a 8-12 bp extension was added to the 5' of each primer. The primer extension includes an endonuclease restriction site. The restriction sites were selected using two parameters: (a). The site did not exist in the cDNA sequence; and (b). The restriction sites in the forward and reverse primers were designed such that the digested cDNA is inserted in the sense formation into the binary vector utilized for transformation.

Each digested PCR product was inserted into a high copy vector pBlue-script KS plasmid vector [pBlue-script KS plasmid vector, Hypertext Transfer Protocol://World Wide Web (dot) stratagene (dot) com/manuals/212205 (dot) pdf] or into plasmids originating from this vector. In cases where the pGXN/ pGXNa high copy vector (originated from pBlue-script KS) was used, the PCR product was inserted upstream to the NOS terminator (SEQ ID NO: 4194) originated from pBI 101.3 binary vector (GenBank Accession No. U12640, nucleotides 4356 to 4693) and downstream to the 35S promoter. The digested products and the linearized plasmid vector were ligated using T4 DNA ligase enzyme (Roche, Switzerland). In some cases PCR products were cloned without digestion into pCR-Blunt II-TOPO vector (Invitrogen).

Sequencing of the amplified PCR products was performed, using ABI 377 sequencer (Amersham Biosciences Inc). In all cases, after confirmation of the sequence of the cloned genes, the cloned cDNA accompanied or not with the NOS terminator was introduced into the modified pGI binary vector containing the 6669 promoter [pQFN or pQYN_6669] according to Table 27, via digestion with appropriate restriction endonucleases. In any case the insert was followed by single copy of the NOS terminator (SEQ ID NO:4194).

High copy plasmids containing the cloned genes were digested with restriction endonucleases (New England BioLabs Inc) and cloned into binary vectors according to Table 27, below.

### Binary vectors used for cloning:

***Evolution of binary vectors construction:*** The plasmid pPI was constructed by inserting a synthetic poly-(A) signal sequence, originating from pGL3 basic plasmid vector (Promega, Acc No U47295; bp 4658-4811) into the *Hind*III restriction site of the binary vector pBI101.3 (Clontech, Acc. No. U12640). pGI (pBXYN) is similar to pPI, but the original gene in the backbone, the GUS gene, was replaced by the GUS-Intron gene followed by the NOS terminator (SEQ ID NO:4194) (Vancanneyt. G, et al MGG 220, 245-50, 1990). The modified pGI vector (pQXYN) is a modified version of the pGI vector in which the cassette is inverted between the left and right borders so the gene and its corresponding promoter are close to the right border and the NPTII gene is close to the left border.

***Vectors used for cloning the polynucleotides:*** Cloned genes were digested from the high copy vectors and cloned into one of the following binary vectors: pQFN or pQYN_6669.

pQFN (see Figure 2) and pQYN_6669 (see Figure 1) are modified pGI vectors in which the 35S promoter was replaced by the new At6669 promoter (SEQ ID NO:4198). pQYN_6669 contains the GUSintron sequence, while pQFN lacks the GUSintron sequence

**Table 27**

| ***Restriction enzyme sites used to clone identified genes into binary vectors*** | | | | |
|---|---|---|---|---|
| ***Gene name*** | ***Binary vector*** | ***Restriction enzymes used for cloning into binary vector-FORWARD*** | ***Restriction enzymes used for cloning into binary vector-REVERSE*** | ***Restriction enzymes used for digesting the binary vector*** |
| LYM1 | pQFN | Sail | EcoRI | SalI, EcoRI |
| LYM10 | pQFN | Xhol | KpnI | XhoI, KpnI |
| LYM100 | pQYN_6669 | Sail | EcoRI | SalI, EcoRI |
| LYM 102 | pQFN | BamHI | XhoI | BamHI, XhoI |
| LYM 103 | pQFN | BamHI | XhoI | BamHI, XhoI |
| LYM105 | pQFN | BamHI | XhoI | BamHI, XhoI |
| LYM106 | pQYN_6669 | SalI | EcoRI | SalI, EcoRI |
| LYM 107 | pQFN | BamHI | XhoI | BamHI, XhoI |
| LYM109 | pQFN | XhoI | StuI | XhoI, StuI |
| LYM110 | pQFN | BamHI | XhoI | BamHI, XhoI |
| LYM111 | pQFN | XhoI | EcoRI | XhoI, EcoRI |
| LYM112 | pQFN | BamHI | XhoI | BamHI. XhoI |
| LYM113 | pQYN_6669 | SalI | EcoRI | SalI, EcoRI |
| LYM115 | pQFN | BamHI | XhoI | BamHI, XhoI |
| LYM116 | pQYN_6669 | SalI | EcoRI | SalI, EcoRI |
| LYM117 | pQFN | BamHI | EcoRV | BamHI, StuI |
| LYM118 | pQFN | BamHI | XhoI | BamHI, XhoI |
| LYM119 | pQYN_6669 | SalI | EcoRI | SalI, EcoRI |
| LYM12 | pQFN | XhoI | KpnI | XhoI, KpnI |
| LYM 120 | pQFN | BamHI | XhoI | BamHI, XhoI |
| LYM 121 | pQFN | BamHI | XhoI | BamHI, Xhol |
| LYM122_ G | pQFN | BamHI | XhoI | BamHI, Xhol |
| LYM 122_S | pQFN | BamHI | XhoI | BamHI, XhoI |
| LYM 123 | pQFN | BamHI | XhoI | BamHI, Xhol |
| LYM125 | pQFN | BamHI | KpnI | BamHI, KpnI |
| LYM 126 | pQFN | BamHI | KpnI | BamHI, KpnI |
| LYM127 | pQFN | BamHI | XhoI | BamHI, XhoI |
| LYM 128 | pQFN | BamHI | XhoI | BamHI, XhoI |
| LYM 129 | pQFN | SalI | EcoRI | SalI, EcoRI |
| LYM13 | pQFN | SalI | BamHI | SalI, BamHI |
| LYM 130 | pQYN_6669 | Sail | EcoRI | SalI, EcoRI |
| LYM131 | pQFN | SalI | XhoI | SalI, XhoI |
| LYM 132 | pQFN | BamHI | XhoI | BamHI. Xhol |
| LYM 134 | pQFN | BamHI | XhoI | BamHI, XhoI |
| LYM 135 | pQFN | SalI | KpnI | SalI, KpnI |
| LYM 136 | pQFN | BamHI | KpnI | BamHI, KpnI |
| LYM137 | pQYN_6669 | SalI | EcoRI | SalI, EcoRI |
| LYM138 | pQFN | SalI | Ecl136II | SalI, StuI |
| LYM14 | pQFN | SalI | BamHI | SalI, BamHI |
| LYM 140 | pQFN | Xhol | EcoRI | XhoI, EcoRI |
| LYM 141 | pQFN | BamHI | KpnI | BamHI, KpnI |
| LYM142 | pQYN_6669 | SalI | EcoRI | SalI, EcoRI |
| LYM143 | pQYN_6669 | SalI | EcoRI | SalI, EcoRI |
| LYM 144 | pQFN | SalI | EcoRV | SalI, StuI |
| LYM 145 | pQFN | BamHI | Xhol | BamHI, XhoI |
| LYM 146 | pQFN | KpnI | KpnI | KpnI, KpnI |
| LYM147 | pQFN | SalI | EcoRI | SalI, EcoRI |
| LYM 148 | pQFN | BamHI | XbaI | BamHI, XhoI |
| LYM 149 | pQYN_6669 | SalI | EcoRI | SalI, EcoRI |
| LYM15 | pQFN | SalI | EcoRI | SalI, EcoRI |
| LYM152 | pQYN_6669 | SalI | EcoRI | SalI, EcoRI |
| LYM153 | pQYN_6669 | SalI | EcoRI | SalI, EcoRI |
| LYM154 | pQFN | XhoI | StuI | XhoI, StuI |
| LYM155 | pQYN_6669 | SalI | EcoRI | SalI, EcoRI |
| LYM156 | pQFN | StuI | StuI | SmaI, SmaI |
| LYM 157_ G | pQYN_6669 | SalI | EcoRI | SalI, EcoRI |
| LYM157_S | pQFN | SalI | StuI | SalI, StuI |
| LYM159 | pQYN_6669 | SalI | EcoRI | SalI, EcoRI |
| LYM16 | pQFN | SalI | EcoRI | SalI, EcoRI |
| LYM160 | pQYN_6669 | SalI | EcoRI | SalI, EcoRI |
| LYM161 | pQFN | BamHI | XhoI | BamHI, XhoI |
| LYM162 | pQFN | BamHI | XhoI | BamHI, XhoI |
| LYM 164 | pQYN_6669 | SalI | EcoRI | SalI, EcoRI |
| LYM 165 | pQFN | XhoI | Ecl136II | XhoI, StuI |
| LYM17 | pQFN | SmaI | KpnI | SmaI, KpnI |
| LYM170 | pQYN_6669 | SalI | EcoRI | SalI, EcoRI |
| LYM172 | pQFN | BamHI | Xhol | BamHI, XhoI |
| LYM173 | pQFN | BamHI | XhoI | BamHI, Xhol |
| LYM174 | pQFN | BamHI | KpnI | BamHI, KpnI |
| LYM 175 | pQYN_6669 | SalI | EcoRI | SalI, EcoRI |
| LYM176 | pQYN_6669 | SalI | EcoRI | SalI, EcoRI |
| LYM 178 | pQYN_6669 | SalI | EcoRI | SalI, EcoRI |
| LYM179 | pQFN | SalI | StuI | SalI, StuI |
| LYM180 | pQFN | BamHI | XhoI | BamHI. XhoI |
| LYM181 | pQFN | BamHI | EcoRV | BamHI, StuI |
| LYM183 | pQFN | SalI | XbaI | SalI, StuI |
| LYM184 | pQFN | BamHI | XhoI | BamHI, XhoI |
| LYM 185 | pQFN | BamHI | KpnI | BamHI, KpnI |
| LYM 186 | pQFN | SalI | Ecl1361II | SalI, StuI |
| LYM 188 | pQFN | BamHI | XhoI | BamHI, XhoI |
| LYM 189 | pQYN_6669 | SalI | EcoRI | SalI, EcoRI |
| LYM19 | pQYN_6669 | SalI | EcoRI | SalI, EcoRI |
| LYM 192 | pQFN | Xhol | EcoRV | Xhol, StuI |
| LYM 193 | pQFN | BamHI | XhoI | BamHI, XhoI |
| LYM 194 | pQFN | SalI | XhoI | SalI, SalI |
| LYM 196 | pQFN | BamHI | XhoI | BamHI, Xhol |
| LYM 197 | pQFN | BamHI | XhoI | BamHI, XhoI |
| LYM 198 | pQFN | BamHI | XhoI | BamHI, Xhol |
| LYM2 | pQFN | EcoRV | KpnI | SmaI, KpnI |
| LYM20 | pQFN | EcoRV | KpnI | Smal, KpnI |
| LYM200 | pQFN | BamHI | XhoI | BamHI, XhoI |
| LYM201 | pQFN | BamHI | XhoI | BamHI. Xhol |
| LYM203 | pQFN | BamHI | XhoI | BamHI, XhoI |
| LYM204 | pQFN | BamHI | XhoI | BamHI, XhoI |
| LYM206 | pQFN | XhoI | EcoRV | Xhol, StuI |
| LYM207 | pQFN | BamHI | KpnI | BamHI, KpnI |
| LYM208 | pQFN | BamHI | KpnI | BamHI. KpnI |
| LYM21 | pQFN | EcoRV | KpnI | SmaI, KpnI |
| LYM212 | pQYN_6669 | SalI | EcoRI | SalI, EcoRI |
| LYM213 | pQFN | BamHI | XhoI | BamHI, Xhol |
| LYM215 | pQFN | BamHI | XhoI | BamHI, XhoI |
| LYM217 | pQYN_6669 | SalI | EcoRI | SalI, EcoRI |
| LYM219 | pQFN | BamHI | KpnI | BamHI, KpnI |
| LYM22 | pQYN_6669 | SalI | EcoRI | SalI, EcoRI |
| LYM220 | pQFN | BamHI | EcoRV | BamHI, StuI |
| LYM221 | pQFN | BamHI | XhoI | BamHI, XhoI |
| LYM223 | pQFN | Xhol | EcoRI | XhoI, EcoRI |
| LYM224 | pQFN | BamHI | XhoI | BamHI, XhoI |
| LYM227 | pQFN | BamHI | KpnI | BamHI, KpnI |
| LYM228 | pQFN | StuI | KpnI | KpnI, EcoRV |
| LYM23 | pQFN | BamHI | KpnI | BamHI, KpnI |
| LYM232 | pQFN | BamHI | XhoI | BamHI, XhoI |
| LYM233 | pQFN | BamHI | XhoI | BamHI, XhoI |
| LYM234 | pQFN | BamHI | XhoI | BamHI, Xhol |
| LYM236 | pQFN | SalI | EcoRI | SalI, EcoRI |
| LYM238 | pQFN | SmaI | KpnI | Smal, KpnI |
| LYM239 | pQFN | BamHI | XhoI | BamHI, XhoI |
| LYM24 | pQFN | SalI | EcoRI | SalI, EcoRI |
| LYM240 | pQFN | BamHI | KpnI | BamHI, KpnI |
| LYM241 | pQFN | BamHI | XhoI | BamHI, XhoI |
| LYM242 | pQFN | BamHI | XhoI | BamHI, XhoI |
| LYM243 | pQFN | BamHI | XhoI | BamHI, XhoI |
| LYM245 | pQFN | BamHI | KpnI | BamHI, KpnI |
| LYM248 | pQFN | BamHI | EcoRV | BamHI, StuI |
| LYM249 | pQFN | BamHI | KpnI | BamHI. KpnI |
| LYM250 | pQFN | SalI | XbaI | SalI, StuI |
| LYM251 | pQFN | SalI | Ecl136II | SalI, StuI |
| LYM252 | pQFN | BamHI | KpnI | BamHI, KpnI |
| LYM254 | pQFN | SalI | BamHI | SalI, BamHI |
| LYM255 | pQFN | BamHI | XhoI | BamHI, XhoI |
| LYM256 | pQFN | BamHI | XhoI | BamHI, XhoI |
| LYM26 | pQYN_6669 | SalI | EcoRI | SalI, EcoRI |
| LYM260 | pQFN | BamHI | KpnI | BamHI, Kpnl |
| LYM261 | pQFN | SmaI | KpnI | SmaI, Kpnl |
| LYM267 | pQFN | SalI | EcoRV | SalI, StuI |
| LYM268 | pQFN | XhoI | EcoRV | XhoI, StuI |
| LYM270 | pQFN | BamHI | XhoI | BamHI, Xhol |
| LYM271 | pQFN | BamHI | XhoI | BamHI, XhoI |
| LYM273_ G | pQFN | BamHI | XhoI | BamHI, XhoI |
| LYM273_S | pQFN | BamHI | XhoI | BamHI, Xhol |
| LYM274 | pQFN | BamHI | XhoI | BamHI, Xhol |
| LYM277 | pQFN | SalI | Ecl136II | SalI, StuI |
| LYM278 | pQYN_6669 | SalI | EcoRI | SalI, EcoRI |
| LYM283 | pQFN | SmaI | KpnI | Smal, Kpnl |
| LYM284 | pQFN | BamHI | KpnI | BamHI, KpnI |
| LYM285 | pQFN | XhoI | EcoRV | XhoI, StuI |
| LYM287 | pQFN | XhoI | EcoRV | XhoI, StuI |
| LYM288 | pQFN | XhoI | EcoRI | XhoI, EcoRI |
| LYM289 | pQYN_6669 | SalI | EcoRI | SalI, EcoRI |
| LYM290 | pQFN | BamHI | KpnI | BamHI, KpnI |
| LYM291 | pQFN | SalI | BamHI | SalI, BamHI |
| LYM293 | pQFN | Xhol | EcoRI | XhoI, EcoRI |
| LYM3 | pQFN | XhoI | KpnI | XhoI, KpnI |
| LYM30 | pQFN | SalI | XhoI | SalI, Xhol |
| LYM31 | pQFN | SalI | Xhol | SalI, XhoI |
| LYM32 | pQFN | BamHI | KpnI | BamHI, KpnI |
| LYM34 | pQFN | BamHI | KpnI | BamHI, Kpnl |
| LYM35 | pQYN_6669 | SalI | EcoRI | SalI, EcoRI |
| LYM36 | pQFN | StuI | StuI | StuI, StuI |
| LYM37 | pQYN_6669 | SalI | EcoRI | SalI, EcoRI |
| LYM38 | pQFN | SalI | BamHI | SalI, BamHI |
| LYM4 | pQFN | EcoRV | KpnI | SmaI, KpnI |
| LYM40 | pQFN | SalI | EcoRV | SalI, StuI |
| LYM41 | pQYN_6669 | SalI | EcoRI | SalI, EcoRI |
| LYM42 | pQYN_6669 | SalI | EcoRI | SalI, EcoRI |
| LYM43 | pQYN_6669 | SalI | EcoRI | SalI, EcoRI |
| LYM44 | pQYN_6669 | SalI | EcoRI | SalI, EcoRI |
| LYM5 | pQFN | SalI | BamHI | SalI, BamHI |
| LYM51 | pQYN_6669 | SalI | EcoRI | SalI, EcoRI |
| LYM52 | pQFN | Xhol | EcoRV | XhoI, StuI |
| LYM53 | pQYN_6669 | SalI | EcoRI | SalI, EcoRI |
| LYM56 | pQYN_6669 | SalI | EcoRI | SalI, EcoRI |
| LYM57 | pQFN | EcoRV | XhoI | SmaI, XhoI |
| LYM6 | pQFN | SmaI | KpnI | SmaI, KpnI |
| LYM61 | pQFN | BamHI | XhoI | BamHI, XhoI |
| LYM62 | pQFN | BamHI | KpnI | BamHI, Kpnl |
| LYM66 | pQFN | EcoRV | XhoI | SmaI, XhoI |
| LYM67 | pQYN_6669 | Sail | EcoRI | SalI, EcoRI |
| LYM68 | pQFN | SalI | XhoI | SalI, XhoI |
| LYM69 | pQYN_6669 | SalI | EcoRI | SalI, EcoRI |
| LYM7 | pQFN | SalI | EcoRI | SalI, EcoRI |
| LYM73 | pQFN | SalI | StuI | SalI, StuI |
| LYM74 | pQFN | Sail | Ecl136II | SalI, StuI |
| LYM79 | pQYN_6669 | SalI | EcoRI | SalI, EcoRI |
| LYM8 | pQFN | XhoI | KpnI | Xhol, KpnI |
| LYM82 | pQYN_6669 | SalI | EcoRI | SalI, EcoRI |
| LYM83 | pQFN | BamHI | Xhol | BamHI, XhoI |
| LYM84 | pQFN | BamHI | XhoI | BamHI, XhoI |
| LYM86 | pQFN | BamHI | XhoI | BamHI, XhoI |
| LYM88 | pQFN | BamHI | XhoI | BamHI, XhoI |
| LYM89 | pQYN_6669 | SalI | EcoRI | SalI, EcoRI |
| LYM9 | pQFN | SalI | EcoRI | SalI, EcoRI |
| LYM90 | pQYN_6669 | SalI | EcoRI | SalI, EcoRI |
| LYM91 | pQYN_6669 | SalI | EcoRI | SalI, EcoRI |
| LYM93 | pQFN | SalI | Xhol | SalI, XhoI |
| LYM95 | pQYN_6669 | SalI | EcoRI | SalI, EcoRI |
| LYM99 | pQFN | BamHI | KpnI | BamHI, Kpnl |

Table 27.

**Table 28**

| ***Genes cloned from cDNA libraries or genomic DNA in a High copy number plasmid*** | | | | | |
|---|---|---|---|---|---|
| ***Gene Name*** | ***High copy plasmid*** | ***Amplified from*** | | ***Polynucleotide SEQ ID NO:*** | ***Polypeptide SEQ ID NO:*** |
| | | ***Organism*** | ***Origin*** | | |
| LYM1 | pGXN (pKG+Nos+35S) | RICE Oryza sativa L. Japonica ND | cDNA | 3481 | 240 |
| LYM10 | pKS(Pks_J) | RICE Oryza sativa L. Japonica ND | cDNA | 3490 | 249 |
| LYM100 | pGXN (pKG+Nos+35S) | BARLEY Hordeum vulgare L. Manit | cDNA | 3542 | 301 |
| LYM102 | pKS(Pks_J) | RICE Oryza sativa L. Japonica ND | cDNA | 3543 | 302 |
| LYM 103 | pKS(Pks_J) | MAIZE Zea mays L. Pioneer 30G54 | cDNA | 3544 | 3689 |
| LYM105 | pKS(Pks_J) | BARLEY Hordeum vulgare L. Manit | cDNA | 3545 | 3690 |
| LYM106 | pGXN (pKG+Nos+35S) | BARLEY Hordeum vulgare L. Manit | cDNA | 3546 | 305 |
| LYM 107 | pKS(Pks_J) | MAIZE Zea mays L. ND | cDNA | 3589 | 349 |
| LYM 109 | pGXNa | MAIZE Zea mays L. ND | cDNA | 3590 | 3702 |
| LYM110 | pKS(Pks_J) | MAIZE Zea mays L. ND | cDNA | 3547 | 3691 |
| LYM111 | pGXNa | MAIZE Zea mays L. Pioneer 30G54 | cDNA | 3548 | 307 |
| LYM112 | pKS(Pks_J) | MAIZE Zea mays L. Pioneer 30G54 | cDNA | 3591 | 3703 |
| LYM113 | pGXN (pKG+Nos+35S) | MAIZE Zea mays L. Pioneer 30G54 | cDNA | 3592 | 352 |
| LYM115 | pKS(Pks_J) | MAIZE Zea mays L. ND | cDNA | 3593 | 3704 |
| LYM116 | pGXN (pKG+Nos+35S) | MAIZE Zea mays L. ND | cDNA | 3594 | 354 |
| LYM117 | Topo B | MAIZE Zea mays L. ND | cDNA | 3595 | 3705 |
| LYM118 | | | GeneArt | 3596 | 356 |
| LYM119 | pGXN (pKG+Nos+35S) | MAIZE Zea mays L. ND | cDNA | 3549 | 3692 |
| LYM12 | pKS(Pks_J) | RICE Oryza sativa L. Japonica ND | cDNA | 3491 | 250 |
| LYM 120 | pKS(Pks_J) | RICE Oryza sativa L. Japonica ND | cDNA | 3550 | 309 |
| LYM121 | pKS(Pks_J) | RICE Oryza sativa L. Japonica ND | cDNA | 3597 | 357 |
| LYM122_G | Topo B | RICE Oryza sativa L. Japonica ND | Genomic | 3739 | 310 |
| LYM122_S | | | GeneArt | 3551 | 310 |
| LYM 123 | | | GeneArt | 3598 | 358 |
| LYM 125 | Topo B | RICE Oryza sativa L. Japonica ND | cDNA | 3552 | 311 |
| LYM 126 | | | GeneArt | 3553 | 312 |
| LYM127 | Topo B | RICE Oryza sativa L. Japonica ND | cDNA | 3554 | 313 |
| LYM 128 | pKS(Pks_J) | RICE Oryza sativa L. Japonica ND | cDNA | 3555 | 314 |
| LYM 129 | pGXN (pKG+Nos+35S) | RICE Oryza sativa L. Indica ND+ RICE Oryza sativa L. Japonica ND | cDNA | 3556 | 315 |
| LYM13 | pKS(Pks_J) | RICE Oryza sativa L. Japonica ND | cDNA | 3492 | 251 |
| LYM 130 | pGXN (pKG+Nos+35S) | RICE Oryza sativa L. Indica ND | cDNA | 3557 | 316 |
| LYM131 | pGXNa | RICE Oryza sativa L. Japonica ND | cDNA | 3558 | 3693 |
| LYM 132 | pKS(Pks_J) | RICE Oryza sativa L. Japonica ND | cDNA | 3559 | 318 |
| LYM 134 | pKS(Pks_J) | RICE Oryza sativa L. Japonica ND | cDNA | 3560 | 319 |
| LYM 135 | Topo B | RICE Oryza sativa L. Japonica ND | cDNA | 3599 | 359 |
| LYM 136 | pKS(Pks_J) | RICE Oryza sativa L. Japonica ND | cDNA | 3561 | 3694 |
| LYM 137 | pGXN (pKG+Nos+35S) | BARLEY Hordeum vulgare L. Manit | cDNA | 3562 | 321 |
| LYM 138 | pGXN (pKG+Nos+35S) | RICE Oryza sativa L. Japonica ND | cDNA | 3600 | 360 |
| LYM14 | pKS(Pks_J) | RICE Oryza sativa L. Japonica ND | cDNA | 3493 | 252 |
| LYM140 | pGXNa | BARLEY Hordeum vulgare L. Manit | cDNA | 3563 | 322 |
| LYM141 | Topo B | RICE Oryza sativa L. Japonica ND | cDNA | 3564 | 323 |
| LYM142 | pGXN (pKG+Nos+35S) | BARLEY Hordeum vulgare L. Manit | cDNA | 3565 | 324 |
| LYM 143 | pGXN (pKG+Nos+35S) | RICE Oryza sativa L. Japonica ND | cDNA | 3566 | 325 |
| LYM 144 | pKS(Pks_J) | RICE Oryza sativa L. Japonica ND | cDNA | 3567 | 3695 |
| LYM 145 | pKS(Pks_J) | RICE Oryza sativa L. ND | cDNA | 3568 | 327 |
| LYM 146 | Topo B | MAIZE Zea mays L. ND | Genomic | 3601 | 3706 |
| LYM147 | pGXN (pKG+Nos+35S) | MAIZE Zea mays L. ND | cDNA | 3602 | 3707 |
| LYM 148 | pKS(Pks_J) | BARLEY Hordeum vulare L. Manit | cDNA | 3569 | 3696 |
| LYM 149 | pGXN (pKG+Nos+35S) | BARLEY Hordeum vulpare L. Manit | cDNA | 3570 | 329 |
| LYM15 | pGXN (pKG+Nos+35S) | RICE Oryza sativa L. Japonica ND | cDNA | 3494 | 253 |
| LYM152 | pGXN (pKG+Nos+35S) | ARABIDOPSIS Arabidopsis thaliana Transgenic Columbia | cDNA | 3571 | 330 |
| LYM153 | pGXN (pKG+Nos+35S) | RICE Oryza sativa L. Japonica ND | cDNA | 3572 | 331 |
| LYM 154 | | | GeneArt | 3603 | 363 |
| LYM 155 | pGXN (pKG+Nos+35S) | BARLEY Hordeum vulgare L. Manit | cDNA | 3604 | 3708 |
| LYM156 | pGXNa | BARLEY Hordeum vulgare L. Manit | cDNA | 3573 | 332 |
| LYM157_G | pGXN (pKG+Nos+35S) | BARLEY Hordeum vulgare L. Manit | cDNA | 3574 | 333 |
| LYM157_S | | | GeneArt | 3574 | 333 |
| LYM159 | pGXN (pKG+Nos+35S) | BARLEY Hordeum vulgare L. Manit | cDNA | 3575 | 3697 |
| LYM16 | pGXN (pKG+Nos+35S) | RICE Oryza sativa L. ND | cDNA | 3495 | 254 |
| LYM 160 | pGXN (pKG+Nos+35S) | BARLEY Hordeum vulgare L. Manit | cDNA | 3576 | 3698 |
| LYM 161 | pKS(Pks_J) | BARLEY Hordeum vulgare L. Manit | cDNA | 3577 | 3699 |
| LYM162 | pKS(Pks_J) | MAIZE Zea mays L. ND | cDNA | 3578 | 337 |
| LYM164 | pGXN (pKG+Nos+35S) | RICE Oryza sativa L. ND | cDNA | 3579 | 3700 |
| LYM165 | Topo B | MAIZE Zea mays L. Pioneer 30G54 | cDNA | 3580 | 339 |
| LYM17 | pKS(Pks_J) | RICE Oryza sativa L. ND | cDNA | 3496 | 255 |
| LYM 170 | pGXN (pKG+Nos+35S) | RICE Oryza sativa L. ND | cDNA | 3581 | 341 |
| LYM172 | pKS(Pks_J) | RICE Oryza sativa L. Indica ND | cDNA | 3582 | 342 |
| LYM 173 | pKS(Pks_J) | RICE Oryza sativa L. Japonica ND | cDNA | 3583 | 343 |
| LYM174 | pKS(Pks_J) | SORGHUM Sorghum bicolor Monsanto S5 | cDNA | 3584 | 344 |
| LYM 175 | pGXN (pKG+Nos+35S) | RICE Oryza sativa L. ND | cDNA | 3585 | 345 |
| LYM176 | pGXN (pKG+Nos+35S) | RICE Oryza sativa L. Japonica ND | cDNA | 3586 | 346 |
| LYM 178 | pGXN (pKG+Nos+35S) | ARABIDOPSIS Arabidopsis thaliana ND | cDNA | 3587 | 347 |
| LYM179 | pGXNa | MAIZE Zea mays L. Pioneer 30G54 | cDNA | 3588 | 3701 |
| LYM180 | pKS(Pks_J) | BARLEY Hordeum vulgare L. Manit | cDNA | 3605 | 365 |
| LYM181 | Topo B | BARLEY Hordeum vulgare L. Manit | cDNA | 3606 | 366 |
| LYM183 | Topo B | BARLEY Hordeum vulgare L. Manit | cDNA | 3655 | 3733 |
| LYM 184 | Topo B | BARLEY Hordeum vulgare L. Manit | cDNA | 3607 | 3709 |
| LYM 185 | pKS(Pks_J) | BARLEY Hordeum vulgare L. Manit | cDNA | 3608 | 369 |
| LYM 186 | pGXN (pKG+Nos+35S) | BARLEY Hordeum vulgare L. Manit | cDNA | 3609 | 3710 |
| LYM188 | pKS(Pks_J) | BARLEY Hordeum vulgare L. Manit | cDNA | 3610 | 371 |
| LYM 189 | pGXN (pKG+Nos+35S) | BARLEY Hordeum vulgare L. Manit | cDNA | 3611 | 3711 |
| LYM19 | pGXN (pKG+Nos+35S) | RICE Oryza sativa L. Japonica ND | cDNA | 3497 | 256 |
| LYM 192 | pKS(Pks_J) | BARLEY Hordeum vulgare L. Manit | cDNA | 3612 | 3712 |
| LYM 193 | pKS(Pks_J) | BARLEY Hordeum vulgare L. Manit | cDNA | 3613 | 374 |
| LYM194 | | | GeneArt | 3614 | 3713 |
| LYM 196 | Topo B | MAIZE Zea mays L. ND | cDNA | 3615 | 376 |
| LYM 197 | pKS(Pks_J) | MAIZE Zea mays L. ND | cDNA | 3616 | 3714 |
| LYM 198 | pKS(Pks_J) | MAIZE Zea mays L. ND | cDNA | 3617 | 378 |
| LYM2 | pKS(Pks_J) | RICE Oryza sativa L. ND | cDNA | 3482 | 241 |
| LYM20 | pKS(Pks_J) | RICE Oryza sativa L. Japonica ND | cDNA | 3498 | 257 |
| LYM200 | pKS(Pks_J) | MAIZE Zea mays L. ND | cDNA | 3657 | 419 |
| LYM201 | pKS(Pks_J) | MAIZE Zea mays L. ND | cDNA | 3618 | 379 |
| LYM203 | pKS(Pks_J) | MAIZE Zea mays L. ND | cDNA | 3619 | 380 |
| LYM204 | Topo B | MAIZE Zea mays L. Pioneer 30G54 | cDNA | 3620 | 381 |
| LYM206 | pKS(Pks_J) | MAIZE Zea mays L. ND | cDNA | 3621 | 3715 |
| LYM207 | Topo B | MAIZE Zea mays L. Pioneer 30G54 | cDNA | 3622 | 3716 |
| LYM208 | pKS(Pks_J) | MAIZE Zea mays L. Pioneer 30G54 | cDNA | 3623 | 384 |
| LYM21 | pKS(Pks_J) | RICE Oryza sativa L. Japonica ND | cDNA | 3499 | 258 |
| LYM212 | pGXN (pKG+Nos+35S) | MAIZE Zea mays L. ND | cDNA | 3624 | 3717 |
| LYM213 | pKS(Pks_J) | MAIZE Zea mays L. ND | cDNA | 3625 | 386 |
| LYM215 | pKS(Pks_J) | MAIZE Zea mays L. ND | cDNA | 3626 | 3718 |
| LYM217 | pGXN | MAIZE Zea mays L. ND | cDNA | 3627 | 3719 |
| LYM219 | pKS(Pks_J) | MAIZE Zea mays L. Pioneer 30G54 | cDNA | 3628 | 3720 |
| LYM22 | pGXN (pKG+Nos+35S) | RICE Oryza sativa L. Japonica ND | cDNA | 3500 | 259 |
| LYM220 | pKS(Pks_J) | MAIZE Zea mays L. Pioneer 30G54 | cDNA | 3629 | 3721 |
| LYM221 | pKS(Pks_J) | MAIZE Zea mays L. Pioneer 30G54 | cDNA | 3630 | 3722 |
| LYM223 | pGXNa | MAIZE Zea mays L. Pioneer 30G54 | cDNA | 3631 | 392 |
| LYM224 | pKS(Pks_J) | MAIZE Zea mays L. Pioneer 30G54 | cDNA | 3632 | 3723 |
| LYM227 | | | GeneArt | 3633 | 394 |
| LYM228 | Topo B | MAIZE Zea mays L. Pioneer 30G54 | cDNA | 3634 | 3724 |
| LYM23 | pKS(Pks_J) | RICE Oryza sativa L. Japonica ND | cDNA | 3501 | 260 |
| LYM232 | Topo B | RICE Oryza sativa L. Japonica ND | cDNA | 3635 | 3725 |
| LYM233 | | | GeneArt | 3636 | 397 |
| LYM234 | | | GeneArt | 3637 | 398 |
| LYM236 | pGXN (pKG+Nos+35S) | RICE Oryza sativa L. Japonica ND | cDNA | 3638 | 3726 |
| LYM238 | Topo B | RICE Oryza sativa L. Japonica ND | cDNA | 3639 | 400 |
| LYM239 | pKS(Pks_J) | RICE Oryza sativa L. Japonica ND | cDNA | 3640 | 3727 |
| LYM24 | pGXN (pKG+Nos+35S) | RICE Oryza sativa L. Japonica ND | cDNA | 3502 | 261 |
| LYM240 | pKS(Pks_J) | RICE Oryza sativa L. Japonica ND | cDNA | 3641 | 402 |
| LYM241 | Topo B | RICE Oryza sativa L. Japonica ND | cDNA | 3642 | 3728 |
| LYM242 | pKS(Pks_J) | RICE Oryza sativa L. Indica ND | cDNA | 3643 | 404 |
| LYM243 | pKS(Pks_J) | RICE Oryza sativa L. Japonica ND | cDNA | 3644 | 405 |
| LYM245 | pKS(Pks_J) | RICE Oryza sativa L. Japonica ND | cDNA | 3645 | 406 |
| LYM248 | pKS(Pks_J) | RICE Oryza sativa L. Indica ND | cDNA | 3646 | 3729 |
| LYM249 | Topo B | RICE Oryza sativa L. Japonica ND+ RICE Oryza sativa L. ND | cDNA | 3647 | 3730 |
| LYM250 | pGXN (pKG+Nos+35S) | RICE Oryza sativa L. Japonica ND | cDNA | 3648 | 409 |
| LYM251 | Topo B | RICE Oryza sativa L. Japonica ND | cDNA | 3649 | 410 |
| LYM252 | pKS(Pks_J) | RICE Oryza sativa L. Indica ND+ RICE Oryza sativa L. Japonica ND | cDNA | 3650 | 411 |
| LYM254 | Topo B | RICE Oryza sativa L. Japonica ND | cDNA | 3651 | 3731 |
| LYM255 | pKS(Pks_J) | RICE Oryza sativa L. Japonica ND | cDNA | 3652 | 3732 |
| LYM256 | pKS(Pks_J) | RICE Oryza sativa L. Japonica ND | cDNA | 3656 | 418 |
| LYM26 | pGXN (pKG+Nos+35S) | BARLEY Hordeum vulgare L. Manit | cDNA | 3503 | 262 |
| LYM260 | Topo B | RICE Oryza saliva L. Japonica ND | cDNA | 3653 | 414 |
| LYM261 | Topo B | RICE Oryza sativa L. Japonica ND | cDNA | 3654 | 415 |
| LYM267 | pKS(Pks_J) | MAIZE Zea mays L. ND | cDNA | 3658 | 420 |
| LYM268 | Topo B | RICE Oryza sativa L. Indica ND+ RICE Oryza sativa L. Japonica ND | cDNA | 3659 | 421 |
| LYM270 | pKS(Pks_J) | MAIZE Zea mays L. ND | cDNA | 3660 | 422 |
| LYM27 1 | pKS(Pks_J) | MAIZE Zea mays L. Pioneer 30G54 | cDNA | 3661 | 423 |
| LYM273_G | pKS(Pks_J) | RICE Oryza sativa L. Japonica ND | Genomic | 3738 | 425 |
| LYM273.S | | | GeneArt | 3662 | 425 |
| LYM274 | pKS(Pks_J) | RICE Oryza sativa L. Japonica ND | cDNA | 3663 | 3734 |
| LYM277 | Topo B | RICE Oryza sativa L. Japonica ND | cDNA | 3664 | 3735 |
| LYM278 | pGXN (pKG+Nos+35S) | BARLEY Hordeum vulgare L. Manit | cDNA | 3665 | 3736 |
| LYM283 | Topo B | RICE Oryza sativa L. ND | cDNA | 3666 | 429 |
| LYM284 | pKS(Pks_J) | RICE Oryza sativa L. Japonica ND | cDNA | 3667 | 430 |
| LYM285 | pKS(Pks_J) | RICE Oryza sativa L. Japonica ND | cDNA | 3668 | 431 |
| LYM287 | pKS(Pks_J) | RICE Oryza sativa L. Japonica ND | cDNA | 3669 | 432 |
| LYM288 | pGXNa | RICE Oryza sativa L. Indica ND+ RICE Oryza sativa L. Japonica ND | cDNA | 3670 | 3737 |
| LYM289 | pGXN (pKG+Nos+35S) | BARLEY Hordeum vulgare L. Manit | cDNA | 3671 | 434 |
| LYM290 | pKS(Pks_J) | MAIZE Zea mays L. ND | cDNA | 3672 | 435 |
| LYM291 | pKS(Pks_J) | RICE Oryza sativa L. Japonica ND | cDNA | 3673 | 436 |
| LYM293 | pGXNa | RICE Oryza sativa L. Indica ND | cDNA | 3674 | 437 |
| LYM3 | pKS(Pks_J) | RICE Oryza sativa L. Indica ND | cDNA | 3483 | 3675 |
| LYM30 | pGXNa | RICE Oryza sativa L. ND | cDNA | 3504 | 3677 |
| LYM31 | pGXNa | RICE Oryza sativa L. ND | cDNA | 3505 | 264 |
| LYM32 | | | GeneArt | 3506 | 265 |
| LYM34 | pKS(Pks_J) | RICE Oryza sativa L. Indica ND | cDNA | 3507 | 3678 |
| LYM35 | pGXN (pKG+Nos+35S) | RICE Oryza sativa L. ND | cDNA | 3508 | 267 |
| LYM36 | | | GeneArt | 3509 | 268 |
| LYM37 | pGXN (pKG+Nos+35S) | RICE Oryza sativa L. Japonica ND | cDNA | 3510 | 269 |
| LYM38 | | | GeneArt | 3511 | 270 |
| LYM4 | pKS(Pks_J) | RICE Oryza sativa L. ND | cDNA | 3484 | 243 |
| LYM40 | Topo B | RICE Oryza sativa L. Japonica ND | cDNA | 3512 | 271 |
| LYM41 | pGXN (pKG+Nos+35S) | RICE Oryza sativa L. Japonica ND | cDNA | 3513 | 272 |
| LYM42 | pGXN (pKG+Nos+35S) | RICE Oryza sativa L. Japonica ND | cDNA | 3514 | 273 |
| LYM43 | pGXN (pKG+Nos+35S) | RICE Oryza sativa L. ND | cDNA | 3515 | 274 |
| LYM44 | pGXN (pKG+Nos+35S) | RICE Oryza sativa L. Japonica ND | cDNA | 3516 | 275 |
| LYM5 | pKS(Pks_J) | RICE Oryza sativa L. Indica ND | cDNA | 3485 | 244 |
| LYM51 | pGXN (pKG+Nos+35S) | BARLEY Hordeum vulgare L. Manit | cDNA | 3517 | 3679 |
| LYM52 | pKS(Pks_J) | BARLEY Hordeum vulgare L. Manit | cDNA | 3518 | 277 |
| LYM53 | pGXN (pKG+Nos+35S) | MAIZE Zea mays L. ND | cDNA | 3519 | 3680 |
| LYM56 | pGXN (pKG+Nos+35S) | BARLEY Hordeum vulgare L. Manit | cDNA | 3520 | 3681 |
| LYM57 | pKS(Pks_J) | RICE Oryza sativa L. Japonica ND | cDNA | 3521 | 3682 |
| LYM6 | pKS(Pks_J) | RICE Oryza sativa L. ND | cDNA | 3486 | 3676 |
| LYM61 | pKS(Pks_J) | MAIZE Zea mays L. ND | cDNA | 3522 | 3683 |
| LYM62 | pKS(Pks_J) | MAIZE Zea mays L. ND | cDNA | 3523 | 3684 |
| LYM66 | pKS(Pks_J) | BARLEY Hordeum vulgare L. Manit | cDNA | 3524 | 3685 |
| LYM67 | pGXN (pKG+Nos+35S) | RICE Oryza sativa L. ND | cDNA | 3525 | 284 |
| LYM68 | pGXNa | RICE Oryza sativa L. ND | cDNA | 3526 | 3686 |
| LYM69 | pGXN (pKG+Nos+35S) | RICE Oryza sativa L. ND | cDNA | 3527 | 286 |
| LYM7 | pGXN (pKG+Nos+35S) | RICE Oryza sativa L. ND | cDNA | 3487 | 246 |
| LYM73 | Topo B | RICE Oryza sativa L. ND | cDNA | 3528 | 287 |
| LYM74 | | | GeneArt | 3529 | 288 |
| LYM79 | pGXN (pKG+Nos+35S) | MAIZE Zea mays L. ND | cDNA | 3530 | 3687 |
| LYM8 | pKS(Pks_J) | RICE Oryza sativa L. Japonica ND | cDNA | 3488 | 247 |
| LYM82 | pGXN (pKG+Nos+35S) | BARLEY Hordeum vulgare L. Manit | cDNA | 3531 | 290 |
| LYM83 | Topo B | BARLEY Hordeum vulgare L. Manit | cDNA | 3532 | 3688 |
| LYM84 | pKS(Pks_J) | BARLEY Hordeum vulgare L. Manit | cDNA | 3533 | 292 |
| LYM86 | pKS(Pks_J) | RICE Oryza sativa L. Indica ND | cDNA | 3534 | 293 |
| LYM88 | pKS(Pks_J) | ARABIDOPSIS Arabidopsis thaliana Transgenic Columbia | cDNA | 3535 | 294 |
| LYM89 | pGXN (pKG+Nos+35S) | ARABIDOPSIS Arabidopsis thaliana Transgenic Columbia | cDNA | 3536 | 295 |
| LYM9 | pGXN (pKG+Nos+35S) | RICE Oryza sativa L. ND | cDNA | 3489 | 248 |
| LYM90 | pGXN (pKG+Nos+35S) | BARLEY Hordeum vulgare L. Manit | cDNA | 3537 | 296 |
| LYM91 | pGXN (pKG+Nos+35S) | BARLEY Hordeum vulgare L. Manit | cDNA | 3538 | 297 |
| LYM93 | Topo B | BARLEY Hordeum vulgare L. Manit | cDNA | 3539 | 298 |
| LYM95 | pGXN (pKG+Nos+35S) | BARLEY Hordeum vulgare L. Manit | cDNA | 3541 | 300 |
| LYM99 | pKS(Pks_J) | BARLEY Hordeum vulgare L. Manit | cDNA | 3540 | 299 |

Table 28: Cloned and synthetic genes are provided along with the sequence identifiers of their polynucleotides and polypeptides. Also provided are the source organism, tissue and the cloning vectors. ND = not a determined ecotype.

Selected DNA sequences were synthesized by a commercial supplier GeneArt, GmbH [Hypertext Transfer Protocol://World Wide Web (dot) geneart (dot) com/)]. Synthetic DNA is designed in silico. Suitable restriction enzymes sites were added to the cloned sequences at the 5' end and at the 3' end to enabled later cloning into the pQFN/ pQYN_6669 binary vectors downstream of the 6669 promoter (SEQ ID NO:4198).

### EXAMPLE 8

### TRANSFORMING AGROBACTERIUM TUMEFACIENS CELLS WITH BINARY VECTORS HARBORING PUTATIVE GENES

Each of the binary vectors described in Example 7 above are used to transform *Agrobacterium* cells. Two additional binary constructs, having a GUS/Luciferase reporter gene replacing the selected gene (positioned downstream of the At6669 promoter), are used as negative controls.

The binary vectors are introduced to *Agrobacterium tumefaciens* GV301, or LB4404 competent cells (about 10⁹ cells/mL) by electroporation. The electroporation is performed using a MicroPulser electroporator (Biorad), 0.2 cm cuvettes (Biorad) and EC-2 electroporation program (Biorad). The treated cells are cultured in LB liquid medium at 28 °C for 3 hours, then plated over LB agar supplemented with gentamycin (50 mg/L; for *Agrobacterium* strains GV301) or streptomycin (300 mg/L; for *Agrobacterium* strain LB4404) and kanamycin (50 mg/L) at 28 °C for 48 hours. *Abrobacterium* colonies which developed on the selective media are analyzed by PCR using the primers which are designed to span the inserted sequence in the pPI plasmid. The resulting PCR products are isolated and sequenced as described in Example 3 above, to verify that the correct yield sequences are properly introduced to the *Agrobacterium* cells.

### EXAMPLE 9

### PRODUCING TRANSGENIC ARABIDOPSIS PLANTS EXPRESSING SELECTED GENES

### Materials and Experimental Methods

***Plant transformation*** - The *Arabidopsis thaliana* var Columbia (T₀ plants) were transformed according to the Floral Dip procedure [Clough SJ, Bent AF. (1998) Floral dip: a simplified method for Agrobacterium-mediated transformation of Arabidopsis thaliana. Plant J. 16(6): 735-43; and Desfeux C, Clough SJ, Bent AF. (2000) Female reproductive tissues are the primary targets of Agrobacterium-mediated transformation by the Arabidopsis floral-dip method. Plant Physiol. 123(3): 895-904] with minor modifications. Briefly, *Arabidopsis thaliana* Columbia (Col0) T₀ plants were sown in 250 ml pots filled with wet peat-based growth mix. The pots were covered with aluminum foil and a plastic dome, kept at 4 °C for 3-4 days, then uncovered and incubated in a growth chamber at 18-24 °C under 16/8 hours light/dark cycles. The T₀ plants were ready for transformation six days before anthesis.

Single colonies of *Agrobacterium* carrying the binary vectors harboring the yield genes were cultured in LB medium supplemented with kanamycin (50 mg/L) and gentamycin (50 mg/L). The cultures were incubated at 28 °C for 48 hours under vigorous shaking and centrifuged at 4000 rpm for 5 minutes. The pellets comprising *Agrobacterium* cells were resuspended in a transformation medium which contained half-strength (2.15 g/L) Murashige-Skoog (Duchefa); 0.044 µM benzylamino purine (Sigma); 112 µg/L B5 Gambourg vitamins (Sigma); 5 % sucrose; and 0.2 ml/L Silwet L-77 (OSI Specialists, CT) in double-distilled water, at pH of 5.7.

Transformation of T₀ plants was performed by inverting each plant into an *Agrobacterium* suspension such that the above ground plant tissue was submerged for 3-5 seconds. Each inoculated T₀ plant was immediately placed in a plastic tray, then covered with clear plastic dome to maintain humidity and was kept in the dark at room temperature for 18 hours to facilitate infection and transformation. Transformed (transgenic) plants were then uncovered and transferred to a greenhouse for recovery and maturation. The transgenic T₀ plants were grown in the greenhouse for 3-5 weeks until siliques were brown and dry, then seeds were harvested from plants and kept at room temperature until sowing.

For generating T₁ and T₂ transgenic plants harboring the genes, seeds collected from transgenic T₀ plants were surface-sterilized by soaking in 70 % ethanol for 1 minute, followed by soaking in 5 % sodium hypochlorite and 0.05 % triton for 5 minutes. The surface-sterilized seeds were thoroughly washed in sterile distilled water then placed on culture plates containing half-strength Murashig-Skoog (Duchefa); 2 % sucrose; 0.8 % plant agar; 50 mM kanamycin; and 200 mM carbenicylin (Duchefa). The culture plates were incubated at 4 °C for 48 hours then transferred to a growth room at 25 °C for an additional week of incubation. Vital T₁ *Arabidopsis* plants were transferred to a fresh culture plates for another week of incubation. Following incubation the T₁ plants were removed from culture plates and planted in growth mix contained in 250 ml pots. The transgenic plants were allowed to grow in a greenhouse to maturity. Seeds harvested from T₁ plants were cultured and grown to maturity as T₂ plants under the same conditions as used for culturing and growing the T₁ plants.

### EXAMPLE 10

### IMPROVED TRANSGENIC PLANT PERFORMANCE-GREENHOUSE ASSAYS

To analyze the effect of expression of the isolated polynucleotides in plants, plants performance was tested under greenhouse conditions.

***Greenhouse assays* -** The plants were analyzed for their overall size, growth rate, flowering, seed yield, weight of 1,000 seeds, dry matter and harvest index (HI-seed yield/dry matter). Transgenic plants performance was compared to control plants grown in parallel under the same conditions. Mock- transgenic plants expressing the uidA reporter gene (GUS-Intron) or with no gene at all, under the same promoter were used as control.

The experiment was planned in nested randomized plot distribution. For each gene, three to five independent transformation events were analyzed from each construct. In cases where a certain event appears more than once, the event was tested in several independent experiments.

Tables 29 and 31 specify the parameters measured in plants in the greenhouse assays (till seed maturation and bolting assay, respectively).

***Digital imaging*** - A laboratory image acquisition system, which consists of a digital reflex camera (Canon EOS 300D) attached with a 55 mm focal length lens (Canon EF-S series), mounted on a reproduction device (Kaiser RS), which included 4 light units (4 x 150 Watts light bulb) is used for capturing images of plant samples.

The image capturing process was repeated every 2 days starting from day 1 after transplanting till day 16. Same camera, placed in a custom made iron mount, was used for capturing images of larger plants sawn in white tubs in an environmental controlled greenhouse. The tubs were square shape include 1.7 liter trays. During the capture process, the tubs were placed beneath the iron mount, while avoiding direct sun light and casting of shadows.

An image analysis system was used, which consists of a personal desktop computer (Intel P4 3.0 GHz processor) and a public domain program - ImageJ 1.39 (Java based image processing program which was developed at the U.S National Institutes of Health and freely available on the internet at Hypertext Transfer Protocol://rsbweb (dot) nih (dot) gov/). Images were captured in resolution of 10 Mega Pixels (3888 x 2592 pixels) and stored in a low compression JPEG (Joint Photographic Experts Group standard) format. Next, analyzed data was saved to text files and processed using the JMP statistical analysis software (SAS institute).

***Leaf growth analysis*** - Using the digital analysis leaves data was calculated, including leaf number, rosette area, rosette diameter, leaf blade area, plot coverage, leaf petiole length.

***The vegetative growth rate of the plant was defined by formulas XIII, XIV, XV and XVI.*** $Relative growth rate of leaf blade are = Regression coefficient of leaf area along time course.$ $Relative growth rate of rosette area = Regresssion coefficient of rosette area along time course.$ $Relative growth rate of rosette diameter = Regression coefficient of rosette diameter along time course.$ $Relative growth rate of plot coverage = Regression coefficient of plot coverage along time course.$

***Seeds average weight (Seed weight or 1000 seed weight)*** - At the end of the experiment all seeds were collected. The seeds were scattered on a glass tray and a picture was taken. Using the digital analysis, the number of seeds in each sample was calculated.

***Plant dry weight and seed yield*** - On about day 80 from sowing, the plants were harvested and left to dry at 30 °C in a drying chamber. The biomass and seed weight of each plot were measured and divided by the number of plants in each plot. Dry weight = total weight of the vegetative portion above ground (excluding roots) after drying at 30 °C in a drying chamber;$Seed yield per plant = total seed weight per plant \left(gr .\right) .$

1000 seed weight (the weight of 1000 seeds) (gr.).

The harvest index was calculated using Formula IV (Harvest Index = Average seed yield per plant/ Average dry weight) as described above.

***Oil percentage in seeds*** - At the end of the experiment all seeds from plots A-C were collected. Columbia seeds from 3 plots were mixed grounded and then mounted onto the extraction chamber. 210 ml of n-Hexane (Cat No. 080951 Biolab Ltd.) were used as the solvent. The extraction was performed for 30 hours at medium heat 50 °C. Once the extraction has ended the n-Hexane was evaporated using the evaporator at 35 °C and vacuum conditions. The process was repeated twice. The information gained from the Soxhlet extractor (Soxhlet, F. Die gewichtsanalytische Bestimmung des Milchfettes, Polytechnisches J. (Dingler's) 1879, 232, 461) was used to create a calibration curve for the Low Resonance NMR. The content of oil of all seed samples was determined using the Low Resonance NMR (MARAN Ultra- Oxford Instrument) and its MultiQuant sowftware package.

***Oil yield* -** The oil yield was calculated using Formula IX (described above).

Silique length analysis - On day 50 from sowing, 30 siliques from different plants in each plot were sampled in block A. The chosen siliques were green-yellow in color and were collected from the bottom parts of a grown plant's stem. A digital photograph was taken to determine silique's length.

***Statistical analyses*** - To identify genes conferring significantly improved tolerance to abiotic stresses, the results obtained from the transgenic plants were compared to those obtained from control plants. To identify outperforming genes and constructs, results from the independent transformation events tested were analyzed separately. Data was analyzed using Student's t-test and results were considered significant if the p value was less than 0.1. The JMP statistics software package was used (Version 5.2.1, SAS Institute Inc., Cary, NC, USA).

### Experimental Results

Plants expressing the polynucleotides were assayed for a number of commercially desired traits. In cases where a certain event appears more than once, the event was tested in several independent experiments.

**Table 29**

| ***Measured parameters at the greenhouse till seed maturation assay (T2 experiment) for transformed agriculture improving trait genes*** | |
|---|---|
| ***Tested Parameters*** | ***Id*** |
| Dry Weight (gr) | **A** |
| Harvest Index | **B** |
| Leaf Blade Area TP4 (cm²) | **C** |
| Leaf Number TP4 | **D** |
| Leaf Petiole Length TP4 (cm) | **E** |
| Petiole Relative Area TP4 | **F** |
| Plot Coverage TP4 (cm²) | **G** |
| RGR Of Leaf Blade Area | **H** |
| RGR Of Plot Coverage | **I** |
| RGR Of Rosette Area | **J** |
| RGR Of Rosette Diameter | **K** |
| Rosette Area TP4 (cm²) | **L** |
| Rosette Diameter TP4 (cm) | **M** |
| Seed Yield (gr) | **N** |
| Seeds Weight (gr) | **O** |
| Blade Relative Area TP4 | **P** |
| Oil Content | **Q** |
| RGR Of Leaf Number | **R** |

Table 29: Provided are the identification (ID) letters of each of the Tested Parameters. RGR-Relative Growth Rate; TP4- time point 4.

**Table 30**

| ***Results obtained in a Greenhouse till seed maturation assay*** | | | | | | | | | | | |
|---|---|---|---|---|---|---|---|---|---|---|---|
| **Gene name** | **Event** | **ID** | **Mean** | **P value** | ***% incr. vs. cons.*** | **Gene name** | **Event** | **ID** | **Mean** | **P value** | **% incr. vs. cont.** |
| LYM 16 | 1162 3.2 | A | 0.739 | 2.97E -02 | 10.4 | LYM 9 | 1163 3.7 | P | 93.11 5 | 4.23E -03 | 3.2 |
| LYM 57 | 1201 2.6 | A | 0.841 | 4.23E -02 | 25.7 | LYM 15 | 1161 1.3 | P | 93.13 1 | 5.66E -03 | 3.2 |
| LYM 17 | 1168 1.4 | A | 0.728 | 5.16E -02 | 8.8 | LYM 4 | 1170 2.1 | P | 93.16 7 | 7.54E -03 | 3.2 |
| LYM 10 | 1174 4.1 | A | 0.723 | 8.27E -02 | 8 | LYM 17 | 1168 2.3 | P | 92.74 7 | 8.70E -03 | 2.8 |
| LYM 95 | 1212 1.3 | A | 0.779 | 9.81E -02 | 16.5 | LYM 17 | 1168 4.5 | P | 92.55 4 | 1.28E -02 | 2.6 |
| CON TRO L | - | A | 0.669 | - | 0 | LYM 2 | 1169 1.2 | P | 92.87 5 | 1.35E -02 | 2.9 |
| LYM 16 | 1162 3.5 | B | 0.543 | 6.50E -05 | 45.1 | LYM 7 | 1159 4.3 | P | 92.51 3 | 1.79E -02 | 2.5 |
| LYM 24 | 1206 3.3 | B | 0.515 | 2.78E -04 | 37.6 | LYM 67 | 1178 2.5 | P | 94.03 7 | 1.83E -02 | 4.2 |
| LYM 10 | 1174 1.4 | B | 0.499 | 3.74E -04 | 33.4 | LYM 44 | 1188 4.3 | P | 92.30 6 | 2.09E -02 | 2.3 |
| LYM 7 | 1159 4.2 | B | 0.498 | 1.10E -03 | 33.1 | LYM 62 | 1202 2.4 | P | 92.29 6 | 2.21E -02 | 2.3 |
| LYM 44 | 1188 5.3 | B | 0.473 | 1.50E -03 | 26.6 | LYM 19 | 1175 1.4 | P | 92.24 4 | 2.38E -02 | 2.2 |
| LYM 15 | 1161 1.3 | B | 0.477 | 3.91E -03 | 27.7 | LYM 17 | 1168 4.4 | P | 92.43 8 | 3.04E -02 | 2.4 |
| LYM 2 | 1169 5.1 | B | 0.457 | 4.23E -03 | 222 | LYM 31 | 1192 3.1 | P | 92.39 9 | 3.32E -02 | 2.4 |
| LYM 30 | 1191 3.3 | B | 0.462 | 4.25E -03 | 23.6 | LYM 34 | 1190 3.3 | P | 92.07 | 3.49E -02 | 2 |
| LYM 8 | 1198 4.1 | B | 0.472 | 4.44E -03 | 26.3 | LYM 12 | 1187 1.3 | P | 91.99 9 | 3.94E -02 | 2 |
| LYM 31 | 1192 3.1 | B | 0.471 | 4.64E -03 | 26 | LYM 15 | 1161 4.3 | P | 91.99 6 | 5.05E -02 | 1.9 |
| LYM 14 | 1205 1.1 | B | 0.454 | 5.37E -03 | 21.5 | LYM 15 | 1161 4.4 | P | 91.87 7 | 5.09E -02 | 1.8 |
| LYM 2 | 1169 2.3 | B | 0.546 | 5.62E -03 | 46 | LYM 10 | 1174 1.4 | P | 91.97 9 | 5.99E -02 | 1.9 |
| LYM 16 | 1162 4.6 | B | 0.468 | 8.76E -03 | 25.1 | LYM 57 | 1201 2.4 | P | 91.75 2 | 6.64E -02 | 1.7 |
| LYM 66 | 1195 4.4 | B | 0.455 | 9.16E -03 | 21.7 | LYM 24 | 1206 3.3 | P | 92.88 3 | 6.73E -02 | 2.9 |
| LYM 34 | 1190 4.3 | B | 0.442 | 1.11E -02 | 18.3 | LYM 16 | 1162 4.6 | P | 91.72 3 | 7.65E -02 | 1.6 |
| LYM 53 | 1184 3.2 | B | 0.506 | 1.24E -02 | 35.4 | LYM 26 | 1182 4.3 | P | 91.67 2 | 7.95E -02 | 1.6 |
| LYM 35 | 1181 2.4 | B | 0.446 | 1.49E -02 | 19.2 | LYM 66 | 1195 5.2 | P | 91.69 3 | 8.09E -02 | 1.6 |
| LYM 4 | 1170 6.5 | B | 0.456 | 1.57E -02 | 21.9 | LYM 62 | 1202 3.7 | P | 92.17 | 8.37E -02 | 2.1 |
| LYM 15 | 1161 2.2 | B | 0.437 | 1.59E -02 | 16.9 | LYM 30 | 1191 3.3 | P | 91.63 7 | 8.66E -02 | 1.5 |
| LYM 19 | 1175 4.1 | B | 0.448 | 1.60E -02 | 19.8 | LYM 12 | 1187 3.4 | P | 91.84 | 9.17E -02 | 1.8 |
| LYM 9 | 1163 4.6 | B | 0.466 | 1.61E -02 | 24.6 | LYM 51 | 1189 1.1 | P | 92.63 | 9.56E -02 | 2.6 |
| LYM 1 | 1160 1.1 | B | 0.465 | 1.63E -02 | 24.3 | CON TRO L | - | P | 90.23 9 | - | 0 |
| LYM 57 | 1201 3.1 | B | 0.429 | 3.09E -02 | 14.8 | LYM 17 | 1168 4.5 | Q | 31.92 5 | 1.22E -04 | 13.3 |
| LYM 17 | 1168 4.5 | B | 0.501 | 3.38E -02 | 34.1 | LYM 66 | 1195 2.1 | Q | 31.76 | 1.43E -04 | 12.7 |
| LYM 30 | 1191 2.6 | B | 0.506 | 3.67E -02 | 35.3 | LYM 34 | 1190 4.3 | Q | 31.58 5 | 2.06E -04 | 12.1 |
| LYM 24 | 1206 1.2 | B | 0.481 | 4.69E -02 | 28.5 | LYM 17 | 1168 2.3 | Q | 32.16 | 2.39E -04 | 14.1 |
| LYM 82 | 1220 3.2 | B | 0.53 | 4.78E -02 | 41.7 | LYM 82 | 1220 3.2 | Q | 31.18 5 | 4.56E -04 | 10.7 |
| LYM 31 | 1192 4.4 | B | 0.437 | 4.78E -02 | 16.7 | LYM 2 | 1169 2.3 | Q | 31.17 | 7.38E -04 | 10.6 |
| LYM 6 | 1173 5.1 | B | 0.42 | 5.60E -02 | 12.3 | LYM 14 | 1205 1.1 | Q | 30.42 5 | 2.85E -03 | 8 |
| LYM 8 | 1198 3.1 | B | 0.47 | 6.47E -02 | 25.8 | LYM 43 | 1179 1.2 | Q | 31.01 5 | 3.17E -03 | 10.1 |
| LYM 6 | 1173 4.3 | B | 0.418 | 6.56E -02 | 11.9 | LYM 43 | 1179 3.2 | Q | 31.92 | 4.77E -03 | 13.3 |
| LYM 30 | 1191 2.7 | B | 0.512 | 6.75E -02 | 36.9 | LYM 26 | 1182 4.3 | Q | 30.13 | 5.77E -03 | 6.9 |
| LYM 26 | 1182 4.3 | B | 0.483 | 6.80E -02 | 29.2 | LYM 51 | 1189 3.4 | Q | 30.12 | 7.02E -03 | 6.9 |
| LYM 8 | 1198 2.4 | B | 0.475 | 7.82E -02 | 27.1 | LYM 10 | 1174 1.4 | Q | 30.01 | 8.17E -03 | 6.5 |
| LYM 12 | 1187 1.1 | B | 0.457 | 8.07E -02 | 22.2 | LYM 22 | 1176 1.3 | Q | 30.89 5 | 8.20E -03 | 9.6 |
| LYM 67 | 1178 2.6 | B | 0.49 | 8.15E -02 | 31.1 | LYM 15 | 1161 1.3 | Q | 30.05 | 8.49E -03 | 6.6 |
| LYM 22 | 1176 4.1 | B | 0.474 | 8.22E -02 | 26.8 | LYM 24 | 1206 3.3 | Q | 30.21 5 | 1.03E -02 | 7.2 |
| LYM 51 | 1189 3.4 | B | 0.476 | 8.62E -02 | 27.4 | LYM 43 | 1179 2.2 | Q | 29.98 5 | 1.19E -02 | 6.4 |
| LYM 21 | 1167 4.5 | B | 0.516 | 8.83E -02 | 38 | LYM 62 | 1202 3.2 | Q | 29.85 5 | 1.23E -02 | 5.9 |
| LYM 7 | 1159 4.3 | B | 0.464 | 8.85E -02 | 24 | LYM 7 | 1159 1.5 | Q | 29.84 5 | 1.27E -02 | 5.9 |
| LYM 24 | 1206 4.1 | B | 0.491 | 8.92E -02 | 31.2 | LYM 66 | 1195 5.2 | Q | 30.36 5 | 1.58E -02 | 7.7 |
| LYM 2 | 1169 1.2 | B | 0.454 | 9.14E -02 | 21.4 | LYM 24 | 1206 1.2 | Q | 29.76 | 1.61E -02 | 5.6 |
| LYM 13 | 1177 2.2 | B | 0.474 | 9.21E -02 | 26.9 | LYM 69 | 1185 2.2 | Q | 29.82 | 1.91E -02 | 5.8 |
| LYM 68 | 1194 2.3 | B | 0.426 | 9.22E -02 | 13.9 | LYM 62 | 1202 3.7 | Q | 29.69 | 2.03E -02 | 5.3 |
| LYM 44 | 1188 4.3 | B | 0.414 | 9.80E -02 | 10.6 | LYM 17 | 1168 4.4 | Q | 29.68 | 2.16E -02 | 5.3 |
| CON TRO L | - | B | 0.374 | - | 0 | LYM 7 | 1159 2.1 | Q | 31.66 | 3.45E -02 | 12.3 |
| LYM 95 | 1212 1.3 | C | 0.73 | 2.06E -04 | 46.2 | LYM 31 | 1192 3.1 | Q | 29.53 | 3.69E -02 | 4.8 |
| LYM 10 | 1174 1.2 | C | 0.655 | 3.02E -03 | 31.2 | LYM 53 | 1184 1.2 | Q | 30.06 | 3.89E -02 | 6.7 |
| LYM 21 | 1167 1.2 | C | 0.619 | 4.67E -03 | 23.9 | LYM 57 | 1201 2.6 | Q | 29.38 | 4.78E -02 | 4.2 |
| LYM 62 | 1202 4.2 | C | 0.626 | 7.02E -03 | 25.3 | LYM 66 | 1195 4.4 | Q | 31.52 | 5.68E -02 | 11.8 |
| LYM 66 | 1195 3.1 | C | 0.602 | 1.02E -02 | 20.5 | LYM 14 | 1205 2.4 | Q | 29.42 | 5.94E -02 | 4.4 |
| LYM 10 | 1174 4.1 | C | 0.602 | 1.02E -02 | 20.5 | LYM 51 | 1189 4.2 | Q | 30.18 5 | 6.04E -02 | 7.1 |
| LYM 4 | 1170 6.5 | C | 0.575 | 3.74E -02 | 15.2 | LYM 68 | 1194 1.3 | Q | 30.51 5 | 6.20E -02 | 8.3 |
| LYM 44 | 1188 2.1 | C | 0.569 | 5.06E -02 | 13.9 | LYM 8 | 1198 4.1 | Q | 29.39 5 | 6.25E -02 | 4.3 |
| LYM 66 | 1195 5.2 | C | 0.586 | 5.09E -02 | 17.4 | LYM 30 | 1191 2.6 | Q | 31.81 5 | 6.57E -02 | 12.9 |
| LYM 82 | 1220 1.1 | C | 0.567 | 6.28E -02 | 13.6 | LYM 13 | 1177 2.1 | Q | 29.53 | 8.65E -02 | 4.8 |
| LYM 68 | 1194 1.4 | C | 0.564 | 6.49E -02 | 13.1 | LYM 24 | 1206 4.1 | Q | 30.50 5 | 9.05E -02 | 8.2 |
| LYM 95 | 1212 1.2 | C | 0.557 | 9.41E -02 | 11.6 | LYM 44 | 1188 5.4 | Q | 29.57 | 9.26E -02 | 4.9 |
| CON TRO L | - | C | 0.499 | - | 0 | LYM | 1160 1.1 | Q | 29.68 5 | 9.43E -02 | 5.3 |
| LYM 31 | 1192 3.1 | D | 9.125 | 4.78E -04 | 8.8 | CON TRO L | - | Q | 28.18 3 | - | 0 |
| LYM 26 | 1182 4.6 | D | 8.938 | 4.23E -03 | 6.5 | LYM 175 | 1265 1.6 | A | 1.514 | 2.92E -03 | 50.2 |
| LYM 4 | 1170 5.2 | D | 8.938 | 4.23E -03 | 6.5 | LYM 256 | 1332 1.2 | A | 1.169 | 5.61E -02 | 16 |
| LYM 9 | 1163 2.1 | D | 8.938 | 4.23E -03 | 6.5 | LYM 147 | 1258 1.4 | A | 1.154 | 6.79E -02 | 14.5 |
| LYM 44 | 1188 2.1 | D | 8.875 | 4.88E -03 | 5.8 | CON TRO L | - | A | 1.008 | - | 0 |
| LYM 8 | 1198 4.1 | D | 8.875 | 4.88E -03 | 5.8 | LYM 207 | 1325 1.4 | B | 0.356 | 7.41E -02 | 8.2 |
| LYM 4 | 1170 2.1 | D | 9.5 | 5.72E -03 | 13.3 | LYM 73 | 1262 4.1 | B | 0.362 | 9.94E -02 | 9.9 |
| LYM 10 | 1174 4.5 | D | 8.813 | 1.53E -02 | 5.1 | CON TRO L | - | B | 0.329 | - | 0 |
| LYM 24 | 1206 1.1 | D | 8.813 | 1.53E -02 | 5.1 | LYM 206 | 1260 1.3 | C | 0.313 | 3.92E -03 | 31.8 |
| LYM 66 | 1195 2.1 | D | 8.75 | 1.97E -02 | 4.3 | LYM 207 | 1325 1.4 | C | 0.281 | 2.47E -02 | 18.2 |
| LYM 69 | 1185 1.2 | D | 8.75 | 1.97E -02 | 4.3 | LYM 241 | 1327 1.2 | C | 0.291 | 3.27E -02 | 22.5 |
| LYM 95 | 1212 1.3 | D | 8.75 | 1.97E -02 | 4.3 | LYM 159 | 1335 4.5 | C | 0.272 | 4.39E -02 | 14.8 |
| LYM 53 | 1184 2.4 | D | 9 | 3.32E -02 | 7.3 | LYM 91 | 1328 3.1 | C | 0.268 | 7.20E -02 | 13 |
| LYM 16 | 1162 3.2 | D | 8.875 | 6.05E -02 | 5.8 | CON TRO L | - | C | 0.237 | - | 0 |
| LYM 37 | 1180 3.2 | D | 8.875 | 6.05E -02 | 5.8 | LYM 175 | 1265 3.3 | D | 9 | 1.81E -02 | 3 |
| LYM 57 | 1201 2.6 | D | 8.875 | 6.05E -02 | 5.8 | LYM 206 | 1260 3.1 | D | 9 | 1.81E -02 | 3 |
| LYM 15 | 1161 4.4 | D | 8.688 | 6.06E -02 | 3.6 | LYM 147 | 1258 1.4 | D | 9.25 | 7.10E -02 | 5.9 |
| LYM 43 | 1179 1.5 | D | 8.688 | 6.06E -02 | 3.6 | LYM 147 | 1258 4.4 | D | 9.25 | 7.10E -02 | 5.9 |
| LYM 44 | 1188 5.3 | D | 8.625 | 8.97E -02 | 2.8 | CON TRO L | - | D | 8.734 | - | 0 |
| LYM 53 | 1184 4.2 | D | 8.625 | 8.97E -02 | 2.8 | LYM 207 | 1325 1.4 | E | 0.494 | 2.25E -03 | 29 |
| LYM 7 | 1159 4.3 | D | 8.625 | 8.97E -02 | 2.8 | LYM 206 | 1260 1.3 | E | 0.49 | 3.51E -03 | 27.9 |
| CON TRO L | - | D | 8.388 | - | 0 | LYM 91 | 1328 3.1 | E | 0.451 | 2.44E -02 | 17.8 |
| LYM 95 | 1212 1.3 | E | 0.871 | 6.40E -05 | 20.5 | CON TRO L | - | E | 0.383 | - | 0 |
| LYM 10 | 1174 1.2 | E | 0.854 | 1.68B -04 | 18 | LYM 175 | 1265 1.6 | F | 8.457 | 9.98E -03 | 14.9 |
| LYM 51 | 1189 3.4 | E | 0.814 | 1.64E -03 | 12.5 | LYM 241 | 1327 1.2 | F | 7.999 | 8.84E -02 | 8.7 |
| LYM 26 | 1182 4.1 | E | 0.864 | 3.71E -03 | 19.4 | CON TRO L | - | F | 7.36 | - | 0 |
| LYM 16 | 1162 3.2 | E | 0.793 | 8.69E -03 | 9.6 | LYM 241 | 1327 1.2 | G | 13.92 4 | 3.22E -02 | 20.3 |
| LYM 12 | 1187 1.1 | E | 0.79 | 8.71E -03 | 9.2 | LYM 256 | 1332 2.3 | G | 13.01 2 | 5.32E -02 | 12.5 |
| LYM 41 | 1183 4.3 | E | 0.788 | 9.62E -03 | 9 | LYM 91 | 1328 3.1 | G | 12.99 6 | 5.81E -02 | 12.3 |
| LYM 44 | 1188 5.4 | E | 0.791 | 2.21E -02 | 9.3 | CON TRO L | - | G | 11.57 | - | 0 |
| LYM 17 | 1168 1.4 | E | 0.788 | 2.38E -02 | 8.9 | LYM 206 | 1260 1.3 | H | 0.038 | 9.68E -03 | 39.1 |
| LYM 21 | 1167 3.1 | E | 0.822 | 2.48E -02 | 13.7 | LYM 206 | 1260 2.1 | H | 0.037 | 3.48E -02 | 35.7 |
| LYM 20 | 1171 1.1 | E | 0.786 | 4.67E -02 | 8.7 | LYM 147 | 1258 4.4 | H | 0.036 | 4.44E -02 | 30.6 |
| LYM 4 | 1170 6.5 | E | 0.818 | 5.29E -02 | 13.1 | LYM 147 | 1258 3.3 | H | 0.035 | 4.93E -02 | 29.1 |
| LYM 68 | 1194 2.3 | E | 0.786 | 6.52E -02 | 8.7 | LYM 203 | 1266 4.1 | H | 0.035 | 7.71E -02 | 27.4 |
| LYM 53 | 1184 1.1 | E | 0.823 | 9.25E -02 | 13.8 | LYM 159 | 1335 4.6 | H | 0.035 | 8.34E -02 | 28.1 |
| CON TRO L | - | E | 0.723 | - | 0 | LYM 241 | 1327 1.2 | H | 0.034 | 9.00E -02 | 24.6 |
| LYM 34 | 1190 2.2 | F | 13.36 3 | 5.21E -03 | 33.8 | CON TRO L | - | H | 0.027 | - | 0 |
| LYM 17 | 1168 1.4 | F | 11.76 4 | 3.07E -02 | 17.8 | LYM 206 | 1260 1.3 | I | 1.904 | 2.91E -02 | 34.1 |
| LYM 37 | 1180 2.2 | F | 11.83 9 | 5.95E -02 | 18.5 | LYM 147 | 1258 3.3 | I | 1.834 | 5.96E -02 | 29.1 |
| CON TRO L | - | F | 9.989 | - | 0 | LYM 147 | 1258 4.4 | I | 1.823 | 6.86E -02 | 28.3 |
| LYM 21 | 1167 1.2 | G | 29.31 8 | 2.73E -03 | 29 | CON TRO L | - | I | 1.421 | - | 0 |
| LYM 95 | 1212 1.3 | G | 35.39 8 | 5.12E -03 | 55.7 | LYM 206 | 1260 1.3 | J | 0.238 | 2.91E -02 | 34.1 |
| LYM 44 | 1188 2.1 | G | 28.41 9 | 6.04E -03 | 25 | LYM 147 | 1258 3.3 | J | 0.229 | 5.96E -02 | 29.1 |
| LYM 10 | 1174 1.2 | G | 31.63 6 | 7.14E -03 | 39.1 | LYM 147 | 1258 4.4 | J | 0.228 | 6.86E -02 | 28.3 |
| LYM 66 | 1195 3.1 | G | 28.02 | 8.78E -03 | 23.2 | CON TRO L | - | J | 0.178 | - | 0 |
| LYM 4 | 1170 6.5 | G | 28.51 5 | 1.11E -02 | 25.4 | LYM 206 | 1260 1.3 | K | 0.227 | 3.54E -02 | 26.3 |
| LYM 62 | 1202 4.2 | G | 30.01 7 | 1.16E -02 | 32 | LYM 203 | 1266 4.1 | K | 0.225 | 6.18E -02 | 25.1 |
| LYM 66 | 1195 5.2 | G | 27.27 7 | 1.76E -02 | 20 | LYM 159 | 1335 4.6 | K | 0.22 | 8.46E -02 | 22.6 |
| LYM 68 | 1194 1.4 | G | 26.64 6 | 3.40E -02 | 17.2 | LYM 206 | 1260 2.1 | K | 0.223 | 8.54E -02 | 24 |
| LYM 82 | 1220 1.1 | G | 26.54 8 | 4.02E -02 | 16.8 | LYM 241 | 1327 1.2 | K | 0.217 | 9.49E -02 | 21.1 |
| LYM 9 | 1163 2.1 | G | 26.84 5 | 4.65E -02 | 18.1 | CON TRO L | - | K | 0.179 | - | 0 |
| LYM 53 | 1184 1.1 | G | 28.48 2 | 7.52E -02 | 25.3 | LYM 241 | 1327 1.2 | L | 1.74 | 3.22E -02 | 20.3 |
| LYM 95 | 1212 1.2 | G | 26.98 6 | 9.98E -02 | 18.7 | LYM 256 | 1332 2.3 | L | 1.627 | 5.32E -02 | 12.5 |
| CON TRO L | - | G | 22.73 5 | - | 0 | LYM 91 | 1328 3.1 | L | 1.625 | 5.81E -02 | 12.3 |
| LYM 95 | 1212 1.3 | H | 0.094 | 1.34E -02 | 47.3 | CON TRO L | - | L | 1.446 | - | 0 |
| LYM 10 | 1174 1.2 | H | 0.084 | 7.76E -02 | 31.9 | LYM 206 | 1260 1.3 | M | 2.453 | 2.09E -03 | 18 |
| CON TRO L | - | H | 0.064 | - | 0 | LYM 241 | 1327 1.2 | M | 2.397 | 1.28E -02 | 15.3 |
| LYM 95 | 1212 1.3 | I | 4.691 | 6.13E -03 | 54.8 | LYM 207 | 1325 1.4 | M | 2.296 | 3.04E -02 | 10.4 |
| 10 | 1174 1.2 | I | 4.228 | 3.51E -02 | 39.5 | LYM 147 | 1258 4.4 | M | 2.386 | 8.91E -02 | 14.8 |
| LYM 62 | 1202 4.2 | I | 3.957 | 8.88E -02 | 30.6 | LYM 256 | 1332 2.3 | M | 2.239 | 9.17E -02 | 7.7 |
| LYM 10 | 1174 4.5 | I | 3.988 | 9.32E -02 | 31.6 | CON TRO L | - | M | 2.079 | - | 0 |
| CON TRO L | - | I | 3.03 | - | 0 | LYM 236 | 1259 4.3 | 0 | 0.026 | 6.00E -02 | 15.3 |
| LYM 95 | 1212 1.3 | J | 0.586 | 8.07E -03 | 52.2 | LYM 147 | 1258 4.4 | O | 0.028 | 6.46E -02 | 22.5 |
| LYM 10 | 1174 1.2 | J | 0.529 | 4.45E -02 | 37.2 | CON TRO L | - | 0 | 0.023 | - | 0 |
| CON TRO L | - | J | 0.385 | - | 0 | LYM 157 | 1334 1.3 | P | 93.97 7 | 3.21E -02 | 2.1 |
| LYM 95 | 1212 1.3 | K | 0.411 | 5.44E -03 | 28.5 | LYM 256 | 1332 4.2 | P | 93.95 4 | 3.46E -02 | 2.1 |
| LYM 10 | 1174 1.2 | K | 0.392 | 2.01E -02 | 22.4 | LYM 159 | 1335 4.5 | P | 93.98 3 | 7.24E -02 | 2.1 |
| LYM 10 | 1174 4.5 | K | 0.383 | 4.42E -02 | 19.7 | LYM 223 | 1267 1.2 | P | 93.58 7 | 8.19E -02 | 1.7 |
| LYM 10 | 1174 4.1 | K | 0.378 | 5.54E -02 | 18.2 | LYM 157 | 1334 1.7 | P | 93.61 6 | 9.88E -02 | 1.7 |
| LYM 26 | 1182 4.1 | K | 0.377 | 6.36E -02 | 17.6 | CON TRO L | - | P | 92.01 8 | - | 0 |
| LYM 21 | 1167 1.2 | K | 0.377 | 6.69E -02 | 17.8 | LYM 250 | 1261 3.4 | Q | 30.53 5 | 3.32E -03 | 7.9 |
| LYM 68 | 1194 1.4 | K | 0.373 | 7.72E -02 | 16.6 | LYM 206 | 1260 1.2 | Q | 29.68 5 | 1.79E -02 | 4.9 |
| CON TRO L | - | K | 0.32 | - | 0 | LYM 147 | 1258 2.3 | Q | 29.84 | 3.32E -02 | 5.5 |
| LYM 21 | 1167 1.2 | L | 3.665 | 3.75E -03 | 26.8 | LYM 203 | 1266 2.3 | Q | 31.71 5 | 3.46E -02 | 12.1 |
| LYM 95 | 1212 1.3 | L | 4.425 | 5.70E-03 | 53.1 | LYM 157 | 1334 | Q | 29.42 | 4.35E -02 | 4 |
| LYM 10 | 1174 1.2 | L | 3.954 | 8.47E -03 | 36.9 | LYM 206 | 1260 3.2 | Q | 29.34 | 6.50E -02 | 3.7 |
| LYM 44 | 1188 2.1 | L | 3.552 | 8.51E -03 | 22.9 | LYM 91 | 1328 3.1 | Q | 29.24 5 | 7.37E -02 | 3.3 |
| LYM 66 | 1195 3.1 | L | 3.502 | 1.25E -02 | 21.2 | CON TRO L | - | Q | 28.29 8 | - | 0 |
| LYM 10 | 1174 4.1 | L | 3.498 | 1.28E -02 | 21.1 | LYM 206 | 1260 3.1 | R | 0.682 | 1.24E -02 | 27.2 |
| LYM 62 | 1202 4.2 | L | 3.752 | 1.43E -02 | 29.9 | CON TRO L | - | R | 0.536 | - | 0 |
| LYM 4 | 1170 6.5 | L | 3.564 | 1.51E -02 | 23.4 | LYM 113 | 1244 3.1 | B | 0.532 | 9.57E -03 | 25.9 |
| LYM 66 | 1195 5.2 | L | 3.41 | 2.53E -02 | 18 | LYM 267 | 1360 4.5 | B | 0.48 | 2.91E -02 | 13.6 |
| LYM 68 | 1194 1.4 | L | 3.331 | 4.96E -02 | 15.3 | LYM 285 | 1273 4.7 | B | 0.477 | 3.69E -02 | 12.8 |
| LYM 82 | 1220 1.1 | L | 3.319 | 5.85E -02 | 14.8 | LYM 113 | 1244 4.4 | B | 0.464 | 9.15E -02 | 9.7 |
| LYM 9 | 1163 2.1 | L | 3.356 | 6.43E -02 | 16.1 | CON TRO L | - | B | 0.423 | - | 0 |
| LYM 53 | 1184 1.1 | L | 3.56 | 8.80E -02 | 23.2 | CON TRO L | - | E | 0.515 | - | 0 |
| CON TRO L | - | L | 2.889 | - | 0 | LYM 255 | 1308 1.5 | F | 8.406 | 9.80E -05 | 18.5 |
| LYM 95 | 1212 1.3 | M | 3.993 | 1.50E -05 | 26.6 | LYM 116 | 1320 4.6 | F | 7.893 | 5.11E -04 | 11.2 |
| LYM 10 | 1174 1.2 | M | 3.71 | 3.24E -04 | 17.6 | LYM 287 | 1277 1.6 | F | 7.885 | 5.55E -04 | 11.1 |
| LYM 62 | 1202 4.2 | M | 3.594 | 1.18E -03 | 14 | LYM 284 | 1288 4.6 | F | 7.834 | 7.75E -04 | 10.4 |
| LYM 10 | 1174 4.1 | M | 3.58 | 1.97E -03 | 13.5 | LYM 239 | 1304 1.7 | F | 7.666 | 3.45E -03 | 8 |
| LYM 82 | 1220 1.1 | M | 3.499 | 8.49E -03 | 10.9 | LYM 113 | 1244 4.5 | F | 8.133 | 6.77E -03 | 14.6 |
| LYM 44 | 1188 5.4 | M | 3.407 | 2.47E -02 | 8 | LYM 287 | 1277 1.7 | F | 7.628 | 7.48E -03 | 7.5 |
| LYM 9 | 1163 2.1 | M | 3.475 | 2.70E -02 | 10.2 | LYM 110 | 1292 3.5 | F | 8.115 | 1.19E -02 | 14.4 |
| LYM 20 | 1171 1.1 | M | 3.387 | 2.94E -02 | 7.4 | LYM 52 | 1289 5.7 | F | 7.749 | 1.33E -02 | 9.2 |
| LYM 66 | 1195 3.1 | M | 3.494 | 3.07E -02 | 10.8 | LYM 238 | 1276 4.8 | F | 8.587 | 4.56E -02 | 21 |
| LYM 21 | 1167 3.1 | M | 3.421 | 3.23E -02 | 8.5 | LYM 112 | 1337 4.4 | F | 7.715 | 6.97E -02 | 8.7 |
| LYM 51 | 1189 3.4 | M | 3.481 | 3.65E -02 | 10.4 | LYM 56 | 1311 2.5 | F | 7.798 | 9.00E -02 | 9.9 |
| LYM 43 | 1179 2.2 | M | 3.43 | 5.63E -02 | 8.7 | CON TRO L | - | F | 7.096 | - | 0 |
| LYM 26 | 1182 4.1 | M | 3.537 | 6.22E -02 | 12.1 | LYM 56 | 1311 1.7 | N | 0.554 | 7.80E -02 | 10.8 |
| LYM 17 | 1168 2.3 | M | 3.353 | 6.60E -02 | 6.3 | CON TRO L | - | N | 0.5 | - | 0 |
| LYM 16 | 1162 3.2 | M | 3.512 | 6.70E -02 | 11.3 | LYM 255 | 1308 2.9 | O | 0.025 | 3.34E -02 | 7.8 |
| LYM 66 | 1195 5.2 | M | 3.419 | 8.25E -02 | 8.4 | LYM 141 | 1240 2.4 | O | 0.027 | 3.92E -02 | 19.5 |
| LYM 17 | 1168 1.4 | M | 3.326 | 8.35E -02 | 5.5 | LYM 113 | 1244 2.1 | O | 0.025 | 4.02E -02 | 7.8 |
| LYM 41 | 1183 4.3 | M | 3.318 | 9.59E -02 | 5.2 | CON TRO L | - | O | 0.023 | - | 0 |
| LYM 21 | 1167 1.2 | M | 3.649 | 9.83E -02 | 15.7 | LYM 181 | 1357 2.2 | P | 95.24 7 | 4.17E -02 | 1.9 |
| CON TRO L | - | M | 3.154 | - | 0 | LYM 239 | 1304 4.9 | P | 95.31 7 | 9.21E -02 | 2 |
| LYM 66 | 1195 4.4 | N | 0.311 | 2.80E -03 | 25.1 | LYM 232 | 1302 3.6 | P | 93.97 6 | 9.43E -02 | 0.6 |
| LYM 31 | 1192 3.1 | N | 0.302 | 5.66E -03 | 21.3 | LYM 156 | 1296 3.5 | P | 94.45 2 | 9.56E -02 | 1.1 |
| LYM 8 | 1198 2.4 | N | 0.301 | 5.78E -03 | 21.2 | CON TRO L | - | P | 93.44 1 | - | 0 |
| LYM 17 | 1168 3.1 | N | 0.296 | 1.02E -02 | 18.9 | LYM 255 | 1308 2.9 | Q | 34.41 5 | 9.06E -03 | 8.2 |
| LYM 7 | 1159 4.3 | N | 0.284 | 4.39E -02 | 14 | LYM 112 | 1337 2.3 | Q | 34.23 | 1.03E -02 | 7.6 |
| LYM 57 | 1201 2.6 | N | 0.285 | 5.22E -02 | 14.6 | LYM 196 | 1361 3.1 | Q | 33.73 5 | 3.50E -02 | 6.1 |
| LYM 17 | 1168 4.5 | N | 0.277 | 8.28E -02 | 11.1 | LYM 121 | 1321 1.8 | Q | 33.46 | 7.26E -02 | 5.2 |
| LYM 13 | 1177 2.2 | N | 0.311 | 9.17E -02 | 25.1 | LYM 287 | 1277 1.7 | Q | 33.23 | 7.99E -02 | 4.5 |
| CON TRO L | - | N | 0.249 | - | 0 | LYM 56 | 1311 1.7 | Q | 33.21 | 8.34E -02 | 4.4 |
| LYM 12 | 1187 2.1 | O | 0.024 | 1.60E -05 | 19.6 | LYM 156 | 1296 3.3 | Q | 33.4 | 9.09E -02 | 5 |
| LYM 22 | 1176 2.1 | O | 0.022 | 4.50E -05 | 13.3 | LYM 238 | 1276 1.6 | Q | 34.2 | 9.81E -02 | 7.5 |
| LYM 43 | 1179 2.2 | O | 0.026 | 4.80E -05 | 33.1 | CON TRO L | - | Q | 31.80 9 | - | 0 |
| LYM 31 | 1192 3.4 | O | 0.022 | 1.05E -04 | 12.1 | | | | | | |
| LYM 43 | 1179 1.2 | O | 0.022 | 1.77E -04 | 11.2 | | | | | | |
| LYM 53 | 1184 1.1 | O | 0.022 | 3.43E -04 | 10 | | | | | | |
| LYM 95 | 1212 1.3 | O | 0.026 | 3.71E -04 | 33.1 | | | | | | |
| LYM 95 | 1212 1.2 | O | 0.023 | 3.96E -04 | 14.5 | | | | | | |
| LYM 10 | 1174 4.1 | O | 0.023 | 2.55E -03 | 15.8 | | | | | | |
| LYM 4 | 1170 2.1 | O | 0.021 | 3.43E -03 | 8.2 | | | | | | |
| LYM 82 | 1220 4.6 | O | 0.021 | 4.85E -03 | 6.4 | | | | | | |
| LYM 82 | 1220 4.2 | O | 0.021 | 6.10E -03 | 6.4 | | | | | | |
| LYM 34 | 1190 4.3 | O | 0.021 | 6.18E -03 | 6.7 | | | | | | |
| LYM 14 | 1205 4.2 | O | 0.021 | 1.02E -02 | 5.5 | | | | | | |
| LYM 66 | 1195 4.4 | O | 0.023 | 3.15E -02 | 14.6 | | | | | | |
| LYM 6 | 1173 4.3 | O | 0.024 | 6.98E -02 | 22.1 | | | | | | |
| LYM 16 | 1162 3.2 | O | 0.023 | 7.55E -02 | 15.8 | | | | | | |
| LYM 82 | 1220 1.1 | O | 0.024 | 9.20E -02 | 21.9 | | | | | | |
| CON TRO L | - | O | 0.02 | - | 0 | | | | | | |

Table 30. Results of the greenhouse experiments. Provided are the measured values of each tested parameter [parameters (ID.) A-P according to the parameters described in Table 29 above] in plants expressing the indicated polynucleotides. "Ev" = event; "P" = P-value; "Mean " = the average of measured parameter across replicates. % incr. vs. cont. = percentage of increase versus control (as compared to control).

**Table 31**

| | |
|---|---|
| ***Measured parameters at the greenhouse till bolting assay* (*T2 experiment) for transformed agriculture improving trait genes*** | |
| ***Tested Parameters*** | ***ID*** |
| Blade Relative Area TP4 | A |
| Dry Weight (gr) | B |
| Fresh Weight (gr) | C |
| Leaf Blade Area TP4 (cm²) | D |
| Leaf Number TP4 | E |
| Leaf Petiole Length TP4 (cm) | F |
| Petiole Relative Area TP4 | G |
| Plot Coverage TP4 (cm²) | H |
| RGR Of Leaf Blade Area | J |
| RGR Of Leaf Number | K |

| ***Tested Parameters*** | ***ID*** |
|---|---|
| RGR Of Plot Coverage | L |
| RGR Of Rosette Area | M |
| RGR Of Rosette Diameter | N |
| Rosette Area TP4 (cm²) | O |
| Rosette Diameter TP4 (cm) | P |

Table 31: Provided are the identification (ID) letters of each of the Tested Parameters. TP4-time Point 4; RGR - Relative Growth Rate.

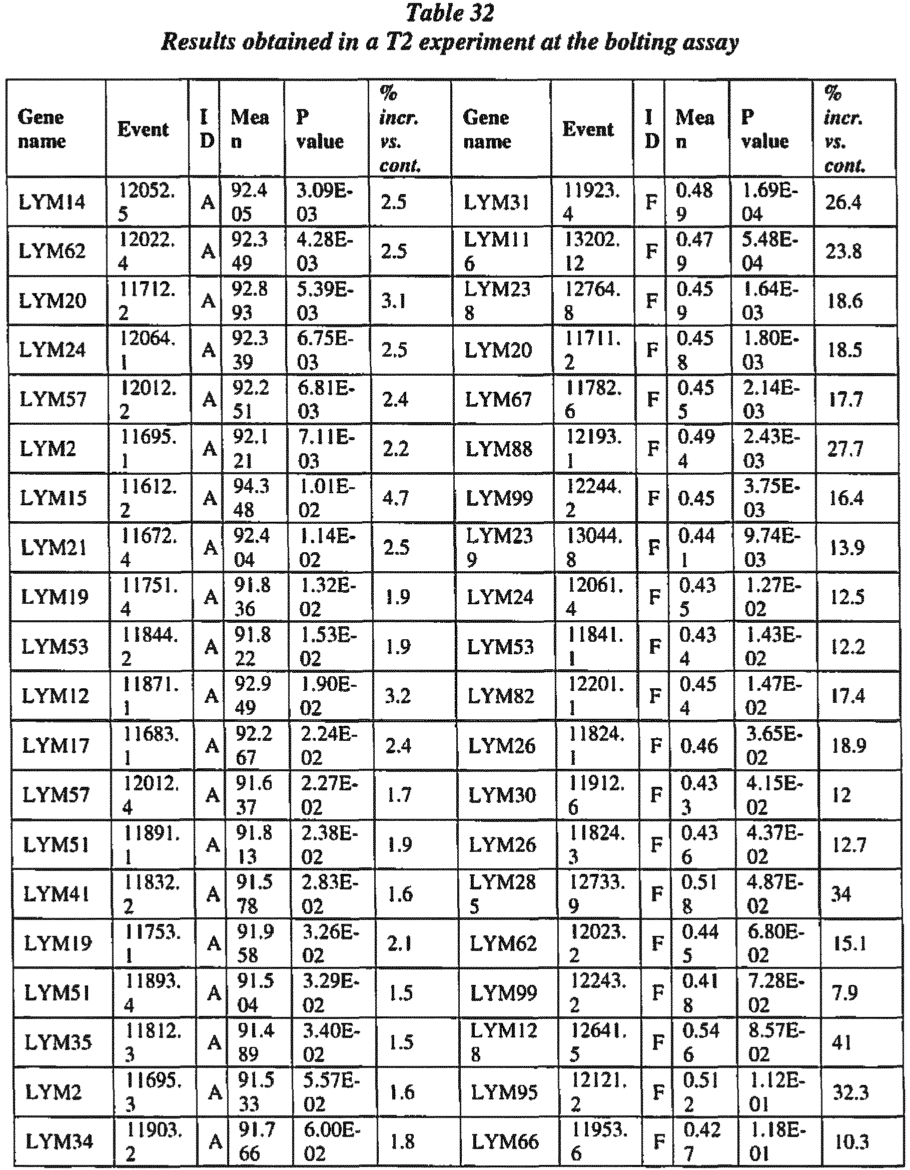

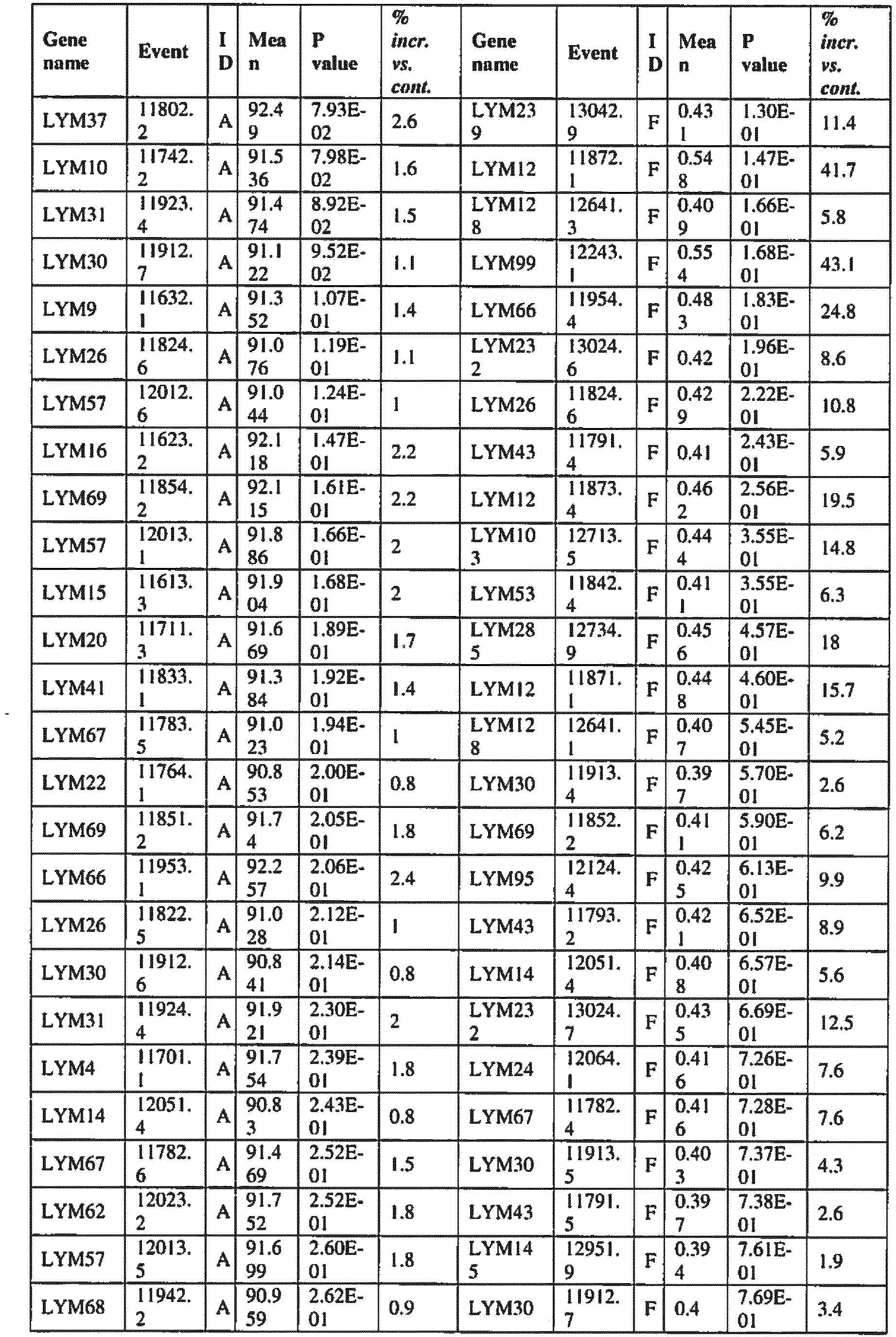

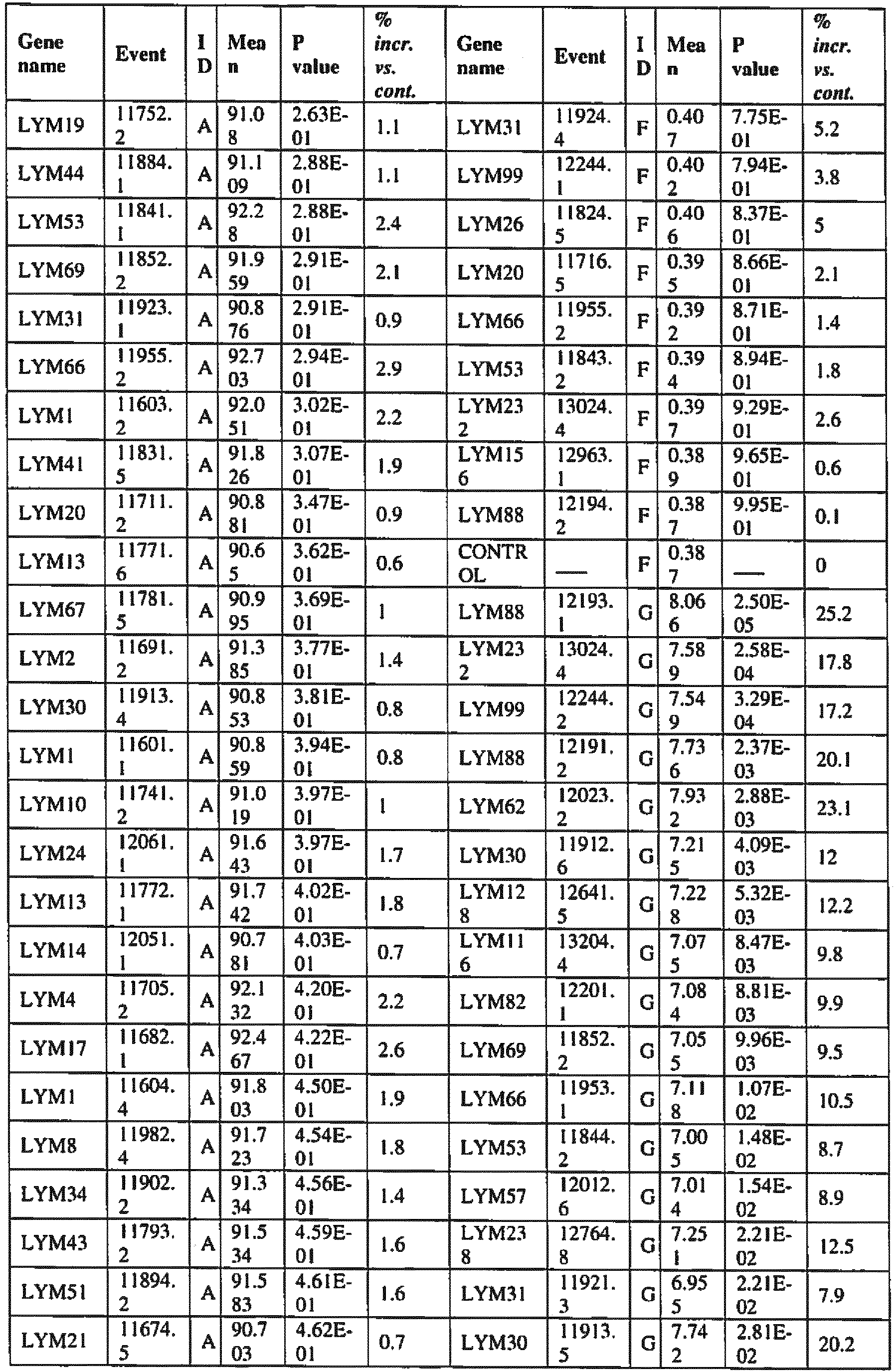

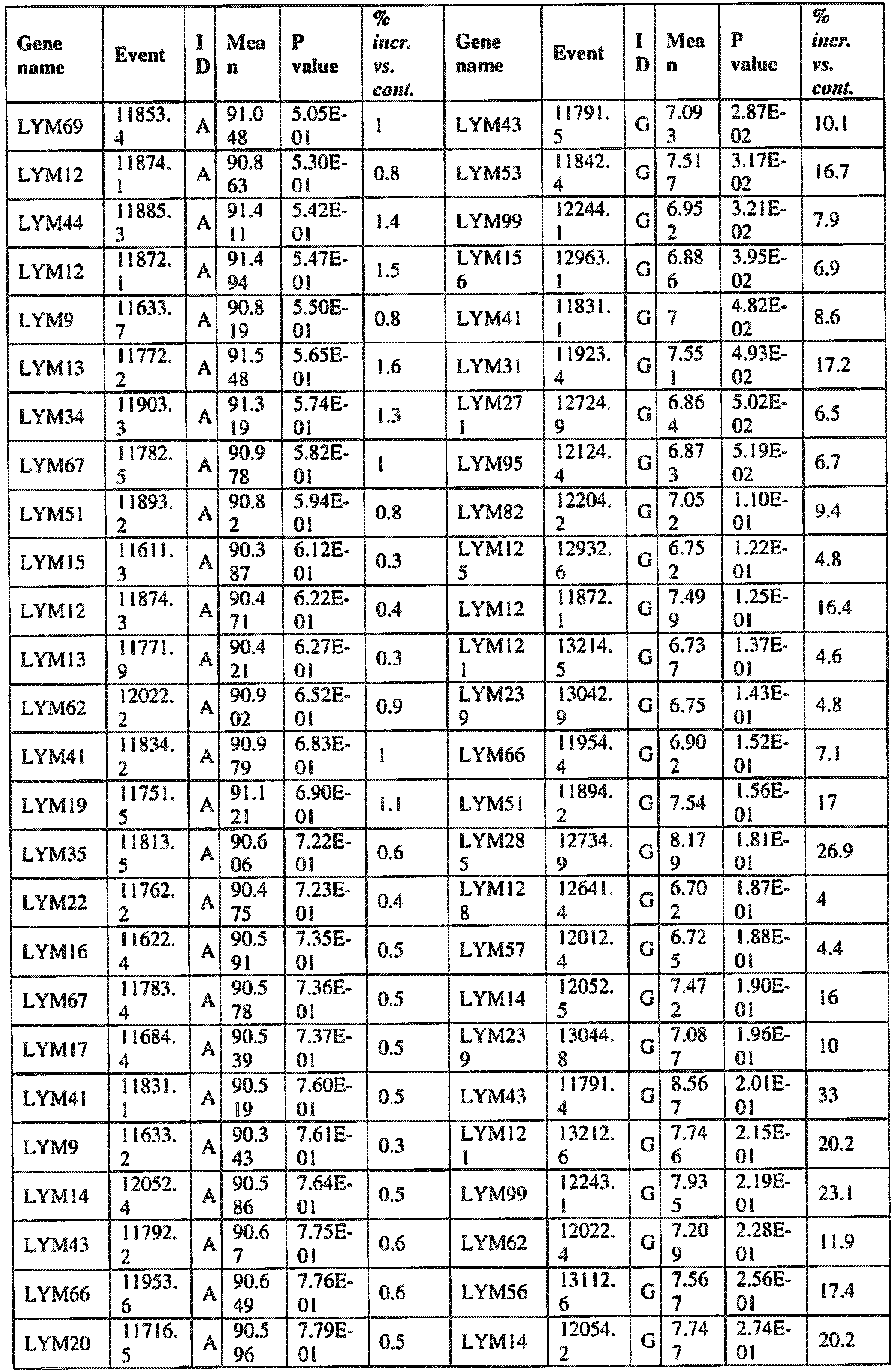

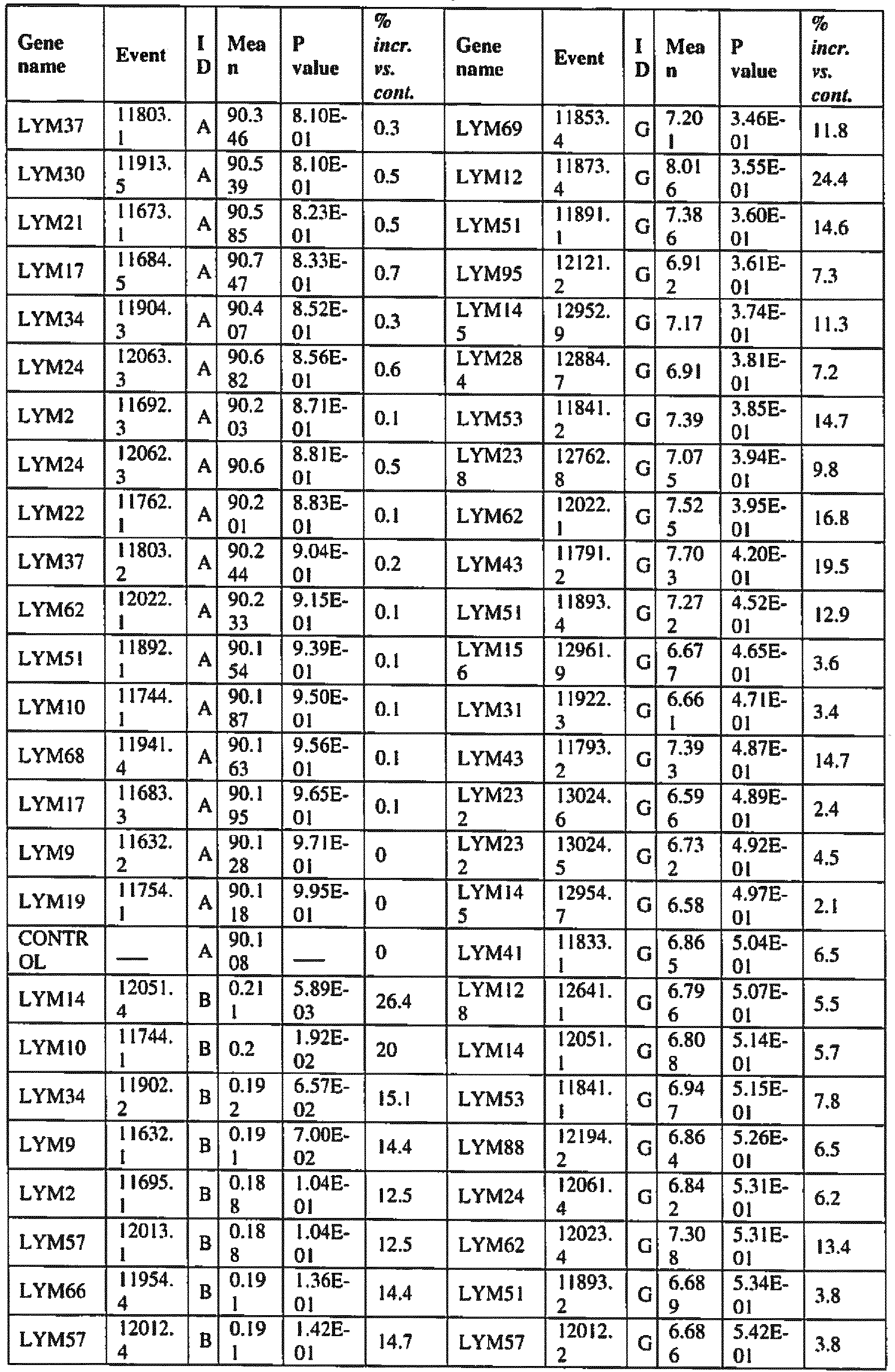

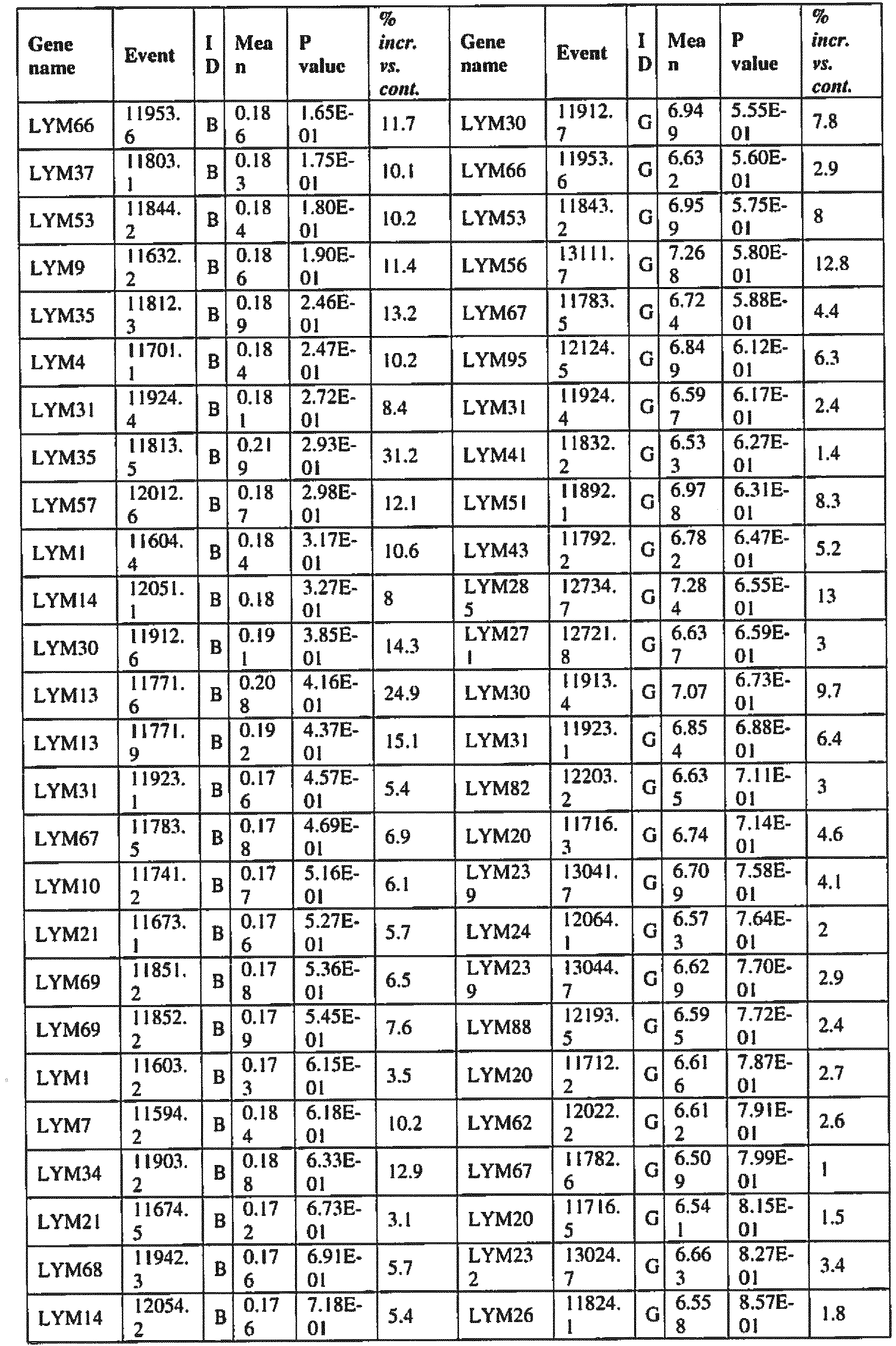

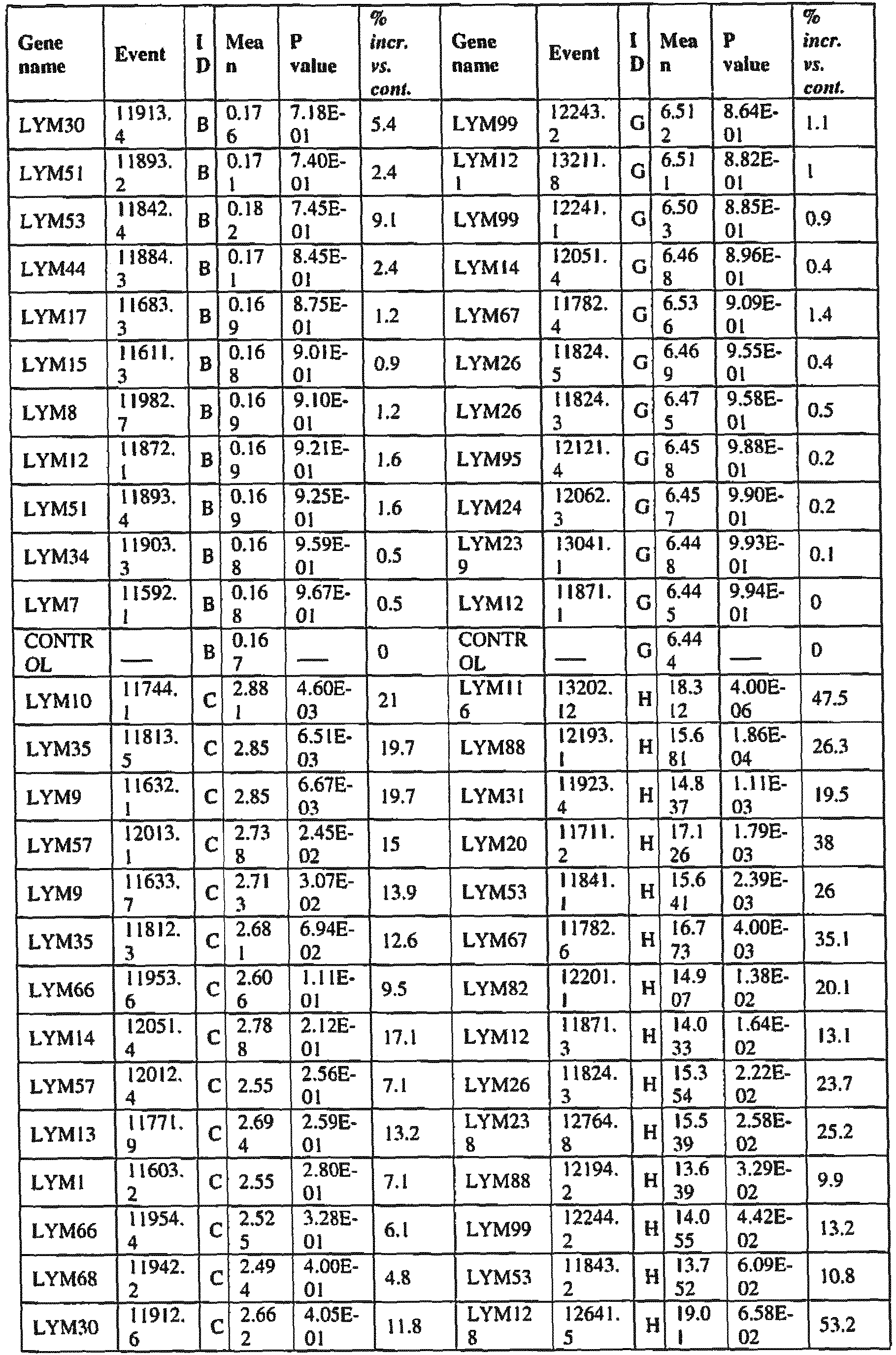

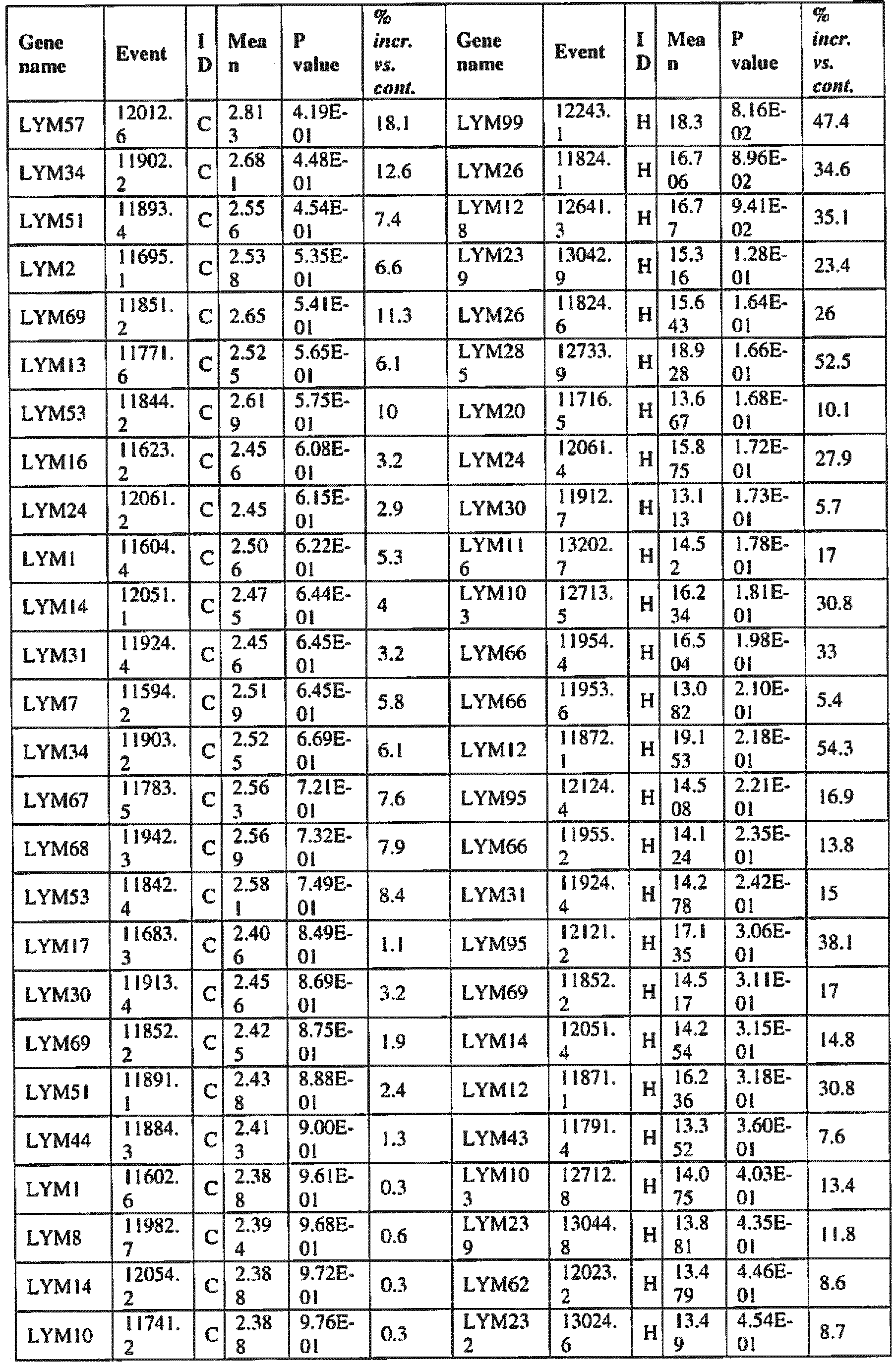

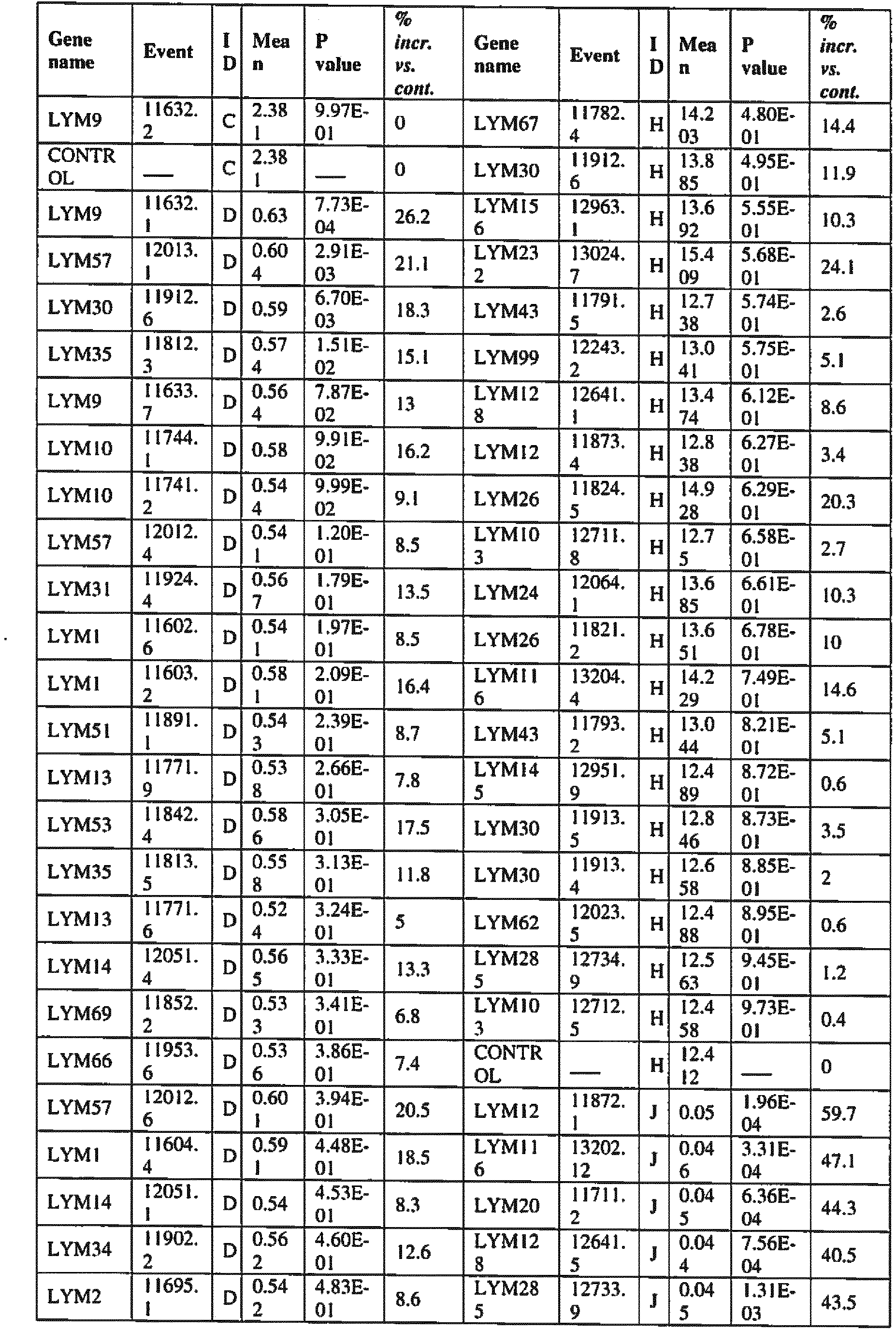

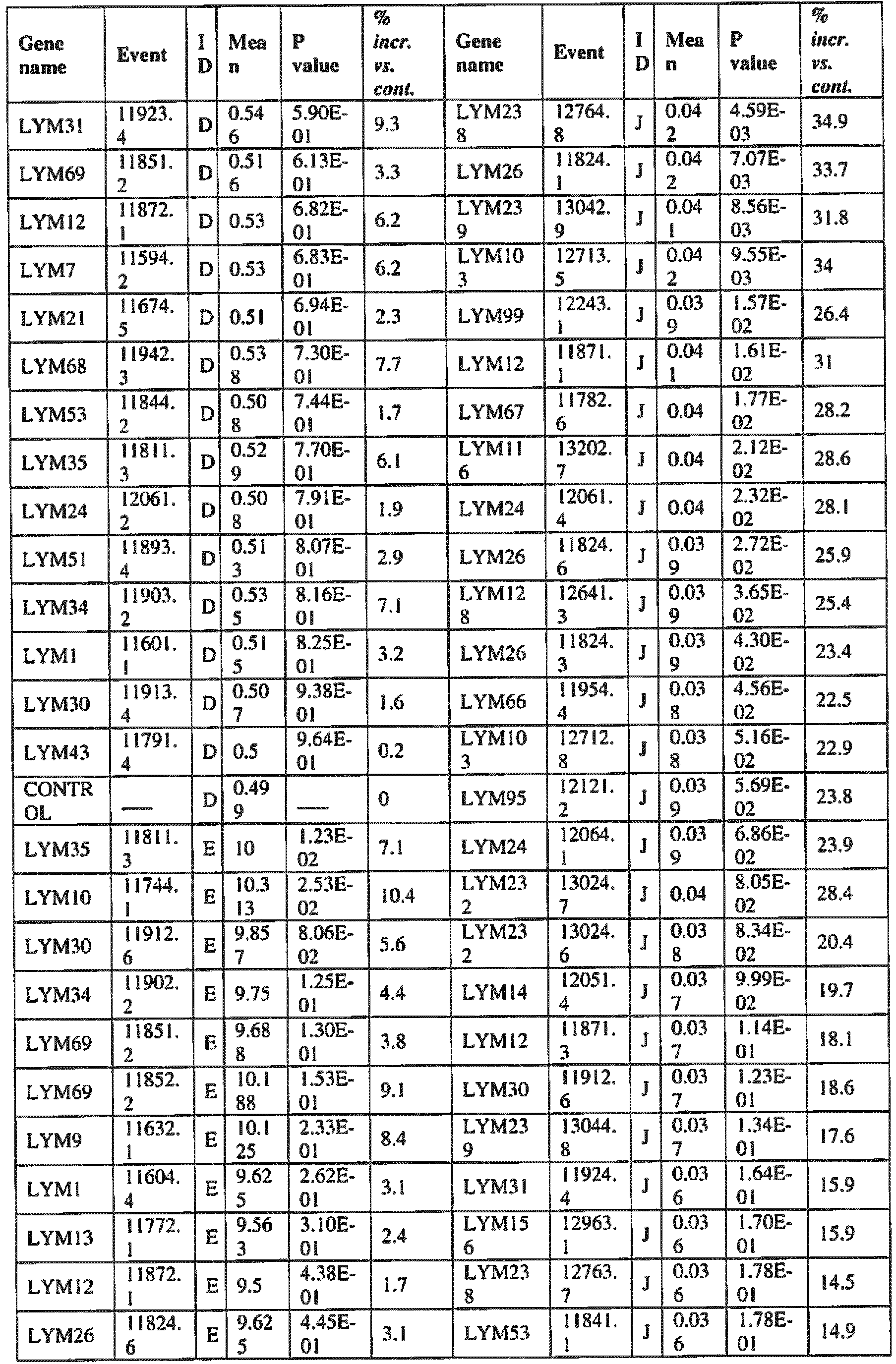

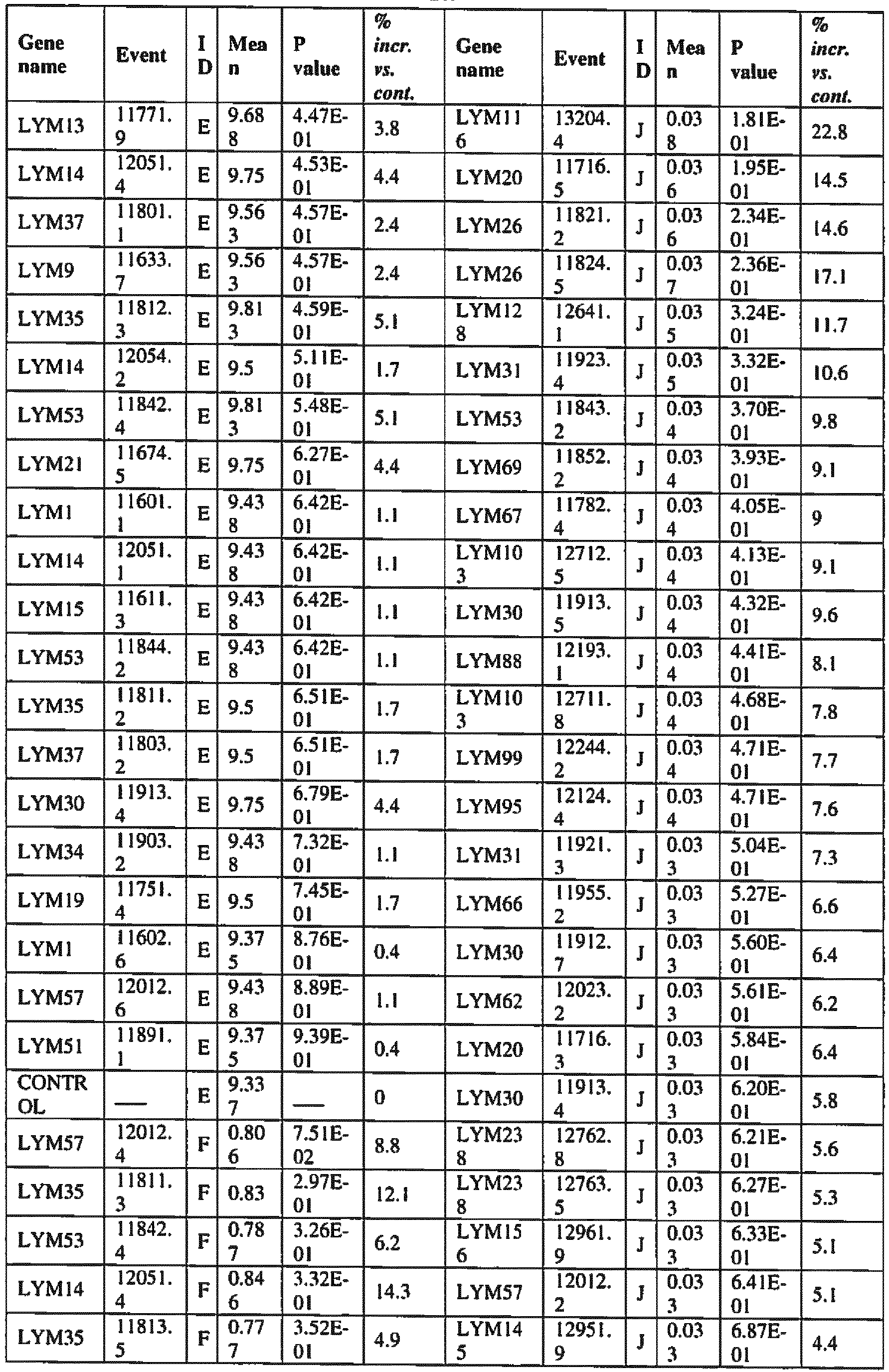

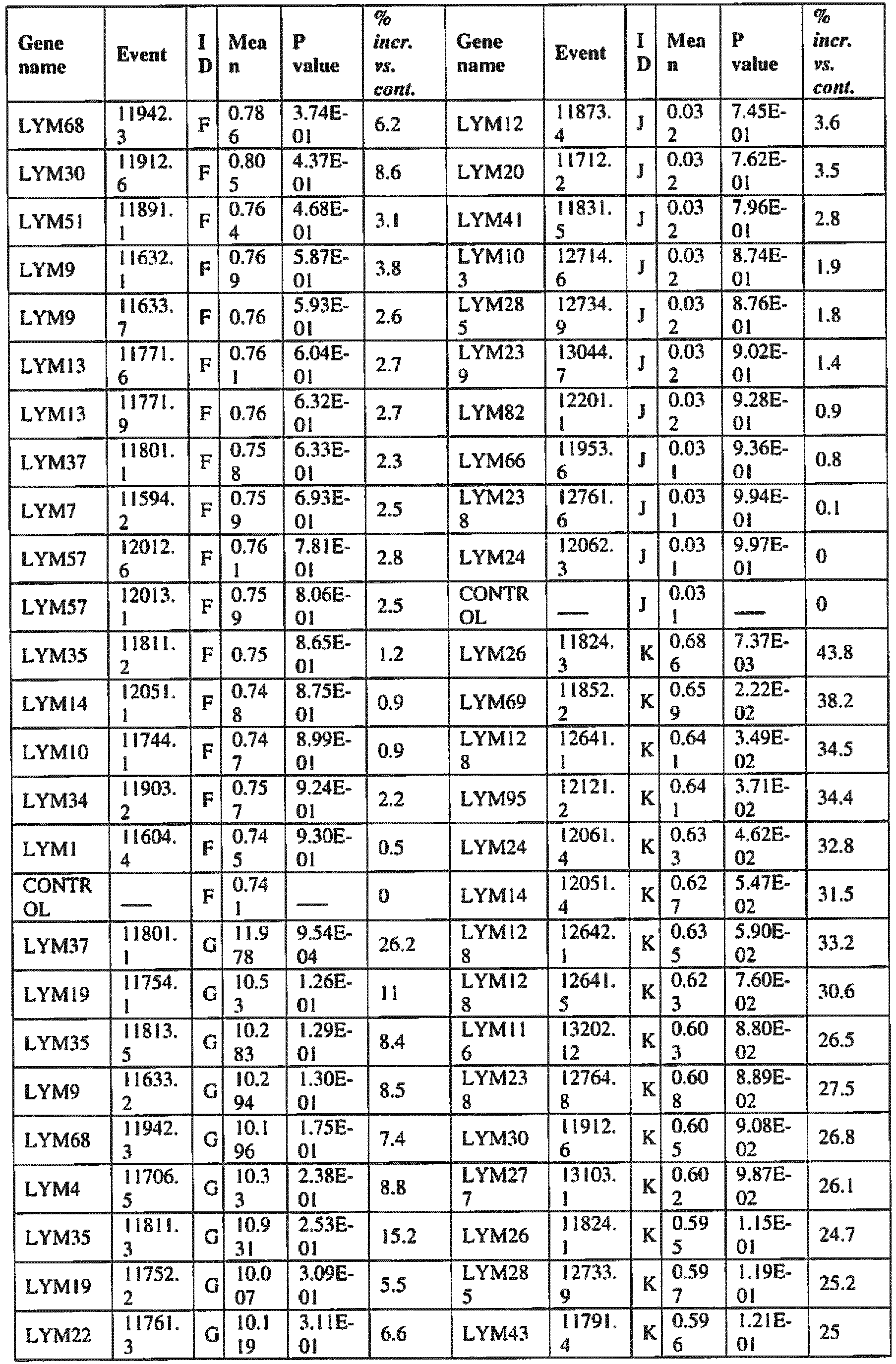

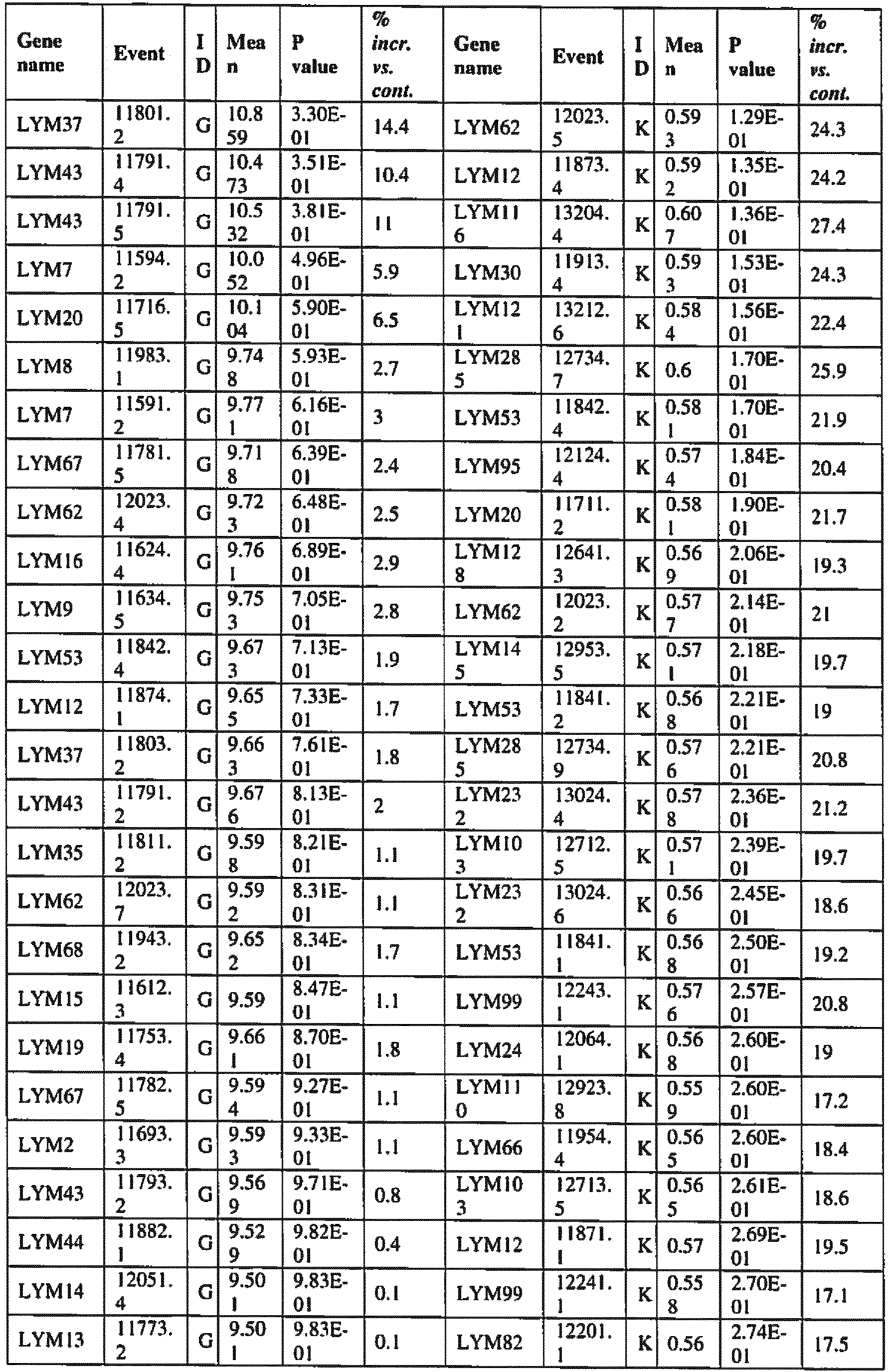

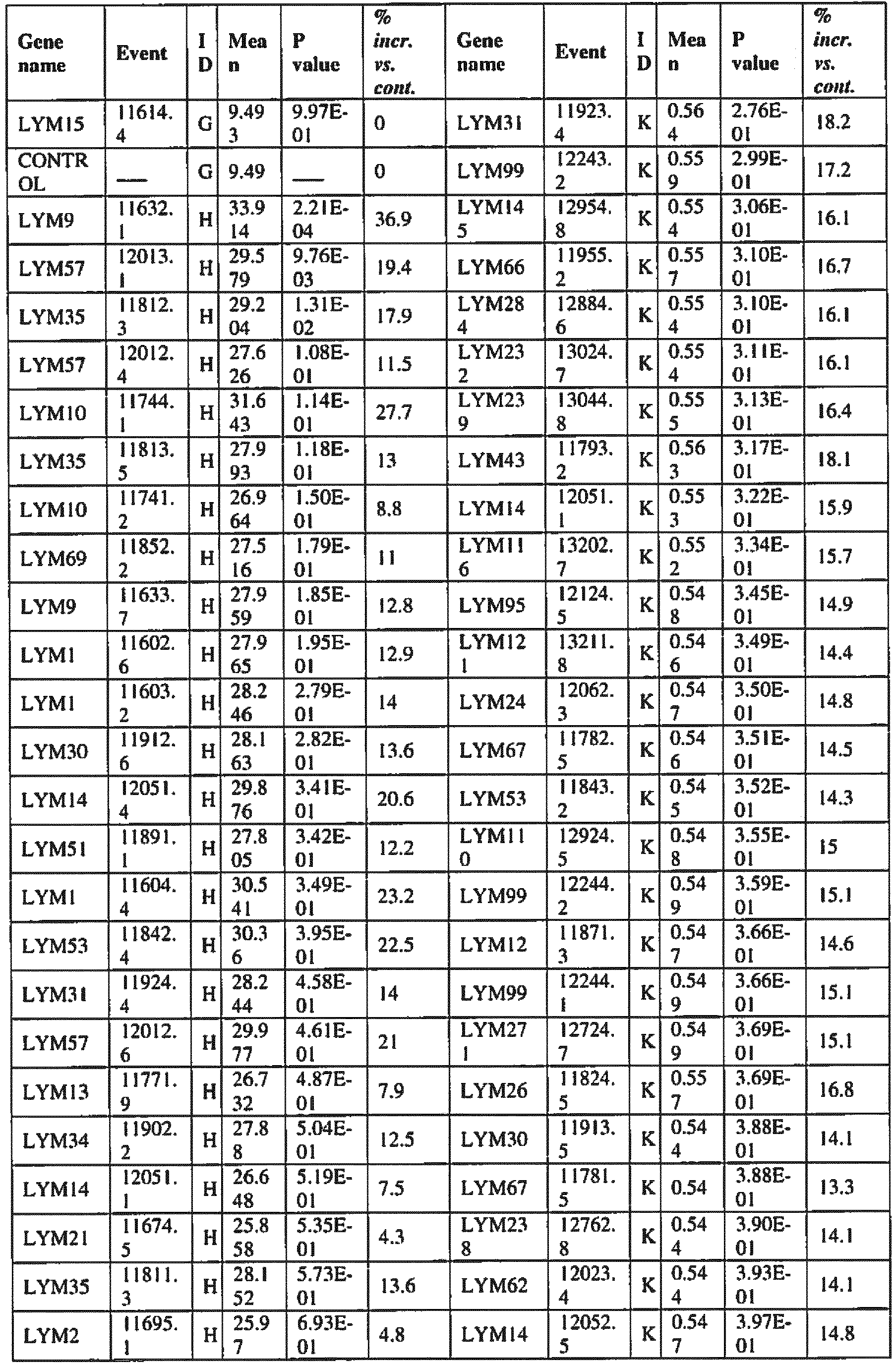

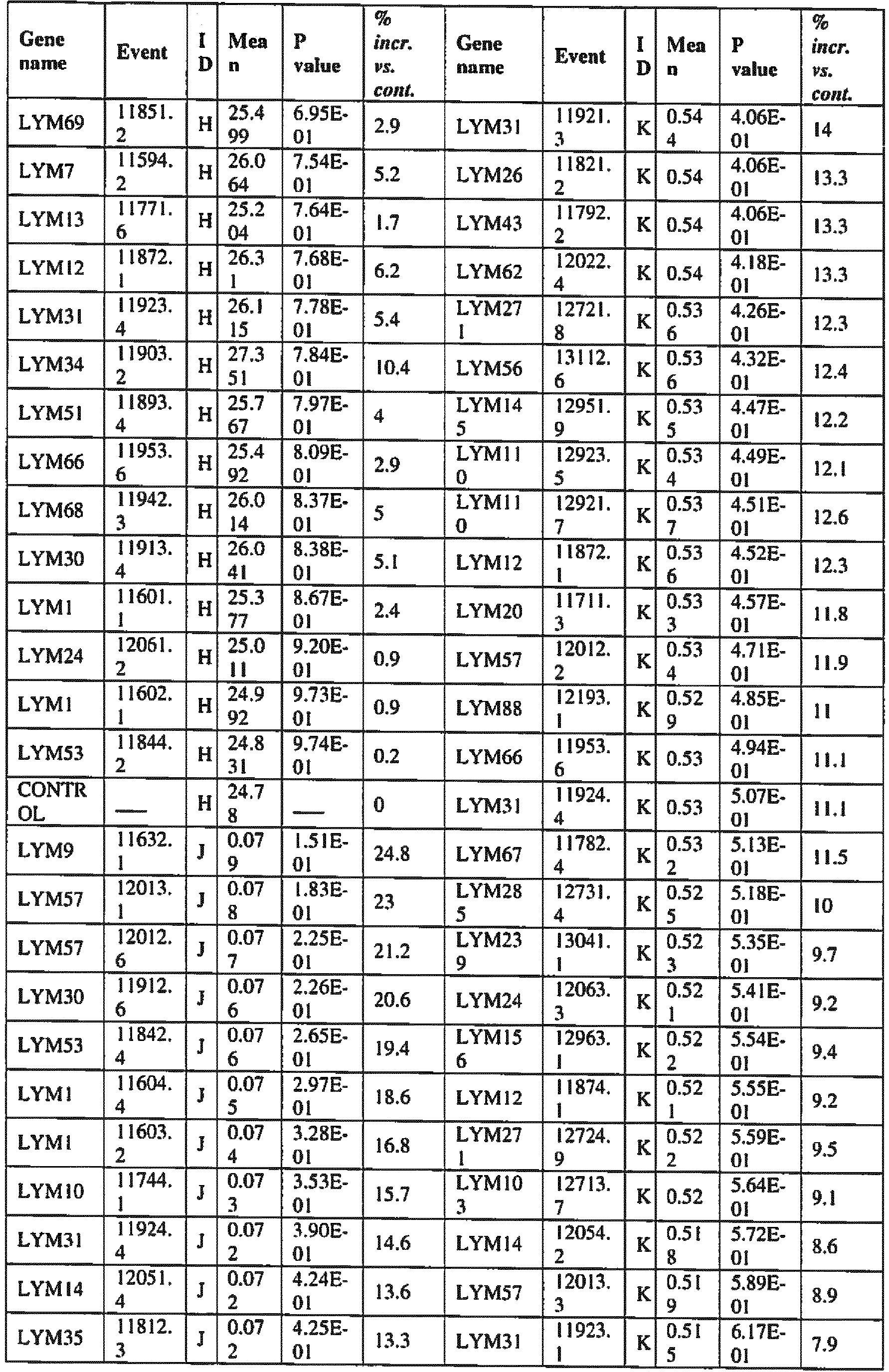

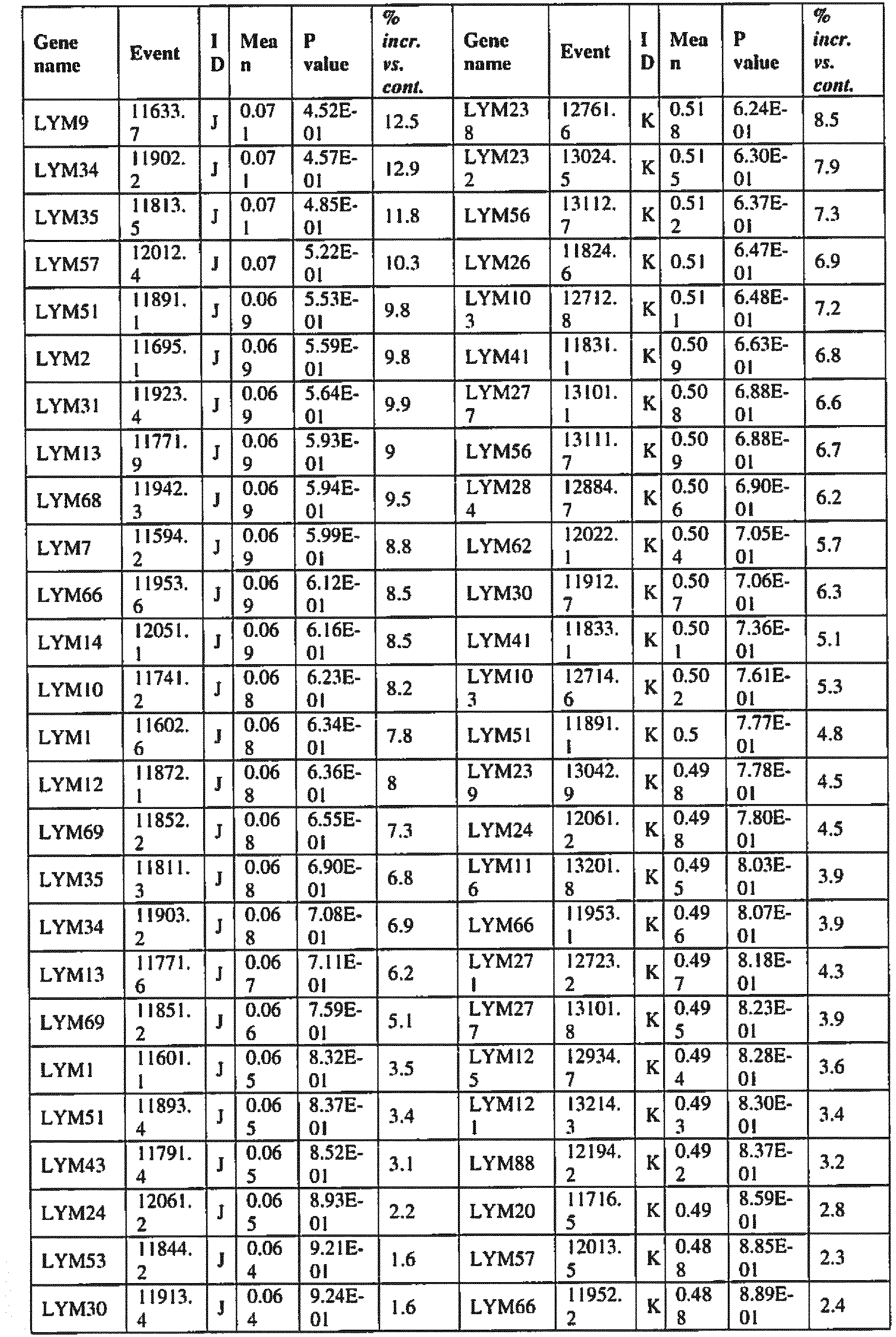

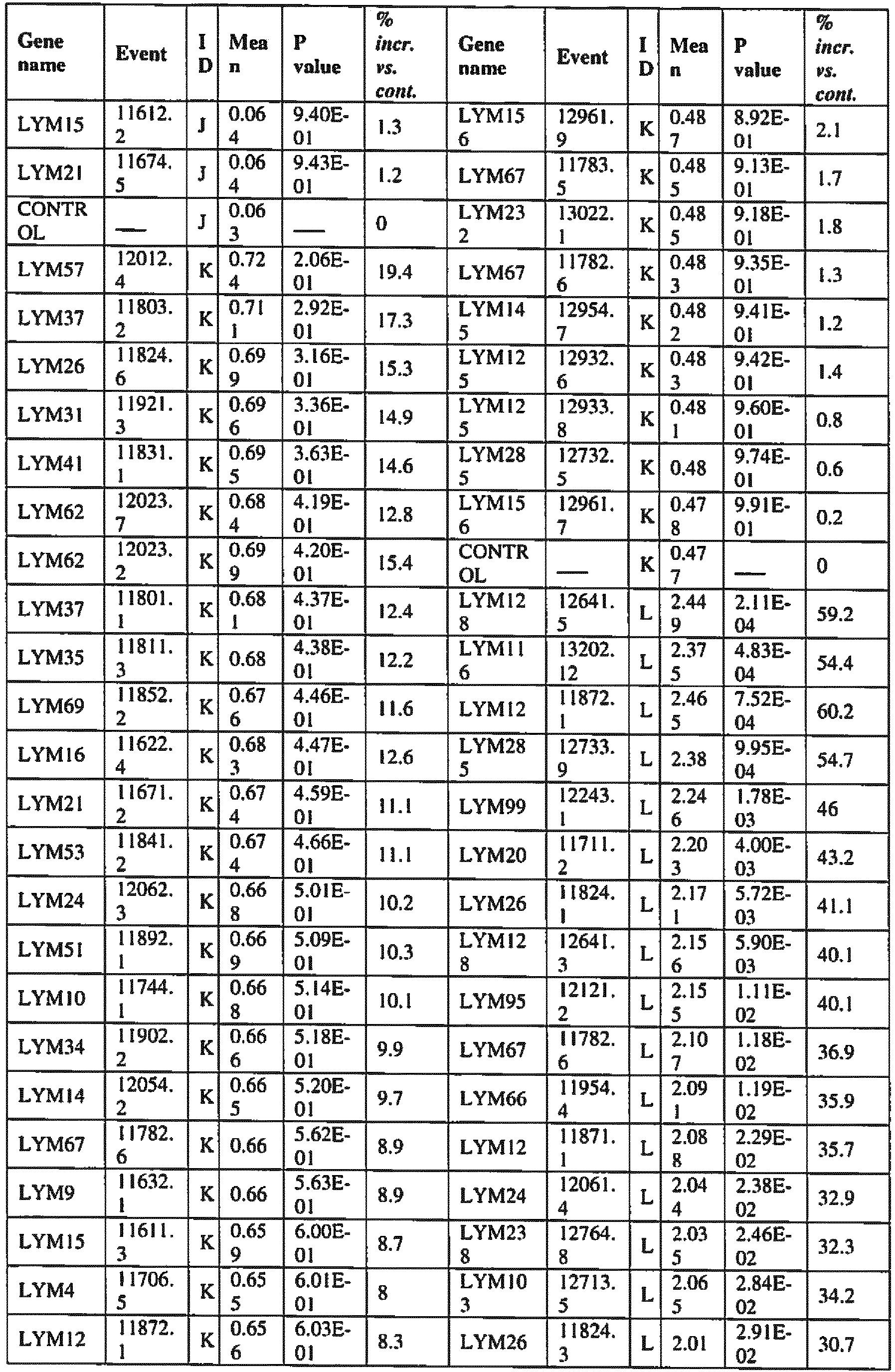

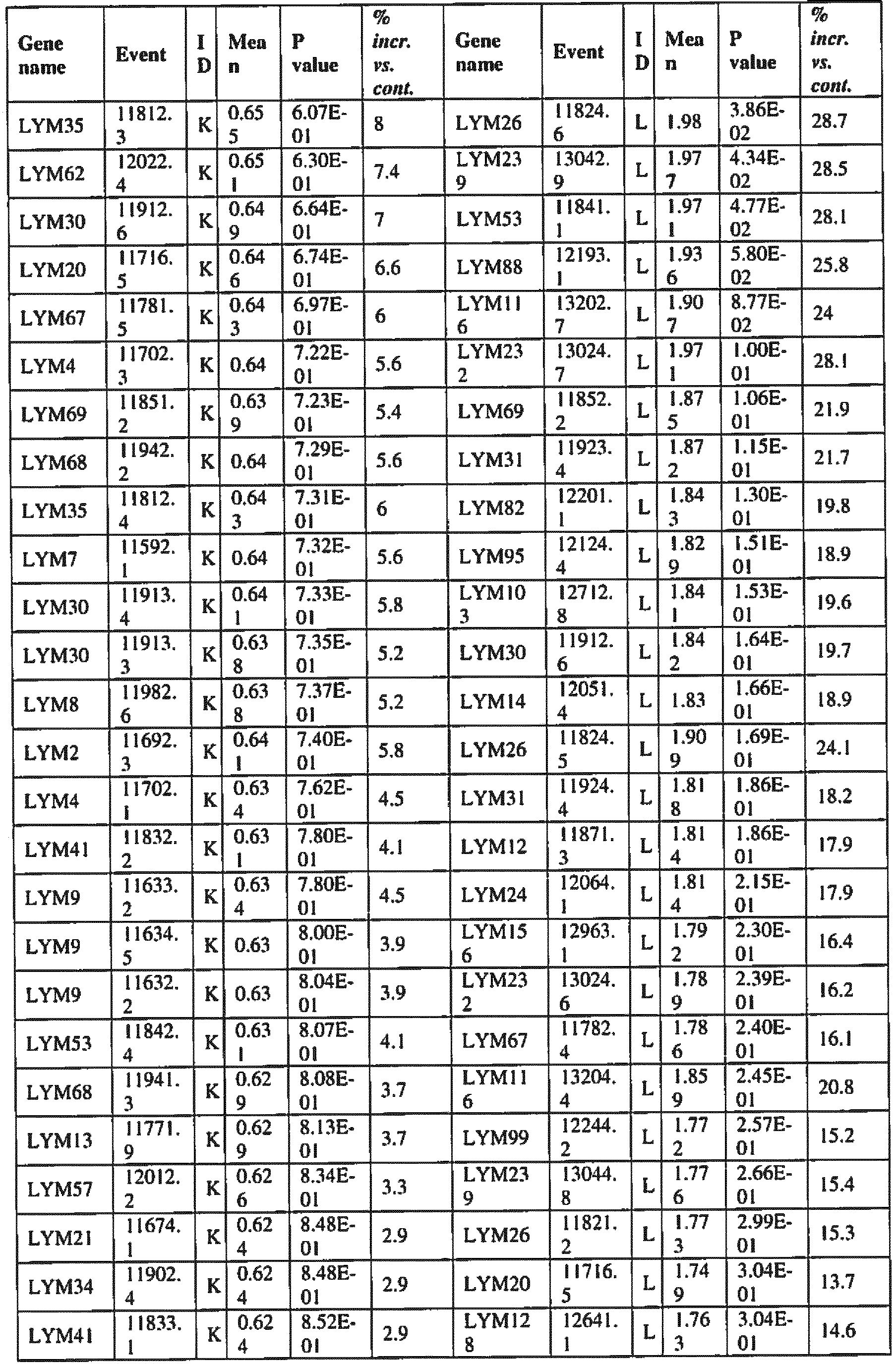

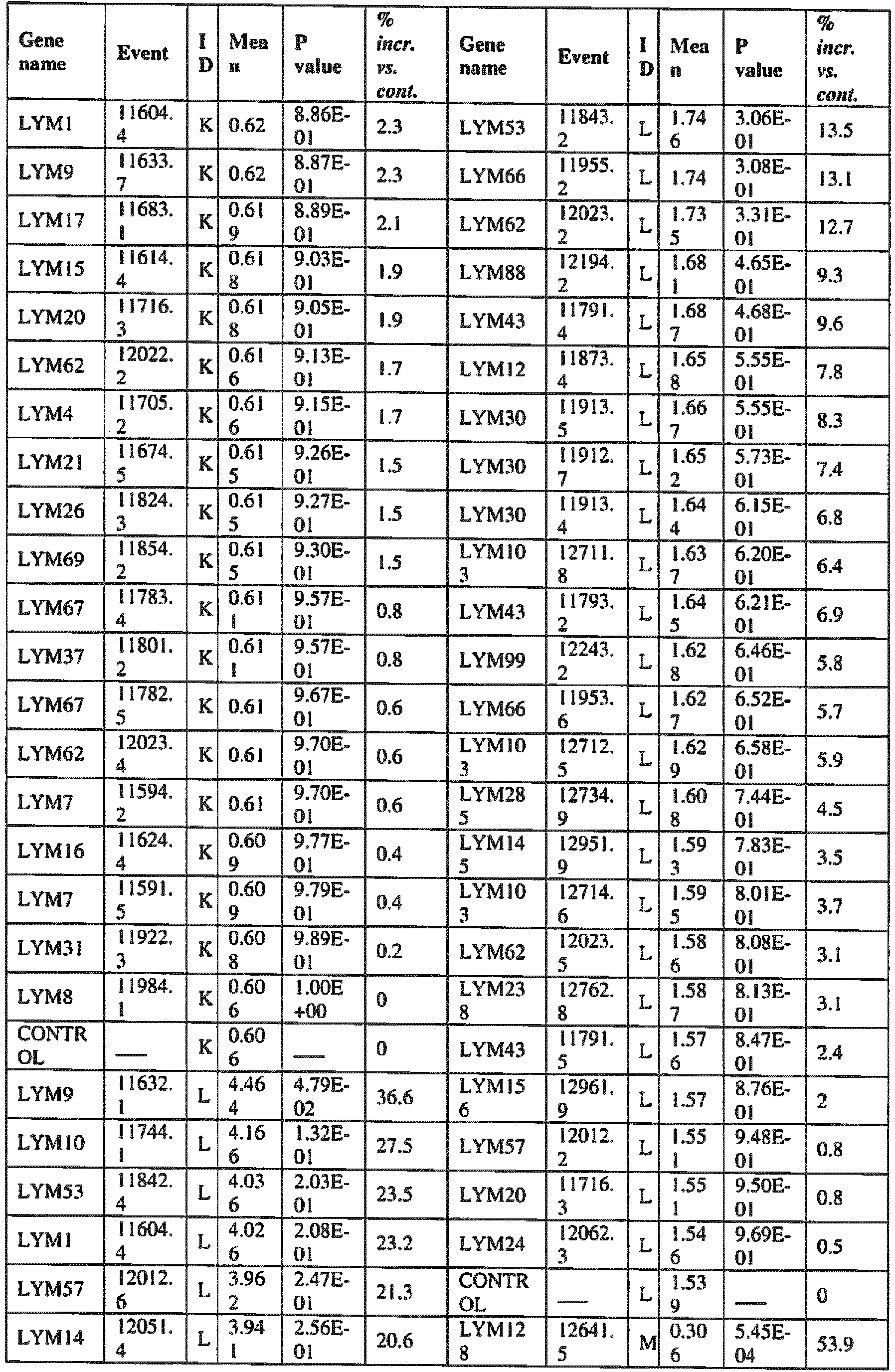

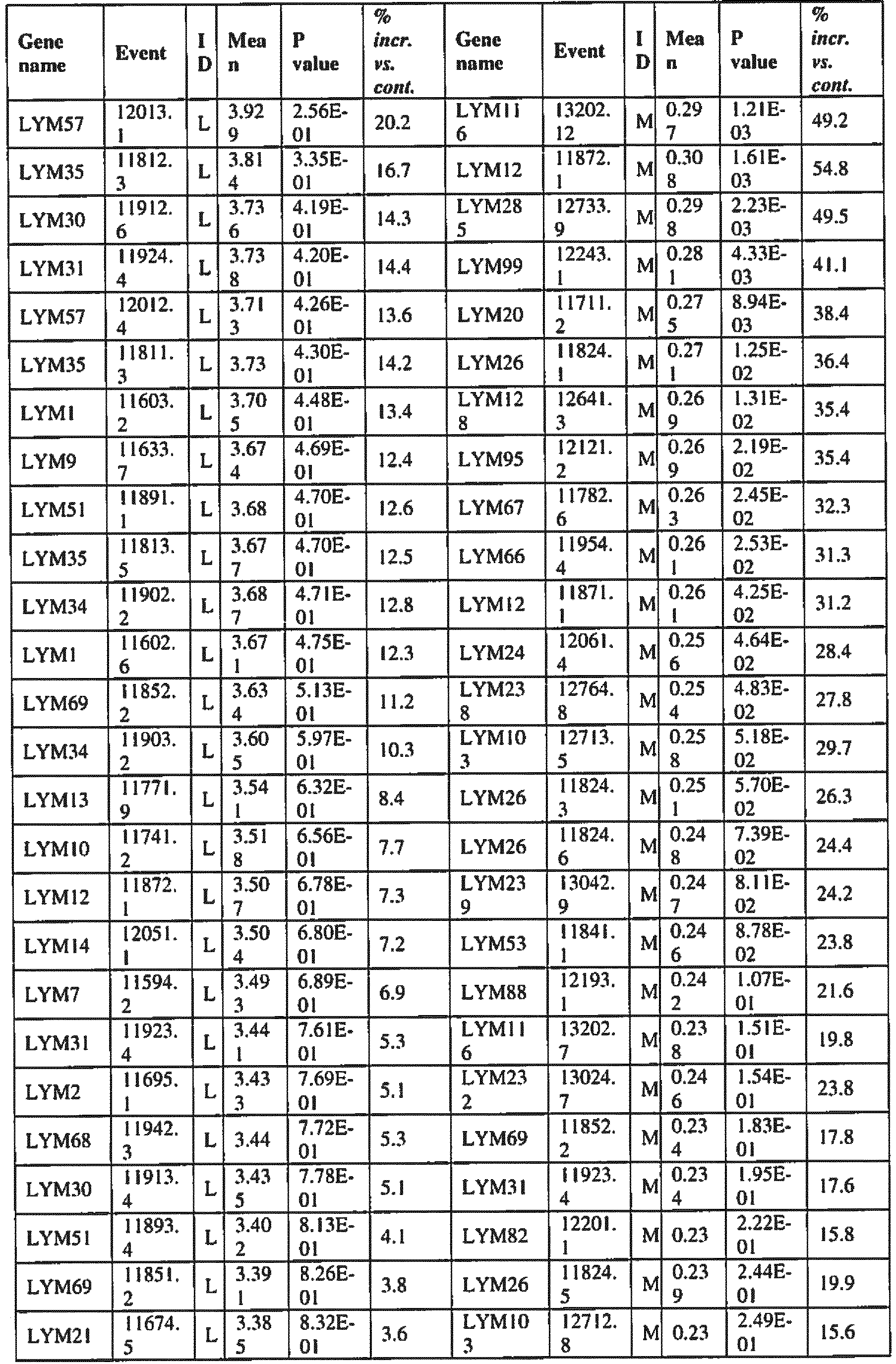

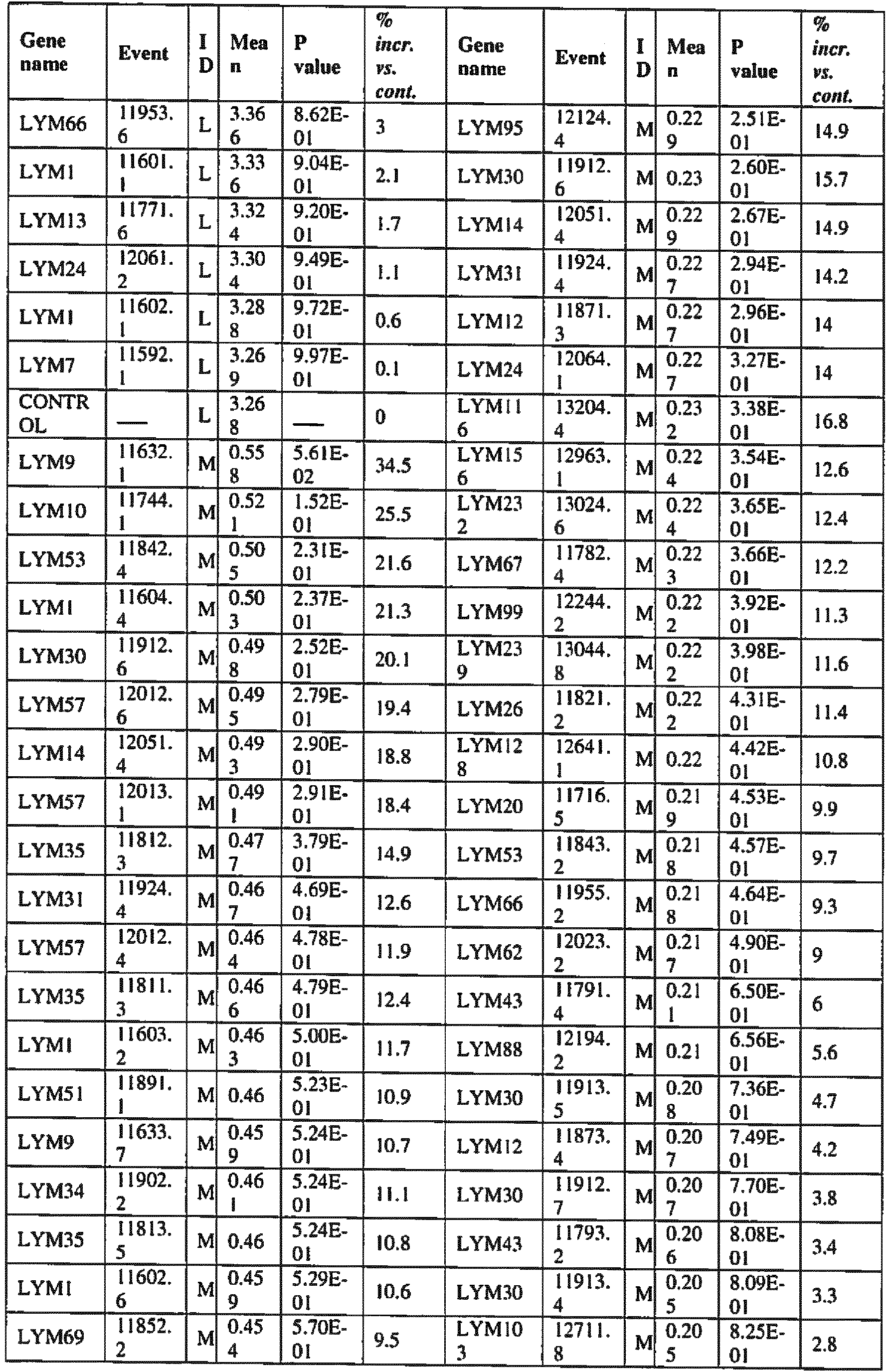

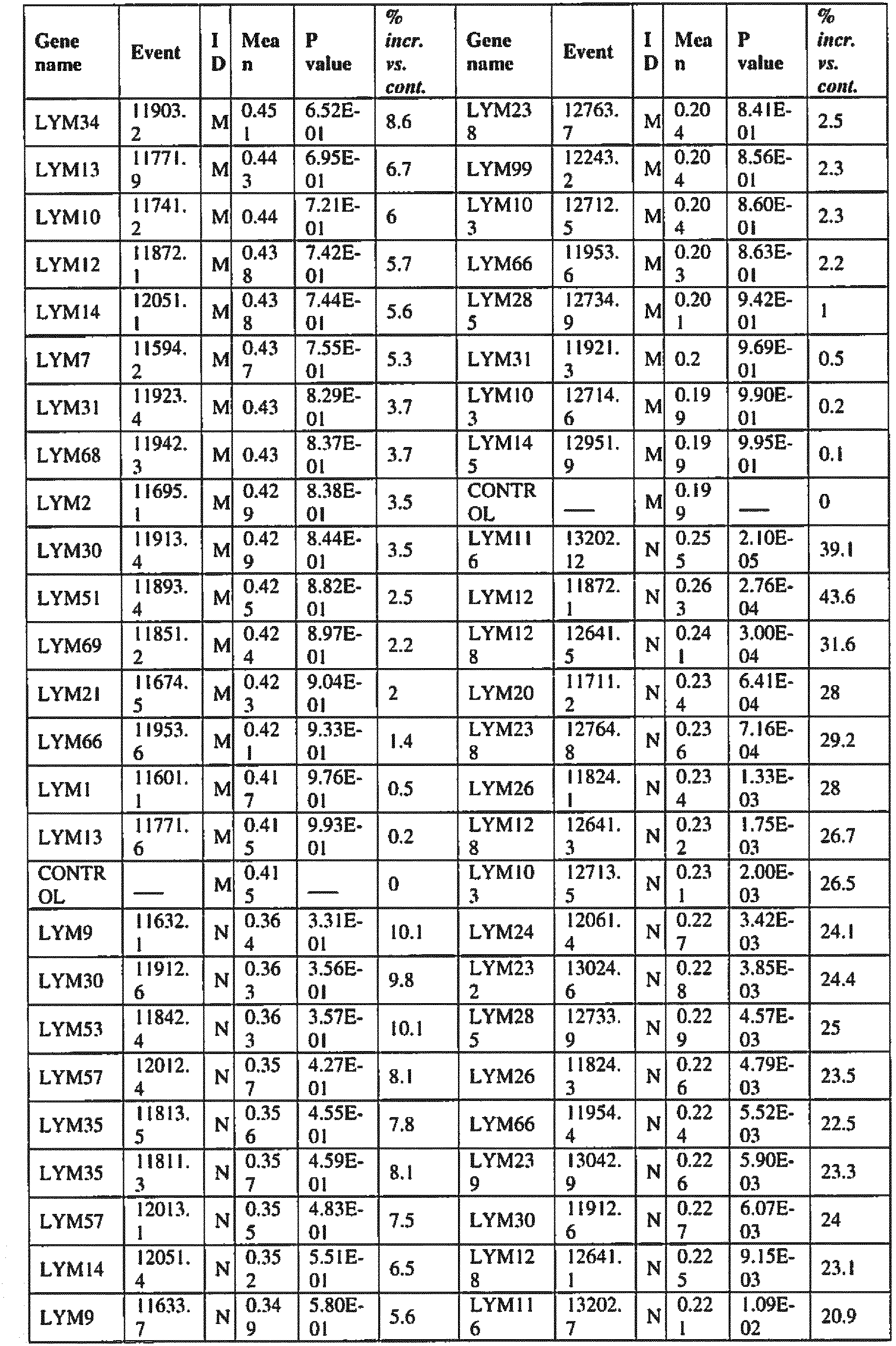

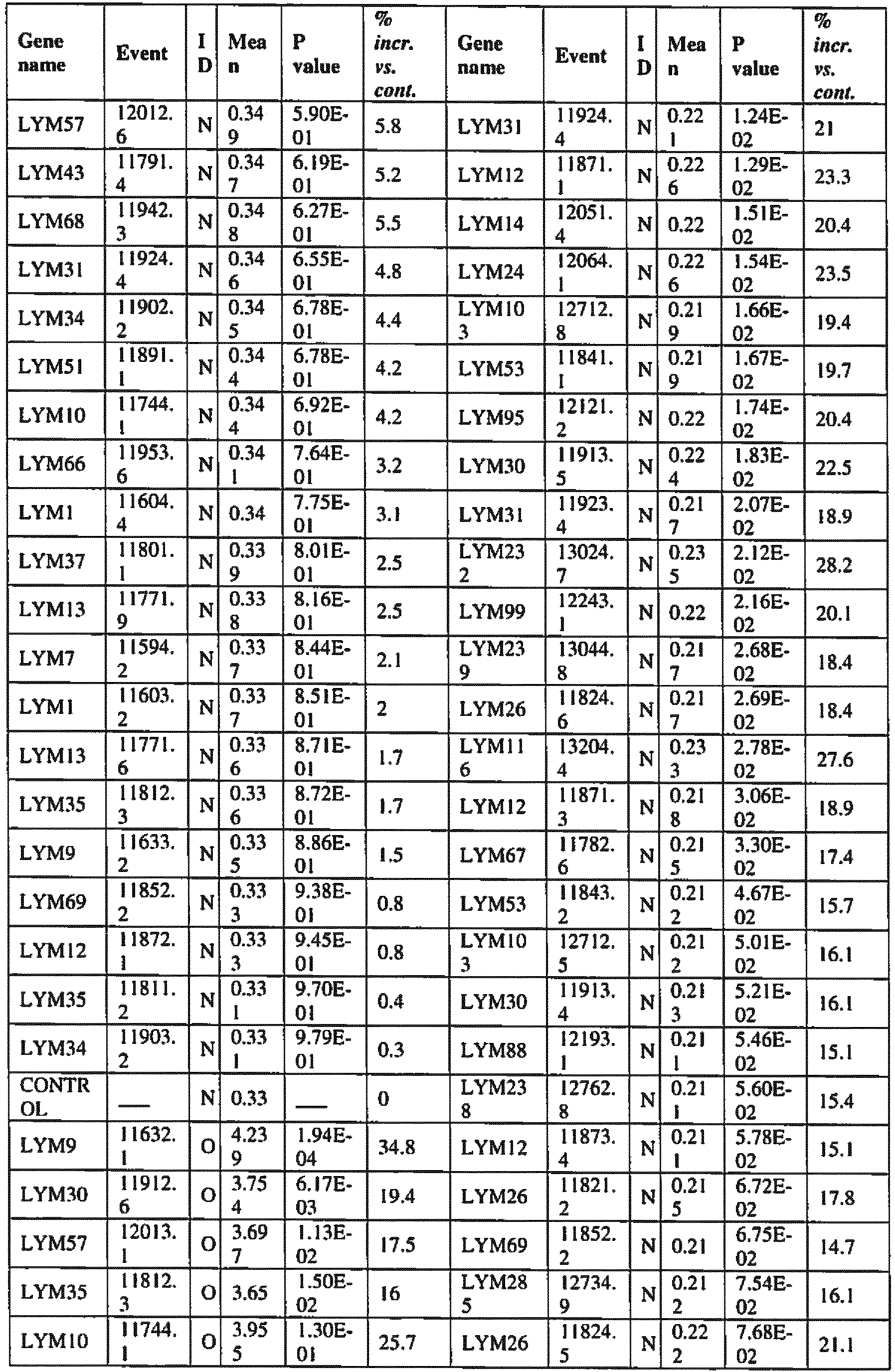

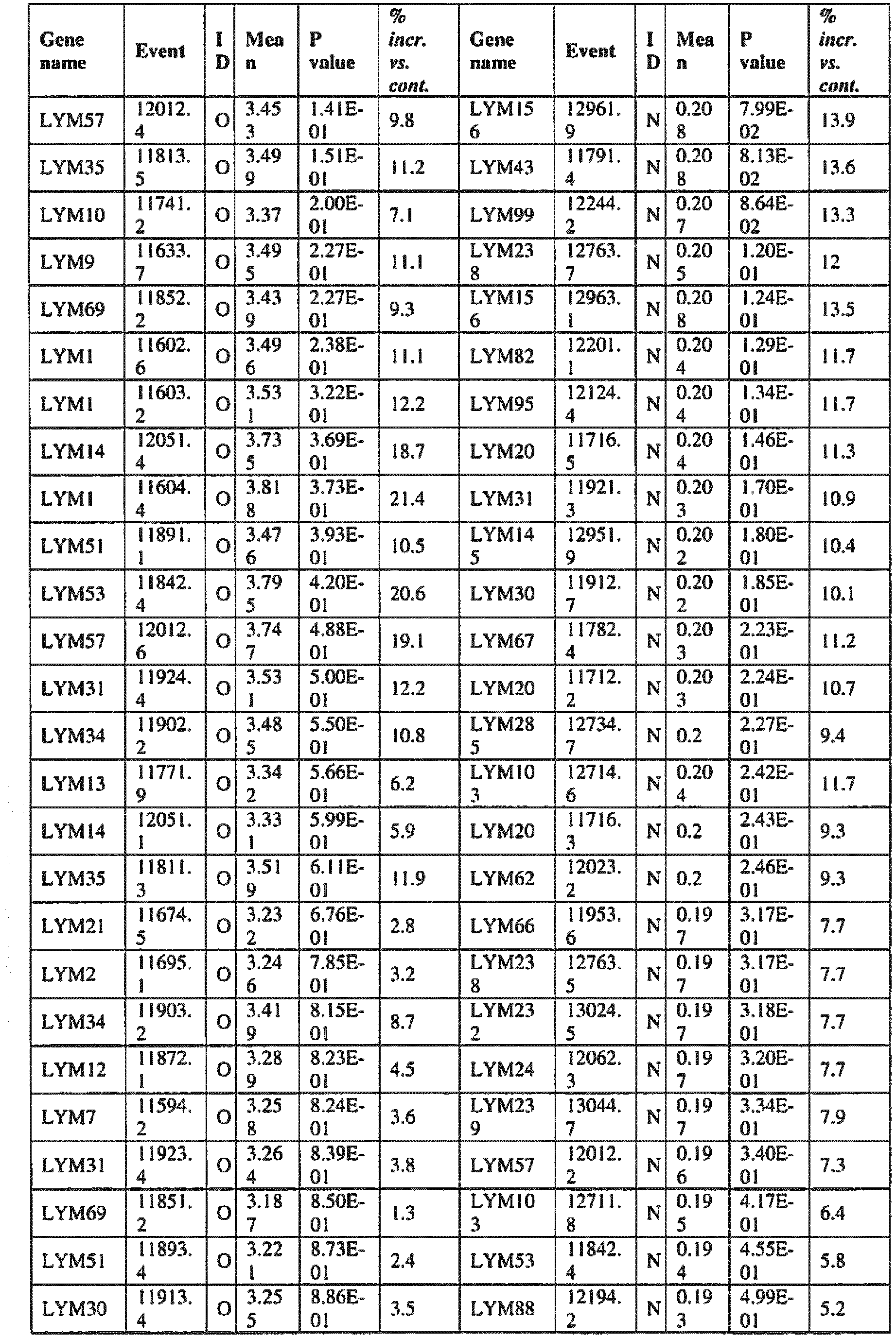

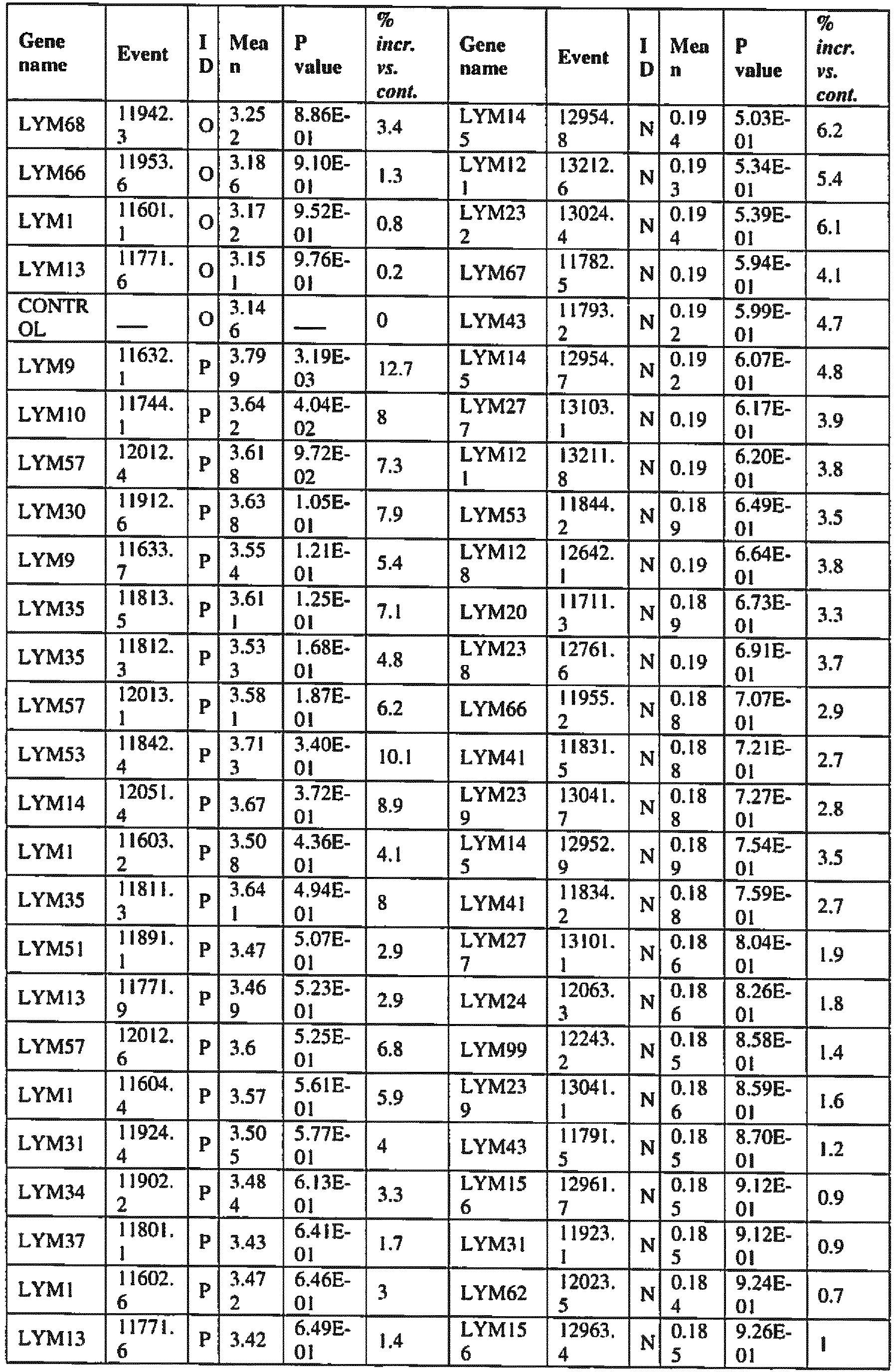

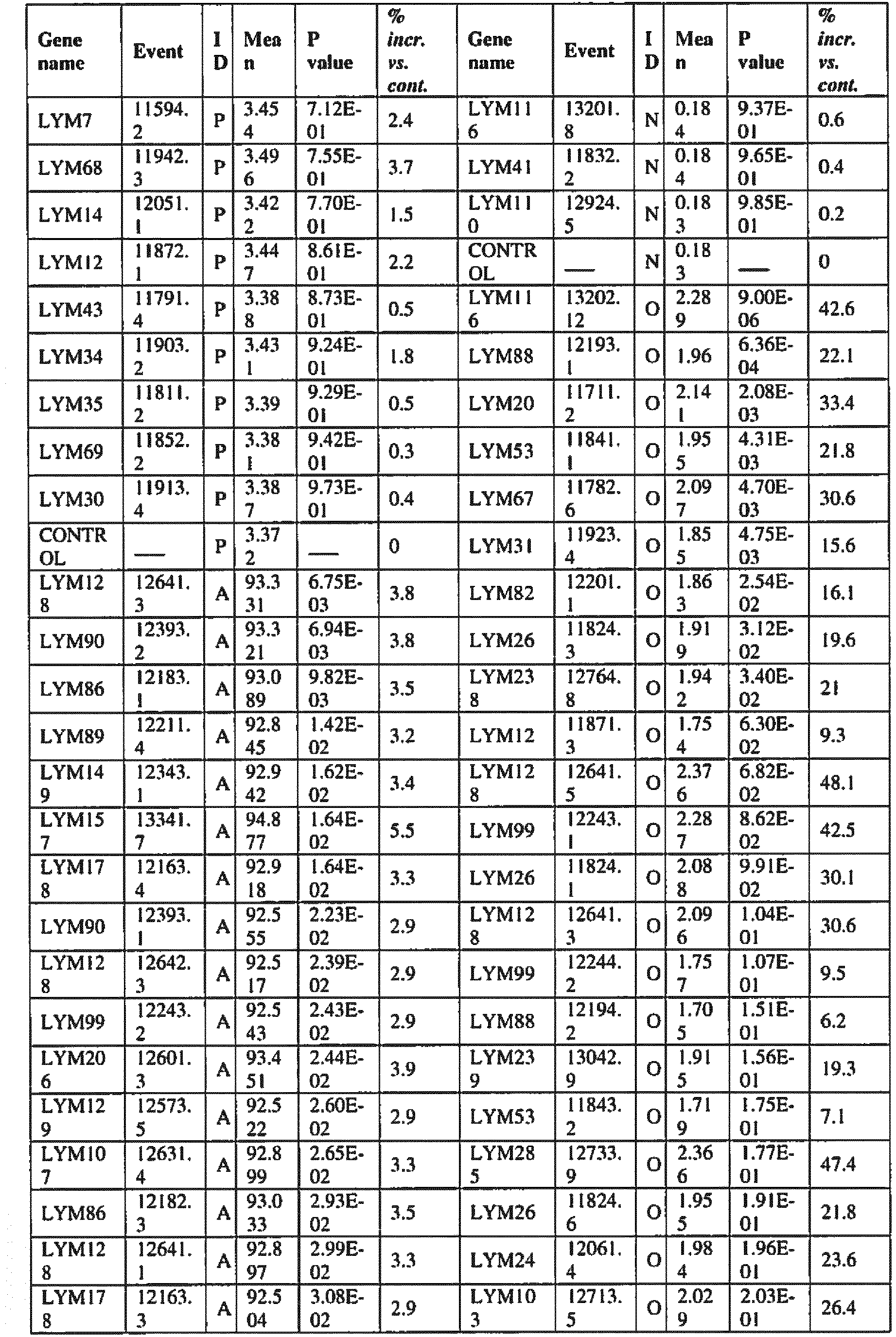

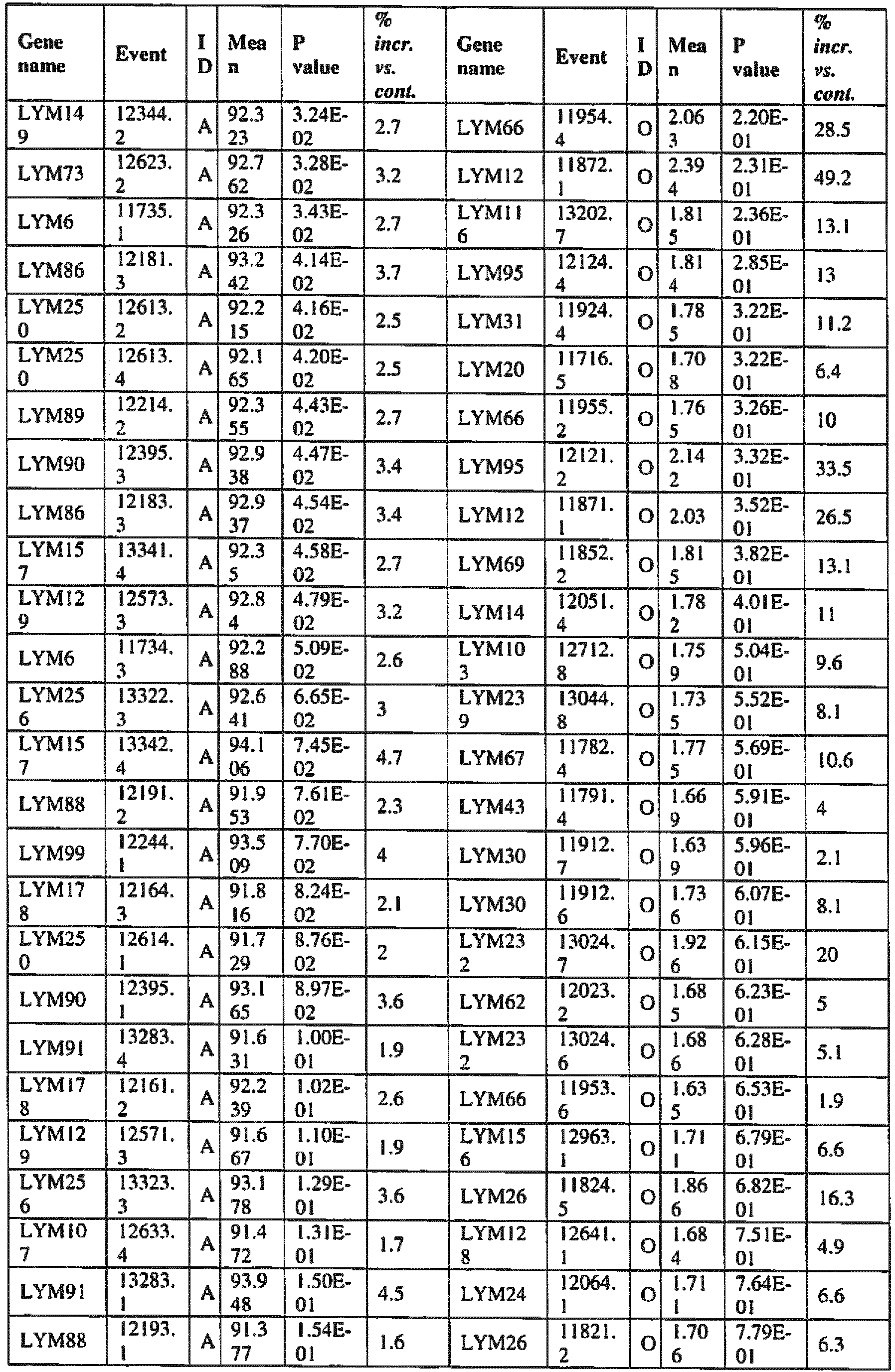

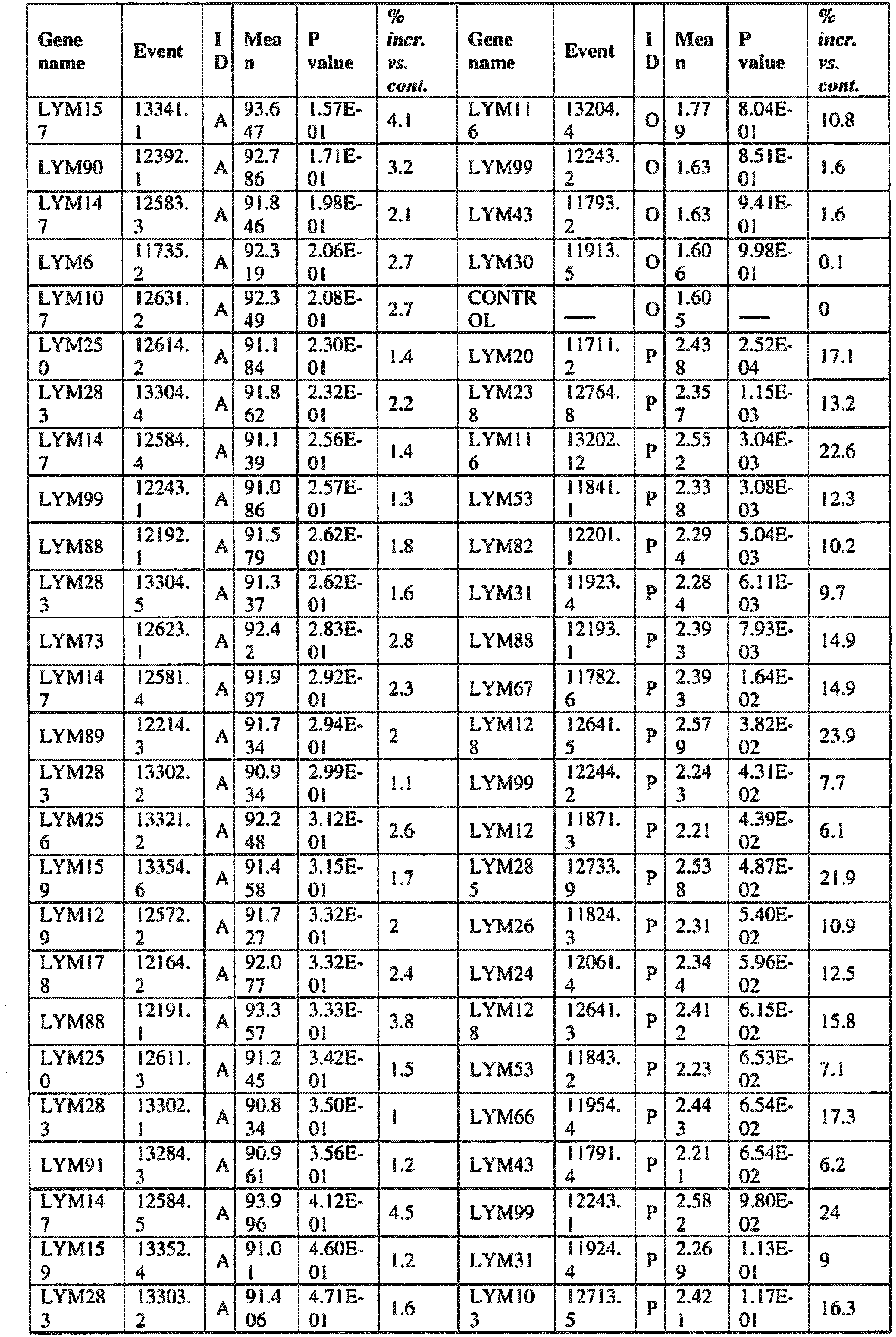

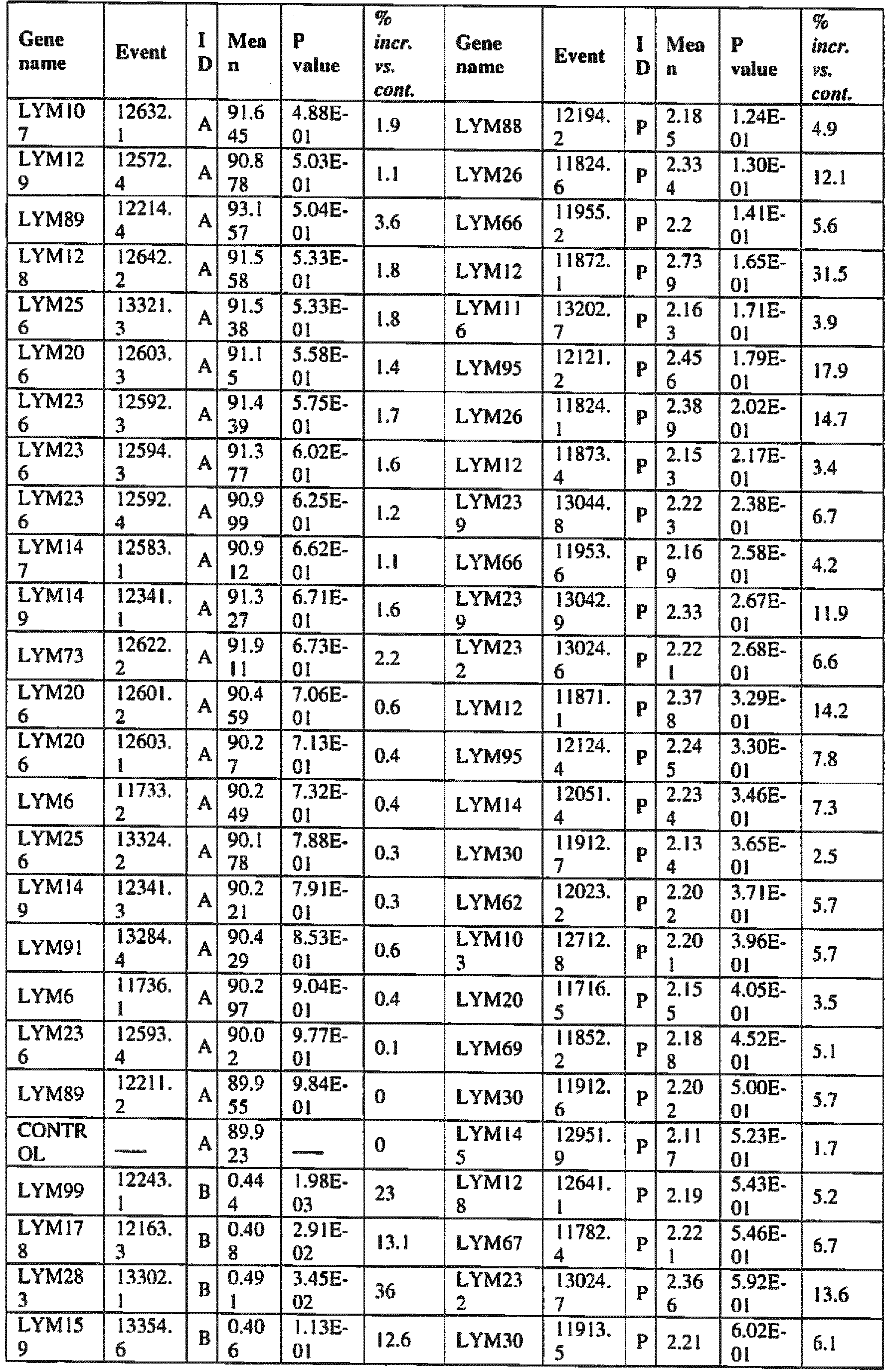

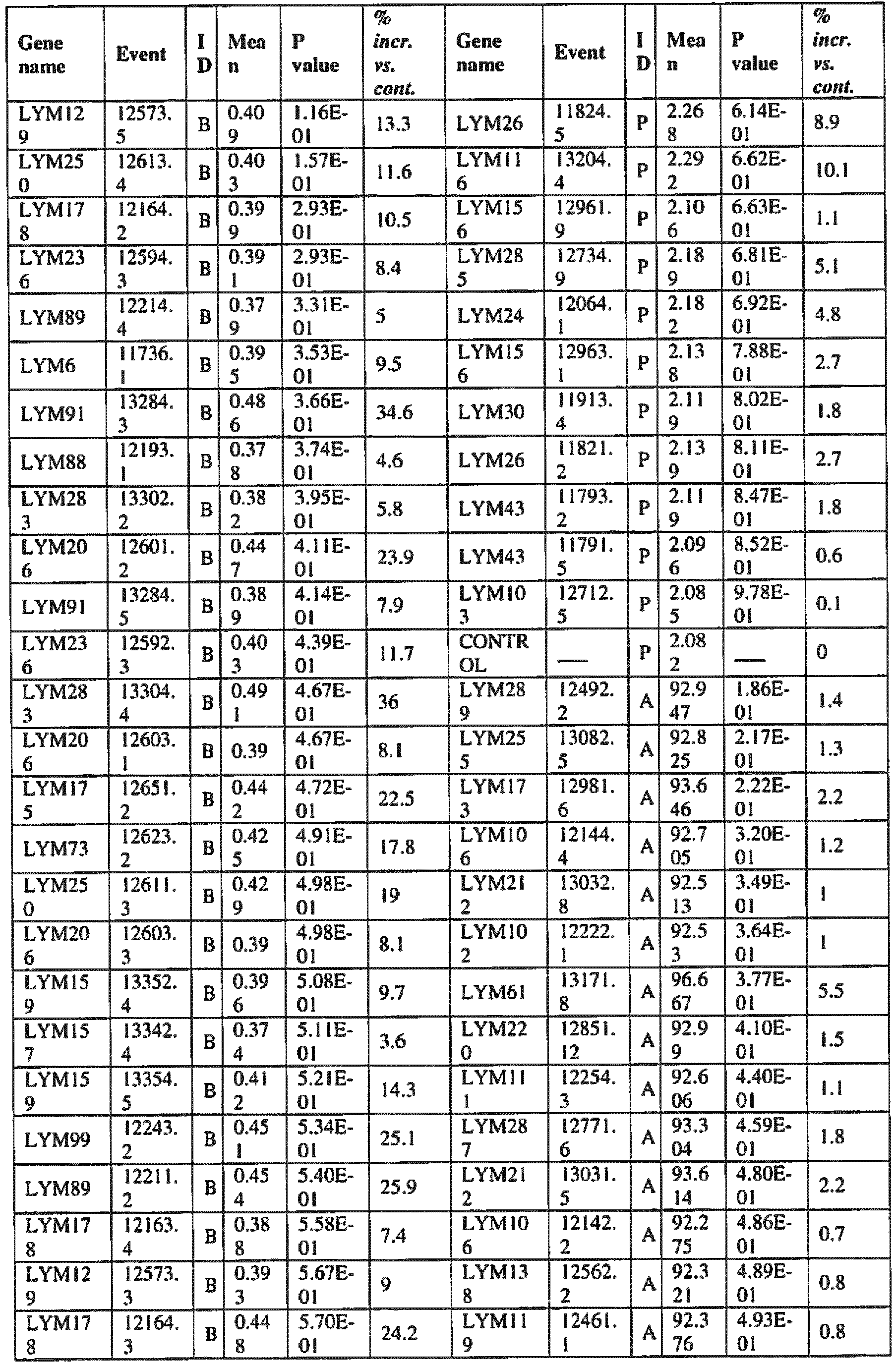

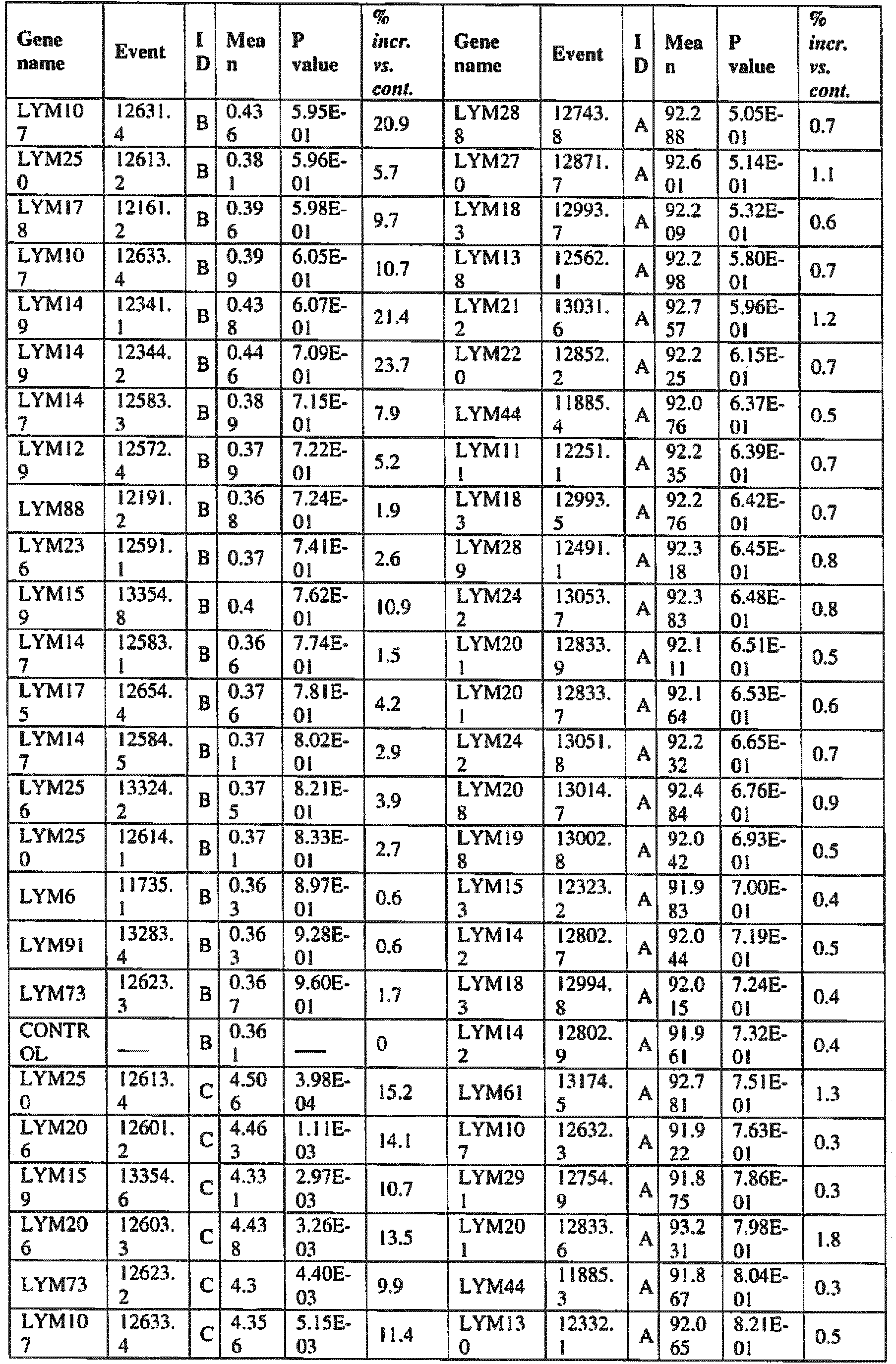

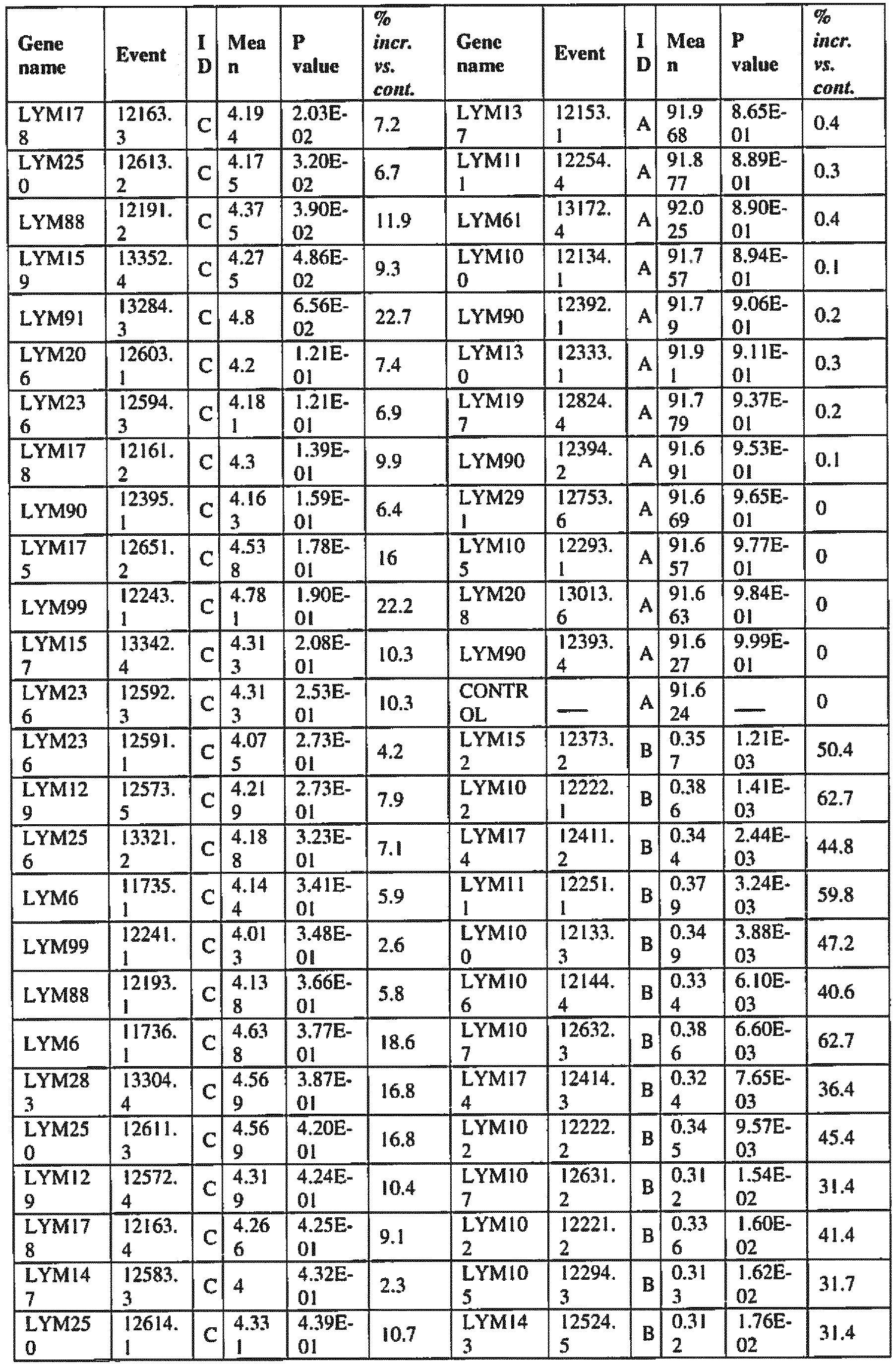

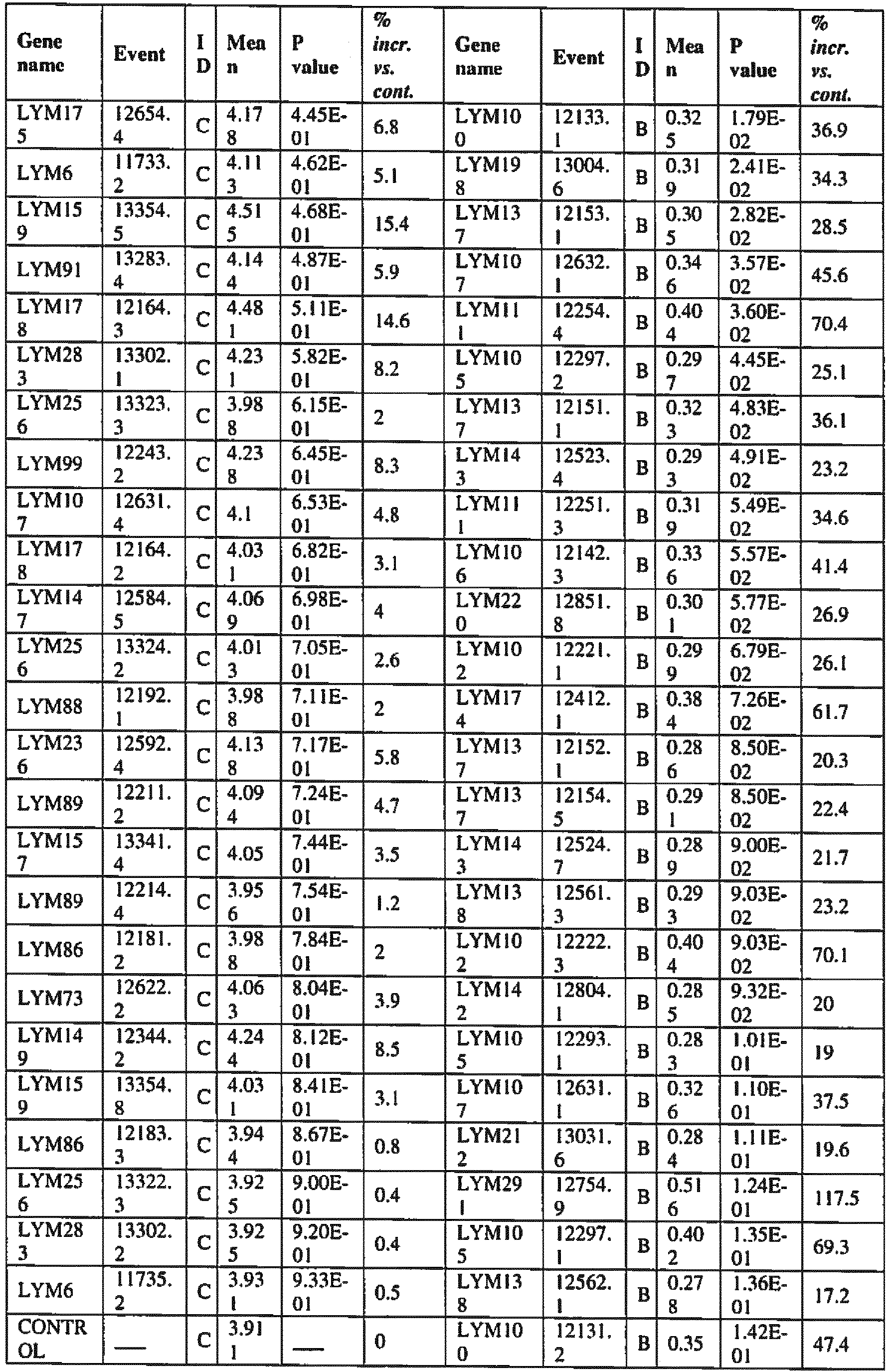

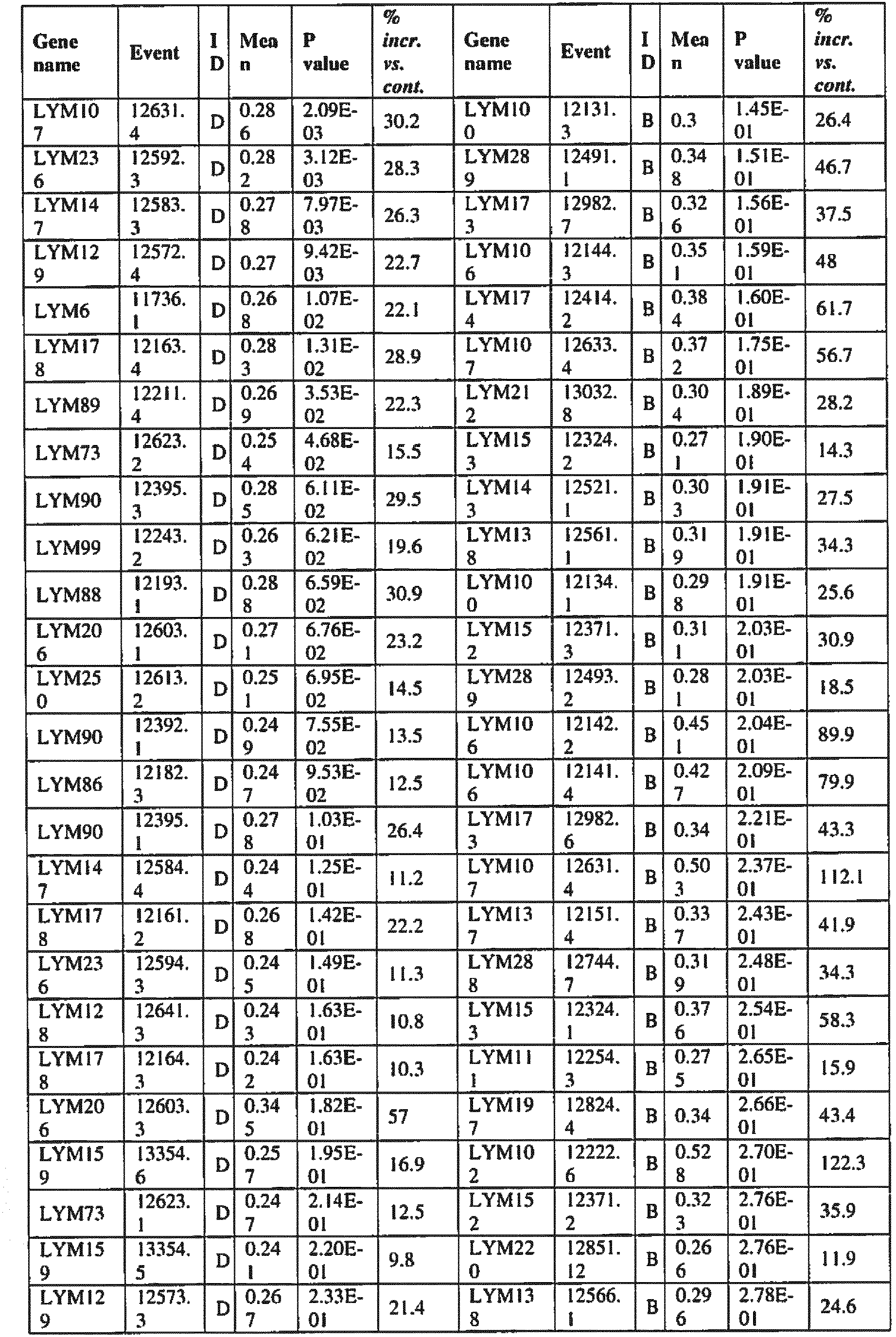

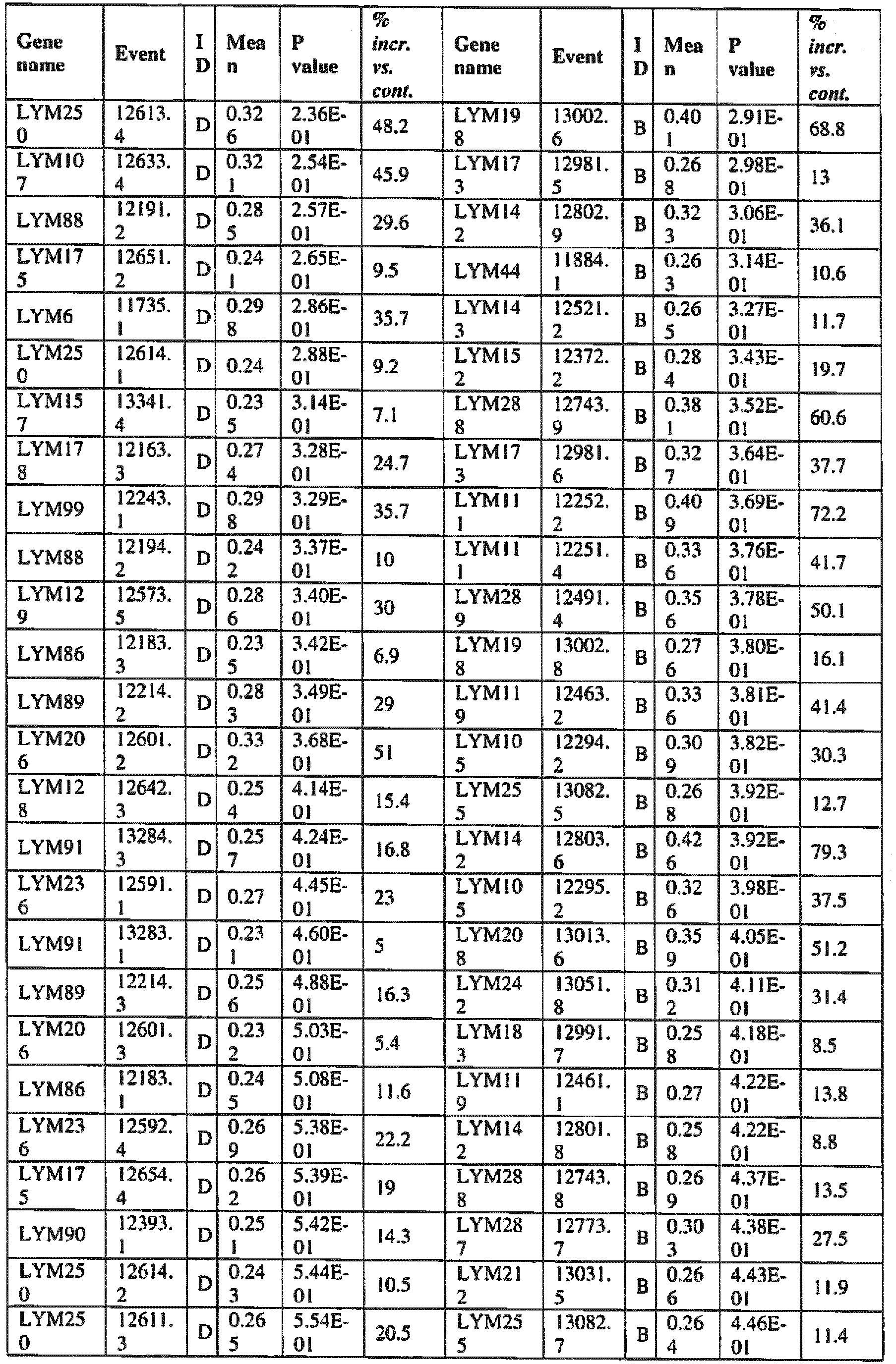

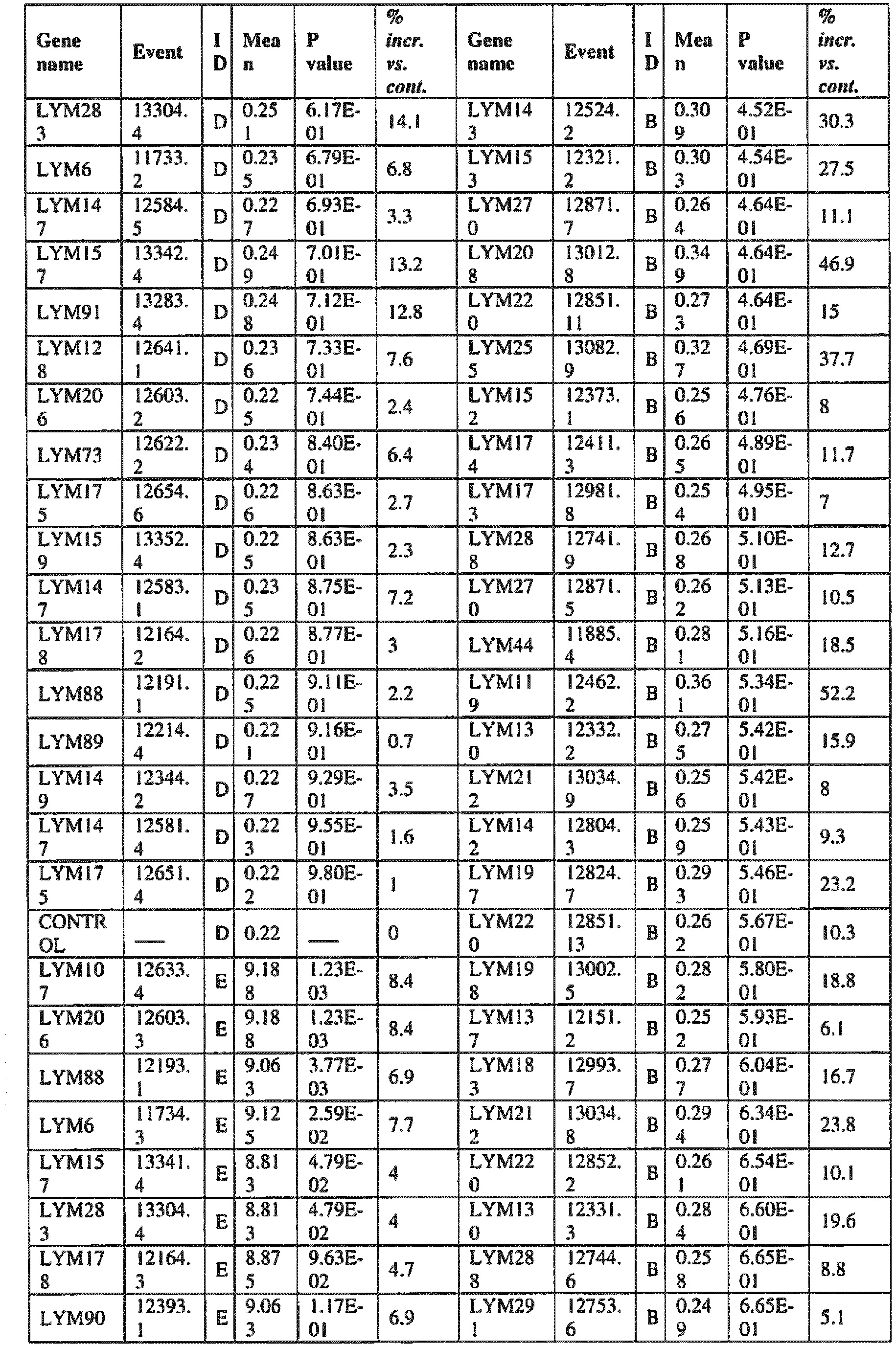

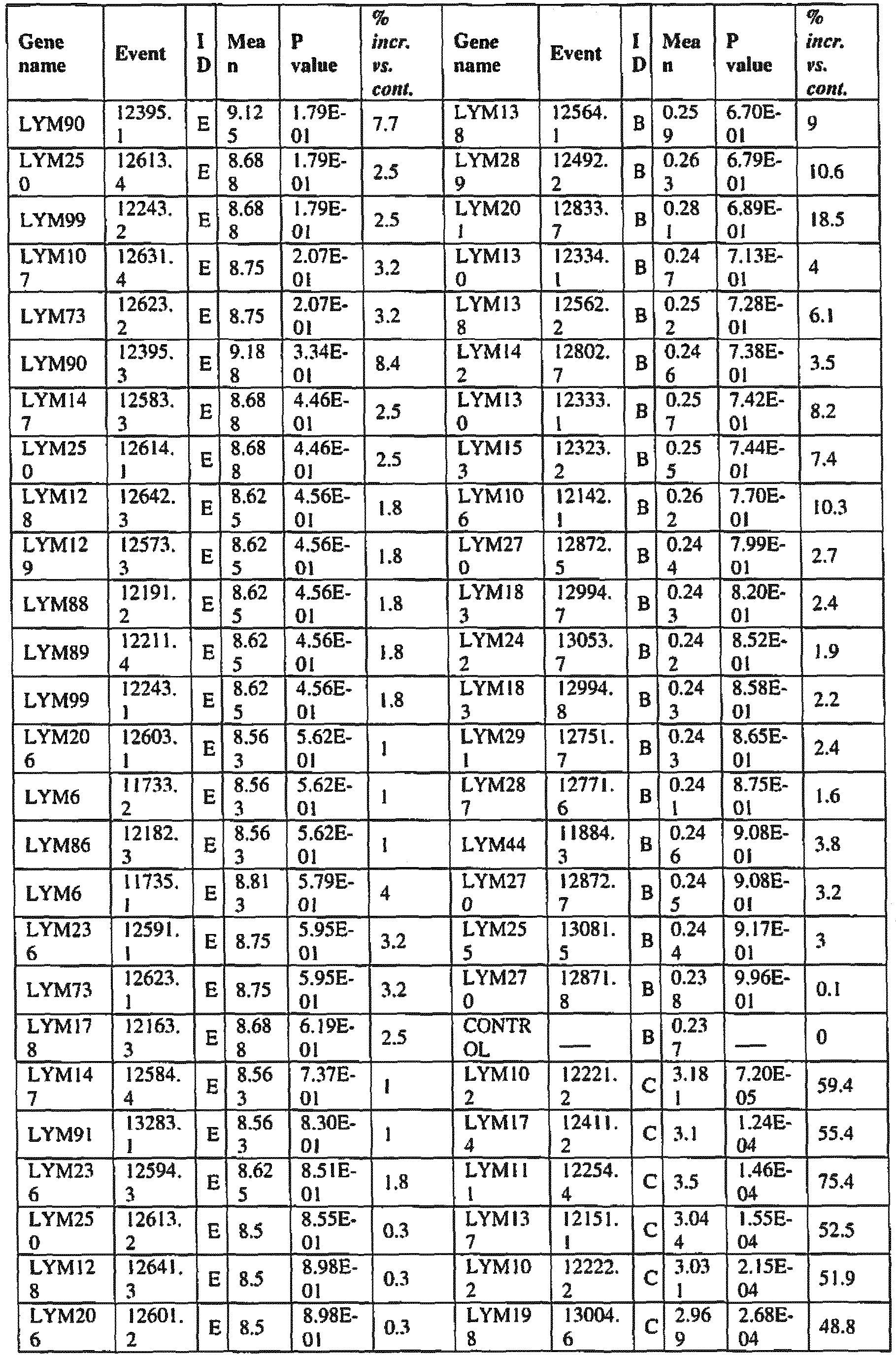

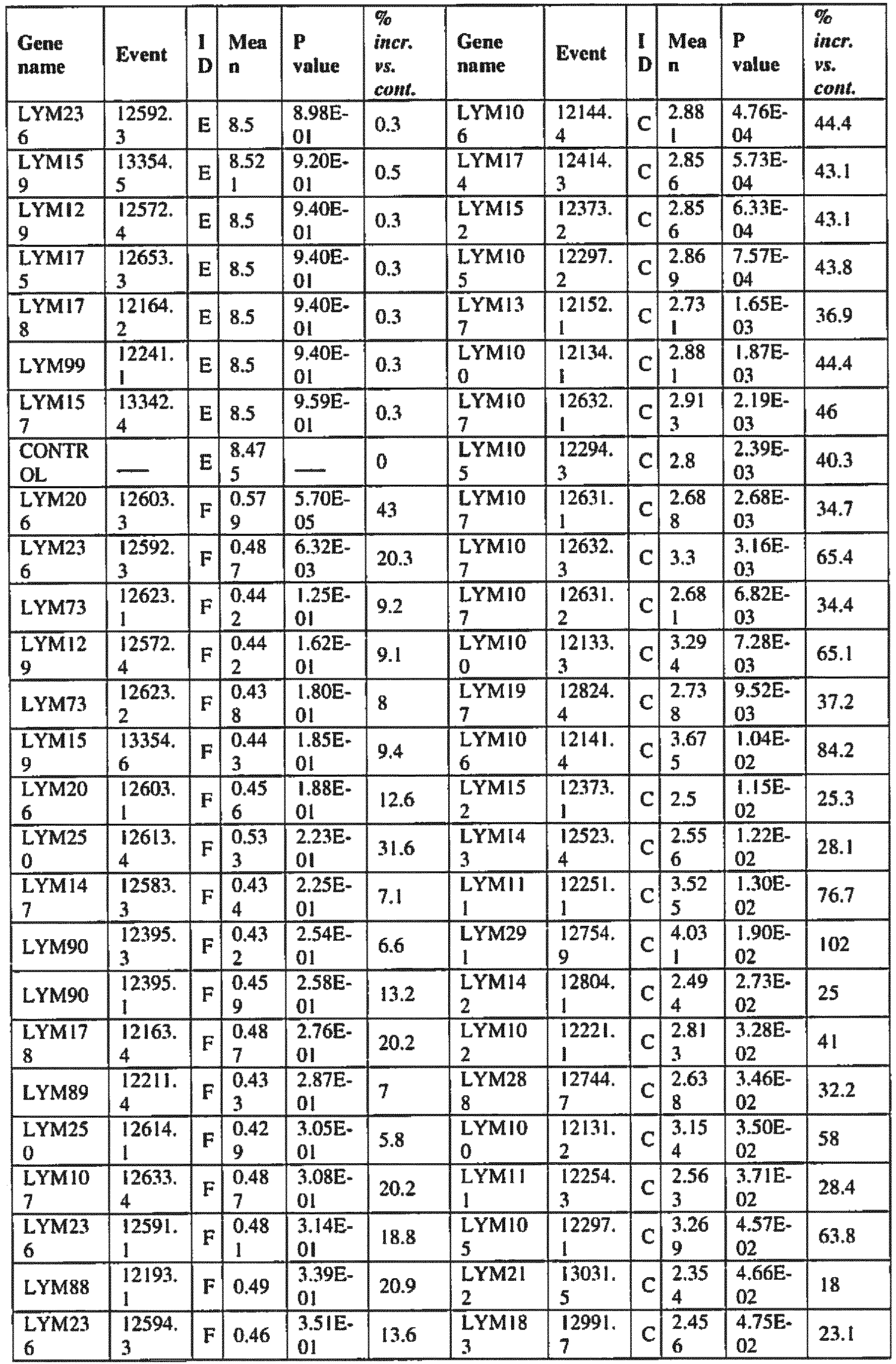

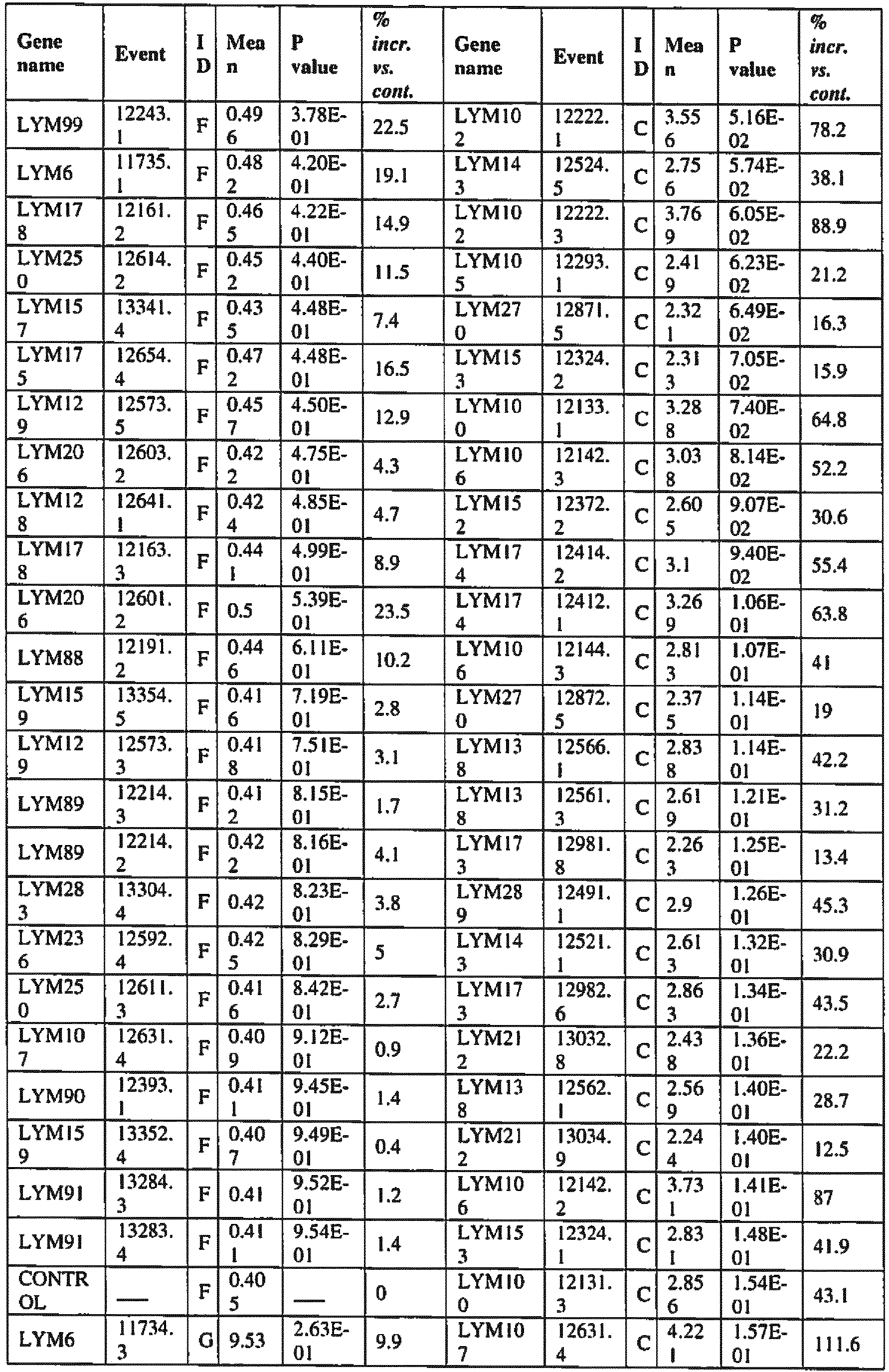

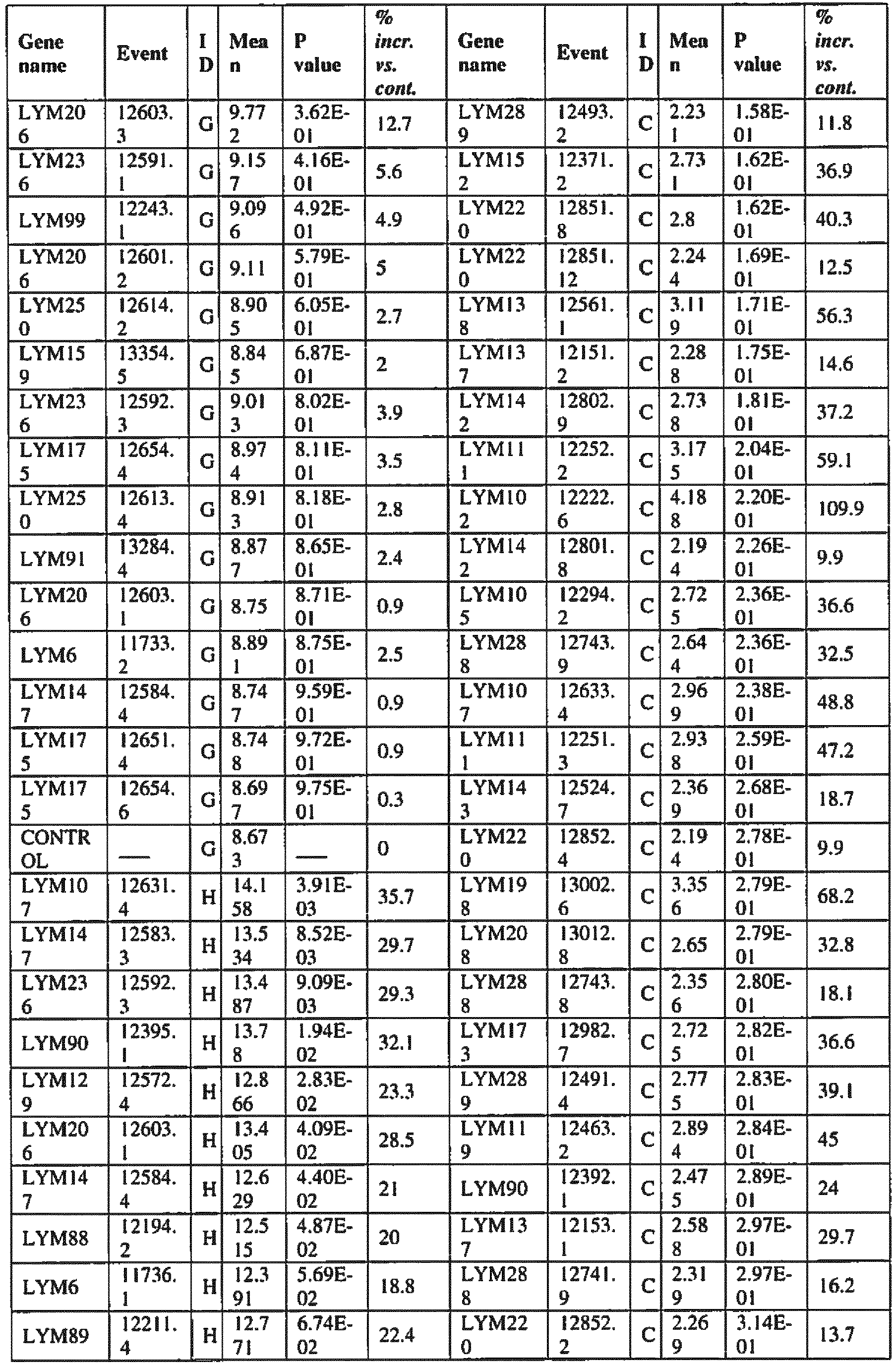

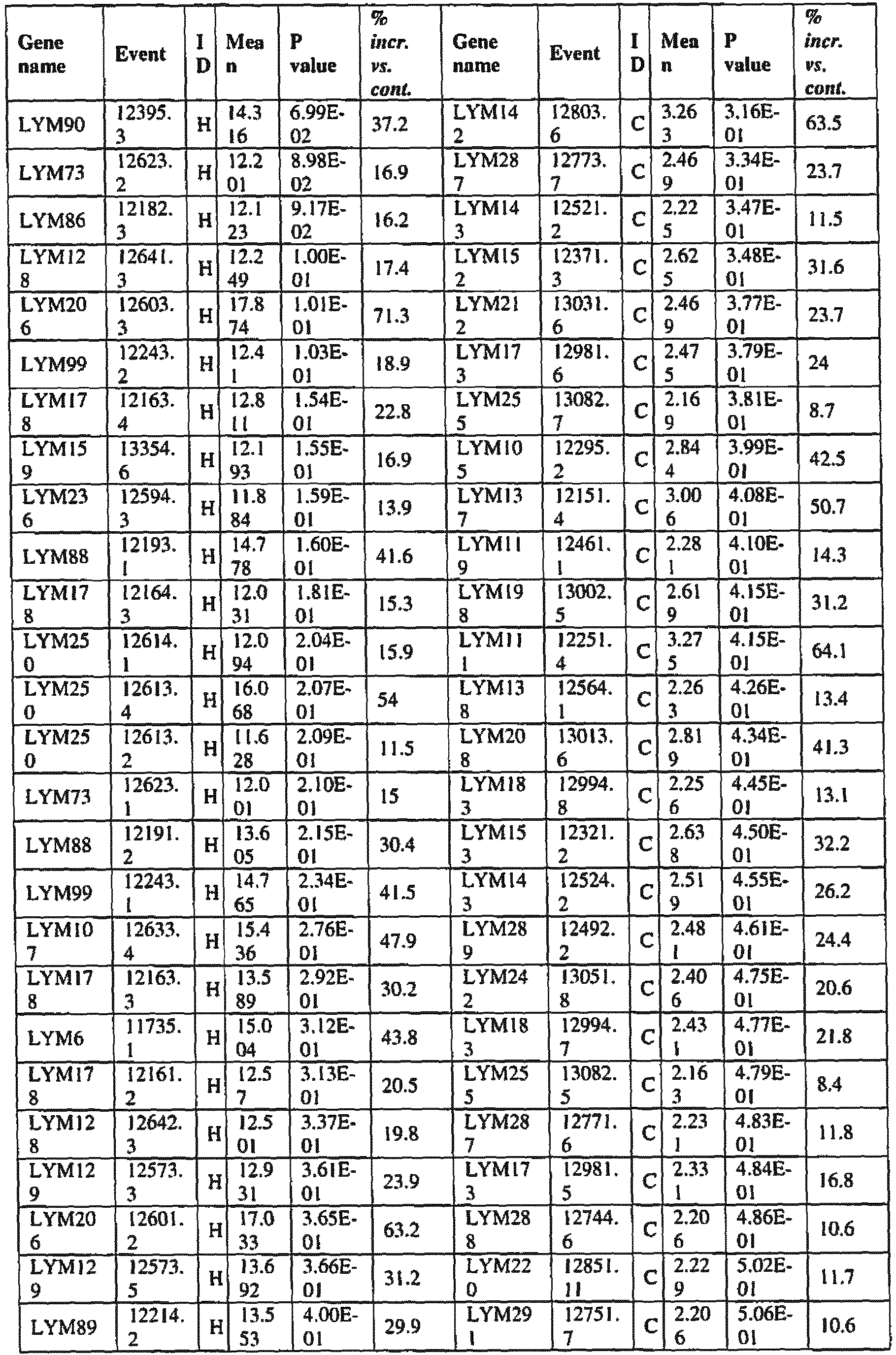

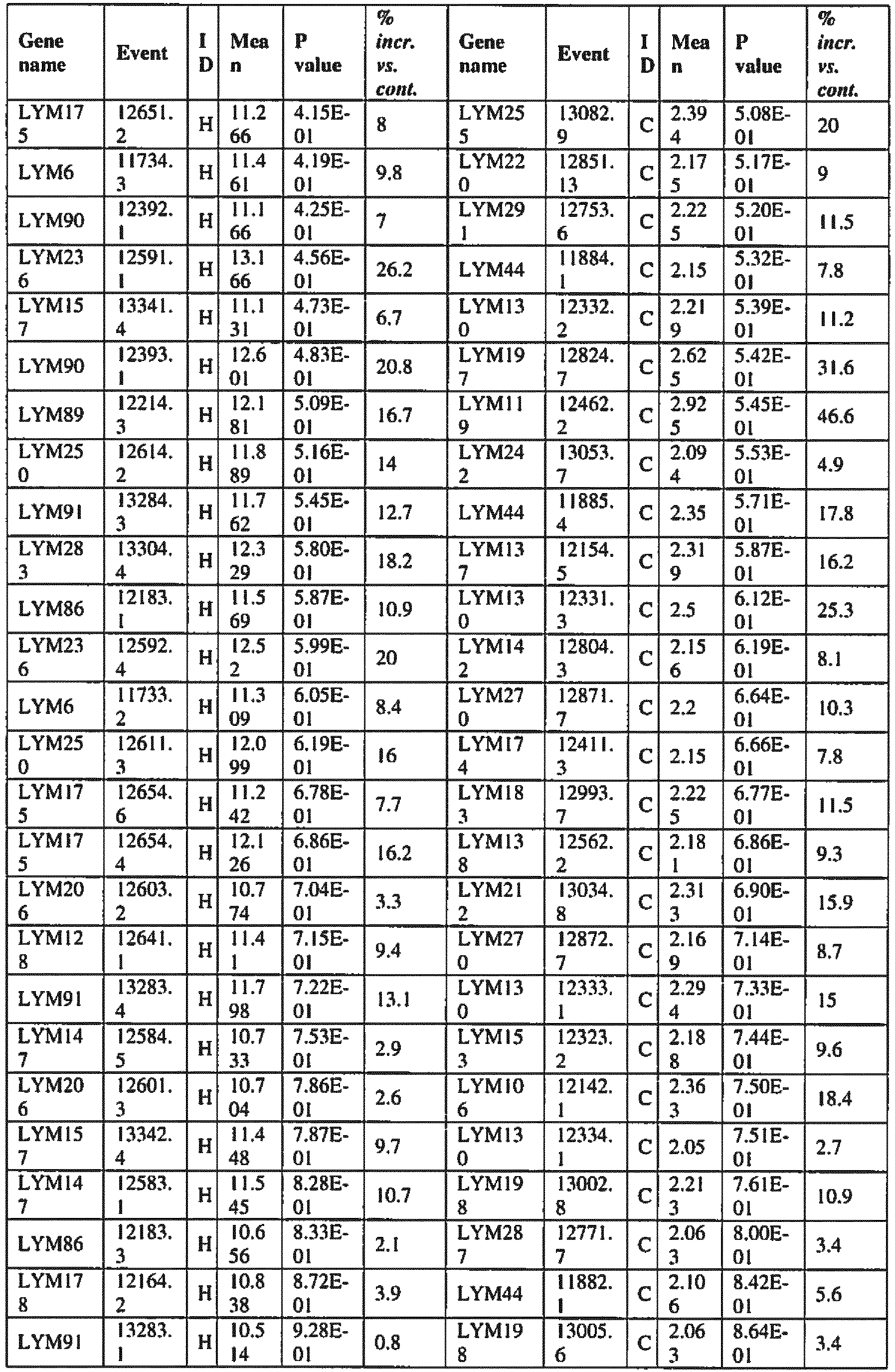

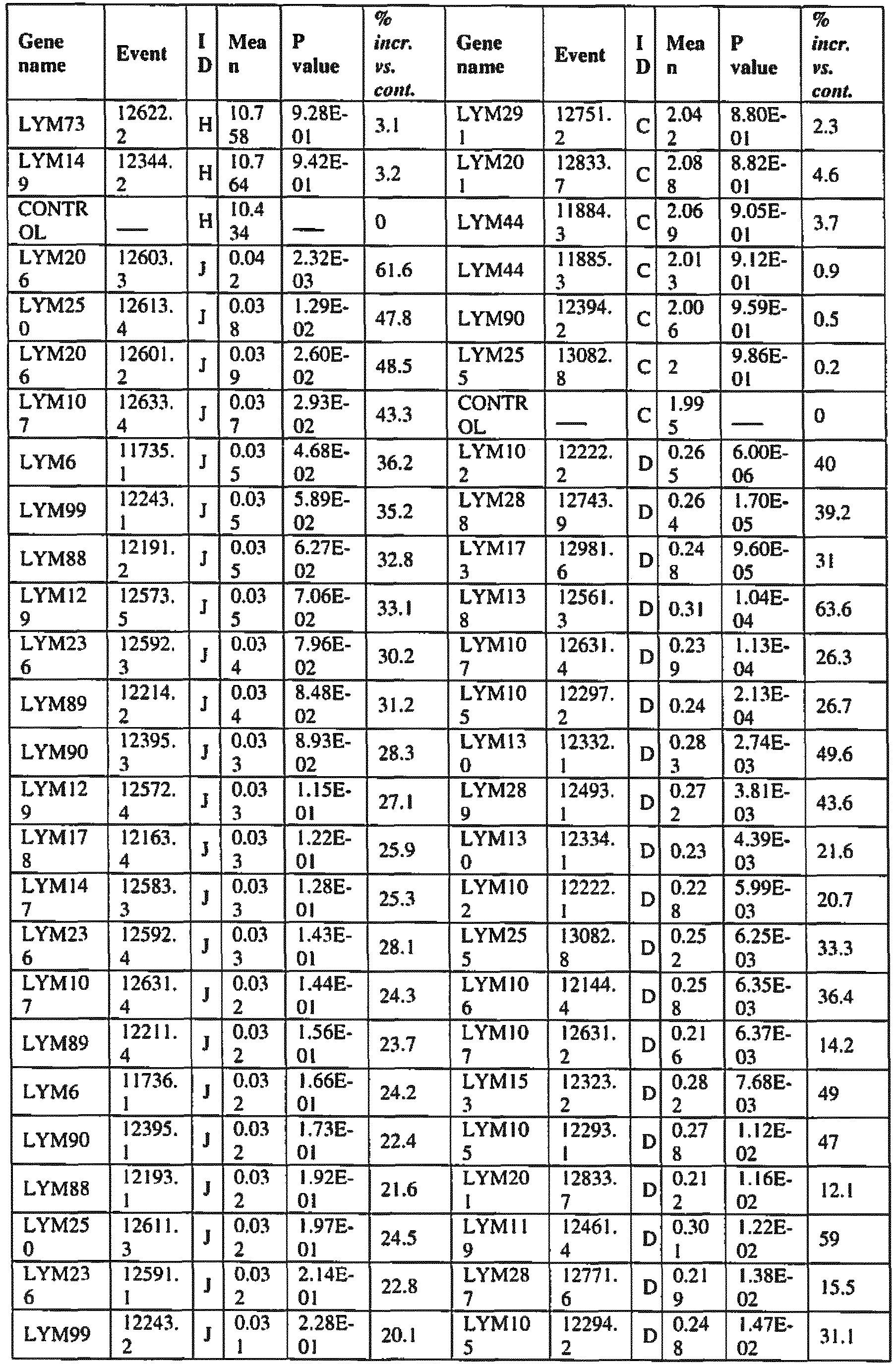

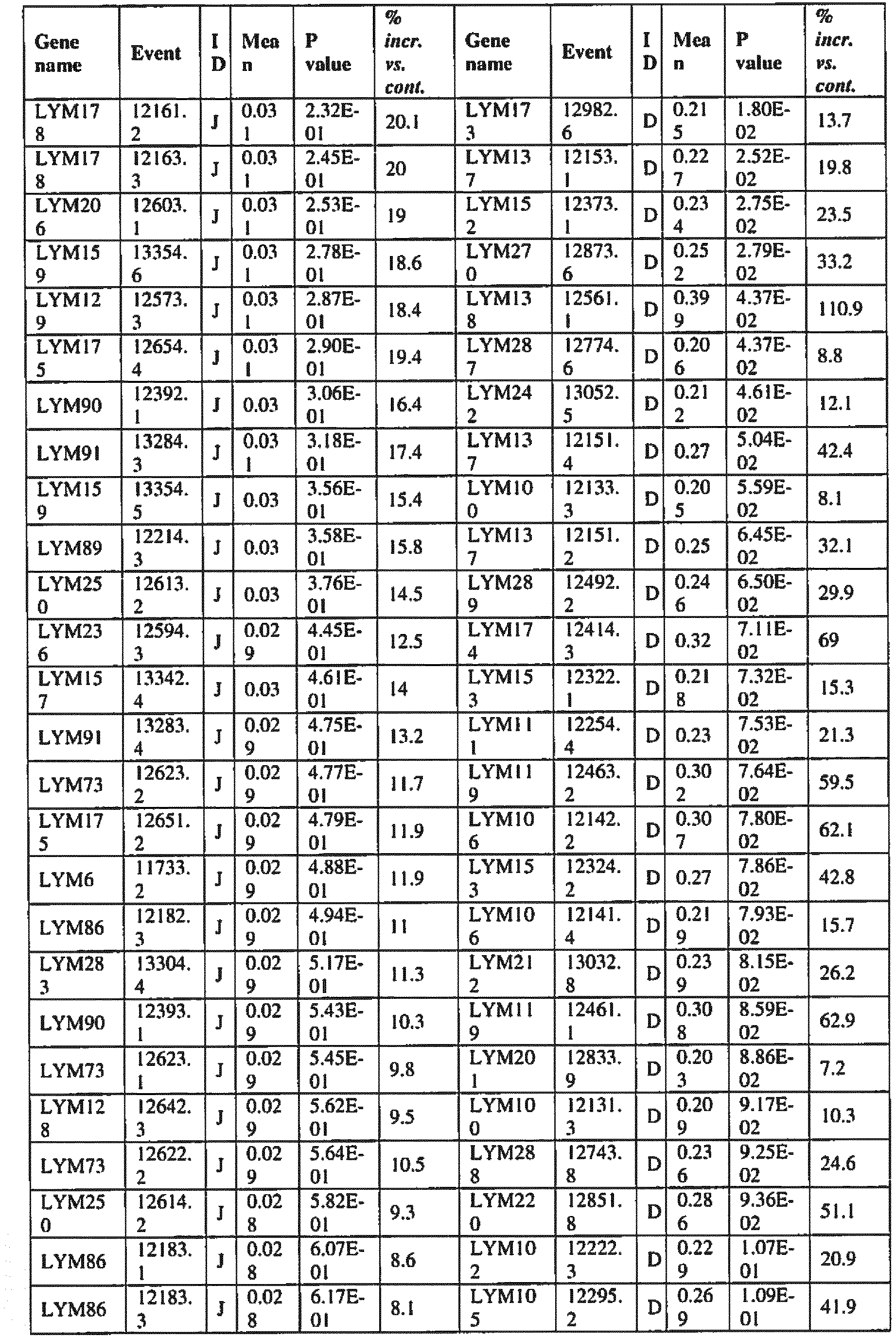

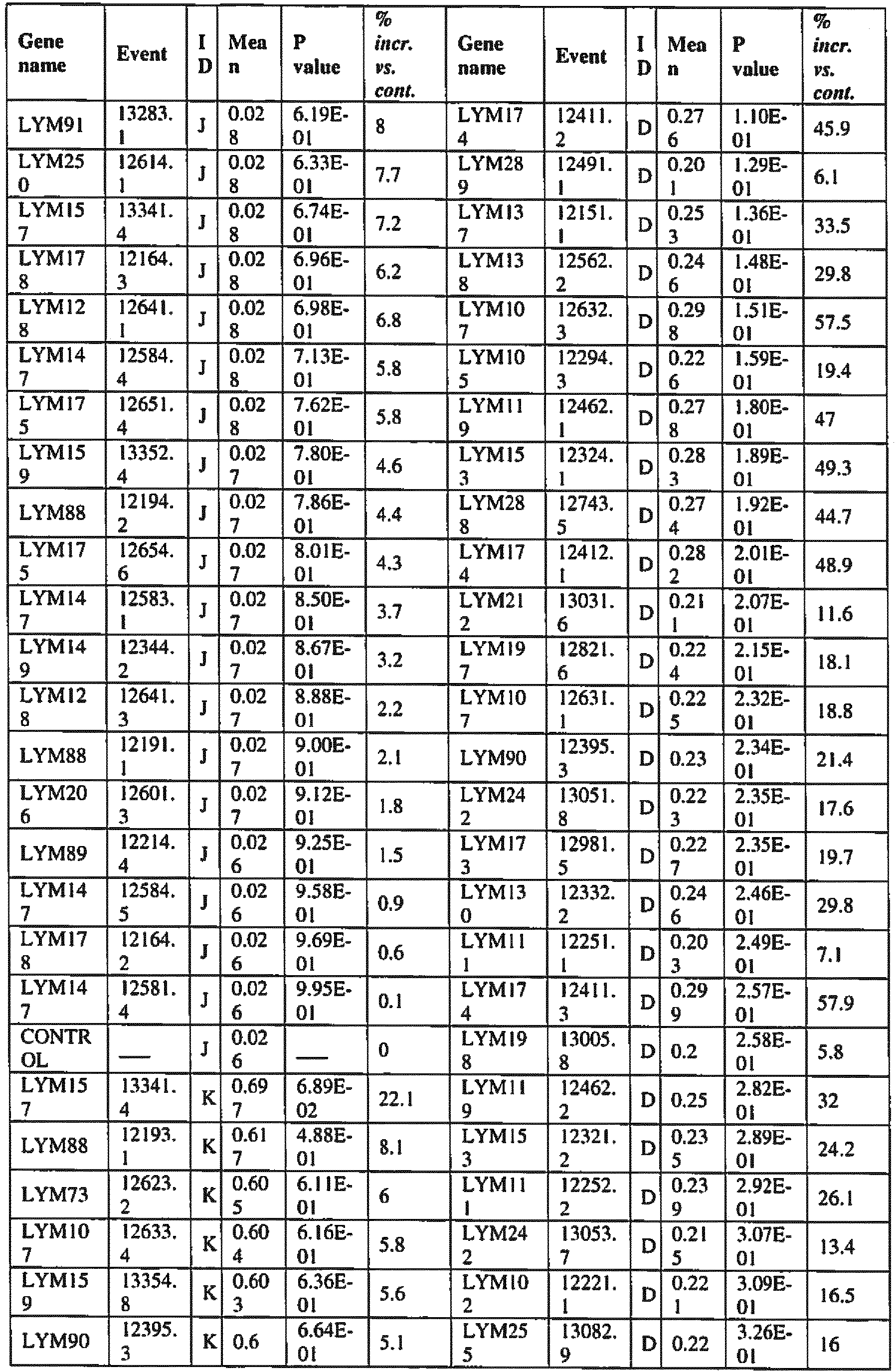

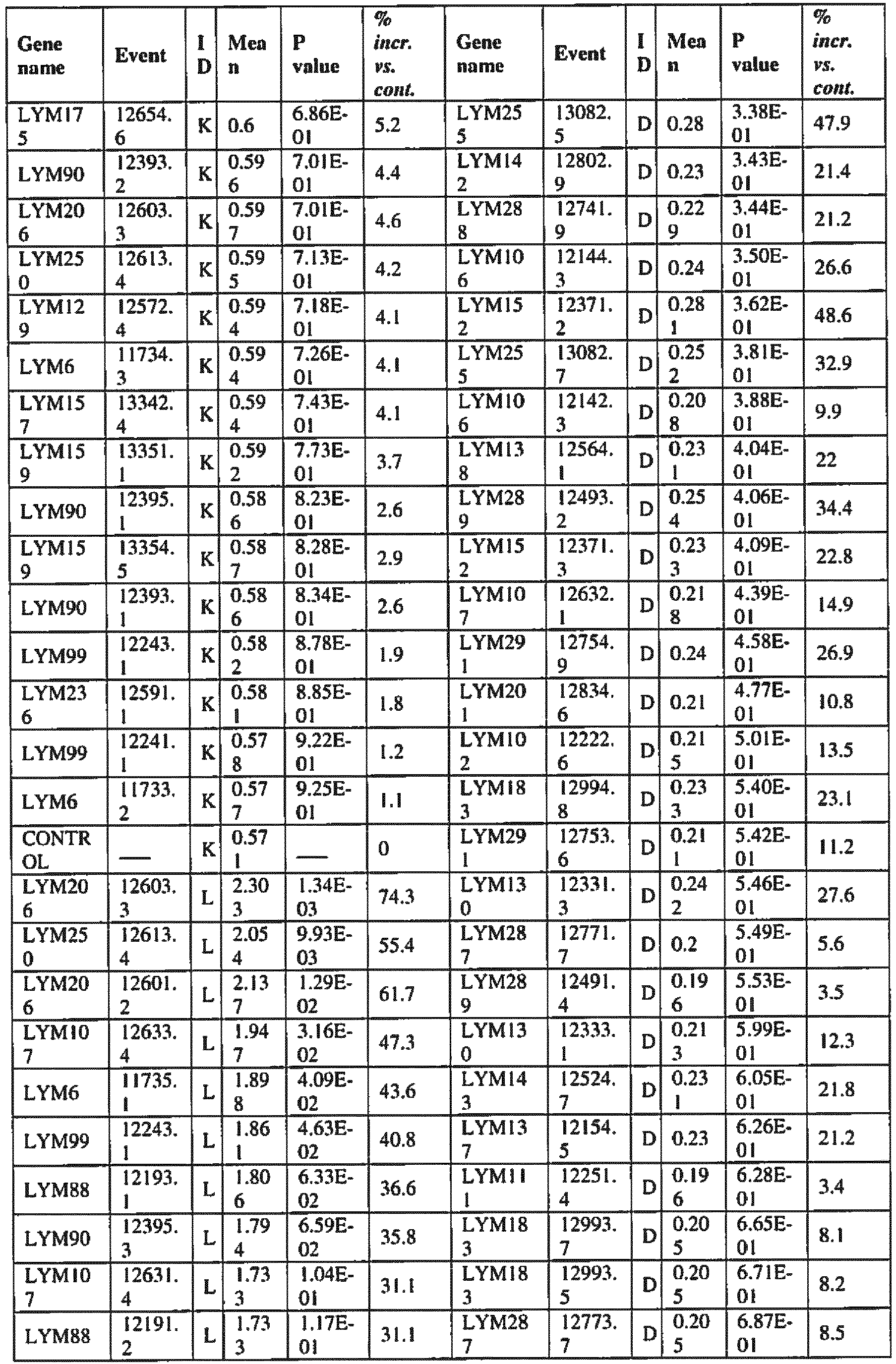

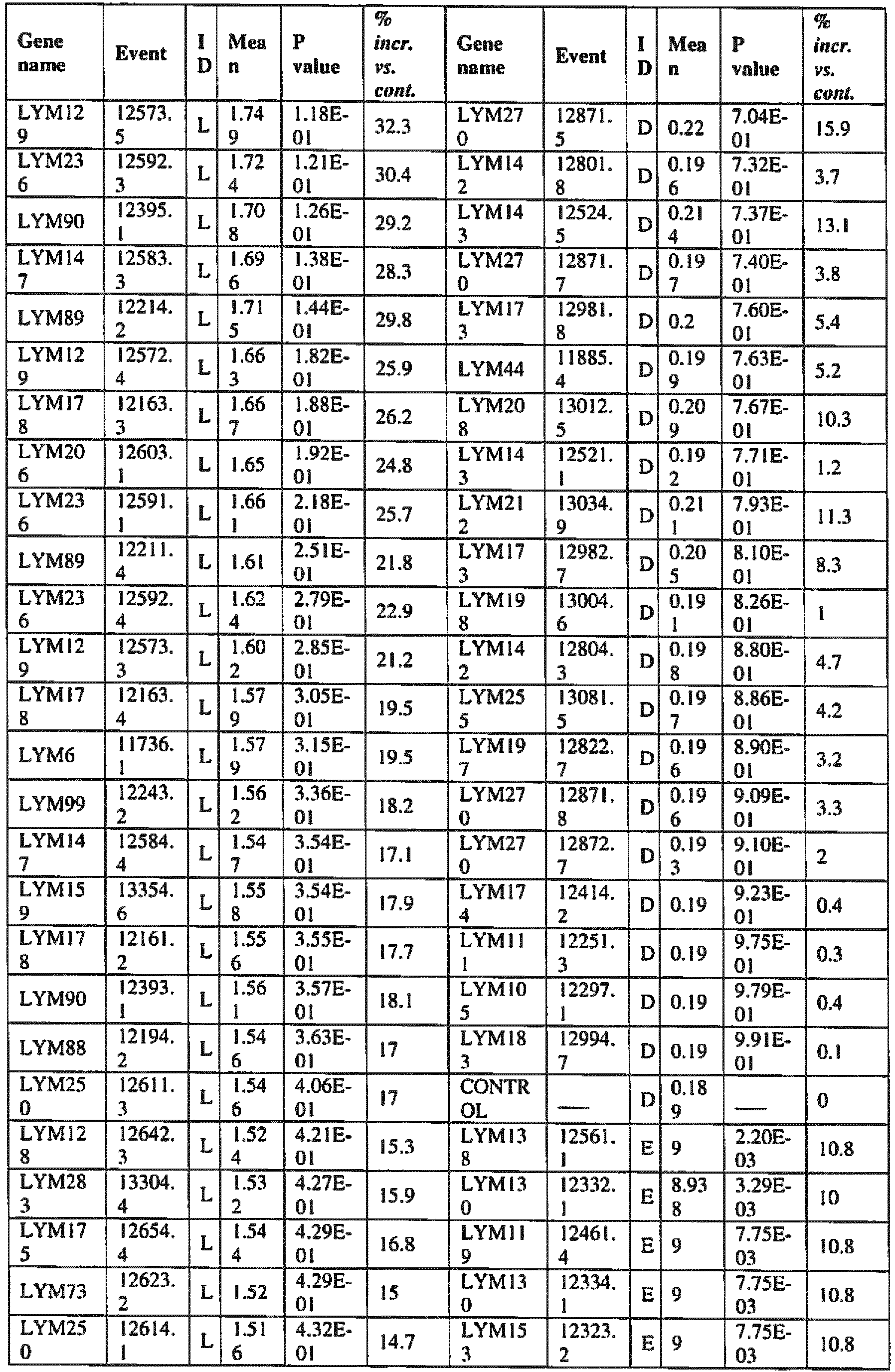

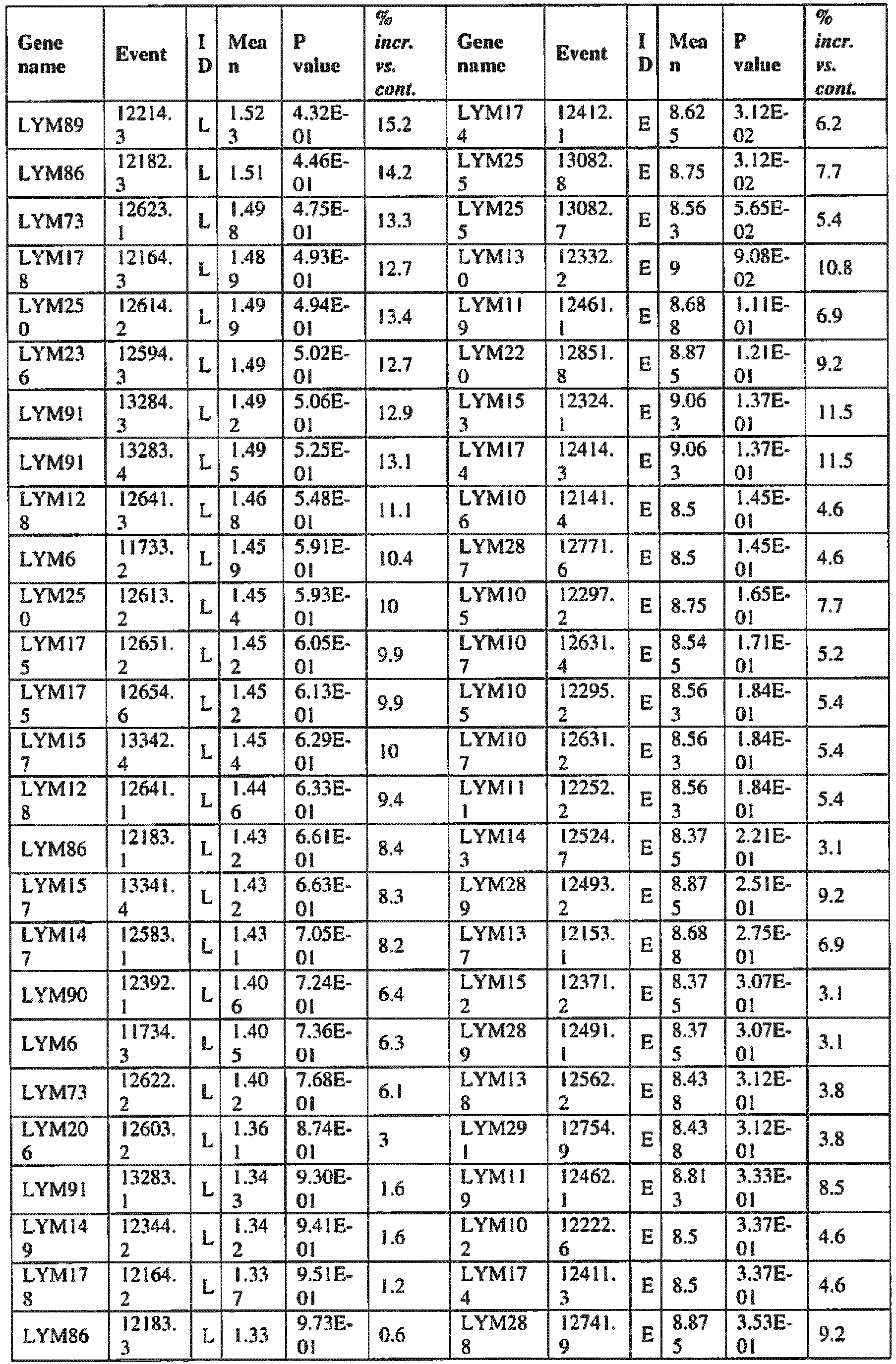

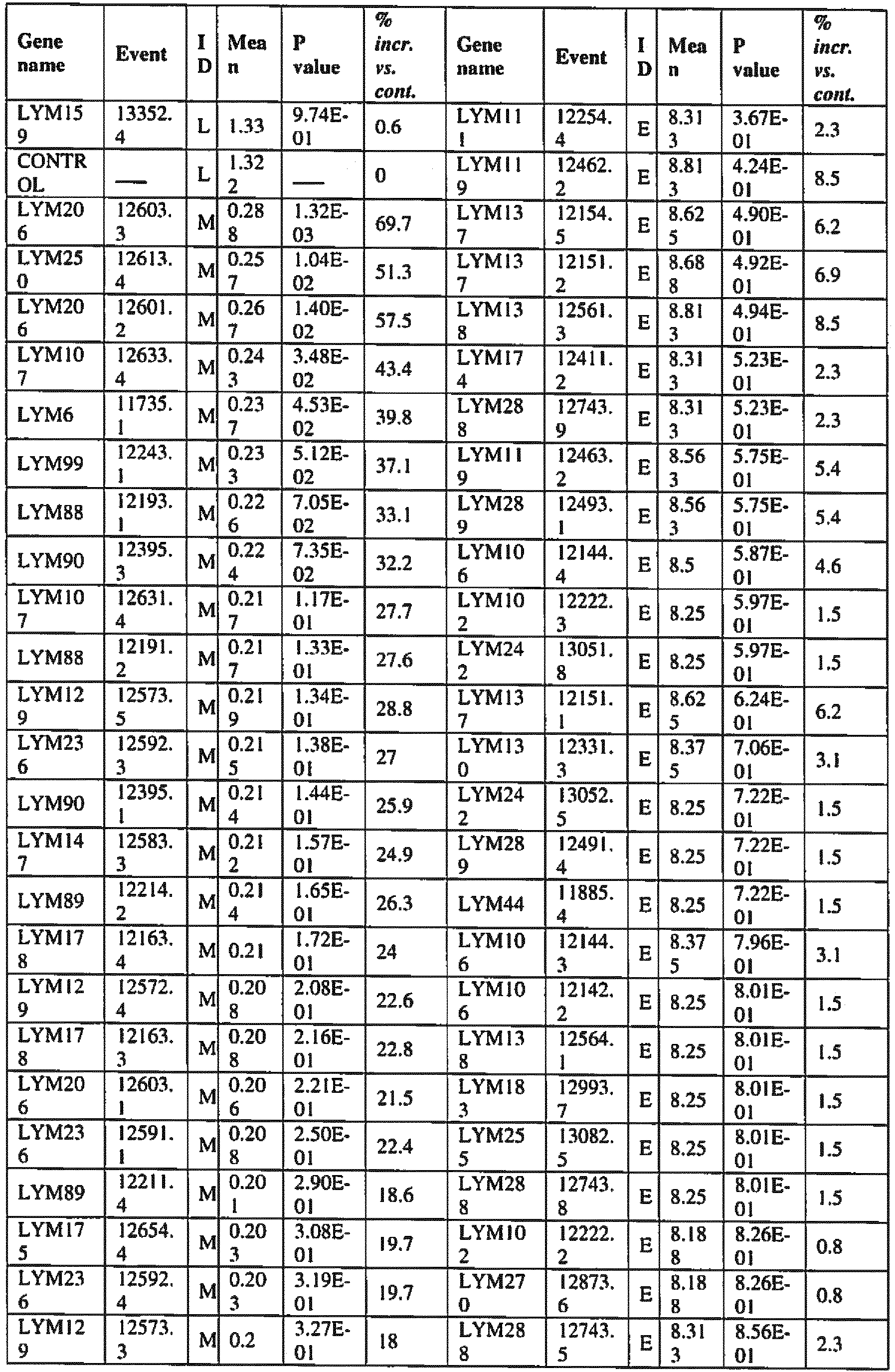

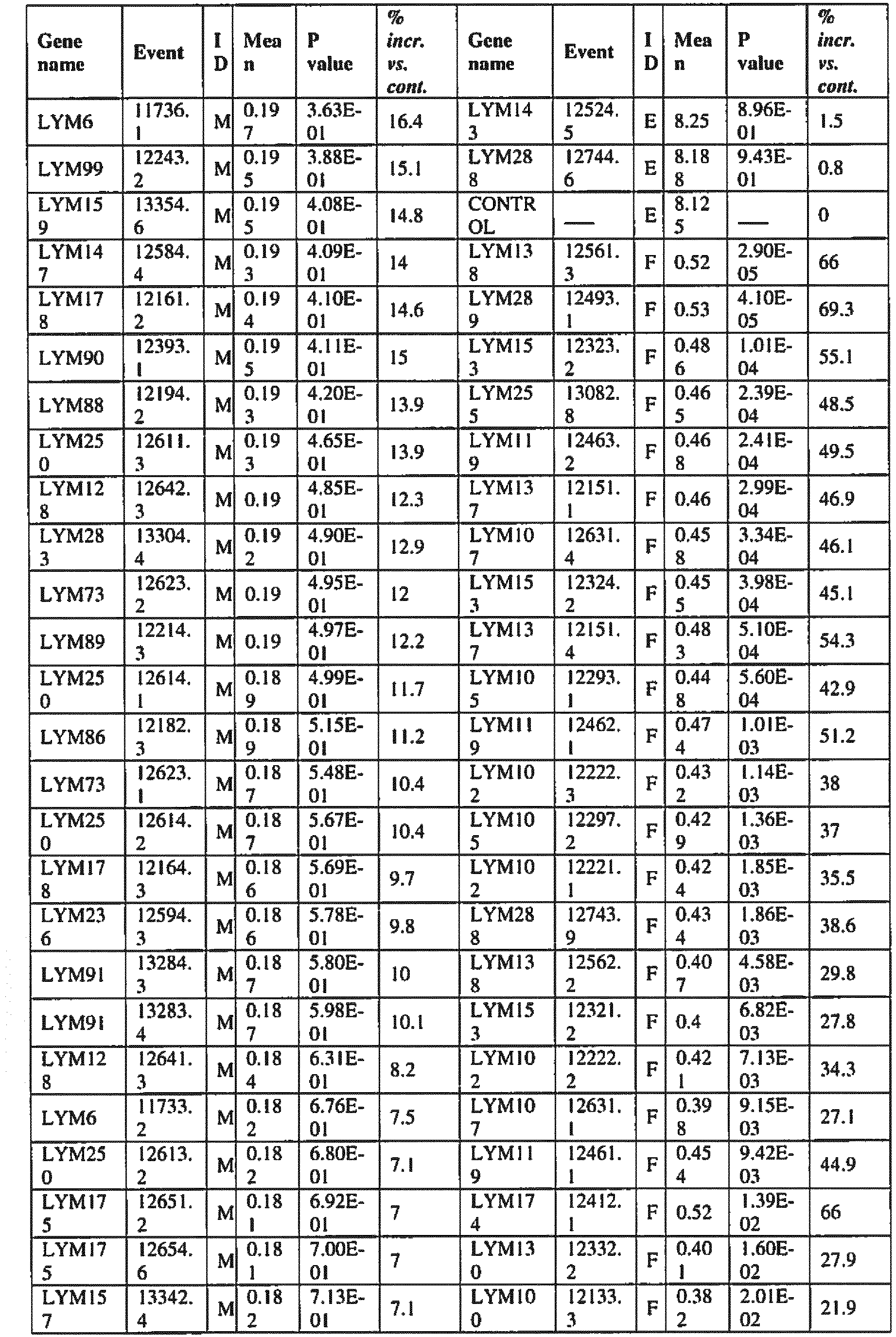

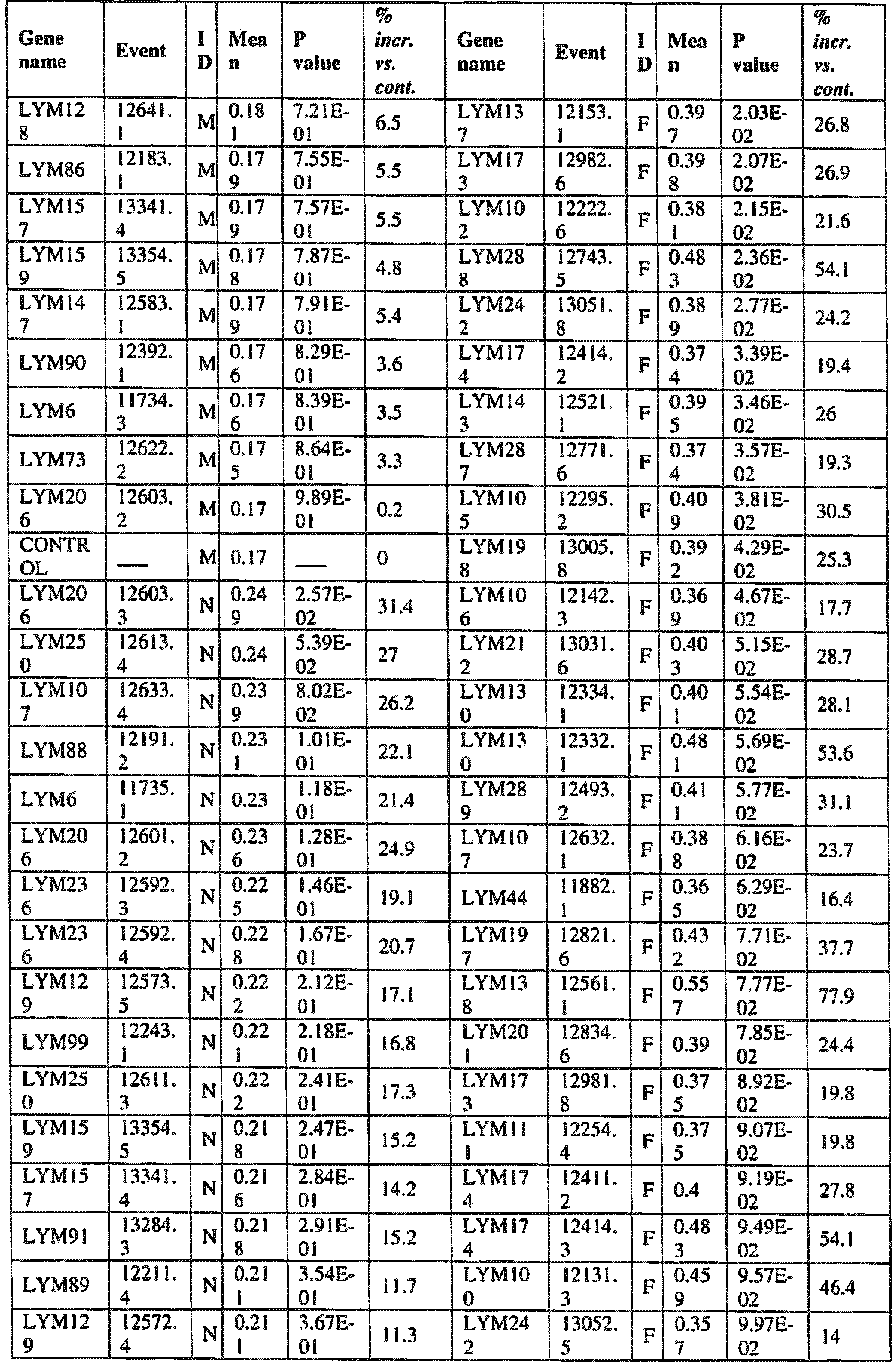

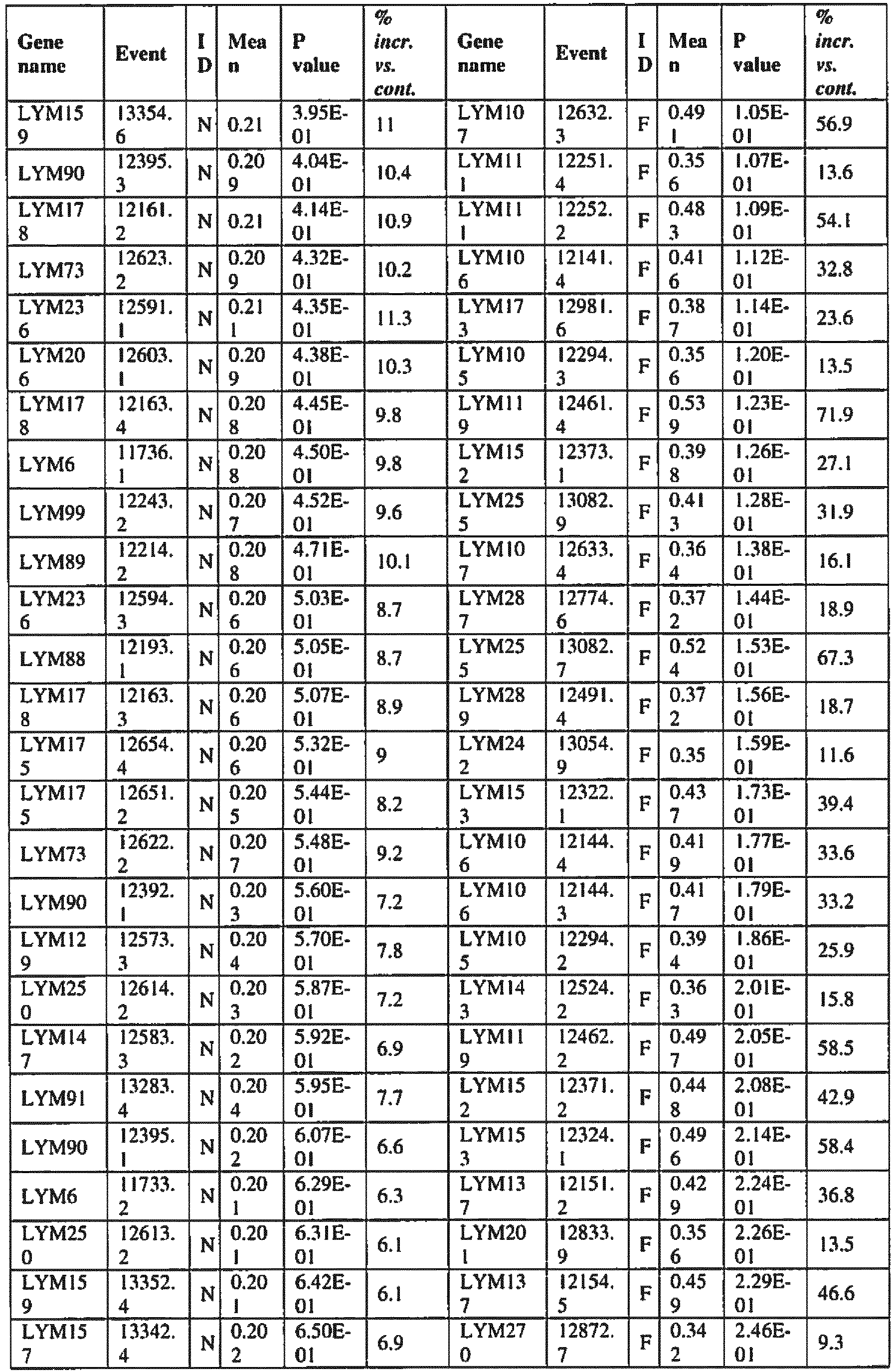

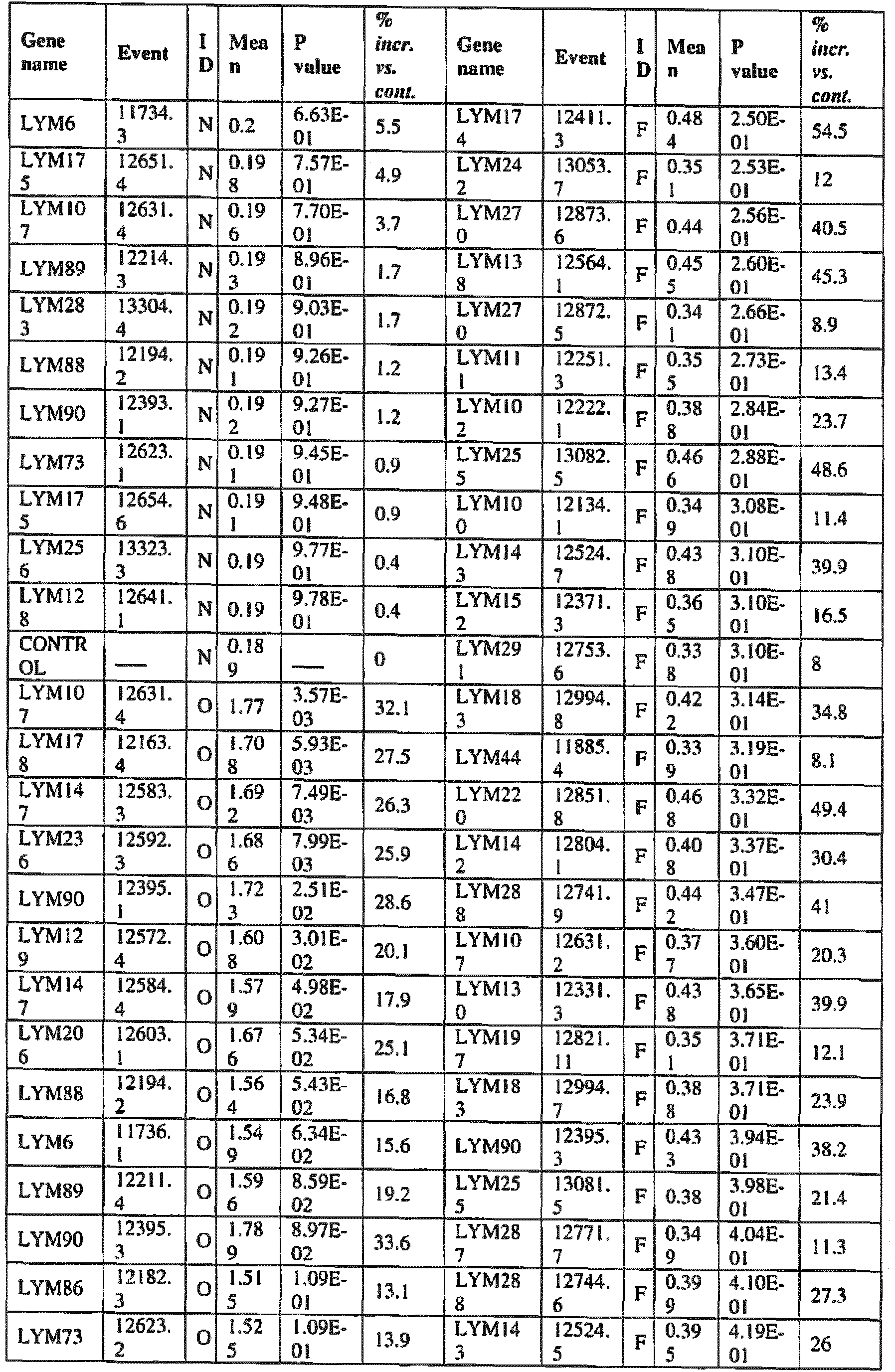

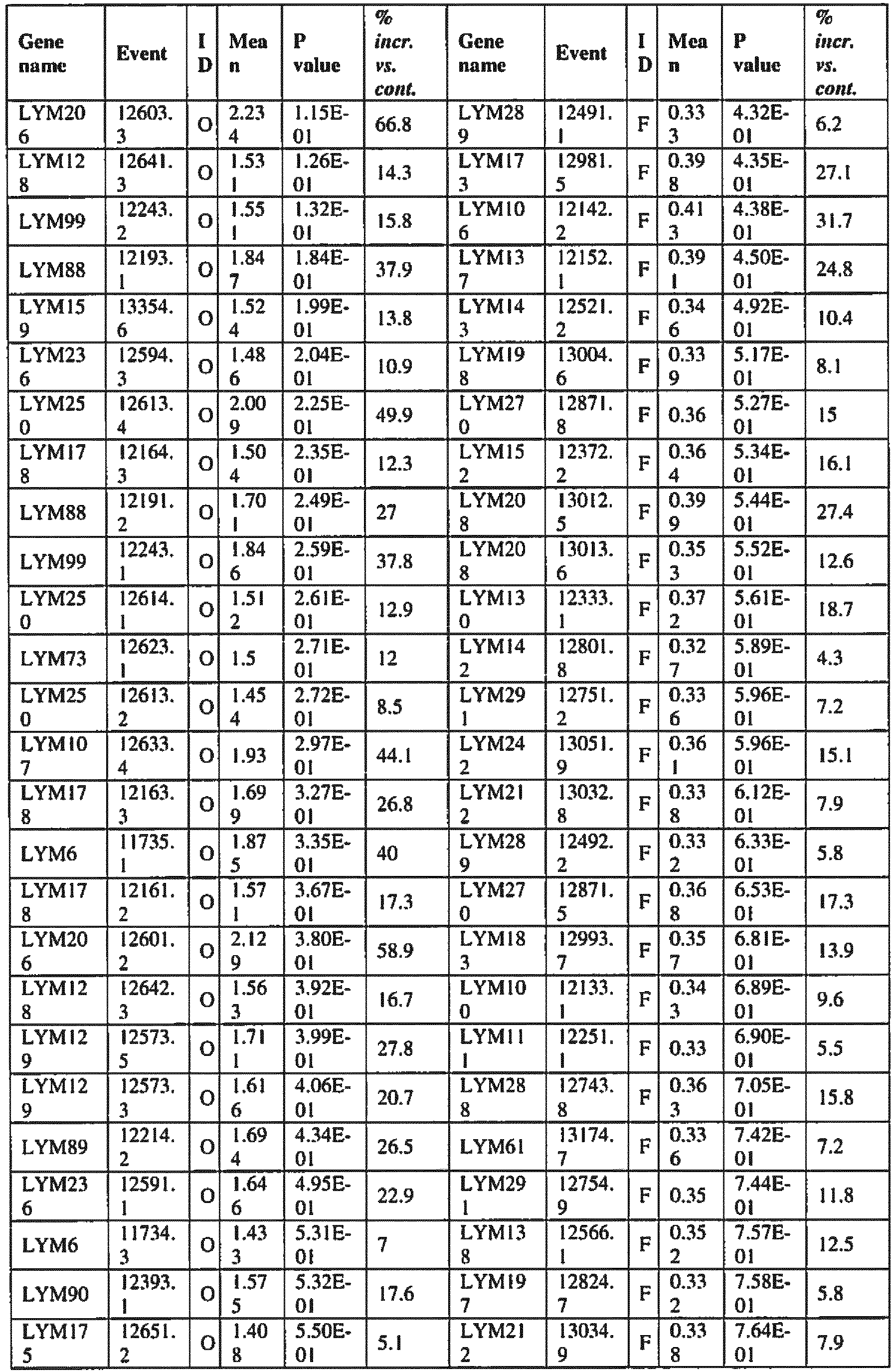

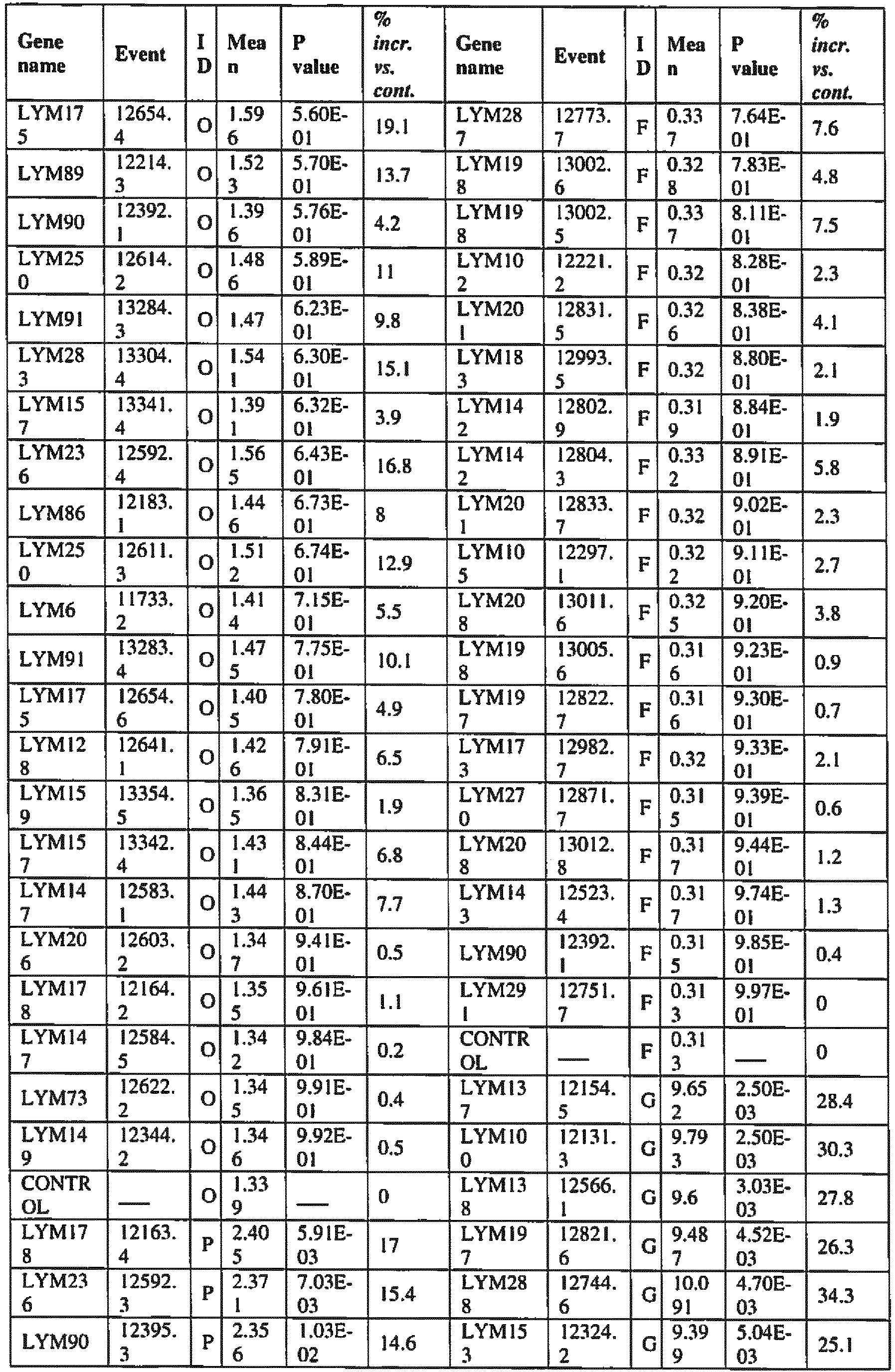

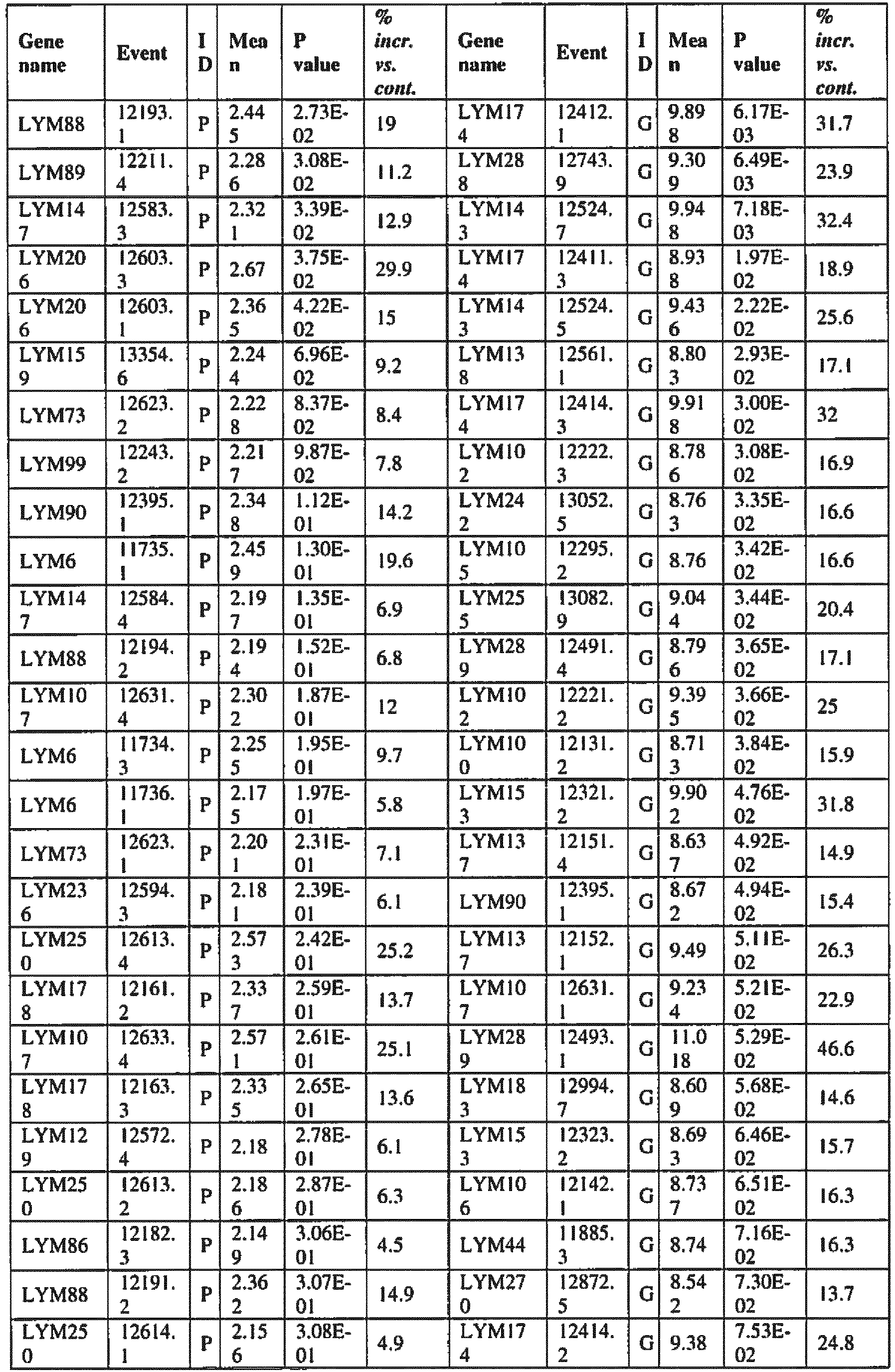

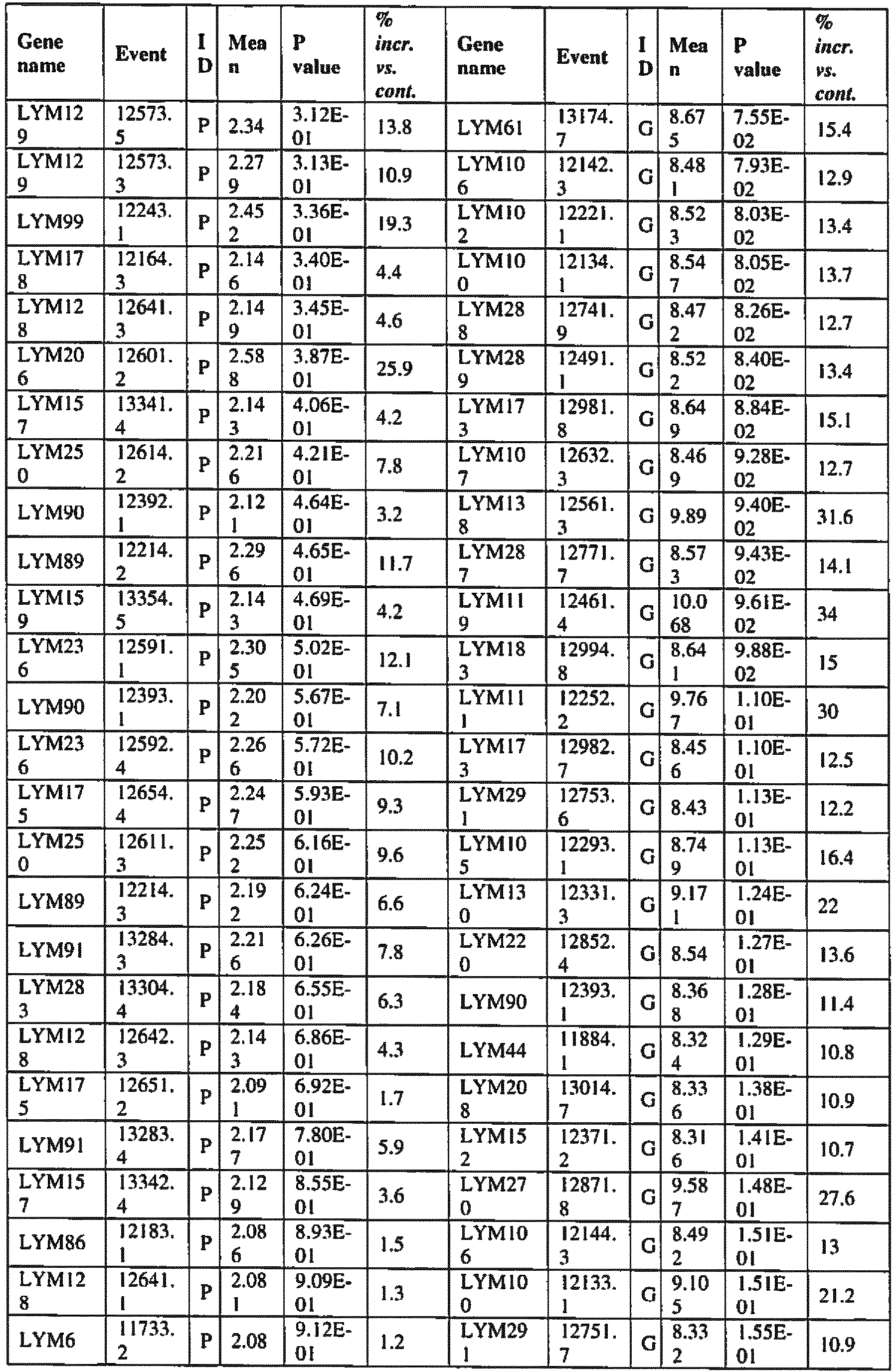

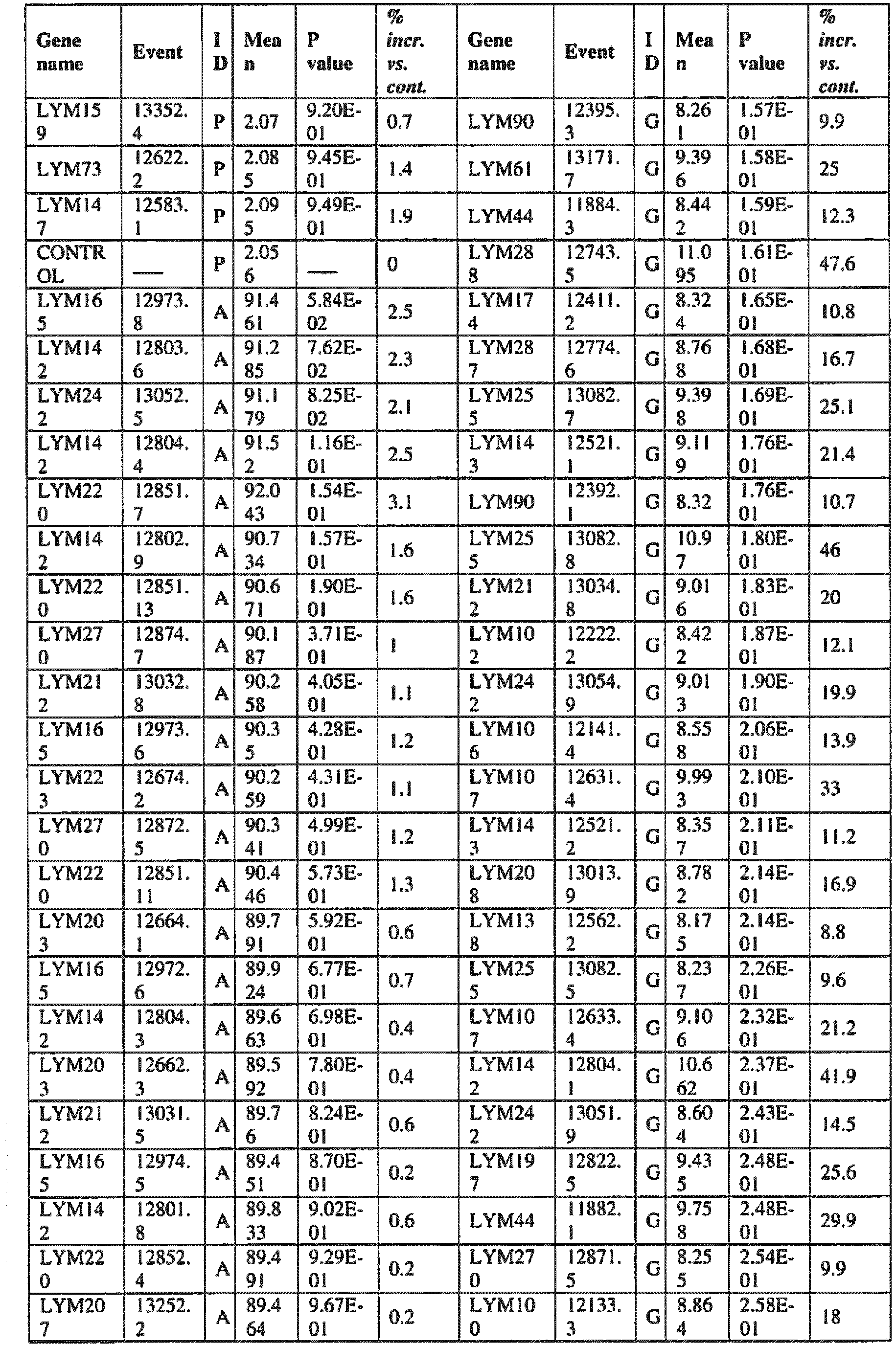

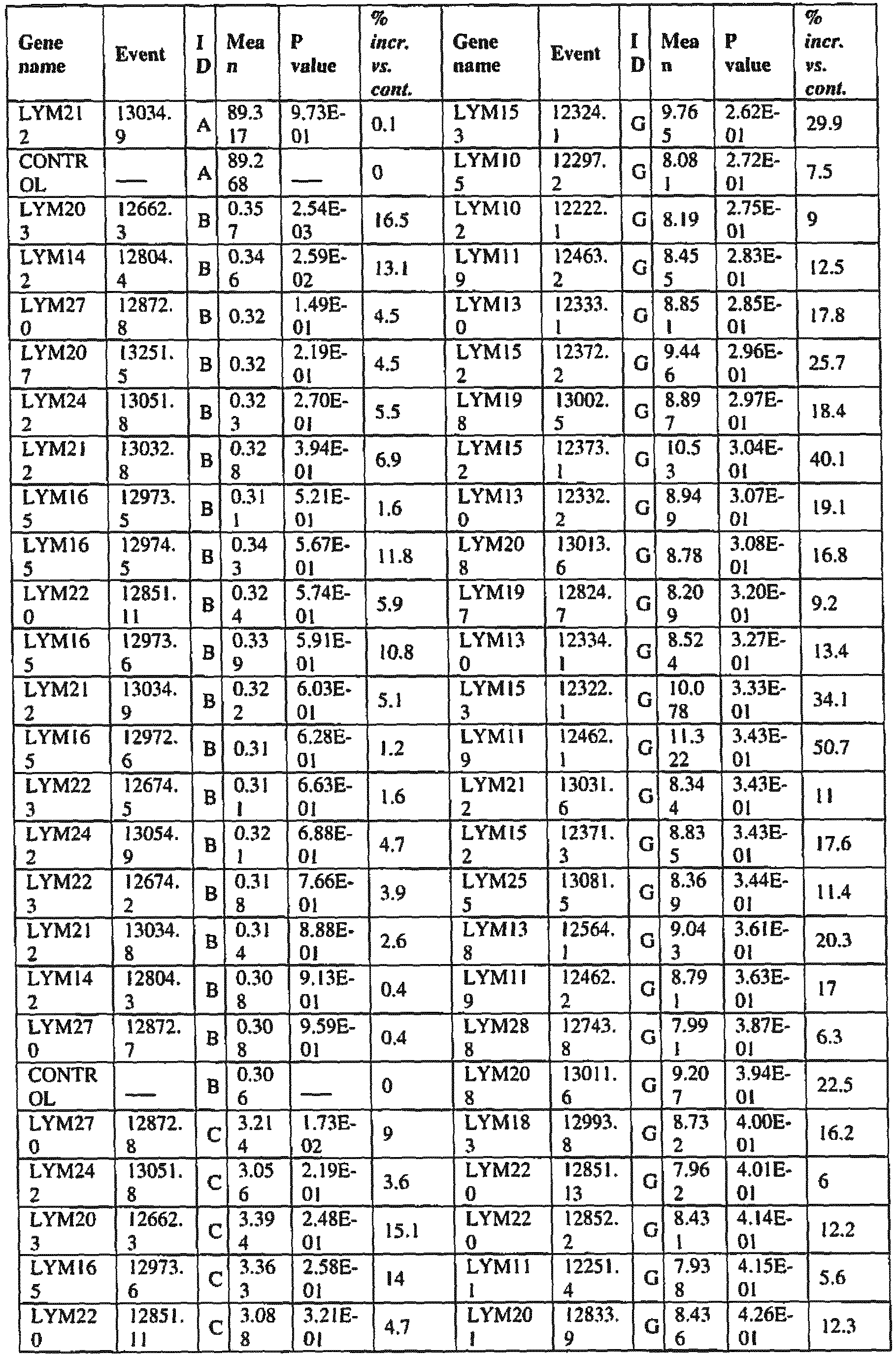

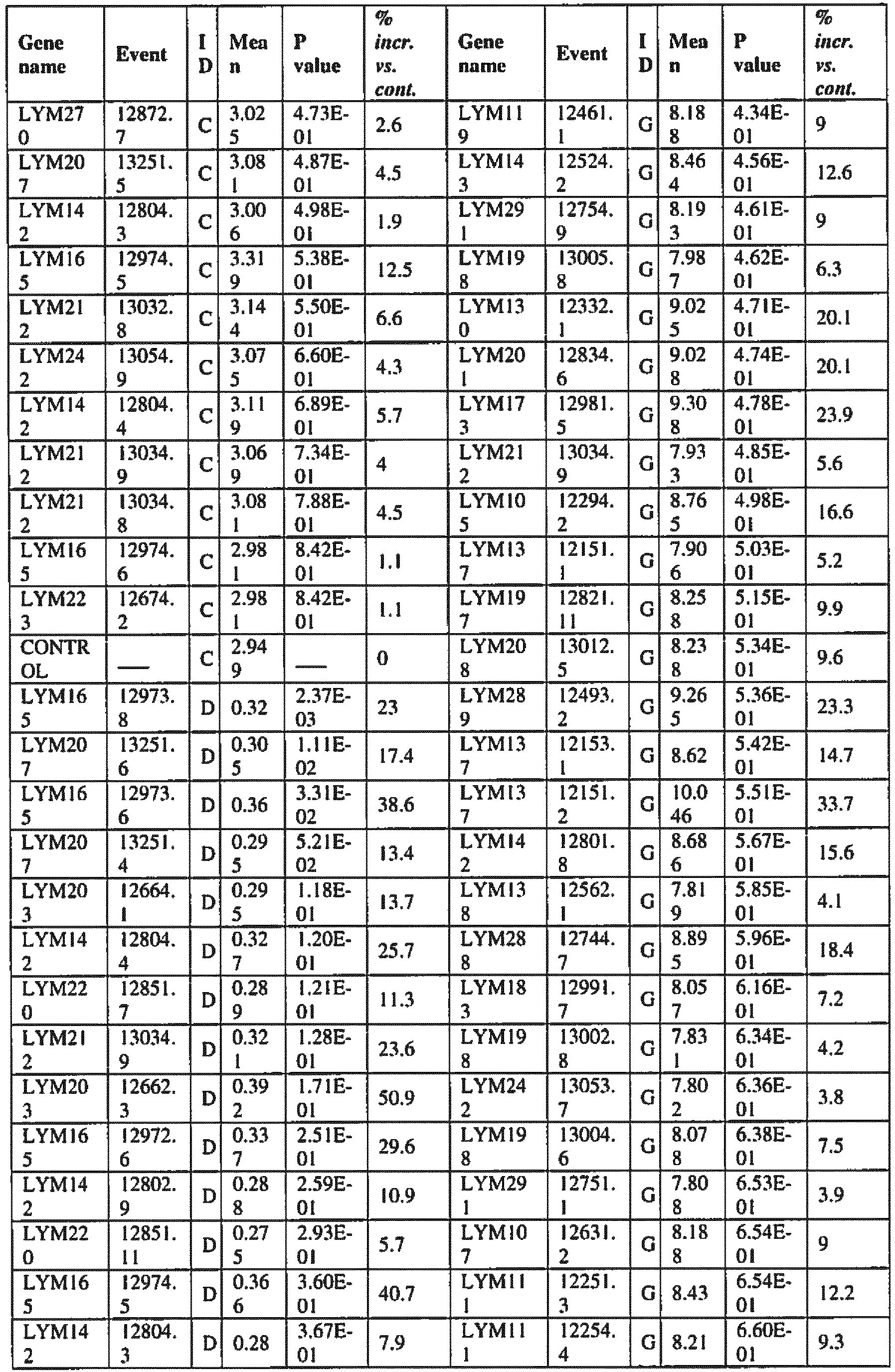

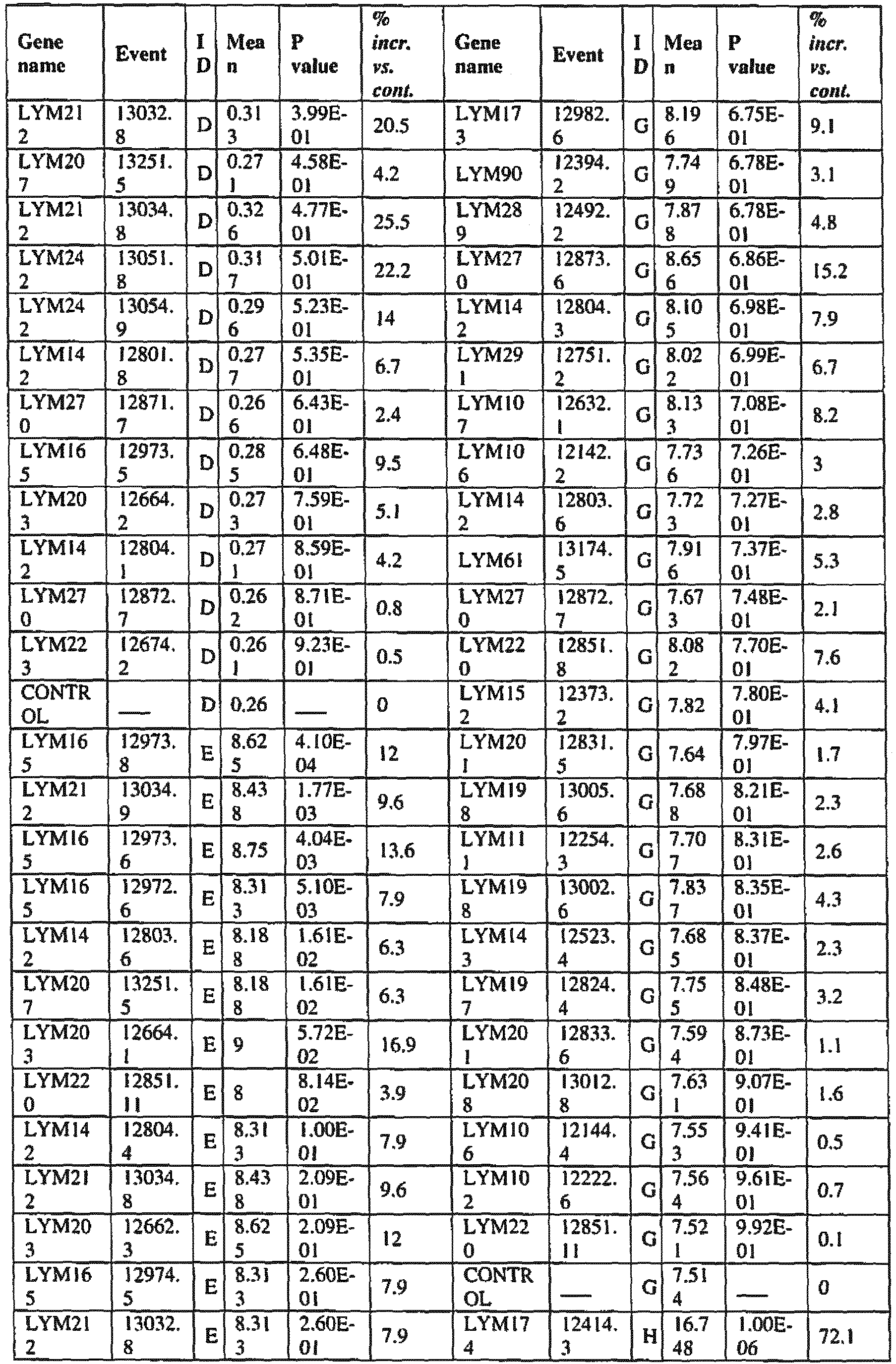

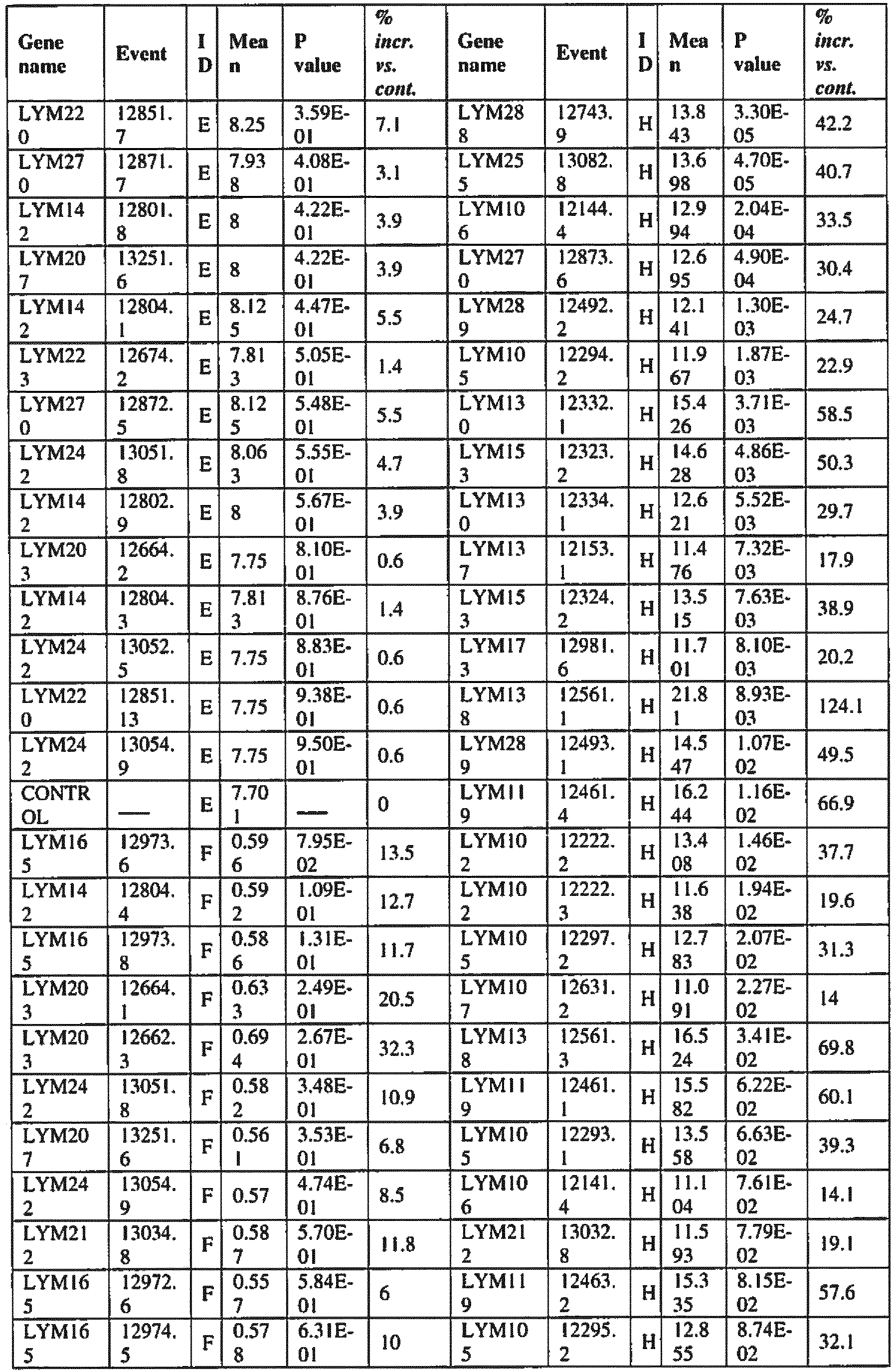

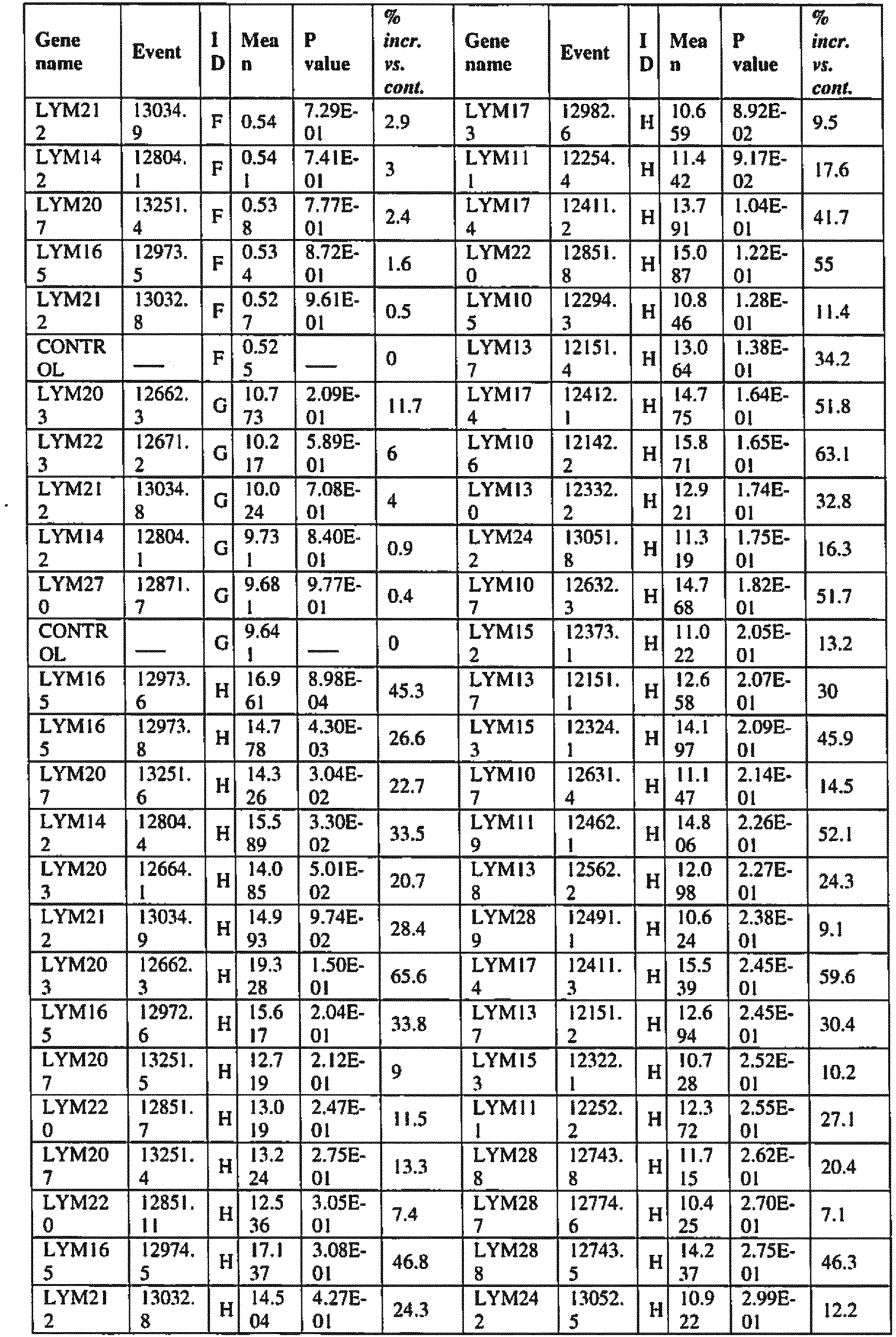

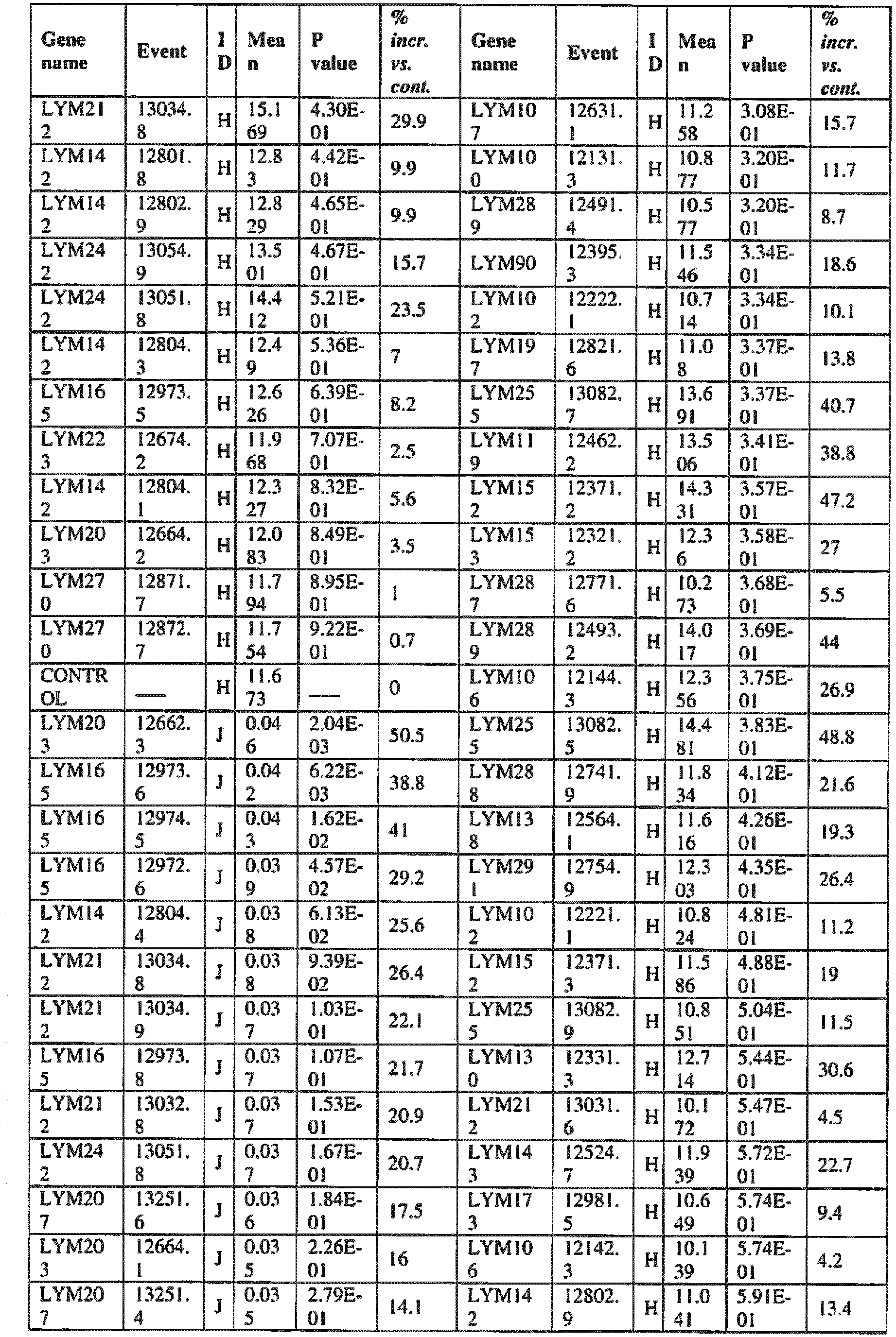

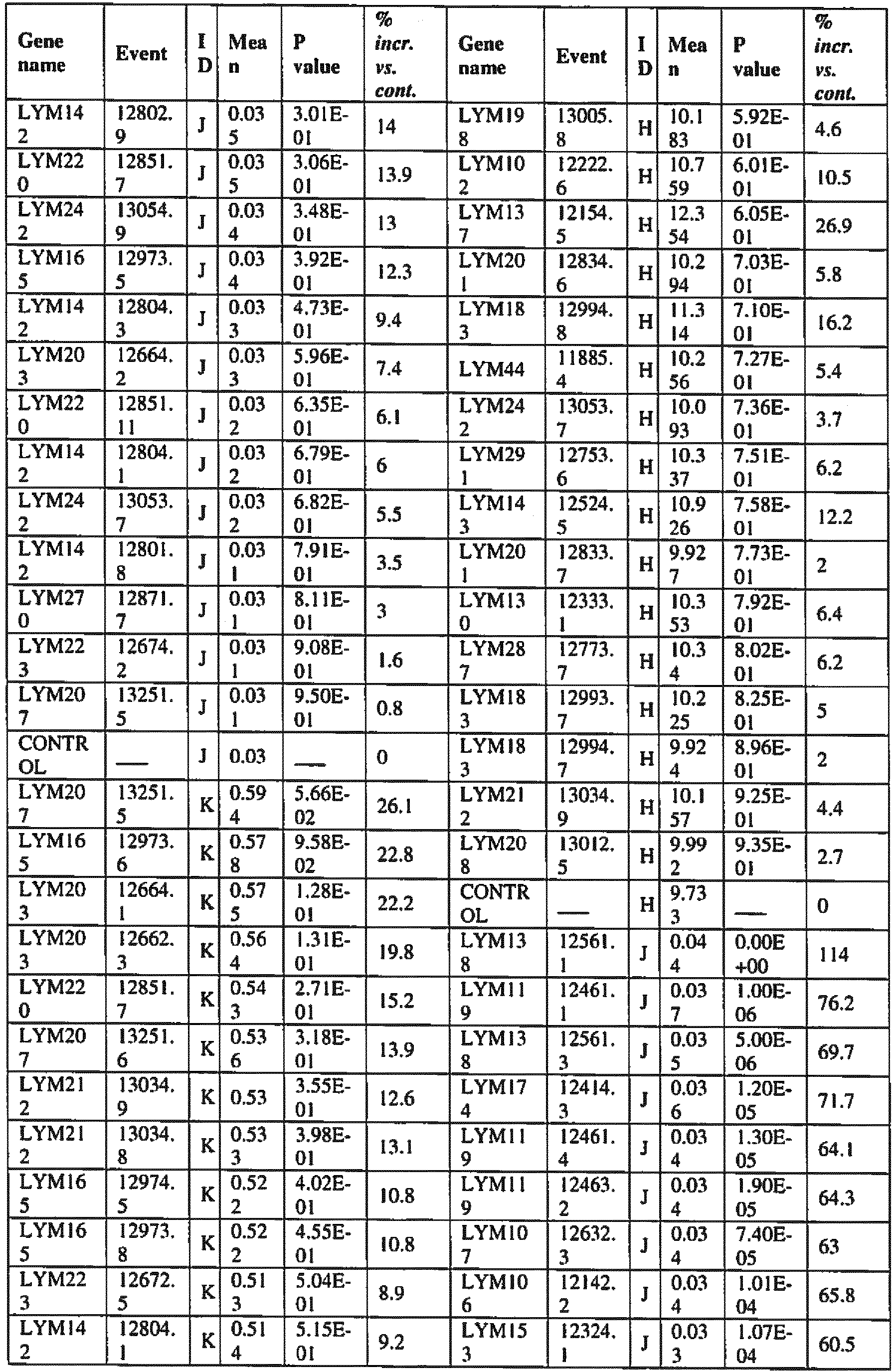

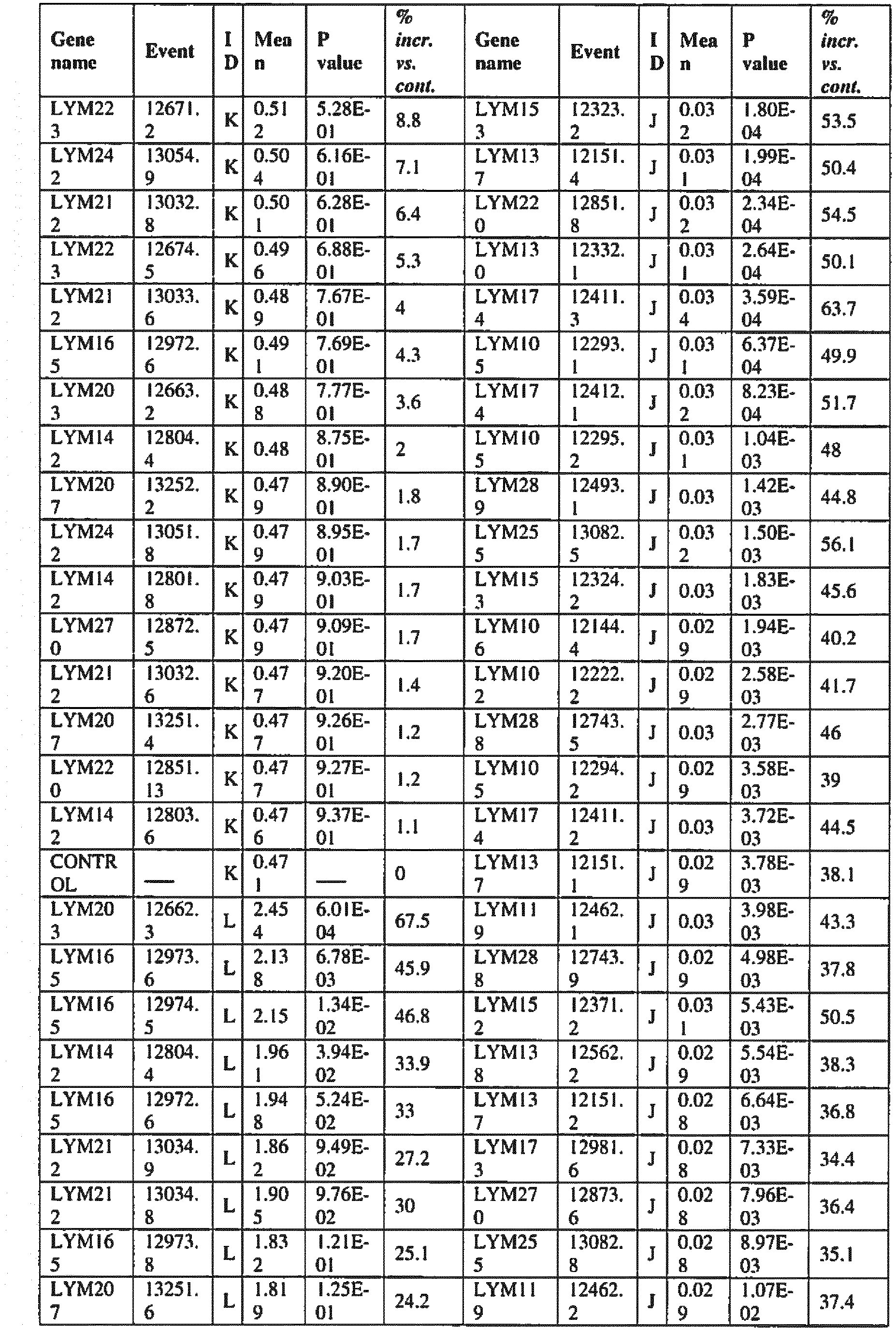

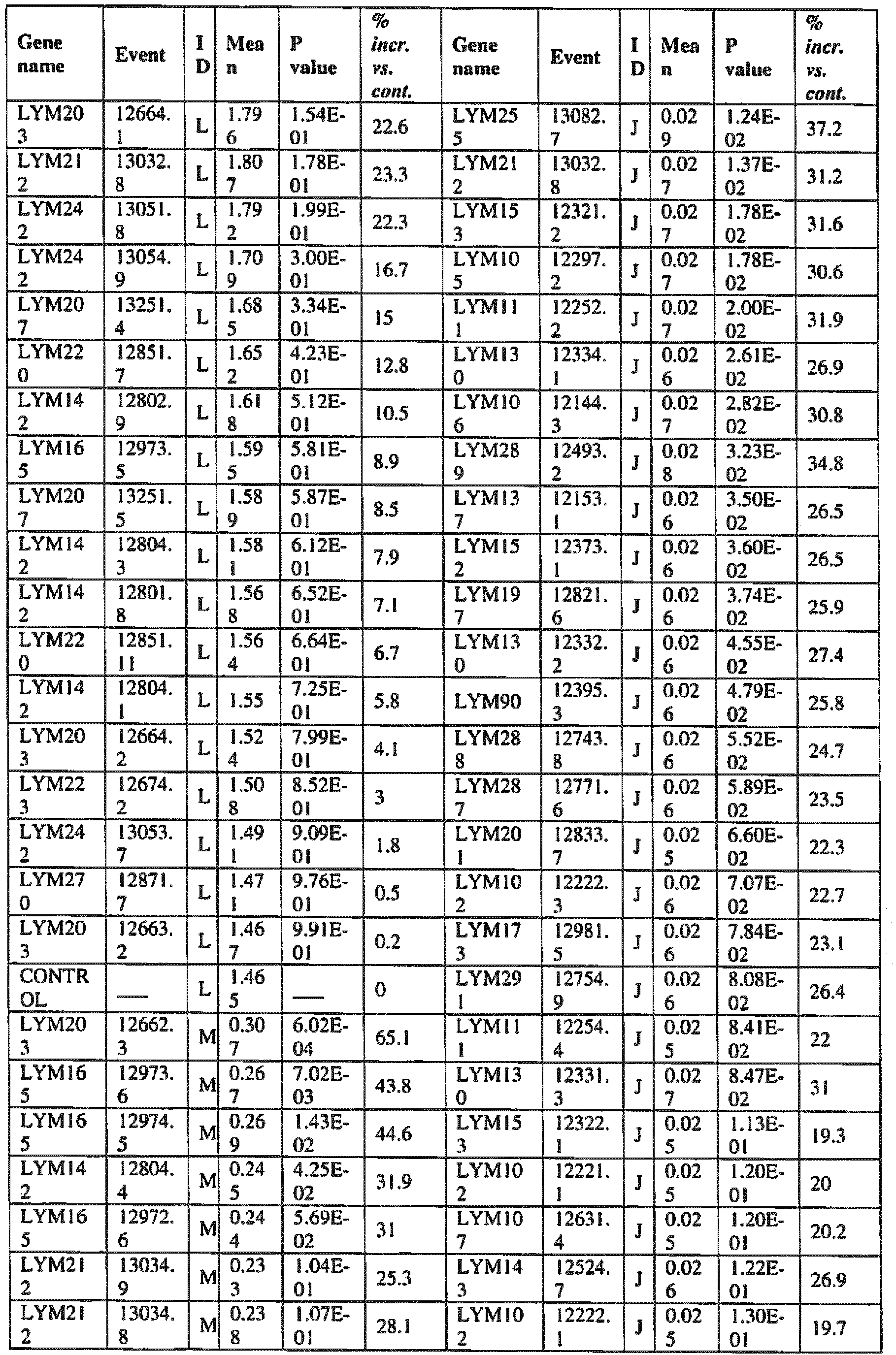

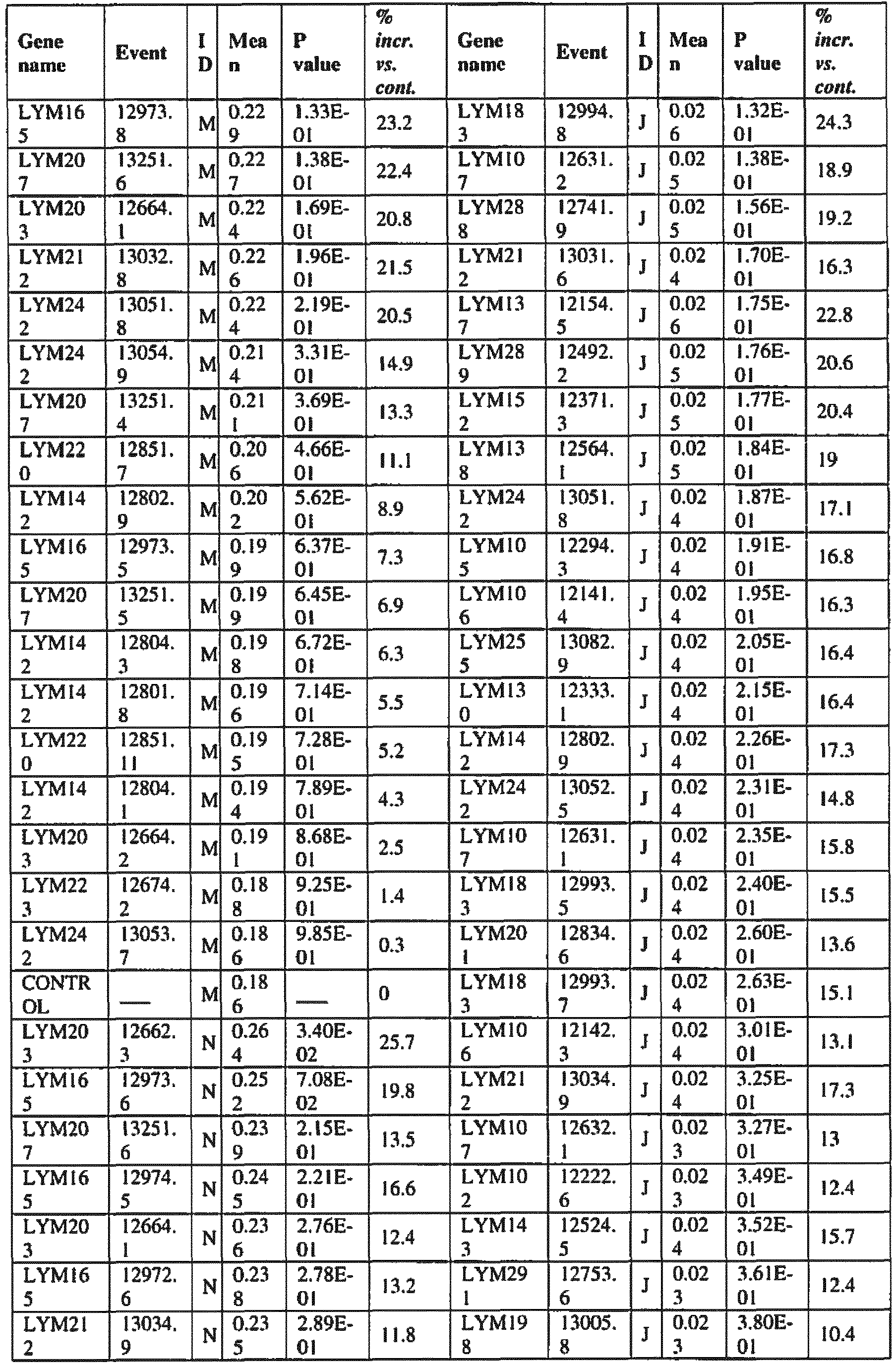

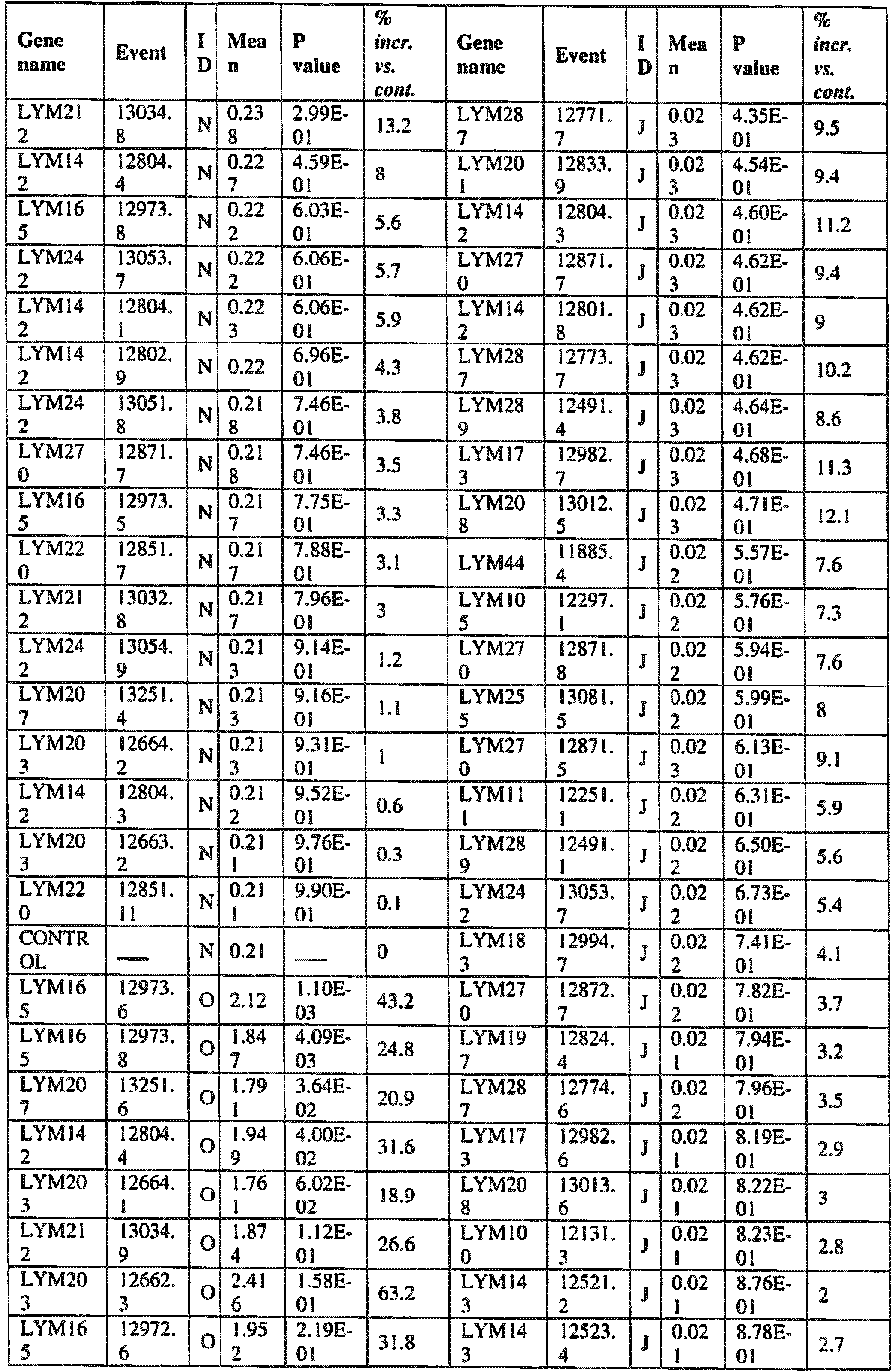

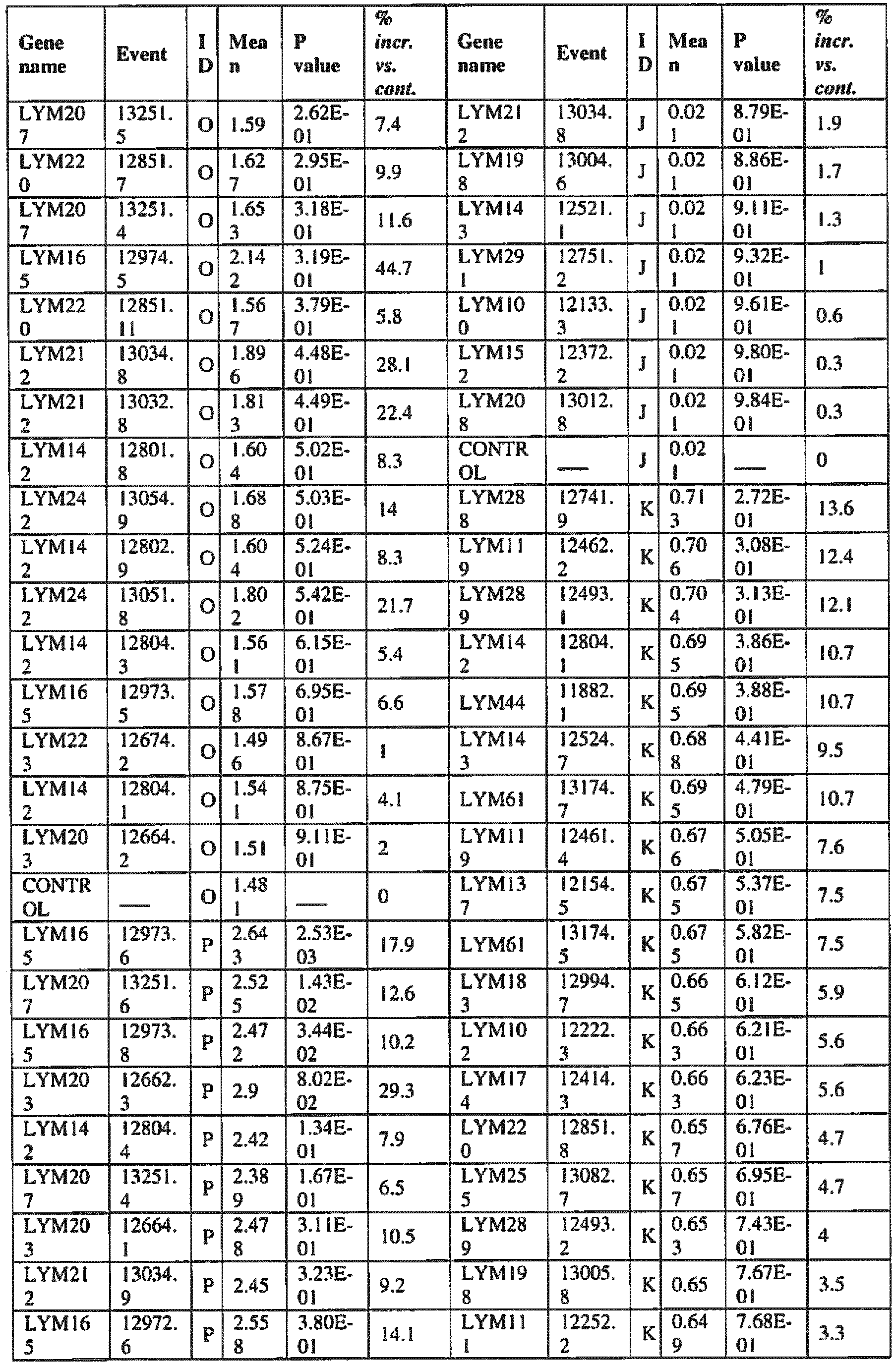

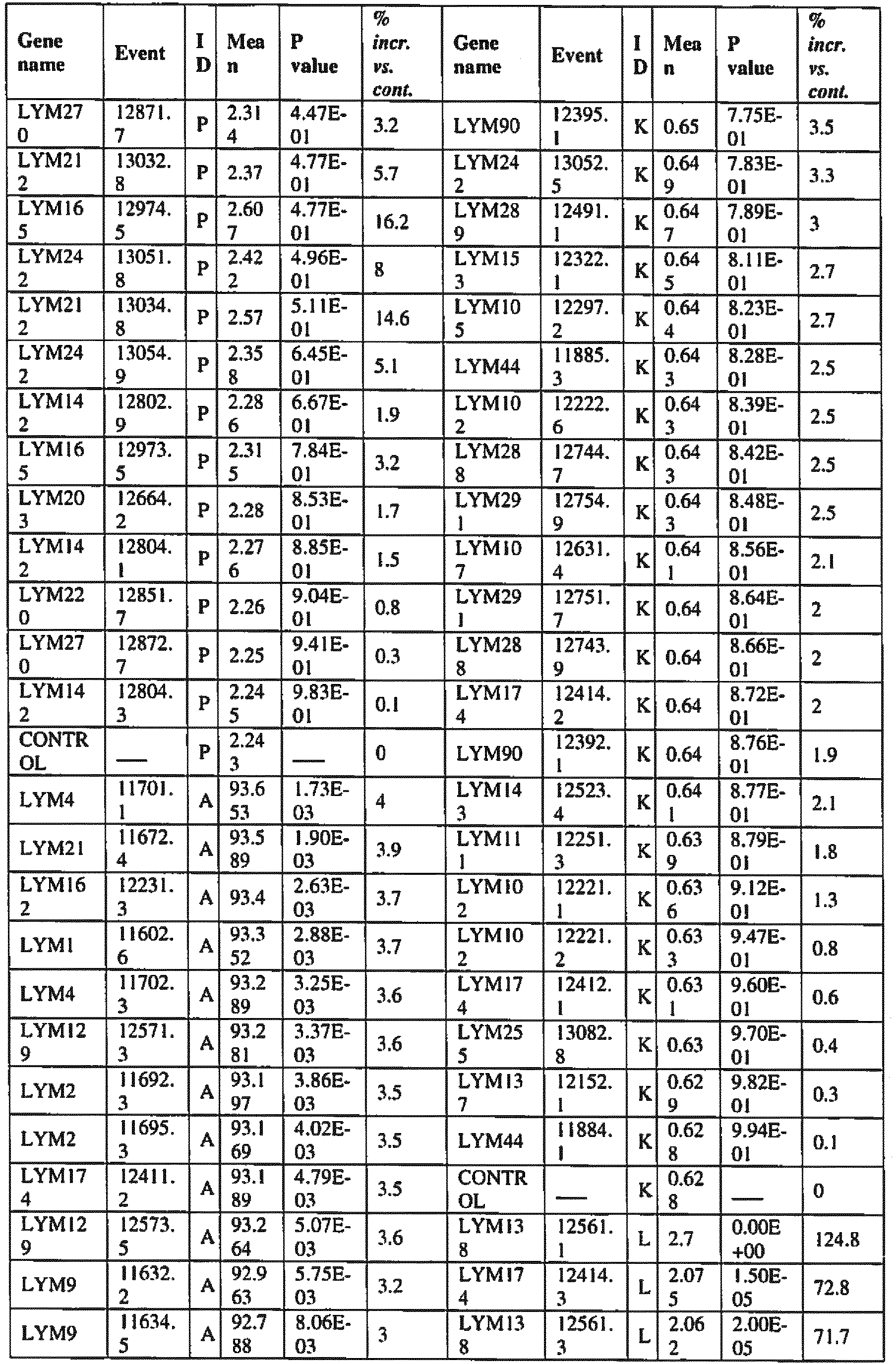

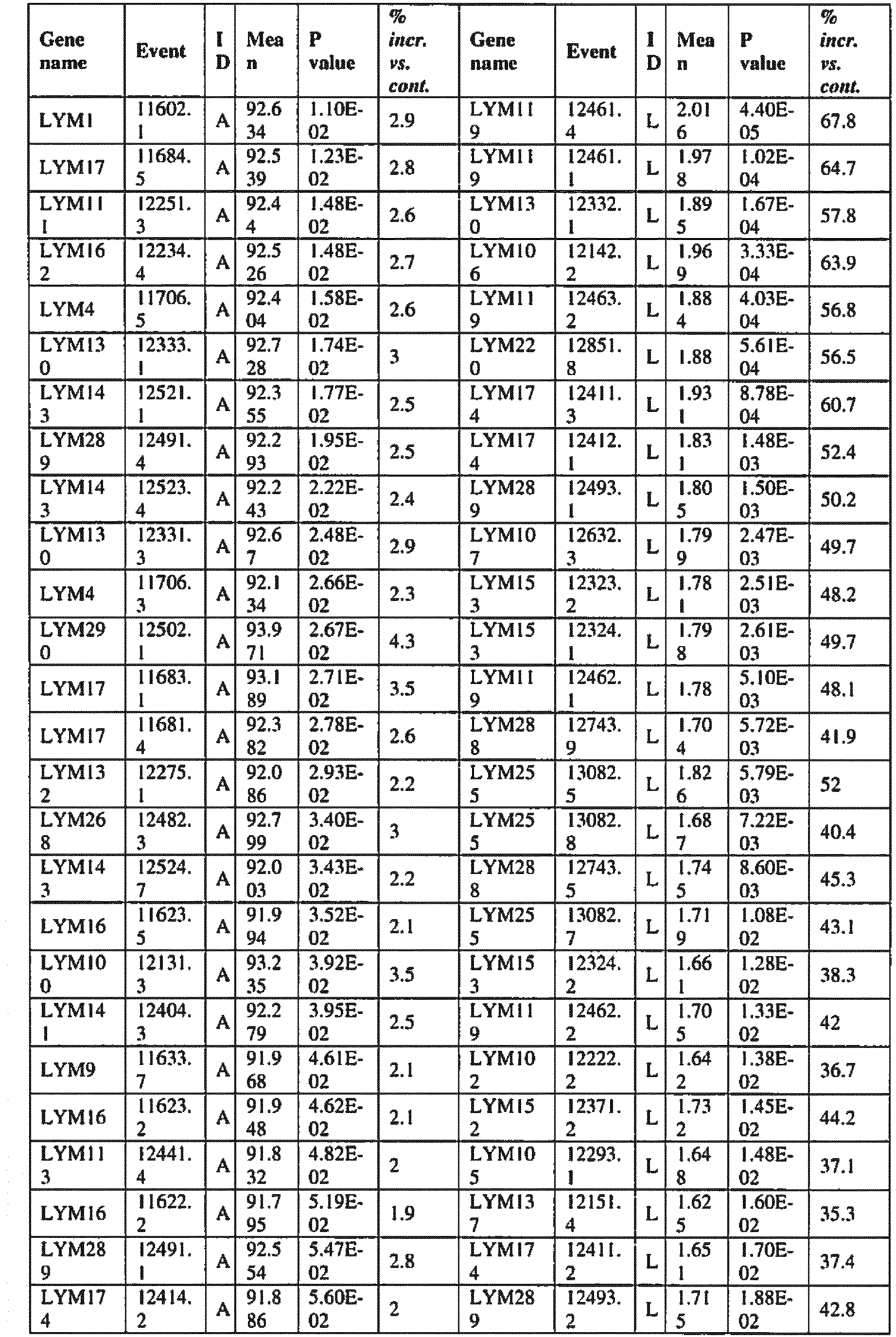

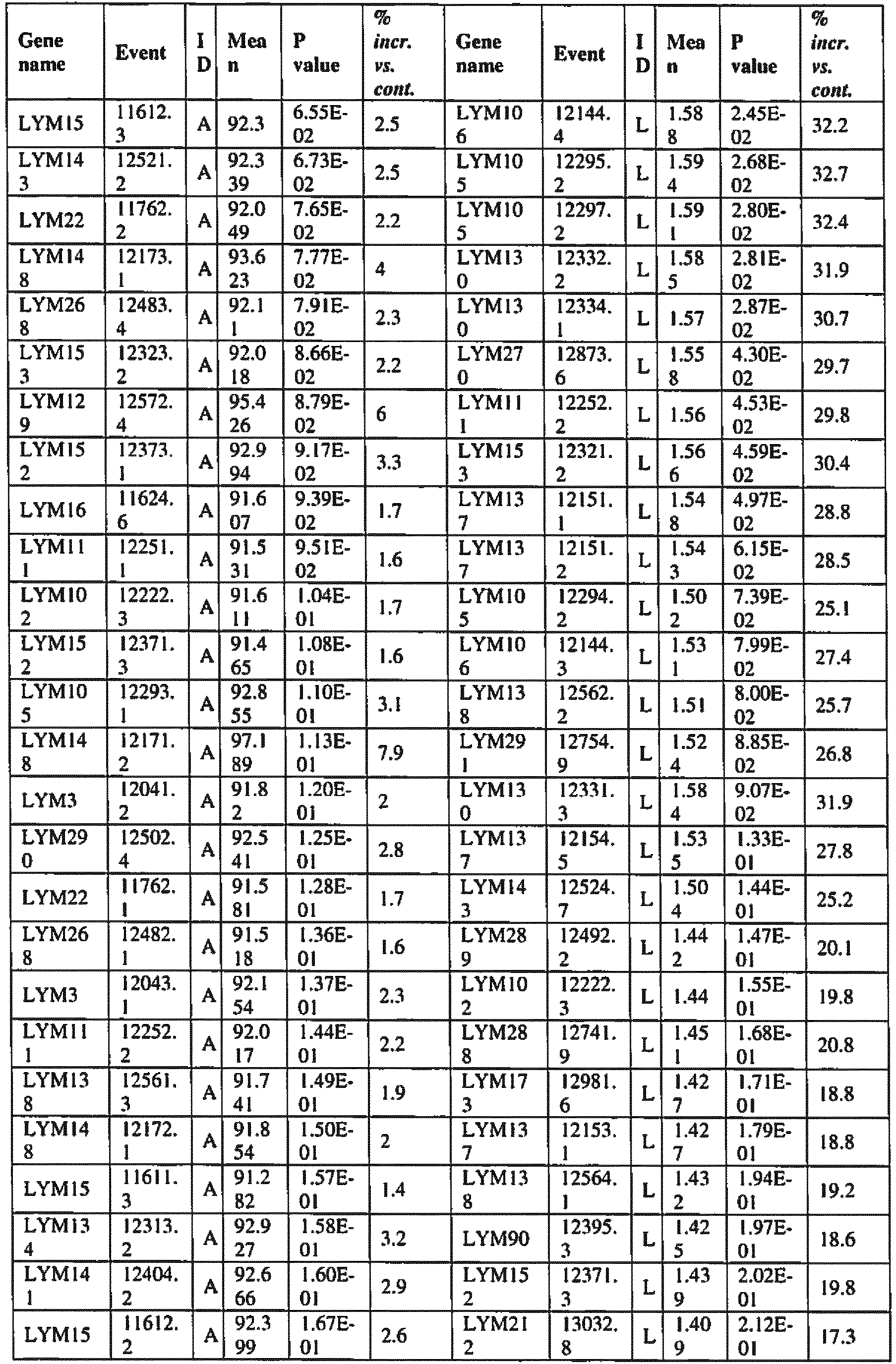

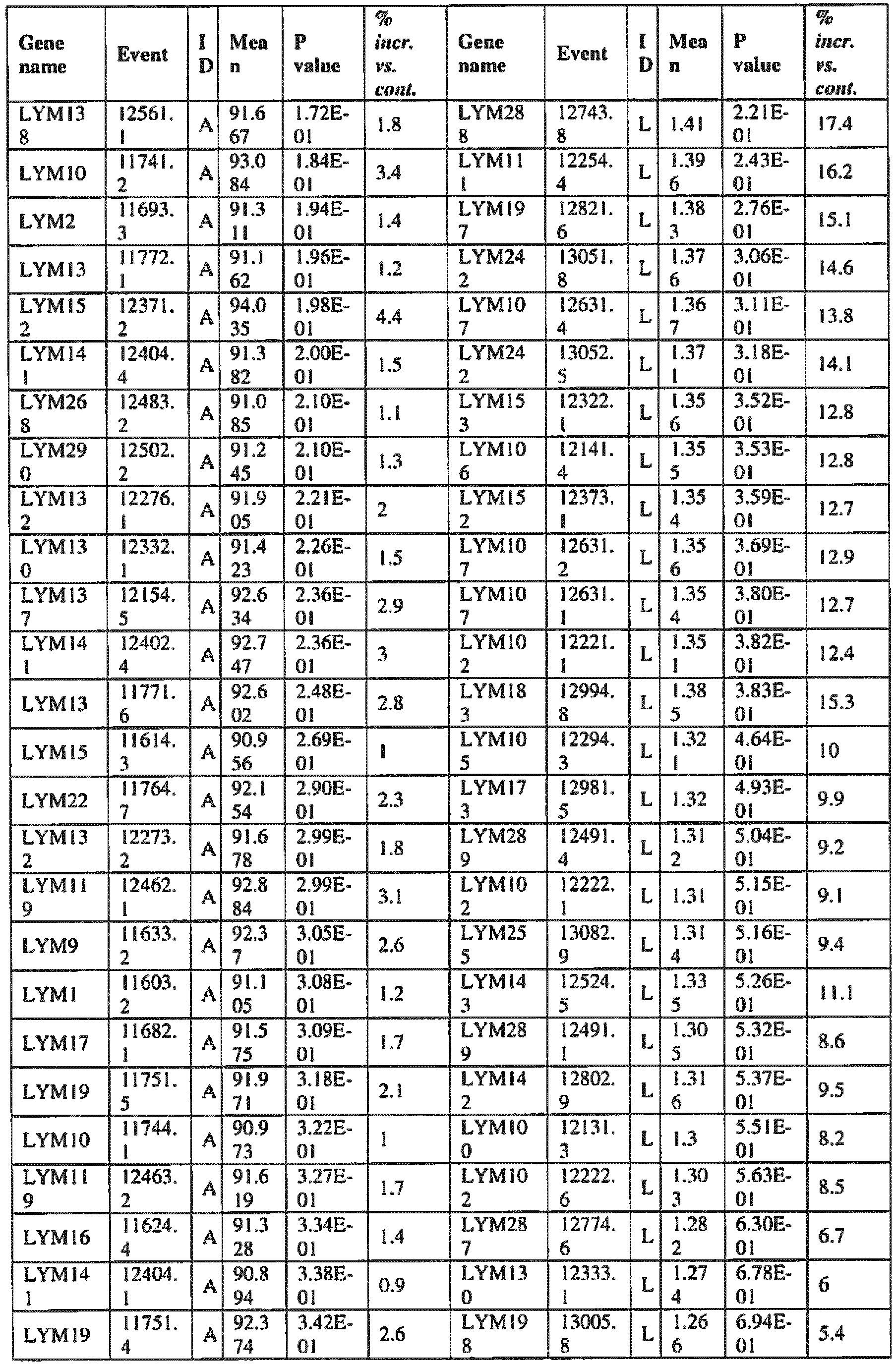

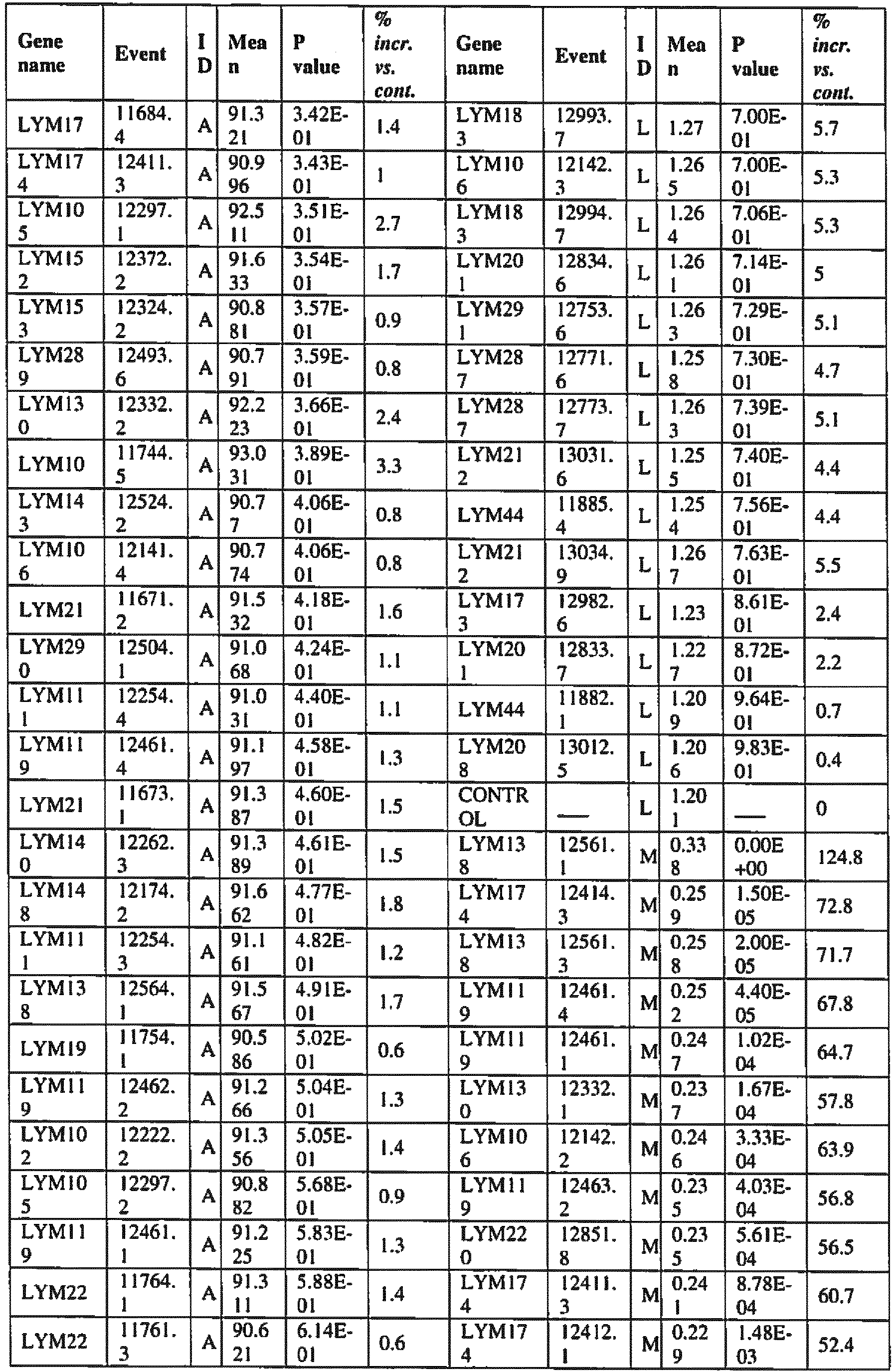

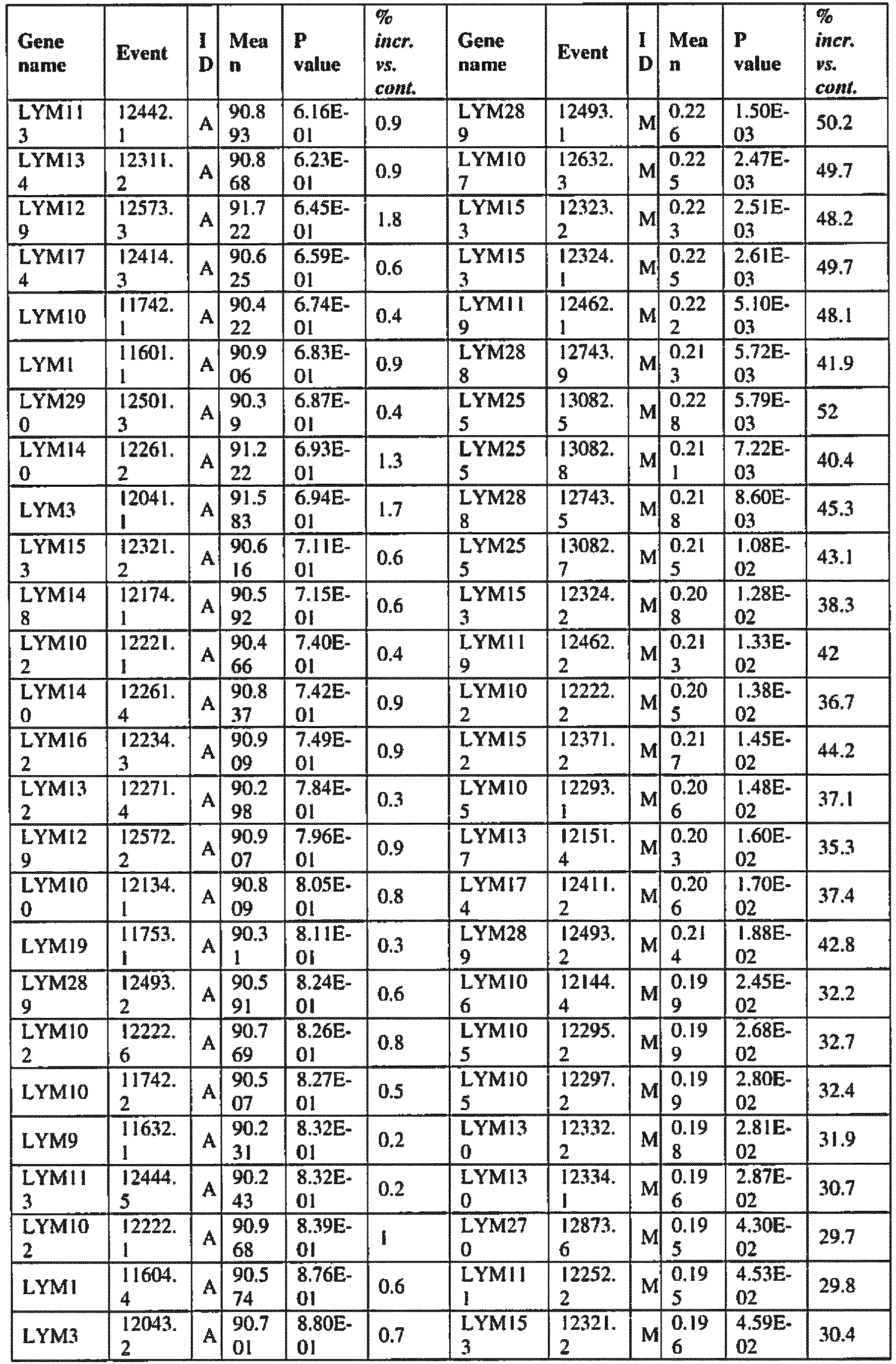

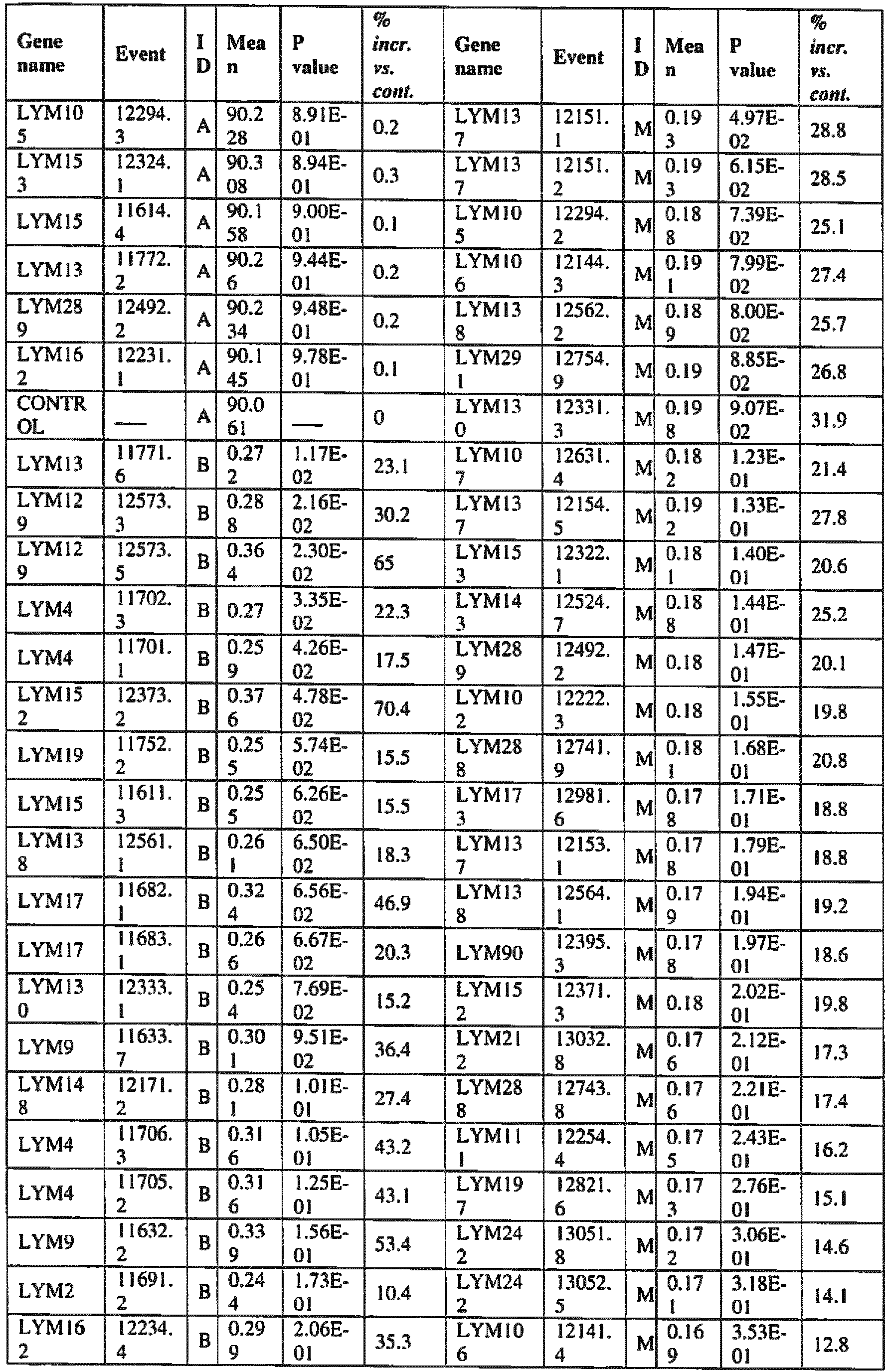

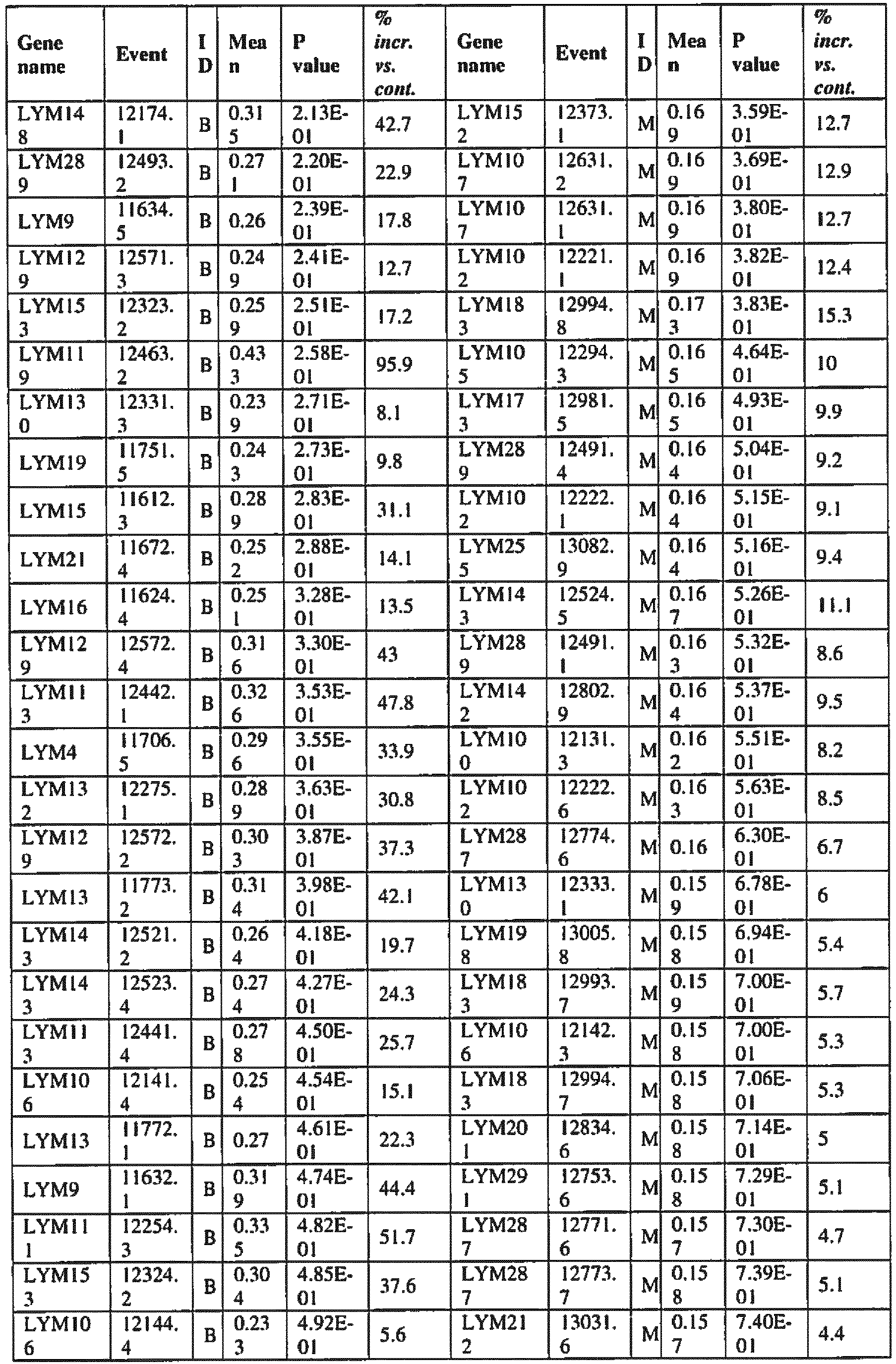

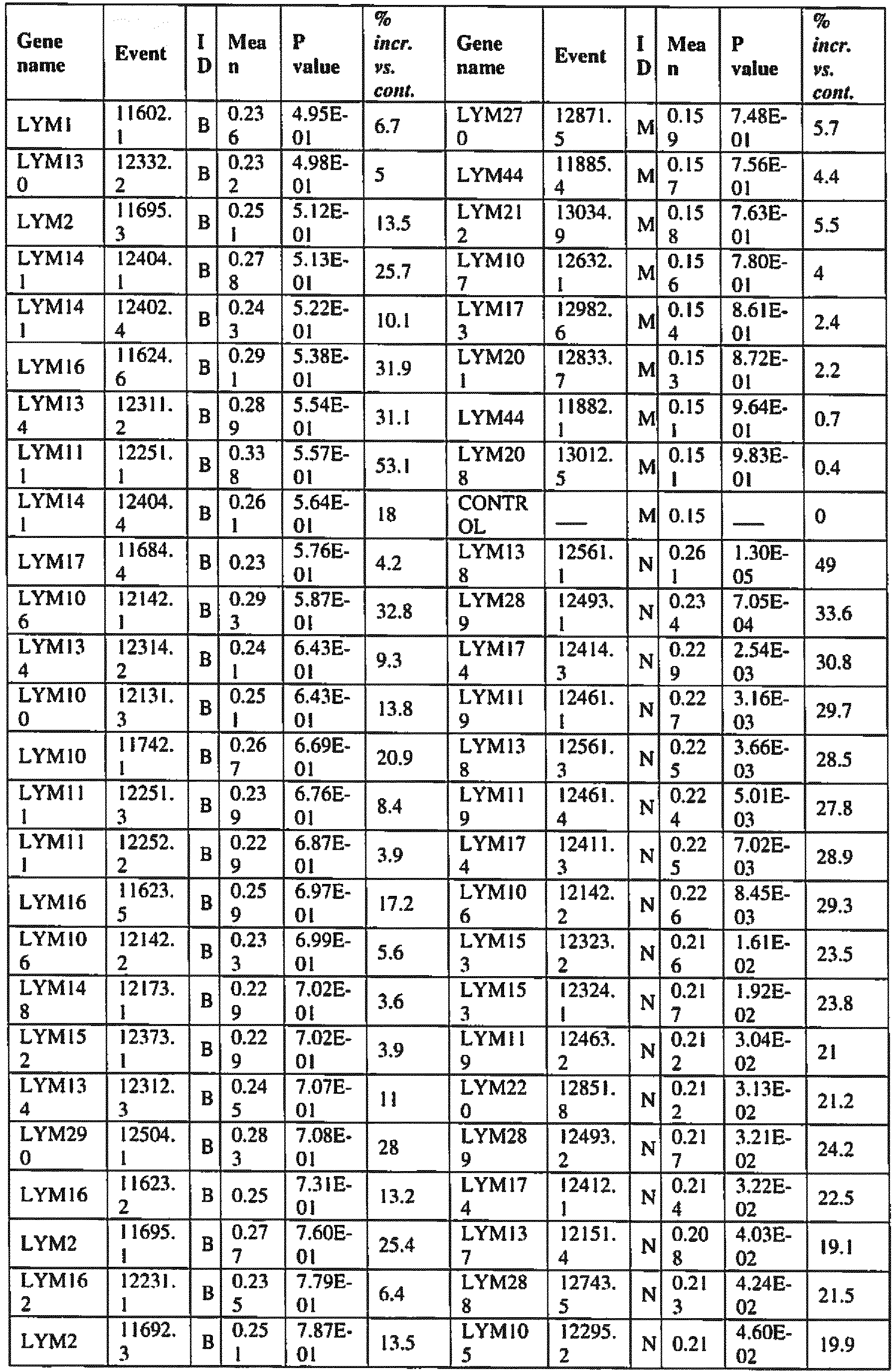

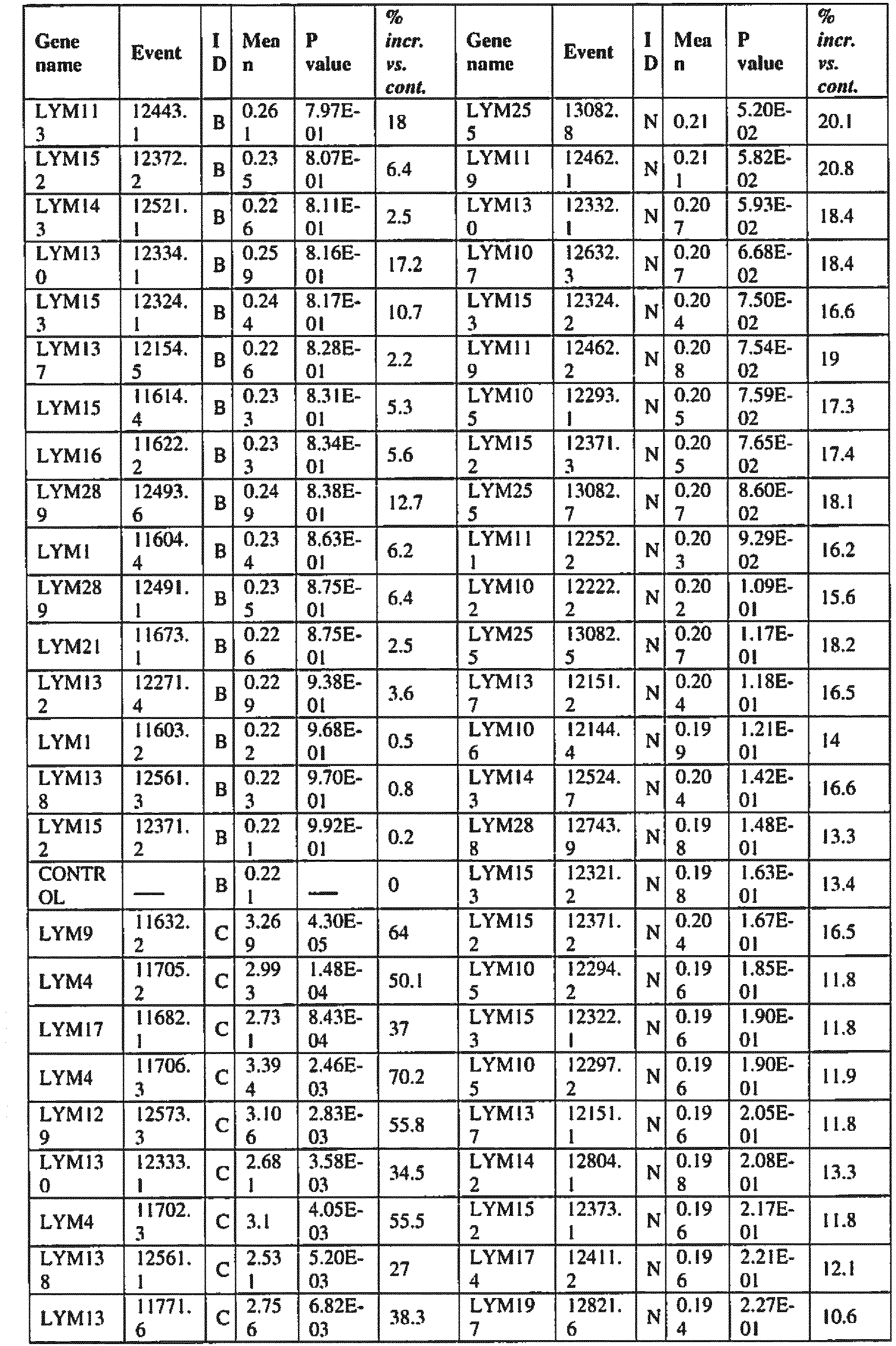

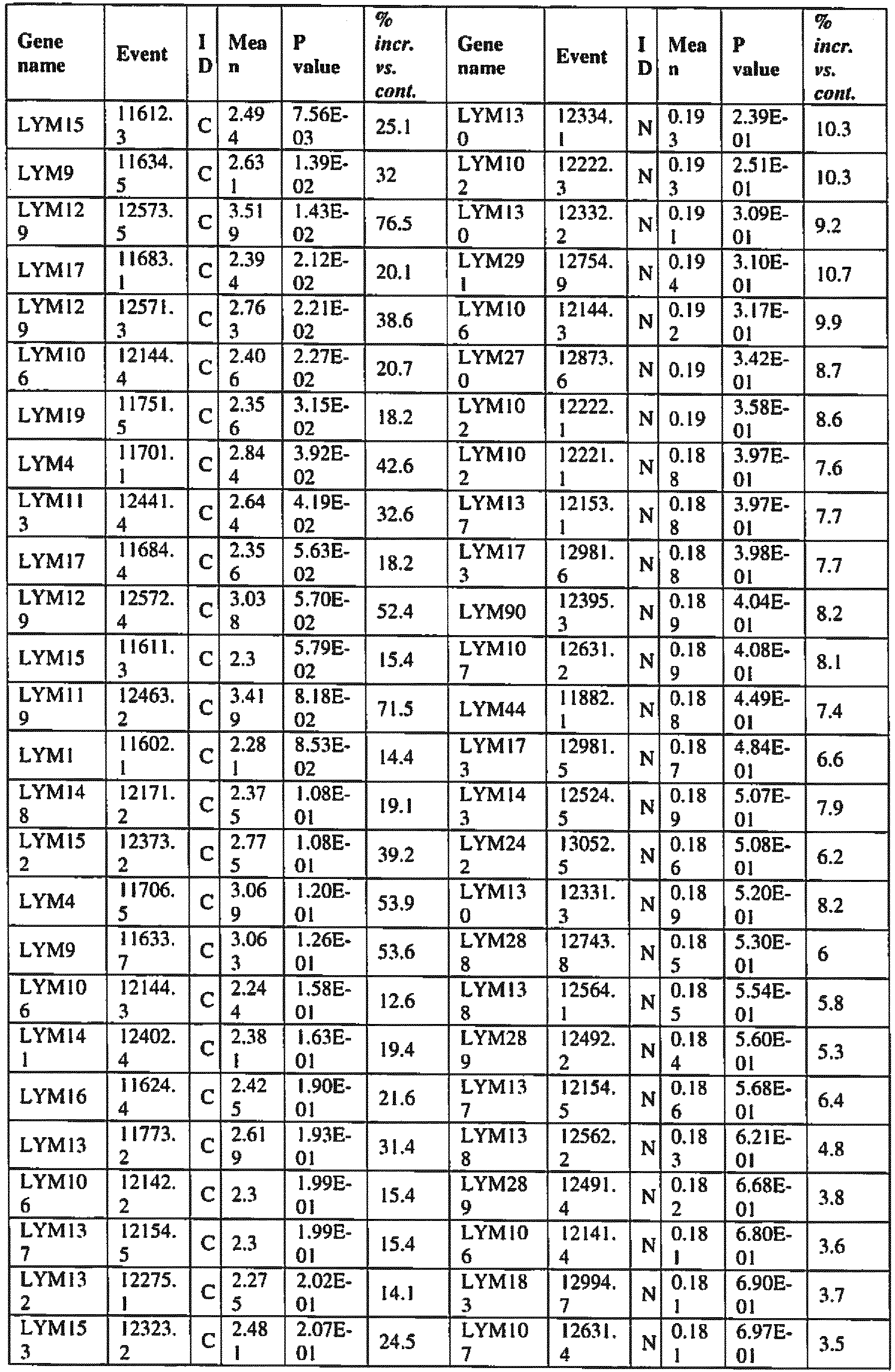

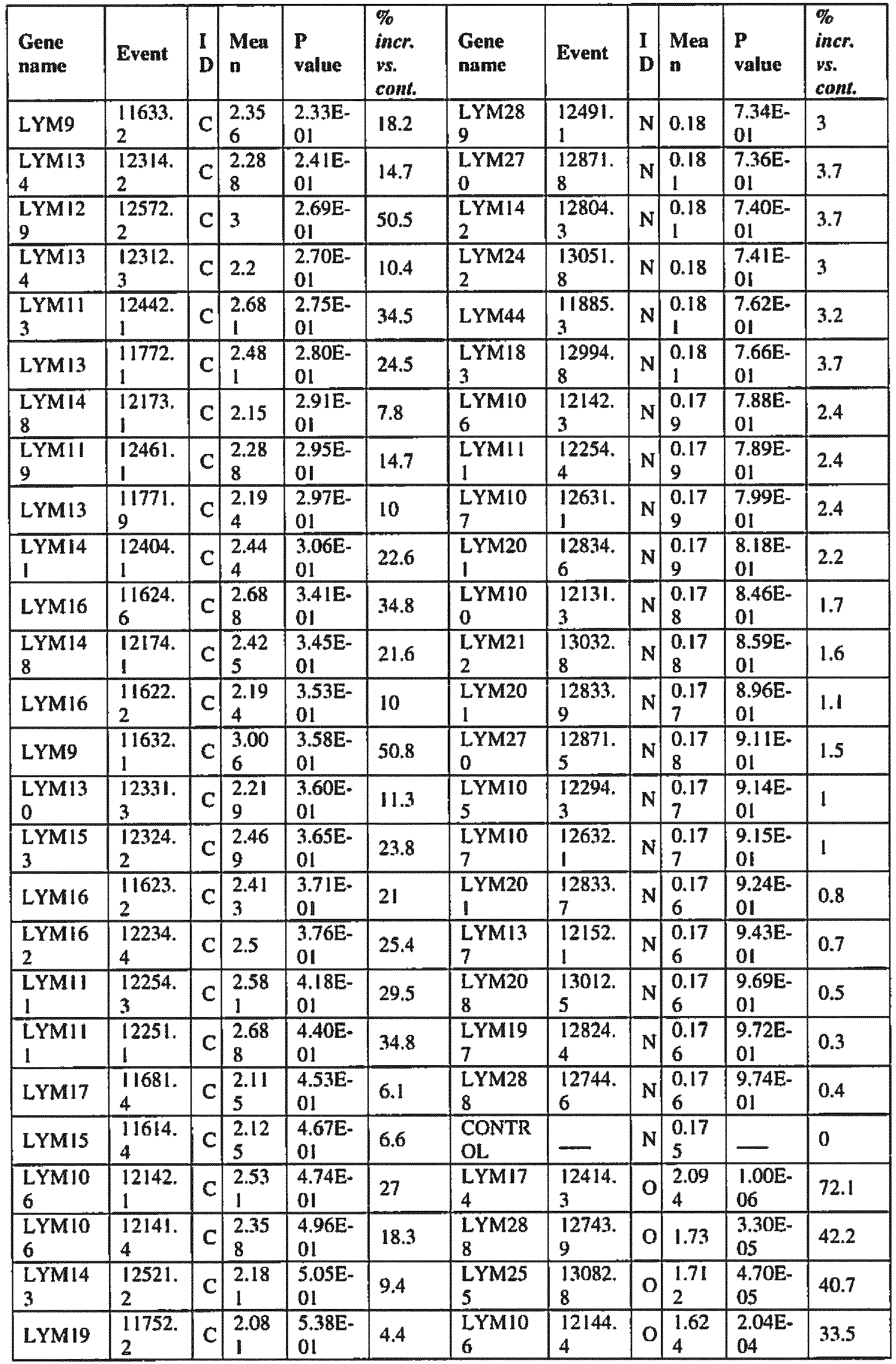

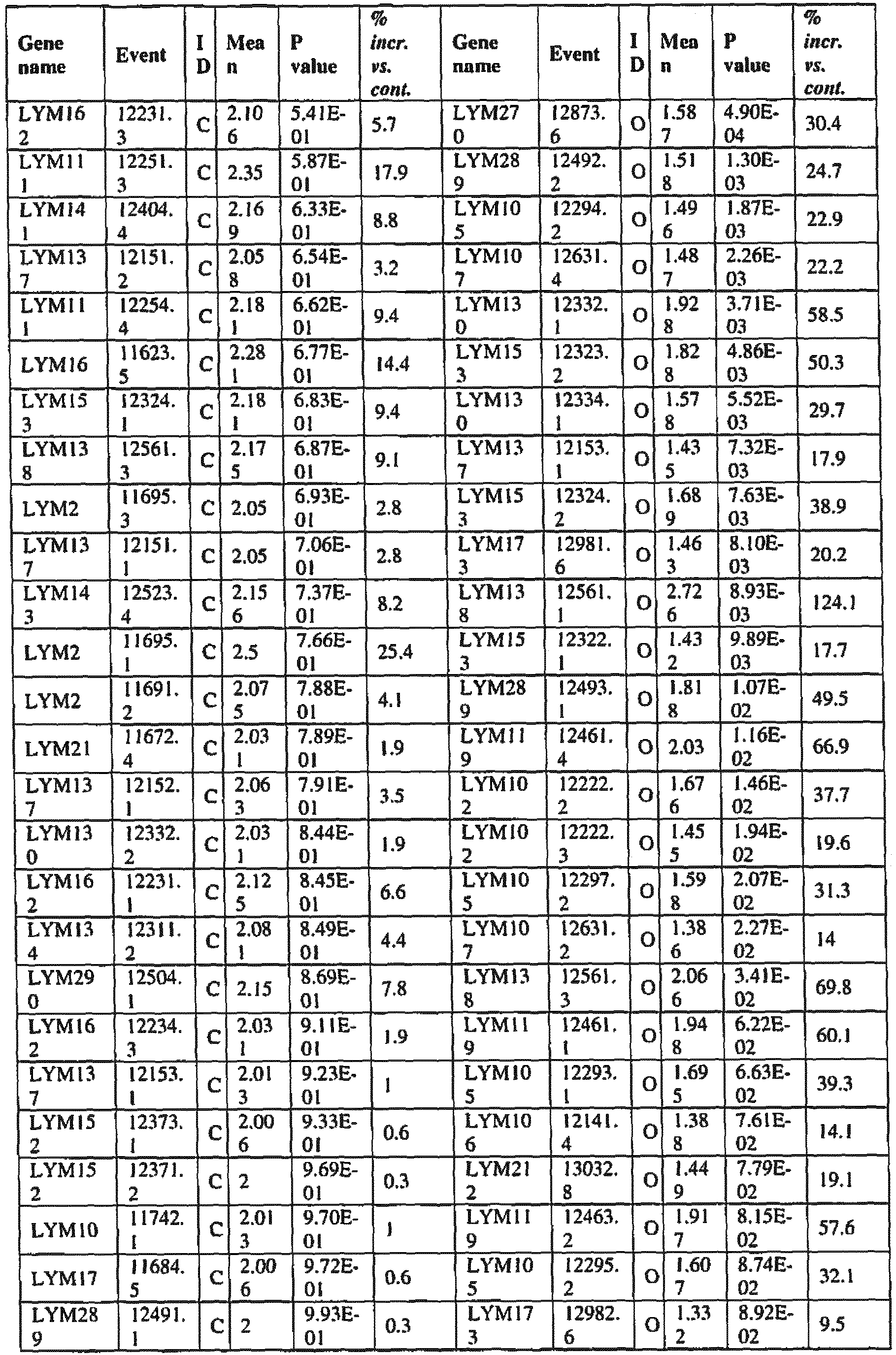

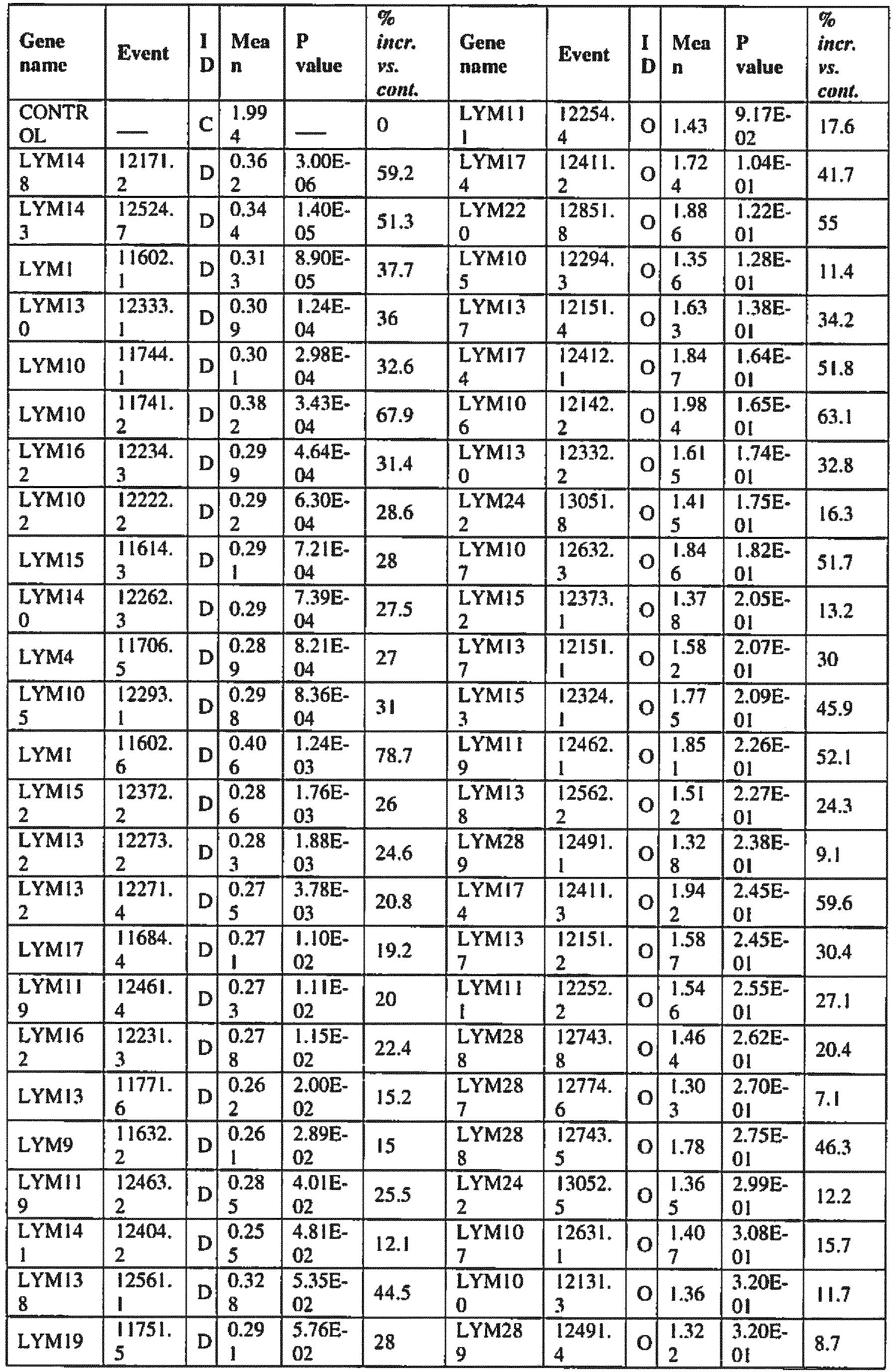

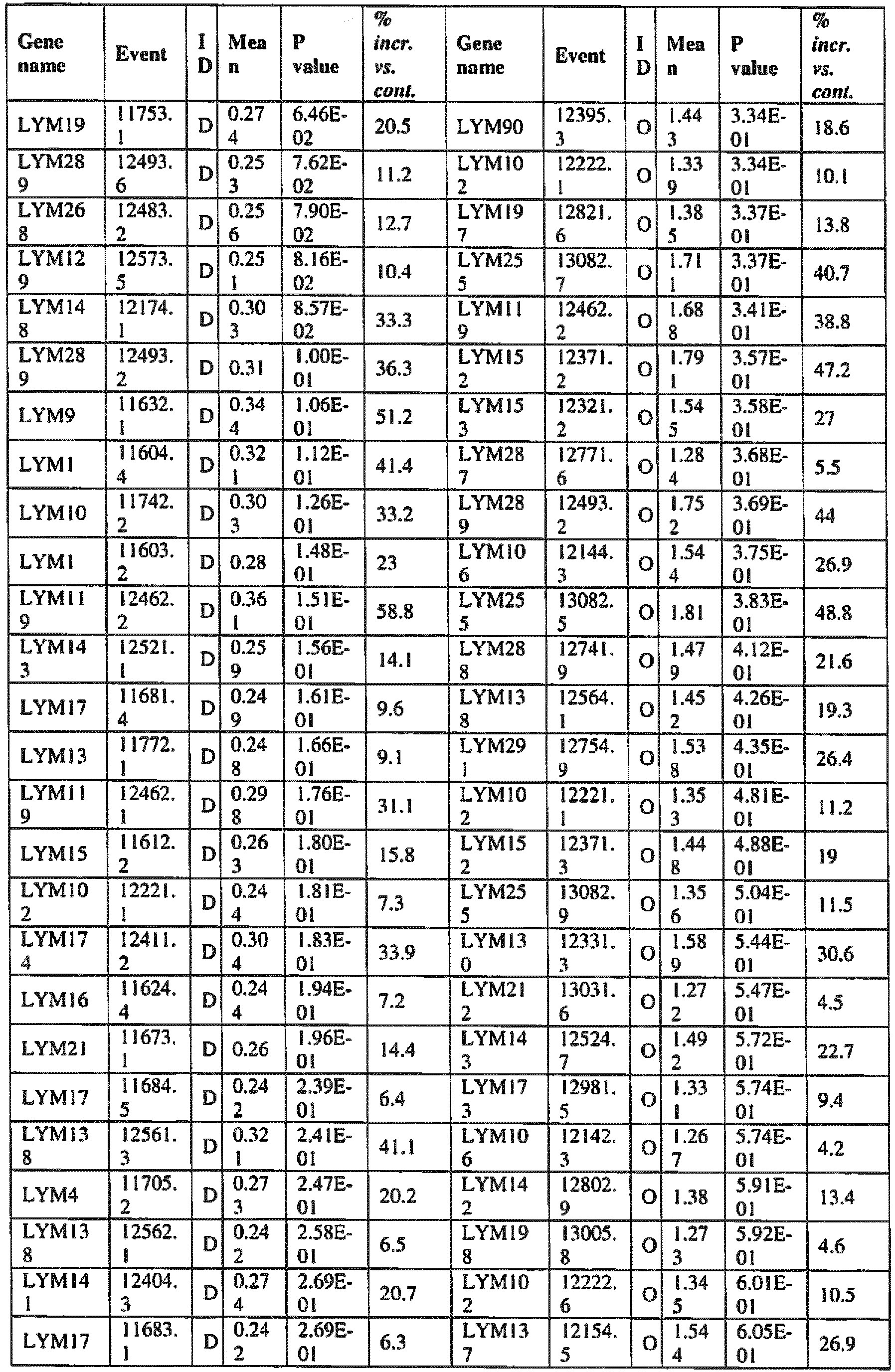

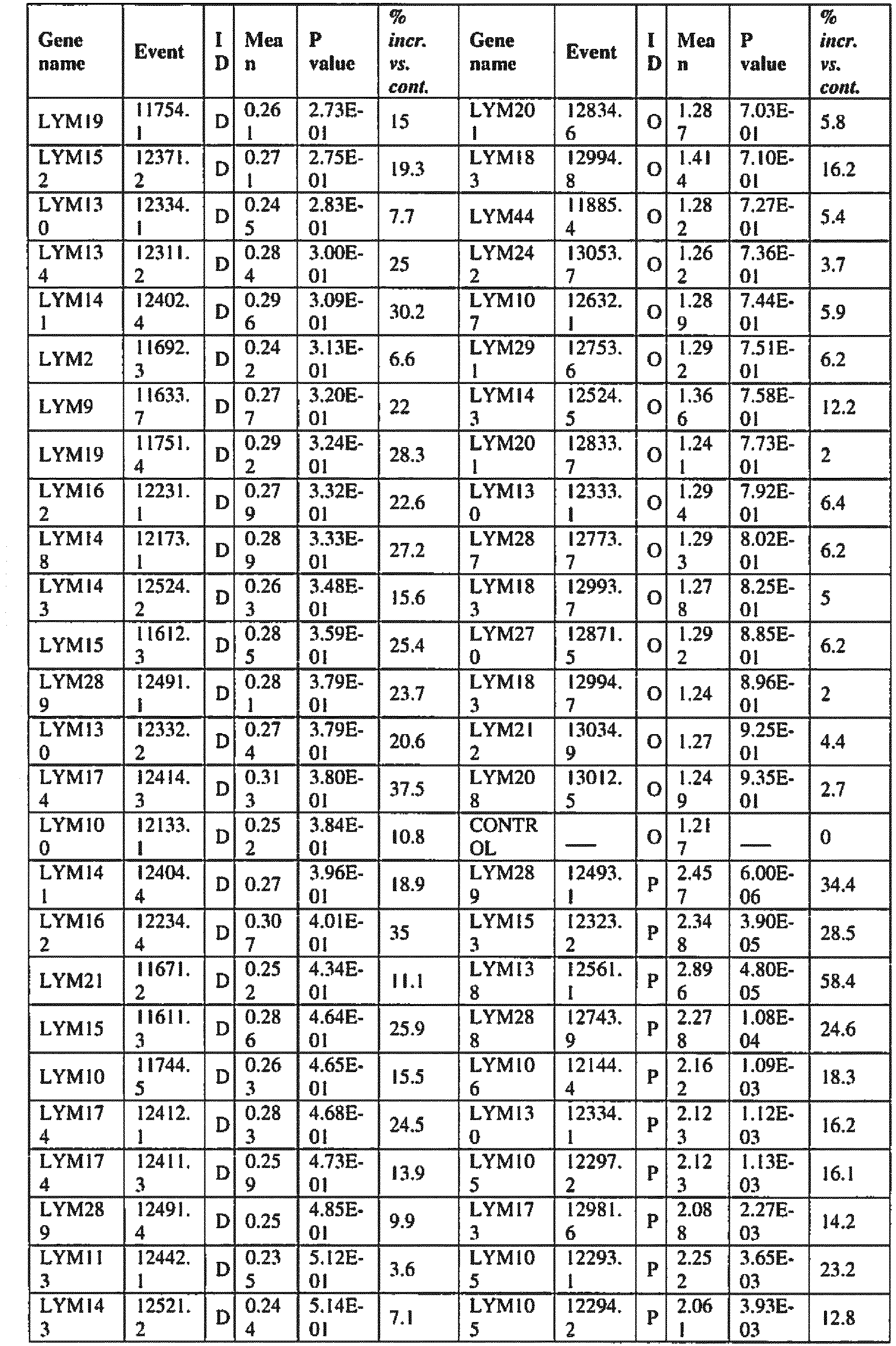

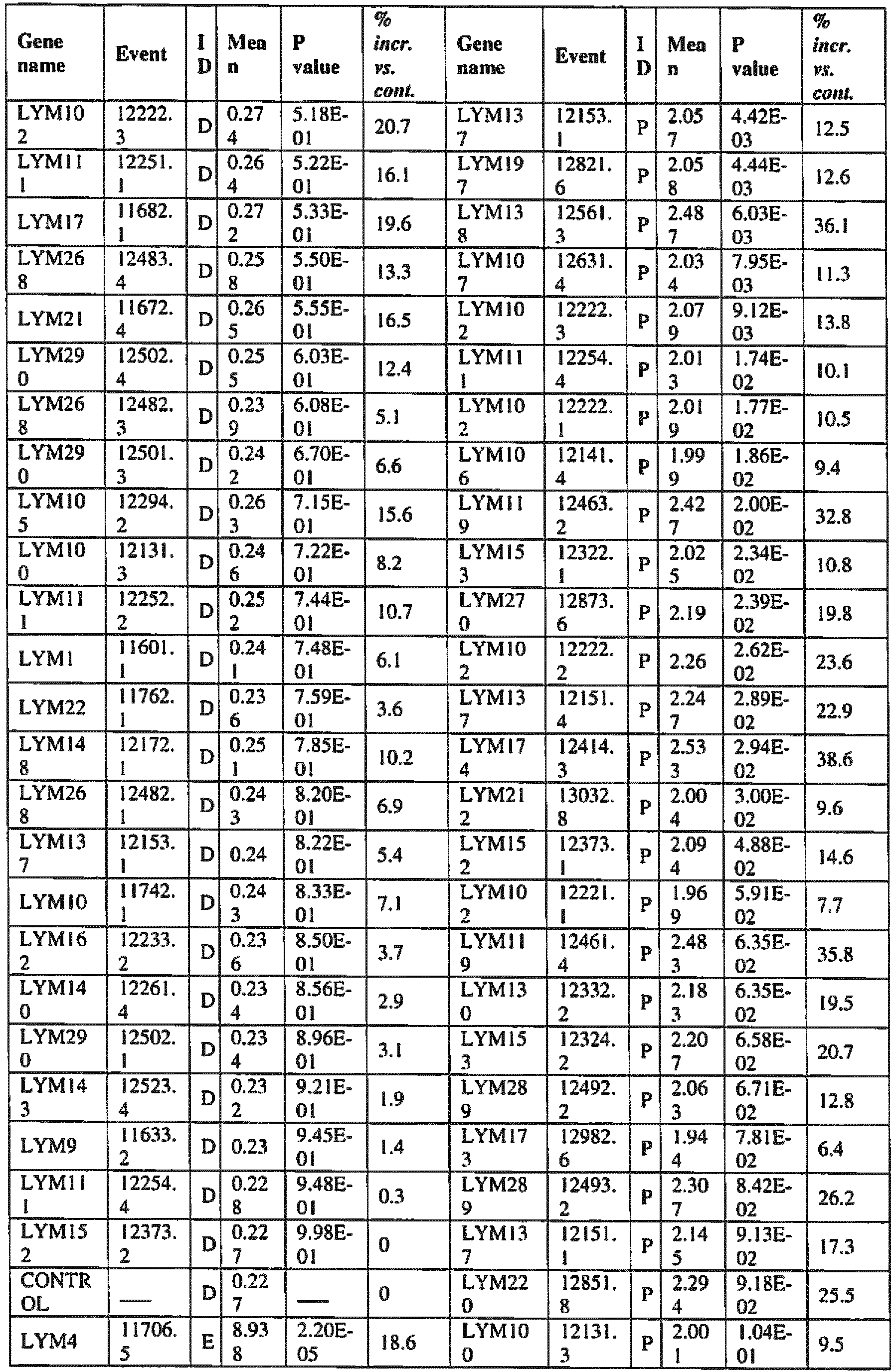

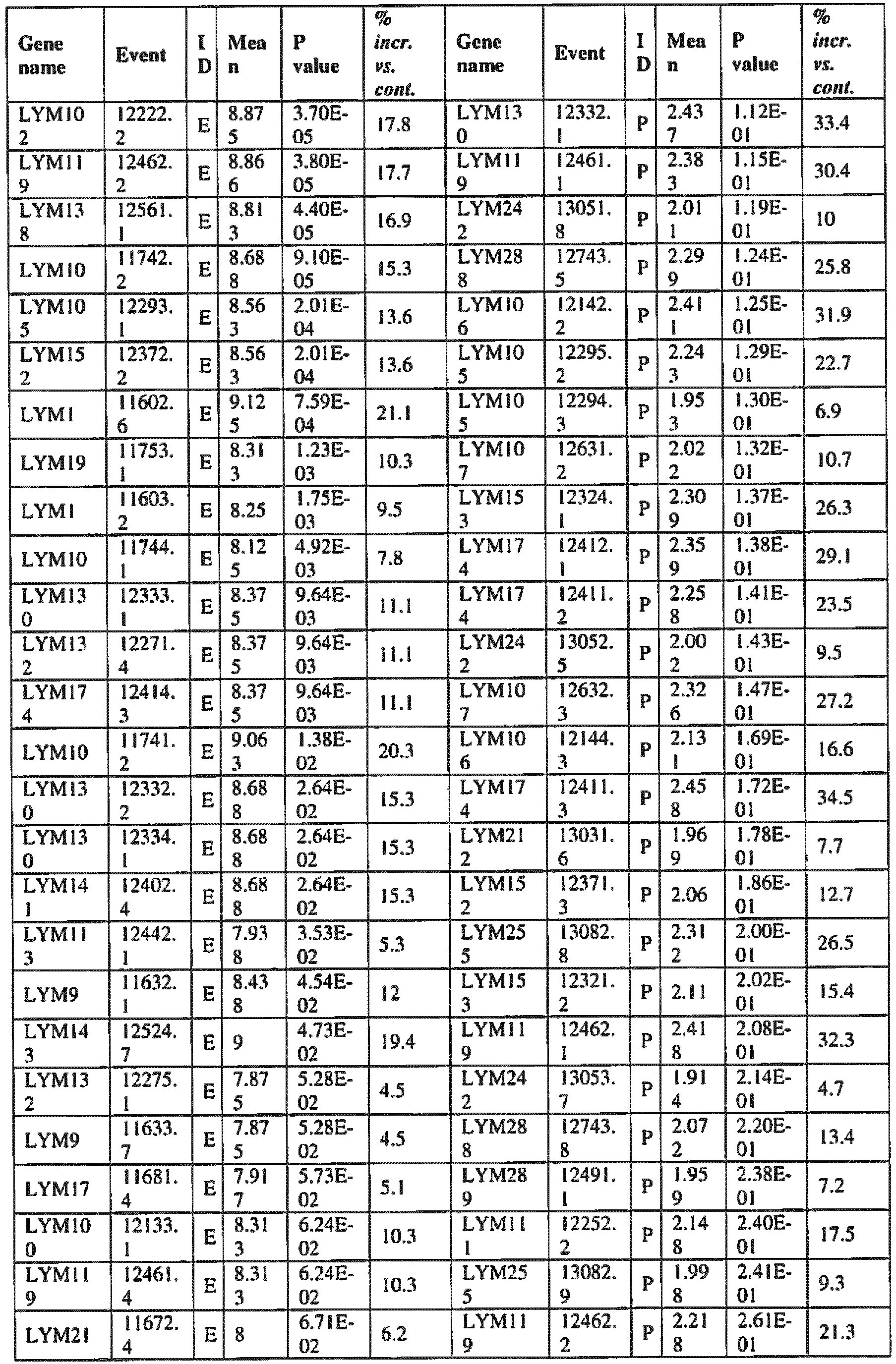

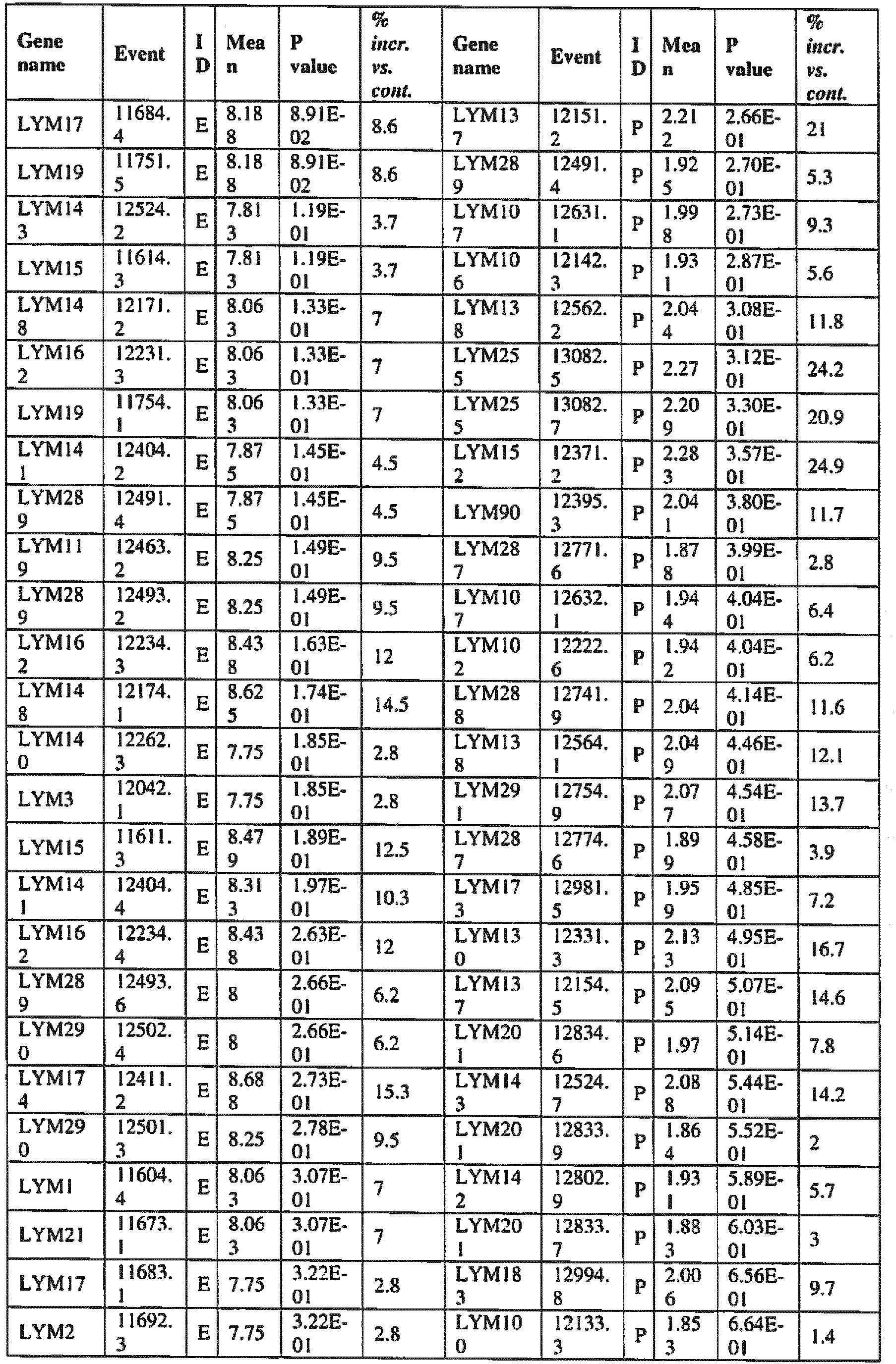

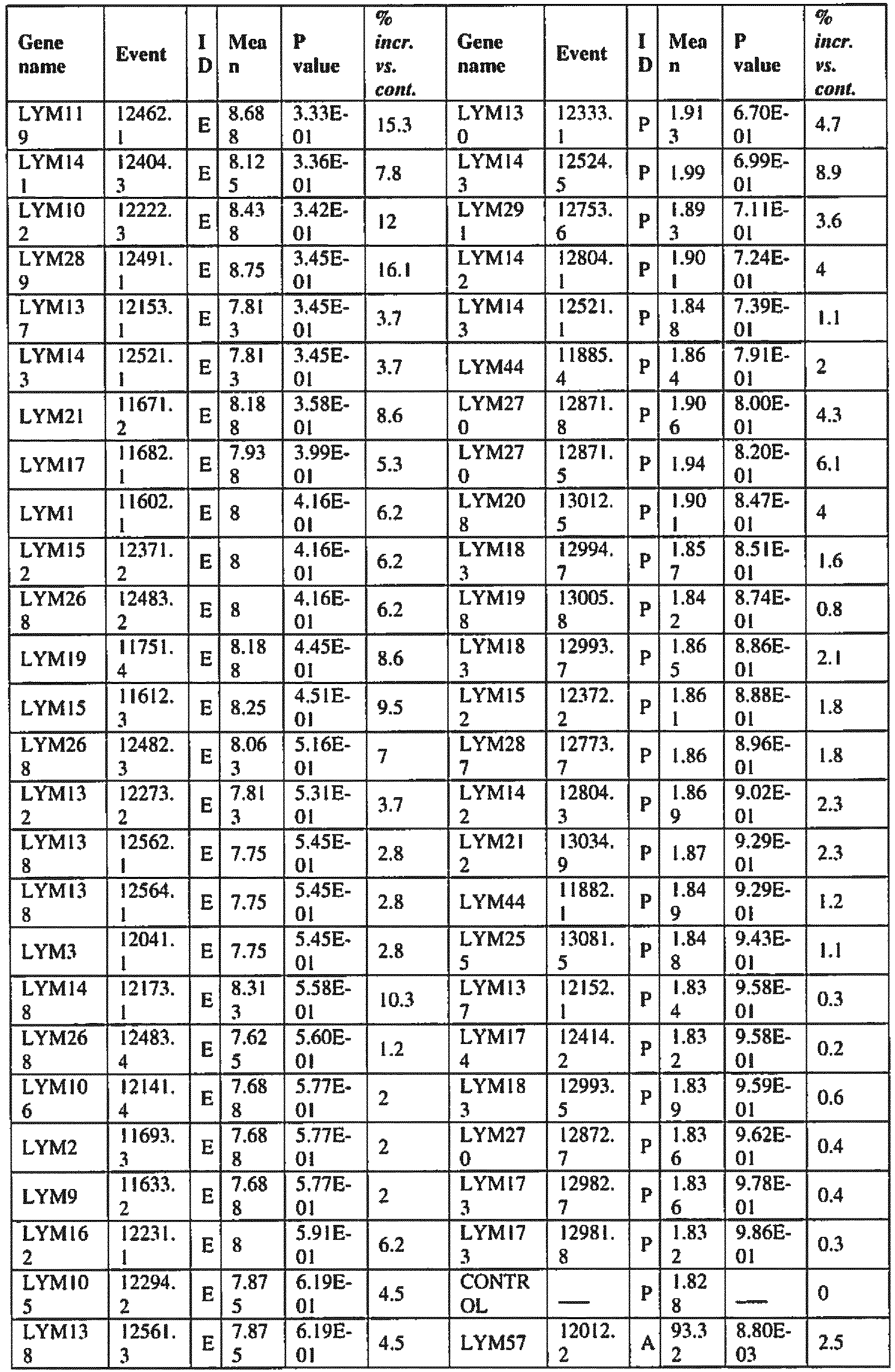

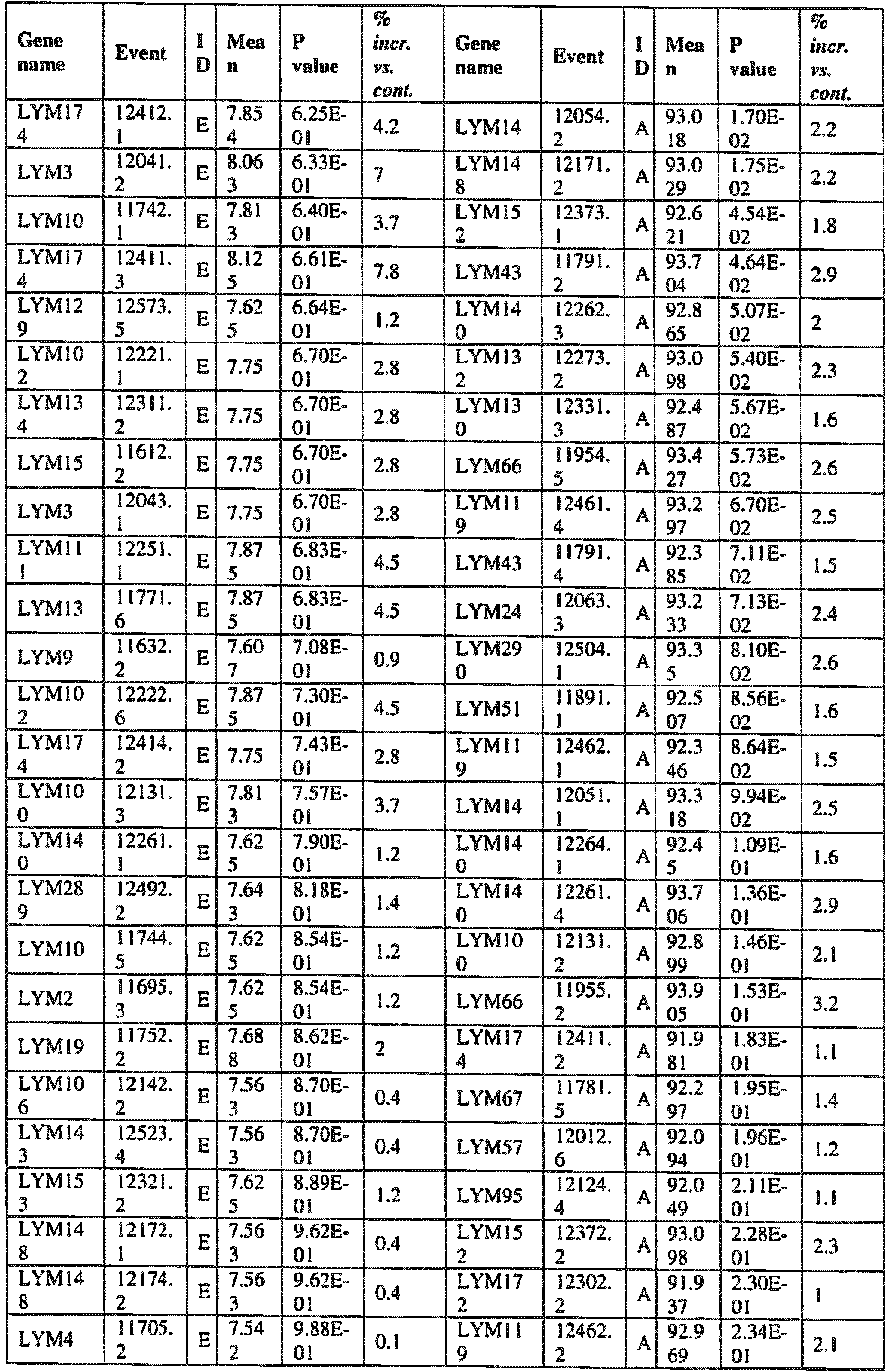

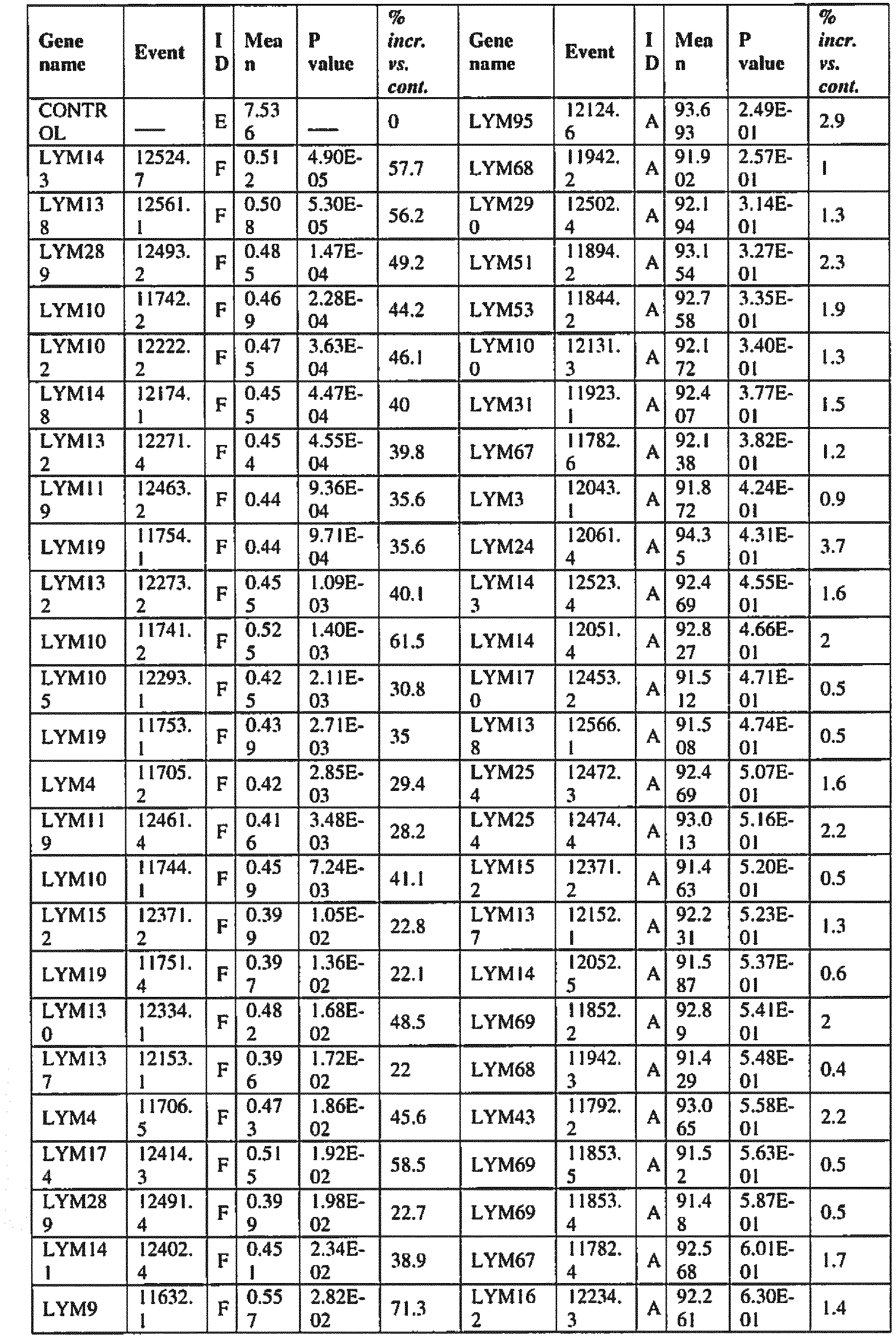

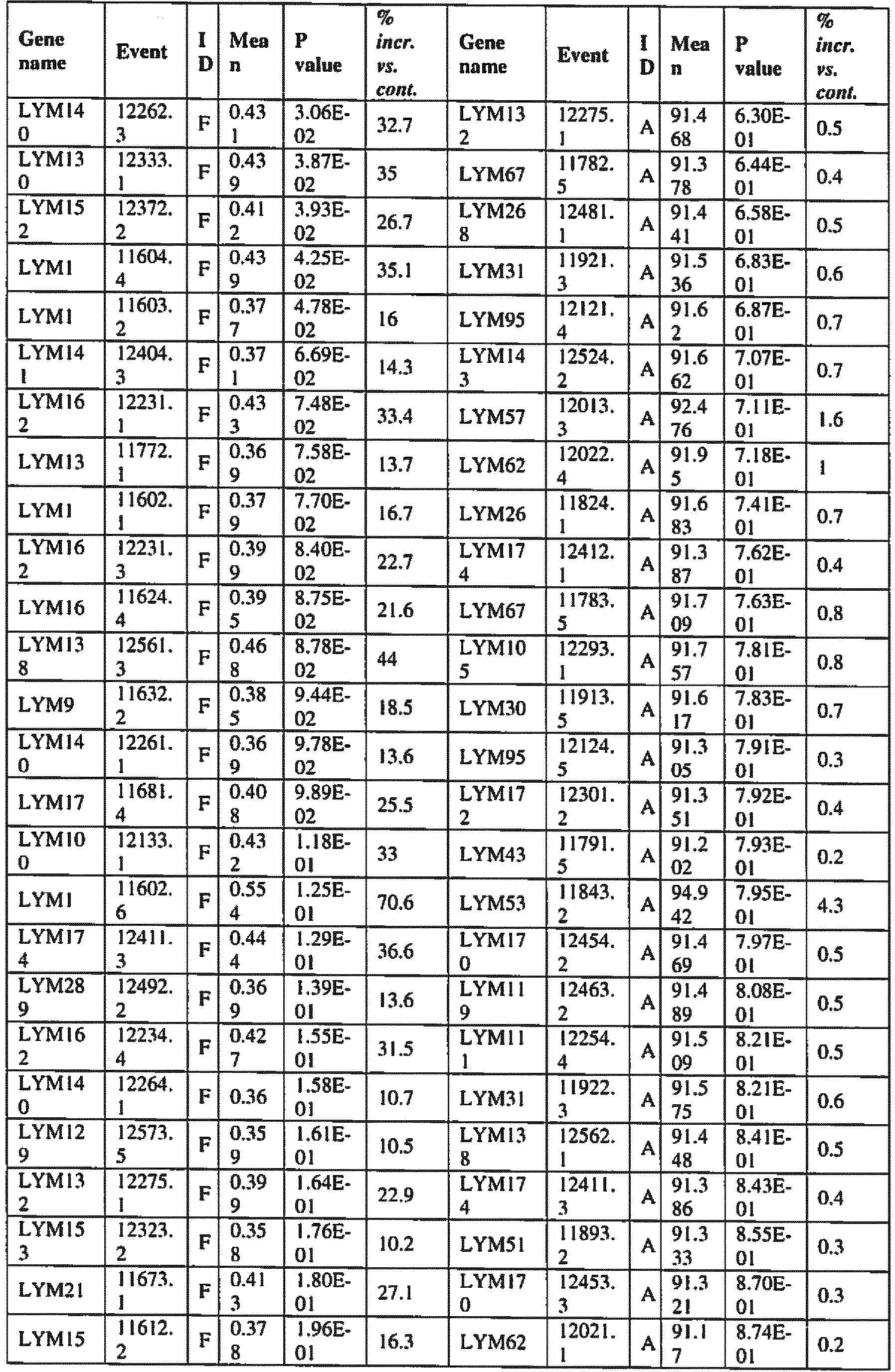

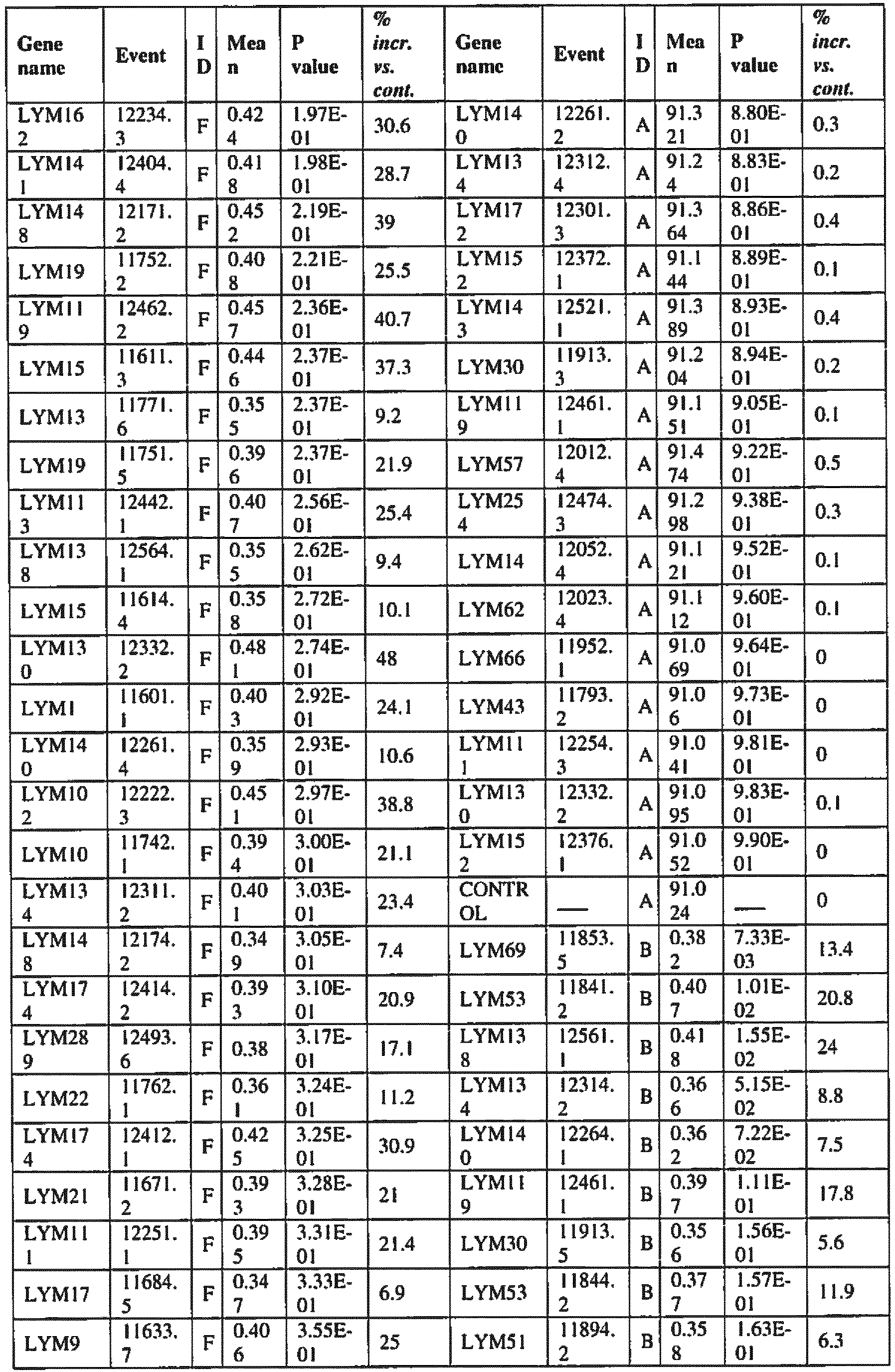

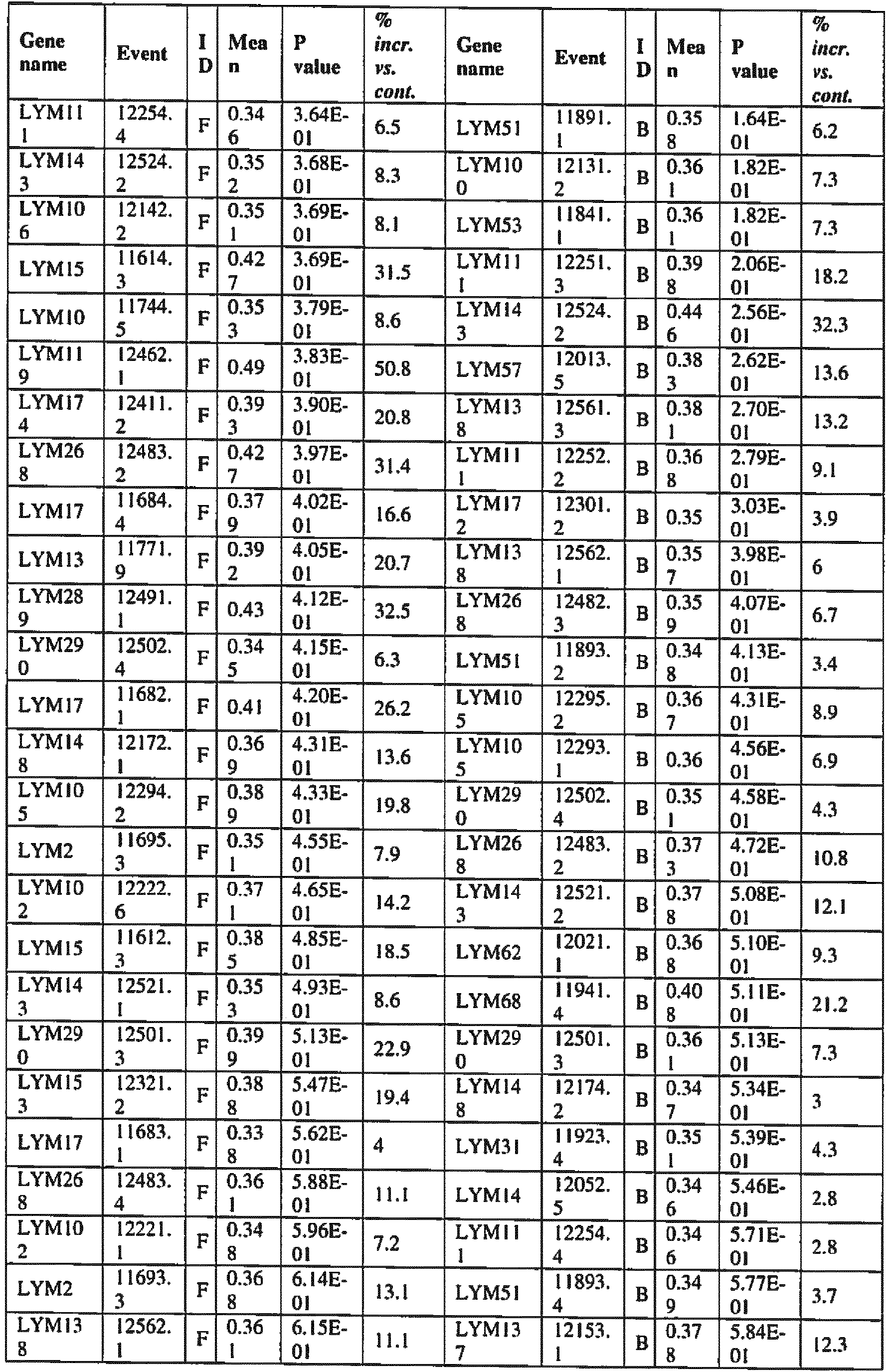

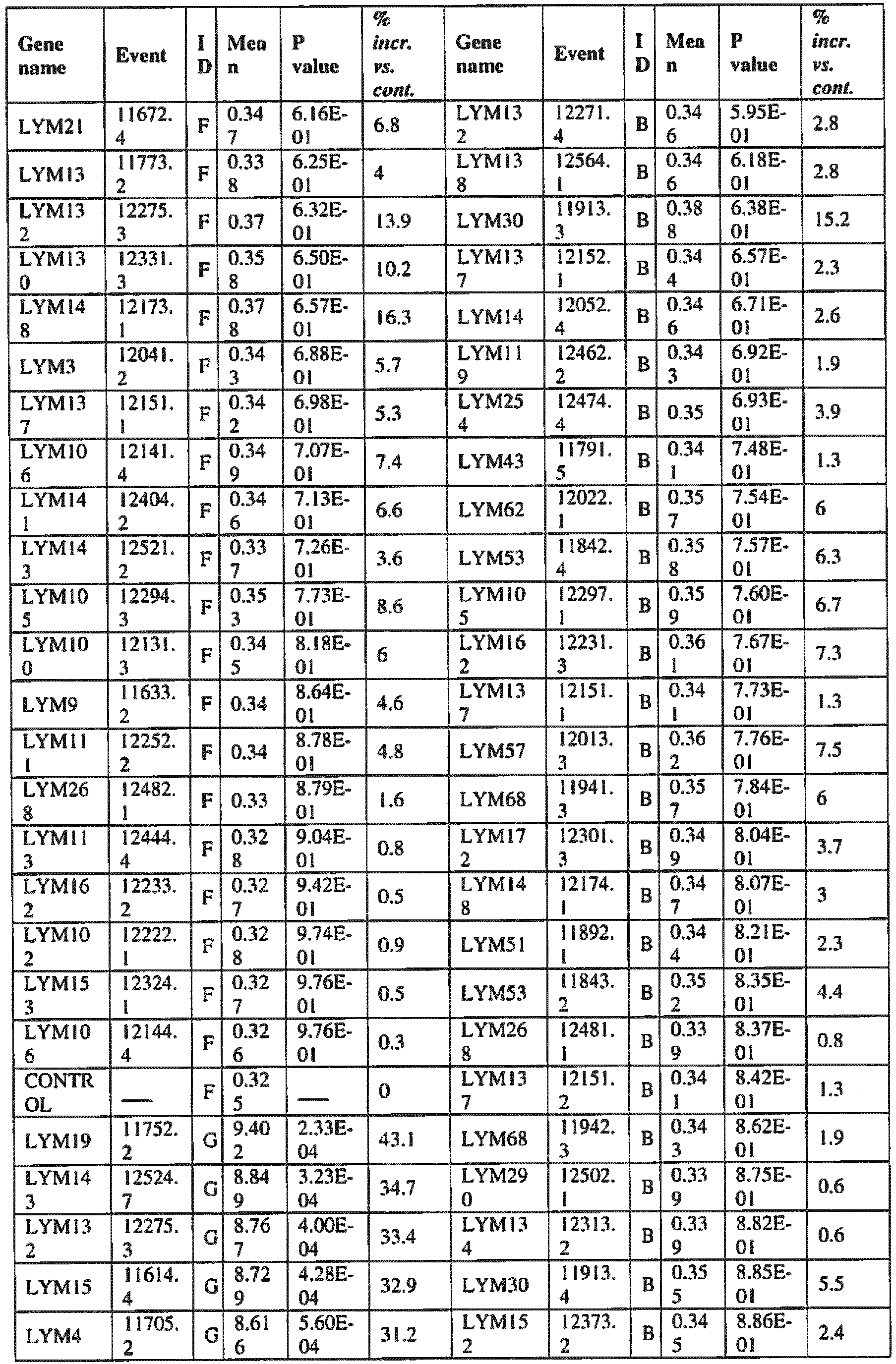

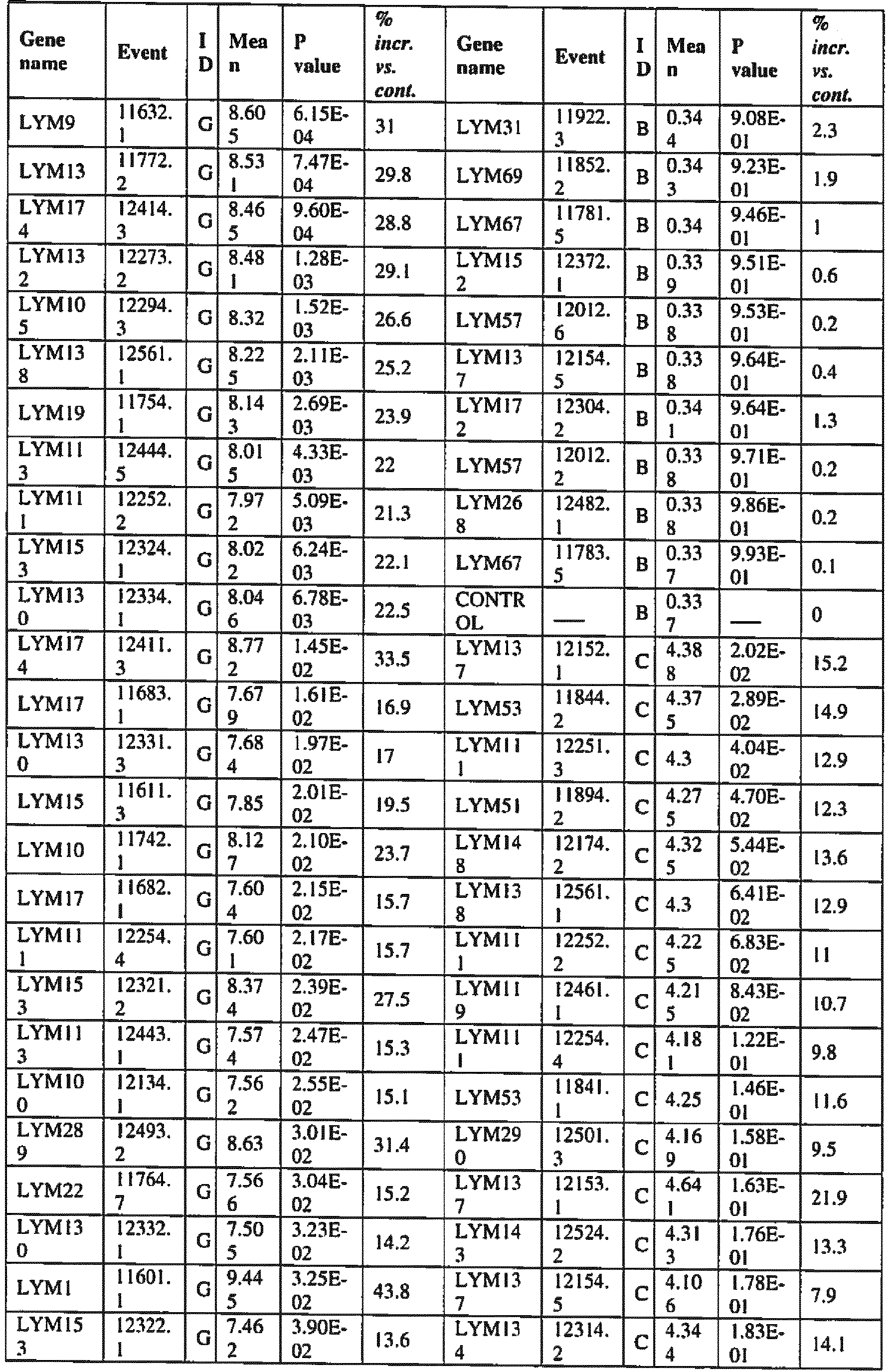

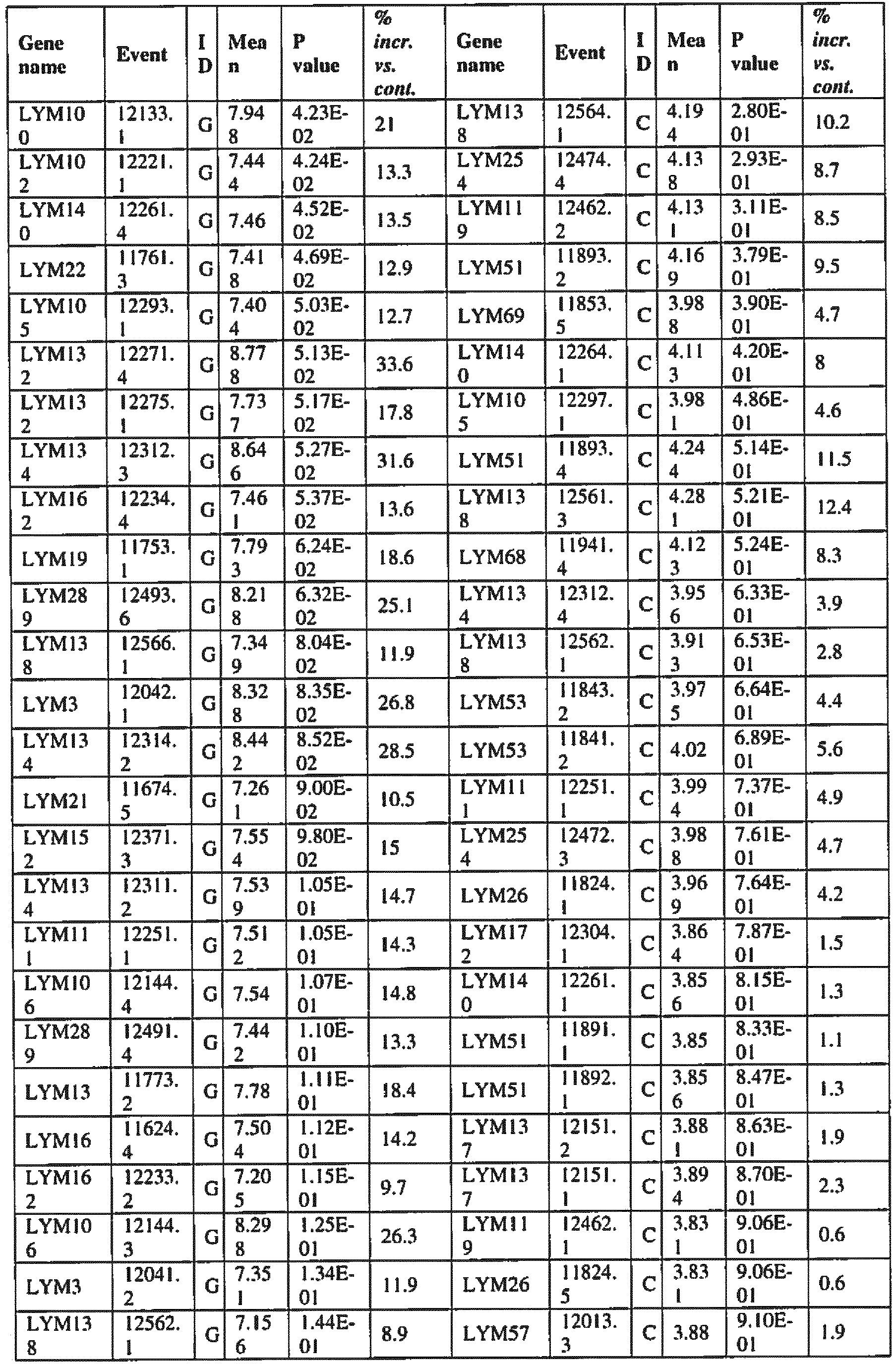

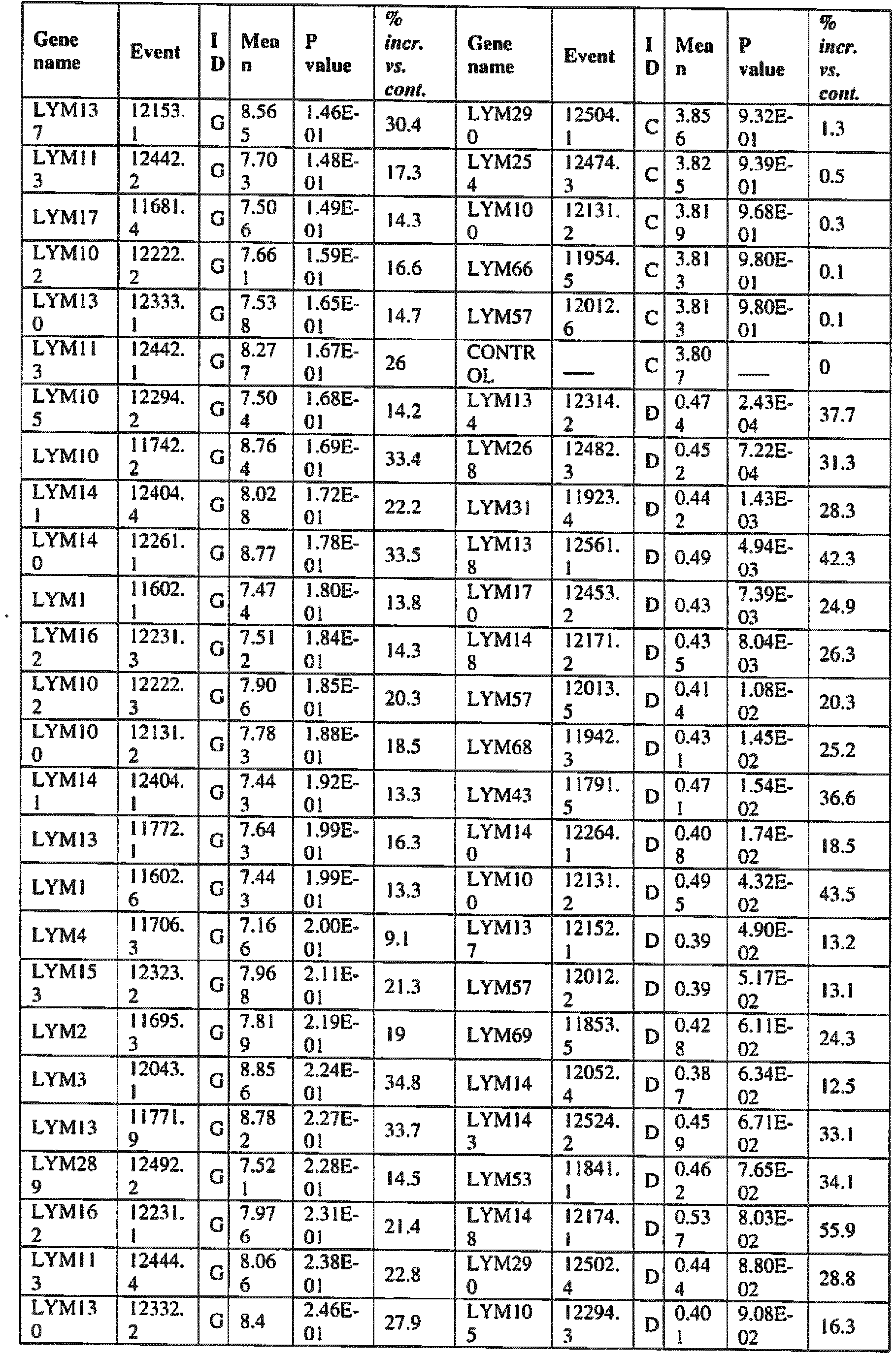

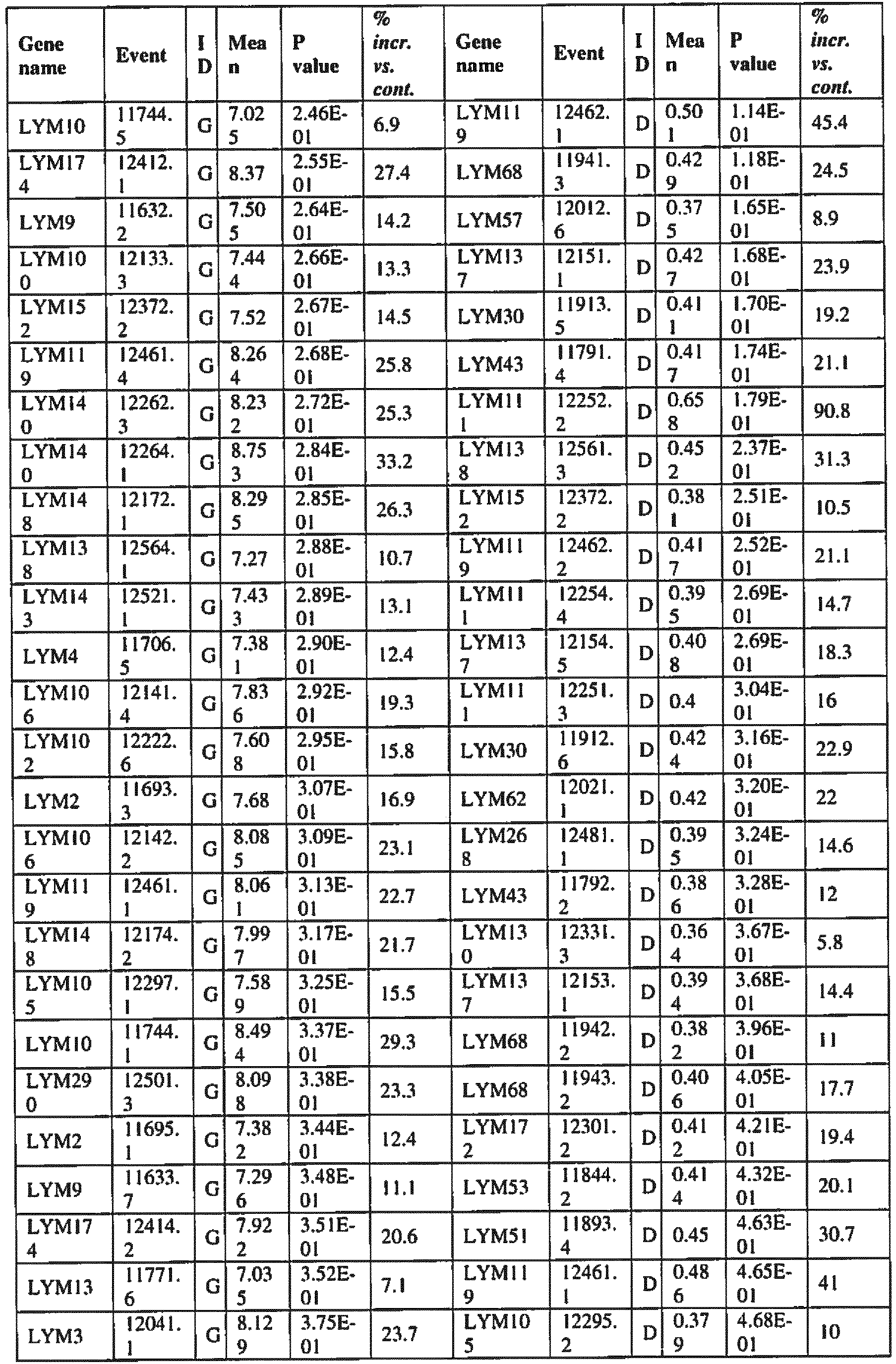

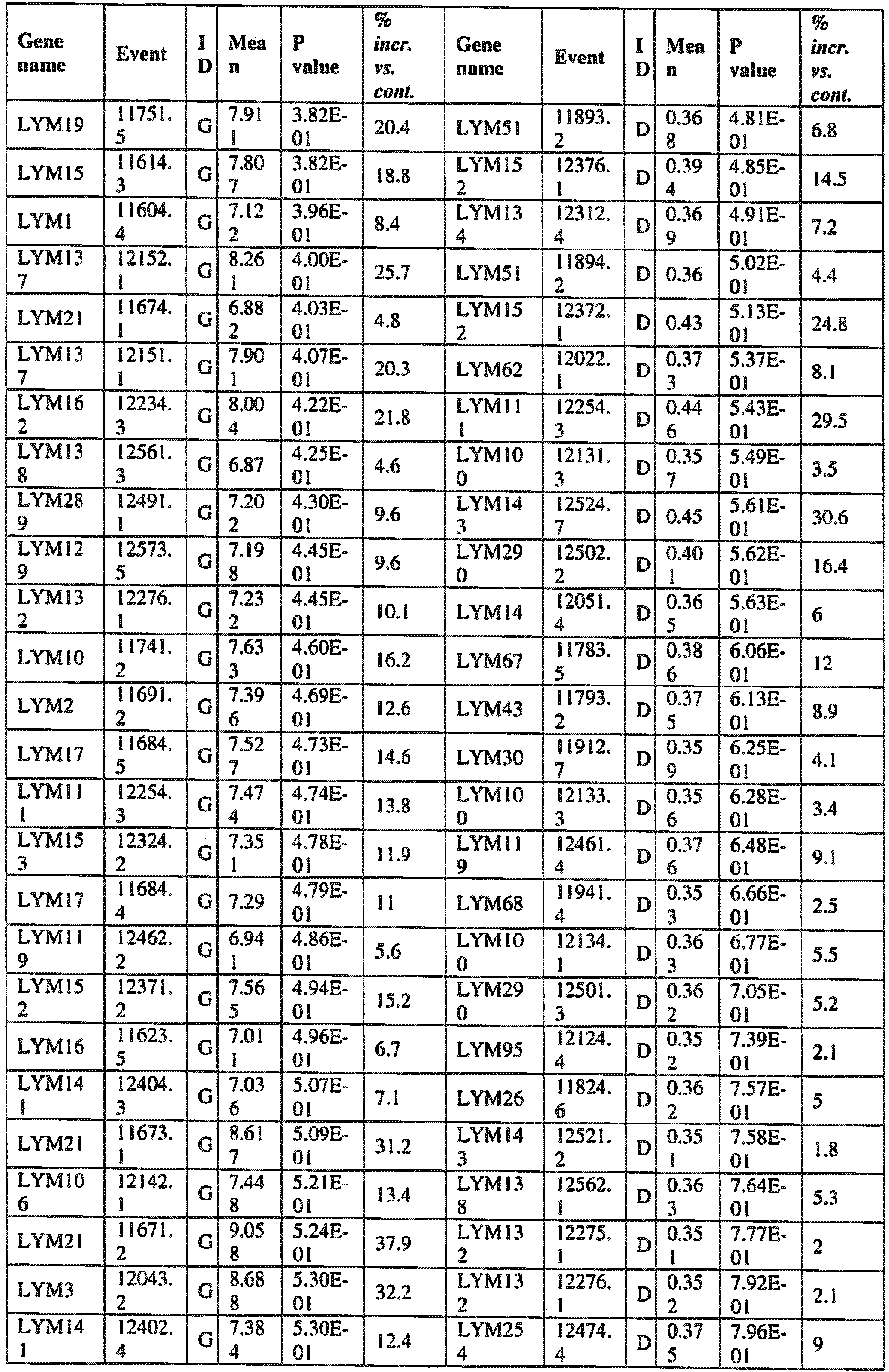

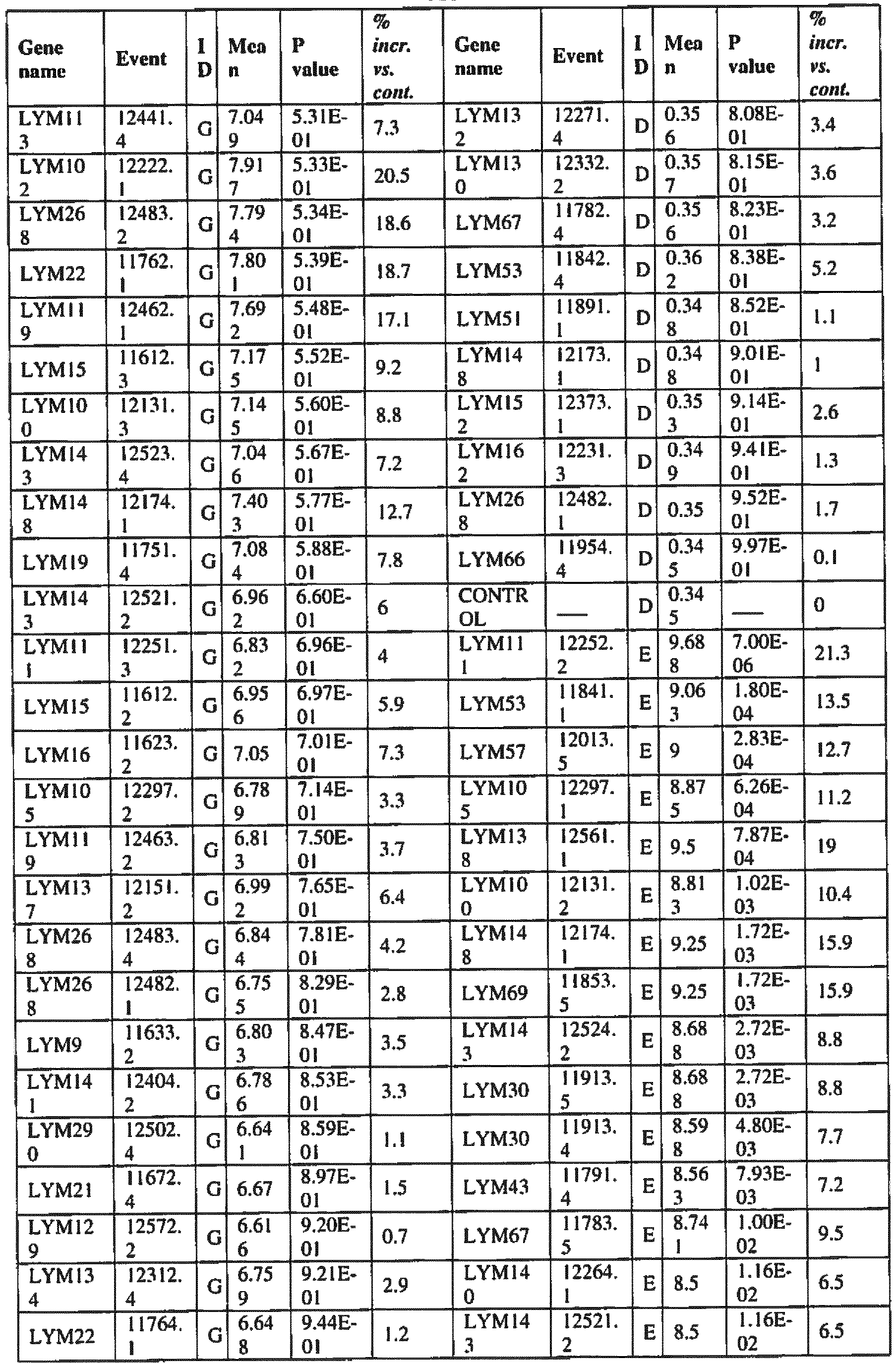

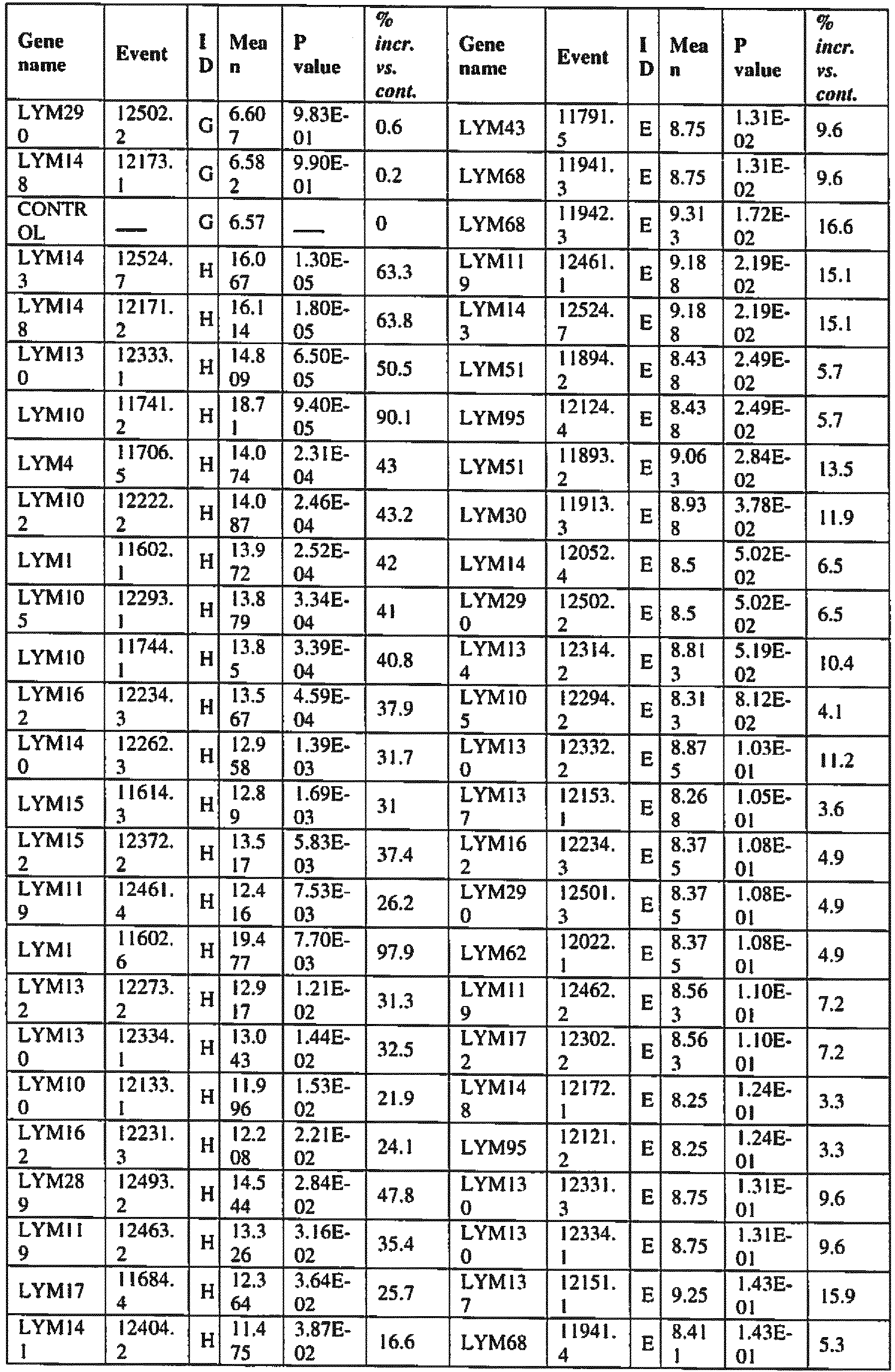

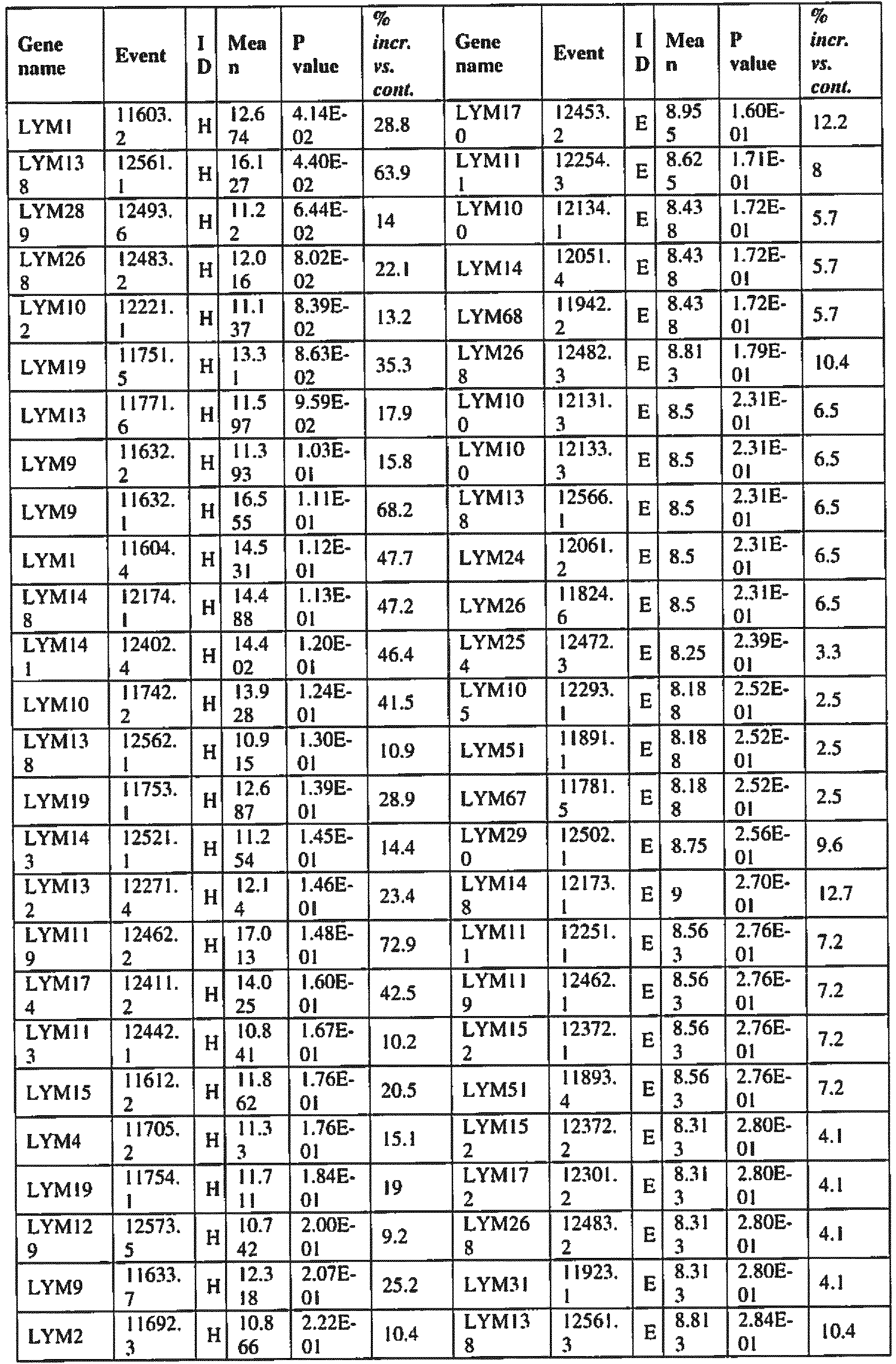

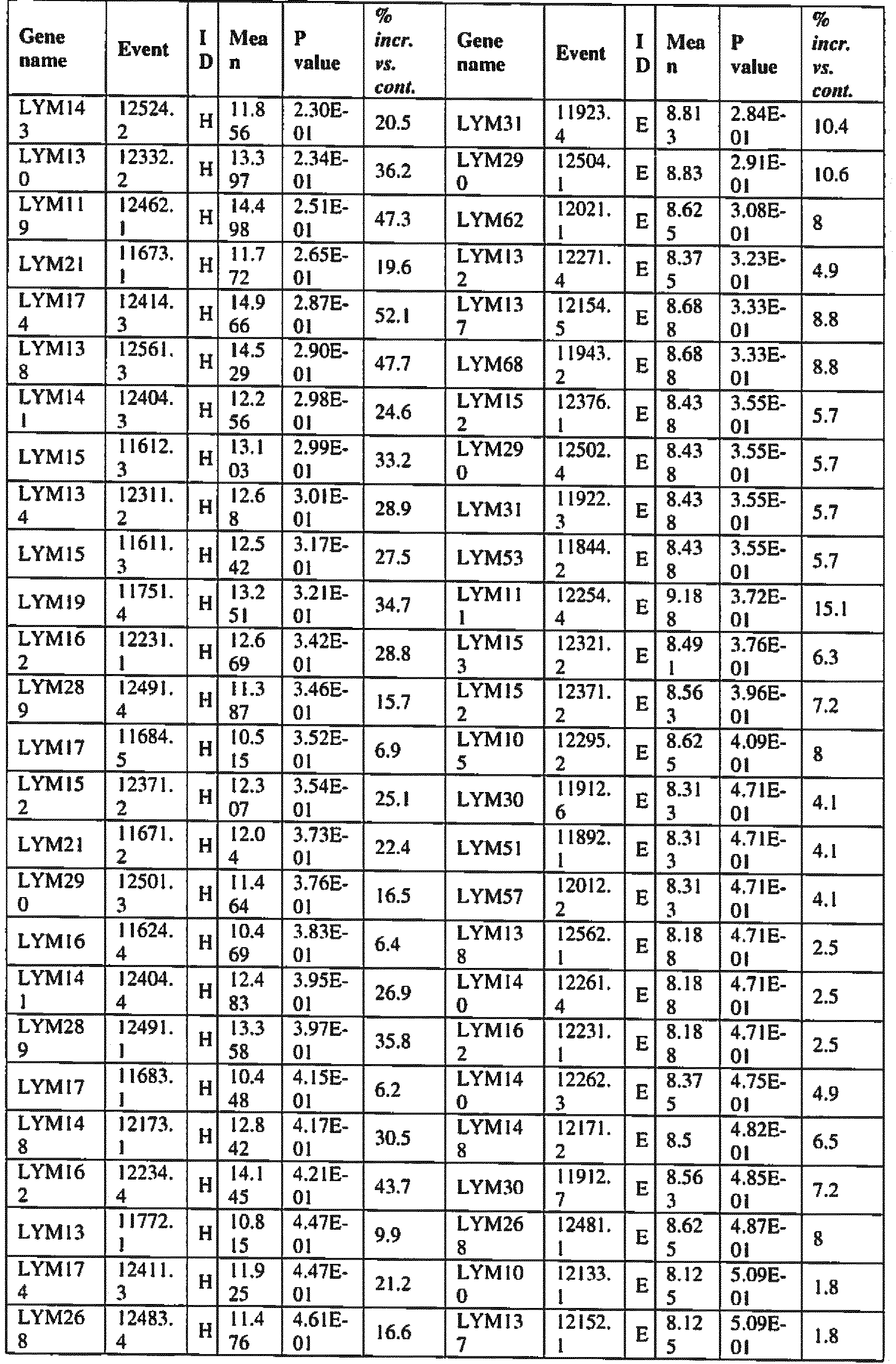

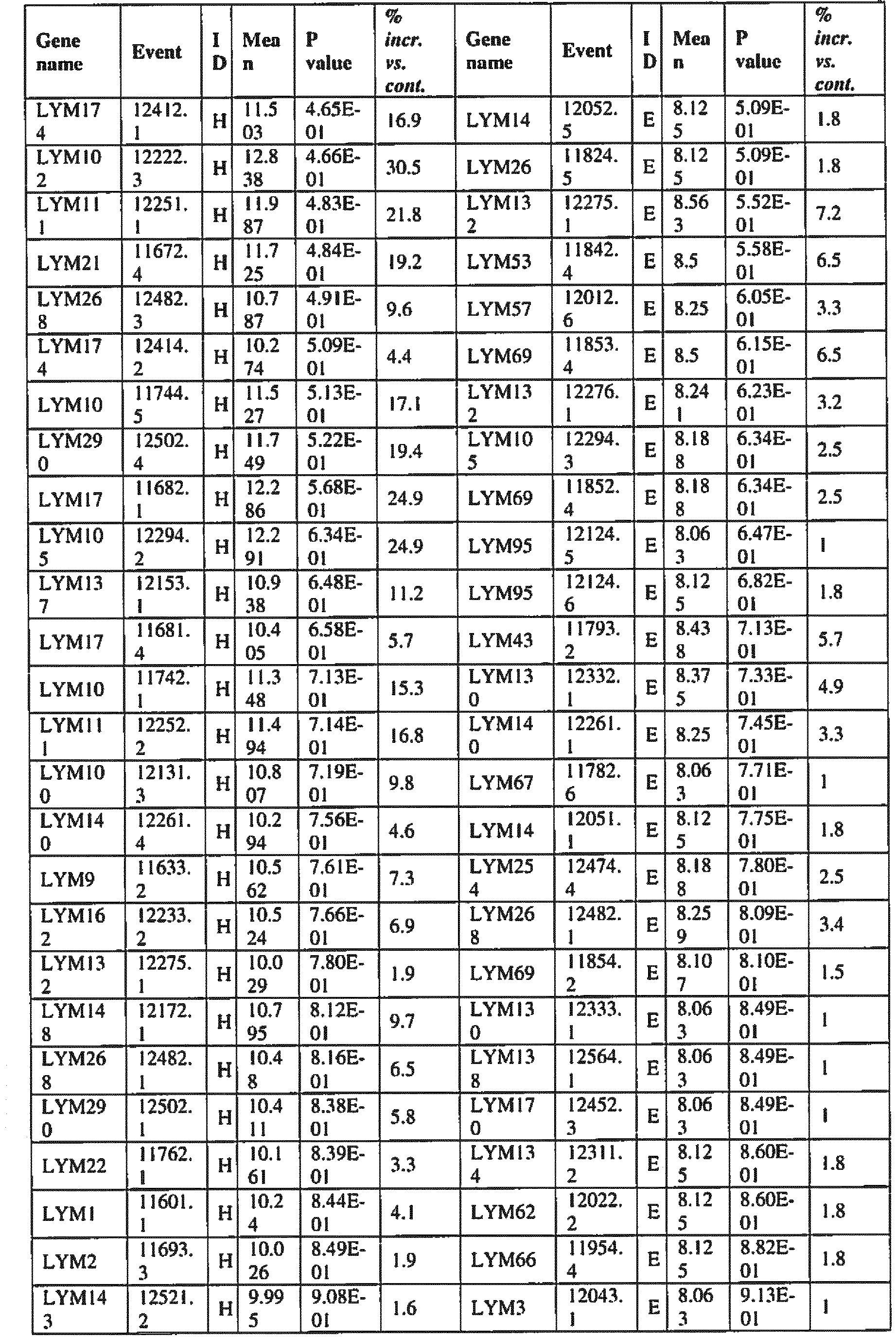

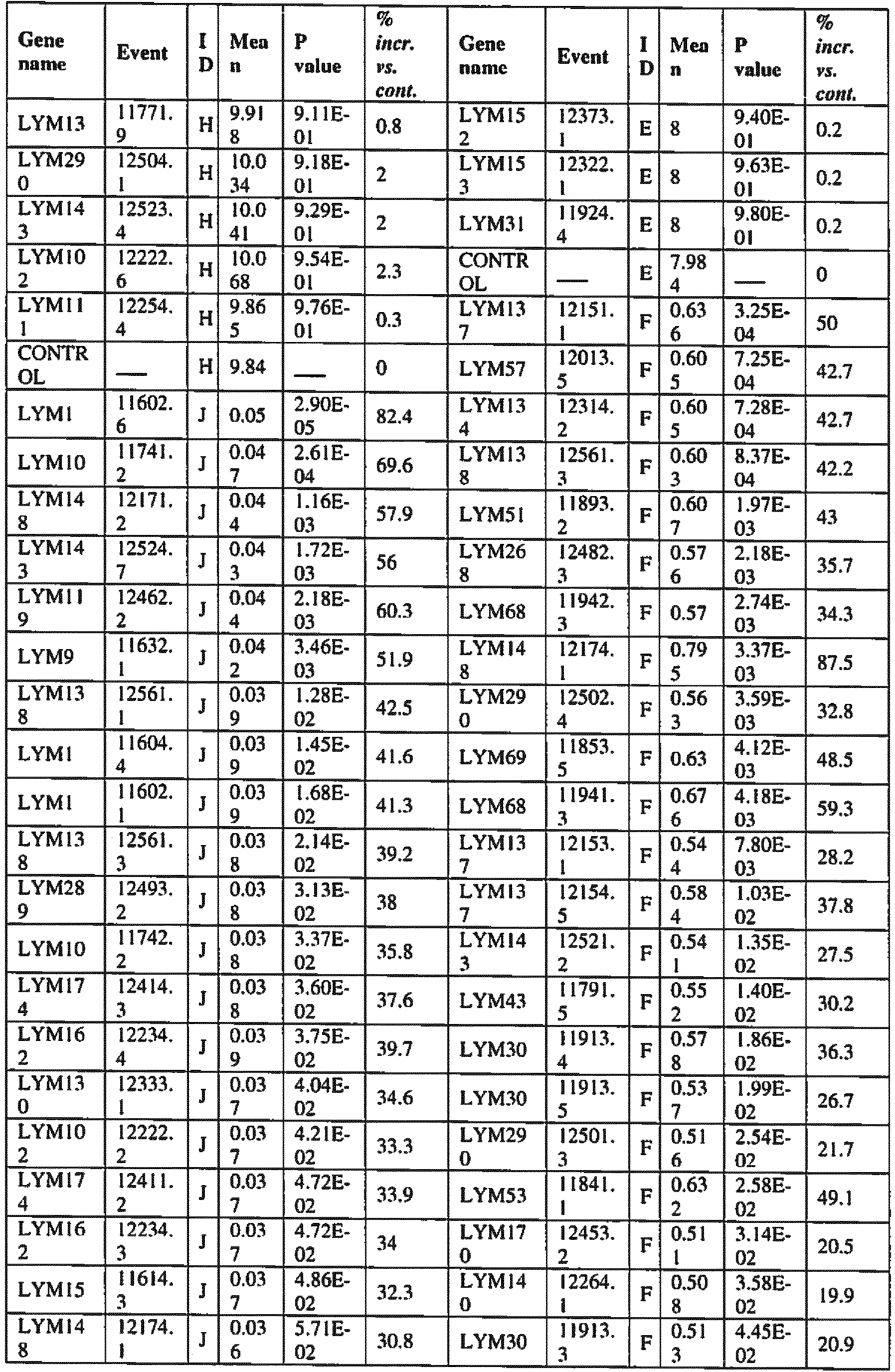

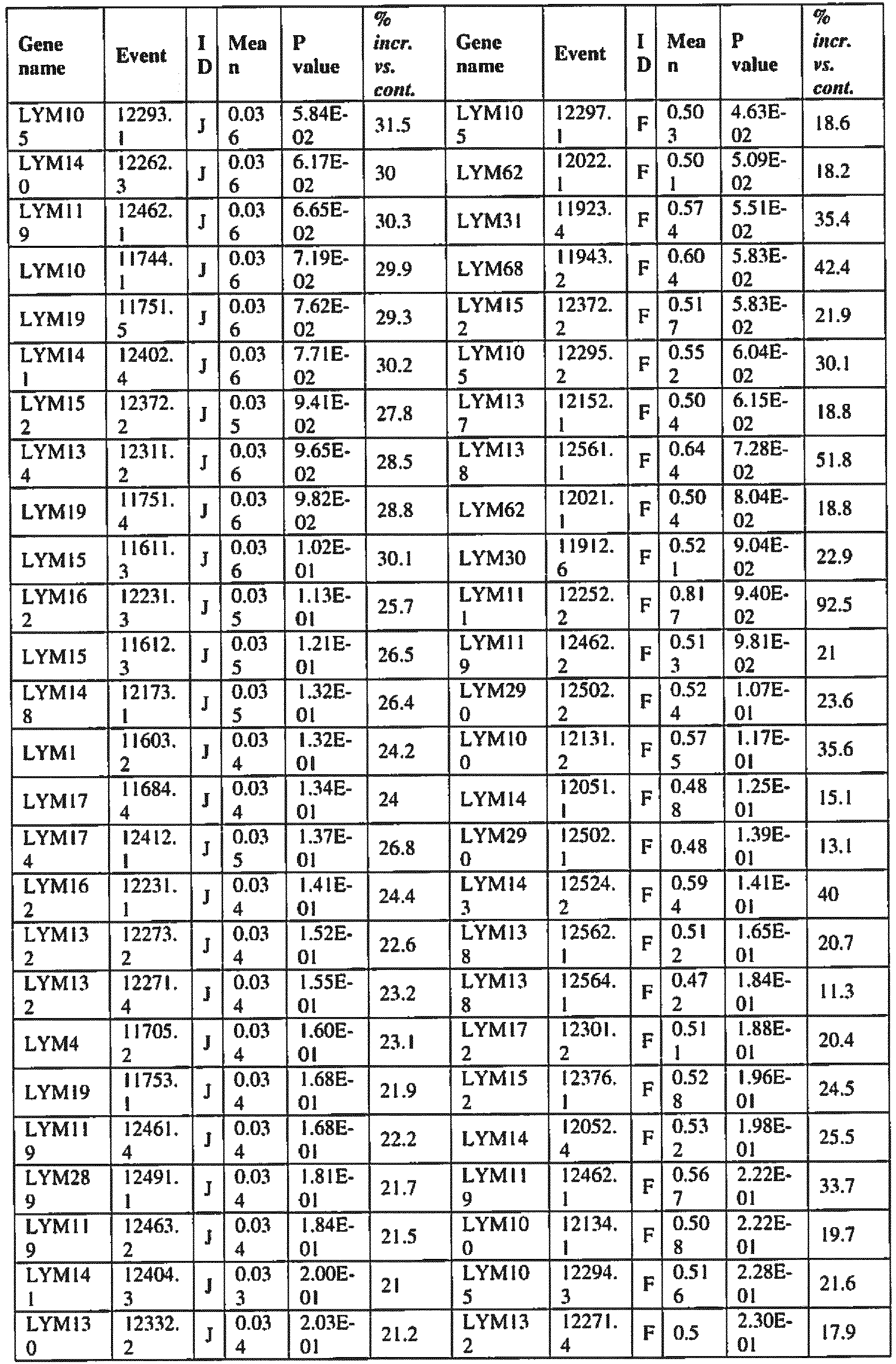

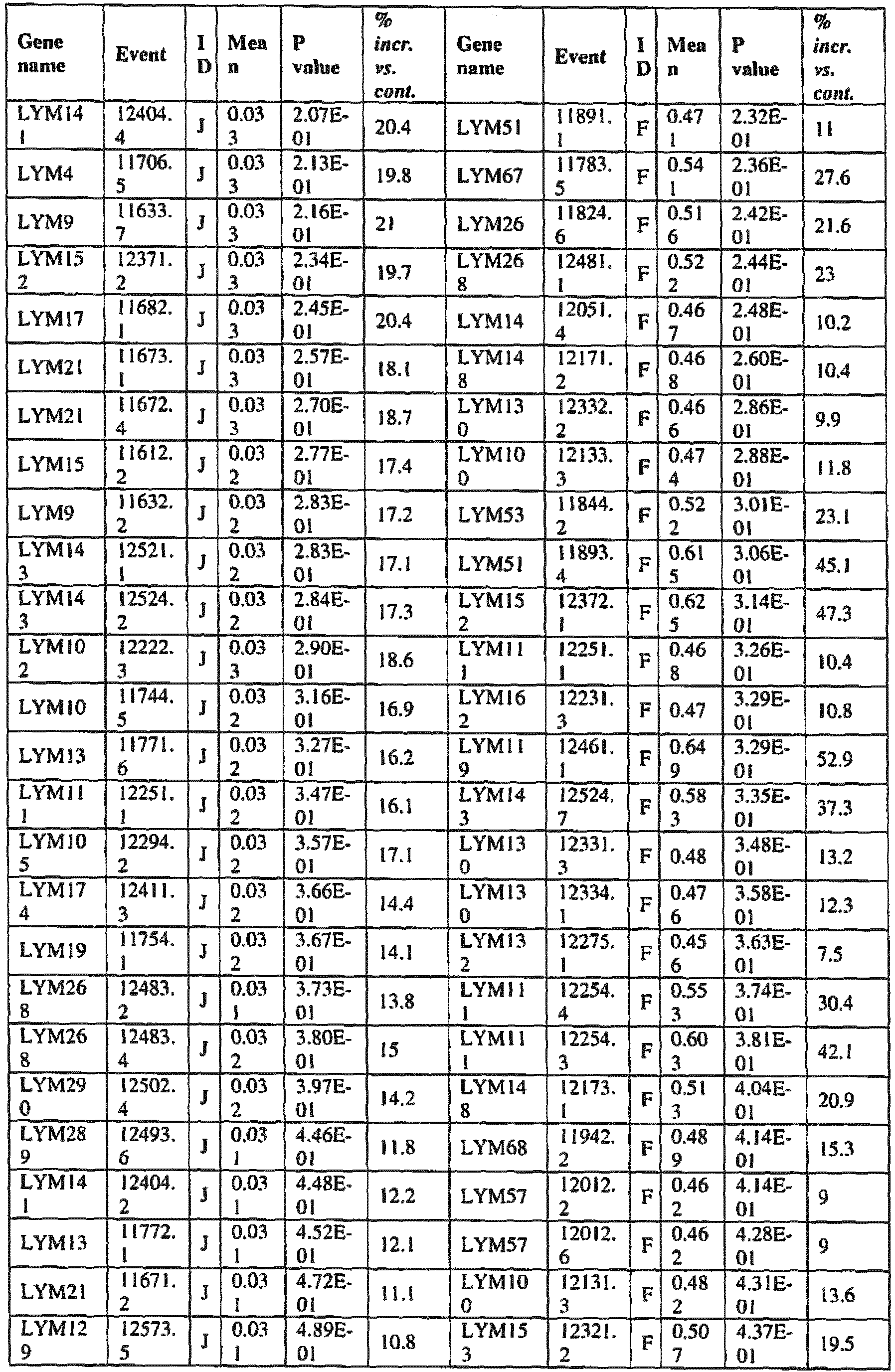

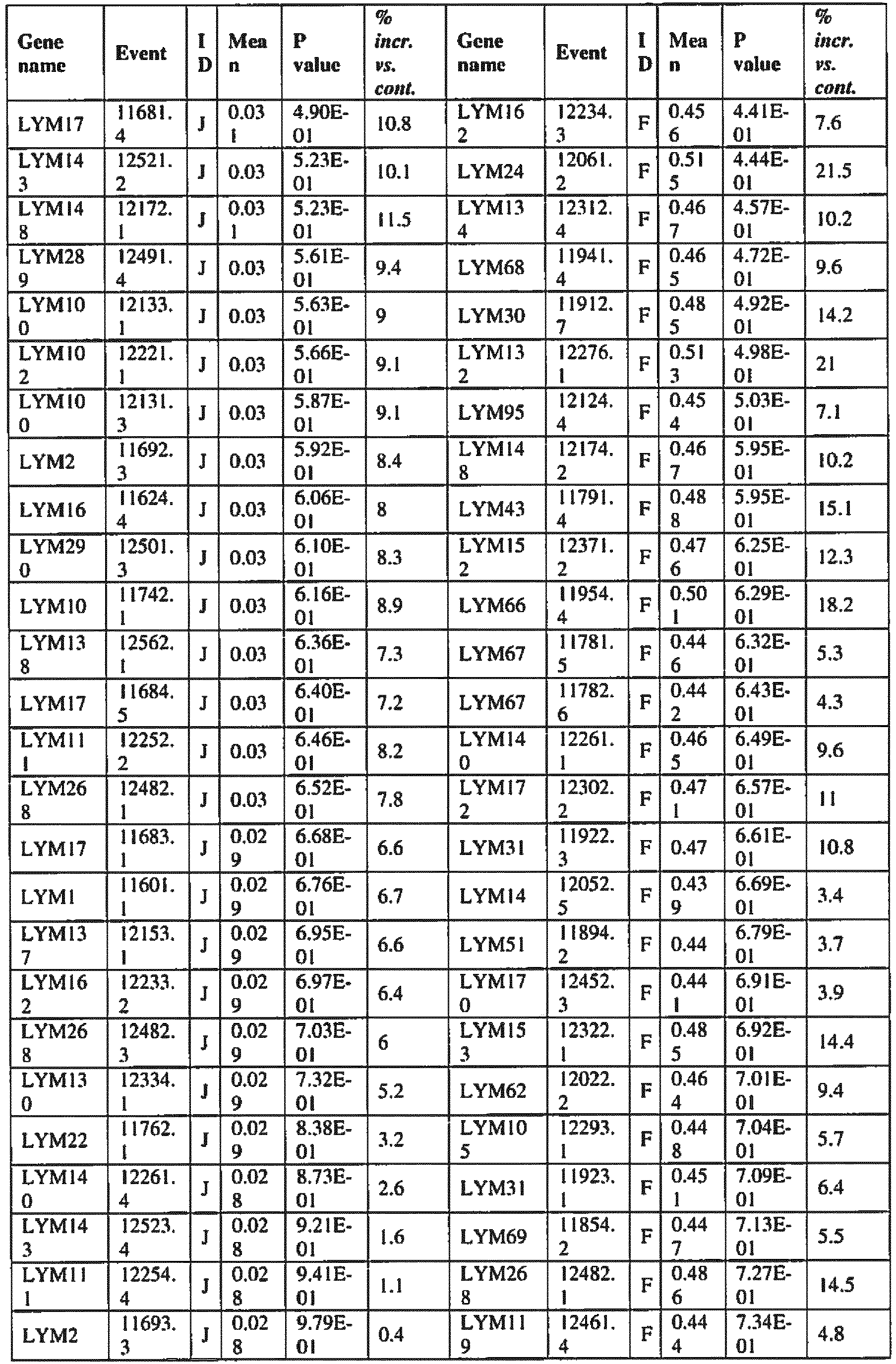

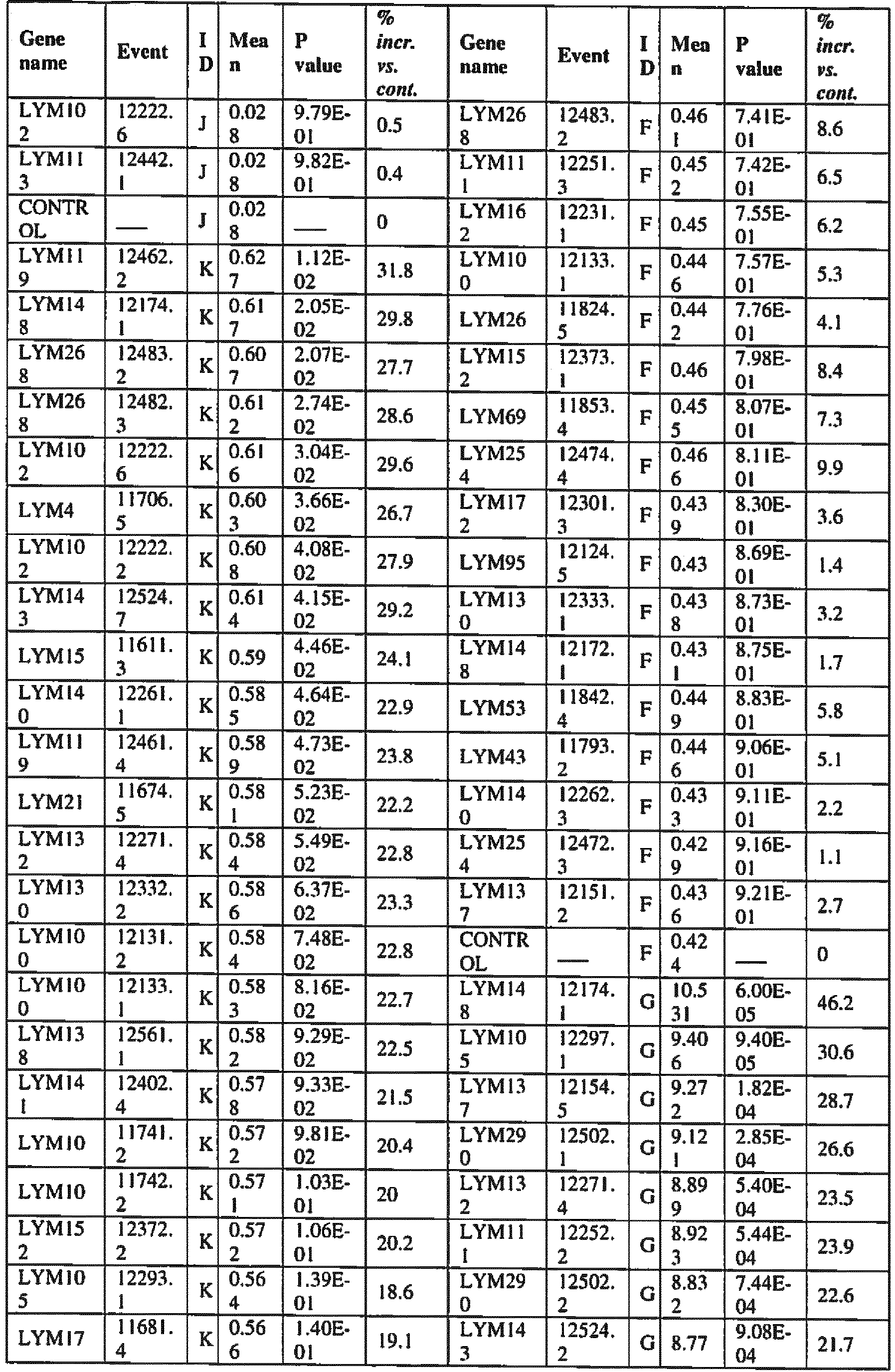

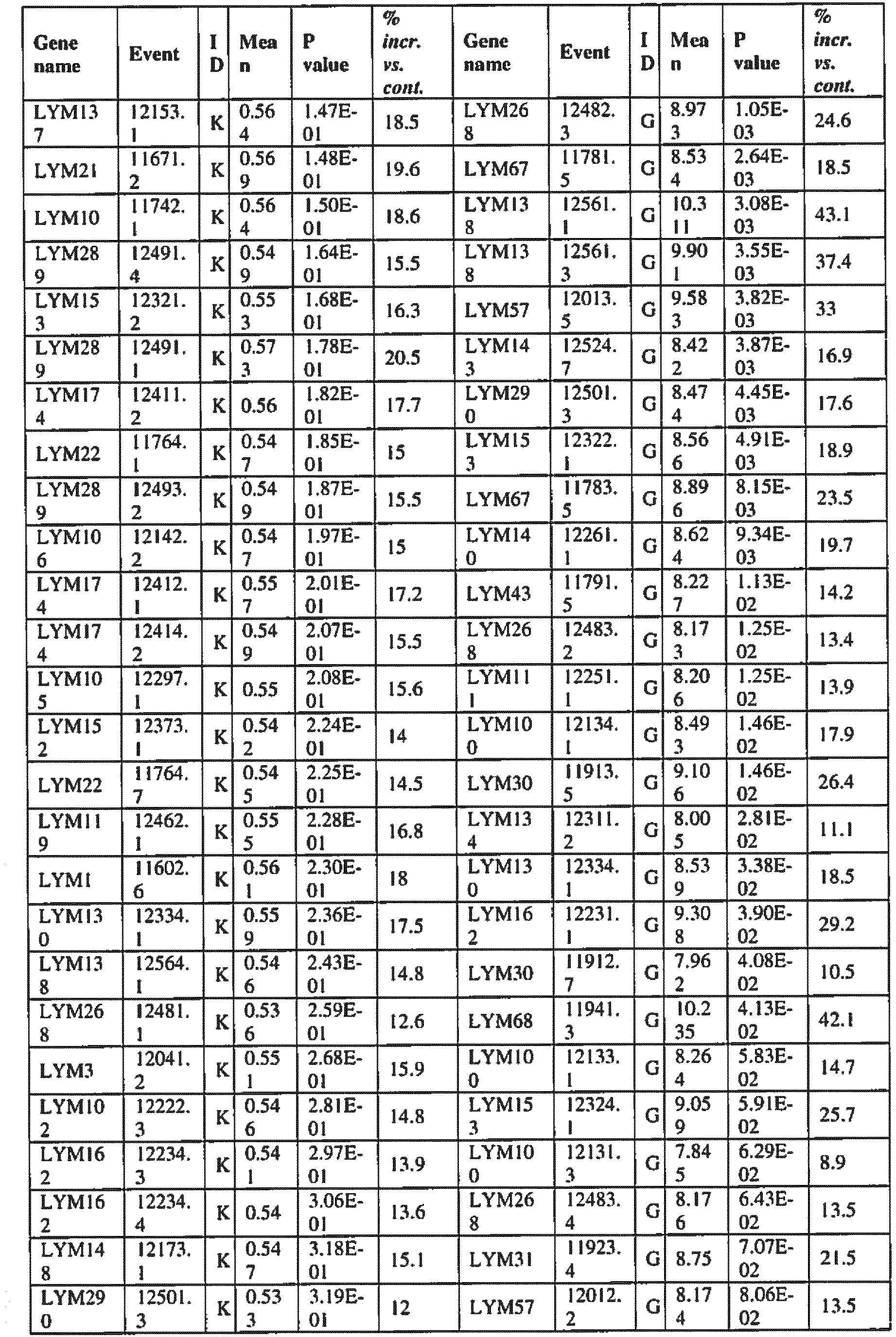

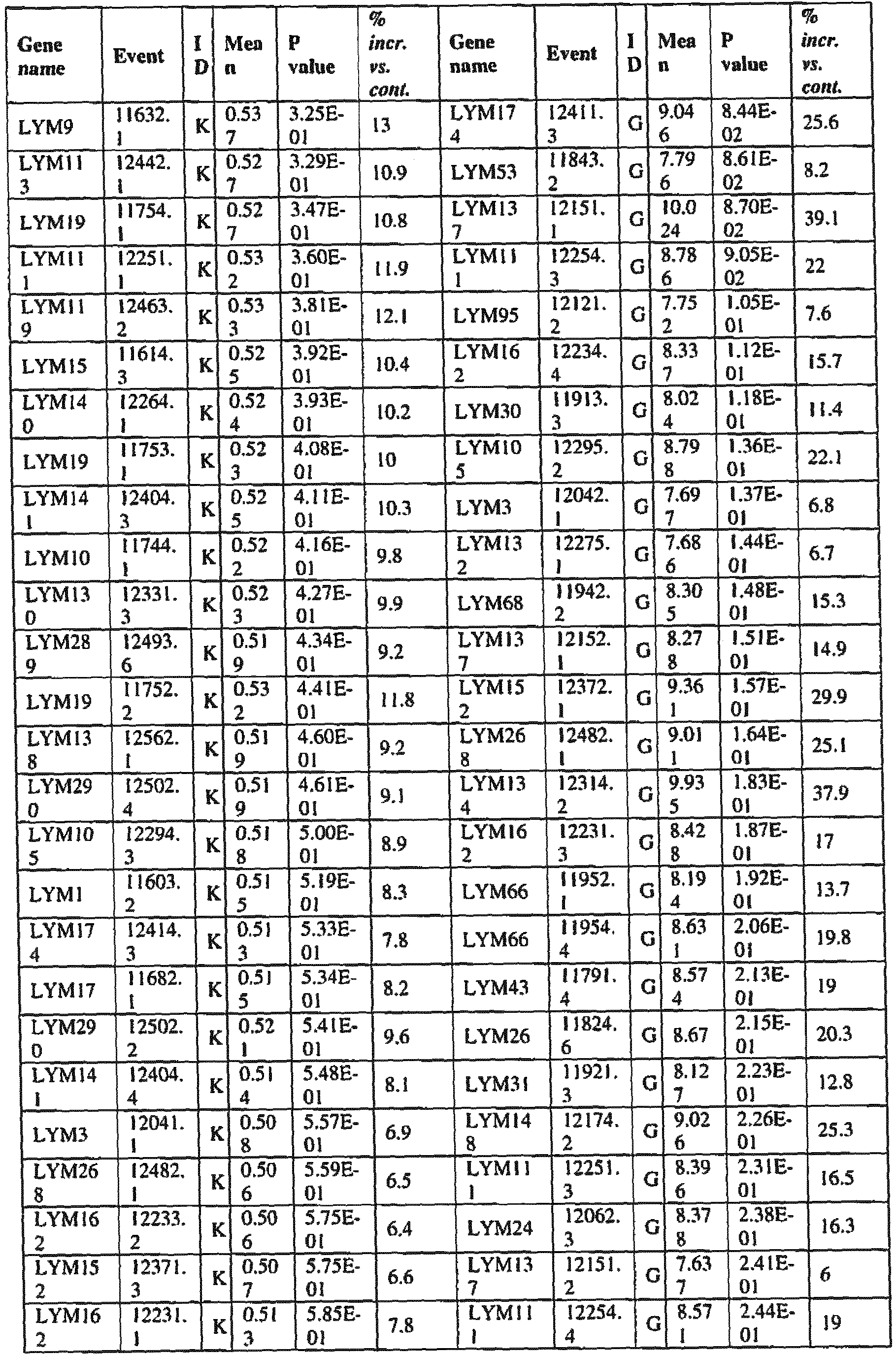

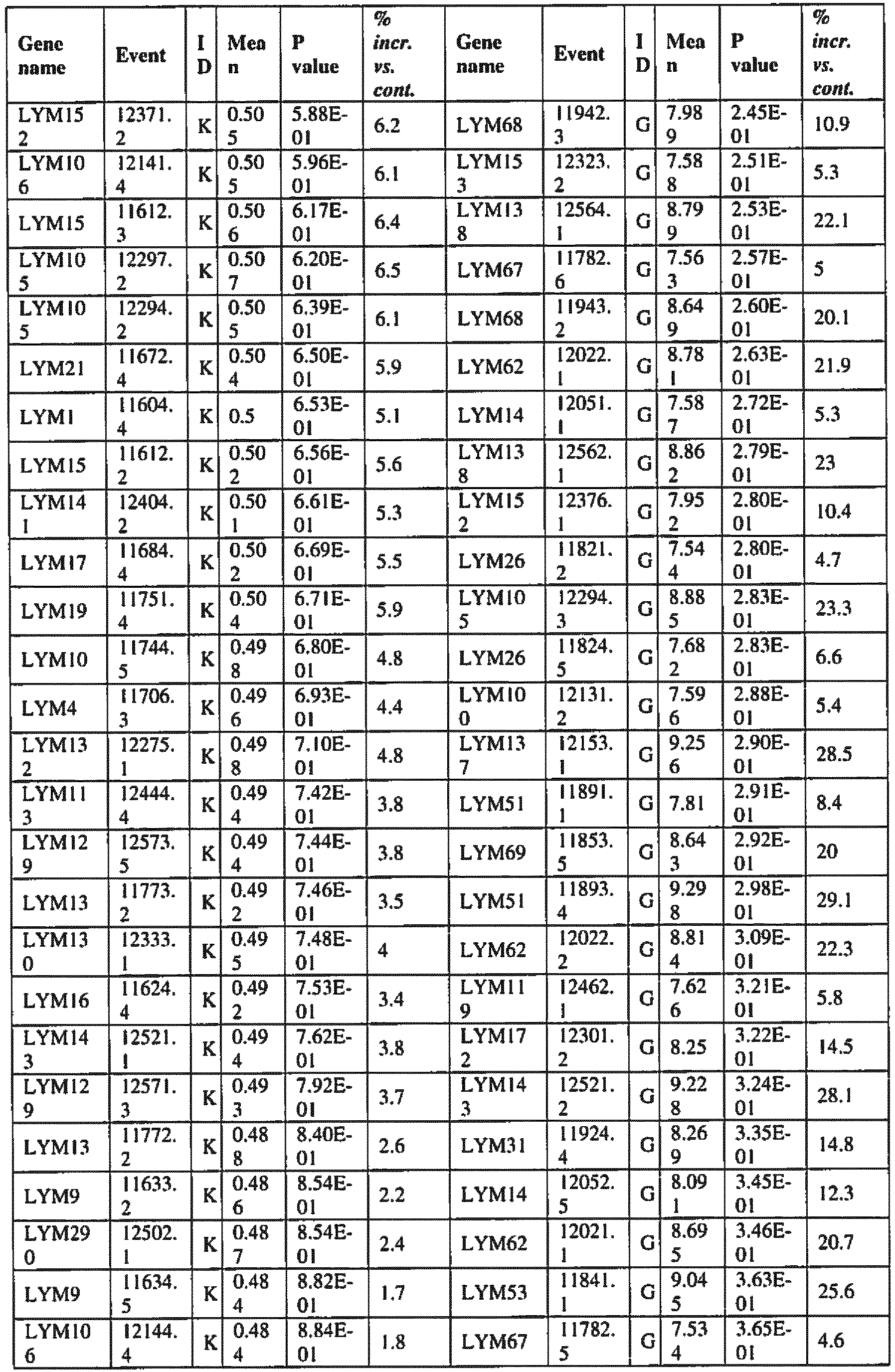

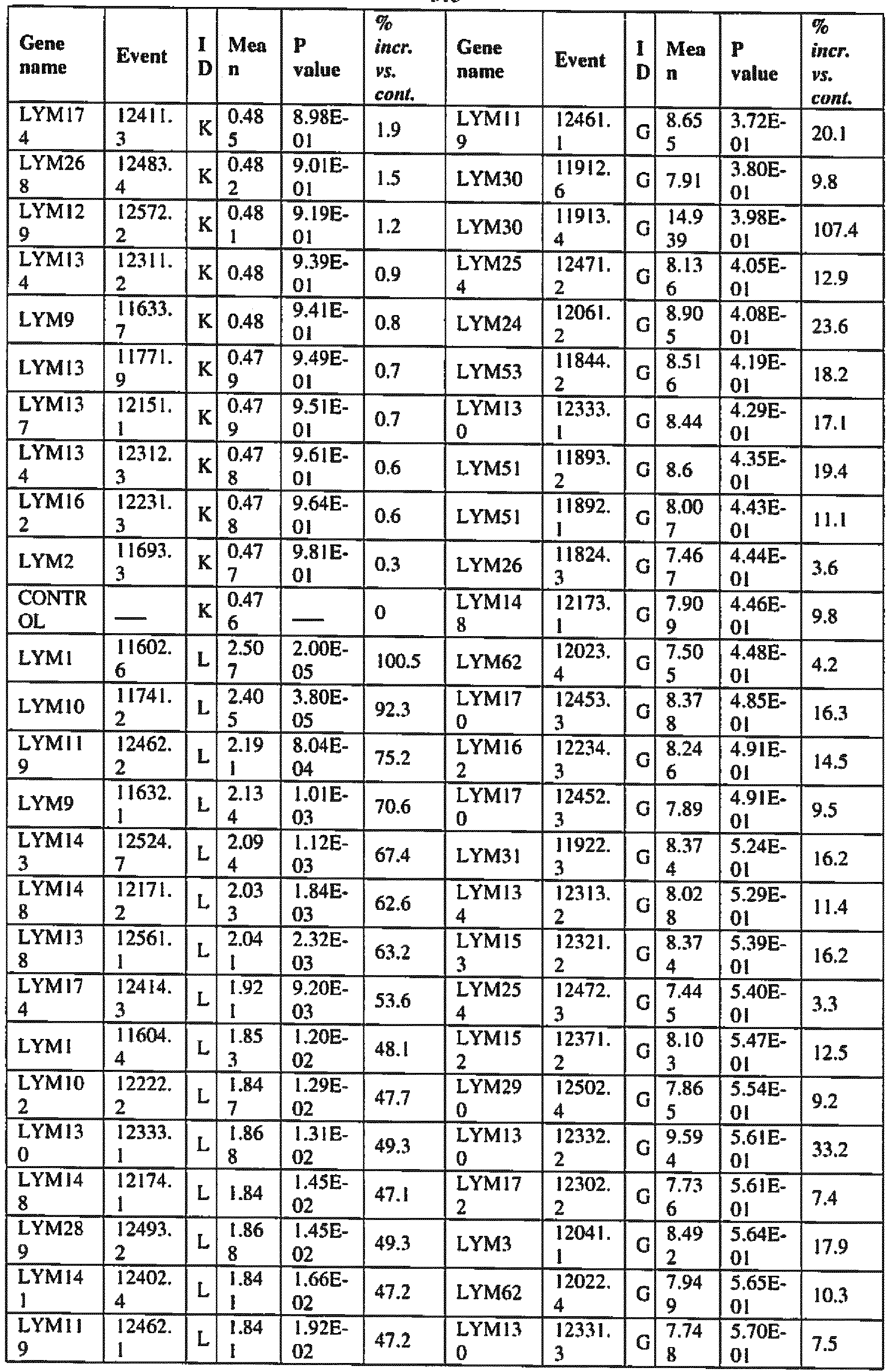

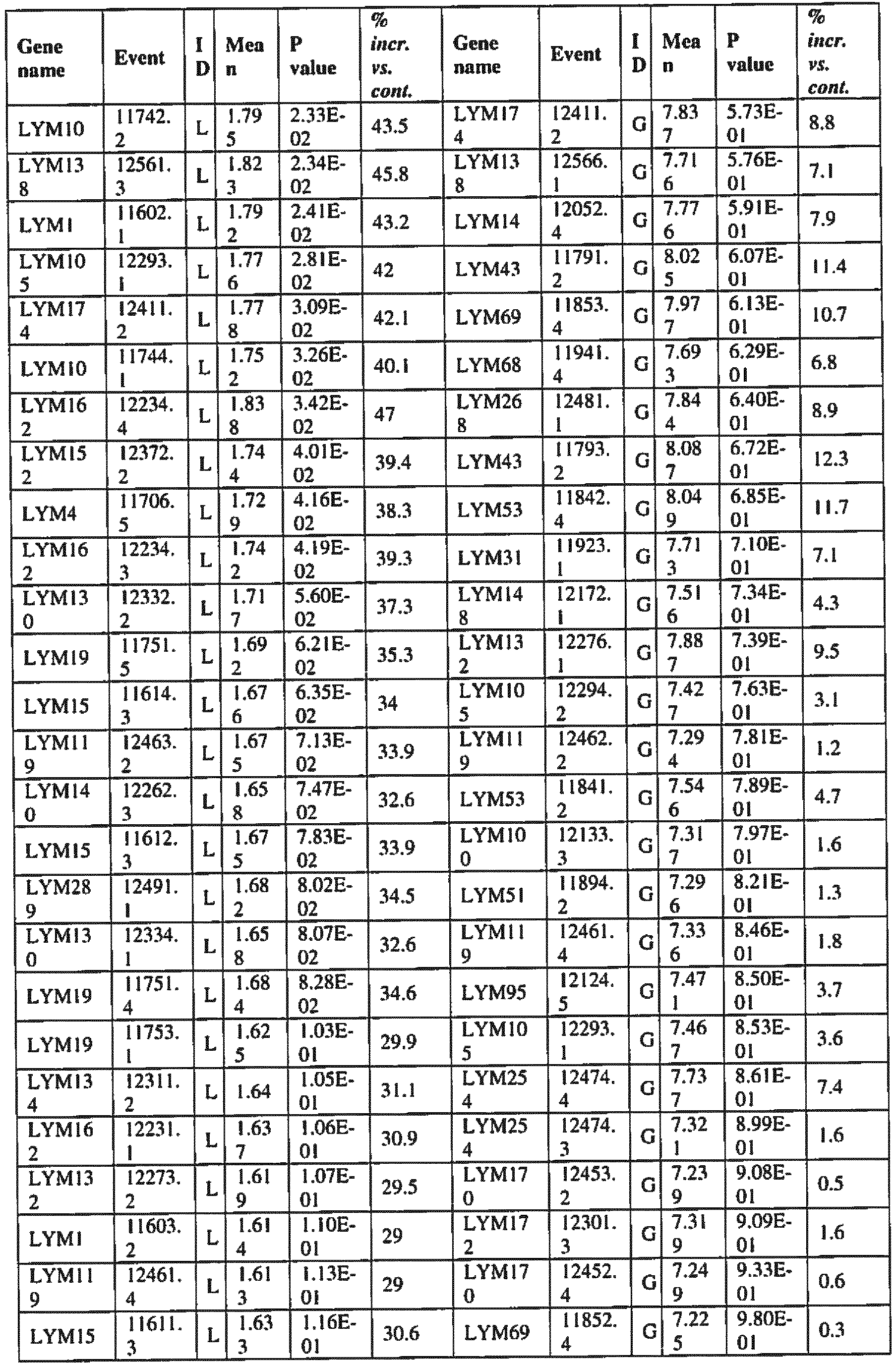

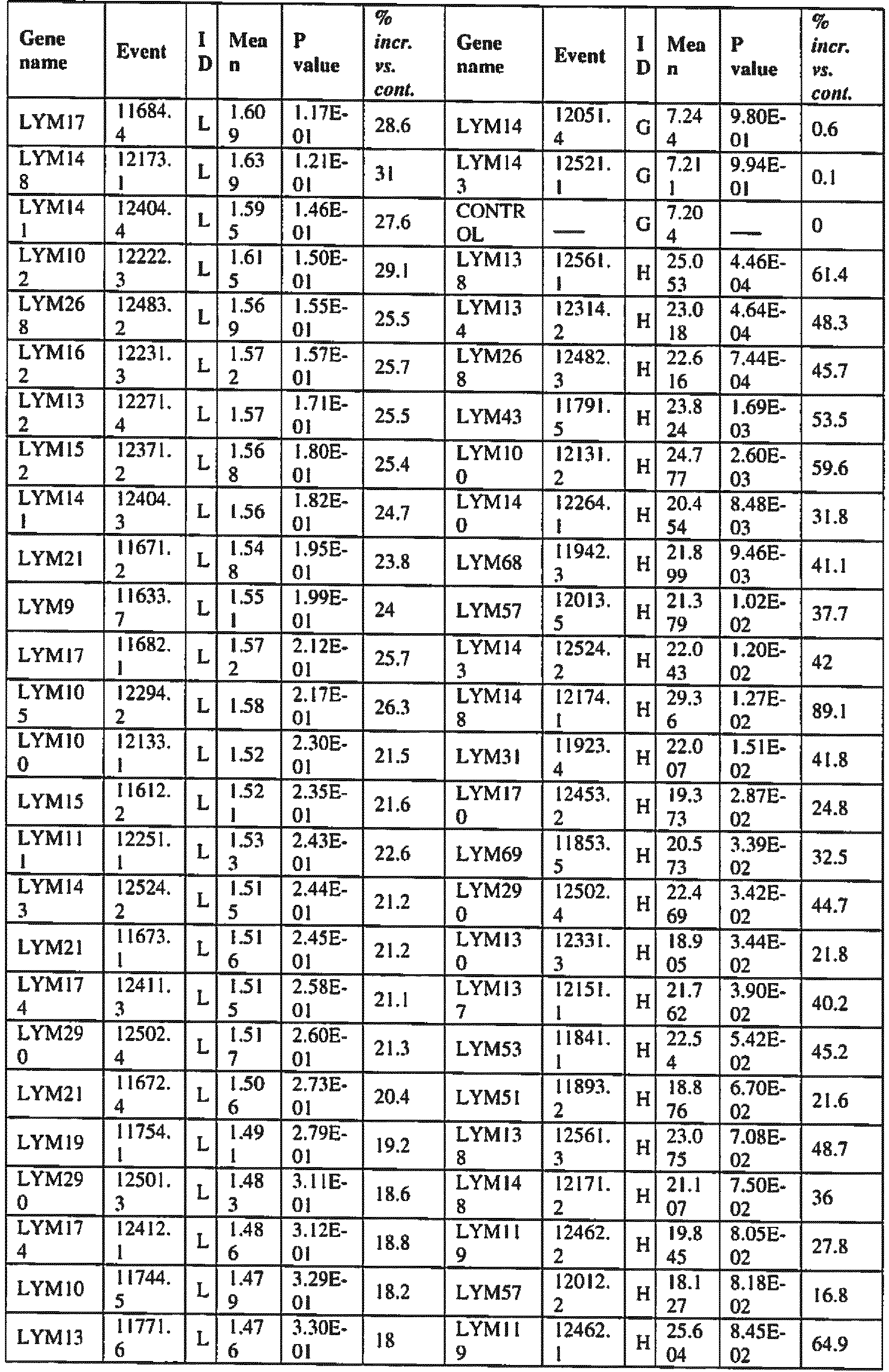

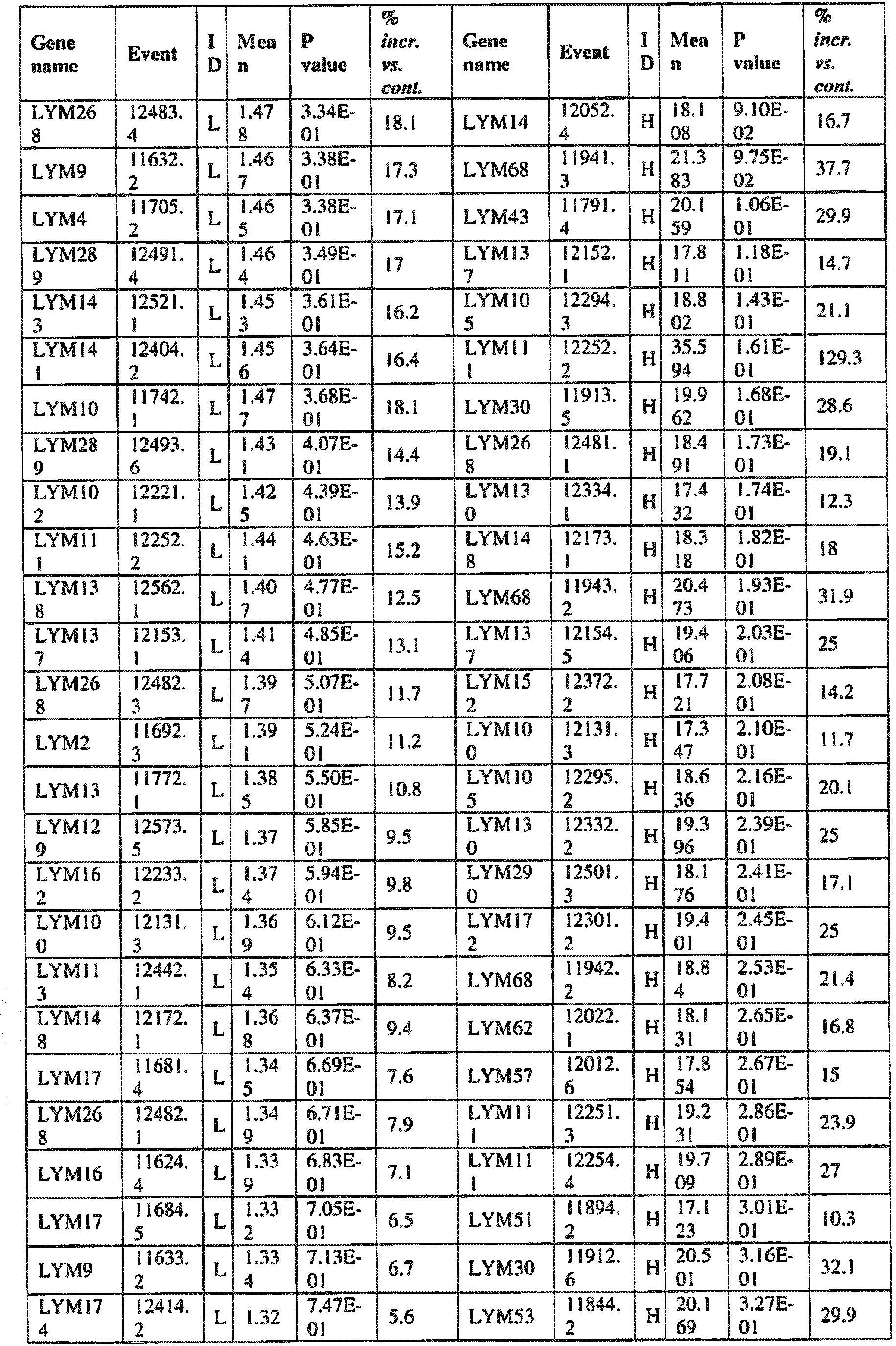

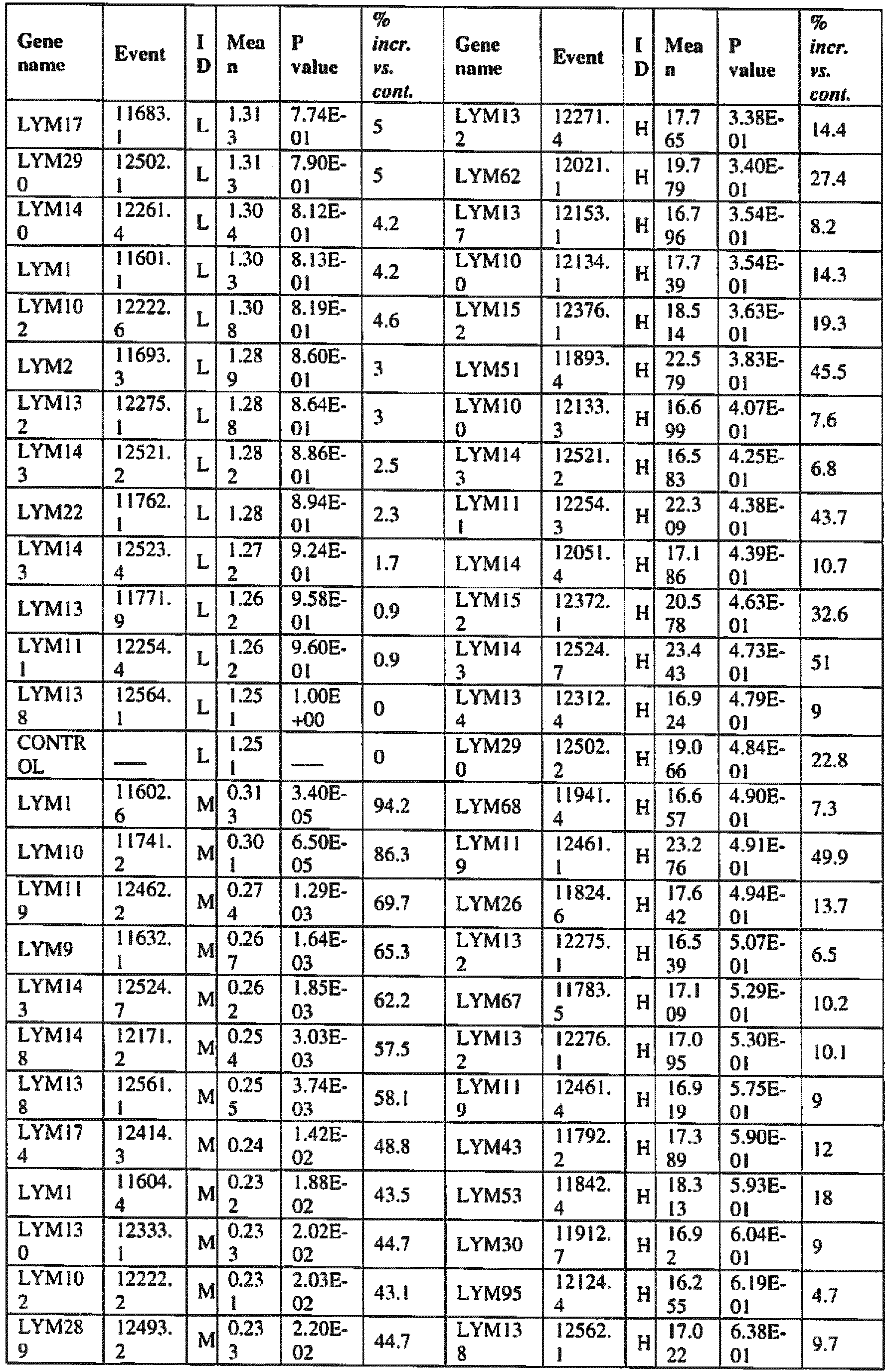

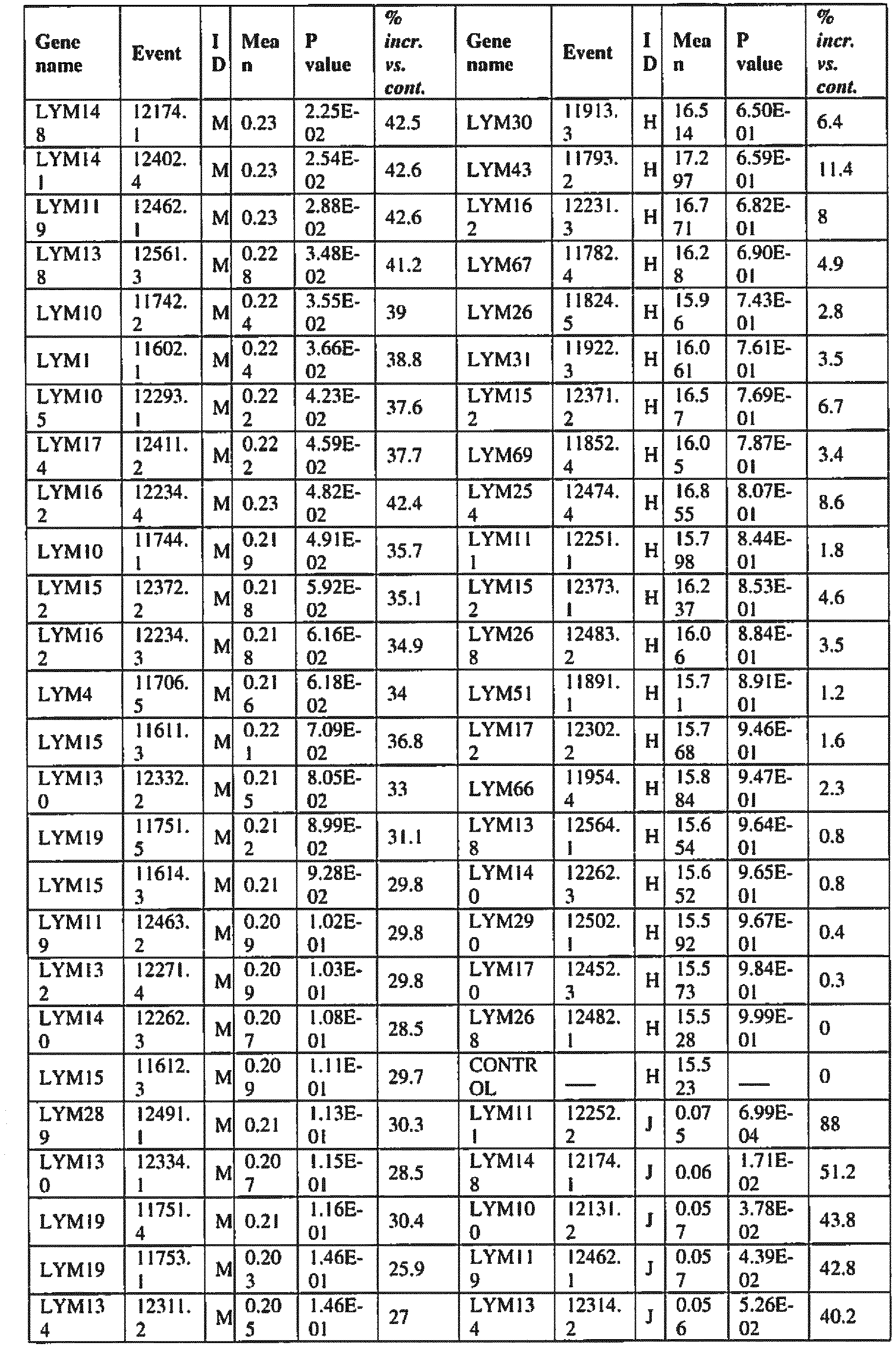

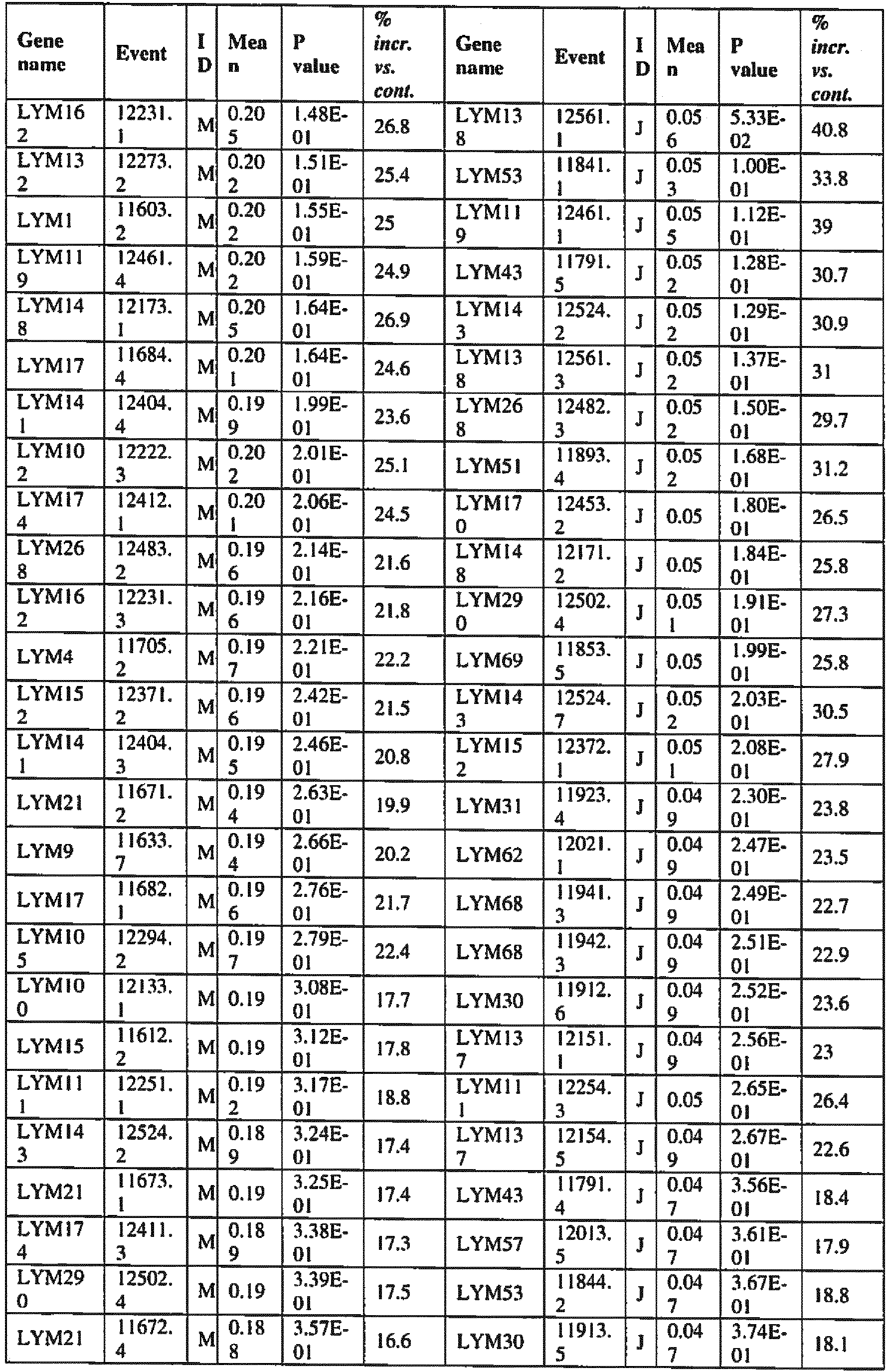

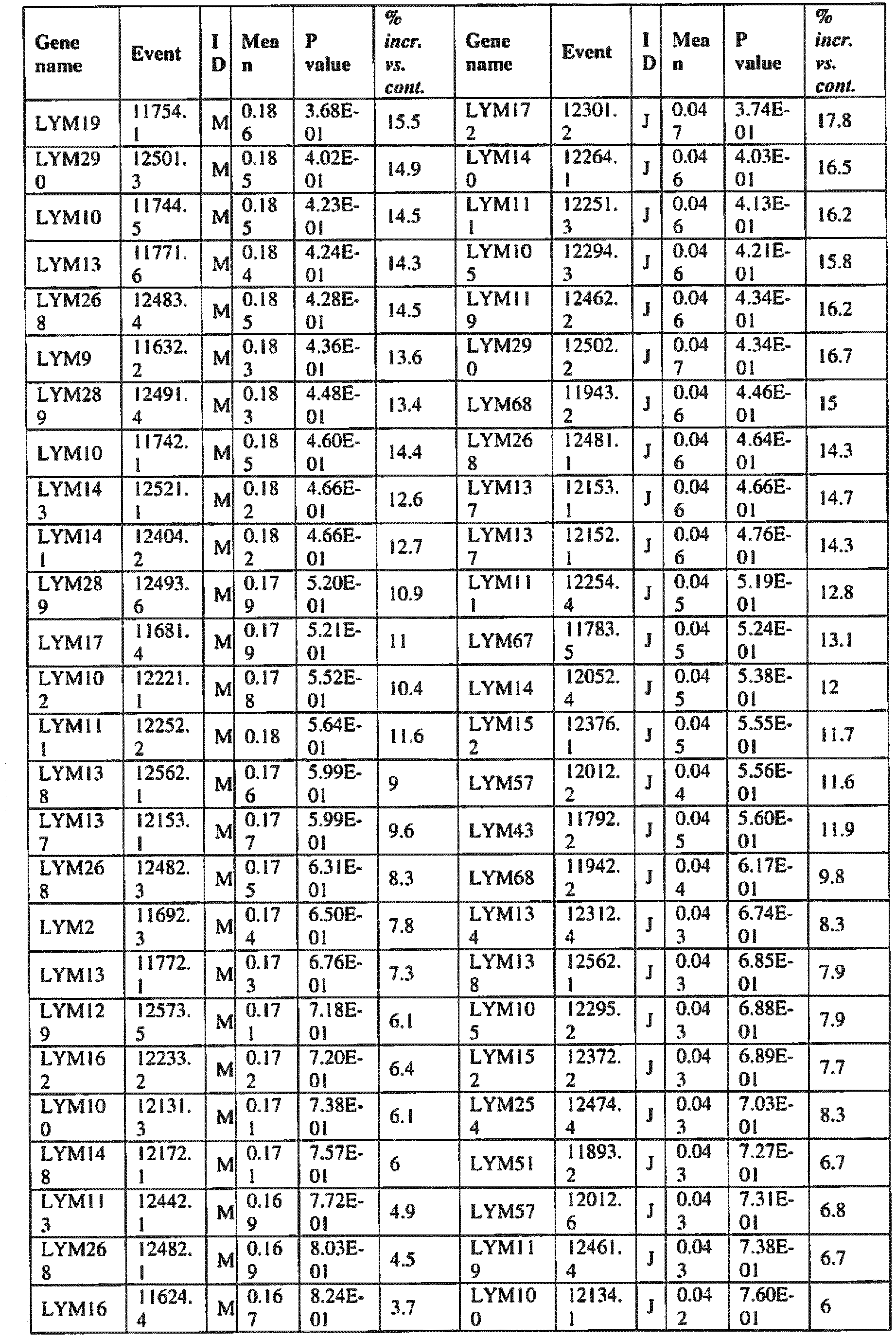

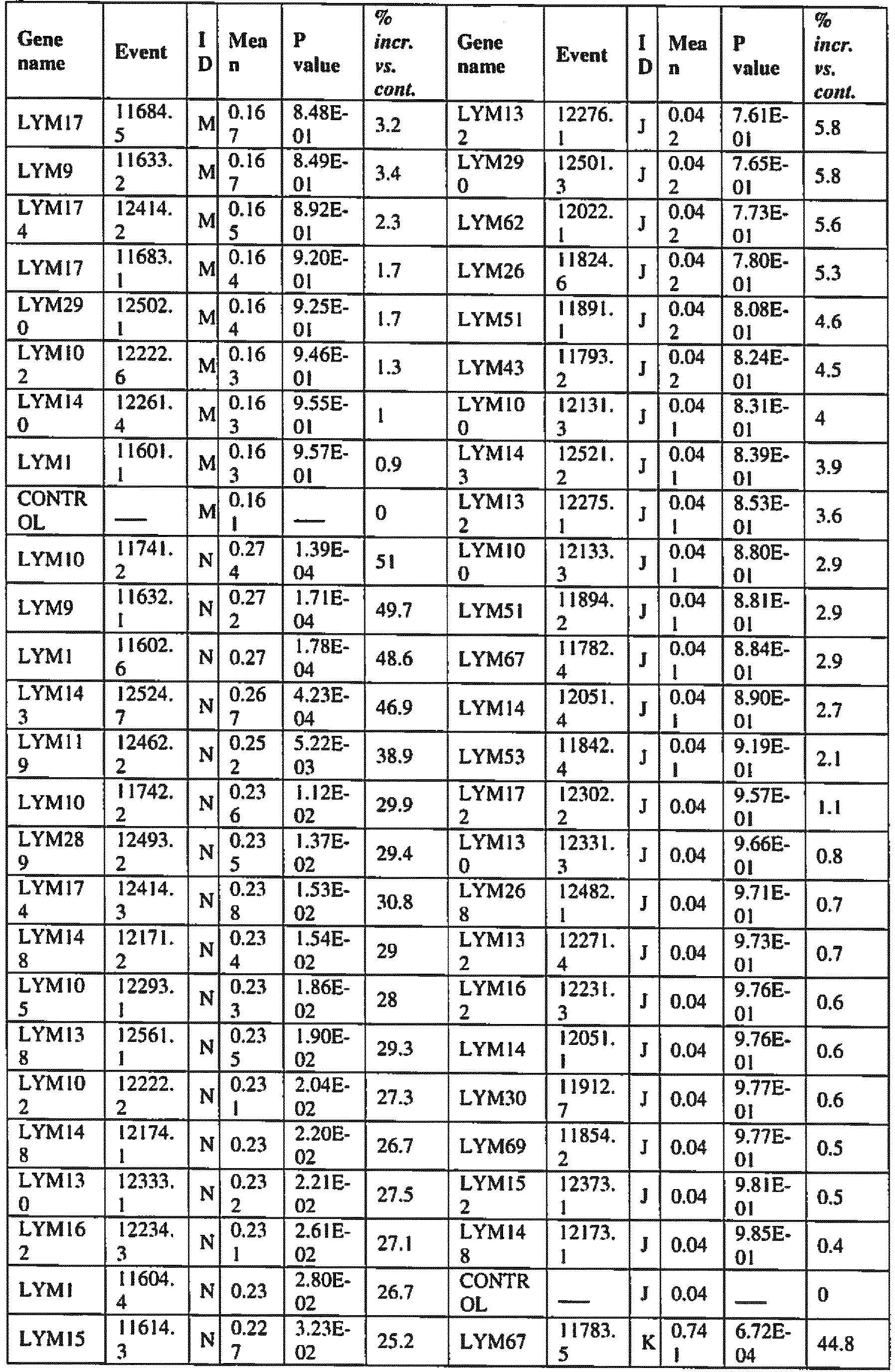

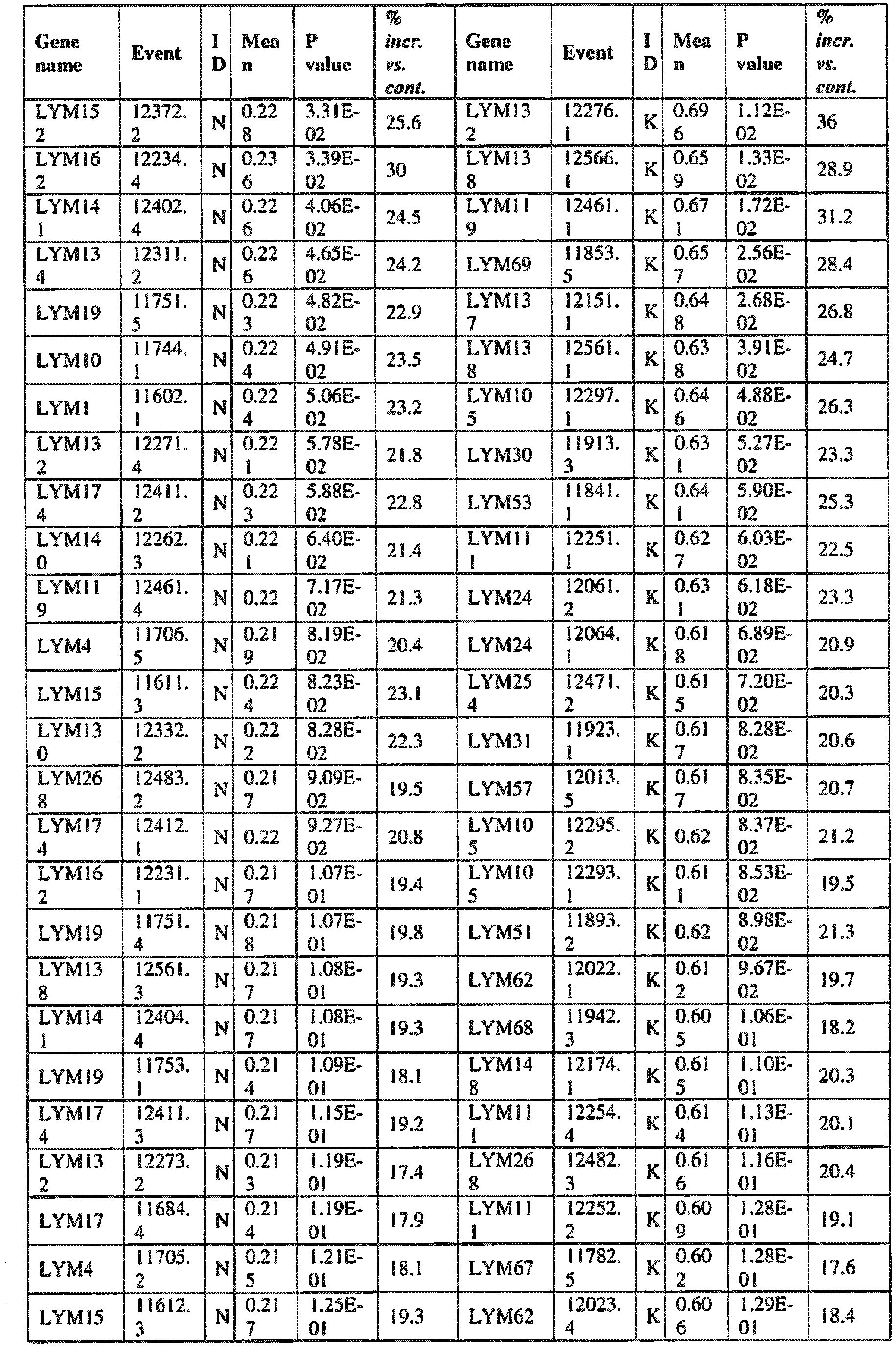

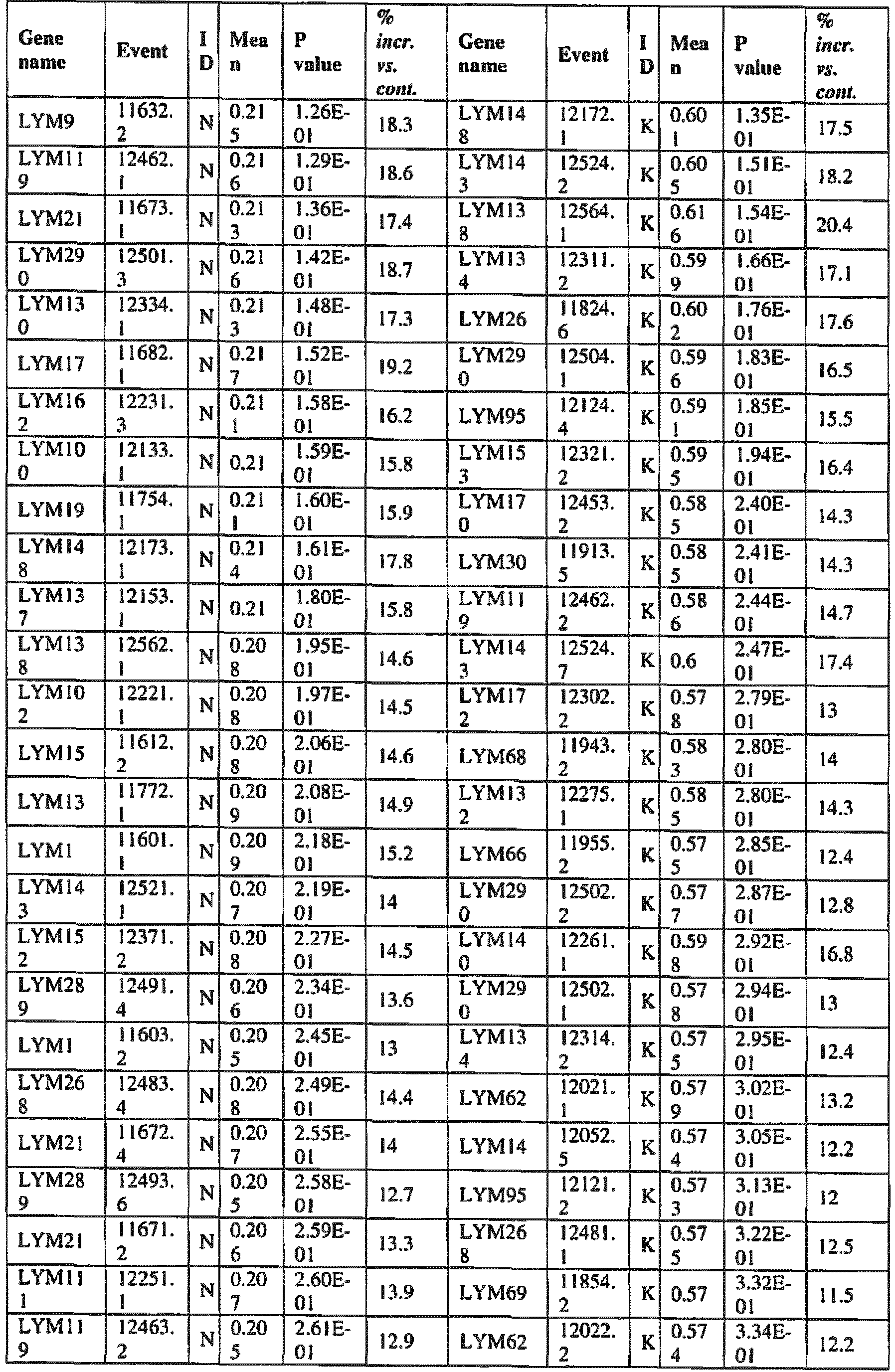

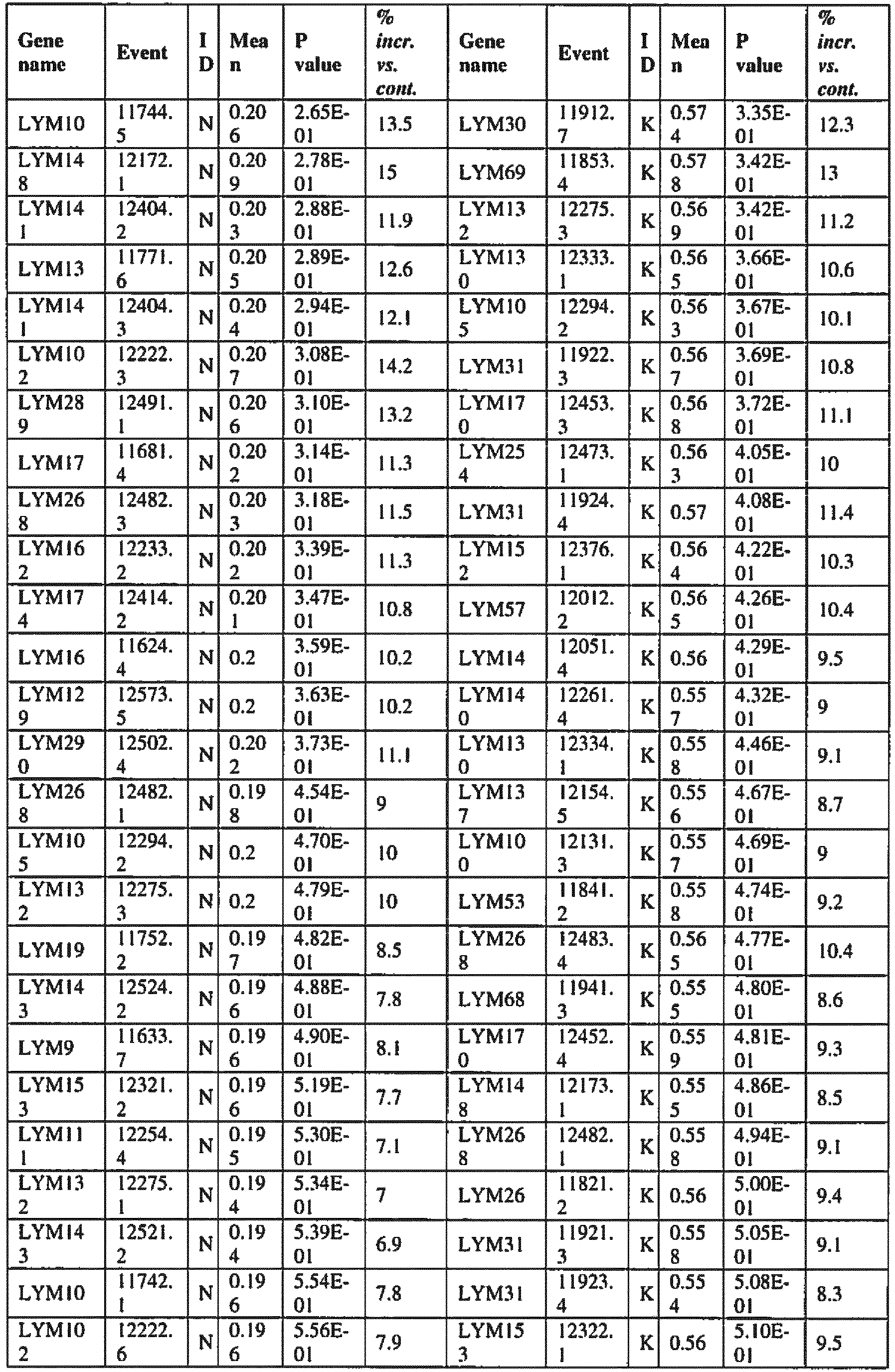

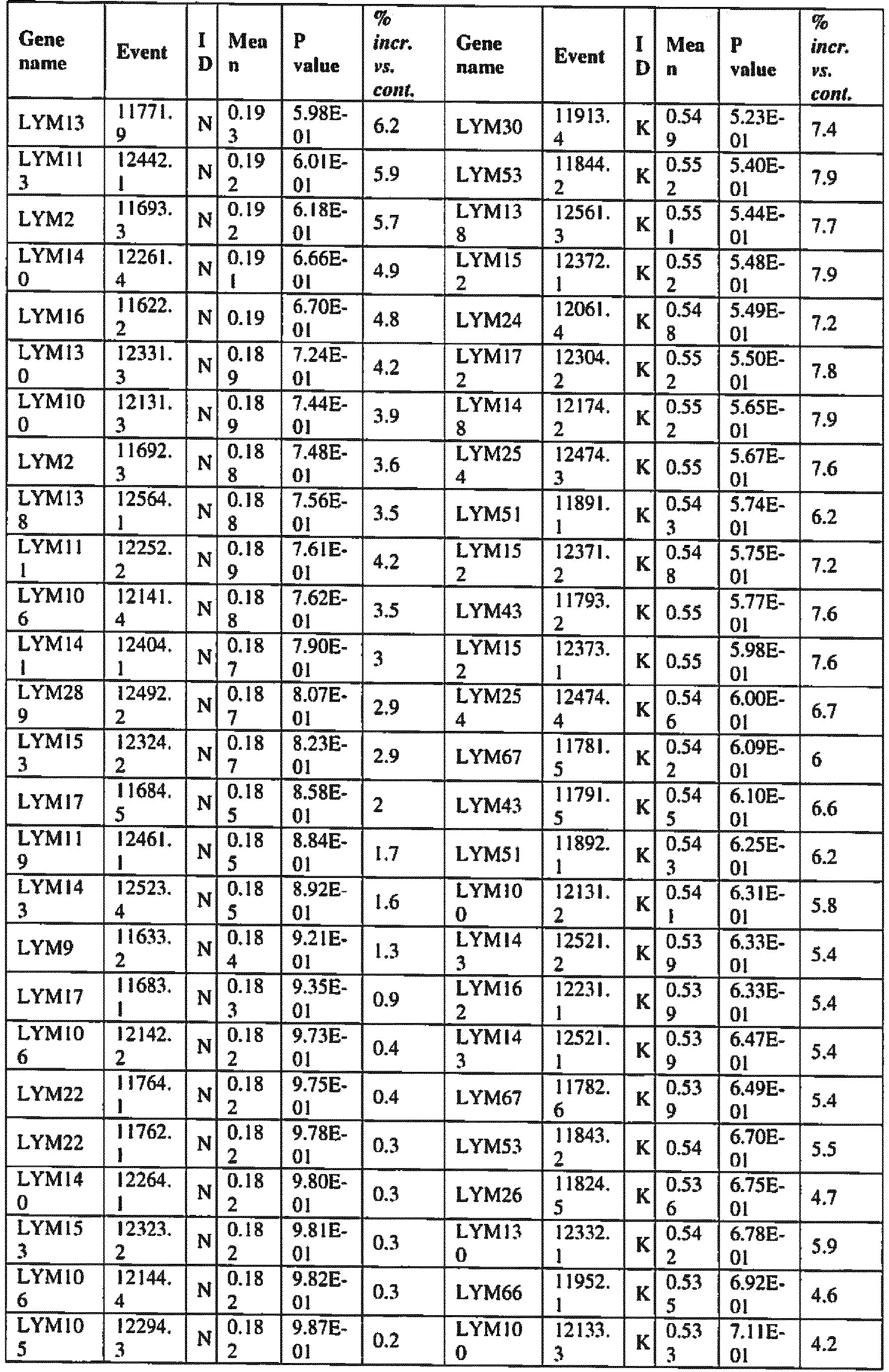

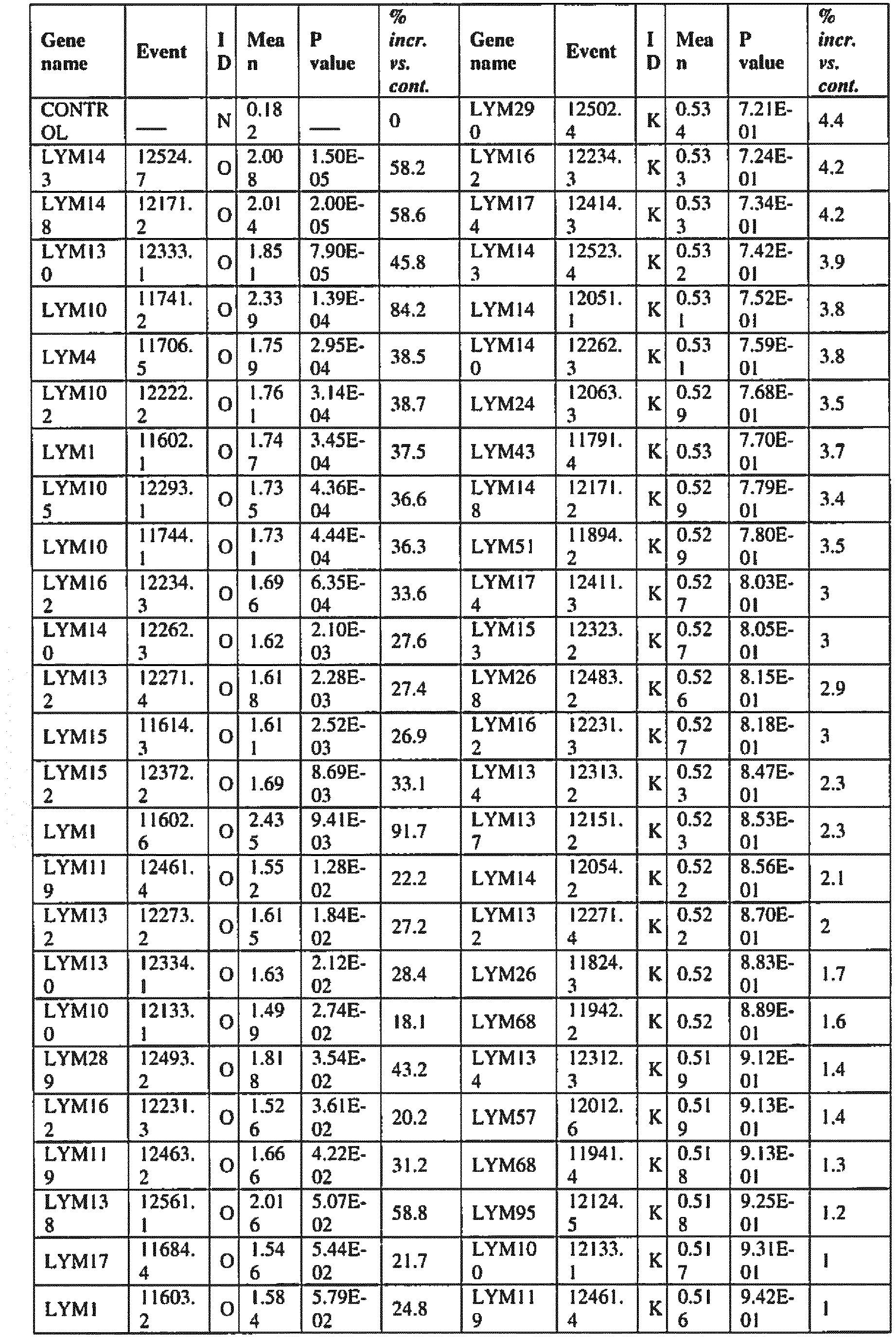

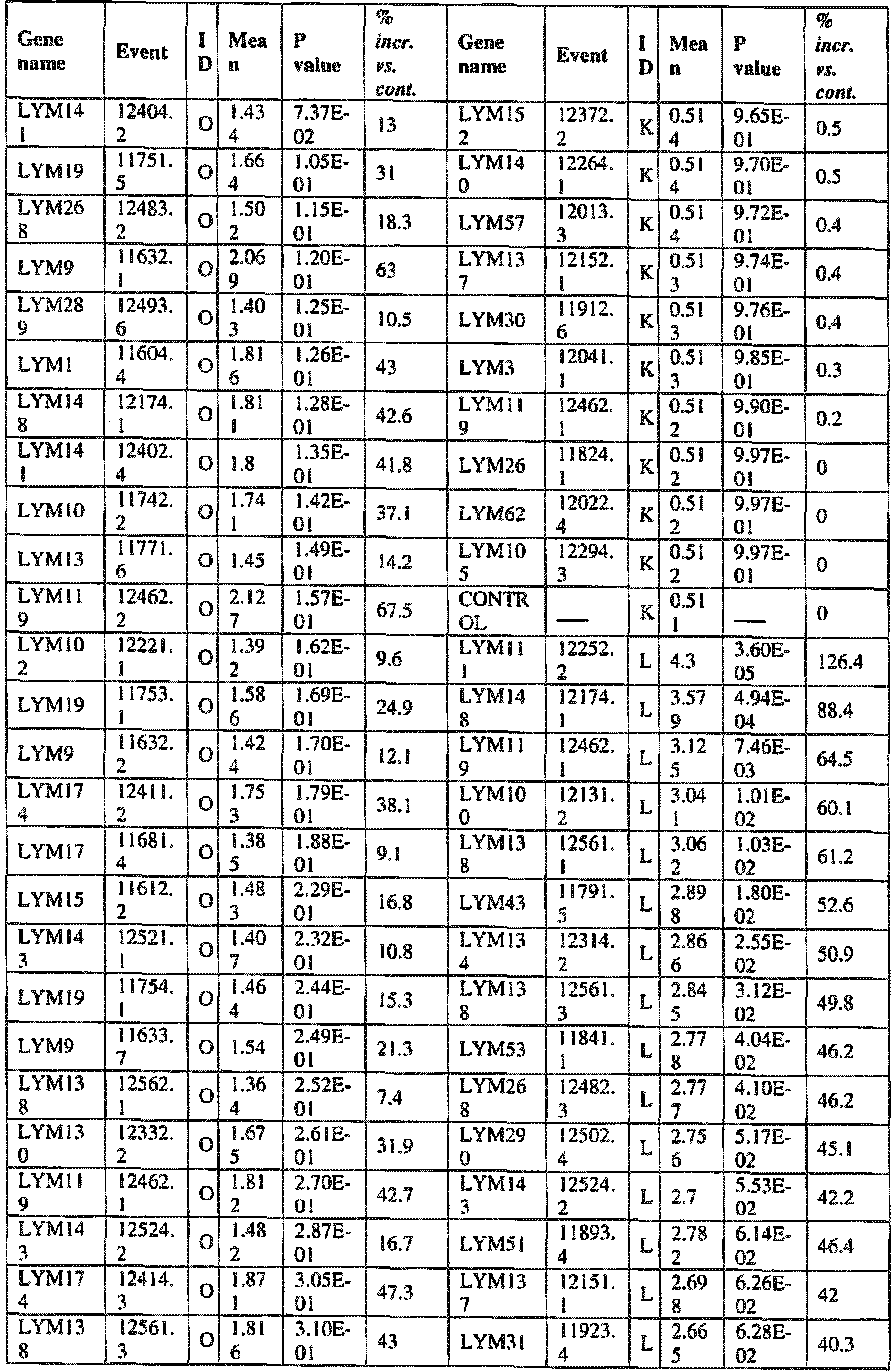

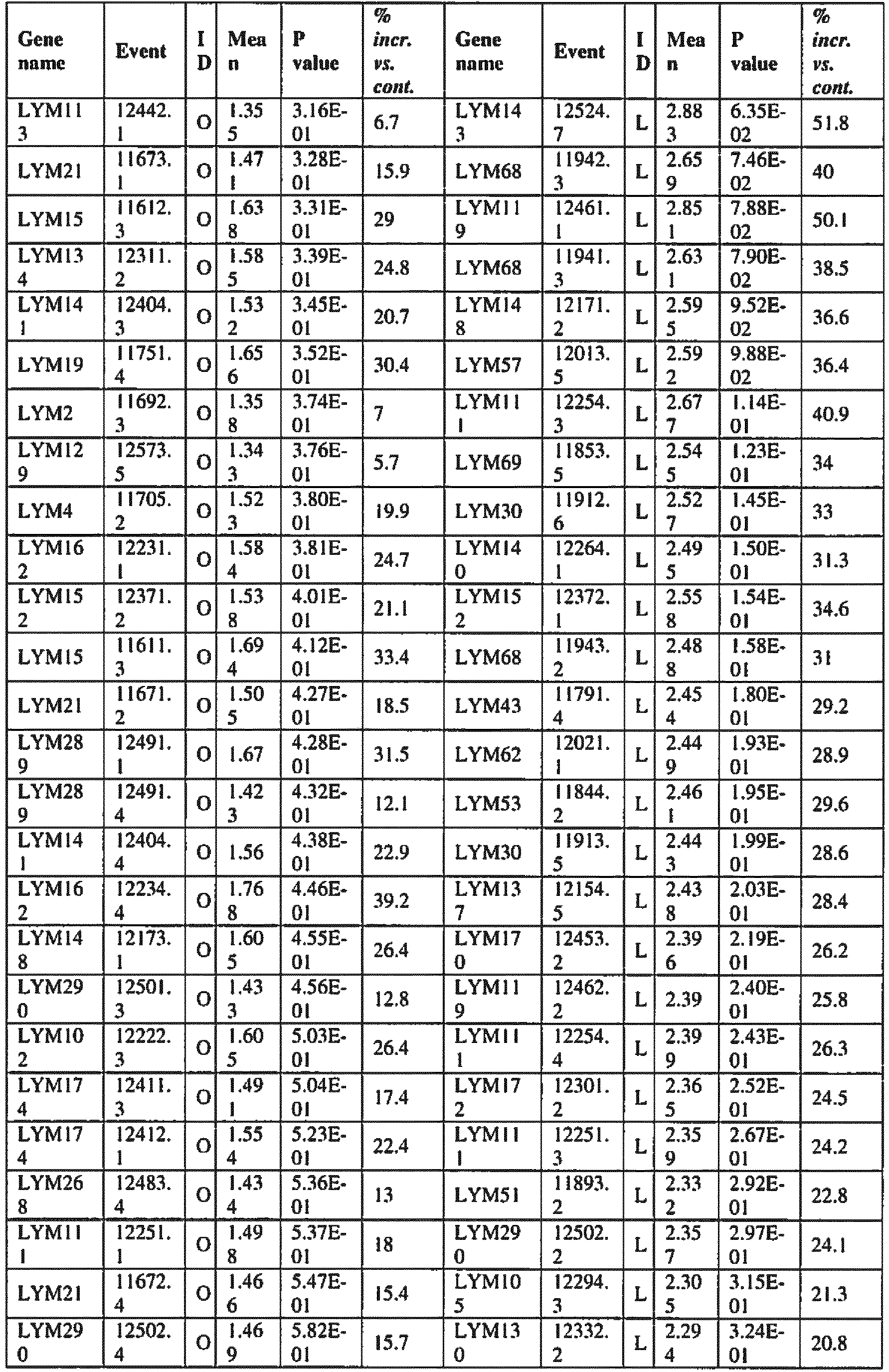

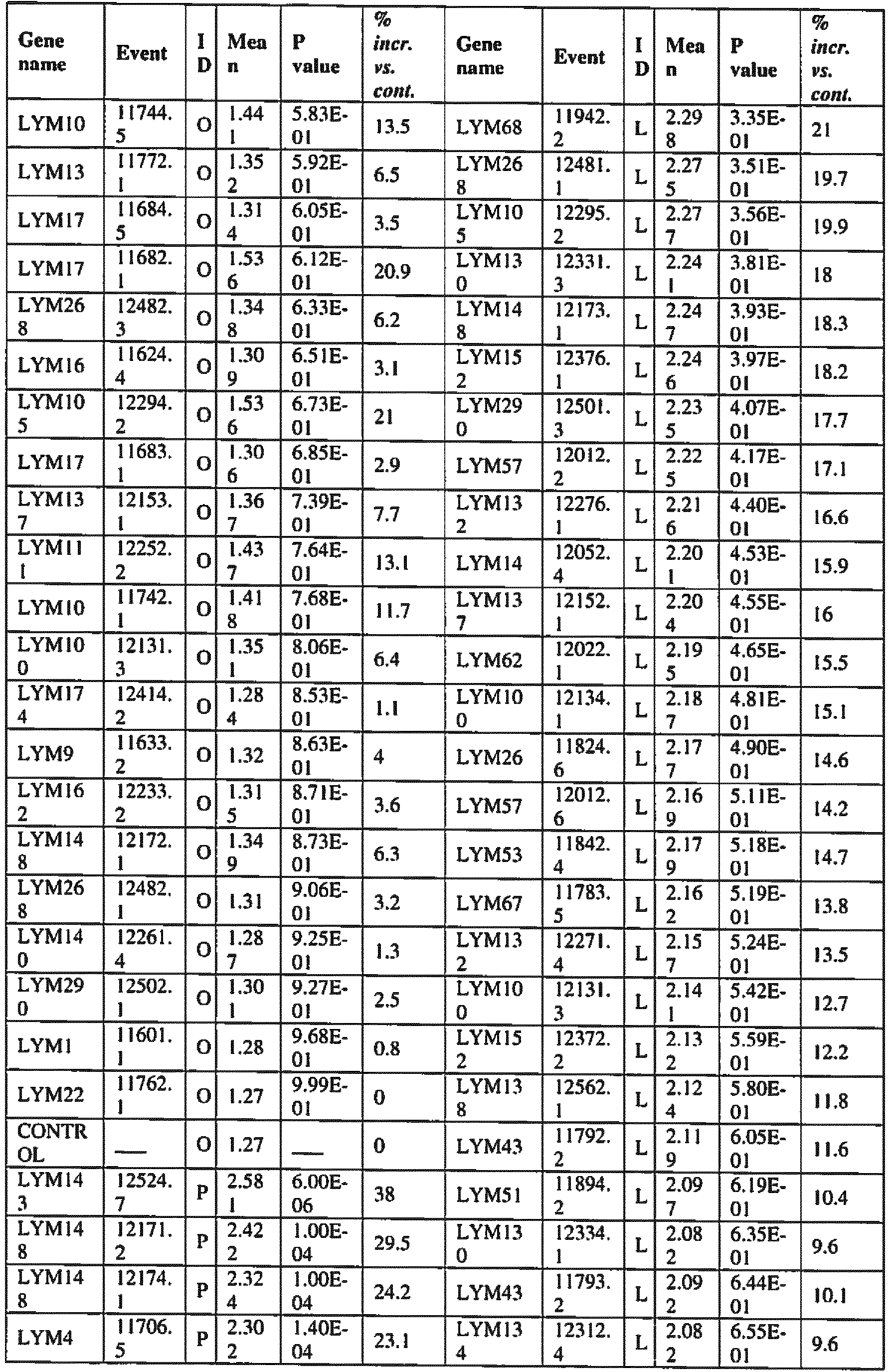

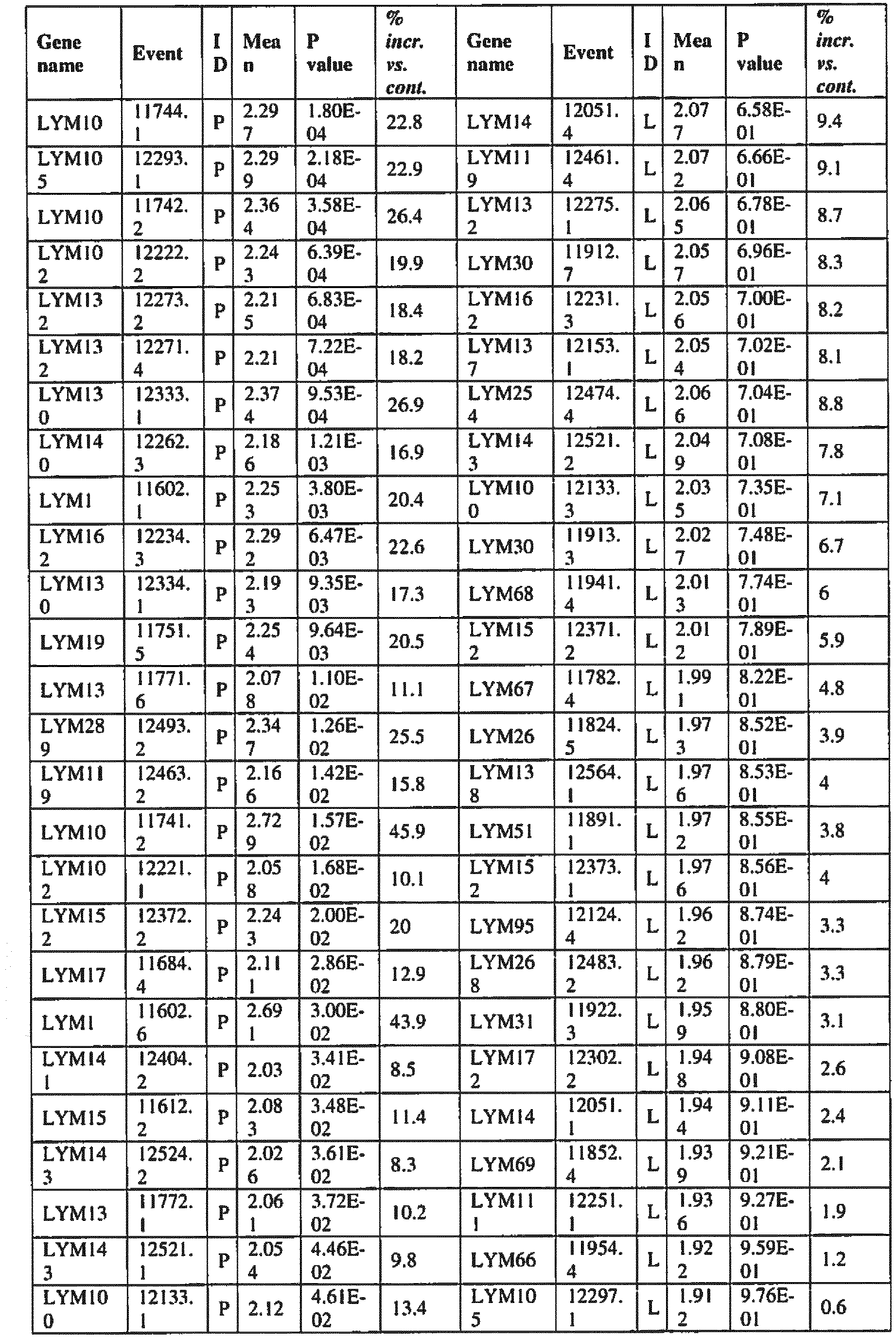

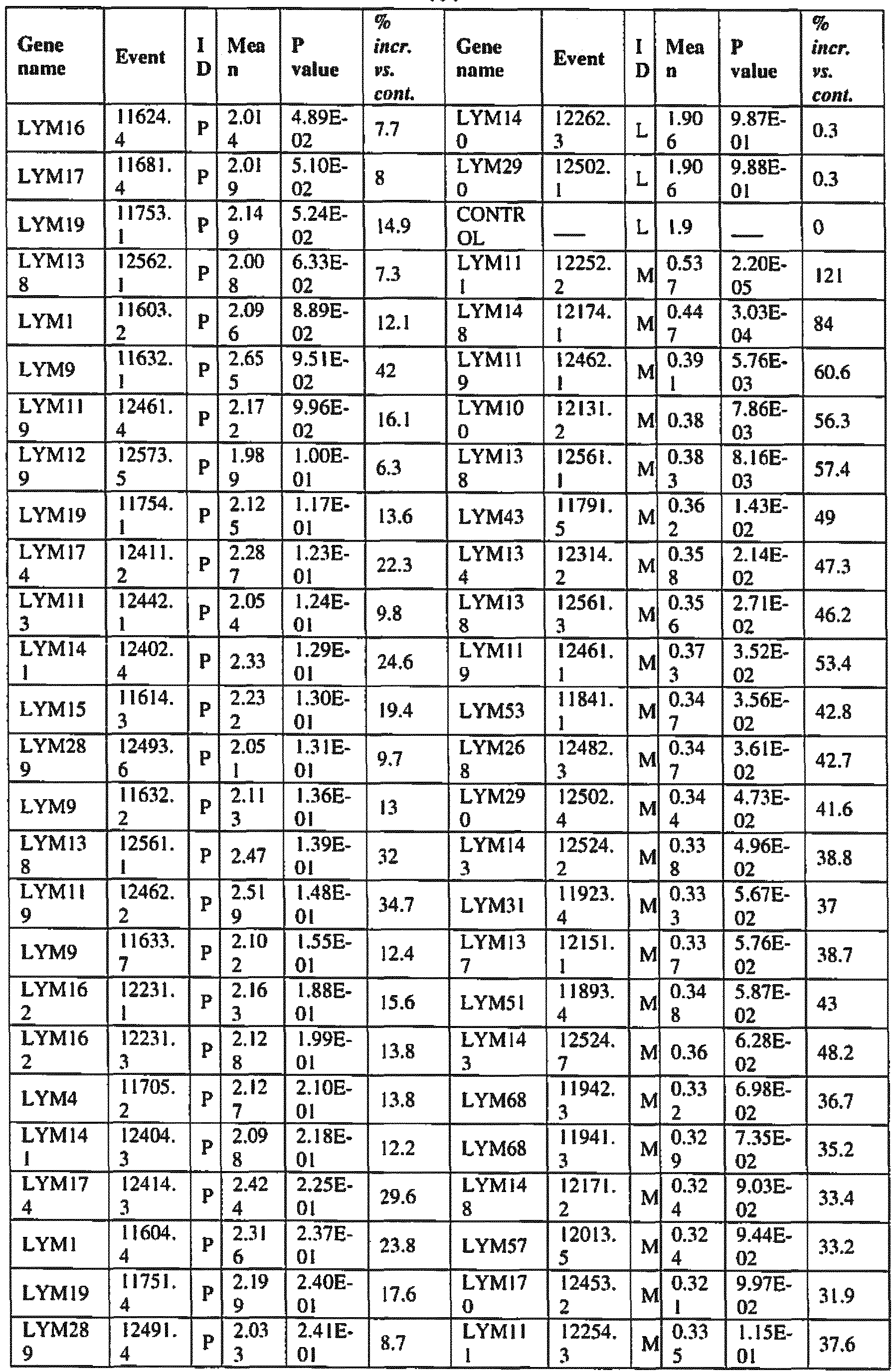

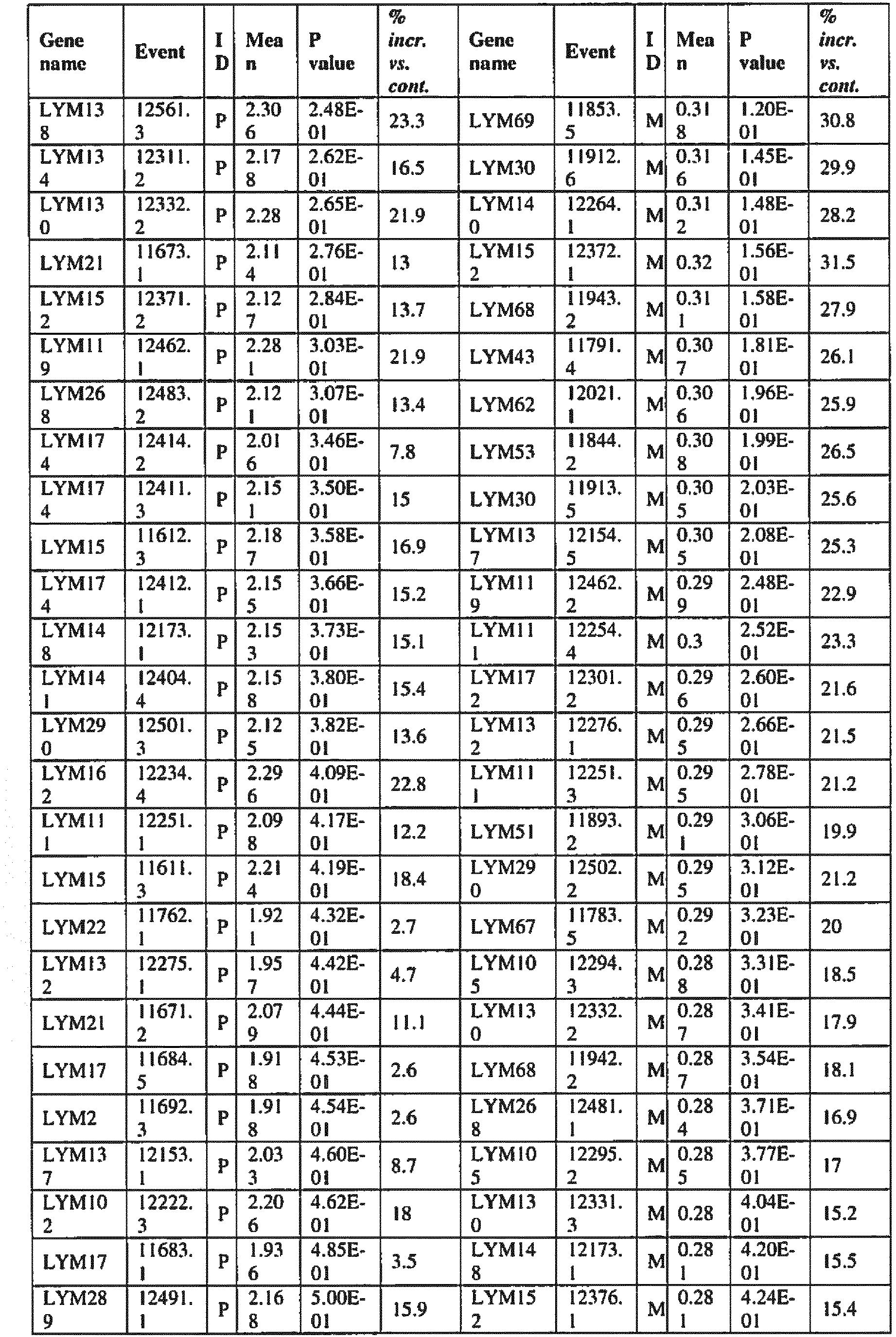

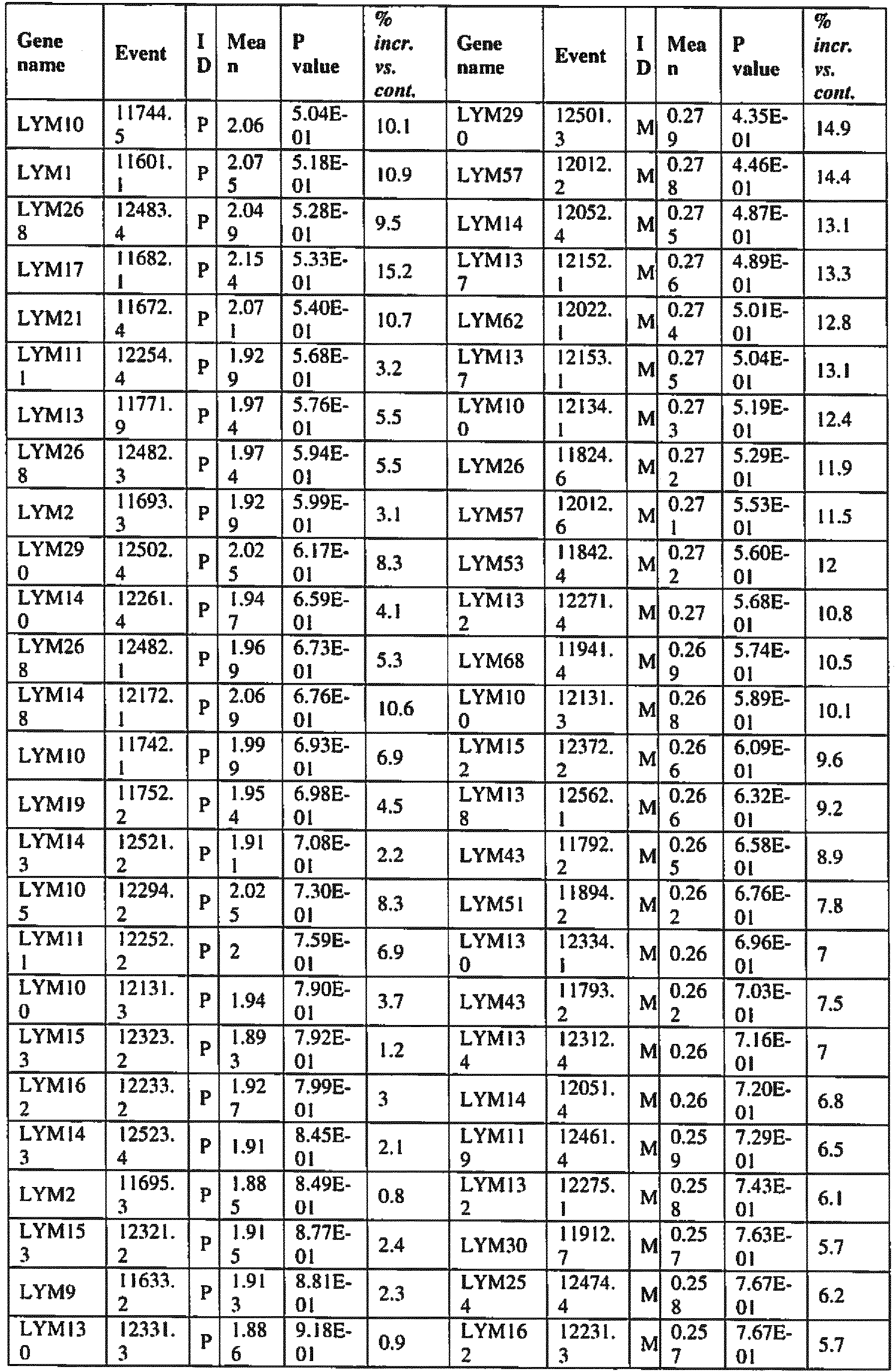

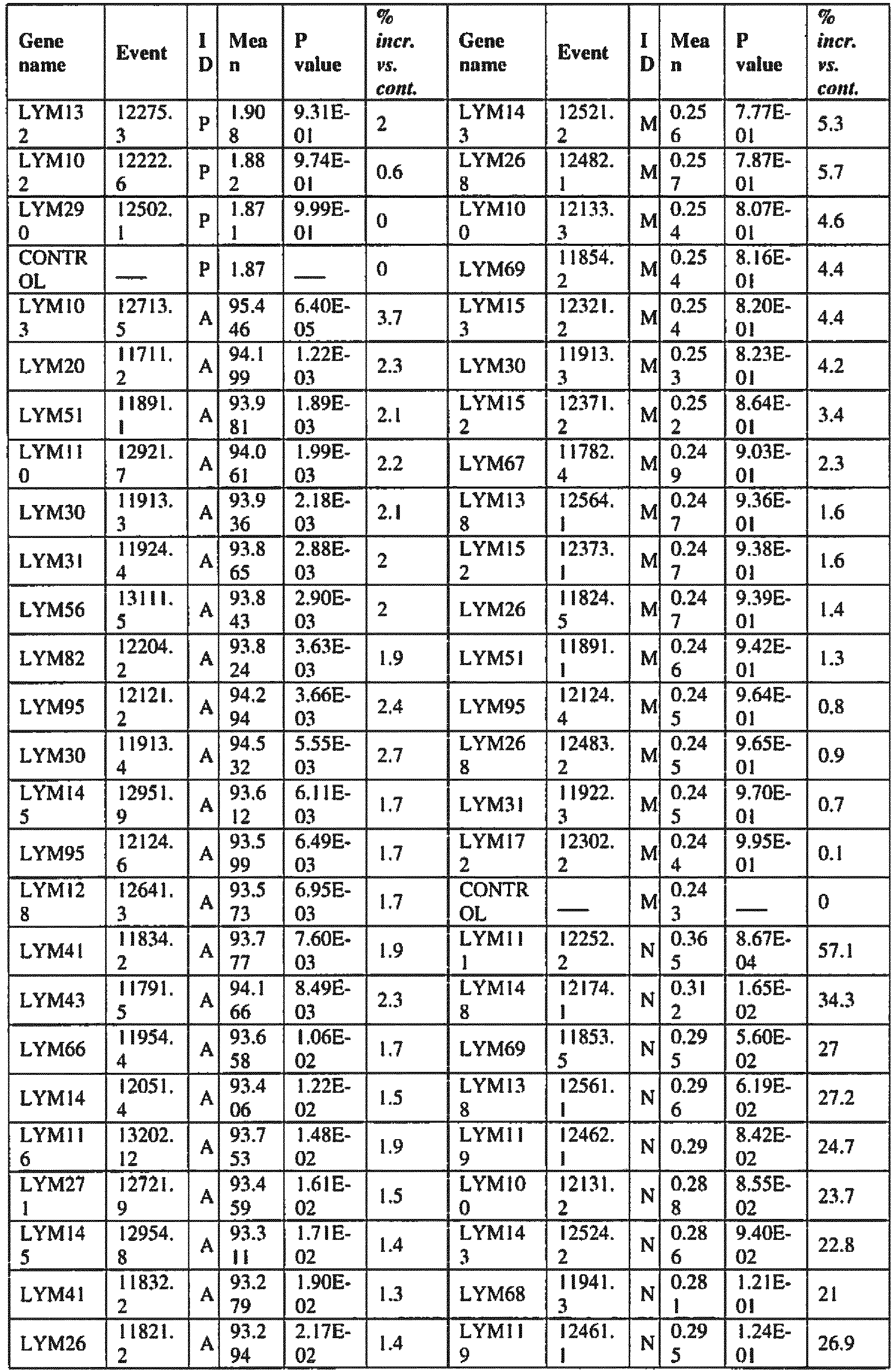

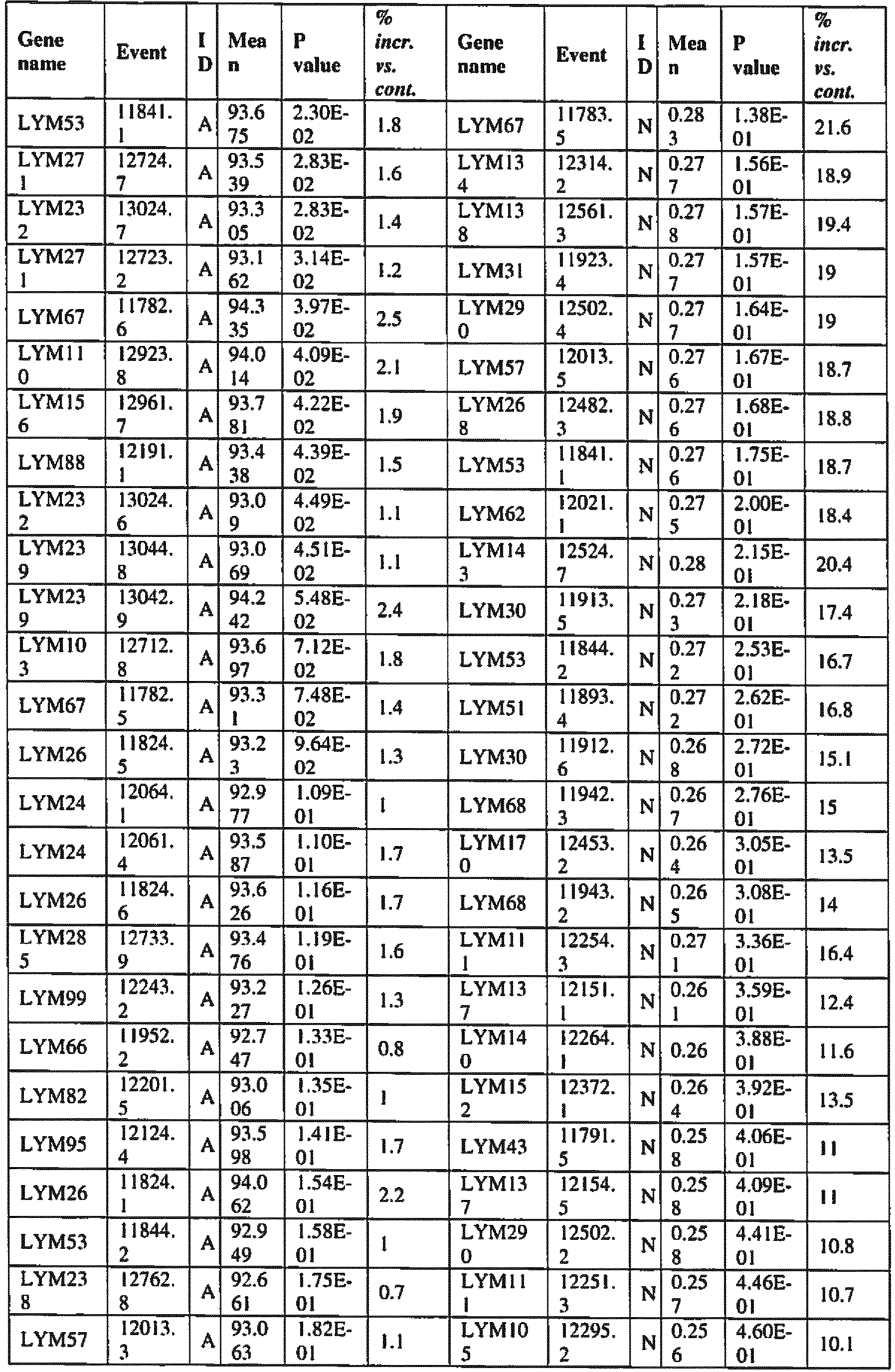

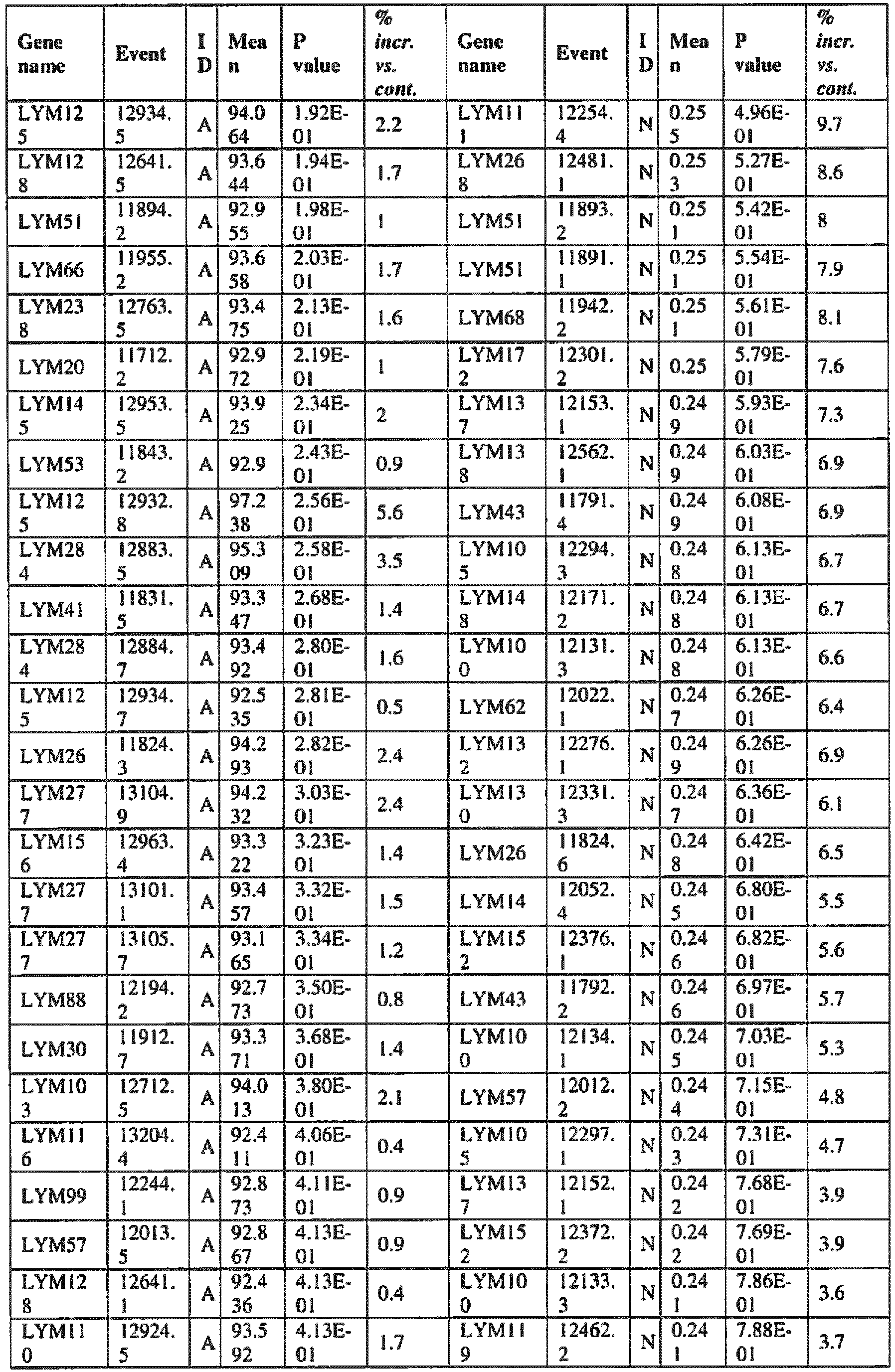

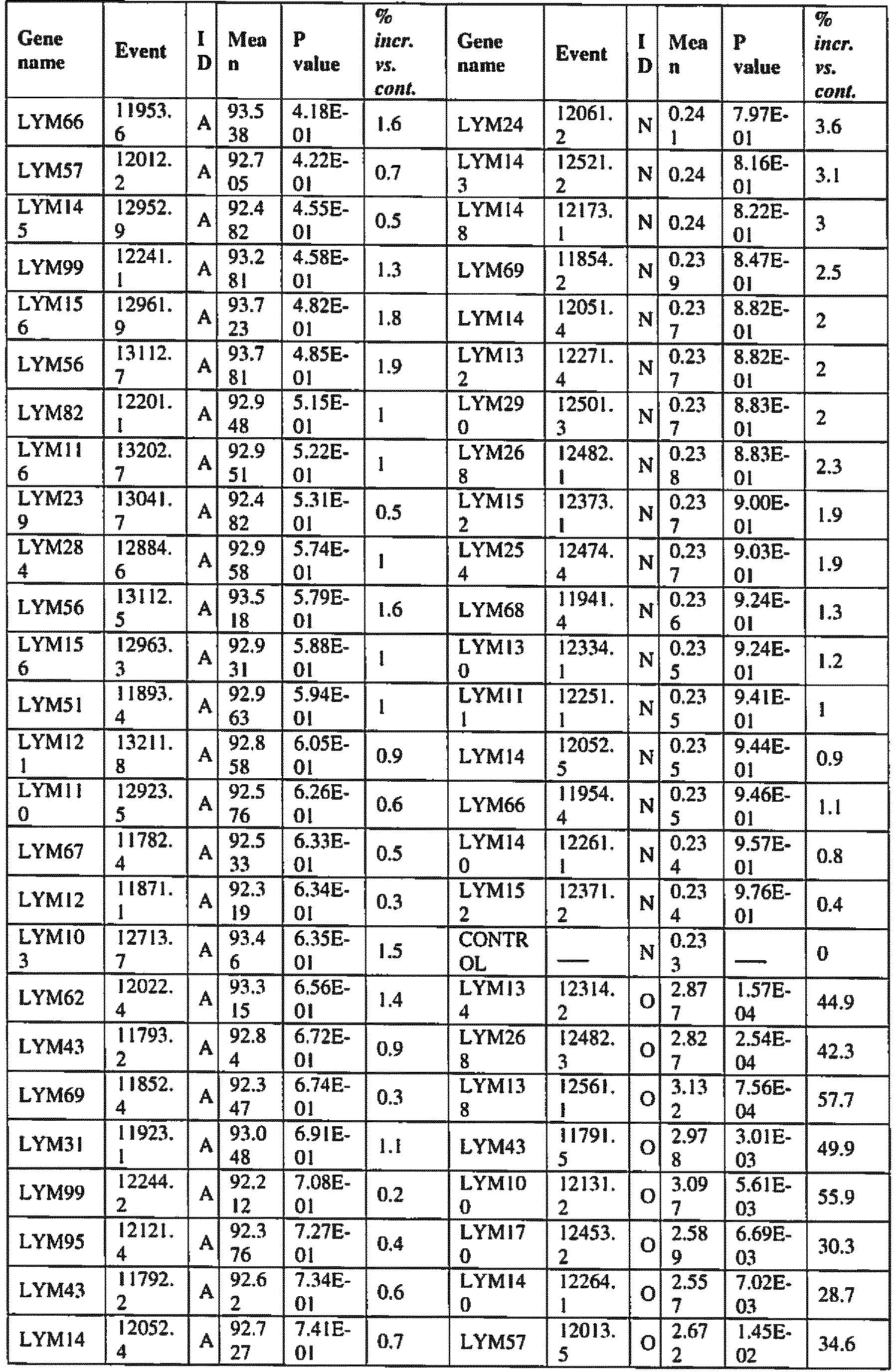

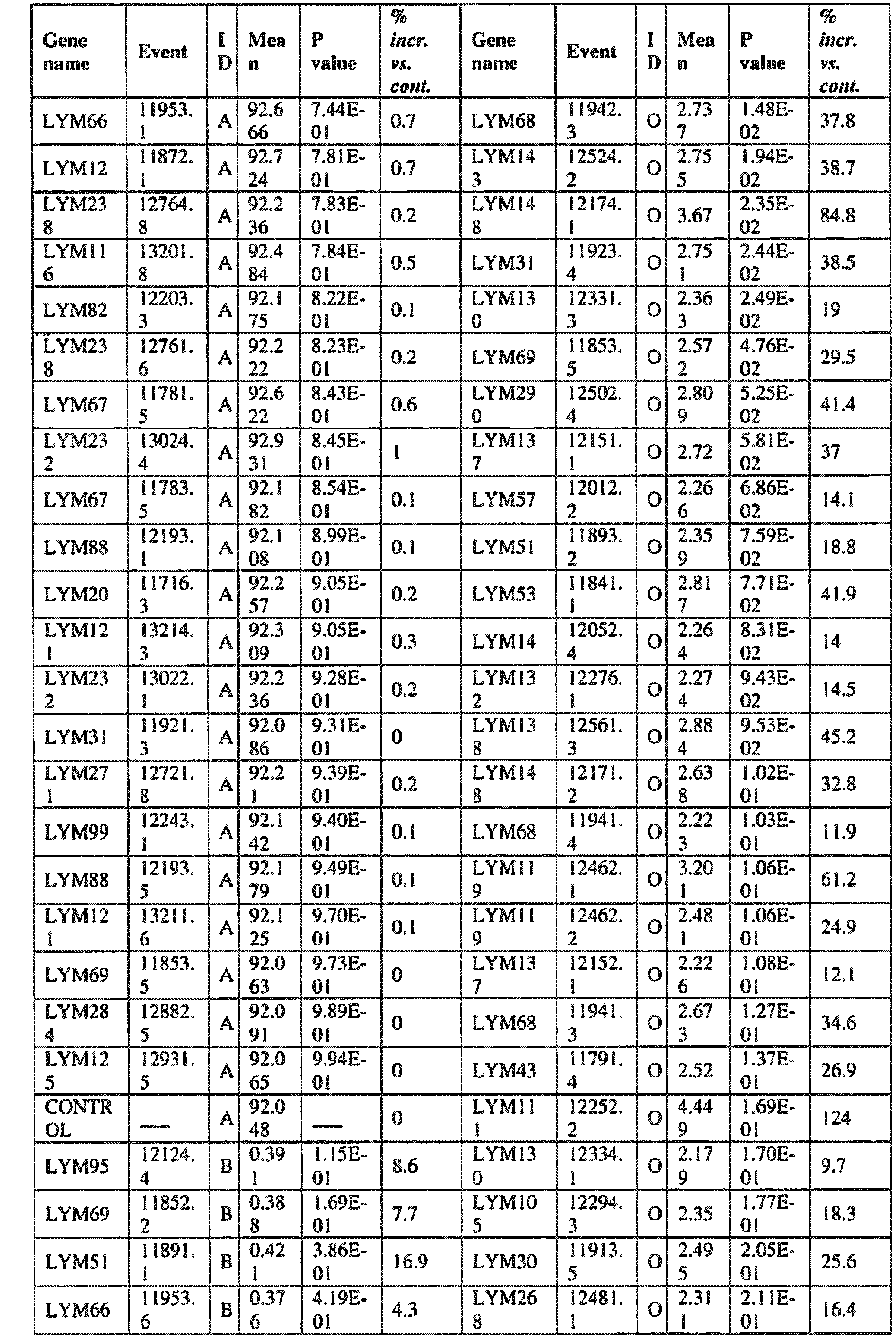

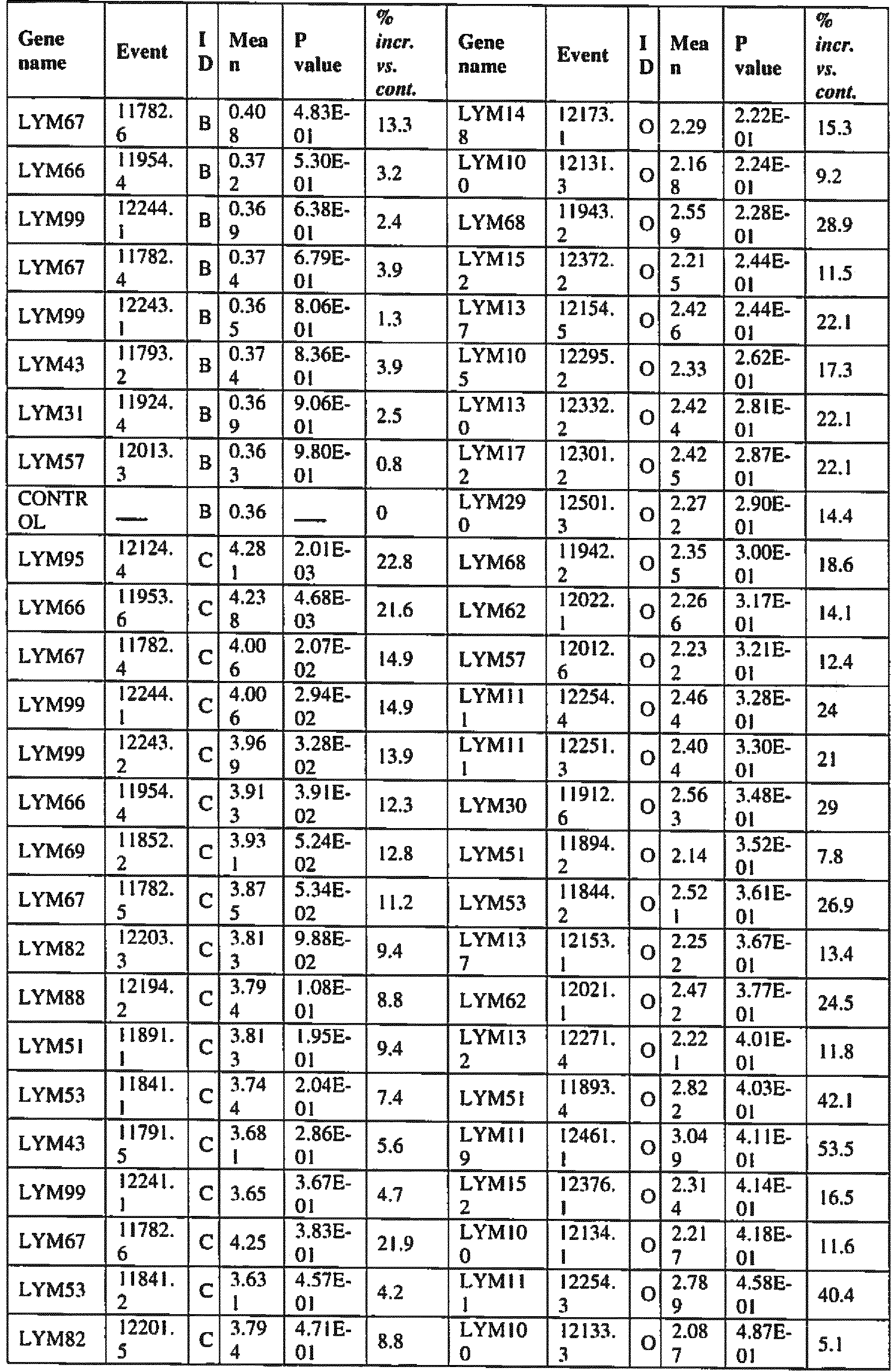

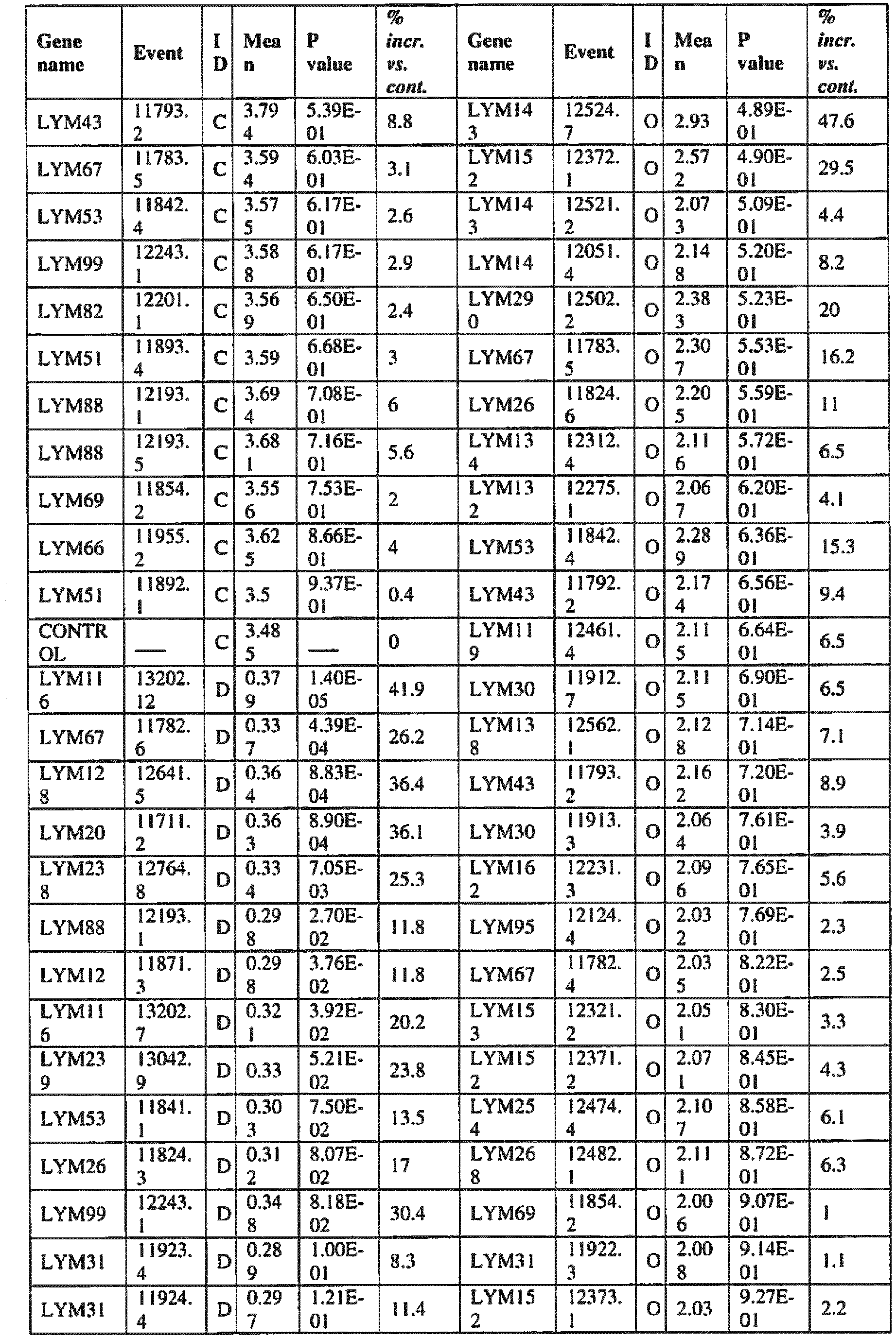

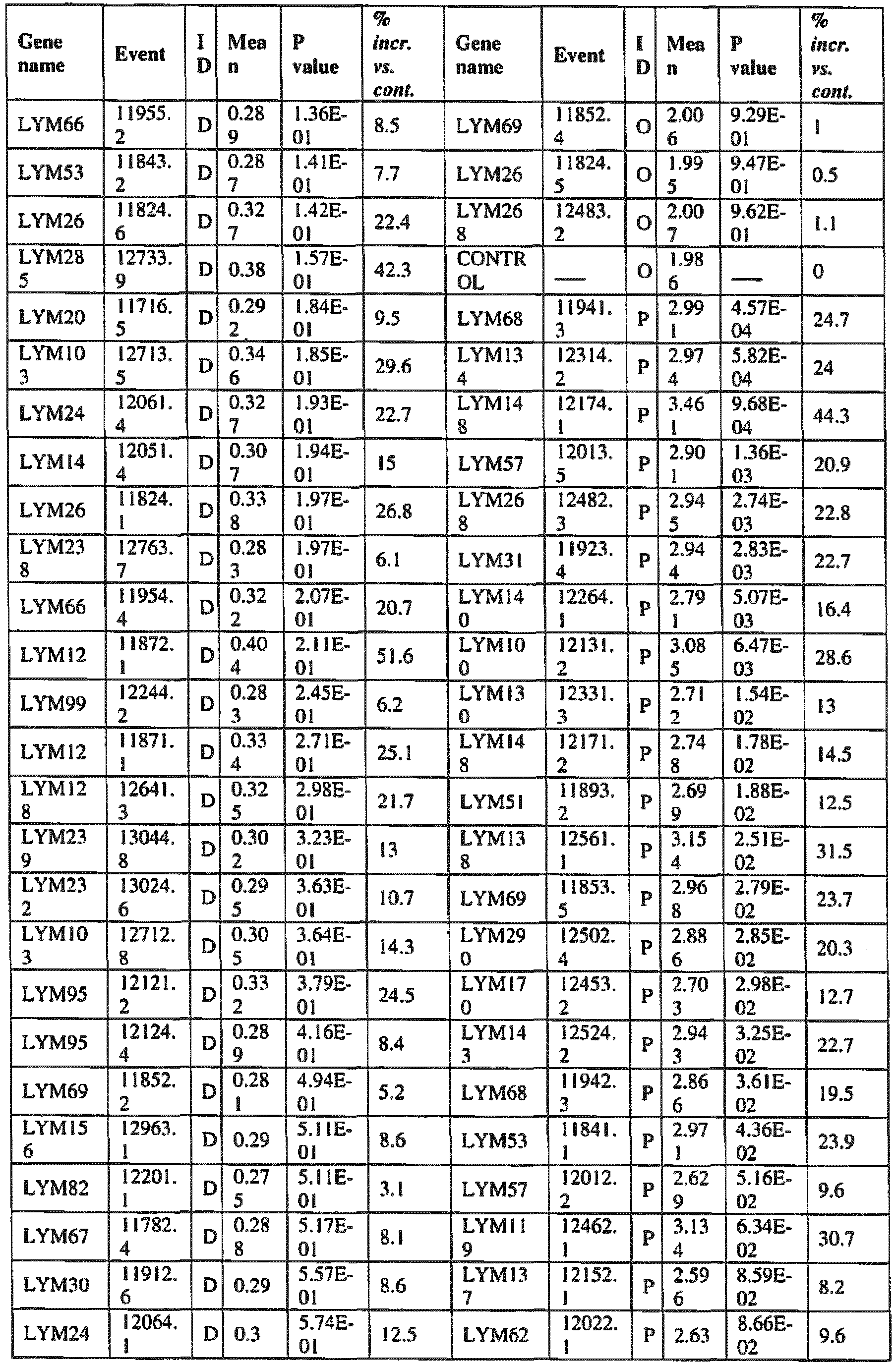

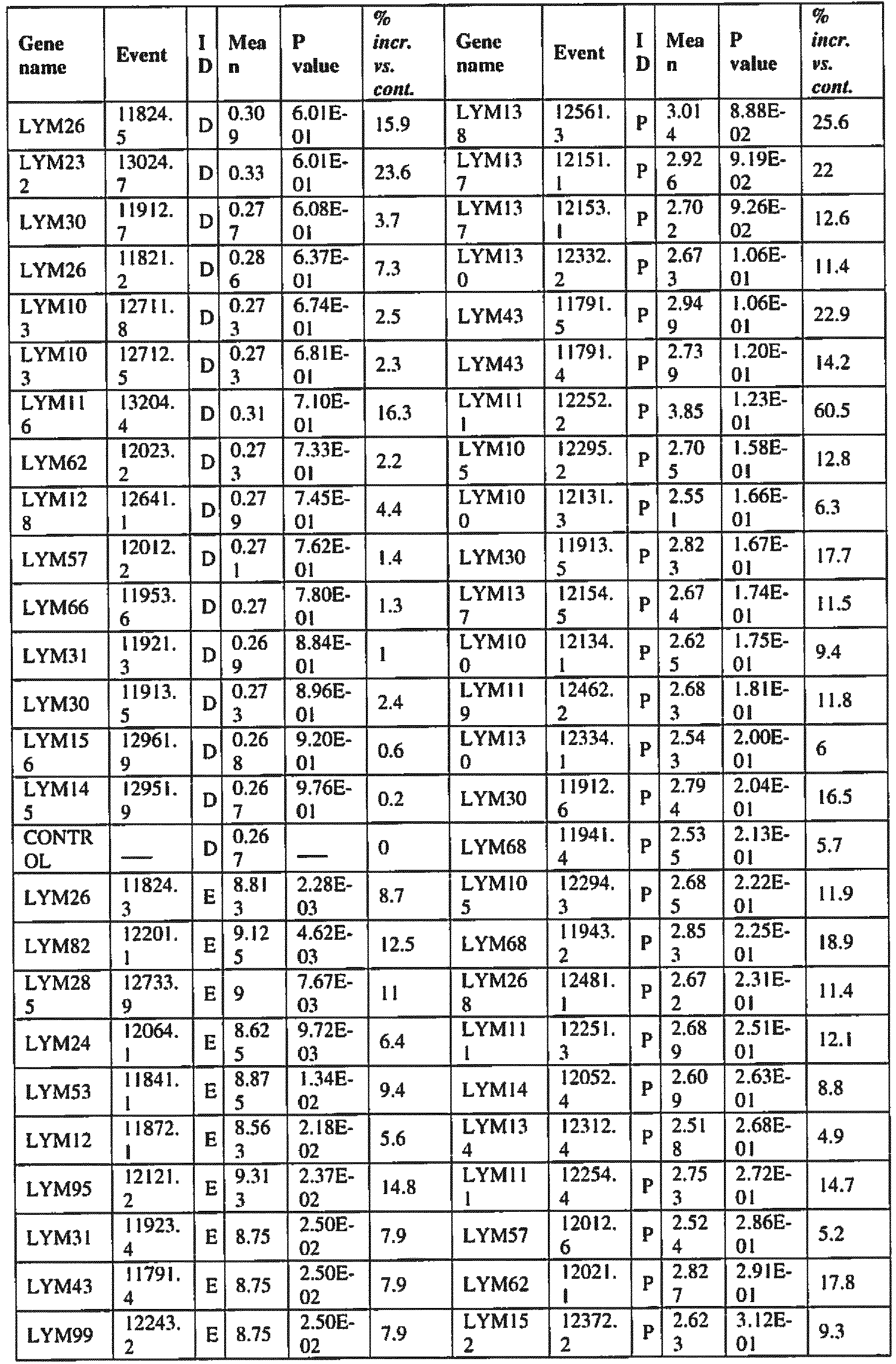

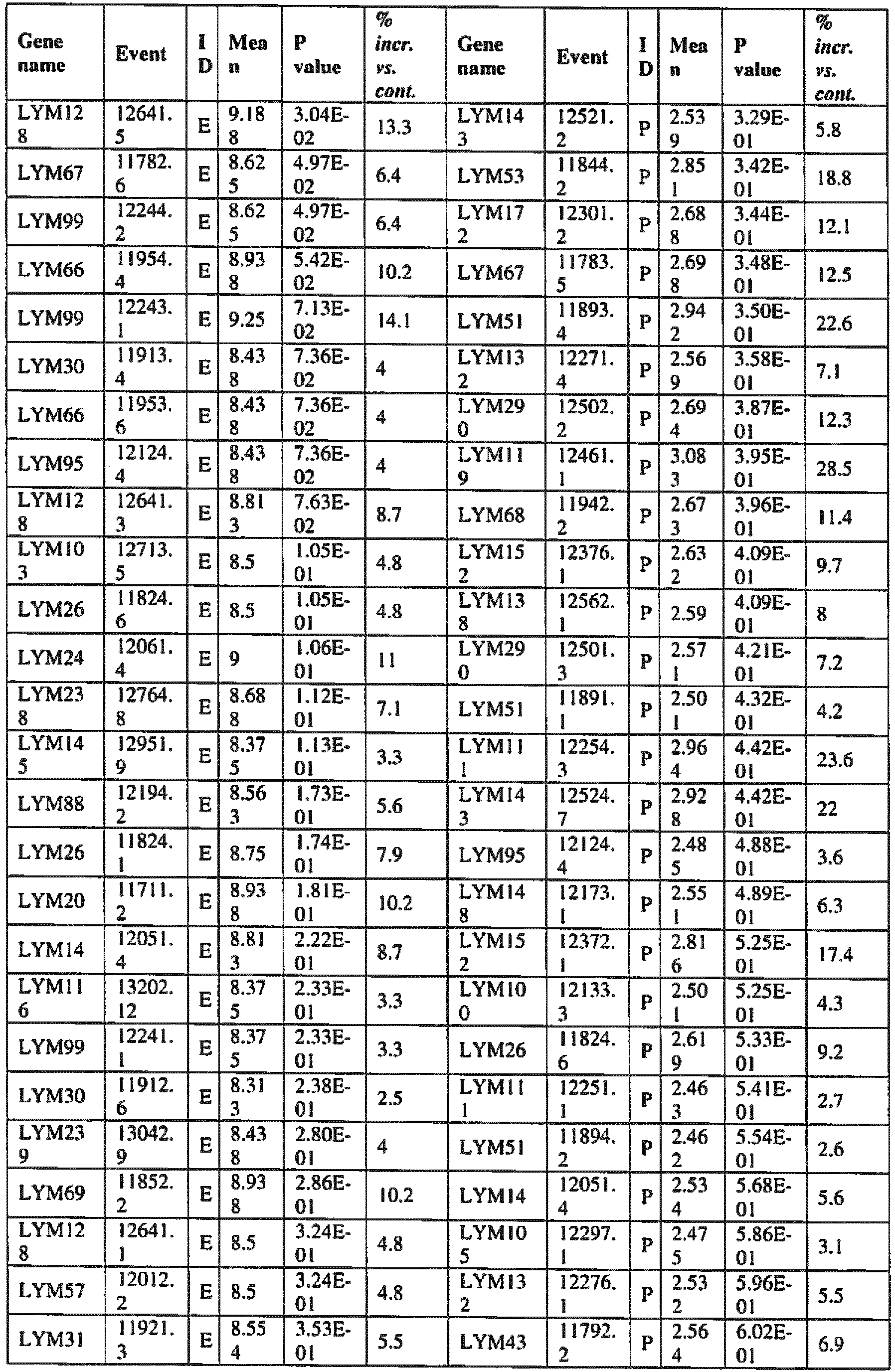

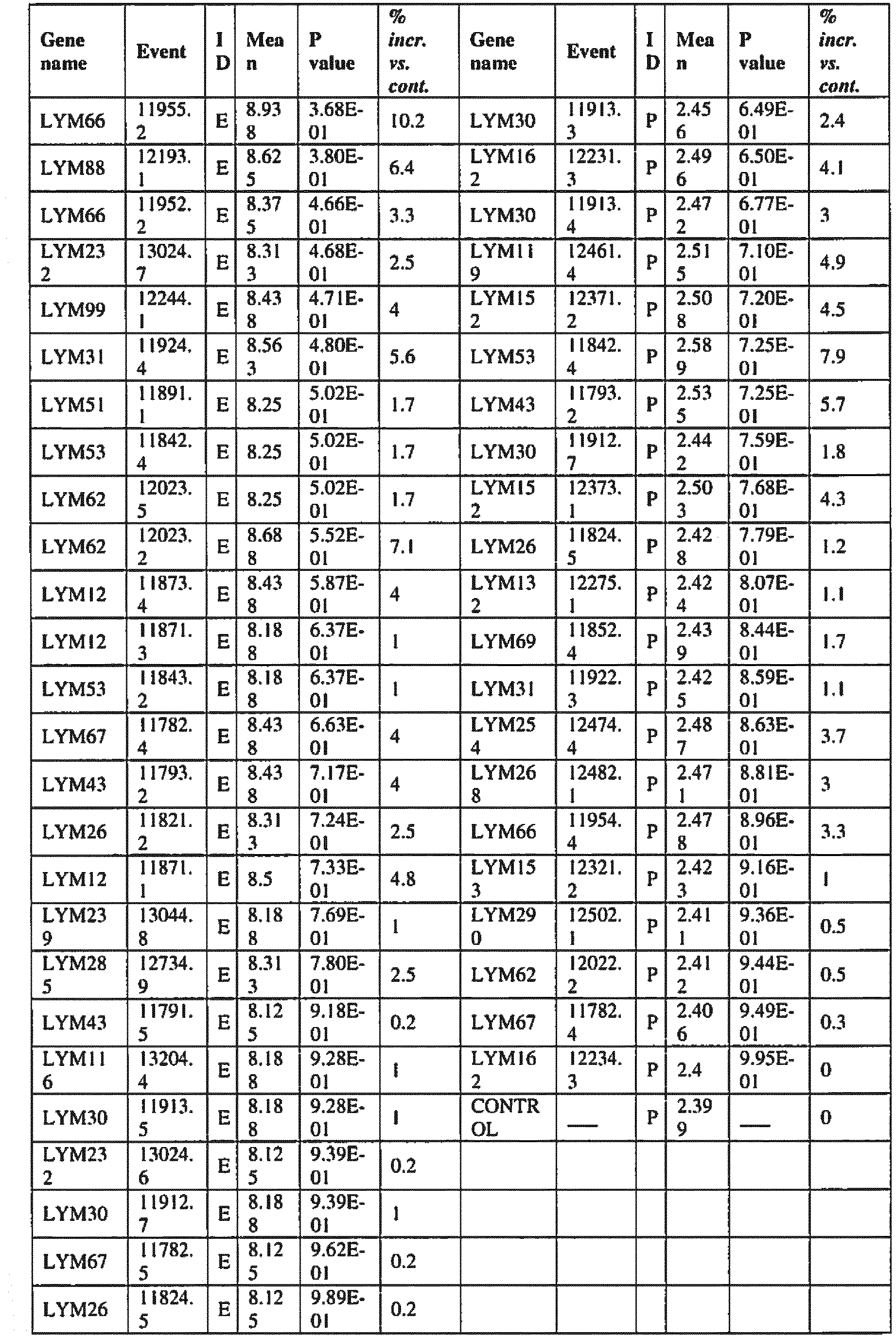

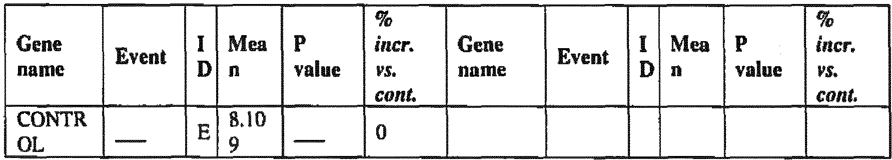

Table 32. Results of the greenhouse experiments. Provided are the measured values of each tested parameter [parameters (ID.) A-P according to the parameters described in Table 31 above] in plants expressing the indicated polynucleotides. "Ev" = event; "P" = P-value; "Mean " = the average of measured parameter across replicates. % incr. vs. cont. = percentage of increase versus control (as compared to control).

### EXAMPLE 11

### IMPROVED TRANSGENIC PLANT PERFORMANCE - TISSUE CULTURE ASSAYS

To analyze the effect of expression of the isolated polynucleotides in plants, plants performance was tested in tissue culture.

***Plant growth under favorable conditions in tissue culture using T1 or T2 plants*** - The experiments used either T2 seeds (T2 experiments herein below) or T1 seeds (T1 experiments herein below). Surface sterilized T1 or T2 seeds were sown in basal media [50% Murashige-Skoog medium (MS) supplemented with 0.8% plant agar as solidifying agent] in the presence of Kanamycin (for selecting only transgenic plants). After sowing, plates were transferred for 2-3 days for stratification at 4 °C and then grown at 25 °C under 12-hour light 12-hour dark daily cycles for 7 to 10 days. At this time point, at T2 experiments seedlings randomly chosen were carefully transferred to plates containing 0.5 MS media. Each plate contained 5 seedlings of the same transgenic event, and 3-4 different plates (replicates) for each event. For each polynucleotide, at least four independent transformation events were analyzed from each construct. Plants expressing the polynucleotides were compared to the average measurement of the control plants (empty vector or GUS reporter gene under the same promoter) used in the same experiment. Alternatively, at T1 experiments seedlings randomly chosen were carefully transferred to plates containing 0.5 MS media. Each plate contained 5 T1 seedlings representing 5 independent transgenic events, and 3-4 different plates (total of 15-20 events). Plants expressing the polynucleotides were compared to the average measurement of the control plants (empty vector or GUS reporter gene under the same promoter) used in the same experiment.

***Plant growth under low nitrogen conditions in Tissue culture using T2 plants*** - Surface sterilized seeds were sown in basal media [50 % Murashige-Skoog medium (MS) supplemented with 0.8 % plant agar as solidifying agent] in the presence of Kanamycin (used as a selecting agent). After sowing, plates were transferred for 2-3 days for stratification at 4 °C and then grown at 25 °C under 12-hour light 12-hour dark daily cycles for 7 to 10 days. At this time point, seedlings randomly chosen were carefully transferred to plates containing ½ MS media (15 mM N) for the normal nitrogen concentration treatment and 0.75 mM nitrogen for the low nitrogen concentration treatments. Each plate contained 5 seedlings of the same transgenic event, and 3-4 different plates (replicates) for each event. For each polynucleotide at least four independent transformation events were analyzed from each construct. Plants expressing the polynucleotides were compared to the average measurement of the control plants (empty vector or GUS reporter gene under the same promoter) used in the same experiment.

For both experiments the same plant parameters were being measured and being described below:
***Digital imaging*** - A laboratory image acquisition system, which consists of a digital reflex camera (Canon EOS 300D) attached with a 55 mm focal length lens (Canon EF-S series), mounted on a reproduction device (Kaiser RS), which included 4 light units (4x150 Watts light bulb) and located in a darkroom, is used for capturing images of plantlets sawn in agar plates.

The image capturing process is repeated every 4 days starting at day 1 till day 10. An image analysis system was used, which consists of a personal desktop computer (Intel P4 3.0 GHz processor) and a public domain program - ImageJ 1.39 [Java based image processing program which was developed at the U.S. National Institutes of Health and freely available on the internet at Hypertext Transfer Protocol://rsbweb (dot) nih (dot) gov/]. Images were captured in resolution of 10 Mega Pixels (3888 x 2592 pixels) and stored in a low compression JPEG (Joint Photographic Experts Group standard) format. Next, analyzed data was saved to text files and processed using the JMP statistical analysis software (SAS institute).

***Seedling analysis*** - Using the digital analysis seedling data was calculated, including leaf area, root coverage and root length.

The relative growth rate for the various seedling parameters was calculated according to the following formulas XVII, VII and XVIII. $Relative growth rate of leaf area = Regression coefficient of leaf area along time course.$ $Relative growth rattet of root coverage = Regression coefficient of root coverage along time course.$ $Relative growth of root length = Regression coefficient of root length along time course.$

At the end of the experiment, plantlets were removed from the media and weighed for the determination of plant fresh weight. Plantlets were then dried for 24 hours at 60 °C, and weighed again to measure plant dry weight for later statistical analysis. Growth rate is determined by comparing the leaf area coverage, root coverage and root length, between each couple of sequential photographs, and results were used to resolve the effect of the gene introduced on plant vigor under optimal conditions. Similarly, the effect of the gene introduced on biomass accumulation, under optimal conditions, is determined by comparing the plants' fresh and dry weight to that of control plants (containing an empty vector or the GUS reporter gene under the same promoter). From every construct created, 3-5 independent transformation events were examined in replicates.

*Statistical analyses* - To identify genes conferring significantly improved plant vigor or enlarged root architecture, the results obtained from the transgenic plants were compared to those obtained from control plants. To identify outperforming genes and constructs, results from the independent transformation events tested were analyzed separately (T2 experiments) or as average of events (T1 experiments). To evaluate the effect of a gene event (T2 experiments) or gene (T1 experiment) over a control the data is analyzed by Student's t-test and the p value is calculated. Results were considered significant if p ≤ 0.1. The JMP statistics software package was used (Version 5.2.1, SAS Institute Inc., Cary, NC, USA).

Tables 33, 35 and 37 specify the parameters measured in plants in the tissue culture assays (T2 plants, T1 plants and low nitrogen T2 plants).

### Experimental Results

Plants expressing the polynucleotides were assayed for a number of commercially desired traits. In cases where a certain event appears more than once, the event was tested in several independent experiments.

**Table 33**

| ***Measured parameters at the tissue culture assay (T2 experiment) for transformed agriculture improving trait genes*** | |
|---|---|
| Tested Parameters | ID |
| Dry Weight (gr) | A |
| Fresh Weight (gr) | B |
| RGR Of Root Coverage | C |
| Roots Coverage TP3 (cm²) | D |
| RGR Of Roots Length | E |
| Roots Length TP3 (cm) | F |
| Leaf Area TP3 (cm) | G |
| RGR Of Leaf Area | H |

Table 33: Provided are the identification (ID) letters of each of the Tested Parameters. TP3 -Time Point 3; RGR- Relative Growth Rate.

**Table 34**

| ***Results obtained in a T2 experiment at tire tissue culture assay*** | | | | | | | | | | | |
|---|---|---|---|---|---|---|---|---|---|---|---|
| ***Gene name*** | ***Event*** | ***I D*** | ***Mea n*** | ***P value*** | ***% incr. vs. cont.*** | ***Gene name*** | ***Event*** | ***I D*** | ***Mea n*** | ***P value*** | ***% incr. vs*. *cont.*** |
| LYM37 | 11801. 2 | A | 0.00 7 | 4.96E-04 | 60.3 | LYM1 | 1160 2.6 | H | 0.10 4 | 0.00E +00 | 124 |
| LYM34 | 11902. 2 | A | 0.00 6 | 2.28E-02 | 33.5 | LYM 132 | 1227 5.3 | H | 0.08 5 | 0.00E +00 | 82.4 |
| LYM34 | 11904. 3 | A | 0.00 6 | 7.24E-02 | 27.6 | LYM 178 | 1216 4.3 | H | 0.10 6 | 0.00E +00 | 128.4 |
| LYM34 | 11901. 1 | A | 0.00 5 | 8.11E-02 | 15.8 | LYM 132 | 1227 1.4 | H | 0.07 5 | 1.00E-06 | 61.2 |
| LYM35 | 11813. 5 | A | 0.00 5 | 8.90E-02 | 14.7 | LYM1 | 1160 1.1 | H | 0.07 4 | 2.00E-06 | 60.1 |
| CONTR OL | - | A | 0.00 5 | - | 0 | LYM 178 | 1216 3.3 | H | 0.09 3 | 2.00E-06 | 100.8 |
| LYM34 | 11904. 3 | B | 0.11 9 | 8.4 1E-02 | 27.7 | LYM132 | 1227 6.1 | H | 0.08 6 | 6.00E-06 | 84.4 |
| LYM35 | 11813. 5 | B | 0.10 9 | 9.32E-02 | 16.8 | LYM134 | 1231 4.2 | H | 0.07 5 | 3.10E-05 | 61.1 |
| CONTR OL | - | B | 0.09 3 | - | 0 | LYM6 | 1173 6.1 | H | 0.07 7 | 5.10E-05 | 66 |
| LYM37 | 11801. 1 | C | 0.69 7 | 1.40E-05 | 69.9 | LYM268 | 1248 2.1 | H | 0.07 2 | 6.60E-05 | 54.4 |
| LYM12 | 11874. 1 | C | 0.59 | 3.88E-02 | 43.9 | LYM129 | 1257 3.5 | H | 0.07 8 | 1.22E-04 | 68.6 |
| LYM35 | 11813. 5 | C | 0.51 8 | 7.38E-02 | 26.2 | LYM1 | 1160 3.2 | H | 0.06 6 | 5.10E-04 | 42.6 |
| LYM37 | 11803. 2 | C | 0.52 7 | 8.20E-02 | 28.3 | LYM268 | 1248 3.2 | H | 0.07 2 | 5.20E-04 | 54.1 |
| LYM34 | 11902. 2 | C | 0.50 5 | 9.84E-02 | 23.1 | LYM134 | 1231 3.2 | H | 0.09 8 | 8.99E-04 | 111.5 |
| CONTR OL | - | C | 0.41 | - | 0 | LYM178 | 1216 4.2 | H | 0.06 4 | 1.35E-03 | 37.5 |
| LYM37 | 11801. 1 | D | 6.17 8 | 1.90E-05 | 69.5 | LYM5 | 1243 2.1 | H | 0.07 2 | 1.67E-03 | 54.4 |
| LYM41 | 11831. 5 | D | 4.33 1 | 7.99E-02 | 18.8 | LYM268 | 1248 3.4 | H | 0.07 | 3.94E-03 | 50.7 |
| CONTR OL | - | D | 3.64 5 | - | 0 | LYM268 | 1248 2.3 | H | 0.06 4 | 5.44E-03 | 36.9 |
| LYM37 | 11801. 1 | E | 0.5 | 9.40E-05 | 48.1 | LYM5 | 1243 5.1 | H | 0.067 | 9.04E-03 | 44.9 |
| LYM34 | 11902. 4 | E | 0.43 4 | 4.61E-03 | 28.8 | LYM6 | 1173 5.1 | H | 0.06 4 | 1.69E-02 | 37.6 |
| LYM12 | 11871. 1 | E | 0.42 1 | 1.67E-02 | 24.7 | LYM6 | 1173 5.2 | H | 0.05 9 | 1.93E-02 | 27.1 |
| LYM12 | 11874. 1 | E | 0.47 5 | 2.60E-02 | 40.6 | LYM129 | 1257 2.2 | H | 0.05 9 | 2.39E-02 | 26.6 |
| LYM12 | 11873. 4 | E | 0.43 7 | 2.78E-02 | 29.5 | LYM 129 | 1257 1.3 | H | 0.06 1 | 5.64E-02 | 31 |
| LYM12 | 11872. 1 | E | 0.41 9 | 3.43E-02 | 24.3 | LYM132 | 1227 3.2 | H | 0.06 1 | 6.13E-02 | 32.2 |
| LYM37 | 11803. 2 | E | 0.44 | 4.37E-02 | 30.4 | LYM1 | 1160 2.1 | H | 0.05 7 | 6.26E-02 | 21.7 |
| LYM134 | 12312. 3 | E | 0.41 3 | 4.44E-02 | 22.3 | LYM5 | 1243 6.2 | H | 0.05 9 | 8.93E-02 | 27.3 |
| LYM 178 | 12164. 2 | E | 0.45 5 | 4.69E-02 | 34.8 | CONTR OL | - | H | 0.04 6 | - | 0 |
| LYM34 | 11903. 3 | E | 0.41 8 | 4.86E-02 | 24 | LYM236 | 1259 4.3 | A | 0.00 8 | 1.51E-03 | 116.1 |
| LYM41 | 11831. 1 | E | 0.41 | 5.41E-02 | 21.5 | LYM254 | 1247 1.1 | A | 0.00 8 | 2.78E-03 | 98.7 |
| LYM178 | 12161. 1 | E | 0.40 3 | 7.68E-02 | 19.6 | LYM162 | 1223 1.1 | A | 0.00 6 | 3.13E-03 | 46.6 |
| LYM41 | 11833. 1 | E | 0.41 5 | 8.54E-02 | 23.1 | LYM42 | 1199 2.2 | A | 0.00 6 | 4.31E-03 | 47.3 |
| CONTR OL | - | E | 0.33 7 | - | 0 | LYM148 | 1217 1.2 | A | 0.00 6 | 5.66E-03 | 53.1 |
| LYM37 | 11801. 1 | F | 6.04 7 | 6.62E-04 | 37 | LYM 170 | 1245 2.3 | A | 0.00 8 | 1.06E-02 | 100.6 |
| LYM34 | 11902. 4 | F | 5.24 6 | 9.78E-04 | 18.8 | LYM250 | 1261 3.2 | A | 0.00 8 | 1.81E-02 | 93.6 |
| LYM41 | 11831. 5 | F | 5.45 | 2.57E-03 | 23.5 | LYM 172 | 1230 1.3 | A | 0.00 5 | 2.24E-02 | 40.8 |
| LYM41 | 11831.1 | F | 5.583 | 4.31E-03 | 26.5 | LYM206 | 1260 3.2 | A | 0.007 | 2.33E-02 | 69.1 |
| LYM12 | 11873. 4 | F | 5.77 6 | 1.16E-02 | 30.8 | LYM236 | 1259 2.3 | A | 0.01 1 | 2.33E-02 | 194.5 |
| LYM37 | 11801. 2 | F | 5.41 1 | 1.89E-02 | 22.6 | LYM 140 | 1226 1.4 | A | 0.00 5 | 2.36E-02 | 37.6 |
| LYM178 | 12161. 1 | F | 5.19 7 | 2.62E-02 | 17.7 | LYM236 | 1259 1.1 | A | 0.00 8 | 4.87E-02 | 94.9 |
| LYM35 | 11813. 5 | F | 5.42 1 | 3.0 1E-02 | 22.8 | LYM 172 | 1230 4.1 | A | 0.00 6 | 5.05E-02 | 46 |
| LYM12 | 11871. 1 | F | 4.91 | 3.40E-02 | 11.2 | LYM 170 | 1245 3.3 | A | 0.00 8 | 5.28E-02 | 102.6 |
| LYM34 | 11903. 3 | F | 5.27 1 | 5.80E-02 | 19.4 | LYM99 | 1224 4.2 | A | 0.00 5 | 5.35E-02 | 31.8 |
| LYM12 | 11872. 1 | F | 4.93 3 | 9.08E-02 | 11.8 | LYM 176 | 1238 5.1 | A | 0.00 8 | 6.09E-02 | 92.9 |
| LYM37 | 11803. 2 | F | 5.32 5 | 9.97E-02 | 20.6 | LYM254 | 1247 3.1 | A | 0.00 6 | 6.78E-02 | 51.1 |
| CONTR OL | - | F | 4.41 4 | - | 0 | LYM206 | 1260 1.3 | A | 0.00 5 | 8.81E-02 | 23.5 |
| LYM34 | 11902. 2 | G | 0.51 | 1.12E-04 | 35.6 | CONTR OL | - | A | 0.00 4 | - | 0 |
| LYM 132 | 12271. 4 | G | 0.53 5 | 1.40E-04 | 42.2 | LYM250 | 1261 3.2 | B | 0.15 5 | 1.17E-03 | 115.8 |
| LYM37 | 11801. 1 | G | 0.53 | 3.38E-03 | 41.1 | LYM 170 | 1245 2.3 | B | 0.15 | 1.88E-03 | 108.2 |
| LYM35 | 11813. 5 | G | 0.53 2 | 4.76E-03 | 41.6 | LYM 140 | 1226 1.4 | B | 0.10 3 | 2.97E-03 | 42.9 |
| LYM41 | 11831. 5 | G | 0.48 5 | 5.92E-03 | 29.1 | LYM254 | 1247 1.1 | B | 0.14 6 | 4.61E-03 | 103.2 |
| LYM34 | 11904. 3 | G | 0.51 1 | 1.03E-02 | 36 | LYM42 | 1199 2.2 | B | 0.11 2 | 6.00E-03 | 55 |
| LYM37 | 11801. 2 | G | 0.55 3 | 2.50E-02 | 47.2 | LYM236 | 1259 4.3 | B | 0.16 6 | 7.83E-03 | 130.6 |
| LYM35 | 11814. 1 | G | 0.49 6 | 4.22E-02 | 31.9 | LYM148 | 1217 1.2 | B | 0.11 8 | 9.52E-03 | 63.8 |
| LYM37 | 11803. 2 | G | 0.46 | 4.24E-02 | 22.3 | LYM99 | 1224 4.2 | B | 0.09 4 | 1.23E-02 | 31 |
| LYM35 | 11812. 4 | G | 0.49 5 | 4.88E-02 | 31.7 | LYM89 | 1221 1.2 | B | 0.15 7 | 1.54E-02 | 117.7 |
| LYM37 | 11802. 2 | G | 0.69 8 | 5.08E-02 | 85.6 | LYM 162 | 1223 1.1 | B | 0.10 9 | 1.59E-02 | 52 |
| LYM 178 | 12161. 1 | G | 0.43 2 | 5.38E-02 | 15 | LYM236 | 1259 2.3 | B | 0.21 8 | 1.74E-02 | 202.4 |
| LYM41 | 11831. 1 | G | 0.46 7 | 8.64E-02 | 24.4 | LYM 172 | 1230 4.1 | B | 0.11 8 | 2.52E-02 | 63.6 |
| LYM178 | 12163. 3 | G | 0.43 9 | 8.80E-02 | 16.7 | LYM 176 | 1238 5.1 | B | 0.14 7 | 2.76E-02 | 103.9 |
| LYM37 | 11803. 1 | G | 0.44 5 | 9.23E-02 | 18.3 | LYM254 | 1247 3.1 | B | 0.12 3 | 3.29E-02 | 70.5 |
| CONTR OL | - | G | 0.37 6 | - | 0 | LYM206 | 1260 3.2 | B | 0.13 9 | 3.64E-02 | 92.4 |
| LYM37 | 11802. 2 | H | 0.07 1 | 8.35E-04 | 82.3 | LYM236 | 1259 1.1 | B | 0.14 8 | 3.71E-02 | 105.8 |
| LYM37 | 11801. 1 | H | 0.05 6 | 2.75E-03 | 44.2 | LYM 170 | 1245 3.3 | B | 0.15 6 | 4.30E-02 | 116.8 |
| LYM37 | 11801. 2 | H | 0.05 4 | 6.30E-03 | 40.3 | LYM250 | 1261 4.1 | B | 0.09 | 4.32E-02 | 24.8 |
| LYM35 | 11813. 5 | H | 0.05 1 | 1.52E-02 | 32.4 | LYM 172 | 1230 2.2 | B | 0.08 9 | 4.68E-02 | 23.7 |
| LYM 132 | 12271. 4 | H | 0.05 2 | 1.70E-02 | 34.1 | LYM250 | 1261 3.4 | B | 0.09 9 | 4.78E-02 | 37 |
| LYM37 | 11803. 2 | H | 0.05 | 2.33E-02 | 29.6 | LYM206 | 1260 1.3 | B | 0.1 | 5.38E-02 | 39.3 |
| LYM35 | 11812. 4 | H | 0.05 2 | 2.59E-02 | 33.2 | LYM140 | 1226 1.2 | B | 0.08 9 | 5.71E-02 | 23.4 |
| LYM34 | 11902. 2 | H | 0.04 9 | 2.97E-02 | 27.4 | LYM206 | 1260 3.1 | B | 0.08 8 | 6.78E-02 | 22.5 |
| LYM34 | 11904. 3 | H | 0.05 | 7.81E-02 | 28.4 | LYM 170 | 1245 3.2 | B | 0.09 4 | 7.55E-02 | 30.7 |
| LYM12 | 11874. 1 | H | 0.05 | 9.07E-02 | 30.1 | LYM206 | 1260 2.1 | B | 0.12 3 | 8.33E-02 | 70.1 |
| LYM35 | 11814. 1 | H | 0.04 7 | 9.82E-02 | 22.5 | LYM 170 | 1245 2.4 | B | 0.12 8 | 8.37E-02 | 77.1 |
| CONTR OL | - | H | 0.03 9 | - | 0 | LYM140 | 1226 2.2 | B | 0.09 | 9.03E-02 | 24.7 |
| LYM82 | 12203. 5 | A | 0.01 1 | 1.26E-03 | 111.5 | LYM176 | 1238 4.1 | B | 0.09 6 | 9.40E-02 | 33 |
| LYM268 | 12482. 1 | A | 0.00 7 | 2.56E-03 | 34.2 | CONTR OL | - | B | 0.07 2 | - | 0 |
| LYM82 | 12201. 2 | A | 0.00 8 | 5.53E-03 | 51.6 | LYM172 | 1230 1.3 | C | 0.90 9 | 0.00E +00 | 131.8 |
| LYM5 | 12436. 1 | A | 0.00 6 | 8.15E-03 | 24.2 | LYM 176 | 1238 4.1 | C | 0.86 9 | 0.00E +00 | 121.6 |
| LYM86 | 12183. 1 | A | 0.00 7 | 8.76E-03 | 43.6 | LYM206 | 1260 3.2 | C | 0.96 2 | 0.00E +00 | 145.4 |
| LYM 129 | 12573. 1 | A | 0.01 | 1.15E-02 | 91.5 | LYM236 | 1259 1.1 | C | 1.00 8 | 0.00E +00 | 157.2 |
| LYM268 | 12484. 2 | A | 0.00 9 | 2.45E-02 | 73.6 | LYM236 | 1259 2.3 | C | 0.99 9 | 0.00E +00 | 154.8 |
| LYM268 | 12483. 4 | A | 0.01 4 | 2.66E-02 | 180.3 | LYM236 | 1259 4.3 | C | 0.86 2 | 0.00E +00 | 119.8 |
| LYM 129 | 12572. 2 | A | 0.00 7 | 2.78E-02 | 38.7 | LYM250 | 1261 3.2 | C | 0.93 7 | 0.00E +00 | 139 |
| LYM8 | 11984. 1 | A | 0.01 1 | 3.56E-02 | 125.9 | LYM254 | 1247 1.1 | C | 0.83 2 | 0.00E +00 | 112.2 |
| LYM86 | 12183. 3 | A | 0.00 7 | 9.36E-02 | 39.7 | LYM170 | 1245 2.4 | C | 0.78 4 | 1.00E-06 | 100.1 |
| CONTR OL | - | A | 0.00 5 | - | 0 | LYM170 | 1245 2.3 | C | 0.82 6 | 1.00E-05 | 110.8 |
| LYM268 | 12482. 1 | B | 0.13 8 | 1.55E-03 | 47.8 | LYM170 | 1245 3.2 | C | 0.78 9 | 1.00E-05 | 101.4 |
| LYM82 | 12203. 5 | B | 0.18 9 | 5.29E-03 | 102.1 | LYM148 | 1217 1.2 | C | 0.80 9 | 1.10E-05 | 106.5 |
| LYM86 | 12183. 1 | B | 0.14 2 | 1.04E-02 | 51.8 | LYM206 | 1260 2.1 | C | 0.89 5 | 1.10E-05 | 128.4 |
| LYM129 | 12573. 1 | B | 0.17 1 | 1.16E-02 | 83.7 | LYM 172 | 1230 4.1 | C | 0.71 | 1.30E-05 | 81 |
| LYM268 | 12484. 2 | B | 0.14 6 | 1.19E-02 | 56.8 | LYM 162 | 1223 1.1 | C | 0.89 8 | 1.70B-05 | 129 |
| LYM82 | 12201. 2 | B | 0.13 3 | 1.75E-02 | 42.9 | LYM 162 | 1223 1.2 | C | 0.85 6 | 2.40E-05 | 118.5 |
| LYM268 | 12483. 4 | B | 0.24 3 | 2.03E-02 | 160.1 | LYM 176 | 1238 1.3 | C | 0.69 1 | 4.30E-05 | 76.2 |
| LYM 129 | 12572. 2 | B | 0.12 6 | 4.67E-02 | 35.3 | LYM250 | 1261 4.1 | C | 0.74 | 8.00E-05 | 88.8 |
| LYM5 | 12436. 1 | B | 0.11 | 7.62E-02 | 18.2 | LYM206 | 1260 3.3 | C | 0.73 6 | 1.02E-04 | 87.7 |
| CONTR OL | - | B | 0.09 3 | - | 0 | LYM89 | 1221 4.2 | C | 0.71 3 | 1.44E-04 | 81.9 |
| LYM268 | - 12483. 4 | C | 1.19 3 | - 0.00E +00 | 184.9 | LYM 176 | 1238 5.1 | C | 0.84 7 | 1.46E-04 | 116 |
| LYM82 | 12203. 5 | C | 0.93 3 | 1.00E-06 | 122.9 | LYM172 | 1230 2.2 | C | 0.77 7 | 1.54E-04 | 98.2 |
| LYM8 | 11984. 1 | C | 0.84 6 | 1.20E-05 | 102.1 | LYM254 | 1247 1.2 | C | 0.65 7 | 2.37E-04 | 67.6 |
| LYM82 | 12203. 2 | C | 1.04 7 | 2.30E-05 | 149.9 | LYM99 | 1224 4.2 | C | 0.66 5 | 5.09E-04 | 69.6 |
| LYM268 | 12484. 2 | C | 0.74 9 | 4.40E-05 | 79 | LYM250 | 1261 1.3 | C | 0.66 6 | 5.70E-04 | 69.8 |
| LYM5 | 12436. 2 | C | 0.70 8 | 1.17E-04 | 69 | LYM 172 | 1230 1.2 | C | 0.62 1 | 6.43E-04 | 58.4 |
| LYM44 | 11884. 3 | C | 0.68 3 | 5.09E-04 | 63.2 | LYM 170 | 1245 3.3 | C | 0.70 9 | 6.67E-04 | 80.9 |
| LYM86 | 12183. 3 | C | 0.59 9 | 4.64E-03 | 43.1 | LYM89 | 1221 4.1 | C | 0.69 4 | 7.89E-04 | 77.2 |
| LYM 129 | 12573. 1 | C | 0.63 4 | 4.95E-03 | 51.5 | LYM236 | 1259 2.4 | C | 0.69 8 | 8.40E-04 | 78 |
| LYM129 | 12572. 2 | C | 0.61 6 | 5.99E-03 | 47.2 | LYM42 | 1199 2.2 | C | 0.75 | 8.94E-04 | 91.4 |
| LYM86 | 12182. 3 | C | 0.58 5 | 1.21E. 02 | 39.6 | LYM140 | 1226 1.2 | C | 0.63 4 | 1.55E-03 | 61.8 |
| LYM268 | 12481. 3 | C | 0.65 5 | 1.25E-02 | 56.5 | LYM89 | 1221 4.3 | C | 0.63 6 | 1.76E-03 | 62.3 |
| LYM5 | 12436. 1 | C | 0.59 2 | 1.26E-02 | 41.3 | LYM89 | 1221 1.2 | C | 0.69 | 1.81E-03 | 76 |
| LYM44 | 11882. 1 | C | 0.61 2 | 1.33E-02 | 46.2 | LYM 162 | 1223 3.2 | C | 0.59 4 | 2.55E-03 | 51.5 |
| LYM268 | 12482. 1 | C | 0.55 8 | 3.20E-02 | 33.2 | LYM99 | 1224 4.1 | C | 0.63 1 | 3.12E-03 | 60.9 |
| LYM44 | 11885. 3 | C | 0.54 6 | 5.94E-02 | 30.3 | LYM 140 | 1226 1.4 | C | 0.61 4 | 3.48E-03 | 56.5 |
| CONTR OL | - | C | 0.41 9 | - | 0 | LYM254 | 1247 2.3 | C | 0.60 9 | 4.02E-03 | 55.4 |
| LYM268 | 12483. 4 | D | 10.3 19 | 2.40E-05 | 190.3 | LYM162 | 1223 4.3 | C | 0.59 1 | 4.28E-03 | 50.8 |
| LYM5 | 12436. 2 | D | 6.06 9 | 1.53E-03 | 70.7 | LYM3 | 1204 1.2 | C | 0.58 4 | 5.59E-03 | 48.9 |
| LYM44 | 11884. 3 | D | 5.87 6 | 2.29E-03 | 65.3 | LYM99 | 1224 1.1 | C | 0.57 3 | 6.37E-03 | 46.2 |
| LYM82 | 12203. 5 | D | 8.17 | 2.69E-03 | 129.8 | LYM89 | 1221 4.4 | C | 0.60 1 | 7.47E-03 | 53.3 |
| LYM268 | 12484. 2 | D | 6.62 7 | 3.76E-03 | 86.4 | LYM250 | 1261 3.4 | C | 0.59 8 | 7.72E-03 | 52.6 |
| LYM86 | 12183. 3 | D | 5.31 3 | 3.87E-03 | 49.5 | LYM290 | 1250 2.1 | C | 0.63 5 | 8.21E-03 | 61.9 |
| LYM86 | 12182. 3 | D | 4.99 6 | 8.33E-03 | 40.6 | LYM290 | 1250 1.3 | C | 0.63 6 | 1.00E-02 | 62.3 |
| LYM129 | 12572. 2 | D | 5.33 6 | 1.25E-02 | 50.1 | LYM148 | 1217 3.5 | C | 0.60 8 | 1.17E-02 | 55.2 |
| LYM129 | 12573. 1 | D | 5.39 | 1.80E-02 | 51.6 | LYM148 | 1217 3.1 | C | 0.55 5 | 1.44E-02 | 41.6 |
| LYM5 | 12436. 1 | D | 5.20 3 | 2.16E-02 | 46.4 | LYM206 | 1260 1.3 | C | 0.60 6 | 1.63E-02 | 54.7 |
| LYM82 | 12203. 2 | D | 9.17 2 | 2.69E-02 | 158 | LYM148 | 1217 2.1 | C | 0.54 4 | 3.03E-02 | 38.8 |
| LYM8 | 11984. 1 | D | 7.27 7 | 3.07E-02 | 104.7 | LYM254 | 1247 4.3 | C | 0.54 3 | 3.66E-02 | 38.4 |
| LYM44 | 11885. 3 | D | 4.76 8 | 3.08E. 02 | 34.1 | LYM3 | 1204 1.1 | C | 0.53 1 | 3.68E-02 | 35.5 |
| LYM268 | 12482. 1 | D | 4.71 4 | 3.75E-02 | 32.6 | LYM99 | 1224 3.2 | C | 0.58 4 | 4.06E-02 | 48.9 |
| LYM44 | 11882. 1 | D | 5.20 5 | 6.64E-02 | 46.4 | CONTR OL | - | C | 0.39 2 | - | 0 |
| CONTR OL | - | D | 3.55 5 | - | 0 | LYM176 | 1238 4.1 | D | 7.65 5 | 0.00E +00 | 132.7 |
| LYM268 | 12483. 4 | E | 0.52 9 | 1.08E-02 | 41.8 | LYM172 | 1230 4.1 | D | 6.29 8 | 1.30E-05 | 91.4 |
| LYM44 | 11884. 3 | E | 0.50 4 | 2.2 1E-02 | 35.2 | LYM170 | 1245 2.4 | D | 6.86 3 | 3.10E-05 | 108.6 |
| LYM82 | 12203. 2 | E | 0.52 8 | 2.55E-02 | 41.7 | LYM176 | 1238 1.3 | D | 6.03 9 | 3.20E-05 | 83.6 |
| LYM82 | 12203. 5 | E | 0.49 4 | 6.19E-02 | 32.5 | LYM254 | 1247 1.2 | D | 5.66 3 | 1.13E-04 | 72.2 |
| LYM86 | 12182. 3 | E | 0.47 4 | 7.22E-02 | 27.2 | LYM172 | 1230 1.3 | D | 7.85 3 | 3.12E-04 | 138.7 |
| LYM86 | 12183. 3 | E | 0.46 9 | 9.75E-02 | 25.7 | LYM172 | 1230 1.2 | D | 5.34 7 | 3.95E-04 | 62.5 |
| CONTROL OL | - | E | 0.37 3 | - | 0 | LYM254 | 1247 1.1 | D | 7.28 | 5.95E-04 | 121.3 |
| LYM268 | 12483. 4 | F | 6.55 1 | 4.20E-05 | 48.7 | LYM99 | 1224 1.1 | D | 4.88 5 | 1.02E-03 | 48.5 |
| LYM82 | 12203. 5 | F | 6.54 | 4.30E-05 | 48.4 | LYM162 | 1223 3.2 | D | 5.09 5 | 1.13E-03 | 54.9 |
| LYM82 | 12203. 2 | F | 6.36 6 | 2.44E-04 | 44.5 | LYM236 | 1259 1.1 | D | 8.65 5 | 1.52E-03 | 163.1 |
| LYM44 | 11884. 3 | F | 5.87 5 | 6.63E-04 | 33.4 | LYM236 | 1259 4.3 | D | 7.79 6 | 2.20E-03 | 137 |
| LYM86 | 12182. 3 | F | 5.58 6 | 2.72E-03 | 26.8 | LYM162 | 1223 4.3 | D | 5.02 3 | 2.44E-03 | 52.7 |
| LYM86 | 12183. 3 | F | 5.72 4 | 3.20E-03 | 29.9 | LYM206 | 1260 3.2 | D | 8.33 | 2.58E-03 | 153.2 |
| LYM8 | 11983. 1 | F | 5.55 6 | 3.24E-03 | 26.1 | LYM 148 | 1217 3.1 | D | 4.69 2 | 2.92E-03 | 42.6 |
| LYM5 | 12436. 1 | F | 5.83 1 | 3.79E-03 | 32.3 | LYM236 | 1259 2.3 | D | 8.56 4 | 2.99E-03 | 160.3 |
| LYM268 | 12484. 2 | F | 5.89 8 | 5.85E-03 | 33.9 | LYM3 | 1204 1.2 | D | 5.08 3 | 3.81E-03 | 54.5 |
| LYM86 | 12181. 2 | F | 5.39 6 | 7.40E-03 | 22.5 | LYM 140 | 1226 1.4 | D | 5.32 1 | 3.93E-03 | 61.7 |
| LYM 129 | 12572. 2 | F | 5.55 5 | 8.48E-03 | 26.1 | LYM250 | 1261 3.2 | D | 8.02 2 | 4.46E-03 | 143.9 |
| LYM5 | 12432. 2 | F | 5.25 2 | 2.59E-02 | 19.2 | LYM148 | 1217 1.2 | D | 7.07 6 | 4.80E-03 | 115.1 |
| LYM44 | 11882. 1 | F | 5.40 9 | 3.28E-02 | 22.8 | LYM 170 | 1245 3.2 | D | 6.92 | 5.2 1E-03 | 110.4 |
| LYM5 | 12432. 1 | F | 5.11 6 | 3.85E-02 | 16.1 | LYM89 | 1221 4.3 | D | 5.75 | 6.25E-03 | 74.8 |
| LYM44 | 11885. 4 | F | 5.08 5 | 4.84E-02 | 15.4 | LYM206 | 1260 3.3 | D | 6.59 9 | 8.50E-03 | 100.6 |
| LYM 129 | 12573. 1 | F | 5.08 2 | 5.14E-02 | 15.3 | LYM89 | 1221 4.2 | D | 6.28 5 | 1.03E-02 | 91 |
| LYM5 | 12436. 2 | F | 5.77 2 | 5.3 1E-02 | 31 | LYM99 | 1224 4.2 | D | 5.74 2 | 1.05E-02 | 74.5 |
| LYM5 | 12435. 1 | F | 5.49 8 | 6.21E-02 | 24.8 | LYM250 | 1261 4.1 | D | 6.30 8 | 1.09E-02 | 91.8 |
| LYM8 | 11982. 6 | F | 5.08 9 | 7.04E-02 | 15.5 | LYM 170 | 1245 2.3 | D | 7.21 | 1.17E-02 | 119.2 |
| LYM8 | 11984. 1 | F | 5.99 9 | 9.59E-02 | 36.2 | LYM140 | 1226 1.2 | D | 5.41 | 1.38E-02 | 64.5 |
| CONTR OL | - - | F | 4.40 6 | - - | 0 | LYM254 | 1247 2.3 | D | 5.26 8 | 1.47E-02 | 60.1 |
| LYM5 | 12436. 1 | G | 0.63 8 | 1.29E-04 | 50.3 | LYM250 | 1261 1.3 | D | 5.79 6 | 1.51E-02 | 76.2 |
| LYM 129 | 12573. 1 | G | 0.84 9 | 1.56E-04 | 99.9 | LYM89 | 1221 4.1 | D | 6.21 | 1.65E-02 | 88.8 |
| LYM82 | 12203. 5 | G | 0.84 7 | 2.51E-04 | 99.4 | LYM172 | 1230 2.2 | D | 6.67 2 | 1.89E-02 | 102.8 |
| LYM86 | 12183. 3 | G | 0.68 7 | 4.03E-04 | 61.7 | LYM206 | 1260 2.1 | D | 7.77 2 | 1.91E-02 | 136.2 |
| LYM268 | 12482. 1 | G | 0.60 3 | 5.49E-04 | 42 | LYM162 | 1223 1.1 | D | 7.88 5 | 1.92E-02 | 139.7 |
| LYM268 | 12483. 4 | G | 1.05 4 | 6.89E-04 | 148 | LYM3 | 1204 1.1 | D | 4.68 4 | 2.07E-02 | 42.4 |
| LYM82 | 12201. 2 | G | 0.64 3 | 1.52E-03 | 51.4 | LYM 162 | 1223 1.2 | D | 7.35 9 | 2.76E-02 | 123.7 |
| LYM268 | 12484. 2 | G | 0.76 8 | 1.52E-03 | 80.8 | LYM236 | 1259 2.4 | D | 6.22 4 | 2.84E-02 | 89.2 |
| LYM86 | 12183. 1 | G | 0.60 2 | 2.17E-03 | 41.6 | LYM99 | 1224 4.1 | D | 5.46 7 | 2.84E-02 | 66.2 |
| LYM44 | 11882. 1 | G | 0.54 4 | 4.43E-03 | 28.1 | LYM 176 | 1238 5.1 | D | 7.50 3 | 2.87E-02 | 128.1 |
| LYM44 | 11885. 3 | G | 0.55 2 | 4.98E-03 | 29.9 | LYM290 | 1250 1.2 | D | 4.36 7 | 2.94E-02 | 32.8 |
| LYM82 | 12204. 6 | G | 0.55 8 | 7.03E-03 | 31.3 | LYM148 | 1217 2.1 | D | 4.71 1 | 3.20E-02 | 43.2 |
| LYM 129 | 12572. 2 | G | 0.67 1 | 7.23E-03 | 57.9 | LYM 170 | 1245 3.3 | D | 6.28 2 | 3.38E-02 | 91 |
| LYM44 | 11884. 3 | G | 0.53 2 | 7.42E-03 | 25.3 | LYM250 | 1261 3.4 | D | 5.34 5 | 3.40E-02 | 62.5 |
| LYM5 | 12436. 2 | G | 0.54 5 | 8.39E-03 | 28.2 | LYM89 | 1221 1.2 | D | 6.24 7 | 4.75E-02 | 89.9 |
| LYM8 | 11984. 1 | G | 0.84 9 | 1.84E-02 | 99.8 | LYM89 | 1221 4.4 | D | 4.93 2 | 4.87E-02 | 49.9 |
| LYM86 | 12182. 3 | G | 0.52 6 | 2.43E-02 | 23.7 | LYM42 | 1199 2.2 | D | 6.54 | 5.04E-02 | 98.8 |
| LYM6 | 11735. 2 | G | 0.49 | 8.02E-02 | 15.4 | LYM148 | 1217 3.5 | D | 5.37 2 | 6.79E-02 | 63.3 |
| LYM82 | 12203. 2 | G | 0.83 4 | 8.07E-02 | 96.2 | LYM254 | 1247 4.3 | D | 4.62 5 | 6.91E-02 | 40.6 |
| CONTR OL | - | G | 0.42 5 | - | 0 | LYM290 | 1250 2.1 | D | 5.37 | 7.87E-02 | 63.2 |
| LYM129 | 12573. 1 | H | 0.08 5 | 0.00E +00 | 100.9 | LYM206 | 1260 1.3 | D | 5.29 6 | 8.29E-02 | 61 |
| LYM268 | 12483. 4 | H | 0.10 8 | 0.00E +00 | 156.5 | CONTR OL | - | D | 3.29 | - | 0 |
| LYM8 | 11984. 1 | H | 0.08 9 | 0.00E +00 | 110.3 | LYM236 | 1259 1.1 | E | 0.59 4 | 2.26E-03 | 47.4 |
| LYM82 | 12203. 5 | H | 0.08 8 | 1.00E-06 | 108.4 | LYM172 | 1230 2.2 | E | 0.59 6 | 3.04E-03 | 47.9 |
| LYM268 | 12484. 2 | H | 0.07 4 | 2.30E-05 | 75.8 | LYM 176 | 1238 1.3 | E | 0.57 7 | 4.24E-03 | 43.2 |
| LYM86 | 12183. 3 | H | 0.06 6 | 2.60E-04 | 56.3 | LYM140 | 1226 1.2 | E | 0.56 6 | 4.95E-03 | 40.6 |
| LYM268 | 12482. 1 | H | 0.06 2 | 1.28E-03 | 46.3 | LYM99 | 1224 4.1 | E | 0.56 6 | 6.16E-03 | 40.6 |
| LYM129 | 12572. 2 | H | 0.06 5 | 1.65E-03 | 53.7 | LYM172 | 1230 4.1 | E | 0.56 4 | 7.50E-03 | 40.1 |
| LYM82 | 12203. 2 | H | 0.08 7 | 1.78E-03 | 106.5 | LYM 170 | 1245 3.2 | E | 0.56 3 | 7.87E-03 | 39.8 |
| LYM5 | 12436. 1 | H | 0.06 | 2.89E-03 | 41.7 | LYM 170 | 1245 2.4 | E | 0.56 7 | 8.02E-03 | 40.8 |
| LYM86 | 12183. 1 | H | 0.06 | 5.56E-03 | 41 | LYM254 | 1247 2.3 | E | 0.55 3 | 8.60E-03 | 37.2 |
| LYM5 | 12436. 2 | H | 0.05 9 | 8.00E-03 | 39 | LYM148 | 1217 1.2 | E | 0.56 3 | 9.63E-03 | 39.9 |
| LYM82 | 12201. 2 | H | 0.05 9 | 8.96E-03 | 40.3 | LYM206 | 1260 2.1 | E | 0.56 2 | 1.25E-02 | 39.6 |
| LYM82 | 12204. 6 | H | 0.05 7 | 1.25E-02 | 34.6 | LYM99 | 1224 4.2 | E | 0.54 | 1.39E-02 | 34 |
| LYM44 | 11884. 3 | H | 0.05 6 | 1.64E-02 | 32.6 | LYM236 | 1259 2.3 | E | 0.55 5 | 2.06E-02 | 37.7 |
| LYM268 | 12481. 3 | H | 0.06 1 | 2.53E-02 | 44.7 | LYM250 | 1261 3.2 | E | 0.54 2 | 2.10E-02 | 34.5 |
| LYM44 | 11885. 3 | H | 0.05 6 | 2.57E-02 | 33.6 | LYM254 | 1247 1.2 | E | 0.55 | 2.13E-02 | 36.6 |
| LYM44 | 11882. 1 | H | 0.05 5 | 3.23E-02 | 29.4 | LYM206 | 1260 3.2 | E | 0.55 7 | 2.43E-02 | 38.4 |
| LYM5 | 12432. 2 | H | 0.05 7 | 5.25E-02 | 34.7 | LYM 170 | 1245 2.3 | E | 0.53 8 | 2.59E-02 | 33.7 |
| LYM86 | 12182. 3 | H | 0.05 3 | 7.08E-02 | 24.8 | LYM89 | 1221 4.4 | E | 0.55 7 | 2.64E-02 | 38.2 |
| CONTR OL | - | H | 0.04 2 | - | 0 | LYM236 | 1259 2.4 | E | 0.53 4 | 2.66E-02 | 32.6 |
| LYM89 | 12214. 3 | A | 0.00 6 | 5.91E-04 | 43.4 | LYM206 | 1260 3.3 | E | 0.54 2 | 2.79E-02 | 34.5 |
| LYM250 | 12611. 3 | A | 0.00 6 | 4.67E-03 | 44.1 | LYM 172 | 1230 1.3 | E | 0.52 7 | 3.29E-02 | 30.7 |
| CONTR OL | - | A | 0.00 4 | - | 0 | LYM172 | 1230 1.2 | E | 0.52 3 | 3.72E-02 | 29.8 |
| LYM250 | 12611. 3 | C | 0.72 4 | 1.68E-02 | 39.6 | LYM148 | 1217 3.1 | E | 0.54 7 | 3.85E-02 | 35.7 |
| CONTR OL | - | C | 0.51 9 | - | 0 | LYM89 | 1221 4.1 | E | 0.55 4 | 3.89E-02 | 37.6 |
| LYM250 | 12611. 3 | D | 6.63 3 | 3.09E-02 | 40.5 | LYM89 | 1221 1.2 | E | 0.52 9 | 3.96E-02 | 31.3 |
| CONTR OL | - | D | 4.72 1 | - | 0 | LYM250 | 1261 4.1 | E | 0.53 5 | 4.14E-02 | 32.8 |
| LYM250 | 12611. 3 | E | 0.53 1 | 7.01E-02 | 18.9 | LYM 162 | 1223 1.1 | E | 0.54 | 4.51E-02 | 34.1 |
| CONTR OL | - | E | 0.44 7 | - | 0 | LYM42 | 1199 2.2 | E | 0.52 8 | 5.37E-02 | 31.2 |
| LYM250 | 12611. 3 | F | 6.62 7 | 1.48E-03 | 22.7 | LYM206 | 1260 1.3 | E | 0.51 | 6.40E-02 | 26.6 |
| LYM236 | 12591. 1 | F | 6.19 7 | 1.53E-02 | 14.7 | LYM250 | 1261 1.3 | E | 0.52 5 | 6.99E-02 | 30.4 |
| LYM99 | 12243. 2 | F | 6.22 | 2.59E-02 | 15.1 | LYM206 | 1260 4.3 | E | 0.50 3 | 7.47E-02 | 24.9 |
| CONTR OL | - | F | 5.40 2 | - | 0 | LYM236 | 1259 4.3 | E | 0.51 | 8.71E-02 | 26.7 |
| LYM250 | 12611. 3 | G | 0.63 3 | 4.48E-03 | 30.7 | LYM 176 | 1238 5.1 | E | 0.52 6 | 8.97E-02 | 30.6 |
| LYM89 | 12214. 3 | G | 0.63 2 | 3.84E-02 | 30.6 | LYM254 | 1247 1.1 | E | 0.51 3 | 9.45E-02 | 27.4 |
| CONTR OL | - | G | 0.48 4 | - | 0 | LYM176 | 1238 4.1 | E | 0.50 7 | 9.46E-02 | 26 |
| LYM250 | 12611. 3 | H | 0.06 6 | 4.26E-02 | 29.7 | CONTR OL | - | E | 0.40 3 | - | 0 |
| CONTR OL | - | H | 0.05 1 | - | 0 | LYM 170 | 1245 3.2 | F | 7.24 6 | 1.00E-06 | 71.9 |
| LYM 130 | 12332. 2 | A | 0.00 8 | 7.52E-04 | 183.7 | LYM170 | 1245 2.4 | F | 7.20 7 | 1.00E-06 | 71 |
| LYM 102 | 12222. 1 | A | 0.00 6 | 03 | 86.6 | LYM236 | 1259 1.1 | F | 7.23 9 | 1.00E-06 | 71.7 |
| LYM 130 | 12334. 1 | A | 0.00 7 | 2.90E-03 | 123.4 | LYM 148 | 1217 1.2 | F | 6.95 1 | 3.00E-06 | 64.9 |
| LYM138 | 12561. 3 | A | 0.00 5 | 3.10E-03 | 74.1 | LYM 172 | 1230 1.3 | F | 7.00 6 | 3.00E-06 | 66.2 |
| LYM141 | 12404. 2 | A | 0.00 6 | 8.08E-03 | 106.7 | LYM99 | 1224 4.2 | F | 6.7 | 3.00E-06 | 59 |
| LYM 106 | 12142. 2 | A | 0.00 4 | 1.83E-02 | 48.1 | LYM 176 | 1238 1.3 | F | 7.08 9 | 4.00E-06 | 68.2 |
| LYM 138 | 12566. 1 | A | 0.00 4 | 1.99E-02 | 46.4 | LYM 176 | 1238 4.1 | F | 7.17 1 | 3.30E-05 | 70.1 |
| LYM 100 | 12133. 3 | A | 0.00 5 | 2.31E-02 | 62.3 | LYM99 | 1224 4.1 | F | 6.71 5 | 3.40E-05 | 59.3 |
| LYM141 | 12404. 3 | A | 0.00 8 | 3.47E-02 | 151 | LYM254 | 1247 2.3 | F | 6.57 3 | 3.80E-05 | 56 |
| LYM106 | 12144. 3 | A | 0.00 5 | 3.82E-02 | 59 | LYM172 | 1230 4.1 | F | 6.85 2 | 4.90E-05 | 62.6 |
| LYM119 | 12462. 1 | A | 0.00 4 | 6.49E-02 | 46.4 | LYM140 | 1226 1.2 | F | 6.62 3 | 5.40E-05 | 57.1 |
| LYM 130 | 12331. 3 | A | 0.00 4 | 7.03E-02 | 48.1 | LYM 170 | 1245 2.3 | F | 6.77 7 | 6.70E-05 | 60.8 |
| LYM 137 | 12152. 1 | A | 0.00 8 | 7.19E-02 | 160.3 | LYM3 | 1204 1.1 | F | 6.22 8 | 7.30E-05 | 47.8 |
| LYM100 | 12131. 2 | A | 0.00 4 | 7.74E-02 | 45.6 | LYM148 | 1217 3.5 | F | 6.63 9 | 7.60E-05 | 57.5 |
| LYM102 | 12221. 2 | A | 0.00 7 | 8.15E-02 | 117.6 | LYM206 | 1260 3.2 | F | 7.10 5 | 1.19E-04 | 68.6 |
| LYM 130 | 12332. 1 | A | 0.00 4 | 9.91E-02 | 48.1 | LYM254 | 1247 1.1 | F | 6.78 1 | 1.27E-04 | 60.9 |
| CONTR OL | - | A | 0.00 3 | - | 0 | LYM89 | 1221 4.2 | F | 7.10 8 | 1.46E-04 | 68.6 |
| LYM141 | 12404. 2 | B | 0.13 4 | 1.20E-05 | 79 | LYM206 | 1260 3.1 | F | 5.79 3 | 2.05E-04 | 37.4 |
| LYM138 | 12561. 3 | B | 0.11 4 | 2.78E-04 | 52.9 | LYM250 | 1261 3.2 | F | 6.78 2 | 2.08E-04 | 60.9 |
| LYM 102 | 12222. 1 | B | 0.11 3 | 9.40E-04 | 51.4 | LYM140 | 1226 1.4 | F | 6.07 9 | 2.18E-04 | 44.2 |
| LYM 130 | 12332. 1 | B | 0.11 7 | 7.14E-03 | 55.7 | LYM172 | 1230 1.2 | F | 6.31 7 | 2.83E-04 | 49.9 |
| LYM 130 | 12332. 2 | B | 0.18 9 | 1.06E-02 | 152.1 | LYM290 | 1250 1.2 | F | 5.64 8 | 3.03E-04 | 34 |
| LYM106 | 12142. 2 | B | 0.10 4 | 1.16E-02 | 38.3 | LYM290 | 1250 1.3 | F | 6.47 2 | 3.12E-04 | 53.6 |
| LYM 130 | 12334. 1 | B | 0.17 6 | 1.48E-02 | 135 | LYM236 | 1259 4.3 | F | 7.03 3 | 3.99E-04 | 66.9 |
| LYM141 | 12404. 3 | B | 0.15 5 | 3.07E-02 | 106.5 | LYM89 | 1221 1.2 | F | 6.81 3 | 4.03E-04 | 61.6 |
| LYM138 | 12566. 1 | B | 0.1 | 3.93E-02 | 33.3 | LYM 162 | 1223 1.1 | F | 7.20 3 | 5.06E-04 | 70.9 |
| LYM 137 | 12152. 1 | B | 0.16 4 | 4.75E-02 | 119.7 | LYM 148 | 1217 2.1 | F | 5.78 9 | 5.46E-04 | 37.3 |
| LYM 100 | 12131. 2 | B | 0.10 8 | 5.45E-02 | 44.1 | LYM99 | 1224 1.1 | F | 5.77 | 6.25E-04 | 36.9 |
| LYM119 | 12462. 1 | B | 0.11 | 6.36E-02 | 46.8 | LYM206 | 1260 2.1 | F | 6.96 | 6.53E-04 | 65.1 |
| LYM113 | 12444. 5 | B | 0.14 4 | 6.90E-02 | 91.8 | LYM236 | 1259 2.4 | F | 6.74 1 | 7.46E-04 | 59.9 |
| CONTR OL | - | B | 0.07 5 | - | 0 | LYM236 | 1259 2.3 | F | 6.47 1 | 8.01E-04 | 53.5 |
| LYM137 | 12153. 1 | C | 0.75 | 3.30E-05 | 73.3 | LYM 162 | 1223 1.2 | F | 6.80 5 | 8.61E-04 | 61.4 |
| LYM 102 | 12222. 1 | C | 0.68 1 | 3.41E-04 | 57.4 | LYM250 | 1261 3.4 | F | 6.19 6 | 1.05E-03 | 47 |
| LYM141 | 12404. 3 | C | 0.73 9 | 1.80E-03 | 70.8 | LYM 176 | 1238 5.1 | F | 6.84 2 | 1.76E-03 | 62.3 |
| LYM137 | 12152. 1 | C | 0.63 | 2.56E-03 | 45.6 | LYM 172 | 1230 2.2 | F | 6.85 1 | 1.79E-03 | 62.5 |
| LYM 138 | 12561. 3 | C | 0.61 5 | 2.31E-02 | 42.1 | LYM250 | 1261 4.1 | F | 6.73 3 | 1.94E-03 | 59.7 |
| LYM130 | 12332. 2 | C | 0.58 8 | 2.32E-02 | 36 | LYM206 | 1260 3.3 | F | 6.66 6 | 1.97E-03 | 58.2 |
| LYM102 | 12221. 2 | C | 0.57 8 | 5.38E-02 | 33.7 | LYM42 | 1199 2.2 | F | 6.76 5 | 2.50E-03 | 60.5 |
| LYM100 | 12133. 3 | C | 0.55 5 | 5.62E-02 | 28.4 | LYM89 | 1221 4.1 | F | 6.51 9 | 2.76E-03 | 54.7 |
| LYM141 | 12404. 2 | C | 0.54 7 | 7.44E-02 | 26.5 | LYM254 | 1247 1.2 | F | 6.58 9 | 2.87E-03 | 56.3 |
| CONTR OL | - - | C | 0.43 3 | - - | 0 | LYM 162 | 1223 4.3 | F | 5.85 8 | 3.38E-03 | 39 |
| LYM 102 | 12222. 1 | D | 5.94 2 | 1.65E-03 | 46.8 | LYM3 | 1204 1.2 | F | 5.20 3 | 3.62E-03 | 23.4 |
| LYM 137 | 12152. 1 | D | 5.64 8 | 2.45E-03 | 39.5 | LYM250 | 1261 1.3 | F | 6.66 4 | 3.93E-03 | 58.1 |
| LYM137 | 12153. 1 | D | 6.55 1 | 2.59E-03 | 61.8 | LYM206 | 1260 1.3 | F | 5.60 5 | 5.24E-03 | 33 |
| LYM100 | 12133. 3 | D | 5.09 9 | 5.27E-02 | 26 | LYM89 | 1221 4.3 | F | 5.83 7 | 6.12E-03 | 38.5 |
| LYM 130 | 12332. 2 | D | 5.25 3 | 6.20E-02 | 29.8 | LYM 162 | 1223 3.2 | F | 5.95 8 | 8.02E-03 | 41.4 |
| CONTR OL | - - | D | 4.04 8 | - - | 0 | LYM254 | 1247 4.3 | F | 5.75 8 | 8.41E-03 | 36.6 |
| LYM137 | 12153. 1 | E | 0.56 9 | 1.28E-03 | 41.7 | LYM3 | 1204 3.2 | F | 4.99 3 | 1.23E-02 | 18.5 |
| LYM141 | 12404. 3 | E | 0.55 9 | 1.44E-02 | 39.3 | LYM42 | 1199 2.1 | F | 5.51 4 | 1.45E-02 | 30.8 |
| LYM 138 | 12561. 3 | E | 0.51 | 3.15E-02 | 27 | LYM290 | 1250 2.1 | F | 5.90 9 | 2.95E-02 | 40.2 |
| LYM 102 | 12222. 1 | E | 0.50 1 | 4.11E-02 | 24.8 | LYM148 | 1217 3.1 | F | 6.08 6 | 3.14E-02 | 44.4 |
| LYM 100 | 12133. 3 | E | 0.49 3 | 5.35E-02 | 22.9 | LYM206 | 1260 4.3 | F | 4.95 9 | 4.80E-02 | 17.7 |
| CONTR OL | - | E | 0.40 1 | - - | 0 | LYM 148 | 1217 4.1 | F | 4.97 1 | 6.23E-02 | 17.9 |
| LYM1 38 | 12561. 3 | F | 6.64 9 | 2.18E-03 | 31.5 | LYM99 | 1224 3.2 | F | 5.67 5 | 6.51E-02 | 34.6 |
| LYM100 | 12133. 3 | F | 6.46 9 | 2.22E-03 | 27.9 | LYM140 | 1226 2.3 | F | 5.43 7 | 8.77E-02 | 29 |
| LYM138 | 12562. 1 | F | 5.95 7 | 5.93E-03 | 17.8 | LYM42 | 1199 4.1 | F | 5.06 8 | 9.11E-02 | 20.2 |
| LYM 102 | 12222. 1 | F | 6.27 | 6.30E-03 | 24 | CONTR OL | - - | F | 4.21 5 | - - | 0 |
| LYM 137 | 12153. 1 | F | 6.74 3 | 9.51E-03 | 33.3 | LYM254 | 1247 1.1 | G | 0.77 3 | 3.00E-06 | 72.9 |
| LYM113 | 12444. 5 | F | 5.95 7 | 1.07E-02 | 17.8 | LYM99 | 1224 4.2 | G | 0.56 9 | 1.23E-03 | 27.3 |
| LYM 102 | 12221. 2 | F | 5.89 2 | 1.12E-02 | 16.5 | LYM206 | 1260 1.3 | G | 0.63 | 1.78E-03 | 40.9 |
| LYM137 | 12152. 1 | F | 5.94 9 | 1.63E-02 | 17.6 | LYM 170 | 1245 2.3 | G | 1.02 4 | 2.21E-03 | 129 |
| LYM119 | 12461. 4 | F | 5.79 3 | 2.27E-02 | 14.5 | LYM 176 | 1238 4.1 | G | 0.67 5 | 2.30E-03 | 51 |
| LYM 130 | 12332. 2 | F | 5.63 8 | 4.49E-02 | 11.5 | LYM250 | 1261 3.2 | G | 0.88 6 | 2.49E-03 | 98.2 |
| LYM113 | 12443. 1 | F | 5.68 1 | 8.19E-02 | 12.3 | LYM42 | 1199 2.2 | G | 0.68 8 | 4.52E-03 | 53.8 |
| CONTR OL | - - | F | 5.05 8 | - - | 0 | LYM172 | 1230 1.2 | G | 0.55 5 | 4.63E-03 | 24.2 |
| LYM 102 | 12222. 1 | G | 0.64 1 | 5.60E-03 | 61.2 | LYM236 | 1259 4.3 | G | 0.92 3 | 4.63E-03 | 106.6 |
| LYM138 | 12561. 3 | G | 0.56 3 | 7.37E-03 | 41.4 | LYM 162 | 1223 1.1 | G | 0.70 4 | 5.43E-03 | 57.5 |
| LYM 130 | 12332. 2 | G | 0.73 3 | 8.67E-03 | 84.3 | LYM148 | 1217 1.2 | G | 0.74 4 | 6.77E-03 | 66.5 |
| LYM137 | 12152. 1 | G | 0.75 2 | 1.54E-02 | 88.9 | LYM236 | 1259 2.3 | G | 1.06 3 | 6.78E-03 | 137.7 |
| LYM141 | 12404. 2 | G | 0.53 8 | 2.41E-02 | 35.3 | LYM172 | 1230 4.1 | G | 0.62 3 | 7.31E-03 | 39.4 |
| LYM138 | 12566. 1 | G | 0.5 | 2.70E-02 | 25.7 | LYM206 | 1260 3.2 | G | 0.74 9 | 9.52E-03 | 67.5 |
| LYM 130 | 12334. 1 | G | 0.54 4 | 4.27E-02 | 36.6 | LYM 176 | 1238 5.1 | G | 0.99 7 | 1.09E-02 | 123.1 |
| LYM 143 | 12521. 2 | G | 0.47 8 | 7.49E-02 | 20.2 | LYM250 | 1261 4.2 | G | 0.57 2 | 1.38E-02 | 27.9 |
| LYM138 | 12562. 1 | G | 0.47 6 | 9.14E-02 | 19.7 | LYM89 | 1221 4.2 | G | 0.54 7 | 1.62E-02 | 22.4 |
| CONTR OL | - - | G | 0.39 8 | - - | 0 | LYM250 | 1261 3.4 | G | 0.68 1 | 1.94E-02 | 52.3 |
| LYM141 | 12404. 3 | H | 0.07 7 | 5.80E-05 | 96.5 | LYM 170 | 1245 2.4 | G | 0.76 | 2.00E-02 | 70 |
| LYM 102 | 12222. 1 | H | 0.06 5 | 8.00E-05 | 65.8 | LYM89 | 1221 1.2 | G | 0.71 4 | 2.05E-02 | 59.8 |
| LYM137 | 12152. 1 | H | 0.07 3 | 8.50E-05 | 85.8 | LYM140 | 1226 1.4 | G | 0.70 1 | 2.10E-02 | 56.9 |
| LYM 130 | 12332. 2 | H | 0.06 8 | 9.70E-05 | 73.7 | LYM170 | 1245 3.2 | G | 0.64 2 | 2.18E-02 | 43.5 |
| LYM137 | 12153. 1 | H | 0.06 6 | 1.26E-03 | 66.9 | LYM 140 | 1226 2.2 | G | 0.56 9 | 2.43E-02 | 27.3 |
| LYM 138 | 12561. 3 | H | 0.05 4 | 1.01E-02 | 37.1 | LYM 170 | 1245 3.3 | G | 0.90 9 | 2.78E-02 | 103.3 |
| LYM141 | 12404. 2 | H | 0.05 3 | 1.35E-02 | 34.4 | LYM236 | 1259 1.1 | G | 0.87 4 | 2.83E-02 | 95.5 |
| LYM 102 | 12221. 2 | H | 0.05 8 | 1.40E-02 | 46.1 | LYM89 | 1221 4.4 | G | 0.53 7 | 2.84E-02 | 20.2 |
| LYM 130 | 12334. 1 | H | 0.05 2 | 3.72E-02 | 30.7 | LYM99 | 1224 3.1 | G | 0.62 3 | 3.48E-02 | 39.3 |
| LYM138 | 12562. 1 | H | 0.05 | 4.54E-02 | 26 | LYM89 | 1221 4.3 | G | 0.63 6 | 4.21E-02 | 42.2 |
| LYM138 | 12566. 1 | H | 0.04 8 | 7.16E-02 | 22.8 | LYM 176 | 1238 4.2 | G | 0.59 1 | 4.33E-02 | 32.1 |
| LYM100 | 12133. 3 | H | 0.04 9 | 7.66E-02 | 25.2 | LYM206 | 1260. 3.3 | G | 0.58 5 | 4.64E-02 | 31 |
| LYM106 | 12144. 3 | H | 0.04 9 | 8.55E-02 | 24 | LYM 140 | 1226 1.2 | G | 0.51 | 5.15E-02 | 14 |
| CONTR OL | | H | 0.03 9 | - - | 0 | LYM176 | 1238 2.2 | G | 0.58 5 | 5.38E-02 | 30.8 |
| LYM 153 | 12321. 2 | A | 0.01 2 | 9.21E-03 | 102.4 | LYM42 | 1199 2.1 | G | 0.64 1 | 5.45E-02 | 43.4 |
| LYM152 | 12376. 1 | A | 0.01 1 | 4.22E-02 | 75.2 | LYM 148 | 1217 3.5 | G | 0.67 3 | 5.74E-02 | 50.6 |
| LYM148 | 12174. 1 | A | 0.00 8 | 5.19E-02 | 28.7 | LYM89 | 1221 4.1 | G | 0.63 1 | 5.75E-02 | 41.2 |
| LYM 140 | 12262. 2 | A | 0.00 8 | 9.10E-02 | 23.8 | LYM206 | 1260 2.1 | G | 0.73 2 | 5.75E-02 | 63.6 |
| CONTR OL | - - | A | 0.00 6 | - - | 0 | LYM254 | 1247 3.1 | G | 0.56 8 | 5.82E-02 | 27 |
| LYM153 | 12321. 2 | B | 0.24 5 | 2.61E-03 | 69.9 | LYM3 | 1204 1.2 | G | 0.66 5 | 6.39E-02 | 48.8 |
| LYM152 | 12376. 1 | B | 0.20 3 | 8.19E-02 | 40.9 | LYM42 | 1199 3.1 | G | 0.52 8 | 6.87E-02 | 18.2 |
| CONTR OL | - - | B | 0.14 4 | - - | 0 | LYM172 | 1230 1.3 | G | 0.54 9 | 8.21E-02 | 22.8 |
| LYM152 | 12376. 1 | C | 1.18 4 | 0.00E +00 | 138.6 | LYM176 | 1238 1.3 | G | 0.56 3 | 8.39E-02 | 26 |
| LYM153 | 12321. 2 | C | 1.04 1 | 1.30E-05 | 109.9 | LYM290 | 1250 4.1 | G | 0.65 9 | 8.59E-02 | 47.4 |
| LYM170 | 12452. 3 | C | 0.84 | 2.70E-05 | 69.4 | CONTR OL | - - | G | 0.44 7 | - - | 0 |
| LYM149 | 12344. 2 | C | 0.82 9 | 5.50E-05 | 67.1 | LYM 170 | 1245 2.3 | H | 0.09 9 | 0.00E +00 | 113.2 |
| LYM172 | 12301. 2 | C | 0.90 5 | 6.80E-05 | 82.3 | LYM236 | 1259 2.3 | H | 0.10 9 | 0.00E +00 | 135.8 |
| LYM174 | 12412. 1 | C | 0.80 9 | 9.50E-05 | 63.2 | LYM250 | 1261 3.2 | H | 0.08 8 | 0.00E +00 | 90.5 |
| LYM153 | 12322. 1 | C | 0.87 7 | 1.92E-04 | 76.8 | LYM236 | 1259 4.3 | H | 0.08 8 | 1.00E-06 | 89 |
| LYM172 | 12301. 3 | C | 0.86 | 2.07E-04 | 73.3 | LYM254 | 1247 1.1 | H | 0.07 7 | 1.00E-06 | 64.9 |
| LYM 176 | 12381. 3 | C | 0.78 1 | 3.32E-04 | 57.5 | LYM 176 | 1238 5.1 | H | 0.09 6 | 6.00E-06 | 106.2 |
| LYM170 | 12454. 2 | C | 0.76 6 | 5.34E-04 | 54.4 | LYM206 | 1260 3.2 | H | 0.07 8 | 1.20E-05 | 68.2 |
| LYM174 | 12411. 3 | C | 0.74 8 | 1.82E-03 | 50.8 | LYM170 | 1245 3.3 | H | 0.09 2 | 7.00E-05 | 97.6 |
| LYM 162 | 12234. 4 | C | 0.74 3 | 2.64E-03 | 49.7 | LYM 170 | 1245 2.4 | H | 0.07 8 | 8.10E-05 | 67.9 |
| LYM289 | 12493. 2 | C | 0.74 7 | 3.09E-03 | 50.6 | LYM236 | 1259 1.1 | H | 0.08 7 | 1.34E-04 | 87.2 |
| LYM174 | 12411. 2 | C | 0.78 5 | 4.17E-03 | 58.3 | LYM162 | 1223 1.1 | H | 0.07 1 | 2.54E-04 | 52.4 |
| LYM111 | 12254. 3 | C | 0.76 4 | 4.75E-03 | 54 | LYM89 | 1221 1.2 | H | 0.07 1 | 5.66E-04 | 52 |
| LYM 148 | 12174. 1 | C | 0.72 5 | 5.96E-03 | 46.1 | LYM42 | 1199 2.2 | H | 0.06 6 | 5.77E-04 | 43 |
| LYM 162 | 12234. 3 | C | 0.73 3 | 1.17E-02 | 47.7 | LYM206 | 1260 1.3 | H | 0.06 5 | 6.02E-04 | 39.1 |
| LYM148 | 12171. 2 | C | 0.68 4 | 2.29E-02 | 37.8 | LYM148 | 1217 1.2 | H | 0.06 9 | 9.52E-04 | 49.3 |
| LYM 105 | 12293. 1 | C | 0.65 8 | 2.67E-02 | 32.6 | LYM 176 | 1238 4.1 | H | 0.06 6 | 1.86E-03 | 42.7 |
| LYM 162 | 12231. 2 | C | 0.68 | 2.78E-02 | 37.1 | LYM206 | 1260 2.1 | H | 0.07 3 | 2.16E-03 | 58.3 |
| LYM174 | 12414. 3 | C | 0.65 | 5.52E-02 | 30.9 | LYM172 | 1230 4.1 | H | 0.06 3 | 2.61E-03 | 36.2 |
| LYM 170 | 12452. 4 | C | 0.63 6 | 5.67E-02 | 28.2 | LYM250 | 1261 3.4 | H | 0.06 6 | 2.68E-03 | 43 |
| LYM153 | 12323. 2 | C | 0.68 4 | 5.84E-02 | 37.9 | LYM 170 | 1245 3.2 | H | 0.06 4 | 3.33E-03 | 38.6 |
| LYM 148 | 12173. 5 | C | 0.65 2 | 6.99E-02 | 31.4 | LYM 140 | 1226 1.4 | H | 0.06 6 | 4.14E-03 | 41.3 |
| LYM290 | 12502. 4 | C | 0.63 5 | 7.97E-02 | 28.1 | LYM99 | 1224 3.1 | H | 0.06 3 | 6.82E-03 | 35.1 |
| LYM254 | 12474. 4 | C | 0.61 7 | 8.58E-02 | 24.4 | LYM99 | 1224 4.2 | H | 0.05 9 | 7.05E-03 | 27.3 |
| LYM148 | 12173. 1 | C | 0.63 2 | 9.21E-02 | 27.3 | LYM89 | 1221 4.1 | H | 0.06 5 | 7.10E-03 | 40.5 |
| CONTR OL | - - | C | 0.49 6 | - - | 0 | LYM206 | 1260 3.3 | H | 0.06 1 | 8.74E-03 | 31.6 |
| LYM152 | 12376. 1 | D | 10.1 84 | 0.00E +00 | 134.7 | LYM250 | 1261 4.2 | H | 0.06 | 9.60E-03 | 29.4 |
| LYM170 | 12452. 3 | D | 7.08 | 7.80E-05 | 63.2 | LYM42 | 1199 2.1 | H | 0.06 4 | 1.49E-02 | 37.4 |
| LYM176 | 12381. 3 | D | 6.67 | 2.52E-04 | 53.7 | LYM254 | 1247 3.1 | H | 0.06 | 1.84E-02 | 28.6 |
| LYM149 | 12344. 2 | D | 7.18 2 | 2.74E-04 | 65.5 | LYM290 | 1250 4.1 | H | 0.06 5 | 2.04E-02 | 39.9 |
| LYM174 | 12412. 1 | D | 6.75 1 | 3.53E-04 | 55.6 | LYM89 | 1221 4.3 | H | 0.06 1 | 3.34E-02 | 30.8 |
| LYM 170 | 12454. 2 | D | 6.62 6 | 3.98E-04 | 52.7 | LYM 172 | 1230 1.2 | H | 0.05 7 | 3.68E-02 | 22.3 |
| LYM174 | 12411. 3 | D | 6.49 7 | 3.90E-03 | 49.7 | LYM42 | 1199 3.1 | H | 0.05 8 | 3.99E-02 | 24.2 |
| LYM289 | 12493. 2 | D | 6.46 9 | 8.75E-03 | 49.1 | LYM89 | 1221 4.4 | H | 0.05 7 | 4.67E-02 | 22.5 |
| LYM162 | 12234. 4 | D | 6.25 3 | 1.01E-02 | 44.1 | LYM148 | 1217 3.5 | H | 0.06 2 | 4.81E-02 | 33.2 |
| LYM 105 | 12293. 1 | D | 5.67 | 1.26E-02 | 30.7 | LYM 140 | 1226 1.2 | H | 0.05 6 | 5.27E-02 | 19.9 |
| LYM 172 | 12301. 3 | D | 7.41 2 | 1.63E-02 | 70.8 | LYM236 | 1259 2.4 | H | 0.06 1 | 6.49E-02 | 30.4 |
| LYM 172 | 12301. 2 | D | 7.67 4 | 1.83E-02 | 76.9 | LYM250 | 1261 4.1 | H | 0.05 5 | 9.32E-02 | 19.6 |
| LYM 153 | 12321. 2 | D | 8.90 1 | 1.85E-02 | 105.2 | LYM 176 | 1238 1.3 | H | 0.05 6 | 9.61E-02 | 20.1 |
| LYM 153 | 12322. 1 | D | 7.58 | 2.24E-02 | 74.7 | LYM290 | 1250 1.3 | H | 0.06 3 | 9.74E-02 | 35.7 |
| LYM148 | 12174. 1 | D | 6.39 | 2.38E-02 | 47.3 | CONTR OL | - | H | 0.04 6 | - | 0 |
| LYM254 | 12474. 4 | D | 5.37 7 | 2.95E-02 | 23.9 | LYM90 | 1239 2.1 | A | 0.00 5 | 1.00E-06 | 108.9 |
| LYM111 | 12254. 3 | D | 6.64 2 | 3.13E-02 | 53.1 | LYM213 | 1284 2.9 | A | 0.00 4 | 5.60E-05 | 48.5 |
| LYM 170 | 12452. 4 | D | 5.43 8 | 5.31E-02 | 25.3 | LYM 107 | 1263 3.4 | A | 0.00 6 | 1.12E-04 | 153.5 |
| LYM 148 | 12171. 2 | D | 6.00 8 | 5.50E-02 | 38.5 | LYM157 | 1334 1.1 | A | 0.00 6 | 1.62E-03 | 130.7 |
| LYM174 | 12411. 2 | D | 6.70 3 | 6.07E-02 | 54.5 | LYM90 | 1239 4.2 | A | 0.00 5 | 2.24E-03 | 78.2 |
| LYM162 | 12231. 2 | D | 5.94 8 | 7.11E-02 | 37.1 | LYM 107 | 1263 1.4 | A | 0.00 5 | 9.45E-03 | 104 |
| LYM 162 | 12234. 3 | D | 6.31 9 | 8.38E-02 | 45.6 | LYM180 | 1344 1.7 | A | 0.00 4 | 1.15E-02 | 58.4 |
| LYM 172 | 12304. 1 | D | 5.12 6 | 9.20E-02 | 18.2 | LYM90 | 1239 5.3 | A | 0.00 5 | 1.27E-02 | 115.8 |
| CONTR OL | - | D | 4.33 9 | - | 0 | LYM248 | 1350 1.6 | A | 0.00 4 | 1.40E-02 | 74.3 |
| LYM176 | 12381. 3 | E | 0.57 | 2.35E-02 | 42.8 | LYM52 | 1289 5.7 | A | 0.00 5 | 1.64E-02 | 87.1 |
| LYM 172 | 12301. 2 | E | 0.56 3 | 3.24E-02 | 41.1 | LYM52 | 1289 4.6 | A | 0.00 5 | 2.41E-02 | 93.1 |
| LYM 174 | 12412. 1 | E | 0.56 | 3.53E-02 | 40.3 | LYM213 | 1284 1.8 | A | 0.00 7 | 2.71E-02 | 161.4 |
| LYM153 | 12322. 1 | E | 0.54 7 | 5.02E-02 | 37.1 | LYM68 | 1194 2.2 | A | 0.00 4 | 3.03E-02 | 77.2 |
| LYM254 | 12474. 4 | E | 0.54 5 | 5.08E-02 | 36.5 | LYM 157 | 1334 1.3 | A | 0.00 7 | 3.06E-02 | 193.1 |
| LYM111 | 12254. 3 | E | 0.54 2 | 6.45E-02 | 35.8 | LYM 180 | 1344 1.5 | A | 0.00 3 | 3.17E-02 | 20.8 |
| LYM 170 | 12454. 2 | E | 0.53 7 | 6.57E-02 | 34.5 | LYM52 | 1289 1.8 | A | 0.00 7 | 3.60E-02 | 183.2 |
| LYM 172 | 12301. 3 | E | 0.54 3 | 6.79E-02 | 36.1 | LYM68 | 1194 3.5 | A | 0.00 4 | 3.69E-02 | 46.5 |
| LYM148 | 12174. 1 | E | 0.53 5 | 6.85E-02 | 34 | LYM213 | 1284 1.6 | A | 0.00 8 | 3.70E-02 | 235.6 |
| LYM152 | 12376. 1 | E | 0.54 3 | 7.05E-02 | 36.1 | LYM117 | 1362 2.6 | A | 0.00 5 | 4.17E-02 | 85.1 |
| LYM174 | 12411. 2 | E | 0.53 5 | 7.56E-02 | 34.1 | LYM117 | 1362 1.8 | A | 0.00 4 | 4.82E-02 | 57.4 |
| LYM 153 | 12321. 2 | E | 0.54 3 | 7.70E-02 | 36.1 | LYM52 | 1289 4.8 | A | 0.00 8 | 5.80E-02 | 217.8 |
| LYM 170 | 12452. 3 | E | 0.53 | 8.57E-02 | 32.8 | LYM68 | 1194 1.3 | A | 0.00 4 | 5.93E-02 | 41.6 |
| LYM148 | 12171. 2 | E | 0.52 3 | 9.20E-02 | 31.1 | LYM248 | 1350 2.7 | A | 0.00 4 | 6.41E-02 | 65.3 |
| CONTR OL | - | E | 0.39 9 | - | 0 | LYM117 | 1362 4.4 | A | 0.00 4 | 7.16E-02 | 72.3 |
| LYM149 | 12344. 2 | F | 6.80 1 | 1.57E-03 | 33.2 | LYM52 | 1289 4.7 | A | 0.00 6 | 7.32E-02 | 152.5 |
| LYM152 | 12376. 1 | F | 7.02 3 | 1.62E-03 | 37.5 | LYM157 | 1334 1.4 | A | 0.00 5 | 7.5 1E-02 | 106.9 |
| LYM148 | 12171. 2 | F | 6.66 3 | 2.42E-03 | 30.5 | LYM213 | 1284 1.7 | A | 0.00 5 | 8.38E-02 | 114.9 |
| LYM254 | 12474. 4 | F | 6.67 1 | 2.45E-03 | 30.7 | LYM 180 | 1344 3.7 | A | 0.00 3 | 8.44E-02 | 28.7 |
| LYM174 | 12412. 1 | F | 6.79 1 | 2.67E-03 | 33 | LYM180 | 1344 2.6 | A | 0.00 4 | 9.89E-02 | 50.5 |
| LYM 170 | 12454. 2 | F | 6.64 1 | 2.92E-03 | 30.1 | CONTR OL | - | A | 0.00 3 | - | 0 |
| LYM170 | 12452. 3 | F | 6.74 2 | 3.91E-03 | 32 | LYM90 | 1239 2.1 | B | 0.15 2 | 1.06E-03 | 81.1 |
| LYM 172 | 12301. 3 | F | 6.70 6 | 5.41E- 03 | 31.3 | LYM 107 | 1263 1.4 | B | 0.13 6 | 2.69E-03 | 61.9 |
| LYM148 | 12174. 1 | F | 6.44 9 | 5.65E-03 | 26.3 | LYM90 | 1239 5.3 | B | 0.13 5 | 5.72E-03 | 60.8 |
| LYM153 | 12321. 2 | F | 6.67 8 | 5.68E- 03 | 30.8 | LYM157 | 1334 1.1 | B | 0.14 6 | 1.69E-02 | 73.9 |
| LYM153 | 12322. 1 | F | 6.44 3 | 5.80E-03 | 26.2 | LYM213 | 1284 1.6 | B | 0.20 3 | 1.93E-02 | 141.6 |
| LYM176 | 12381. 3 | F | 6.44 2 | 5.82E-03 | 26.2 | LYM117 | 1362 1.8 | B | 0.10 5 | 2.79E-02 | 24.6 |
| LYM289 | 12493. 2 | F | 6.37 | 7.79E-03 | 24.8 | LYM107 | 1263 3.4 | B | 0.14 4 | 3.61E-02 | 71.1 |
| LYM 172 | 12301. 2 | F | 6.40 1 | 8.13E-03 | 25.4 | LYM68 | 1194 2.2 | B | 0.11 1 | 4.71E-02 | 31.8 |
| LYM174 | 12414. 3 | F | 6.25 9 | 1.53E-02 | 22.6 | LYM52 | 1289 4.6 | B | 0.13 1 | 4.73E-02 | 55.6 |
| LYM174 | 12411. 2 | F | 6.33 8 | 1.62E-02 | 24.1 | LYM52 | 1289 4.8 | B | 0.18 4 | 4.84E-02 | 118.7 |
| LYM148 | 12173. 5 | F | 6.26 8 | 1.64E-02 | 22.7 | LYM157 | 1334 1.3 | B | 0.16 5 | 5.02E-02 | 96.8 |
| LYM140 | 12262. 3 | F | 6.2 | 1.65E-02 | 21.4 | LYM52 | 1289 4.7 | B | 0.16 4 | 5.10E-02 | 94.7 |
| LYM111 | 12254. 3 | F | 6.35 2 | 1.66E-02 | 24.4 | LYM52 | 1289 1.8 | B | 0.14 7 | 5.39E-02 | 75.3 |
| LYM 176 | 12384. 1 | F | 6.08 7 | 2.67E-02 | 19.2 | LYM90 | 1239 4.2 | B | 0.11 | 5.40E-02 | 30.6 |
| LYM170 | 12453. 2 | F | 6.09 8 | 3.46E-02 | 19.4 | LYM52 | 1289 5.7 | B | 0.15 | 5.54E-02 | 78.1 |
| LYM111 | 12252. 2 | F | 6.02 9 | 3.90E-02 | 18.1 | LYM248 | 1350 2.7 | B | 0.12 | 6.5 8E-02 | 43.3 |
| LYM162 | 12234. 4 | F | 5.98 | 4.10E-02 | 17.1 | LYM213 | 1284 2.9 | B | 0.09 9 | 6.68E-02 | 17.3 |
| LYM 153 | 12323. 2 | F | 6.03 5 | 4.13E-02 | 18.2 | LYM117 | 1362 2.6 | B | 0.13 6 | 6.92E-02 | 61.7 |
| LYM 162 | 12234. 3 | F | 6.27 6 | 4.14E-02 | 22.9 | LYM213 | 1284 1.8 | B | 0.18 6 | 9.20E-02 | 121.5 |
| LYM 170 | 12452. 4 | F | 5.96 | 4.49E-02 | 16.7 | CONTR OL | - | B | 0.08 4 | - | 0 |
| LYM149 | 12343. 2 | F | 6.25 5 | 5.80E-02 | 22.5 | LYM 107 | 1263 3.4 | C | 0.99 1 | 0.00E +00 | 94.3 |
| LYM162 | 12231. 2 | F | 6.01 9 | 6.32E-02 | 17.9 | LYM 107 | 1263 1.4 | C | 0.94 4 | 0.00E+00 | 85.1 |
| LYM 152 | 12373. 2 | F | 5.84 6 | 7.37E-02 | 14.5 | LYM52 | 1289 4.6 | C | 1.01 2 | 0.00E +00 | 98.3 |
| LYM289 | 12493. 6 | F | 5.78 7 | 9.53E-02 | 13.3 | LYM52 | 1289 1.8 | C | 0.92 8 | 0.00E +00 | 81.9 |
| CONTR OL | - - | F | 5.10 6 | - - | 0 | LYM68 | 1194 2.2 | C | 0.98 | 0.00E +00 | 92.1 |
| LYM152 | 12376. 1 | G | 0.99 3 | 4.96E-04 | 50.8 | LYM52 | 1289 4.8 | C | 0.95 4 | 1.00E-06 | 87 |
| LYM153 | 12321. 2 | G | 1.04 8 | 4.01E-03 | 59.2 | LYM213 | 1284 1.6 | C | 0.87 | 3.00E-06 | 70.5 |
| LYM 148 | 12174. 1 | G | 0.91 6 | 6.30E-03 | 39.2 | LYM90 | 1239 2.1 | C | 0.86 9 | 1.10E-05 | 70.4 |
| LYM290 | 12502. 4 | G | 0.79 9 | 4.73E-02 | 21.3 | LYM157 | 1334 1.3 | C | 0.93 6 | 3.10E-05 | 83.5 |
| LYM172 | 12301. 2 | G | 0.86 2 | 5.63E-02 | 30.9 | LYM157 | 1334 1.1 | C | 0.82 3 | 1.04E-04 | 61.3 |
| LYM140 | 12262. 2 | G | 0.81 4 | 8.61E-02 | 23.6 | LYM90 | 1239 5.3 | C | 0.82 | 1.17E-04 | 60.6 |
| LYM174 | 12411. 3 | G | 0.86 7 | 9.28E-02 | 31.7 | LYM157 | 1334 1.4 | C | 0.81 2 | 1.51E-04 | 59.2 |
| CONTR OL | - | G | 0.65 8 | - | 0 | LYM90 | 1239 4.2 | C | 0.77 9 | 2.54E-04 | 52.8 |
| LYM152 | 12376. 1 | H | 0.10 3 | 1.39E-04 | 65.4 | LYM68 | 1194 1.4 | C | 0.78 8 | 3.36E-04 | 54.5 |
| LYM153 | 12321. 2 | H | 0.10 6 | 2.48E-04 | 69.7 | LYM248 | 1350 1.6 | C | 0.78 4 | 5.63E-04 | 53.7 |
| LYM172 | 12301. 2 | H | 0.08 8 | 1.64E-02 | 41 | LYM 107 | 1263 1.1 | C | 0.76 4 | 6.45E-04 | 49.7 |
| LYM 148 | 12174. 1 | H | 0.08 6 | 1.81E-02 | 38.1 | LYM117 | 1362 2.6 | C | 0.74 | 1.38E-03 | 45.1 |
| LYM174 | 12411. 3 | H | 0.08 5 | 4.51E-02 | 36.4 | LYM52 | 1289 5.7 | C | 0.72 4 | 2.87E-03 | 42 |
| CONTR OL | - | H | 0.06 3 | - | 0 | LYM52 | 1289 4.7 | C | 0.74 1 | 3.13E-03 | 45.2 |
| LYM 175 | 12651. 2 | A | 0.00 5 | 1.27E-03 | 85.3 | LYM90 | 1239 3.1 | C | 0.72 8 | 8.19E-03 | 42.6 |
| LYM107 | 12632. 1 | A | 0.00 5 | 7.98E-03 | 96.3 | LYM157 | 1334 2.4 | C | 0.71 | 1.13E-02 | 39.2 |
| LYM203 | 12663. 2 | A | 0.00 6 | 1.07E-02 | 104.6 | LYM 107 | 1263 1.2 | C | 0.67 1 | 2.27E-02 | 31.6 |
| LYM128 | 12642. 1 | A | 0.00 4 | 3.17E-02 | 53.2 | LYM213 | 1284 1.7 | C | 0.68 | 2.56E-02 | 33.4 |
| LYM128 | 12641. 3 | A | 0.00 4 | 5.95E-02 | 64.2 | LYM68 | 1194 2.3 | C | 0.67 9 | 3.65E-02 | 33 |
| LYM175 | 12654. 4 | A | 0.00 4 | 7.53E-02 | 47.7 | LYM90 | 1239 5.1 | C | 0.67 6 | 5.36E-02 | 32.6 |
| CONTR OL | - | A | 0.00 3 | - | 0 | LYM68 | 1194 1.3 | C | 0.64 4 | 5.48E-02 | 26.2 |
| LYM 107 | 12631. 1 | B | 0.09 2 | 9.39E-03 | 28.3 | LYM248 | 1350 3.5 | C | 0.64 7 | 6.20E-02 | 26.8 |
| LYM175 | 12654. 4 | B | 0.11 2 | 1.18E-02 | 55.8 | LYM117 | 1362 1.8 | C | 0.64 7 | 6.25E-02 | 26.8 |
| LYM203 | 12663. 2 | B | 0.12 2 | 1.66E-02 | 69.5 | LYM117 | 1362 4.7 | C | 0.63 6 | 7.06E-02 | 24.6 |
| LYM 107 | 12632. 1 | B | 0.12 5 | 2.43E-02 | 73.7 | LYM117 | 1362 3.9 | C | 0.62 9 | 9.82E-02 | 23.2 |
| LYM175 | 12651. 2 | B | 0.11 7 | 2.91E-02 | 62.4 | CONTR OL | | C | 0.51 | - | 0 |
| LYM128 | 12642. 1 | B | 0.09 6 | 3.18E-02 | 33.2 | LYM213 | 1284 1.6 | D | 7.63 8 | 4.50E-05 | 69.8 |
| LYM128 | 12641. 3 | B | 0.09 6 | 6.27E-02 | 33.4 | LYM117 | 1362 2.6 | D | 6.2 1 9 | 9.60E-05 | 38.3 |
| LYM203 | 12662. 2 | B | 0.15 | 7.88E-02 | 108.2 | LYM 107 | 1263 1.2 | D | 5.85 2 | 3.86E-04 | 30.1 |
| CONTR OL | - | B | 0.07 2 | - - | 0 | LYM68 | 1194 2.2 | D | 8.37 1 | 4.18E-04 | 86.1 |
| LYM128 | 12642. 1 | C | 0.59 | 4.98E-04 | 67 | LYM52 | 1289 1.8 | D | 8.04 8 | 4.29E-04 | 78.9 |
| LYM203 | 12663. 2 | C | 0.49 6 | 2.37E-02 | 40.5 | LYM90 | 1239 4.2 | D | 6.75 1 | 1.42E-03 | 50.1 |
| LYM203 | 12662. 2 | C | 0.52 9 | 5.42E-02 | 49.7 | LYM107 | 1263 1.1 | D | 6.53 1 | 2.91E-03 | 45.2 |
| LYM 128 | 12641. 3 | C | 0.44 7 | 7.46E-02 | 26.7 | LYM52 | 1289 5.7 | D | 6.18 3 | 5.76E-03 | 37.5 |
| LYM 107 | 12631. 2 | C | 0.48 2 | 8.17E-02 | 36.6 | LYM52 | 1289 4.6 | D | 8.57 2 | 6.41E-03 | 90.6 |
| CONTR OL | - | C | 0.35 3 | - | 0 | LYM107 | 1263 1.4 | D | 8.24 9 | 7.3 1E-03 | 83.4 |
| LYM128 | 12642. 1 | D | 5.09 8 | 4.34E-03 | 62 | LYM90 | 1239 2.1 | D | 7.58 6 | 1.12E-02 | 68.6 |
| LYM128 | 12641. 3 | D | 4.17 6 | 1.25E-02 | 32.7 | LYM52 | 1289 4.8 | D | 8.22 8 | 1.31E-02 | 82.9 |
| LYM203 | 12663. 2 | D | 4.26 6 | 7.47E-02 | 35.6 | LYM 107 | 1263 3.4 | D | 8.64 4 | 1.42E-02 | 92.2 |
| CONTR OL | - | D | 3.14 6 | - | 0 | LYM90 | 1239 5.3 | D | 7.12 | 1.67E-02 | 58.3 |
| LYM128 | 12641. 3 | E | 0.44 7 | 9.19E-04 | 33 | LYM157 | 1334 1.4 | D | 7.11 4 | 1.98E-02 | 58.2 |
| LYM 128 | 12641. 5 | E | 0.43 3 | 3.42E-03 | 28.7 | LYM68 | 1194 1.4 | D | 6.66 3 | 2.04E-02 | 48.1 |
| LYM 128 | 12642. 1 | E | 0.44 | 4.86E-03 | 30.8 | LYM117 | 1362 4.7 | D | 5.48 2 | 2.19E-02 | 21.9 |
| LYM203 | 12663. 2 | E | 0.41 3 | 2.97E-02 | 22.8 | LYM68 | 1194 1.3 | D | 5.57 8 | 2.27E-02 | 24 |
| LYM203 | 12662. 2 | E | 0.40 4 | 3.66E-02 | 20.3 | LYM 157 | 1334 1.1 | D | 7.02 6 | 2.67E-02 | 56.2 |
| LYM128 | 12641. 1 | E | 0.41 4 | 8.08E-02 | 23.3 | LYM248 | 1350 1.6 | D | 6.80 9 | 3.00E-02 | 51.4 |
| CONTR OL | - | E | 0.33 6 | - | 0 | LYM117 | 1362 1.8 | D | 5.70 3 | 4.86E-02 | 26.8 |
| LYM 128 | 12642. 1 | F | 6.01 2 | 8.60E-05 | 45.8 | LYM52 | 1289 4.7 | D | 6.31 4 | 5.92E-02 | 40.4 |
| LYM 128 | 12641. 3 | F | 5.47 9 | 1.32E-04 | 32.9 | LYM157 | 1334 1.3 | D | 8.09 2 | 7.45E-02 | 79.9 |
| LYM203 | 12663. 2 | F | 5.07 | 7.26E-03 | 23 | LYM213 | 1284 1.7 | D | 6.06 2 | 8.34E-02 | 34.8 |
| LYM203 | 12664. 1 | F | 4.69 9 | 7.69E-03 | 14 | LYM 180 | 1344 3.7 | D | 5.25 9 | 8.41E-02 | 16.9 |
| LYM203 | 12662. 2 | F | 5.04 1 | 1.20E-02 | 22.3 | LYM 157 | 1334 2.4 | D | 6.14 3 | 8.49E-02 | 36.6 |
| LYM128 | 12641. 5 | F | 4.68 6 | 1.21E-02 | 13.7 | CONTR OL | - | D | 4.49 8 | - | 0 |
| LYM 175 | 12651. 4 | F | 4.80 2 | 1.96E-02 | 16.5 | LYM68 | 1194 1.4 | E | 0.61 6 | 1.55E-03 | 41.7 |
| LYM 107 | 12632. 3 | F | 4.57 3 | 3.01E-02 | 10.9 | LYM107 | 1263 1.1 | E | 0.59 8 | 3.20E-03 | 37.5 |
| LYM 107 | 12631. 2 | F | 5.32 5 | 7.35E-02 | 29.1 | LYM68 | 1194 2.2 | E | 0.59 3 | 4.66E-03 | 36.4 |
| LYM 128 | 12641. 1 | F | 5.15 1 | 7.83E-02 | 24.9 | LYM52 | 1289 4.6 | E | 0.59 6 | 4.93E-03 | 37.2 |
| CONTR OL | - | F | 4.12 3 | - | 0 | LYM248 | 1350 3.5 | E | 0.58 8 | 5.03E-03 | 35.2 |
| LYM 107 | 12632. 1 | G | 0.52 5 | 1.77E-03 | 47.2 | LYM90 | 1239 3.1 | E | 0.58 7 | 5.49E-03 | 34.9 |
| LYM128 | 12642. 1 | G | 0.56 8 | 2.05E-03 | 59.3 | LYM52 | 1289 5.7 | E | 0.58 6 | 5.79E-03 | 34.7 |
| LYM128 | 12641. 3 | G | 0.51 9 | 4.67E-03 | 45.3 | LYM68 | 1194 1.3 | E | 0.58 6 | 6.68E-03 | 34.7 |
| LYM203 | 12663. 2 | G | 0.58 3 | 1.40E-02 | 63.4 | LYM52 | 1289 4.8 | E | 0.57 9 | 9.24E-03 | 33.1 |
| LYM 107 | 12631. 1 | G | 0.44 7 | 4.06E-02 | 25.4 | LYM90 | 1239 4.2 | E | 0.56 9 | 1.49E-02 | 30.9 |
| LYM 175 | 12651. 2 | G | 0.56 8 | 5.89E-02 | 59.1 | LYM157 | 1334 1.3 | E | 0.55 4 | 2.88E-02 | 27.3 |
| LYM203 | 12662. 2 | G | 0.60 9 | 5.96E-02 | 70.7 | LYM213 | 1284 1.6 | E | 0.55 3 | 2.96E-02 | 27.1 |
| CONTR OL | - | G | 0.35 7 | - | 0 | LYM248 | 1350 1.6 | E | 0.55 8 | 3.17E-02 | 28.3 |
| LYM128 | 12642. 1 | H | 0.05 7 | 4.93E-03 | 57.5 | LYM 107 | 1263 3.4 | E | 0.54 6 | 4.23E-02 | 25.5 |
| LYM203 | 12663. 2 | H | 0.05 6 | 9.28E-03 | 55.3 | LYM157 | 1334 1.1 | E | 0.54 5 | 4.29E-02 | 25.2 |
| LYM203 | 12662. 2 | H | 0.06 | 1.20E-02 | 65 | LYM 180 | 1344 1.7 | E | 0.54 1 | 5.32E-02 | 24.4 |
| LYM128 | 12641. 3 | H | 0.05 1 | 2.49E-02 | 40.3 | LYM157 | 1334 1.4 | E | 0.54 | 5.93E-02 | 24.2 |
| LYM 175 | 12651. 2 | H | 0.05 6 | 2.52E-02 | 55.8 | LYM 107 | 1263 1.2 | E | 0.53 9 | 6.08E-02 | 24 |
| LYM 107 | 12632. 1 | H | 0.05 1 | 2.94E-02 | 40.3 | LYM90 | 1239 2.1 | E | 0.53 3 | 8.04E-02 | 22.5 |
| CONTR OL | - | H | 0.03 6 | - | 0 | LYM68 | 1194 2.3 | E | 0.52 6 | 9.63E-02 | 20.9 |
| LYM224 | 13435. 3 | A | 0.00 7 | 0.00E +00 | 90.7 | CONTR OL | - | E | 0.43 5 | - | 0 |
| LYM217 | 13182. 1 | A | 0.00 9 | 9.40E-05 | 160 | LYM107 | 1263 3.4 | F | 7.25 1 | 0.00E +00 | 30.4 |
| LYM23 | 12783. 5 | A | 0.00 7 | 6.40E-04 | 99 | LYM68 | 1194 2.2 | F | 7.60 7 | 0.00E +00 | 36.8 |
| LYM164 | 12813. 5 | A | 0.01 | 1.20E-03 | 169 | LYM90 | 1239 3.1 | F | 7.20 6 | 0.00E +00 | 29.6 |
| LYM164 | 12813. 8 | A | 0.00 9 | 2.03E-03 | 143.6 | LYM52 | 1289 4.8 | F | 6.93 2 | 3.00E-06 | 24.7 |
| LYM251 | 13073. 8 | A | 0.00 7 | 3.58E-03 | 84.6 | LYM157 | 1334 1.3 | F | 6.85 4 | 1.40E-05 | 23.3 |
| LYM122 | 13712. 3 | A | 0.00 5 | 3.83E-03 | 45.5 | LYM90 | 1239 4.2 | F | 6.94 1 | 1.40E-05 | 24.9 |
| LYM274 | 13414. 2 | A | 0.00 9 | 4.46E-03 | 138.1 | LYM68 | 1194 1.3 | F | 7.08 2 | 7.00E-05 | 27.4 |
| LYM217 | 13181. 11 | A | 0.00 9 | 4.68E-03 | 136.7 | LYM180 | 1344 1.5 | F | 6.59 6 | 8.70E-05 | 18.6 |
| LYM79 | 13132. 2 | A | 0.01 | 6.39E-03 | 166.9 | LYM157 | 1334 1.4 | F | 6.80 5 | 1.05E-04 | 22.4 |
| LYM273 | 13721. 3 | A | 0.01 1 | 7.17E-03 | 201.2 | LYM248 | 1350 3.5 | F | 7.13 | 1.31E-04 | 28.3 |
| LYM273 | 13721. 1 | A | 0.00 7 | 8.18E-03 | 81.8 | LYM52 | 1289 5.7 | F | 6.93 6 | 2.60E-04 | 24.8 |
| LYM249 | 13631. 1 | A | 0.00 6 | 8.20E-03 | 64.7 | LYM90 | 1239 5.3 | F | 6.74 7 | 7.99E-04 | 21.4 |
| LYM23 | 12783. 7 | A | 0.01 | 8.56E-03 | 162.1 | LYM107 | 1263 1.1 | F | 6.89 | 9.89E-04 | 23.9 |
| LYM122 | 13713. 2 | A | 0.00 7 | 8.63E-03 | 80.4 | LYM180 | 1344 3.7 | F | 6.93 4 | 1.01E-03 | 24.7 |
| LYM249 | 13633. 1 | A | 0.01 | 9.46E-03 | 172.4 | LYM52 | 1289 1.8 | F | 6.48 6 | 1.57E-03 | 16.7 |
| LYM193 | 13484. 3 | A | 0.00 8 | 9.51E-03 | 127.8 | LYM248 | 1350 1.6 | F | 7.25 6 | 2.13E-03 | 30.5 |
| LYM245 | 13061. 6 | A | 0.00 7 | 1.45E-02 | 82.5 | LYM213 | 1284 1.6 | F | 6.73 6 | 3.86E-03 | 21.2 |
| LYM273 | 13722. 4 | A | 0.00 7 | 1.60E-02 | 90.1 | LYM157 | 1334 1.1 | F | 6.31 2 | 3.94E-03 | 13.6 |
| LYM251 | 13072. 8 | A | 0.00 9 | 1.70E-02 | 138.1 | LYM90 | 1239 5.1 | F | 6.79 6 | 4.94E-03 | 22.2 |
| LYM217 | 13181. 7 | A | 0.00 6 | 1.82E-02 | 53 | LYM107 | 1263 1.2 | F | 6.84 6 | 6.89E-03 | 23.2 |
| LYM23 | 12782. 6 | A | 0.00 8 | 2.17E-02 | 114.8 | LYM248 | 1350 2.7 | F | 6.67 3 | 6.91E-03 | 20 |
| LYM122 | 13714. 4 | A | 0.01 | 2.25E-02 | 166.2 | LYM 157 | 1334 2.4 | F | 6.44 2 | 7.43E-03 | 15.9 |
| LYM245 | 13062. 5 | A | 0.00 8 | 2.38E-02 | 131.2 | LYM107 | 1263 1.4 | F | 6.95 9 | 8.62E-03 | 25.2 |
| LYM79 | 13133. 1 | A | 0.00 6 | 3.11E-02 | 62.6 | LYM52 | 1289 4.6 | F | 7.32 8 | 1.06E-02 | 31.8 |
| LYM23 | 12784. 6 | A | 0.00 7 | 4.14E-02 | 95.5 | LYM68 | 1194 1.4 | F | 6.93 7 | 1.08E-02 | 24.8 |
| LYM251 | 13073. 7 | A | 0.00 8 | 4.14E-02 | 126.4 | LYM68 | 1194 2.3 | F | 6.77 1 | 1.09E-02 | 21.8 |
| LYM273 | 13723. 1 | A | 0.00 8 | 4.80E-02 | 123 | LYM117 | 1362 1.8 | F | 6.43 1 | 1.47E-02 | 15.7 |
| LYM224 | 13434. 2 | A | 0.00 6 | 4.97E-02 | 59.9 | LYM 180 | 1344 2.6 | F | 6.00 3 | 1.85E-02 | 8 |
| LYM251 | 13074. 6 | A | 0.00 5 | 5.26E-02 | 49.6 | LYM213 | 1284 2.8 | F | 6.00 1 | 1.91E-02 | 7.9 |
| LYM274 | 13413. 4 | A | 0.00 6 | 5.58E-02 | 76.3 | LYM 180 | 1344 1.7 | F | 6.81 4 | 2.38E-02 | 22.6 |
| LYM273 | 13722. 3 | A | 0.00 7 | 6.00E-02 | 80.4 | LYM90 | 1239 2.1 | F | 6.51 8 | 3.57E-02 | 17.2 |
| LYM224 | 13435. 1 | A | 0.01 | 6.64E-02 | 177.9 | LYM52 | 1289 4.7 | F | 6.23 2 | 5.88E-02 | 12.1 |
| LYM217 | 13183. 2 | A | 0.00 9 | 6.68E-02 | 156.6 | LYM213 | 1284 1.7 | F | 6.42 9 | 9.76E-02 | 15.6 |
| LYM217 | 13181. 6 | A | 0.00 6 | 6.76E-02 | 62.6 | CONTR OL | - | F | 5.55 9 | - | 0 |
| LYM79 | 13133. 3 | A | 0.00 5 | 6.76E-02 | 41.3 | LYM68 | 1194 2.2 | G | 0.71 9 | 1.00E-06 | 54.5 |
| LYM23 | 12783. 8 | A | 0.00 5 | 7.66E-02 | 30.4 | LYM90 | 1239 5.3 | G | 0.72 4 | 1.00E-06 | 55.5 |
| LYM 122 | 13714. 2 | A | 0.00 8 | 7.70E-02 | 114.8 | LYM117 | 1362 3.9 | G | 0.58 6 | 5.20E-05 | 25.8 |
| LYM249 | 13631. 2 | A | 0.00 7 | 8.25E-02 | 105.1 | LYM248 | 1350 1.6 | G | 0.71 9 | 6.60E-05 | 54.4 |
| LYM274 | 13415. 2 | A | 0.00 7 | 8.30E-02 | 83.2 | LYM117 | 1362 2.6 | G | 0.70 9 | 5.32E-04 | 52.2 |
| LYM249 | 13631. 4 | A | 0.00 5 | 8.68E-02 | 31.7 | LYM 107 | 1263 1.4 | G | 0.73 3 | 5.34E-04 | 57.3 |
| LYM251 | 13072. 6 | A | 0.00 9 | 9.27E-02 | 159.3 | LYM180 | 1344 1.7 | G | 0.66 4 | 1.17E-03 | 42.6 |
| LYM240 | 12682. 1 | A | 0.00 9 | 9.57E-02 | 148.4 | LYM52 | 1289 1.8 | G | 0.87 8 | 1.61E-03 | 88.5 |
| CONTR OL | - | A | 0.00 4 | - | 0 | LYM213 | 1284 1.6 | G | 1.09 8 | 2.01E-03 | 135.7 |
| LYM251 | 13073. 8 | B | 0.14 5 | 7.17E-04 | 59.3 | LYM68 | 1194 3.5 | G | 0.65 3 | 2.34E-03 | 40.2 |
| LYM 164 | 12813. 8 | B | 0.16 6 | 8.84E-04 | 82.4 | LYM117 | 1362 1.8 | G | 0.60 8 | 3.36E-03 | 30.6 |
| LYM 122 | 13712. 3 | B | 0.12 2 | 8.94E-04 | 33.4 | LYM90 | 1239 4.2 | G | 0.64 4 | 3.68E-03 | 38.4 |
| LYM217 | 13182. 1 | B | 0.17 9 | 1.19E-03 | 96.3 | LYM52 | 1289 4.6 | G | 0.69 | 3.90E-03 | 48.2 |
| LYM273 | 13721. 3 | B | 0.22 1 | 1.26E-03 | 142.7 | LYM157 | 1334 1.1 | G | 0.80 4 | 3.92E-03 | 72.7 |
| LYM249 | 13631. 1 | B | 0.15 9 | 1.43E-03 | 74.6 | LYM 107 | 1263 3.4 | G | 0.82 9 | 5.17E-03 | 78 |
| LYM273 | 13721. 1 | B | 0.13 2 | 1.54E-03 | 44.4 | LYM52 | 1289 5.7 | G | 0.78 6 | 1.65E-02 | 68.8 |
| LYM193 | 13484. 3 | B | 0.16 5 | 2.86E-03 | 80.7 | LYM52 | 1289 4.8 | G | 0.98 7 | 1.95E-02 | 111.9 |
| LYM79 | 13132. 2 | B | 0.2 | 3.59E-03 | 119.3 | LYM117 | 1362 4.7 | G | 0.59 2 | 2.59E-02 | 27.2 |
| LYM217 | 13181. 11 | B | 0.20 3 | 4.90E-03 | 123.1 | LYM213 | 1284 1.8 | G | 0.74 6 | 2.62E-02 | 60.2 |
| LYM 122 | 13714. 4 | B | 0.24 9 | 4.98E-03 | 172.7 | LYM 180 | 1344 1.5 | G | 0.63 2 | 2.70E-02 | 35.7 |
| LYM273 | 13723. 1 | B | 0.16 5 | 8.86E-03 | 80.7 | LYM117 | 1362 4.4 | G | 0.57 6 | 2.79E-02 | 23.6 |
| LYM274 | 13411. 2 | B | 0.11 4 | 1.00E-02 | 24.9 | LYM248 | 1350 2.7 | G | 0.68 5 | 2.84E-02 | 47.1 |
| LYM274 | 13414. 2 | B | 0.18 9 | 1.01E-02 | 107.4 | LYM107 | 1263 2.1 | G | 0.58 8 | 2.98E-02 | 26.3 |
| LYM249 | 13633. 1 | B | 0.19 5 | 1.09E-02 | 114.2 | LYM157 | 1334 1.4 | G | 0.88 2 | 3.13E-02 | 89.4 |
| LYM245 | 13062. 5 | B | 0.19 7 | 1.37E-02 | 116.1 | LYM68 | 1194 1.3 | G | 0.57 3 | 5.05E-02 | 23 |
| LYM274 | 13413. 4 | B | 0.14 1 | 1.41E-02 | 54.1 | LYM157 | 1334 1.3 | G | 0.83 4 | 5.09E-02 | 79.2 |
| LYM23 | 12783. 7 | B | 0.20 4 | 1.57E-02 | 123.9 | LYM107 | 1263 1.1 | G | 0.68 8 | 5.95E-02 | 47.8 |
| LYM224 | 13435. 3 | B | 0.14 9 | 2.06E-02 | 63.9 | LYM90 | 1239 2.1 | G | 0.63 3 | 6.01E-02 | 35.9 |
| LYM79 | 13134. 3 | B | 0.14 | 2.13E-02 | 53.9 | LYM157 | 1334 1.7 | G | 0.71 7 | 8.11E-02 | 54 |
| LYM251 | 13072. 8 | B | 0.16 1 | 2.19E-02 | 76.5 | LYM52 | 1289 4.7 | G | 0.72 | 9.20E-02 | 54.6 |
| LYM23 | 12783. 8 | B | 0.13 1 | 2.26E-02 | 43.9 | LYM213 | 1284 1.7 | G | 0.69 8 | 9.67E-02 | 49.9 |
| LYM164 | 12813. 5 | B | 0.22 1 | 2.28E-02 | 142.7 | CONTR OL | - | G | 0.46 6 | - | 0 |
| LYM23 | 12782. 7 | B | 0.12 5 | 2.52E-02 | 36.8 | LYM107 | 1263 3.4 | H | 0.08 1 | 0.00E +00 | 83.3 |
| LYM217 | 13181. 7 | B | 0.12 4 | 3.28E-02 | 35.6 | LYM107 | 1263 1.4 | H | 0.07 8 | 0.00E +00 | 75.7 |
| LYM23 | 12782. 6 | B | 0.16 | 3.33E-02 | 76 | LYM117 | 1362 2.6 | H | 0.08 | 0.00E +00 | 79.3 |
| LYM273 | 13722. 3 | B | 0.14 8 | 4.38E-02 | 62.3 | LYM157 | 1334 1.4 | H | 0.09 2 | 0.00E +00 | 108 |
| LYM 122 | 13712. 1 | B | 0.14 7 | 4.52E-02 | 61.4 | LYM 157 | 1334 1.1 | H | 0.08 | 0.00E +00 | 80.9 |
| LYM251 | 13072. 6 | B | 0.23 1 | 4.55E-02 | 153.2 | LYM213 | 1284 1.6 | H | 0.11 1 | 0.00E +00 | 149.2 |
| LYM25 1 | 13074. 6 | B | 0.12 8 | 4.80E-02 | 40.5 | LYM52 | 1289 4.8 | H | 0.10 3 | 0.00E +00 | 131.6 |
| LYM131 | 12791. 5 | B | 0.13 6 | 5.54E-02 | 49.2 | LYM52 | 1289 1.8 | H | 0.09 3 | 0.00E +00 | 108.9 |
| LYM240 | 12682. 1 | B | 0.16 4 | 5.66E-02 | 80.3 | LYM52 | 1289 5.7 | H | 0.08 3 | 0.00E +00 | 87 |
| LYM 122 | 13713. 2 | B | 0.14 6 | 5.87E-02 | 60.6 | LYM90 | 1239 5.3 | H | 0.07 6 | 0.00E +00 | 71 |
| LYM23 | 12784. 6 | B | 0.18 | 6.16E-02 | 97.8 | LYM68 | 1194 2.2 | H | 0.07 4 | 1.00E-06 | 67.4 |
| LYM79 | 13133. 3 | B | 0.13 1 | 6.29E-02 | 43.3 | LYM52 | 1289 4.6 | H | 0.07 5 | 2.00E-06 | 68.1 |
| LYM122 | 13714. 2 | B | 0.17 6 | 6.66E-02 | 93.6 | LYM157 | 1334 1.3 | H | 0.08 5 | 3.00E-06 | 92.7 |
| LYM249 | 13631. 2 | B | 0.17 2 | 6.94E-02 | 88.8 | LYM213 | 1284 1.8 | H | 0.07 4 | 1.70E-05 | 66.3 |
| LYM224 | 13435. 1 | B | 0.22 5 | 7.12E-02 | 146.6 | LYM248 | 1350 1.6 | H | 0.06 9 | 3.10E-05 | 56.2 |
| LYM217 | 13183. 2 | B | 0.19 7 | 7.76E-02 | 115.6 | LYM90 | 1239 4.2 | H | 0.06 8 | 13.80E-05 | 54.1 |
| LYM23 | 12783. 5 | B | 0.16 2 | 8.12E-02 | 77.2 | LYM107 | 1263 1.1 | H | 0.07 3 | 4.60E-05 | 65.3 |
| LYM217 | 13181. 6 | B | 0.14 5 | 8.35E-02 | 59 | LYM68 | 1194 3.5 | H | 0.06 9 | 6.60E-05 | 54.6 |
| LYM40 | 13732. 1 | B | 0.14 3 | 8.41E-02 | 56.4 | LYM52 | 1289 4.7 | H | 0.07 4 | 1.57E-04 | 66.8 |
| LYM79 | 13133. 1 | B | 0.15 6 | 9.62E-02 | 70.9 | LYM90 | 1239 2.1 | H | 0.06 8 | 2.75E-04 | 52.6 |
| LYM273 | 13722. 4 | B | 0.14 | 9.64E-02 | 54 | LYM213 | 1284 1.7 | H | 0.07 | 5.36E-04 | 58.5 |
| CONTR OL | - | B | 0.09 1 | - | 0 | LYM157 | 1334 1.7 | H | 0.07 | 7.79E-04 | 57.3 |
| LYM 122 | 13714. 4 | C | 0.90 5 | 0.00E +00 | 135.4 | LYM117 | 1362 4.7 | H | 0.06 2 | 2.19E-03 | 39.2 |
| LYM122 | 13713. 2 | C | 0.68 1 | 0.00E +00 | 77 | LYM117 | 1362 4.4 | H | 0.06 | 4.46E-03 | 35.7 |
| LYM131 | 12793. 3 | C | 0.66 3 | 0.00E +00 | 72.4 | LYM248 | 1350 2.7 | H | 0.06 3 | 4.46E-03 | 41 |
| LYM164 | 12813. 5 | C | 0.93 3 | 0.00E +00 | 142.7 | LYM 180 | 1344 1.7 | H | 0.06 | 4.78E-03 | 35 |
| LYM 193 | 13482. 4 | C | 0.74 5 | 0.00E +00 | 93.9 | LYM117 | 1362 3.9 | H | 0.05 9 | 7.75E-03 | 31.9 |
| LYM217 | 13181. 6 | C | 0.85 9 | 0.00E +00 | 123.4 | LYM68 | 1194 1.4 | H | 0.06 | 9.46E-03 | 36.1 |
| LYM23 | 12783. 7 | C | 0.78 | 0.00E +00 | 102.9 | LYM 180 | 1344 1.5 | H | 0.05 8 | 1.89E-02 | 30.4 |
| LYM245 | 13062. 5 | C | 0.77 3 | 0.00E +00 | 101.1 | LYM 107 | 1263 2.1 | H | 0.05 7 | 1.94E-02 | 29.2 |
| LYM245 | 13061. 6 | C | 0.64 6 | 0.00E +00 | 68 | LYM90 | 1239 3.1 | H | 0.06 | 2.02E-02 | 36.1 |
| LYM249 | 13631. 2 | C | 0.88 6 | 0.00E +00 | 130.4 | LYM68 | 1194 1.3 | H | 0.05 7 | 2.16E-02 | 29.1 |
| LYM249 | 13633. 1 | C | 0.84 4 | 0.00E +00 | 119.6 | LYM157 | 1334 2.4 | H | 0.05 9 | 2.17E-02 | 33.1 |
| LYM251 | 13073. 8 | C | 1.06 | 0.00E +00 | 175.7 | LYM68 | 1194 2.3 | H | 0.06 3 | 2.18E-02 | 42.3 |
| LYM251 | 13073. 7 | C | 0.91 8 | 0.00E +00 | 138.9 | LYM 180 | 1344 2.6 | H | 0.05 7 | 3.88E-02 | 27.7 |
| LYM251 | 13072. 6 | C | 0.77 7 | 0.00E +00 | 102.2 | LYM117 | 1362 1.8 | H | 0.05 5 | 5.30E-02 | 24.2 |
| LYM251 | 13072. 8 | C | 0.77 | 0.00E +00 | 100.4 | CONTR OL | - | H | 0.04 4 | - | 0 |
| LYM251 | 13074. 6 | C | 0.75 5 | 0.00E +00 | 96.5 | LYM268 | 1248 2.3 | A | 0.00 4 | 6.00E-06 | 66.2 |
| LYM273 | 13723. 1 | C | 0.88 6 | 0.00E +00 | 130.5 | LYM 140 | 1226 1.2 | A | 0.00 4 | 5.80E-05 | 59.4 |
| LYM273 | 13721. 3 | C | 0.75 5 | 0.00E +00 | 96.3 | LYM 162 | 1223 1.3 | A | 0.00 6 | 7.40E-05 | 130.9 |
| LYM273 | 13722. 4 | C | 0.66 5 | 0.00E +00 | 73 | LYM13 | 1177 4.1 | A | 0.00 6 | 1.23E-04 | 132.9 |
| LYM274 | 13414. 2 | C | 0.85 5 | 0.00E +00 | 122.3 | LYM140 | 1226 1.1 | A | 0.00 5 | 2.00E-04 | 74.9 |
| LYM274 | 13415. 2 | C | 0.84 7 | 0.00E +00 | 120.2 | LYM 134 | 1231 4.2 | A | 0.00 4 | 3.70E-04 | 68.1 |
| LYM274 | 13413. 4 | C | 0.82 4 | 0.00E +00 | 114.4 | LYM 162 | 1223 4.3 | A | 0.00 8 | 7.16E-04 | 204.3 |
| LYM40 | 13734. 2 | C | 0.61 1 | 0.00E +00 | 58.9 | LYM132 | 1227 1.4 | A | 0.00 5 | 8.37E-04 | 79.7 |
| LYM79 | 13132. 2 | C | 0.81 1 | 0.00E +00 | 110.9 | LYM140 | 1226 1.4 | A | 0.00 4 | 1.14E-03 | 47.8 |
| LYM79 | 13133. 1 | C | 0.71 | 0.00E +00 | 84.6 | LYM289 | 1249 1.1 | A | 0.00 5 | 1.21E-03 | 110.6 |
| LYM79 | 13134. 3 | C | 0.63 8 | 0.00E +00 | 66 | LYM290 | 1250 4.1 | A | 0.00 4 | 2.88E-03 | 49.8 |
| LYM249 | 13631. 4 | C | 0.59 4 | 1.00E-06 | 54.4 | LYM290 | 1250 2.4 | A | 0.00 5 | 3.71E-03 | 101.9 |
| LYM122 | 13712. 3 | C | 0.62 4 | 2.00E-06 | 62.3 | LYM113 | 1244 4.4 | A | 0.00 5 | 3.73E-03 | 104.8 |
| LYM273 | 13722. 3 | C | 0.65 | 2.00E-06 | 69.2 | LYM140 | 1226 2.2 | A | 0.00 7 | 4.11E-03 | 187.9 |
| LYM23 | 12784. 6 | C | 0.62 5 | 3.00E-06 | 62.5 | LYM 134 | 1231 2.4 | A | 0.00 6 | 5.01E-03 | 122.2 |
| LYM224 | 13435. 1 | C | 0.69 1 | 4.00E-06 | 79.7 | LYM268 | 1248 3.4 | A | 0.00 6 | 5.96E-03 | 150.2 |
| LYM249 | 13631. 1 | C | 0.57 4 | 7.00E-06 | 49.3 | LYM 132 | 1227 5.3 | A | 0.00 5 | 7.41E-03 | 73.9 |
| LYM40 | 13732. 1 | C | 0.72 8 | 1.00E-05 | 89.4 | LYM268 | 1248 2.1 | A | 0.00 9 | 1.25E-02 | 246.9 |
| LYM79 | 13133. 3 | C | 0.58 9 | 1.50E-05 | 53.1 | LYM 102 | 1222 2.3 | A | 0.01 3 | 1.32E-02 | 387.9 |
| LYM240 | 12682. 1 | C | 0.89 8 | 1.80E-05 | 133.5 | LYM3 | 1204 1.2 | A | 0.00 5 | 1.33E-02 | 107.7 |
| LYM 131 | 12791. 8 | C | 0.73 4 | 3.10E-05 | 90.9 | LYM289 | 1249 3.2 | A | 0.00 6 | 1.44E-02 | 144.4 |
| LYM274 | 13413. 1 | C | 0.61 7 | 4.50E-05 | 60.5 | LYM3 | 1204 2.1 | A | 0.00 5 | 1.46E-02 | 108.7 |
| LYM260 | 13095. 7 | C | 0.58 6 | 7.10E-05 | 52.5 | LYM 162 | 1223 4.4 | A | 0.00 5 | 1.69E-02 | 101 |
| LYM23 | 12782. 6 | C | 0.63 5 | 8.80E-05 | 65.2 | LYM113 | 1244 4.5 | A | 0.00 5 | 1.98E-02 | 96. 1 |
| LYM40 | 13732. 2 | C | 0.57 | 1.08E-04 | 48.4 | LYM113 | 1244 2.2 | A | 0.00 5 | 2.39E-02 | 95.2 |
| LYM260 | 13095. 1 | C | 0.76 9 | 1.25E-04 | 100 | LYM289 | 1249 2.2 | A | 0.00 8 | 2.43E-02 | 194.7 |
| LYM 122 | 13712. 1 | C | 0.59 4 | 1.35E-04 | 54.4 | LYM106 | 1214 2.1 | A | 0.00 9 | 2.45E-02 | 261.4 |
| LYM164 | 12814. 8 | C | 0.56 5 | 2.55E-04 | 47 | LYM113 | 1244 2.1 | A | 0.00 6 | 2.58E-02 | 136.7 |
| LYM245 | 13064. 8 | C | 0.57 9 | 3.35E-04 | 50.6 | LYM132 | 1227 3.2 | A | 0.00 3 | 2.75E-02 | 25.6 |
| LYM122 | 13714. 2 | C | 0.61 8 | 3.64E-04 | 60.6 | LYM 134 | 1231 2.3 | A | 0.00 7 | 2.91E-02 | 155.1 |
| LYM164 | 12814. 7 | C | 0.56 8 | 5.97E-04 | 47.6 | LYM148 | 1217 3.1 | A | 0.00 6 | 2.96E-02 | 130 |
| LYM224 | 13434. 2 | C | 0.54 5 | 8.91E-04 | 41.8 | LYM13 | 1177 2.2 | A | 0.00 4 | 2.97E-02 | 36.2 |
| LYM 164 | 12813. 8 | C | 0.53 4 | 9.49E-04 | 38.8 | LYM132 | 1227 6.1 | A | 0.00 8 | 3.01E-02 | 206.3 |
| LYM224 | 13435. 5 | C | 0.55 4 | 1.23E-03 | 44 | LYM129 | 1257 3.1 | A | 0.00 4 | 3.03E-02 | 71 |
| LYM 193 | 13484. 4 | C | 0.53 1 | 1.25E-03 | 38 | LYM102 | 1222 2.1 | A | 0.01 2 | 3.89E-02 | 345.4 |
| LYM23 | 12783. 8 | C | 0.51 5 | 1.59E-03 | 33.9 | LYM 134 | 1231 1.2 | A | 0.00 6 | 4.57E-02 | 122.2 |
| LYM 193 | 13484. 3 | C | 0.53 7 | 2.28E-03 | 39.7 | LYM268 | 1248 1.1 | A | 0.00 4 | 5.25E-02 | 72.9 |
| LYM217 | 13182. 1 | C | 0.54 4 | 3.30E-03 | 41.4 | LYM10 | 1174 4.5 | A | 0.00 4 | 5.94E-02 | 47.8 |
| LYM249 | 13631. 3 | C | 0.49 4 | 5.23E-03 | 28.5 | LYM 148 | 1217 2.1 | A | 0.00 6 | 6.12E-02 | 116.4 |
| LYM79 | 13134. 2 | C | 0.50 4 | 8.40E-03 | 31.1 | LYM113 | 1244 3.1 | A | 0.00 8 | 6.54E-02 | 197.6 |
| LYM273 | 13721. 1 | C | 0.50 5 | 1.06E-02 | 31.4 | LYM289 | 1249 1.4 | A | 0.00 4 | 6.60E-02 | 52.7 |
| LYM260 | 13095. 8 | C | 0.49 7 | 1.36E-02 | 29.2 | LYM 102 | 1222 2.2 | A | 0.00 8 | 7.00E-02 | 222.7 |
| LYM23 | 12782. 7 | C | 0.50 3 | 1.53E-02 | 30.9 | LYM10 | 1174 2.2 | A | 0.00 4 | 8.72E-02 | 44 |
| LYM245 | 13061. 8 | C | 0.48 3 | 1.73E-02 | 25.7 | LYM13 | 1177 2.1 | A | 0.00 5 | 9.00E-02 | 77.8 |
| LYM23 | 12783. 5 | C | 0.48 7 | 1.88E-02 | 26.7 | LYM129 | 1257 3.5 | A | 0.00 5 | 9.06E-02 | 85.5 |
| LYM217 | 13181. 7 | C | 0.47 9 | 3.48E-02 | 24.7 | LYM 102 | 1222 1.1 | A | 0.00 7 | 9.14E-02 | 183.1 |
| LYM224 | 13432. 1 | C | 0.46 5 | 3.71E-02 | 21 | LYM 106 | 1214 4.4 | A | 0.00 4 | 9.31E-02 | 66.2 |
| CONTR OL | - | C | 0.38 4 | - | 0 | LYM3 | 1204 3.2 | A | 0.00 4 | 9.69E-02 | 70 |
| LYM 122 | 13713. 2 | D | 6.38 8 | 0.00E +00 | 77 | CONTR OL | - | A | 0.00 3 | - | 0 |
| LYM249 | 13631. 4 | D | 5.39 8 | 0.00E +00 | 49.6 | LYM13 | 1177 4.1 | B | 0.13 2 | 3.00E-06 | 64.6 |
| LYM273 | 13723. 1 | D | 7.90 7 | 0.00E +00 | 119.1 | LYM3 | 1204 1.2 | B | 0.13 4 | 2.20E-04 | 66.7 |
| LYM249 | 13631. 3 | D | 4.59 9 | 3.10E-05 | 27.4 | LYM 162 | 1223 4.3 | B | 0.18 6 | 4.15E-04 | 131.8 |
| LYM249 | 13631. 1 | D | 5.21 5 | 4.80E-05 | 44.5 | LYM 162 | 1223 4.4 | B | 0.12 | 6.94E-04 | 49.1 |
| LYM251 | 13073. 8 | D | 9.27 6 | 2.48E-04 | 157 | LYM140 | 1226 1.2 | B | 0.10 3 | 8.53E-04 | 28 |
| LYM131 | 12793. 3 | D | 5.81 7 | 2.93E-04 | 61.2 | LYM3 | 1204 3.2 | B | 0.10 4 | 3.07E-03 | 29.9 |
| LYM164 | 12813. 5 | D | 8.33 4 | 4.25E-04 | 130.9 | LYM290 | 1250 2.4 | B | 0.13 | 3.23E-03 | 61.6 |
| LYM245 | 13061. 6 | D | 6.16 3 | 1.03E-03 | 70.8 | LYM 132 | 1227 6.1 | B | 0.15 9 | 3.74E-03 | 98.1 |
| LYM273 | 13722. 4 | D | 5.99 7 | 1.03E-03 | 66.2 | LYM 162 | 1223 1.3 | B | 0.13 6 | 5.97E-03 | 69.7 |
| LYM23 | 12783. 8 | D | 4.63 1 | 2.39E-03 | 28.3 | LYM 134 | 1231 2.3 | B | 0.15 7 | 6.63E-03 | 95.2 |
| LYM40 | 13734. 2 | D | 5.44 6 | 2.86E-03 | 50.9 | LYM289 | 1249 1.1 | B | 0.12 8 | 7.98E-03 | 59 |
| LYM217 | 13181. 6 | D | 7.55 6 | 3.51E-03 | 109.4 | LYM 140 | 1226 2.2 | B | 0.17 4 | 8.75E-03 | 117.3 |
| LYM224 | 13432. 1 | D | 4.16 5 | 4.08E-03 | 15.4 | LYM 132 | 1227 5.3 | B | 0.11 4 | 8.82E-03 | 41.5 |
| LYM274 | 13415. 2 | D | 7.41 9 | 4.10E-03 | 105.6 | LYM289 | 1249 2.2 | B | 0.19 | 1.16E-02 | 137.4 |
| LYM251 | 13074. 6 | D | 6.66 6 | 4.94E-03 | 84.7 | LYM268 | 1248 2.1 | B | 0.19 2 | 1.21E-02 | 139.2 |
| LYM249 | 13633. 1 | D | 8 | 4.95E-03 | 121.7 | LYM113 | 1244 2.1 | B | 0.14 5 | 1.30E-02 | 80.3 |
| LYM23 | 12783. 7 | D | 7.13 7 | 6.65E-03 | 97.7 | LYM268 | 1248 3.4 | B | 0.13 2 | 1.32E-02 | 64.2 |
| LYM79 | 13134. 3 | D | 5.54 | 7.39E-03 | 53.5 | LYM3 | 1204 2.1 | B | 0.14 2 | 1.38E-02 | 77.1 |
| LYM122 | 13714. 4 | D | 8.31 5 | 8.23E-03 | 130.4 | LYM290 | 1250 4.1 | B | 0.09 5 | 1.49E-02 | 17.8 |
| LYM79 | 13133. 1 | D | 6.17 9 | 1.22E-02 | 71.2 | LYM148 | 1217 3.1 | B | 0.14 1 | 1.51E-02 | 75.8 |
| LYM79 | 13132. 2 | D | 7.31 5 | 1.42E-02 | 102.7 | LYM 134 | 1231 2.4 | B | 0.13 9 | 1.55E-02 | 73.1 |
| LYM251 | 13072. 6 | D | 7.17 5 | 1.42E-02 | 98.8 | LYM290 | 1250 2.1 | B | 0.09 6 | 1.59E-02 | 20 |
| LYM25 1 | 13073. 7 | D | 8.23 | 1.56E-02 | 128 | LYM 106 | 1214 4.4 | B | 0.10 3 | 1.67E-02 | 28.3 |
| LYM251 | 13072. 8 | D | 6.89 6 | 1.59E-02 | 91.1 | LYM102 | 1222 2.3 | B | 0.25 2 | 2.42E-02 | 213.8 |
| LYM273 | 13721. 3 | D | 6.84 4 | 1.74E-02 | 89.6 | LYM148 | 1217 2.1 | B | 0.12 7 | 2.50E-02 | 58.1 |
| LYM274 | 13414. 2 | D | 7.66 7 | 1.85E-02 | 112.4 | LYM 106 | 1214 2.1 | B | 0.19 4 | 2.73E-02 | 141.6 |
| LYM245 | 13062. 5 | D | 6.54 3 | 2.21E-02 | 81.3 | LYM 102 | 1222 2.1 | B | 0.23 7 | 3.04E-02 | 195.1 |
| LYM40 | 13732. 2 | D | 5.37 3 | 2.26E-02 | 48.9 | LYM113 | 1244 4.5 | B | 0.13 3 | 3.89E-02 | 65.3 |
| LYM 122 | 13712. 3 | D | 5.38 4 | 2.45E-02 | 49.2 | LYM 129 | 1257 3.5 | B | 0.13 5 | 3.90E-02 | 67.9 |
| LYM224 | 13435. 1 | D | 6.55 4 | 2.96E-02 | 81.6 | LYM 129 | 1257 1.3 | B | 0.15 1 | 4.13E-02 | 88.4 |
| LYM249 | 13631. 2 | D | 7.72 | 3.46E-02 | 113.9 | LYM268 | 1248 2.3 | B | 0.09 4 | 4.38E-02 | 17.1 |
| LYM273 | 13722. 3 | D | 5.77 7 | 3.61E-02 | 60.1 | LYM113 | 1244 4.4 | B | 0.13 6 | 4.55E-02 | 69.1 |
| LYM79 | 13133. 3 | D | 5.12 6 | 3.67E-02 | 42 | LYM289 | 1249 3.2 | B | 0.13 | 4.69E-02 | 62.4 |
| LYM23 | 12784. 6 | D | 5.42 8 | 3.91E-02 | 50.4 | LYM113 | 1244 3.1 | B | 0.18 6 | 5.16E-02 | 131.8 |
| LYM 193 | 13482. 4 | D | 6.65 4 | 4.51E-02 | 84.4 | LYM 134 | 1231 1.2 | B | 0.13 9 | 5.91E-02 | 73.4 |
| LYM 193 | 13484. 4 | D | 4.89 5 | 4.61E-02 | 35.6 | LYM 132 | 1227 1.4 | B | 0.10 5 | 6.45E-02 | 31.3 |
| LYM274 | 13413. 4 | D | 7.88 9 | 4.88E-02 | 118.6 | LYM 129 | 1257 3.1 | B | 0.11 4 | 6.88E-02 | 41.7 |
| LYM 193 | 13481. 2 | D | 4.14 | 5.04E-02 | 14.7 | LYM 140 | 1226 1.4 | B | 0.09 6 | 7.22E-02 | 19.9 |
| LYM40 | 13732. 1 | D | 6.93 9 | 5.19E-02 | 92.3 | LYM 106 | 1214 1.4 | B | 0.12 1 | 7.36E-02 | 50.4 |
| LYM 122 | 13712. 1 | D | 5.60 8 | 5.7 1E-02 | 55.4 | LYM102 | 1222 2.2 | B | 0.19 2 | 7.66E-02 | 139.1 |
| LYM164 | 12813. 8 | D | 4.76 6 | 6.09E-02 | 32 | LYM 162 | 1223 3.2 | B | 0.11 4 | 8.96E-02 | 42.5 |
| LYM 164 | 12814. 8 | D | 5.12 6 | 6.17E-02 | 42 | LYM140 | 1226 1.1 | B | 0.09 6 | 9.02E-02 | 19.5 |
| LYM240 | 12682. 1 | D | 8.44 4 | 6.37E-02 | 134 | LYM13 | 1177 2.2 | B | 0.13 1 | 9.05E-02 | 63.5 |
| LYM260 | 13095. 7 | D | 5.14 7 | 6.73E-02 | 42.6 | CONTR OL | - - | B | 0.08 | - - | 0 |
| LYM274 | 13413. 1 | D | 5.52 8 | 7.60E-02 | 53.2 | LYM 102 | 1222 2.3 | C | 1.24 2 | 0.00E +00 | 203.7 |
| LYM23 | 12783. 5 | D | 4.38 8 | 7.70E-02 | 21.6 | LYM 102 | 1222 2.1 | C | 1.14 8 | 0.00E +00 | 180.7 |
| LYM131 | 12791. 8 | D | 6.88 2 | 8.02E-02 | 90.7 | LYM106 | 1214 2.1 | C | 0.74 4 | 0.00E +00 | 82.1 |
| LYM 193 | 13484. 3 | D | 5.01 9 | 9.01E-02 | 39.1 | LYM113 | 1244 4.4 | C | 0.80 5 | 0.00E +00 | 96.9 |
| CONTR OL | - | D | 3.60 | - | 0 | LYM113 | 1244 3.1 | C | 0.79 4 | 0.00E +00 | 94.1 |
| LYM251 | 13073. 8 | E | 0.58 4 | 1.00E-06 | 58.3 | LYM129 | 1257 3.5 | C | 0.86 1 | 0.00E +00 | 110.7 |
| LYM251 | 13074. 6 | E | 0.57 7 | 1.00E-06 | 56.3 | LYM132 | 1227 6.1 | C | 0.74 9 | 0.00E +00 | 83.3 |
| LYM249 | 13631. 2 | E | 0.58 2 | 4.00E-06 | 57.8 | LYM 148 | 1217 3.1 | C | 0.79 8 | 0.00E +00 | 95.1 |
| LYM 164 | 12811. 8 | E | 0.55 1 | 6.00E-06 | 49.2 | LYM148 | 1217 2.1 | C | 0.73 4 | 0.00E +00 | 79.5 |
| LYM217 | 13181. 6 | E | 0.56 7 | 1.50E-05 | 53.6 | LYM 162 | 1223 4.3 | C | 0.97 8 | 0.00E +00 | 139.2 |
| LYM245 | 13062. 5 | E | 0.53 7 | 3.50E-05 | 45.6 | LYM 162 | 1223 1.2 | C | 0.73 2 | 0.00E +00 | 79 |
| LYM245 | 13064. 8 | E | 0.52 2 | 1.42E-04 | 41.5 | LYM268 | 1248 2.3 | C | 0.78 4 | 0.00E +00 | 91.8 |
| LYM122 | 13713. 2 | E | 0.52 2 | 2.35E-04 | 41.5 | LYM268 | 1248 3.4 | C | 0.76 2 | 0.00E +00 | 86.3 |
| LYM164 | 12813. 5 | E | 0.54 4 | 2.41E-04 | 47.5 | LYM268 | 1248 3.2 | C | 0.74 | 0.00E +00 | 81 |
| LYM122 | 13712. 3 | E | 0.51 9 | 2.99E-04 | 40.7 | LYM289 | 1249 2.2 | C | 0.84 5 | 0.00E +00 | 106.7 |
| LYM251 | 13073. 7 | E | 0.52 7 | 4.09E-04 | 42.7 | LYM289 | 1249 1.1 | C | 0.78 9 | 0.00E +00 | 92.9 |
| LYM273 | 13722. 4 | E | 0.50 5 | 9.21E-04 | 36.9 | LYM289 | 1249 3.6 | C | 0.78 5 | 0.00E +00 | 92 |
| LYM273 | 13723. 1 | E | 0.50 2 | 1.54E-03 | 36.1 | LYM289 | 1249 3.2 | C | 0.72 1 | 0.00E +00 | 76.4 |
| LYM23 | 12783. 7 | E | 0.49 3 | 2.29E-03 | 33.5 | LYM290 | 1250 2.4 | C | 0.77 7 | 0.00E +00 | 90 |
| LYM131 | 12791. 8 | E | 0.48 7 | 2.71E-03 | 31.9 | LYM148 | 1217 3.5 | C | 0.71 1 | 1.00E-06 | 73.9 |
| LYM25 1 | 13072. 8 | E | 0.49 8 | 2.78E-03 | 35 | LYM268 | 1248 2.1 | C | 0.77 2 | 1.00E-06 | 88.9 |
| LYM260 | 13095. 7 | E | 0.49 1 | 3.02E-03 | 32.9 | LYM290 | 1250 1.2 | C | 0.72 4 | 1.00E-06 | 77.1 |
| LYM249 | 13631. 1 | E | 0.48 9 | 3.23E-03 | 32.5 | LYM129 | 1257 1.3 | C | 0.77 6 | 2.00E-06 | 89.9 |
| LYM249 | 13631. 4 | E | 0.49 8 | 3.49E-03 | 34.8 | LYM162 | 1223 1.3 | C | 0.74 6 | 2.00E-06 | 82.5 |
| LYM131 | 12793. 3 | E | 0.48 6 | 3.59E-03 | 31.8 | LYM 102 | 1222 2.2 | C | 0.74 8 | 5.00E-06 | 83.1 |
| LYM251 | 13072. 6 | E | 0.48 3 | 4.03E-03 | 31 | LYM10 | 1174 4.5 | C | 0.71 1 | 7.00E-06 | 73.8 |
| LYM79 | 13132. 2 | E | 0.47 8 | 5.43E-03 | 29.6 | LYM 134 | 1231 4.2 | C | 0.65 8 | 2.30E-05 | 61 |
| LYM79 | 13134. 3 | E | 0.47 3 | 7.86E-03 | 28 | LYM10 | 1174 1.4 | C | 0.68 3 | 2.80E-05 | 67 |
| LYM 193 | 13482. 4 | E | 0.47 8 | 8.17E-03 | 29.6 | LYM113 | 1244 2.1 | C | 0.64 8 | 3.30E-05 | 58.5 |
| LYM79 | 13133. 1 | E | 0.48 1 | 8.28E-03 | 30.4 | LYM13 | 1177 4.1 | C | 0.64 1 | 3.30E-05 | 56.7 |
| LYM274 | 13415. 2 | E | 0.47 5 | 9.01E-03 | 28.6 | LYM134 | 1231 1.2 | C | 0.65 1 | 7.30E-05 | 59.3 |
| LYM40 | 13734. 2 | E | 0.46 9 | 9.05E-03 | 27.2 | LYM289 | 1249 1.4 | C | 0.65 4 | 1.15E-04 | 60 |
| LYM260 | 13095. 1 | E | 0.49 6 | 1.32E-02 | 34.3 | LYM 148 | 1217 1.2 | C | 0.60 8 | 1.49E-04 | 48.7 |
| LYM224 | 13434. 2 | E | 0.46 6 | 1.34E-02 | 26.4 | LYM 140 | 1226 1.4 | C | 0.62 2 | 1.52E-04 | 52.1 |
| LYM79 | 13133. 3 | E | 0.46 5 | 1.55E-02 | 25.9 | LYM 134 | 1231 2.4 | C | 0.61 6 | 1.68E-04 | 50.6 |
| LYM 164 | 12814. 7 | E | 0.46 7 | 1.71E-02 | 26.5 | LYM 148 | 1217 4.1 | C | 0.60 8 | 1.69E-04 | 48.6 |
| LYM274 | 13413. 4 | E | 0.47 5 | 2.10E-02 | 28.6 | LYM 106 | 1214 2.2 | C | 0.64 7 | 2.21E-04 | 58.2 |
| LYM40 | 13732. 1 | E | 0.47 5 | 2.15E-02 | 28.7 | LYM268 | 1248 1.1 | C | 0.63 9 | 2.23E-04 | 56.3 |
| LYM40 | 13732. 2 | E | 0.46 | 2.66E-02 | 24.6 | LYM290 | 1250 2.1 | C | 0.61 | 4.18E-04 | 49.3 |
| LYM260 | 13095. 8 | E | 0.46 2 | 2.67E-02 | 25.3 | LYM290 | 1250 4.1 | C | 0.60 9 | 6.87E-04 | 48.9 |
| LYM23 | 12784. 6 | E | 0.45 3 | 2.97E-02 | 22.8 | LYM113 | 1244 4.5 | C | 0.59 6 | 7.01E-04 | 45.7 |
| LYM274 | 13414. 2 | E | 0.45 7 | 2.97E-02 | 23.7 | LYM 102 | 1222 1.1 | C | 0.64 8 | 1.55E-03 | 58.5 |
| LYM240 | 12682. 1 | E | 0.46 2 | 4.66E-02 | 25.1 | LYM3 | 1204 2.1 | C | 0.57 | 2.95E-03 | 39.4 |
| LYM23 | 12782. 6 | E | 0.45 1 | 5.25E-02 | 22.2 | LYM129 | 1257 3.1 | C | 0.58 1 | 3.85E-03 | 42.1 |
| LYM 193 | 13481. 2 | E | 0.43 9 | 6.58E-02 | 18.9 | LYM140 | 1226 2.3 | C | 0.57 4 | 4.08E-03 | 40.5 |
| LYM23 | 12783. 8 | E | 0.43 8 | 8.19E-02 | 18.6 | LYM134 | 1231 2.3 | C | 0.56 7 | 4.13E. 03 | 38.6 |
| LYM224 | 13435. 5 | E | 0.43 8 | 8.43E-02 | 18.8 | LYM10 | 1174 1.2 | C | 0.55 9 | 4.92E-03 | 36.8 |
| LYM273 | 13722. 3 | E | 0.44 | 9.76E-02 | 19.3 | LYM132 | 1227 3.2 | C | 0.56 3 | 5.24E-03 | 37.6 |
| CONTR OL | - | E | 0.36 9 | - - | 0 | LYM290 | 1250 1.3 | C | 0.55 2 | 5.82E-03 | 34.9 |
| LYM131 | 12791. 8 | F | 6.80 9 | 0.00E +00 | 41.1 | LYM 106 | 1214 1.4 | C | 0.57 7 | 7.47E-03 | 41.2 |
| LYM251 | 13073. 8 | F | 7.17 8 | 0.00E +00 | 48.8 | LYM 140 | 1226 1.1 | C | 0.54 | 1.12E-02 | 32.2 |
| LYM251 | 13074. 6 | F | 7.04 2 | 0.00E +00 | 46 | LYM3 | 1204 1.1 | C | 0.54 7 | 1.19E-02 | 33.7 |
| LYM251 | 13073. 7 | F | 6.98 8 | 0.00E +00 | 44.9 | LYM 132 | 1227 1.4 | C | 0.56 5 | 1.23E-02 | 38.3 |
| LYM25 1 | 13072. 6 | F | 6.46 5 | 0.00E +00 | 34 | LYM10 | 1174 2.2 | C | 0.56 9 | 1.23E-02 | 39.3 |
| LYM40 | 13734. 2 | F | 5.90 2 | 0.00E +00 | 22.3 | LYM140 | 1226 2.2 | C | 0.56 | 1.42E-02 | 36.9 |
| LYM249 | 13631. 3 | F | 5.61 7 | 1.00E-06 | 16.4 | LYM13 | 1177 2.1 | C | 0.53 | 1.46E-02 | 29.6 |
| LYM 164 | 12811. 8 | F | 5.94 1 | 2.00E-06 | 23.1 | LYM3 | 1204 1.2 | C | 0.52 4 | 2.55E-02 | 28.1 |
| LYM79 | 13134. 3 | F | 5.85 2 | 2.00E-06 | 21.3 | LYM1 29 | 1257 2.2 | C | 0.52 1 | 3.13E-02 | 27.5 |
| LYM245 | 13064. 8 | F | 6.21 5 | 3.00E-06 | 28.8 | LYM 102 | 1222 2.6 | C | 0.52 2 | 3.24E-02 | 27.7 |
| LYM274 | 13415. 2 | F | 6.45 4 | 1.40E-05 | 33.8 | LYM13 | 1177 2.2 | C | 0.51 6 | 4.80E-02 | 26.2 |
| LYM274 | 13413. 4 | F | 6.96 3 | 4.00E-05 | 44.3 | LYM132 | 1227 5.1 | C | 0.50 1 | 6.79E-02 | 22.6 |
| LYM193 | 13481. 2 | F | 5.96 | 8.50E-05 | 23.5 | LYM 162 | 1223 4.4 | C | 0.50 1 | 6.95E-02 | 22.5 |
| LYM79 | 13132. 2 | F | 6.12 2 | 1.83E-04 | 26.9 | LYM13 | 1177 1.6 | C | 0.51 9 | 7.56E-02 | 26.9 |
| LYM 122 | 13714. 4 | F | 6.25 4 | 3.39E-04 | 29.6 | LYM140 | 1226 1.2 | C | 0.50 2 | 8.74E-02 | 22.7 |
| LYM 122 | 13712. 1 | F | 6.19 | 3.43E-04 | 28.3 | LYM3 | 1204 3.2 | C | 0.49 4 | 9.78E-02 | 20.9 |
| LYM245 | 13062. 5 | F | 5.76 8 | 3.71E-04 | 19.6 | CONTR OL | - | C | 0.40 9 | - - | 0 |
| LYM249 | 13631. 2 | F | 6.98 7 | 4.32E-04 | 44.8 | LYM268 | 1248 2.3 | D | 6.51 6 | 0.00E +00 | 87.6 |
| LYM193 | 13482. 4 | F | 6.30 8 | 4.55E-04 | 30.8 | LYM268 | 1248 3.4 | D | 6.28 8 | 2.00E-06 | 81 |
| LYM217 | 13181. 6 | F | 6.75 3 | 5.78E-04 | 40 | LYM 148 | 1217 1.2 | D | 5.18 9 | 4.00E-05 | 49.4 |
| LYM164 | 12814. 8 | F | 5.56 1 | 7.94E-04 | 15.3 | LYM289 | 1249 2.2 | D | 7.22 3 | 7.40E-05 | 107.9 |
| LYM131 | 12793. 3 | F | 6.00 3 | 1.10E-03 | 24.4 | LYM 148 | 1217 4.1 | D | 5.09 6 | 9.60E-05 | 46.7 |
| LYM23 | 12783. 7 | F | 6.27 5 | 1.34E-03 | 30.1 | LYM140 | 1226 1.1 | D | 4.51 2 | 3.53E-04 | 29.9 |
| LYM122 | 13713. 2 | F | 6.76 5 | 1.65E-03 | 40.2 | LYM13 | 1177 2.1 | D | 4.46 7 | 8.33E-04 | 28.6 |
| LYM245 | 13061. 6 | F | 6.11 7 | 3.30E-03 | 26.8 | LYM148 | 1217 3.1 | D | 6.76 7 | 1.30E-03 | 94.8 |
| LYM273 | 13722. 4 | F | 6.08 9 | 3.52E-03 | 26.2 | LYM290 | 1250 2.4 | D | 6.42 5 | 2.00E-03 | 85 |
| LYM164 | 12813. 5 | F | 6.91 5 | 5.03E-03 | 43.3 | LYM162 | 1223 4.3 | D | 8.82 | 2.51E-03 | 153.9 |
| LYM249 | 13631. 1 | F | 5.83 | 5.49E-03 | 20.8 | LYM162 | 1223 1.2 | D | 6.02 6 | 2.51E-03 | 73.5 |
| LYM273 | 13723. 1 | F | 6.30 3 | 5.56E-03 | 30.6 | LYM289 | 1249 3.6 | D | 6.41 3 | 2.68E-03 | 84.6 |
| LYM40 | 13732. 1 | F | 6.44 6 | 6.08E-03 | 33.6 | LYM289 | 1249 3.2 | D | 5.99 7 | 3.69E-03 | 72.6 |
| LYM224 | 13435. 1 | F | 6.00 1 | 6.99E-03 | 24.4 | LYM 132 | 1227 6.1 | D | 6.25 7 | 4.48E-03 | 80.1 |
| LYM240 | 12682. 1 | F | 6.59 4 | 9.82E-03 | 36.7 | LYM113 | 1244 2.1 | D | 5.43 5 | 4.57E-03 | 56.5 |
| LYM122 | 13712. 3 | F | 5.82 2 | 1.17E-02 | 20.7 | LYM290 | 1250 1.3 | D | 4.57 3 | 5.64E-03 | 31.7 |
| LYM224 | 13435. 5 | F | 5.72 6 | 1.24E-02 | 18.7 | LYM13 | 1177 4.1 | D | 5.36 3 | 6.31E-03 | 54.4 |
| LYM249 | 13633. 1 | F | 6.40 5 | 1.51E-02 | 32.8 | LYM132 | 1227 5.3 | D | 4.07 6 | 6.87E-03 | 17.3 |
| LYM40 | 13732. 2 | F | 6.01 5 | 1.52E-02 | 24.7 | LYM 148 | 1217 3.5 | D | 5.83 6 | 6.99E-03 | 68 |
| LYM274 | 13414, 2 | F | 5.79 2 | 1.60E-02 | 20.1 | LYM106 | 1214 2.1 | D | 6.27 9 | 7.68E-03 | 80.8 |
| LYM224 | 13432. 1 | F | 5.18 4 | 1.77E-02 | 7.5 | LYM113 | 1244 4.4 | D | 6.70 6 | 9.46E-03 | 93 |
| LYM249 | 13631. 4 | F | 6.1 | 2.05E-02 | 26.4 | LYM113 | 1244 4.5 | D | 4.96 5 | 1.05E-02 | 42.9 |
| LYM260 | 13095. 7 | F | 5.94 3 | 2.05E-02 | 23.2 | LYM102 | 1222 2.3 | D | 10.3 47 | 1.06E-02 | 197.9 |
| LYM260 | 13095. 1 | F | 6.16 5 | 2.12E-02 | 27.8 | LYM289 | 1249 1.1 | D | 6.56 | 1.24E-02 | 88.8 |
| LYM251 | 13072. 8 | F | 5.90 1 | 2.20E-02 | 22.3 | LYM113 | 1244 3.1 | D | 6.78 4 | 1.25E-02 | 95.3 |
| LYM164 | 12814. 7 | F | 5.8 | 2.60E-02 | 20.2 | LYM268 | 1248 3.2 | D | 6.26 3 | 1.30E-02 | 80.3 |
| LYM274 | 13413. 1 | F | 5.83 4 | 2.70E-02 | 20.9 | LYM134 | 1231 2.4 | D | 5.22 4 | 1.38E-02 | 50.4 |
| LYM193 | 13484. 4 | F | 5.59 9 | 3.59E-02 | 16.1 | LYM 134 | 1231 4.2 | D | 5.60 9 | 1.45E-02 | 61.5 |
| LYM79 | 13133. 1 | F | 5.78 2 | 4.30E-02 | 19.8 | LYM148 | 1217 2.1 | D | 6.05 5 | 1.59E-02 | 74.3 |
| LYM273 | 13721. 3 | F | 5.38 6 | 4.48E-02 | 11.6 | LYM 140 | 1226 1.4 | D | 5.19 2 | 1.62E-02 | 49.5 |
| LYM79 | 13133. 3 | F | 5.45 2 | 4.87E-02 | 13 | LYM290 | 1250 1.2 | D | 6.09 7 | 1.82E-02 | 75.5 |
| LYM23 | 12782. 7 | F | 5.25 1 | 6.38E-02 | 8.8 | LYM 129 | 1257 3.5 | D | 7.10 7 | 2.09E-02 | 104.6 |
| LYM260 | 13095. 8 | F | 5.58 8 | 9.86E-02 | 15.8 | LYM290 | 1250 2.1 | D | 5.03 8 | 2.88E-02 | 45 |
| CONTR OL | - | F | 4.82 4 | - - | 0 | LYM3 | 1204 2.1 | D | 4.82 3 | 3.26E-02 | 38.8 |
| LYM224 | 13435. 3 | G | 0.76 4 | 0.00E +00 | 72.3 | LYM162 | 1223 1.3 | D | 6.43 4 | 3.53E-02 | 85.2 |
| LYM40 | 13734. 2 | G | 0.56 7 | 1.08E-04 | 27.9 | LYM 134 | 1231 1.2 | D | 5.55 8 | 3.62E-02 | 60 |
| LYM273 | 13722. 4 | G | 0.85 6 | 4.09E-04 | 93.1 | LYM 102 | 1222 2.1 | D | 9.50 6 | 3.77E-02 | 173.6 |
| LYM273 | 13721. 1 | G | 0.70 1 | 6.35E-04 | 58.1 | LYM 129 | 1257 2.2 | D | 4.35 | 3.96E-02 | 25.2 |
| LYM273 | 13721. 3 | G | 1.06 3 | 7.82E-04 | 139.6 | LYM10 | 1174 1.4 | D | 5.71 2 | 4.08E-02 | 64.4 |
| LYM122 | 13712. 3 | G | 0.63 2 | 8.84E-04 | 42.4 | LYM 129 | 1257 1.3 | D | 6.49 5 | 4.89E-02 | 87 |
| LYM251 | 13073. 8 | G | 0.87 9 | 1.02E-03 | 98.3 | LYM3 | 1204 3.2 | *D* | 4.15 2 | 5.04E-02 | 19.5 |
| LYM23 | 12783. 5 | G | 0.66 3 | 1.60E-03 | 49.5 | LYM10 | 1174 4.5 | D | 5.88 6 | 5.04E-02 | 69.4 |
| LYM164 | 12813. 5 | G | 0.82 4 | 3.06E-03 | 85.8 | LYM 132 | 1227 5.1 | D | 4.22 8 | 5.06E-02 | 21.7 |
| LYM249 | 13633. 1 | G | 0.94 4 | 3.80E-03 | 112.9 | LYM10 | 1174 1.2 | *D* | 4.70 1 | 5.08E-02 | 35.3 |
| LYM79 | 13132. 2 | G | 0.88 1 | 4.30E-03 | 98.7 | LYM268 | 1248 2.1 | D | 6.36 | 5.11E-02 | 83.1 |
| LYM217 | 13182. 1 | G | 0.79 7 | 5.62E-03 | 79.6 | LYM134 | 1231 2.3 | D | 4.74 2 | 5.15E-02 | 36.5 |
| LYM23 | 12783. 7 | G | 0.87 1 | 6.19E-03 | 96.5 | LYM102 | 1222 2.2 | D | 6.47 6 | 5.30E-02 | 86.4 |
| LYM 193 | 13484. 3 | G | 0.77 | 7.00E-03 | 73.6 | LYM268 | 1248 1.1 | D | 5.36 4 | 5.40E-02 | 54.4 |
| LYM249 | 13631. 1 | G | 0.69 6 | 7.39E-03 | 56.9 | LYM162 | 1223 4.4 | D | 4.15 4 | 5.73E-02 | 19.6 |
| LYM251 | 13072. 8 | G | 0.80 7 | 8.13E-03 | 82 | LYM290 | 1250 4.1 | D | 5.10 5 | 5.84E-02 | 47 |
| LYM122 | 13713. 2 | G | 0.72 1 | 8.93E-03 | 62.6 | LYM3 | 1204 1.1 | D | 4.53 8 | 6.02E-02 | 30.7 |
| LYM274 | 13414. 2 | G | 0.89 5 | 1.25E-02 | 101.9 | LYM132 | 1227 3.2 | D | 4.75 5 | 6.21E-02 | 36.9 |
| LYM224 | 13435. 5 | G | 0.54 9 | 1.36E-02 | 23.7 | LYM 106 | 1214 2.2 | D | 5.33 5 | 6.22E-02 | 53.6 |
| LYM79 | 13133. 3 | G | 0.65 2 | 1.38E-02 | 47.1 | LYM289 | 1249 1.4 | D | 5.47 2 | 6.26E-02 | 57.5 |
| LYM217 | 13181. 11 | G | 0.67 7 | 1.41E-02 | 52.7 | LYM3 | 1204 1.2 | D | 4.40 2 | 6.70E-02 | 26.7 |
| LYM273 | 13723. 1 | G | 0.82 5 | 1.50E-02 | 86 | LYM 102 | 1222 2.6 | D | 4.51 8 | 8.47E-02 | 30.1 |
| LYM122 | 13714. 4 | G | 0.93 9 | 1.71E-02 | 111.7 | LYM140 | 1226 2.3 | D | 4.82 6 | 8.91E-02 | 38.9 |
| LYM25 1 | 13073. 7 | G | 0.96 4 | 1.78E-02 | 117.4 | LYM129 | 1257 3.1 | D | 4.88 8 | 9.60E-02 | 40.7 |
| LYM274 | 13411. 2 | G | 0.53 | 2.47E-02 | 19.6 | CONTR OL | - - | D | 3.47 4 | - - | 0 |
| LYM245 | 13062. 5 | G | 0.74 2 | 2.75E-02 | 67.4 | LYM 129 | 1257 3.5 | E | 0.70 9 | 4.00E-06 | 62.2 |
| LYM23 | 12782. 6 | G | 0.79 4 | 2.84E-02 | 79.1 | LYM289 | 1249 3.6 | E | 0.69 7 | 7.00E-06 | 59.3 |
| LYM274 | 13413. 4 | G | 0.79 1 | 3.21E-02 | 78.4 | LYM 162 | 1223 1.2 | E | 0.65 9 | 7.90E-05 | 50.8 |
| LYM122 | 13712. 1 | G | 0.69 5 | 3.25E-02 | 56.7 | LYM 102 | 1222 2.3 | E | 0.67 5 | 8.60E-05 | 54.3 |
| LYM245 | 13061. 6 | G | 0.82 | 3.44E-02 | 84.9 | LYM290 | 1250 2.4 | E | 0.63 2 | 2.93E-04 | 44.5 |
| LYM23 | 12784. 6 | G | 0.75 2 | 3.82E-02 | 69.6 | LYM148 | 1217 3.5 | E | 0.62 5 | 5.90E-04 | 43 |
| LYM251 | 13072. 6 | G | 0.90 7 | 3.90E-02 | 104.5 | LYM 102 | 1222 2.1 | E | 0.63 8 | 7.08E-04 | 46 |
| LYM251 | 13074. 6 | G | 0.64 3 | 3.93E-02 | 45.1 | LYM290 | 1250 1.2 | E | 0.62 2 | 7.15E-04 | 42.1 |
| LYM40 | 13732. 2 | G | 0.56 3 | 4.57E-02 | 27 | LYM268 | 1248 2.3 | E | 0.61 3 | 1.36E-03 | 40.2 |
| LYM23 | 12782. 7 | G | 0.54 1 | 4.89E-02 | 22 | LYM10 | 1174 4.5 | E | 0.60 7 | 1.97E-03 | 38.8 |
| LYM217 | 13181. 7 | G | 0.56 2 | 5.20E-02 | 26.7 | LYM148 | 1217 2.1 | E | 0.6 | 2.71E-03 | 37.1 |
| LYM217 | 13183. 2 | G | 0.91 3 | 5.21E-02 | 106 | LYM 148 | 1217 4.1 | E | 0.59 1 | 3.47E-03 | 35.1 |
| LYM164 | 12813. 8 | G | 0.65 | 5.77E-02 | 46.5 | LYM 140 | 1226 1.4 | E | 0.59 | 4.44E-03 | 34.9 |
| LYM273 | 13722. 3 | G | 0.63 9 | 5.87E-02 | 44.1 | LYM268 | 1248 3.4 | E | 0.59 5 | 4.63E-03 | 36.1 |
| LYM224 | 13435. 1 | G | 0.92 7 | 5.89E-02 | 108.9 | LYM268 | 1248 3.2 | E | 0.58 6 | 5.11E-03 | 34.1 |
| LYM217 | 13181. 6 | G | 0.62 2 | 6.07E-02 | 40.2 | LYM113 | 1244 3.1 | E | 0.58 | 7.96E-03 | 32.6 |
| LYM274 | 13415. 2 | G | 0.71 8 | 6.4 1E-02 | 61.8 | LYM289 | 1249 1.1 | E | 0.57 2 | 1.03E-02 | 30.8 |
| LYM240 | 12682. 1 | G | 0.808 | 6.42E-02 | 82.2 | LYM289 | 1249 2.2 | E | 0.58 2 | 1.07E-02 | 33.1 |
| LYM79 | 13133. 1 | G | 0.60 4 | 6.96E-02 | 36.2 | LYM132 | 1227 3.2 | E | 0.56 6 | 1.31E-02 | 29.5 |
| LYM224 | 13434. 2 | G | 0.64 3 | 7.21E-02 | 44.9 | LYM290 | 1250 1.3 | E | 0.56 6 | 1.39E-02 | 29.4 |
| LYM 122 | 13714. 2 | G | 0.78 | 8.03E-02 | 75.9 | LYM106 | 1214 2.2 | E | 0.56 5 | 1.55E-02 | 29.3 |
| LYM245 | 13064. 8 | G | 0.56 9 | 8.26E-02 | 28.3 | LYM10 | 1174 2.2 | E | 0.56 8 | 1.60E-02 | 30 |
| LYM40 | 13732. 1 | G | 0.68 | 8.52E-02 | 53.3 | LYM13 | 1177 4.1 | E | 0.56 2 | 1.77E-02 | 28.5 |
| LYM249 | 13631. 2 | G | 0.80 4 | 8.60E-02 | 81.3 | LYM113 | 1244 4.4 | E | 0.55 4 | 2.37E-02 | 26.7 |
| LYM23 | 12783. 8 | G | 0.5 | 8.97E-02 | 12.7 | LYM 134 | 1231 1.2 | E | 0.55 3 | 2.63E-02 | 26.4 |
| LYM79 | 13134. 3 | G | 0.55 7 | 9.71E-02 | 25.7 | LYM10 | 1174 1.4 | E | 0.55 4 | 2.69E-02 | 26.7 |
| CONTR OL | - | G | 0.44 3 | - - | 0 | LYM10 | 1174 1.2 | E | 0.54 8 | 3.19E-02 | 25.4 |
| LYM122 | 13714. 4 | H | 0.09 5 | 0.00E +00 | 118.7 | LYM132 | 1227 5.1 | E | 0.55 1 | 3.90E-02 | 26 |
| LYM 122 | 13713. 2 | H | 0.07 2 | 0.00E +00 | 66.2 | LYM132 | 1227 6.1 | E | 0.55 | 3.90E-02 | 25.8 |
| LYM 122 | 13712. 3 | H | 0.06 4 | 0.00E +00 | 47 | LYM290 | 1250 4.1 | E | 0.54 8 | 4.08E-02 | 25.2 |
| LYM164 | 12813. 5 | H | 0.08 | 0.00E +00 | 84.1 | LYM129 | 1257 1.3 | E | 0.54 9 | 4.67E-02 | 25.5 |
| LYM 193 | 13484. 3 | H | 0.07 3 | 0.00E +00 | 67 | LYM289 | 1249 3.2 | E | 0.54 1 | 5.42E-02 | 23.7 |
| LYM217 | 13183. 2 | H | 0.08 9 | 0.00E +00 | 104.8 | LYM289 | 1249 1.4 | E | 0.54 3 | 5.79E-02 | 24.1 |
| LYM217 | 13182. 1 | H | 0.08 4 | 0.00E +00 | 93 | LYM113 | 1244 2.1 | E | 0.54 | 6.19E-02 | 23.4 |
| LYM217 | 13181. 11 | H | 0.06 9 | 0.00E +00 | 58.8 | LYM102 | 1222 2.2 | E | 0.54 1 | 6.54E-02 | 23.6 |
| LYM224 | 13435. 3 | H | 0.07 | 0.00E +00 | 60.5 | LYM134 | 1231 4.2 | E | 0.53 | 7.02E-02 | 21.2 |
| LYM23 | 12783. 7 | H | 0.08 9 | 0.00E +00 | 104 | LYM 102 | 1222 2.6 | E | 0.52 4 | 9.02E-02 | 19.9 |
| LYM23 | 12782. 6 | H | 0.08 2 | 0.00E +00 | 89 | LYM140 | 1226 2.3 | E | 0.53 1 | 9.82E-02 | 21.4 |
| LYM23 | 12784. 6 | H | 0.07 3 | 0.00E +00 | 68.3 | CONTR OL | - - | E | 0.43 7 | - - | 0 |
| LYM23 | 12783. 5 | H | 0.06 6 | 0.00E +00 | 51.8 | LYM 129 | 1257 3.5 | F | 7.52 9 | 0.00E +00 | 48.8 |
| LYM245 | 13061. 6 | H | 0.08 1 | 0.00E +00 | 85.2 | LYM148 | 1217 4.1 | F | 6.57 6 | 0.00E +00 | 30 |
| LYM245 | 13062. 5 | H | 0.07 8 | 0.00E +00 | 79.4 | LYM 102 | 1222 2.6 | F | 6.33 1 | 1.00E-06 | 25.2 |
| LYM249 | 13633. 1 | H | 0.09 8 | 0.00E +00 | 123.9 | LYM134 | 1231 1.2 | F | 6.31 4 | 1.00E-06 | 24.8 |
| LYM249 | 13631. 1 | H | 0.06 8 | 0.00E +00 | 56.9 | LYM290 | 1250 2.4 | F | 6.93 4 | 3.00E-06 | 37.1 |
| LYM251 | 13073. 7 | H | 0.09 7 | 0.00E +00 | 122.4 | LYM132 | 1227 3.2 | F | 6.46 8 | 8.00E-06 | 27.9 |
| LYM25 1 | 13073. 8 | H | 0.08 8 | 0.00E +00 | 102.1 | LYM289 | 1249 1.1 | F | 6.65 7 | 2.20E-05 | 31.6 |
| LYM251 | 13072. 6 | H | 0.08 7 | 0.00E +00 | 100.7 | LYM290 | 1250 1.3 | F | 6.05 9 | 4.00E-05 | 19.8 |
| LYM251 | 13072. 8 | H | 0.08 7 | 0.00E +00 | 99.1 | LYM134 | 1231 4.2 | F | 6.20 4 | 7.70E-05 | 22.6 |
| LYM251 | 13074. 6 | H | 0.06 8 | 0.00E +00 | 56.4 | LYM 148 | 1217 3.1 | F | 6.77 | 1.40E-04 | 33.8 |
| LYM273 | 13721. 3 | H | 0.10 6 | 0.00E +00 | 143.3 | LYM13 | 1177 4.1 | F | 6.45 9 | 1.53E-04 | 27.7 |
| LYM273 | 13723. 1 | H | 0.08 9 | 0.00E +00 | 104 | LYM290 | 1250 1.2 | F | 6.82 3 | 1.66E-04 | 34.9 |
| LYM273 | 13722. 4 | H | 0.08 1 | 0.00E +00 | 85.6 | LYM113 | 1244 4.4 | F | 6.32 5 | 1.73E-04 | 25 |
| LYM273 | 13721. 1 | H | 0.07 4 | 0.00E +00 | 70.2 | LYM10 | 1174 4.5 | F | 7.04 4 | 1.83E-04 | 39.3 |
| LYM273 | 13722. 3 | H | 0.06 7 | 0.00E +00 | 54.8 | LYM268 | 1248 3.2 | F | 6.83 | 1.89E-04 | 35 |
| LYM274 | 13414. 2 | H | 0.09 4 | 0.00E +00 | 115.6 | LYM289 | 1249 2.2 | F | 6.92 6 | 2.40E-04 | 36.9 |
| LYM274 | 13413. 4 | H | 0.07 8 | 0.00E +00 | 78.9 | LYM289 | 1249 3.6 | F | 7.10 8 | 4.29E-04 | 40.5 |
| LYM79 | 13132. 2 | H | 0.09 5 | 0.00E +00 | 117 | LYM 148 | 1217 2.1 | F | 6.75 4 | 6.38E-04 | 33.5 |
| LYM79 | 13133. 3 | H | 0.07 | 0.00E +00 | 60.2 | LYM268 | 1248 2.3 | F | 6.81 4 | 7.13E-04 | 34.7 |
| LYM217 | 13181. 6 | H | 0.06 6 | 1.00E-06 | 51.9 | LYM113 | 1244 3.1 | F | 6.92 5 | 9.38E-04 | 36.9 |
| LYM249 | 13631. 2 | H | 0.08 5 | 1.00E-06 | 95.9 | LYM 140 | 1226 1.4 | F | 6.84 7 | 1.17E-03 | 35.4 |
| LYM274 | 13415. 2 | H | 0.07 4 | 1.00E-06 | 69.9 | LYM148 | 1217 3.5 | F | 6.56 8 | 1.48E-03 | 29.8 |
| LYM79 | 13133. 1 | H | 0.06 4 | 1.00E-06 | 47.7 | LYM113 | 1244 4.5 | F | 6.07 9 | 1.72E-03 | 20.2 |
| LYM164 | 12813. 8 | H | 0.06 7 | 2.00E-06 | 54 | LYM 102 | 1222 2.1 | F | 7.22 6 | 2.12E-03 | 42.8 |
| LYM224 | 13435. 1 | H | 0.08 9 | 2.00E-06 | 104.8 | LYM10 | 1174 1.2 | F | 5.97 6 | 2.26E-03 | 18.1 |
| LYM240 | 12682. 1 | H | 0.08 2 | 2.00E-06 | 89 | LYM 106 | 1214 2.2 | F | 6.15 8 | 2.57E-03 | 21.7 |
| LYM122 | 13714. 2 | H | 0.07 7 | 5.00E-06 | 77.8 | LYM 162 | 1223 1.2 | F | 6.89 9 | 2.78E-03 | 36.4 |
| LYM40 | 13734. 2 | H | 0.05 6 | 5.00E-06 | 28.3 | LYM10 | 1174 1.4 | F | 6.12 8 | 3.19E-03 | 21.2 |
| LYM122 | 13712. 1 | H | 0.06 3 | 1.70E-05 | 43.7 | LYM148 | 1217 1.2 | F | 6.27 6 | 4.06E-03 | 24.1 |
| LYM224 | 13434. 2 | H | 0.06 4 | 2.70E-05 | 47.3 | LYM 162 | 1223 4.3 | F | 6.65 1 | 5.16E-03 | 31.5 |
| LYM40 | 13732. 1 | H | 0.06 8 | 3.00E-05 | 55.4 | LYM268 | 1248 3.4 | F | 6.74 6 | 5.50E-03 | 33.4 |
| LYM217 | 13181. 7 | H | 0.05 7 | 9.40E-05 | 31.2 | LYM 102 | 1222 2.3 | F | 7.19 8 | 7.29E-03 | 42.3 |
| LYM79 | 13134. 3 | H | 0.05 7 | 1.09E-04 | 31.8 | LYM289 | 1249 3.2 | F | 6.30 5 | 7.70E-03 | 24.6 |
| LYM260 | 13095. 7 | H | 0.06 3 | 1.15E-04 | 45.5 | LYM113 | 1244 2.1 | F | 6.43 8 | 1.27E-02 | 27.3 |
| LYM23 | 12782. 7 | H | 0.05 6 | 1.57E-04 | 28 | LYM 102 | 1222 2.2 | F | 6.67 7 | 1.28E-02 | 32 |
| LYM40 | 13732. 2 | H | 0.05 6 | 1.93E-04 | 28.4 | LYM290 | 1250 4.1 | F | 6.29 6 | 1.34E-02 | 24.5 |
| LYM131 | 12791. 5 | H | 0.06 | 3.33E-04 | 37.3 | LYM132 | 1227 5.1 | F | 6.32 2 | 1.46E-02 | 25 |
| LYM245 | 13064. 8 | H | 0.05 7 | 4.25E-04 | 29.9 | LYM132 | 1227 5.3 | F | 5.86 3 | 1.47E-02 | 15.9 |
| LYM274 | 13411. 2 | H | 0.05 3 | 6.16E-04 | 22.6 | LYM132 | 1227 6.1 | F | 6.53 6 | 1.48E-02 | 29.2 |
| LYM79 | 13134. 2 | H | 0.05 4 | 151E-03 | 24.6 | LYM106 | 1214 2.1 | F | 6.22 | 1.59E-02 | 23 |
| LYM260 | 13095.1 | H | 0.07 | 2.31E-03 | 61.7 | LYM10 | 1174 2.2 | F | 6.22 6 | 1.66E-02 | 23.1 |
| LYM23 | 12783. 8 | H | 0.05 2 | 2.84E-03 | 18.8 | LYM 106 | 1214 1.4 | F | 5.81 1 | 2.02E-02 | 14.9 |
| LYM274 | 13413. 1 | H | 0.05 5 | 9.12E-03 | 25.3 | LYM 162 | 1223 1.3 | F | 6.50 8 | 2.18E-02 | 28.7 |
| LYM249 | 13631. 4 | H | 0.05 1 | 9.60E-03 | 17.4 | LYM129 | 1257 1.3 | F | 6.49 9 | 3.26E-02 | 28.5 |
| LYM164 | 12814. 8 | H | 0.06 1 | 1.06E-02 | 39.9 | LYM13 | 1177 2.2 | F | 6.13 | 3.39E-02 | 21.2 |
| LYM224 | 13435. 5 | H | 0.05 | 1.25E-02 | 15.3 | LYM132 | 1227 1.4 | F | 5.81 4 | 4.00E-02 | 14.9 |
| LYM131 | 12791. 8 | H | 0.06 4 | 1.66E-02 | 47.4 | LYM13 | 1177 2.1 | F | 5.74 3 | 6.50E-02 | 13.5 |
| LYM 193 | 13482. 4 | H | 0.05 5 | 2.52E-02 | 26.1 | LYM129 | 1257 3.1 | F | 5.78 3 | 6.75E-02 | 14.3 |
| LYM260 | 13095. 8 | H | 0.05 2 | 4.62E-02 | 19.8 | LYM13 | 1177 3.2 | F | 5.50 5 | 6.85E-02 | 8.8 |
| LYM224 | 13432. 1 | H | 0.04 9 | 4.71E-02 | 13 | LYM3 | 1204 2.1 | F | 5.67 3 | 6.99E-02 | 12.1 |
| CONTR OL | - | H | 0.04 4 | - - | 0 | LYM268 | 1248 1.1 | F | 6.05 6 | 7.44E-02 | 19.7 |
| LYM122 | 13711. 3 | A | 0.01 1 | 1.00E-06 | 231.7 | LYM 134 | 1231 2.4 | F | 6.02 8 | 9.99E-02 | 19.2 |
| LYM234 | 14093. 2 | A | 0.01 | 2.10E-05 | 192.5 | CONTR OL | - | F | 5.05 8 | - | 0 |
| LYM 189 | 13315. 2 | A | 0.01 | 5.08E-04 | 197.1 | LYM134 | 1231 2.4 | G | 0.70 4 | 0.00E +00 | 57.1 |
| LYM217 | 13183. 2 | A | 0.00 8 | 5.11E-04 | 130.3 | LYM140 | 1226 1.2 | G | 0.63 6 | 0.00E +00 | 42 |
| LYM189 | 13314. 5 | A | 0.00 5 | 9.45E-04 | 67.4 | LYM162 | 1223 4.3 | G | 0.91 2 | 1.00E-06 | 103.5 |
| LYM79 | 13134. 3 | A | 0.00 5 | 9.97E-04 | 62 | LYM290 | 1250 2.4 | G | 0.66 7 | 1.30E-05 | 48.9 |
| LYM161 | 13233. 5 | A | 0.00 7 | 1.28E-03 | 124.2 | LYM162 | 1223 4.4 | G | 0.75 | 5.67E-04 | 67.5 |
| LYM217 | 13186. 1 | A | 0.01 2 | 1.32E-03 | 275.4 | LYM 140 | 1226 2.2 | G | 0.90 3 | 6.14E-04 | 101.7 |
| LYM217 | 13181. 9 | A | 0.00 8 | 1.81E-03 | 134.2 | LYM132 | 1227 6.1 | G | 0.82 1 | 1.92E-03 | 83.4 |
| LYM32 | 13964. 1 | A | 0.00 8 | 2.09E-03 | 144.9 | LYM140 | 1226 1.4 | G | 0.54 3 | 2.25E-03 | 21.2 |
| LYM234 | 14092. 5 | A | 0.01 2 | 2.20E-03 | 277 | LYM13 | 1177 2.2 | G | 0.63 4 | 3.99E-03 | 41.6 |
| LYM240 | 12683. 5 | A | 0.01 | 2.37E-03 | 192.5 | LYM289 | 1249 2.2 | G | 0.88 | 4.21E-03 | 96.5 |
| LYM219 | 13332. 9 | A | 0.01 1 | 3.78E-03 | 233.2 | LYM 132 | 1227 1.4 | G | 0.60 9 | 4.24E-03 | 36 |
| LYM278 | 13364. 5 | A | 0.00 4 | 3.79E-03 | 24.4 | LYM289 | 1249 1.1 | G | 0.69 1 | 5.40E-03 | 54.3 |
| LYM245 | 13061. 8 | A | 0.01 3 | 3.90E-03 | 306.9 | LYM268 | 1248 2.1 | G | 0.93 6 | 6.27E-03 | 108.9 |
| LYM234 | 14092. 1 | A | 0.00 5 | 5.44E-03 | 42 | LYM3 | 1204 1.2 | G | 0.66 1 | 6.62E-03 | 47.6 |
| LYM74 | 13954. 1 | A | 0.01 4 | 8.73E-03 | 315.4 | LYM 162 | 1223 1.3 | G | 0.74 8 | 7.40E-03 | 67 |
| LYM74 | 13954. 2 | A | 0.00 9 | 1.17E-02 | 175.6 | LYM268 | 1248 3.4 | G | 0.71 7 | 7.99E-03 | 60.2 |
| LYM 188 | 13244. 6 | A | 0.00 5 | 1.21E-02 | 53.6 | LYM290 | 1250 4.1 | G | 0.56 2 | 8.97E-03 | 25.4 |
| LYM 189 | 13311. 3 | A | 0.00 9 | 1.35E-02 | 186.4 | LYM3 | 1204 3.2 | G | 0.63 5 | 9.85E-03 | 41.8 |
| LYM 189 | 13311. 2 | A | 0.01 | 1.83E-02 | 194.8 | LYM 148 | 1217 3.1 | G | 0.84 9 | 1.05E-02 | 89.5 |
| LYM274 | 13415. 2 | A | 0.01 2 | 1.98E-02 | 273.9 | LYM 13 | 1177 4.1 | G | 0.78 9 | 1.16E-02 | 76.1 |
| LYM79 | 13132. 2 | A | 0.01 3 | 2.07E-02 | 300 | LYM 140 | 1226 1.1 | G | 0.52 1 | 1.27E-02 | 16.3 |
| LYM217 | 13182. 1 | A | 0.01 2 | 2.10E-02 | 269.3 | LYM 106 | 1214 2.1 | G | 0.91 9 | 1.37E-02 | 105.3 |
| LYM240 | 12682. 1 | A | 0.01 2 | 2.22E-02 | 264.7 | LYM134 | 1231 2.3 | G | 0.78 1 | 1.42E-02 | 74.4 |
| LYM 189 | 13315. 1 | A | 0.01 7 | 2.23E-02 | 422.8 | LYM3 | 1204 2.1 | G | 0.69 3 | 2.41E-02 | 54.8 |
| LYM 188 | 13244. 7 | A | 0.00 6 | 2.61E-02 | 87.3 | LYM113 | 1244 2.1 | G | 0.70 2 | 2.51E-02 | 56.8 |
| LYM79 | 13133. 2 | A | 0.00 9 | 3.08E-02 | 161.8 | LYM 102 | 1222 2.3 | G | 1.04 | 2.56E-02 | 132.2 |
| LYM79 | 13133. 3 | A | 0.00 9 | 3.18E-02 | 183.3 | LYM113 | 1244 4.5 | G | 0.67 2 | 3.11E-02 | 50 |
| LYM189 | 13314. 4 | A | 0.00 9 | 3.27E-02 | 167.9 | LYM289 | 1249 3.2 | G | 0.74 7 | 3.12E-02 | 66.7 |
| LYM188 | 13244. 4 | A | 0.01 | 3.40E-02 | 220.2 | LYM 129 | 1257 3.5 | G | 0.69 | 3.20E-02 | 54.1 |
| LYM79 | 13134. 1 | A | 0.01 | 3.50E-02 | 194.8 | LYM113 | 1244 4.4 | G | 0.64 7 | 3.21E-02 | 44.4 |
| LYM274 | 13413. 1 | A | 0.00 9 | 3.63E-02 | 166.4 | LYM 134 | 1231 1.2 | G | 0.70 2 | 3.87E-02 | 56.7 |
| LYM234 | 14092. 8 | A | 0.01 2 | 3.81E-02 | 268.5 | LYM 129 | 1257 3.1 | G | 0.58 2 | 3.90E-02 | 30 |
| LYM74 | 13952. 2 | A | 0.00 9 | 3.97E-02 | 181 | LYM132 | 1227 5.3 | G | 0.61 4 | 3.97E-02 | 37 |
| LYM32 | 13963. 4 | A | 0.00 8 | 4.02E-02 | 151.8 | LYM113 | 1244 3.1 | G | 0.86 5 | 4.24E-02 | 93.2 |
| LYM274 | 13413. 4 | A | 0.00 9 | 4.20E-02 | 169.5 | LYM 102 | 1222 2.1 | G | 1.12 4 | 4.88E-02 | 151 |
| LYM234 | 14091. 1 | A | 0.00 8 | 4.79E-02 | 131.1 | LYM 106 | 1214 4.4 | G | 0.63 8 | 5.62E-02 | 42.4 |
| LYM161 | 13233. 6 | A | 0.01 | 5.45E-02 | 220.9 | LYM 102 | 1222 2.2 | G | 0.88 7 | 6.97E-02 | 98.1 |
| LYM240 | 12683. 9 | A | 0.00 4 | 5.60E-02 | 28.2 | LYM148 | 1217 2.1 | G | 0.64 9 | 7.46E-02 | 44.8 |
| LYM79 | 13131. 1 | A | 0.00 7 | 6.26E-02 | 116.5 | LYM 102 | 1222 1.1 | G | 0.74 4 | 7.72E-02 | 66. 1 |
| LYM32 | 13963. 1 | A | 0.00 5 | 6.95E-02 | 49.7 | LYM10 | 1174 4.5 | G | 0.55 6 | 8.52E-02 | 24.1 |
| LYM273 | 13721. 3 | A | 0.00 6 | 7.08E-02 | 80.4 | LYM290 | 1250 1.2 | G | 0.66 4 | 9.90E-02 | 48.2 |
| LYM79 | 13134. 2 | A | 0.00 4 | 7.89E-02 | 32.8 | CONTR OL | - | G | 0.44 8 | - | 0 |
| LYM217 | 13181. 6 | A | 0.00 5 | 8.18E-02 | 61.2 | LYM102 | 1222 2.1 | H | 0.11 8 | 0.00E +00 | 160.6 |
| LYM122 | 13713. 4 | A | 0.00 5 | 9.83E-02 | 51.2 | LYM 102 | 1222 2.3 | H | 0.11 3 | 0.00E +00 | 149.3 |
| CONTR OL | - | A | 0.00 3 | - | 0 | LYM106 | 1214 2.1 | H | 0.09 1 | 0.00E +00 | 101.4 |
| LYM161 | 13233. 5 | B | 0.14 6 | 0.00E +00 | 123.8 | LYM132 | 1227 6.1 | H | 0.08 4 | 0.00E +00 | 85.3 |
| LYM 189 | 13314. 5 | B | 0.10 3 | 0.00E +00 | 57.6 | LYM 140 | 1226 2.2 | H | 0.08 8 | 0.00E +00 | 94.1 |
| LYM234 | 14093. 2 | B | 0.18 1 | 3.00E-06 | 177.1 | LYM148 | 1217 3.1 | H | 0.08 4 | 0.00E +00 | 86.3 |
| LYM32 | 13964. 1 | B | 0.14 5 | 5.12E-04 | 122.8 | LYM 162 | 1223 4.3 | H | 0.09 1 | 0.00E +00 | 100.3 |
| LYM234 | 14092. 5 | B | 0.21 9 | 6.15E-04 | 235.5 | LYM268 | 1248 2.1 | H | 0.09 4 | 0.00E +00 | 108.2 |
| LYM 122 | 13711. 3 | B | 0.22 5 | 6.83E-04 | 245.3 | LYM289 | 1249 2.2 | H | 0.09 2 | 0.00E +00 | 104 |
| LYM240 | 12683. 5 | B | 0.17 9 | 8.14E-04 | 173.6 | LYM13 | 1177 4.1 | H | 0.07 9 | 1.00E-06 | 74.4 |
| LYM245 | 13061. 8 | B | 0.24 | 1.32E-03 | 268.1 | LYM162 | 1223 4.4 | H | 0.07 4 | 3.00E-06 | 64.8 |
| LYM217 | 13186. 1 | B | 0.19 7 | 1.61E-03 | 202.2 | LYM268 | 1248 3.4 | H | 0.07 6 | 3.00E-06 | 68.5 |
| LYM217 | 13183. 2 | B | 0.14 4 | 1.66E-03 | 120.5 | LYM134 | 1231 2.3 | H | 0.07 7 | 4.00E-06 | 70 |
| LYM189 | 13315. 2 | B | 0.21 2 | 4.60E-03 | 224.4 | LYM113 | 1244 3.1 | H | 0.08 7 | 5.00E-06 | 91.5 |
| LYM219 | 13332. 9 | B | 0.19 4 | 5.69E-03 | 197.9 | LYM162 | 1223 1.3 | H | 0.07 4 | 8.00E-06 | 63.1 |
| LYM74 | 13954. 2 | B | 0.15 7 | 7.07E-03 | 141.3 | LYM290 | 1250 2.4 | H | 0.07 1 | 1.30E-05 | 57.2 |
| LYM217 | 13182. 1 | B | 0.23 1 | 7.34E-03 | 253.5 | LYM129 | 1257 3.5 | H | 0.07 5 | 2.20E-05 | 66.3 |
| LYM234 | 14092. 1 | B | 0.08 7 | 7.90E-03 | 33 | LYM134 | 1231 2.4 | H | 0.06 9 | 3.10E-05 | 52.5 |
| LYM278 | 13364. 5 | B | 0.07 6 | 8.29E-03 | 17 | LYM 102 | 1222 2.2 | H | 0.08 7 | 7.00E-05 | 91.6 |
| LYM74 | 13954. 1 | B | 0.24 8 | 8.32E-03 | 280 | LYM289 | 1249 1.1 | H | 0.06 9 | 9.40E-05 | 53.2 |
| LYM217 | 13181. 9 | B | 0.15 1 | 8.57E-03 | 130.7 | LYM113 | 1244 4.4 | H | 0.07 1 | 1.12E-04 | 56.9 |
| LYM189 | 13311. 2 | B | 0.17 4 | 8.79E-03 | 166.7 | LYM 134 | 1231 1.2 | H | 0.07 1 | 1.62E-04 | 57.5 |
| LYM32 | 13963. 4 | B | 0.16 3 | 8.96E-03 | 149.3 | LYM102 | 1222 1.1 | H | 0.07 6 | 2.25E-04 | 68.8 |
| LYM240 | 12683. 9 | B | 0.07 7 | 9.39E-03 | 18.7 | LYM289 | 1249 3.2 | H | 0.07 1 | 2.37E-04 | 57.5 |
| LYM 189 | 13311. 3 | B | 0.17 6 | 1.20E-02 | 169 | LYM3 | 1204 2.1 | H | 0.06 8 | 4.18E-04 | 49.7 |
| LYM79 | 13134. 1 | B | 0.17 3 | 1.36E-02 | 165.4 | LYM113 | 1244 2.1 | H | 0.06 7 | 7.34E-04 | 49.1 |
| LYM79 | 13133. 3 | B | 0.16 9 | 1.42E-02 | 158.3 | LYM 148 | 1217 2.1 | H | 0.06 8 | 8.70E-04 | 50.9 |
| LYM240 | 12682. 1 | B | 0.20 2 | 1.49E-02 | 209.5 | LYM129 | 1257 1.3 | H | 0.07 | 1.22E-03 | 54.8 |
| LYM79 | 13132. 2 | B | 0.23 7 | 1.69E-02 | 262.6 | LYM113 | 1244 4.5 | H | 0.06 6 | 1.82E-03 | 45.3 |
| LYM188 | 13244. 7 | B | 0.11 6 | 1.77E-02 | 77.9 | LYM290 | 1250 1.2 | H | 0.06 8 | 1.94E-03 | 50 |
| LYM188 | 13244. 6 | B | 0.10 2 | 2.08E-02 | 56.2 | LYM3 | 1204 1.2 | H | 0.06 3 | 2.54E-03 | 38.7 |
| LYM189 | 13315. 1 | B | 0.28 1 | 2.09E-02 | 330 | LYM140 | 1226 1.2 | H | 0.06 1 | 4.70E-03 | 34.5 |
| LYM79 | 13134. 2 | B | 0.08 7 | 2. 10E-02 | 33.3 | LYM3 | 1204 3.2 | H | 0.06 1 | 6.01E-03 | 35.8 |
| LYM 189 | 13314. 4 | B | 0.15 8 | 2.19E-02 | 141.4 | LYM132 | 1227 5.3 | H | 0.06 1 | 9.29E-03 | 34.2 |
| LYM 188 | 13244. 4 | B | 0.19 6 | 2.23E-02 | 200.6 | LYM13 | 1177 2.2 | H | 0.06 | 9.79E-03 | 32.3 |
| LYM161 | 13233. 6 | B | 0.18 5 | 2.28E-02 | 183.3 | LYM106 | 1214 1.4 | H | 0.06 3 | 1.25E-02 | 39.7 |
| LYM274 | 13415. 2 | B | 0.21 7 | 2.29E-02 | 232.4 | LYM 106 | 1214 4.4 | H | 0.06 | 1.94E-02 | 32.6 |
| LYM274 | 13413. 4 | B | 0.15 2 | 2.34E-02 | 133.3 | LYM268 | 1248 1.1 | H | 0.06 1 | 2.01E-02 | 34.4 |
| LYM234 | 14091. 1 | B | 0.14 9 | 2.38E-02 | 129 | LYM 134 | 1231 4.2 | H | 0.05 9 | 2.35E-02 | 29.9 |
| LYM79 | 13133. 2 | B | 0.15 6 | 2.45E-02 | 139.7 | LYM 129 | 1257 3.1 | H | 0.05 7 | 3.09E-02 | 27.1 |
| LYM79 | 13134. 3 | B | 0.10 9 | 2.50E-02 | 67.5 | LYM132 | 1227 1.4 | H | 0.05 7 | 3.88E-02 | 25.2 |
| LYM273 | 13721. 3 | B | 0.11 2 | 2.82E-02 | 71.3 | LYM113 | 1244 2.2 | H | 0.05 9 | 3.90E-02 | 30 |
| LYM274 | 13413. 1 | B | 0.16 7 | 3.18E-02 | 156.6 | LYM10 | 1174 4.5 | H | 0.05 6 | 5.50E-02 | 24.8 |
| LYM217 | 13181. 6 | B | 0.1 | 3.59E-02 | 53.3 | LYM290 | 1250 4.1 | H | 0.05 6 | 5.52E-02 | 23 |
| LYM234 | 14092. 8 | B | 0.20 8 | 3.90E-02 | 219.4 | LYM129 | 1257 2.2 | H | 0.05 5 | 9.34E-02 | 21.4 |
| LYM32 | 13963. 1 | B | 0.09 3 | 5.12E-02 | 42.1 | LYM289 | 1249 1.4 | H | 0.05 6 | 9.88E-02 | 23.2 |
| LYM74 | 13952. 2 | B | 0.15 4 | 5.75E-02 | 136.2 | CONTR OL | - | H | 0.04 5 | - | 0 |
| LYM79 | 13131. 1 | B | 0.15 2 | 6.99E-02 | 133.1 | LYM248 | 1350 2.6 | A | 0.00 4 | 1.00E-05 | 49 |
| LYM161 | 13234. 6 | B | 0.13 6 | 7.51E-02 | 108.5 | LYM79 | 1313 4.7 | A | 0.00 4 | 6.75E-04 | 67 |
| LYM219 | 13334. 8 | B | 0.11 4 | 7.99E-02 | 74.8 | LYM157 | 1334 1.4 | A | 0.00 6 | 1.12E-03 | 127 |
| LYM219 | 13331. 1 | B | 0.14 9 | 9.01E-02 | 127.7 | LYM 180 | 1344 2.7 | A | 0.00 4 | 1.29E-03 | 75 |
| CONTR OL | - | B | 0.06 5 | - | 0 | LYM 180 | 1344 2.6 | A | 0.00 4 | 2.39E-03 | 78 |
| LYM 122 | 13711. 3 | C | 0.94 7 | 0.00E +00 | 73.9 | LYM52 | 1289 4.6 | A | 0.00 4 | 3.08E-03 | 50 |
| LYM161 | 13233. 6 | C | 1.00 1 | 0.00E +00 | 83.9 | LYM157 | 1334 1.1 | A | 0.00 7 | 3.26E-03 | 182 |
| LYM 189 | 13315. 1 | C | 1.28 1 | 0.00E +00 | 135.3 | LYM79 | 1313 1.6 | A | 0.00 6 | 3.45E-03 | 146 |
| LYM 189 | 13315. 2 | C | 1.27 8 | 0.00E +00 | 134.6 | LYM213 | 1284 2.8 | A | 0.00 9 | 5.95E-03 | 260 |
| LYM189 | 13311. 2 | C | 1.04 3 | 0.00E +00 | 91.5 | LYM157 | 1334 1.3 | A | 0.01 3 | 6.38E-03 | 420 |
| LYM219 | 13332. 9 | C | 1.16 9 | 0.00E +00 | 114.6 | LYM 120 | 1338 2.2 | A | 0.00 7 | 1.08E-02 | 192 |
| LYM234 | 14092. 5 | C | 1.19 6 | 0.00E +00 | 119.5 | LYM213 | 1284 2.9 | A | 0.00 8 | 1.10E-02 | 235 |
| LYM240 | 12683. 5 | C | 1.11 4 | 0.00E +00 | 104.6 | LYM117 | 1362 4.7 | A | 0.00 4 | 1.29E-02 | 59 |
| LYM240 | 12682. 1 | C | 1.05 3 | 0.00E +00 | 93.4 | LYM79 | 1313 2.6 | A | 0.00 4 | 1.41E-02 | 64 |
| LYM274 | 13413. 4 | C | 1.20 5 | 0.00E +00 | 121.3 | LYM52 | 1289 5.7 | A | 0.00 9 | 1.45E-02 | 262 |
| LYM274 | 13415. 2 | C | 1.10 8 | 0.00E +00 | 103.4 | LYM 107 | 1263 1.1 | A | 0.00 7 | 1.50E-02 | 189 |
| LYM32 | 13964. 1 | C | 1.00 1 | 0.00E +00 | 83.9 | LYM 120 | 1338 2.8 | A | 0.00 7 | 1.53E-02 | 160 |
| LYM74 | 13954. 1 | C | 1.34 6 | 0.00E +00 | 147.2 | LYM120 | 1338 2.1 | A | 0.00 6 | 1.71E-02 | 157 |
| LYM74 | 13954. 2 | C | 1.13 3 | 0.00E +00 | 108.1 | LYM213 | 1284 1.8 | A | 0.00 5 | 1.96E-02 | 114 |
| LYM79 | 13132. 2 | C | 1.06 6 | 0.00E +00 | 95.8 | LYM227 | 1454 2.1 | A | 0.00 4 | 2.27E-02 | 43 |
| LYM79 | 13134. 1 | C | 1.01 8 | 0.00E +00 | 86.9 | LYM120 | 1338 2.3 | A | 0.00 8 | 2.67E-02 | 225 |
| LYM217 | 13181. 6 | C | 0.97 4 | 1.00E-06 | 78.9 | LYM52 | 1289 4.7 | A | 0.01 1 | 3.21E-02 | 334 |
| LYM245 | 13061. 8 | C | 0.94 9 | 1.00E-06 | 74.3 | LYM227 | 1454 4.2 | A | 0.00 4 | 3.26E-02 | 53 |
| LYM32 | 13963. 4 | C | 0.94 9 | 2.00E-06 | 74.3 | LYM52 | 1289 4.8 | A | 0.00 5 | 3.45E-02 | 88 |
| LYM188 | 13244. 4 | C | 0.90 7 | 3.00E-06 | 66.6 | LYM180 | 1344 1.7 | A | 0.00 9 | 3.65E-02 | 259 |
| LYM 189 | 13311. 3 | C | 0.92 3 | 3.00E-06 | 69.5 | LYM248 | 1350 3.5 | A | 0.00 4 | 3.65E-02 | 75 |
| LYM234 | 14091. 1 | C | 0.98 1 | 1.40E-05 | 80.1 | LYM213 | 1284 1.6 | A | 0.00 6 | 3.85E-02 | 125 |
| LYM217 | 13186. 1 | C | 0.88 9 | 1.90E-05 | 63.3 | LYM107 | 1263 3.4 | A | 0.00 4 | 3.86E-02 | 71 |
| LYM234 | 14092. 8 | C | 0.95 7 | 1.90E-05 | 75.7 | LYM 117 | 1362 2.6 | A | 0.00 4 | 3.99E-02 | 51 |
| LYM234 | 14093. 2 | C | 0.87 2 | 3.90E-05 | 60.1 | LYM227 | 1454 2.3 | A | 0.00 3 | 4.02E-02 | 37 |
| LYM79 | 13133. 2 | C | 0.90 3 | 5.00E-05 | 65.8 | LYM227 | 1454 2.5 | A | 0.00 3 | 4.55E-02 | 32 |
| LYM74 | 13952. 2 | C | 0.95 | 1.17E-04 | 74.4 | LYM120 | 1338 2.4 | A | 0.00 4 | 4.58E-02 | 59 |
| LYM161 | 13233. 5 | C | 0.79 3 | 3.92E-04 | 45.6 | LYM248 | 1350 4.6 | A | 0.00 3 | 4.71E-02 | 29 |
| LYM274 | 13413. 1 | C | 0.91 9 | 7.62E-04 | 68.8 | LYM52 | 1289 1.8 | A | 0.00 4 | 5.0.E-02 | 62 |
| LYM219 | 13331. 1 | C | 0.78 8 | 2.74E-03 | 44.8 | LYM79 | 1313 1.5 | A | 0.00 4 | 5.09E-02 | 54 |
| LYM79 | 13131. 1 | C | 0.82 5 | 3.08E-03 | 51.6 | LYM107 | 1263 2.1 | A | 0.00 4 | 5.18E-02 | 55 |
| LYM240 | 12683. 9 | C | 0.75 3 | 3.59E-03 | 38.2 | LYM248 | 1350 2.7 | A | 0.00 3 | 5.65E-02 | 27 |
| LYM188 | 13244. 7 | C | 0.76 7 | 4.32E-03 | 40.8 | LYM117 | 1362 3.9 | A | 0.00 4 | 5.72E-02 | 63 |
| LYM161 | 13234. 6 | C | 0.77 1 | 5.36E-03 | 41.5 | LYM157 | 1334 2.4 | A | 0.00 3 | 6.07E-02 | 30 |
| LYM79 | 13133. 3 | C | 0.76 5 | 9.03E-03 | 40.4 | LYM117 | 1362 1.8 | A | 0.00 4 | 6.42E-02 | 41 |
| LYM 189 | 13314. 4 | C | 0.75 | 9.65E-03 | 37.7 | LYM 180 | 1344 1.5 | A | 0.00 7 | 9.29E-02 | 178 |
| LYM273 | 13721. 3 | C | 0.70 6 | 2.08E-02 | 29.7 | CONTR OL | - | A | 0.00 3 | - | 0 |
| LYM79 | 13134. 3 | C | 0.70 8 | 2.18E-02 | 30 | LYM79 | 1313 2.6 | B | 0.09 7 | 0.00E +00 | 57 |
| LYM188 | 13244. 6 | C | 0.67 6 | 6.45E-02 | 24.2 | LYM248 | 1350 2.6 | B | 0.08 4 | 3.80E-05 | 36.3 |
| LYM234 | 14092. 1 | C | 0.66 | 8.67E-02 | 21.2 | LYM52 | 1289 4.6 | B | 0.07 7 | 1.17E-03 | 24.1 |
| LYM32 | 13963. 1 | C | 0.68 6 | 9.24E-02 | 26 | LYM120 | 1338 2.8 | B | 0.14 3 | 1.23E-03 | 130.6 |
| CONTR OL | - | C | 0.54 5 | - | 0 | LYM213 | 1284 2.8 | B | 0.19 7 | 1.73E-03 | 218 |
| LYM32 | 13964. 1 | D | 8.65 1 | 0.00E +00 | 79.9 | LYM157 | 1334 1.4 | B | 0.11 7 | 3.42E-03 | 88.4 |
| LYM219 | 13332. 9 | D | 10.4 91 | 1.00E-05 | 118.2 | LYM227 | 1454 4.2 | B | 0.09 3 | 4.06E-03 | 50.6 |
| LYM 122 | 13711. 3 | D | 8.61 1 | 5.80E-05 | 79.1 | LYM120 | 1338 2.2 | B | 0.16 4 | 4.26E-03 | 164.9 |
| LYM161 | 13233. 5 | D | 7.32 6 | 1.20E-04 | 52.3 | LYM107 | 1263 3.4 | B | 0.10 2 | 4.42E-03 | 65.5 |
| LYM 188 | 13244. 4 | D | 8.09 7 | 2.06E-04 | 68.4 | LYM52 | 1289 5.7 | B | 0.18 5 | 4.74E-03 | 198.5 |
| LYM234 | 14092. 5 | D | 10.5 4 | 7.64E-04 | 119.2 | LYM117 | 1362 1.8 | B | 0.08 3 | 5.00E-03 | 33.8 |
| LYM79 | 13132. 2 | D | 9.36 | 3.54E-03 | 94.6 | LYM 180 | 1344 2.6 | B | 0.09 5 | 5.31E-03 | 53.8 |
| LYM74 | 13954. 2 | D | 9.85 5 | 5.72E-03 | 104.9 | LYM 107 | 1263 1.1 | B | 0.18 2 | 5.88E-03 | 193.5 |
| LYM189 | 13315. 2 | D | 10.9 46 | 5.76E-03 | 127.6 | LYM248 | 1350 4.6 | B | 0.08 | 6.04E-03 | 29.8 |
| LYM74 | 13954. 1 | D | 11.9 81 | 6.16E-03 | 149.1 | LYM 157 | 1334 1.1 | B | 0.17 6 | 6.92E-03 | 183.8 |
| LYM274 | 13413. 4 | D | 10.3 17 | 7.08E-03 | 114.5 | LYM 157 | 1334 1.3 | B | 0.28 9 | 8.30E-03 | 367.8 |
| LYM161 | 13233. 6 | D | 8.86 9 | 8.43E-03 | 84.4 | LYM120 | 1338 2.4 | B | 0.08 7 | 8.44E-03 | 41.4 |
| LYM274 | 13415. 2 | D | 9.62 3 | 8.58E-03 | 100.1 | LYM79 | 1313 1.6 | B | 0.15 3 | 9.73E-03 | 146.6 |
| LYM245 | 13061. 8 | D | 8.36 8 | 8.65E-03 | 74 | LYM117 | 1362 2.5 | B | 0.08 9 | 1.06E-02 | 44 |
| LYM 189 | 13315. 1 | D | 11.2 93 | 1.02E-02 | 134.8 | LYM157 | 1334 2.4 | B | 0.09 2 | 1.14E-02 | 49 |
| LYM234 | 14093. 2 | D | 7.63 2 | 1.08E-02 | 58.7 | LYM 120 | 1338 2.3 | B | 0.18 9 | 1.26E-02 | 205.1 |
| LYM240 | 12683. 9 | D | 6.76 1 | 1.26E-02 | 40.6 | LYM52 | 1289 1.8 | B | 0.09 9 | 1.29E-02 | 60.4 |
| LYM 189 | 13311. 3 | D | 8.14 9 | 1.49E-02 | 69.5 | LYM227 | 1454 1.5 | B | 0.08 6 | 1.29E-02 | 39.2 |
| LYM79 | 13134. 1 | D | 9.34 7 | 1.65E-02 | 94.4 | LYM213 | 1284 2.9 | B | 0.18 3 | 1.30E-02 | 195.5 |
| LYM32 | 13963. 4 | D | 8.28 8 | 1.66E-02 | 72.4 | LYM180 | 1344 1.7 | B | 0.20 3 | 1.33E-02 | 227.8 |
| LYM240 | 12682. 1 | D | 9.74 7 | 1.72E-02 | 102.7 | LYM120 | 1338 2.1 | B | 0.14 9 | 1.38E-02 | 140.6 |
| LYM 189 | 13311. 2 | D | 8.99 | 1.94E-02 | 87 | LYM213 | 1284 1.6 | B | 0.12 5 | 1.59E-02 | 102.2 |
| LYM79 | 13134. 3 | D | 6.43 1 | 1.94E-02 | 33.7 | LYM180 | 1344 2.7 | B | 0.10 2 | 2.04E-02 | 64.6 |
| LYM217 | 13186. 1 | D | 7.5 | 1.95E-02 | 56 | LYM52 | 1289 4.7 | B | 0.23 7 | 2.09E-02 | 282.8 |
| LYM217 | 13181. 6 | D | 8.44 3 | 1.96E-02 | 75.6 | LYM117 | 1362 2.6 | B | 0.09 5 | 2.67E-02 | 53.3 |
| LYM273 | 13721. 3 | D | 6.13 2 | 3.30E-02 | 27.5 | LYM213 | 1284 1.8 | B | 0.11 8 | 2.79E-02 | 91.6 |
| LYM234 | 14092. 1 | D | 5.87 8 | 3.41E-02 | 22.2 | LYM248 | 1350 3.5 | B | 0.1 | 2.95E-02 | 62 |
| LYM240 | 12683. 5 | D | 9.95 7 | 3.82E-02 | 107.1 | LYM227 | 1454 2.1 | B | 0.09 6 | 4.16E-02 | 55.3 |
| LYM79 | 13133. 2 | D | 8.25 2 | 4.16E-02 | 71.6 | LYM213 | 1284 1.5 | B | 0.08 9 | 4.72E-02 | 43.5 |
| LYM234 | 14091. 1 | D | 8.58 6 | 5.33E-02 | 78.5 | LYM117 | 1362 3.9 | B | 0.08 9 | 4.77E-02 | 44.2 |
| LYM234 | 14092. 8 | D | 8.37 3 | 5.62E-02 | 74.1 | LYM248 | 1350 2.7 | B | 0.07 6 | 5.33E-02 | 23.7 |
| LYM161 | 13234. 6 | D | 7.15 | 7.26E-02 | 48.7 | LYM 107 | 1263 2.1 | B | 0.09 8 | 6.41E-02 | 57.9 |
| LYM219 | 13331. 1 | D | 7.09 6 | 7.41E-02 | 47.6 | LYM180 | 1344 1.5 | B | 0.16 2 | 6.47E-02 | 162.6 |
| LYM 188 | 13244. 7 | D | 6.69 | 8.77E-02 | 39.1 | LYM227 | 1454 2.5 | B | 0.07 1 | 7.28E-02 | 15.4 |
| CONTR OL | - | D | 4.80 9 | - | 0 | LYM52 | 1289 4.8 | B | 0.11 1 | 7.53E-02 | 79.6 |
| LYM189 | 13315. 2 | E | 0.66 2 | 3.29E-03 | 29.1 | LYM117 | 1362 4.7 | B | 0.08 2 | 7.84E-02 | 33 |
| LYM274 | 13413. 4 | E | 0.64 6 | 1.08E-02 | 26.1 | LYM248 | 1350 1.6 | B | 0.07 7 | 8.28E-02 | 24.4 |
| LYM 189 | 13315. 1 | E | 0.64 1 | 1.59E-02 | 25 | CONTR OL | - | B | 0.06 2 | - | 0 |
| LYM219 | 13332. 9 | E | 0.61 3 | 4.07E-02 | 19.6 | LYM 107 | 1263 1.1 | C | 0.83 1 | 0.00E +00 | 94.2 |
| LYM161 | 13233. 6 | E | 0.60 2 | 7.90E-02 | 17.4 | LYM 120 | 1338 2.3 | C | 1.03 8 | 0.00E +00 | 142.4 |
| LYM32 | 13964. 1 | E | 0.6 | 7.97E-02 | 17 | LYM157 | 1334 1.3 | C | 1.14 1 | 0.00E +00 | 166.5 |
| CONTR OL | - | E | 0.51 3 | - | 0 | LYM157 | 1334 1.4 | C | 0.91 8 | 0.00E +00 | 114.5 |
| LYM219 | 13332. 9 | F | 7.58 6 | 1.00E-06 | 29 | LYM213 | 1284 2.8 | C | 1.03 5 | 0.00E +00 | 141.7 |
| LYM32 | 13964. 1 | F | 6.96 2 | 7.00E-06 | 18.4 | LYM52 | 1289 4.7 | C | 0.8 | 0.00E +00 | 86.9 |
| LYM240 | 12682. 1 | F | 6.84 3 | 3.90E-05 | 16.4 | LYM213 | 1284 2.9 | C | 0.76 2 | 1.00E-06 | 77.9 |
| LYM74 | 13954. 1 | F | 7.08 5 | 4.60E-05 | 20.5 | LYM 120 | 1338 2.2 | C | 0.82 8 | 6.00E-06 | 93.3 |
| LYM79 | 13134. 1 | F | 7.2 | 1.08E-03 | 22.5 | LYM248 | 1350 3.5 | C | 0.74 1 | 6.00E-06 | 73 |
| LYM274 | 13413. 4 | F | 7.26 2 | 1.40E-03 | 23.5 | LYM52 | 1289 5.7 | C | 0.72 4 | 2.50E-05 | 69.1 |
| LYM79 | 13132. 2 | F | 6.66 2 | 1.94E-03 | 13.3 | LYM213 | 1284 1.6 | C | 0.7 | 3.20E-05 | 63.5 |
| LYM189 | 13315. 2 | F | 7.59 1 | 1.99E-03 | 29.1 | LYM120 | 1338 2.1 | C | 0.69 5 | 6.10E-05 | 62.3 |
| LYM234 | 14092. 5 | F | 7.11 7 | 2.84E-03 | 21.1 | LYM 180 | 1344 1.7 | C | 0.76 3 | 8.30E-05 | 78.3 |
| LYM 189 | 13315. 1 | F | 7.39 6 | 2.91E-03 | 25.8 | LYM 180 | 1344 2.7 | C | 0.67 5 | 1.83E-04 | 57.6 |
| LYM74 | 13954. 2 | F | 7.08 9 | 5.06E-03 | 20.6 | LYM79 | 1313 1.5 | C | 0.68 | 2.64E-04 | 58.9 |
| LYM161 | 13233. 6 | F | 6.97 8 | 1.61E-02 | 18.7 | LYM 157 | 1334 1.1 | C | 0.66 6 | 5.55E-04 | 55.6 |
| LYM161 | 13233. 5 | F | 6.50 2 | 2.85E-02 | 10.6 | LYM1 17 | 1362 1.8 | C | 0.65 9 | 8.30E-04 | 53.8 |
| LYM 122 | 13711. 3 | F | 6.64 3 | 3.74E-02 | 13 | LYM79 | 1313 1.6 | C | 0.65 9 | 1.51E-03 | 53.9 |
| LYM240 | 12683. 5 | F | 6.97 8 | 4.00E-02 | 18.7 | LYM52 | 1289 4.8 | C | 0.67 | 1.78E-03 | 56.5 |
| LYM79 | 13134. 3 | F | 6.40 3 | 5.79E-02 | 8.9 | LYM52 | 1289 1.8 | C | 0.62 4 | 1.88E-03 | 45.8 |
| LYM274 | 13415. 2 | F | 6.39 | 6.0 1E-02 | 8.7 | LYM227 | 1454 2.3 | C | 0.64 7 | 2.21E-03 | 51.2 |
| LYM245 | 13061. 8 | F | 6.36 6 | 7.89E-02 | 8.3 | LYM157 | 1334 2.4 | C | 0.61 6 | 4.48E-03 | 43.8 |
| LYM217 | 13181. 6 | F | 6.76 6 | 8.26E-02 | 15.1 | LYM79 | 1313 2.6 | C | 0.60 5 | 4.76E-03 | 41.3 |
| CONTR OL | - | F | 5.87 9 | - | 0 | LYM120 | 1338 2.8 | C | 0.60 7 | 5.79E-03 | 41.7 |
| LYM189 | 13315. 2 | G | 1.28 4 | 1.90E-05 | 167.8 | LYM180 | 1344 1.5 | C | 0.61 1 | 8.13E-03 | 42.7 |
| LYM217 | 13183. 2 | G | 0.97 3 | 4.10E-05 | 103 | LYM117 | 1362 2.6 | C | 0.59 3 | 8.16E-03 | 38.5 |
| LYM74 | 13954. 2 | G | 0.93 1 | 6.30E-05 | 94.1 | LYM248 | 1350 1.6 | C | 0.60 4 | 8.30E-03 | 41.1 |
| LYM234 | 14092. 5 | G | 1.20 4 | 1.92E-04 | 151.1 | LYM213 | 1284 1.5 | C | 0.59 4 | 9.39E-03 | 38.7 |
| LYM122 | 13711. 3 | G | 1.15 9 | 3.08E-04 | 141.7 | LYM227 | 1454 4.2 | C | 0.59 3 | 1.48E-02 | 38.6 |
| LYM240 | 12683. 5 | G | 1.04 2 | 7.28E-04 | 117.2 | LYM 107 | 1263 1.2 | C | 0.58 8 | 1.80E-02 | 37.4 |
| LYM79 | 13134. 3 | G | 0.80 8 | 1.09E-03 | 68.5 | LYM117 | 1362 2.5 | C | 0.60 4 | 1.90E-02 | 41 |
| LYM245 | 13061. 8 | G | 1.27 1 | 1.20E-03 | 165.1 | LYM 107 | 1263 3.4 | C | 0.56 4 | 2.80E-02 | 31.8 |
| LYM79 | 13132. 2 | G | 1.32 | 1.33E-03 | 175.3 | LYM52 | 1289 4.6 | C | 0.54 8 | 4.99E-02 | 27.9 |
| LYM234 | 14093. 2 | G | 0.94 3 | 1.41E-03 | 96.7 | LYM79 | 1313 4.7 | C | 0.53 6 | 7.39E-02 | 25.2 |
| LYM74 | 13954. 1 | G | 1.31 3 | 1.81E-03 | 173.7 | CONTR OL | - | C | 0.42 8 | - | 0 |
| LYM217 | 13186. 1 | G | 0.91 1 | 1.88E-03 | 90.1 | LYM157 | 1334 1.4 | D | 8.10 1 | 0.00E +00 | 121.4 |
| LYM32 | 13963. 1 | G | 0.66 5 | 2.56E-03 | 38.7 | LYM213 | 1284 2.9 | D | 6.77 7 | 1.47E-04 | 85.2 |
| LYM161 | 13233. 6 | G | 1.06 4 | 2.90E-03 | 121.9 | LYM213 | 1284 1.6 | D | 6.03 9 | 2.00E-04 | 65 |
| LYM217 | 13182. 1 | G | 1.08 3 | 3.10E-03 | 125.8 | LYM52 | 1289 4.7 | D | 7.31 4 | 8.85E-04 | 99.9 |
| LYM219 | 13332. 9 | G | 1.06 3 | 4.10E-03 | 121.8 | LYM117 | 1362 2.6 | D | 5.11 3 | 1.50E-03 | 39.7 |
| LYM161 | 13233. 5 | G | 1.09 9 | 4.42E-03 | 129.2 | LYM107 | 1263 1.1 | D | 7.34 6 | 1.55E-03 | 100.8 |
| LYM274 | 13415. 2 | G | 1.15 | 4.92E-03 | 139.8 | LYM180 | 1344 2.7 | D | 6.26 6 | 1.64E-03 | 71.2 |
| LYM274 | 13413. 4 | G | 0.89 9 | 6.80E-03 | 87.4 | LYM120 | 1338 2.1 | D | 6.10 3 | 2.38E-03 | 66.8 |
| LYM 189 | 13315. 1 | G | 1.43 7 | 7.49E-03 | 199.8 | LYM157 | 1334 1.3 | D | 9.88 6 | 2.61E-03 | 170.2 |
| LYM 189 | 13311. 2 | G | 1.02 9 | 7.55E-03 | 114.6 | LYM52 | 1289 1.8 | D | 5.27 9 | 3.81E-03 | 44.3 |
| LYM32 | 13964. 1 | G | 0.84 4 | 7.69E-03 | 76 | LYM248 | 1350 3.5 | D | 6.50 1 | 4.31E. 03 | 77.7 |
| LYM79 | 13134. 1 | G | 0.99 | 1.05E-02 | 106.5 | LYM 107 | 1263 3.4 | D | 5.08 1 | 6.28E-03 | 38.9 |
| LYM234 | 14091. 1 | G | 0.91 9 | 1.09E-02 | 91.6 | LYM79 | 1313 2.6 | D | 5.27 2 | 7.18E-03 | 44.1 |
| LYM 188 | 13244. 4 | G | 1.04 9 | 1.17E-02 | 118.7 | LYM79 | 1313 4.7 | D | 4.84 4 | 7.92E-03 | 32.4 |
| LYM217 | 13181. 9 | G | 0.77 5 | 1.18E-02 | 61.6 | LYM79 | 1313 1.5 | D | 5.96 1 | 1.41E-02 | 62.9 |
| LYM240 | 12683. 9 | G | 0.57 9 | 1.27E-02 | 20.7 | LYM52 | 1289 5.7 | D | 6.30 2 | 1.43E-02 | 72.2 |
| LYM 189 | 13311. 3 | G | 0.98 | 1.37E-02 | 104.4 | LYM157 | 1334 1.1 | D | 6.12 7 | 1.48E-02 | 67.4 |
| LYM278 | 13364. 5 | G | 0.57 4 | 1.37E-02 | 19.7 | LYM213 | 1284 2.8 | D | 9.74 5 | 1.57E-02 | 166.3 |
| LYM240 | 12682. 1 | G | 1.13 2 | 1.51E-02 | 136.1 | LYM157 | 1334 2.4 | D | 5.41 8 | 1.58E-02 | 48.1 |
| LYM79 | 13133. 3 | G | 0.97 4 | 1.51E-02 | 103.1 | LYM 120 | 1338 2.8 | D | 5.23 3 | 2.06E-02 | 43 |
| LYM234 | 14092. 8 | G | 1.15 | 2.17E-02 | 139.7 | LYM120 | 1338 2.3 | D | 9.17 2 | 3.14E-02 | 150.7 |
| LYM188 | 13244. 6 | G | 0.58 | 2.18E-02 | 20.9 | LYM213 | 1284 1.5 | D | 5.04 4 | 3.32E-02 | 37.9 |
| LYM79 | 13134. 2 | G | 0.62 2 | 2.53E-02 | 29.6 | LYM117 | 1362 1.8 | D | 5.51 3 | 4.02E-02 | 50.7 |
| LYM32 | 13963. 4 | G | 0.96 7 | 2.55E-02 | 101.8 | LYM120 | 1338 2.2 | D | 7.20 7 | 4.05E-02 | 97 |
| LYM79 | 13133. 2 | G | 0.95 | 2.59E-02 | 98 | LYM79 | 1313 3.6 | D | 4.40 4 | 4.15E-02 | 20.4 |
| LYM189 | 13314. 5 | G | 0.60 4 | 2.68E-02 | 26 | LYM52 | 1289 4.6 | D | 4.63 7 | 4.35E-02 | 26.7 |
| LYM161 | 13234. 6 | G | 0.98 9 | 2.76E-02 | 106.3 | LYM248 | 1350 1.6 | D | 5.17 8 | 5.20E-02 | 41.5 |
| LYM74 | 13952. 2 | G | 0.83 7 | 3.56E-02 | 74.5 | LYM180 | 1344 1.5 | D | 5.62 1 | 5.27E-02 | 53.6 |
| LYM189 | 13314. 4 | G | 0.80 3 | 3.63E-02 | 67.4 | LYM180 | 1344 1.7 | D | 6.61 6 | 5.74E-02 | 80.8 |
| LYM274 | 13413. 1 | G | 0.82 3 | 4.32E-02 | 71.6 | LYM79 | 1313 1.6 | D | 5.67 2 | 6.56E-02 | 55 |
| LYM79 | 13131. 1 | G | 0.94 1 | 5.88E-02 | 96.2 | LYM227 | 1454 2.3 | D | 5.39 8 | 8.39E-02 | 47.5 |
| LYM188 | 13244. 7 | G | 0.74 1 | 6.09E-02 | 54.5 | CONTR OL | - | D | 3.65 9 | - | 0 |
| LYM234 | 14092. 1 | G | 0.61 6 | 6.23E-02 | 28.6 | LYM157 | 1334 1.3 | E | 0.56 3 | 8.32E-03 | 39.2 |
| LYM219 | 13331. 1 | G | 0.77 8 | 8.27E-02 | 62.3 | LYM248 | 1350 3.5 | E | 0.54 4 | 1.60E-02 | 34.6 |
| CONTR OL | - | G | 0.48 | - | 0 | LYM248 | 1350 1.6 | E | 0.53 1 | 2.70E-02 | 31.5 |
| LYM122 | 13711. 3 | H | 0.12 1 | 0.00E +00 | 155.6 | LYM 120 | 1338 2.2 | E | 0.52 2 | 4.43E-02 | 29.2 |
| LYM161 | 13233. 6 | H | 0.11 2 | 0.00E +00 | 136 | LYM 120 | 1338 2.3 | E | 0.52 3 | 6.14E-02 | 29.4 |
| LYM161 | 13233. 5 | H | 0.11 | 0.00E +00 | 133.1 | LYM 107 | 1263 1.1 | E | 0.51 1 | 6.15E-02 | 26.4 |
| LYM161 | 13234. 6 | H | 0.10 5 | 0.00E +00 | 122 | LYM 120 | 1338 2.8 | E | 0.51 1 | 6.32E-02 | 26.4 |
| LYM188 | 13244. 4 | H | 0.10 5 | 0.00E +00 | 121.6 | LYM52 | 1289 1.8 | E | 0.49 8 | 9.65E-02 | 23.4 |
| LYM189 | 13315. 1 | H | 0.14 6 | 0.00E +00 | 207.9 | CONTR OL | - | E | 0.40 4 | - | 0 |
| LYM189 | 13315. 2 | H | 0.13 3 | 0.00E +00 | 181.1 | LYM 107 | 1263 1.1 | F | 6.51 7 | 0.00E +00 | 32.4 |
| LYM189 | 13311.2 | H | 0.106 | 0.00E+00 | 123.6 | LYM 157 | 13341.4 | F | 6.663 | 0.00E+00 | 35.4 |
| LYM 189 | 13311. 3 | H | 0.09 9 | 0.00E +00 | 109.8 | LYM79 | 1313 1.5 | F | 6.65 8 | 2.30E-05 | 35.3 |
| LYM217 | 13182. 1 | H | 0.11 7 | 0.00E +00 | 146.7 | LYM79 | 1313 2.6 | F | 6.26 9 | 6.80E-05 | 27.4 |
| LYM217 | 13183. 2 | H | 0.09 6 | 0.00E +00 | 102.8 | LYM248 | 1350 3.5 | F | 6.84 9 | 7.40E-05 | 39.1 |
| LYM217 | 13186. 1 | H | 0.09 | 0.00E +00 | 91 | LYM 107 | 1263 3.4 | F | 6.42 | 1.94E-04 | 30.4 |
| LYM219 | 13332. 9 | H | 0.11 | 0.00E +00 | 132.5 | LYM248 | 1350 1.6 | F | 6.55 | 2.54E-04 | 33.1 |
| LYM234 | 14092. 5 | H | 0.12 3 | 0.00E +00 | 160.3 | LYM213 | 1284 2.8 | F | 7.02 9 | 2.62E-04 | 42.8 |
| LYM234 | 14092. 8 | H | 0.11 9 | 0.00E +00 | 151.6 | LYM 180 | 1344 2.7 | F | 6.27 4 | 3.41E-04 | 27.5 |
| LYM234 | 14093. 2 | H | 0.10 1 | 0.00E +00 | 113.6 | LYM213 | 1284 2.9 | F | 6.02 8 | 4.41E-04 | 22.4 |
| LYM234 | 14091. 1 | H | 0.09 4 | 0.00E +00 | 98.2 | LYM213 | 1284 1.6 | F | 5.83 6 | 5.41E-04 | 18.6 |
| LYM240 | 12683. 5 | H | 0.10 9 | 0.00E +00 | 130.7 | LYM120 | 1338 2.2 | F | 6.34 5 | 1.79E-03 | 28.9 |
| LYM240 | 12682. 1 | H | 0.10 9 | 0.00E +00 | 130 | LYM157 | 1334 1.1 | F | 5.67 7 | 2.64E-03 | 15.3 |
| LYM245 | 13061. 8 | H | 0.12 8 | 0.00E +00 | 170.9 | LYM 157 | 1334 1.3 | F | 6.75 2 | 3.09E-03 | 37.2 |
| LYM274 | 13415. 2 | H | 0.11 9 | 0.00E +00 | 152.5 | LYM180 | 1344 1.7 | F | 5.93 5 | 3.68E-03 | 20.6 |
| LYM274 | 13413. 4 | H | 0.09 5 | 0.00E +00 | 101.8 | LYM 180 | 1344 1.5 | F | 6.07 1 | 4.41E-03 | 23.3 |
| LYM274 | 13413. 1 | H | 0.08 7 | 0.00E +00 | 84.2 | LYM52 | 1289 4.7 | F | 6.00 8 | 5.17E-03 | 22.1 |
| LYM32 | 13963. 4 | H | 0.10 2 | 0.00E +00 | 116.1 | LYM120 | 1338 2.3 | F | 6.67 4 | 8.09E-03 | 35.6 |
| LYM32 | 13964. 1 | H | 0.08 9 | 0.00E +00 | 87.8 | LYM52 | 1289 1.8 | F | 5.77 4 | 9.79E-03 | 17.3 |
| LYM74 | 13954. 1 | H | 0.12 8 | 0.00E +00 | 171.4 | LYM79 | 1313 4.7 | F | 5.60 1 | 1.44E-02 | 13.8 |
| LYM74 | 13954. 2 | H | 0.09 3 | 0.00E +00 | 96.9 | LYM227 | 1454 2.3 | F | 5.91 | 1.57E-02 | 20.1 |
| LYM79 | 13132. 2 | H | 0.13 4 | 0.00E +00 | 183.6 | LYM79 | 1313 3.6 | F | 5.77 8 | 2.12E-02 | 17.4 |
| LYM79 | 13133. 3 | H | 0.10 1 | 0.00E +00 | 114.2 | LYM 107 | 1263 1.2 | F | 5.76 4 | 2.69E-02 | 17.1 |
| LYM79 | 13134. 1 | H | 0.1 | 0.00E +00 | 111.5 | LYM120 | 1338 2.1 | F | 5.44 8 | 2.96E-02 | 10.7 |
| LYM79 | 13133. 2 | H | 0.09 7 | 0.00E +00 | 104.7 | LYM117 | 1362 1.8 | F | 5.54 1 | 3.11E-02 | 12.6 |
| LYM79 | 13134. 3 | H | 0.08 2 | 0.00E +00 | 73.1 | LYM1 17 | 1362 2.6 | F | 5.57 8 | 3.16E-02 | 13.3 |
| LYM 189 | 13314. 4 | H | 0.08 3 | 1.00E-06 | 75.5 | LYM157 | 1 334 2.4 | F | 5.90 6 | 4.04E-02 | 20 |
| LYM74 | 13952. 2 | H | 0.08 5 | 1.00E-06 | 80.6 | LYM52 | 1289 4.6 | F | 5.68 6 | 5.87E-02 | 15.5 |
| LYM79 | 13131. 1 | H | 0.09 7 | 1.00E-06 | 104.4 | LYM157 | 1334 1.1 | F | 5.64 7 | 6.06E-02 | 14.7 |
| LYM219 | 13331. 1 | H | 0.08 2 | 1.20E-05 | 73.8 | LYM120 | 1338 2.8 | F | 5.59 7 | 7.48E-02 | 13.7 |
| LYM188 | 13244. 7 | H | 0.07 9 | 1.40E-05 | 66.2 | LYM248 | 1350 2.7 | F | 5.61 7 | 7.50E-02 | 14.1 |
| LYM217 | 13181. 9 | H | 0.07 1 | 7.40E-05 | 50 | CONTR OL | - | F | 4.92 3 | - | 0 |
| LYM219 | 13334. 8 | H | 0.07 5 | 4.81E-04 | 59.2 | LYM79 | 1313 4.7 | G | 0.78 3 | 0.00E +00 | 69 |
| LYM32 | 13963. 1 | H | 0.06 4 | 1.00E-03 | 34.7 | LYM120 | 1338 2.8 | G | 0.93 7 | 6.00E-06 | 102.4 |
| LYM273 | 13721. 3 | H | 0.06 7 | 1.42E-03 | 40.7 | LYM180 | 1344 2.6 | G | 0.70 8 | 1.72E-04 | 52.9 |
| LYM189 | 13314. 5 | H | 0.06 3 | 1.80E-03 | 33.5 | LYM79 | 1313 2.6 | G | 0.63 5 | 2.20E-04 | 37.1 |
| LYM234 | 14092. 1 | H | 0.06 4 | 2.49E-03 | 34.5 | LYM248 | 1350 2.6 | G | 0.6 | 3.71E-04 | 29.4 |
| LYM217 | 13181. 6 | H | 0.06 7 | 2.63E-03 | 41 | LYM227 | 1454 4.2 | G | 0.61 2 | 4.69E-04 | 32 |
| LYM188 | 13244. 6 | H | 0.06 2 | 3.19E-03 | 30.8 | LYM107 | 1263 3.4 | G | 0.69 5 | 6.12E-04 | 50.1 |
| LYM219 | 13334. 7 | H | 0.06 5 | 6.05E-03 | 37.1 | LYM120 | 1338 2.1 | G | 1.05 6 | 8.03E-04 | 128 |
| LYM79 | 13134. 2 | H | 0.06 1 | 6.73E-03 | 28.6 | LYM227 | 1454 2.1 | G | 0.62 5 | 8.16E-04 | 34.9 |
| LYM240 | 12683. 9 | H | 0.05 9 | 1.03E-02 | 25.6 | LYM11 17 | 1362 2.6 | G | 0.67 8 | 8.66E-04 | 46.3 |
| LYM122 | 13713. 4 | H | 0.06 1 | 1.40E-02 | 29.1 | LYM157 | 1334 1.1 | G | 1.03 8 | 1.08E-03 | 124.2 |
| LYM278 | 13364. 5 | H | 0.05 8 | 2.02E-02 | 23.2 | LYM157 | 1334 1.4 | G | 0.73 7 | 1.15E-03 | 59.1 |
| LYM274 | 13413. 3 | H | 0.06 | 2.07E-02 | 27.2 | LYM157 | 1334 1.3 | G | 1.52 8 | 1.56E-03 | 229.9 |
| LYM240 | 12684. 8 | H | 0.05 9 | 5.27E-02 | 24.1 | LYM120 | 1338 2.2 | G | 1.05 7 | 1.65E-03 | 128.2 |
| CONTR OL | - | H | 0.04 7 | - | 0 | LYM107 | 1263 1.1 | G | 1.08 5 | 2.28E-03 | 134.2 |
| LYM1 | 11603. 2 | A | 0.00 6 | 2.00E-06 | 74.1 | LYM227 | 1454 1.5 | G | 0.61 | 2.98E-03 | 31.8 |
| LYM1 | 11601. 1 | A | 0.00 7 | 1.20E-05 | 83.9 | LYM120 | 1338 2.4 | G | 0.61 4 | 4.29E-03 | 32.6 |
| LYM132 | 12275. 3 | A | 0.00 7 | 1.63E-04 | 96.5 | LYM213 | 1284 2.8 | G | 1.20 5 | 4.34E-03 | 160.1 |
| LYM178 | 12164. 2 | A | 0.00 6 | 2.14E-03 | 58 | LYM248 | 1350 3.5 | G | 0.75 1 | 4.94E-03 | 62.2 |
| LYM132 | 12271. 4 | A | 0.00 7 | 3.64E-03 | 97.2 | LYM180 | 1344 2.7 | G | 0.83 9 | 5.84E-03 | 81.2 |
| LYM1 | 11604. 4 | A | 0.00 5 | 3.68E-03 | 29.4 | LYM52 | 1289 5.7 | G | 1.04 4 | 6.49E-03 | 125.4 |
| LYM268 | 12483. 2 | A | 0.00 8 | 6.62E-03 | 111.2 | LYM157 | 1334 2.4 | G | 0.60 9 | 6.86E-03 | 31.5 |
| LYM5 | 12432. 2 | A | 0.00 5 | 6.64E-03 | 28.7 | LYM180 | 1344 1.5 | G | 1.02 7 | 7.3 1E-03 | 121.8 |
| LYM 178 | 12164. 3 | A | 0.01 1 | 1.01E-02 | 206.3 | LYM213 | 1284 1.8 | G | 0.73 2 | 7.99E-03 | 58 |
| LYM 134 | 12314. 2 | A | 0.00 6 | 1.05E-02 | 77.6 | LYM120 | 1338 2.3 | G | 1.09 9 | 8.19E-03 | 137.3 |
| LYM268 | 12482. 3 | A | 0.00 6 | 1.08E-02 | 58.7 | LYM52 | 1289 4.7 | G | 1.15 3 | 8.28E-03 | 148.8 |
| LYM 129 | 12571. 3 | A | 0.00 6 | 1.11E-02 | 54.5 | LYM213 | 1284 2.9 | G | 1.14 3 | 8.30E-03 | 146.8 |
| LYM178 | 12163. 3 | A | 0.01 1 | 1.16E-02 | 202.1 | LYM79 | 1313 1.6 | G | 1.11 | 8.39E-03 | 139.7 |
| LYMI | 11602. 6 | A | 0.01 1 | 1.60E-02 | 197.2 | LYM 180 | 1344 1.7 | G | 1.22 4 | 1.13E-02 | 164.3 |
| LYM1 | 11602. 1 | A | 0.00 5 | 1.79E-02 | 46.2 | LYM157 | 1334 1.1 | G | 0.52 7 | 1.68E-02 | 13.9 |
| LYM268 | 12482. 1 | A | 0.00 7 | 2.35E-02 | 97.9 | LYM227 | 1454 2.5 | G | 0.56 9 | 1.84E-02 | 22.7 |
| LYM132 | 12276. 1 | A | 0.00 9 | 2.60E-02 | 146.9 | LYM52 | 1289 4.6 | G | 0.52 5 | 1.93E-02 | 13.4 |
| LYM129 | 12572. 2 | A | 0.00 5 | 2.65E-02 | 46.2 | LYM52 | 1289 1.8 | G | 0.67 1 | 2.02E-02 | 44.8 |
| LYM5 | 12432. 1 | A | 0.00 6 | 4.79E-02 | 70.6 | LYM117 | 1362 1.8 | G | 0.57 | 2.22E-02 | 23 |
| LYM6 | 11735. 1 | A | 0.00 6 | 5.37E-02 | 58 | LYM213 | 1284 1.6 | G | 0.78 2 | 2.30E-02 | 68.9 |
| LYM 132 | 12273. 2 | A | 0.00 5 | 5.62E-02 | 44.1 | LYM 107 | 1263 2.1 | G | 0.64 8 | 2.97E-02 | 39.9 |
| LYM268 | 12483. 4 | A | 0.00 6 | 6.34E-02 | 65 | LYM117 | 1362 3.9 | G | 0.67 7 | 4.19E-02 | 46 |
| LYM6 | 11736. 1 | A | 0.00 6 | 6.64E-02 | 71.3 | LYM117 | 1362 4.7 | G | 0.69 5 | 5.21E-02 | 49.9 |
| LYM6 | 11733. 2 | A | 0.00 4 | 6.75E-02 | 13.3 | LYM117 | 1362 2.5 | G | 0.63 4 | 5.88E-02 | 36.9 |
| LYM6 | 11735. 2 | A | 0.00 5 | 6.93E-02 | 49 | LYM52 | 1289 4.8 | G | 0.73 | 6.31E-02 | 57.5 |
| LYM 134 | 12313. 2 | A | 0.01 1 | 7.63E-02 | 216.1 | LYM79 | 1313 1.5 | G | 0.65 4 | 7.90E-02 | 41.2 |
| LYM 129 | 12573. 5 | A | 0.00 7 | 7.95E-02 | 102.8 | CONTR OL | - | G | 0.46 3 | - | 0 |
| CONTR OL | - | A | 0.00 4 | - | 0 | LYM 107 | 1263 1.1 | H | 0.10 7 | 0.00E +00 | 141.1 |
| LYM132 | 12275. 3 | B | 0.13 4 | 1.00E-06 | 86.1 | LYM120 | 1338 2.3 | H | 0.11 1 | 0.00E +00 | 148.8 |
| LYM1 | 11603. 2 | B | 0.12 4 | 3.20E-05 | 72.1 | LYM120 | 1338 2.2 | H | 0.10 7 | 0.00E +00 | 139.8 |
| LYM1 | 11601. 1 | B | 0.12 3 | 4. 05 | 71.4 | LYM120 | 1338 2.1 | H | 0.10 2 | 0.00E +00 | 129.7 |
| LYM 132 | 12271. 4 | B | 0.12 4 | 1.69E-04 | 71.8 | LYM 120 | 1338 2.8 | H | 0.09 4 | 0.00E +00 | 111.3 |
| LYM268 | 12483. 2 | B | 0.14 2 | 8.85E-04 | 96.8 | LYM 157 | 1334 1.3 | H | 0.14 6 | 0.00E +00 | 228.5 |
| LYM 178 | 12164. 2 | B | 0.11 1 | 2.98E-03 | 54.4 | LYM157 | 1334 1.1 | H | 0.09 6 | 0.00E +00 | 117 |
| LYM5 | 12432. 2 | B | 0.09 | 6.77E-03 | 25.1 | LYM 180 | 1344 1.7 | H | 0.11 4 | 0.00E +00 | 157.5 |
| LYM 134 | 12314. 2 | B | 0.13 8 | 7.76E-03 | 91.2 | LYM180 | 1344 1.5 | H | 0.09 7 | 0.00E +00 | 119.2 |
| LYM129 | 12571. 3 | B | 0.11 | 9.48E-03 | 52.8 | LYM213 | 1284 2.8 | H | 0.11 4 | 0.00E +00 | 157 |
| LYM1 | 11602. 6 | B | 0.21 4 | 9.84E-03 | 197.7 | LYM213 | 1284 2.9 | H | 0.10 7 | 0.00E +00 | 141.1 |
| LYM268 | 12482. 3 | B | 0.12 4 | 1.45E-02 | 71.8 | LYM52 | 1289 4.7 | H | 0.11 7 | 0.00E +00 | 162.5 |
| LYM268 | 12482. 1 | B | 0.12 9 | 1.64E-02 | 78.6 | LYM52 | 1289 5.7 | H | 0.10 5 | 0.00E +00 | 136 |
| LYM129 | 12572. 2 | B | 0.10 1 | 1.69E-02 | 40.1 | LYM79 | 1313 1.6 | H | 0.10 8 | 0.00E +00 | 142.5 |
| LYM 129 | 12573. 3 | B | 0.08 7 | 1.94E-02 | 21 | LYM180 | 1344 2.7 | H | 0.08 | 1.00E-06 | 79.4 |
| LYM6 | 11735. 2 | B | 0.13 | 2.15E-02 | 80.5 | LYM157 | 1334 1.4 | H | 0.07 7 | 3.00E-06 | 73.4 |
| LYM 178 | 12164. 3 | B | 0.21 5 | 2.21E-02 | 199.1 | LYM213 | 1284 1.6 | H | 0.07 7 | 1.70E-05 | 74.3 |
| LYM 178 | 12163. 3 | B | 0.22 7 | 2.30E-02 | 214.8 | LYM79 | 1313 4.7 | H | 0.07 | 6.90E-05 | 57.7 |
| LYM5 | 12432. 1 | B | 0.11 6 | 2.54E-02 | 60.7 | LYM52 | 1289 4.8 | H | 0.07 5 | 1.89E-04 | 67.6 |
| LYM6 | 11735. 1 | B | 0.10 7 | 2.65E-02 | 48.9 | LYM227 | 1454 4.2 | H | 0.06 7 | 4.24E-04 | 49.8 |
| LYM 132 | 12276. 1 | B | 0.18 | 2.72E-02 | 150.1 | LYM 107 | 1263 3.4 | H | 0.06 6 | 5.81E-04 | 48.6 |
| LYM134 | 12313. 2 | B | 0.22 9 | 3.20E-02 | 217.6 | LYM117 | 1362 2.6 | H | 0.06 6 | 5.92E-04 | 49 |
| LYM268 | 12483. 4 | B | 0.12 8 | 4.11E-02 | 77.8 | LYM248 | 1350 3.5 | H | 0.06 7 | 7.50E-04 | 50.7 |
| LYM134 | 12312. 4 | B | 0.09 6 | 6.12E-02 | 33.5 | LYM213 | 1284 1.8 | H | 0.06 7 | 7.87E-04 | 50.6 |
| LYM 129 | 12573. 5 | B | 0.14 4 | 6.28E-02 | 99.7 | LYM52 | 1289 1.8 | H | 0.06 7 | 9.77E-04 | 49.6 |
| LYM6 | 11736. 1 | B | 0.13 | 7.9 1E-02 | 80.6 | LYM117 | 1362 2.5 | H | 0.06 7 | 9.85E-04 | 51.7 |
| LYM1 | 11604. 4 | B | 0.08 3 | 9.90E-02 | 16 | LYM 180 | 1344 2.6 | H | 0.06 5 | 1.09E-03 | 45.8 |
| CONTR OL | - | B | 0.07 2 | - | 0 | LYM117 | 1362 3.9 | H | 0.06 4 | 3.89E-03 | 44.7 |
| LYM1 | 11602. 6 | C | 0.96 5 | 0.00E +00 | 91.4 | LYM79 | 1313 2.6 | H | 0.06 2 | 5.30E-03 | 38.5 |
| LYM1 | 11601. 1 | C | 0.83 | 8.30E-05 | 64.7 | LYM 107 | 1263 2.1 | H | 0.06 3 | 5.57E-03 | 41.7 |
| LYM268 | 12483. 2 | C | 0.80 3 | 1.01E-04 | 59.3 | LYM 120 | 1338 2.4 | H | 0.06 1 | 5.57E-03 | 38.2 |
| LYM 132 | 12276. 1 | C | 0.93 6 | 1.06E-04 | 85.8 | LYM117 | 1362 4.7 | H | 0.06 4 | 5.91E-03 | 43.9 |
| LYM178 | 12164. 3 | C | 0.83 1 | 1.49E-04 | 64.9 | LYM248 | 1350 2.6 | H | 0.06 | 9.80E-03 | 35.3 |
| LYM 129 | 12571. 3 | C | 0.79 4 | 1.72E-04 | 57.5 | LYM213 | 1284 1.5 | H | 0.06 2 | 1.07E-02 | 39 |
| LYM 129 | 12573. 5 | C | 0.83 | 1.82E-04 | 64.6 | LYM227 | 1454 2.1 | H | 0.05 9 | 1.46E-02 | 33.2 |
| LYM268 | 12482. 3 | C | 0.75 2 | 4.97E-04 | 49.2 | LYM157 | 1334 2.4 | H | 0.06 | 1.46E-02 | 34.4 |
| LYM268 | 12483. 4 | C | 0.75 2 | 8.08E-04 | 49.2 | LYM117 | 1362 1.8 | H | 0.06 | 1.51E-02 | 34.5 |
| LYM178 | 12163. 3 | C | 0.84 9 | 8.09E-04 | 68.5 | LYM79 | 1313 1.5 | H | 0.06 | 2.75E-02 | 35.8 |
| LYM5 | 12435. 1 | C | 0.77 1 | 9.77E-04 | 52.9 | LYM227 | 1454 1.5 | H | 0.05 7 | 3.75E-02 | 28.4 |
| LYM 134 | 12314. 2 | C | 0.73 3 | 1.55E-03 | 45.5 | CONTR OL | - | H | 0.04 4 | - | 0 |
| LYM178 | 12164. 2 | C | 0.67 9 | 7.95E-03 | 34.7 | LYM79 | 1313 1.6 | A | 0.00 4 | 9.00E-06 | 63 |
| LYM 134 | 12313. 2 | C | 0.73 9 | 8.31E-03 | 46.6 | LYM79 | 1313 3.6 | A | 0.00 4 | 1.55E-03 | 61 |
| LYM132 | 12275. 3 | C | 0.67 5 | 1.62E-02 | 34 | LYM79 | 1313 1.5 | A | 0.00 4 | 3.66E-02 | 40 |
| LYM1 | 11603. 2 | C | 0.68 3 | 3.61E-02 | 35.6 | LYM79 | 1313 2.6 | A | 0.00 4 | 3.91E-02 | 47 |
| CONTROL | - | C | 0.50 4 | - | 0 | LYM227 | 1454 4.2 | A | 0.004 | 5.01E-02 | 72 |
| LYM1 | 11602. 6 | D | 8.10 9 | 7.70E-05 | 79.7 | LYM79 | 1313 4.7 | A | 0.00 3 | 5.24E-02 | 39 |
| LYM268 | 12483. 2 | D | 6.77 9 | 9.34E-04 | 50.3 | LYM227 | 1454 2.5 | A | 0.00 3 | 7.71E-02 | 35 |
| LYM268 | 12482. 3 | D | 6.31 8 | 9.47E-04 | 40 | CONTR OL | - | A | 0.00 3 | - | 0 |
| LYM 129 | 12571. 3 | D | 6.58 7 | 1.03E-03 | 46 | LYM79 | 1313 3.6 | B | 0.08 9 | 3.95E-03 | 23.5 |
| LYM134 | 12314. 2 | D | 6.14 6 | 2.72E-03 | 36.2 | LYM227 | 1454 4.2 | B | 0.10 1 | 1.09E-02 | 40.9 |
| LYM1 | 11601. 1 | D | 7.07 4 | 6.19E-03 | 56.8 | LYM227 | 1454 2.1 | B | 0.08 2 | 5.43E-02 | 14.7 |
| LYM268 | 12483. 4 | D | 6.37 5 | 1.10E-02 | 41.3 | LYM79 | 1313 1.5 | B | 0.09 3 | 7.88E-02 | 28.9 |
| LYM178 | 12164. 2 | D | 5.68 1 | 1.80E-02 | 25.9 | LYM79 | 1313 2.6 | B | 0.09 5 | 8.38E-02 | 32.4 |
| LYM129 | 12573. 5 | D | 7.10 5 | 1.84E-02 | 57.5 | LYM227 | 1454 2.5 | B | 0.08 5 | 9.81E-02 | 18.4 |
| LYM178 | 12164. 3 | D | 6.94 1 | 1.87E-02 | 53.8 | CONTR OL | - | B | 0.07 2 | - | 0 |
| LYM 132 | 12276. 1 | D | 7.98 5 | 3.78E-02 | 77 | LYM79 | 1313 4.7 | C | 0.66 5 | 5.00E-05 | 49.9 |
| LYM5 | 12435. 1 | D | 6.43 3 | 4.32E-02 | 42.6 | LYM79 | 1313 2.6 | C | 0.68 3 | 1.75E-04 | 53.9 |
| LYM132 | 12275. 3 | D | 5.76 9 | 5.38E-02 | 27.9 | LYM227 | 1454 4.2 | C | 0.60 6 | 2.21E-03 | 36.6 |
| LYM178 | 12163. 3 | D | 7.17 1 | 6.48E-02 | 59 | LYM79 | 1313 3.6 | C | 0.61 4 | 5.09E-03 | 38.3 |
| CONTROL | - | D | 4.51 1 | - | 0 | LYM79 | 1313 1.5 | C | 0.606 | 6.01E-03 | 36.5 |
| LYM 178 | 12164. 3 | E | 0.61 8 | 6.14E-04 | 35.8 | LYM227 | 1454 2.5 | C | 0.58 9 | 9.41E-03 | 32.9 |
| LYM5 | 12435. 1 | E | 0.6 | 6.85E-04 | 31.8 | LYM79 | 1313 1.6 | C | 0.55 9 | 2.18E-02 | 26 |
| LYM134 | 12314. 2 | E | 0.59 8 | 1.04E-03 | 31.5 | LYM227 | 1454 2.3 | C | 0.57 6 | 2.31E-02 | 29.9 |
| LYM268 | 12482. 3 | E | 0.59 | 1.49E-03 | 29.6 | CONTR OL | - | C | 0.44 4 | - | 0 |
| LYM129 | 12571. 3 | E | 0.59 6 | 1.76E-03 | 31 | LYM79 | 1313 4.7 | D | 5.69 8 | 4.00E-06 | 47.8 |
| LYM132 | 12276. 1 | E | 0.59 5 | 2.11E-03 | 30.8 | LYM79 | 1313 1.6 | D | 5.05 1 | 2.55E-04 | 31 |
| LYM1 | 11602. 6 | E | 0.58 | 4.57E-03 | 27.6 | LYM227 | 1454 4.2 | D | 5.17 6 | 7.52E-03 | 34.3 |
| LYM 132 | 12275. 3 | E | 0.58 7 | 9.38E-03 | 29 | LYM79 | 1313 1.5 | D | 5.50 4 | 5.52E-02 | 42.8 |
| LYM 178 | 12164. 2 | E | 0.55 7 | 1.30E-02 | 22.5 | LYM79 | 1313 2.6 | D | 5.91 1 | 5.61E-02 | 53.3 |
| LYM268 | 12483. 4 | E | 0.53 9 | 4.86E-02 | 18.5 | LYM227 | 1454 2.5 | D | 5.15 9 | 6.06E-02 | 33.8 |
| LYM 178 | 12163. 3 | E | 0.55 3 | 5.88E-02 | 21.4 | LYM79 | 1313 3.6 | D | 5.42 9 | 8.59E-02 | 40.9 |
| LYM1 | 11601. 1 | E | 0.54 3 | 6.29E-02 | 19.2 | CONTR OL | - | D | 3.85 5 | - | 0 |
| LYM 129 | 12573. 5 | E | 0.54 8 | 7.27E-02 | 20.4 | LYM79 | 1313 4.7 | F | 6.19 1 | 5.25E-04 | 18 |
| LYM5 | 12432. 1 | E | 0.53 2 | 7.47E-02 | 16.9 | LYM79 | 1313 2.6 | F | 6.15 8 | 6.45E-03 | 17.4 |
| LYM129 | 12572. 2 | E | 0.52 | 7.70E-02 | 14.4 | LYM79 | 1313 1.5 | F | 6.28 6 | 7.80E-03 | 19.8 |
| CONTR OL | - | E | 0.45 5 | - | 0 | LYM227 | 1454 2.3 | F | 5.86 3 | 7.80E-02 | 11.7 |
| LYM268 | 12482. 3 | F | 6.79 7 | 1.32E-04 | 22.4 | CONTR OL | - | F | 5.24 7 | - | 0 |
| LYM5 | 12435. 1 | F | 6.56 6 | 5.75E-04 | 18.2 | LYM79 | 1313 1.6 | G | 0.75 6 | 8.17E-03 | 48 |
| LYM134 | 12314. 2 | F | 6.85 8 | 2.92E-03 | 23.5 | LYM227 | 1454 4.2 | G | 0.57 5 | 3.56E-02 | 12.6 |
| LYM 132 | 12276. 1 | F | 6.96 8 | 4.43E-03 | 25.4 | CONTR OL | - | G | 0.51 1 | - | 0 |
| LYM129 | 12571. 3 | F | 6.61 4 | 1.13E-02 | 19.1 | LYM79 | 1313 1.6 | H | 0.07 5 | 1.40E-05 | 50.2 |
| LYM178 | 12164. 3 | F | 6.68 5 | 1.23E-02 | 20.3 | LYM227 | 1454 4.2 | H | 0.06 2 | 1.36E-02 | 24.5 |
| LYM268 | 12483. 2 | F | 6.47 7 | 1.34E-02 | 16.6 | LYM79 | 1313 3.6 | H | 0.06 2 | 1.76E-02 | 25.2 |
| LYM268 | 12483. 4 | F | 6.49 4 | 1.43E-02 | 16.9 | LYM79 | 1313 1.5 | H | 0.06 4 | 2.54E-02 | 28.1 |
| LYM1 | 11602. 6 | F | 6.60 7 | 1.52E-02 | 18.9 | LYM227 | 1454 2.5 | H | 0.06 1 | 3.57E-02 | 22.1 |
| LYM1 | 11601. 1 | F | 6.59 5 | 2.97E-02 | 18.7 | LYM79 | 1313 4.7 | H | 0.05 9 | 6.68E-02 | 19.2 |
| LYM 178 | 12164. 2 | F | 6.10 6 | 4.81E-02 | 9.9 | LYM79 | 1313 2.6 | H | 0.05 9 | 8.94E-02 | 18.9 |
| LYM 132 | 12275. 3 | F | 6.54 6 | 7.02E-02 | 17.8 | CONTR OL | - | H | 0.05 | - | 0 |
| LYM129 | 12573. 5 | F | 6.54 4 | 9.24E-02 | 17.8 | | | | | | |
| CONTR OL | - | F | 5.55 5 | - | 0 | | | | | | |
| LYM1 | 11601. 1 | G | 0.75 | 8.00E-06 | 56.3 | | | | | | |
| LYM132 | 12271. 4 | G | 0.76 | 1.00E-05 | 58.3 | | | | | | |
| LYM132 | 12275. 3 | G | 0.84 1 | 4.10E-05 | 75.2 | | | | | | |
| LYM268 | 12482. 1 | G | 0.73 8 | 2.75E-04 | 53.6 | | | | | | |
| LYM 178 | 12164. 3 | G | 1.03 1 | 1.85E-03 | 114.7 | | | | | | |
| LYM1 | 11603. 2 | G | 0.66 5 | 1.89E-03 | 38.5 | | | | | | |
| LYM 134 | 12314. 2 | G | 0.75 9 | 2.43E-03 | 58.1 | | | | | | |
| LYM1 | 11602. 6 | G | 1.00 2 | 3.24E-03 | 108.6 | | | | | | |
| LYM 178 | 12164. 2 | G | 0.61 5 | 7.36E-03 | 28 | | | | | | |
| LYM178 | 12163. 3 | G | 0.92 9 | 1.03E-02 | 93.6 | | | | | | |
| LYM132 | 12276. 1 | G | 0.83 3 | 1.10E-02 | 73.5 | | | | | | |
| LYM6 | 11736.1 | G | 0.75 | 1.25E-02 | 56.2 | | | | | | |
| LYM129 | 12573. 5 | G | 0.78 6 | 2.28E-02 | 63.6 | | | | | | |
| LYM268 | 12483. 2 | G | 0.72 8 | 2.44E-02 | 51.6 | | | | | | |
| LYM6 | 11735. 2 | G | 0.59 4 | 2.79E-02 | 23.7 | | | | | | |
| LYM268 | 12482. 3 | G | 0.63 | 3.55E-02 | 31.3 | | | | | | |
| LYM5 | 12432. 1 | G | 0.74 1 | 3.96E-02 | 54.3 | | | | | | |
| LYM129 | 12572. 2 | G | 0.59 8 | 4.01E-02 | 24.5 | | | | | | |
| LYM1 | 11602. 1 | G | 0.58 8 | 5.84E-02 | 22.4 | | | | | | |
| LYM268 | 12483. 4 | G | 0.70 8 | 6.03E-02 | 47.5 | | | | | | |
| LYM134 | 12313. 2 | G | 0.96 8 | 6.92E-02 | 101.5 | | | | | | |
| CONTR OL | - | G | 0.48 | - | 0 | | | | | | |
| LYM 136 | 13423. 1 | A | 0.00 6 | 4.00E-06 | 156.7 | LYM 160 | 1347 2.1 | A | 0.01 | 0.00E +00 | 138.8 |
| LYM1 | 11601. 1 | A | 0.00 4 | 2.49E-04 | 69.1 | LYM136 | 1342 3.4 | A | 0.00 9 | 1.48E-04 | 118.5 |
| LYM 136 | 13421. 5 | A | 0.00 6 | 1.77E-03 | 129.9 | LYM293 | 1339 1.2 | A | 0.00 7 | 5.51E-03 | 57.6 |
| LYM84 | 13404. 4 | A | 0.00 4 | 3.79E-03 | 81.4 | LYM293 | 1339 1.4 | A | 0.00 9 | 8.63E-03 | 106 |
| LYM1 | 11602. 6 | A | 0.00 7 | 5.63E-03 | 182.5 | LYM 136 | 1342 1.7 | A | 0.00 8 | 1.58E-02 | 94 |
| LYM1 | 11603. 2 | A | 0.00 5 | 7.42E-03 | 125.8 | LYM 136 | 1342 1.8 | A | 0.00 8 | 2.02E-02 | 92.2 |
| LYM84 | 13401. 2 | A | 0.00 5 | 1.15E-02 | 91.8 | LYM160 | 1347 1.1 | A | 0.00 8 | 3.06E-02 | 95.2 |
| LYM84 | 13403. 1 | A | 0.00 7 | 1.33E-02 | 170.1 | LYM84 | 1340 3.1 | A | 0.00 8 | 3.34E-02 | 90.4 |
| LYM84 | 13403. 3 | A | 0.00 5 | 1.48E-02 | 99 | LYM 160 | 1347 2.2 | A | 0.00 7 | 3.80E-02 | 78.5 |
| LYM 136 | 13421. 6 | A | 0.00 5 | 2.79E-02 | 116.5 | LYM84 | 1340 3.3 | A | 0.01 2 | 6.33E-02 | 186 |
| LYM 136 | 13421. 8 | A | 0.00 7 | 2.82E-02 | 189.7 | LYM293 | 1339 2.2 | A | 0.00 7 | 6.99E-02 | 70.1 |
| LYM84 | 13403. 2 | A | 0.00 6 | 2.92E-02 | 156.7 | LYM84 | 1340 1.2 | A | 0.00 7 | 7.32E-02 | 66.6 |
| LYM1 | 11602. 1 | A | 0.00 4 | 3.31E-02 | 70.1 | LYM293 | 1339 1.9 | A | 0.00 7 | 7.86E-02 | 67.8 |
| LYM 136 | 13423. 2 | A | 0.00 4 | 3.85E-02 | 79.4 | LYM 136 | 1342 1.5 | A | 0.00 8 | 8.34E-02 | 99.4 |
| LYM1 | 11604. 4 | A | 0.00 3 | 5.00E-02 | 39.2 | LYM160 | 1347 1.4 | A | 0.00 6 | 9.24E-02 | 31.3 |
| CONTR OL | - | A | 0.00 2 | - | -0 | CONTR OL | - | A | 0.00 4 | - | 0 |
| LYM84 | 13401. 2 | B | 0.09 3 | 1.52E-03 | 80.5 | LYM160 | 1347 2.1 | B | 0.21 8 | 5.01E-03 | 128.9 |
| LYM1 | 11601. 1 | B | 0.09 | 4.19E-03 | 75.6 | LYM136 | 1342 3.4 | B | 0.19 | 7.33E-03 | 100 |
| LYM 136 | 13423. 1 | B | 0.12 5 | 5.82E-03 | 143.9 | LYM136 | 1342 1.8 | B | 0.17 3 | 1.03E-02 | 81.6 |
| LYM84 | 13403. 3 | B | 0.1 | 6.00E-03 | 95.1 | LYM293 | 1339 1.2 | B | 0.13 8 | 1.09E-02 | 44.7 |
| LYM 136 | 13421. 5 | B | 0.10 3 | 8.84E-03 | 100 | LYM84 | 1340 1.2 | B | 0.13 8 | 1.59E-02 | 44.7 |
| LYM136 | 13421. 6 | B | 0.12 3 | 9.57E-03 | 139 | LYM 136 | 1342 1.7 | B | 0.15 3 | 2.38E-02 | 60.5 |
| LYM1 | 11602. 6 | B | 0.13 | 1.32E-02 | 153.7 | LYM84 | 1340 3.3 | B | 0.27 5 | 3.54E-02 | 189.5 |
| LYM 136 | 13423. 2 | B | 0.08 8 | 1.41E-02 | 70.7 | LYM 160 | 1347 2.2 | B | 0.15 5 | 6.63E-02 | 63.2 |
| LYM136 | 13421. 8 | B | 0.15 5 | 2.23E-02 | 202.4 | LYM293 | 1339 1.4 | B | 0.19 3 | 7.66E-02 | 102.6 |
| LYM84 | 13403. 2 | B | 0.15 5 | 2.32E-02 | 202.4 | LYM 136 | 1342 1.5 | B | 0.18 | 8.25E-02 | 89.5 |
| LYM84 | 13404. 4 | B | 0.09 5 | 2.70E-02 | 85.4 | LYM160 | 1347 1.1 | B | 0.18 | 8.25E-02 | 89.5 |
| LYM1 | 11603. 2 | B | 0.10 8 | 2.70E-02 | 109.8 | LYM84 | 1340 3.1 | B | 0.19 5 | 8.89E-02 | 105.3 |
| LYM1 | 11604. 4 | B | 0.07 3 | 3.33E-02 | 41.5 | CONTR OL | - | B | 0.09 5 | - | 0 |
| LYM1 | 11602. 1 | B | 0.08 8 | 3.79E-02 | 70.7 | LYM 136 | 1342 1.5 | G | 1.16 | 1.40E-05 | 113.3 |
| LYM84 | 13403. I | B | 0.15 | 5.94E-02 | 192.7 | LYM160 | 1347 2.1 | G | 1.07 3 | 1.70E-05 | 97.2 |
| CONTR OL | - | B | 0.05 1 | - | 0 | LYM160 | 1347 1.1 | G | 0.97 3 | 1.00E-04 | 78.9 |
| LYM1 | 11601. 1 | G | 0.75 | 7.00E-06 | 88.7 | LYM84 | 1340 3.1 | G | 0.91 5 | 1.04E-04 | 68.3 |
| LYM84 | 13403. 1 | G | 0.75 | 8.00E-06 | 88.7 | LYM136 | 1342 3.4 | G | 1.08 | 1.69E-03 | 98.6 |
| LYM84 | 13404. 4 | G | 0.64 | 9.30E-05 | 61 | LYM 136 | 1342 1.8 | G | 1.03 5 | 3.80E-03 | 90.3 |
| LYM1 | 11603. 2 | G | 0.63 5 | 7.21E-04 | 59.7 | LYM293 | 1339 1.4 | G | 1.19 3 | 5.56E-03 | 119.3 |
| LYM 136 | 13421. 6 | G | 0.68 8 | 1.51E-03 | 73 | LYM84 | 1340 1.2 | G | 0.83 | 7.52E-03 | 52.6 |
| LYM84 | 13401. 2 | G | 0.68 8 | 2.17E-03 | 73 | LYM84 | 1340 3.3 | G | 1.41 8 | 7.77E-03 | 160.7 |
| LYM1 | 11602. 6 | G | 0.78 3 | 3.89E-03 | 96.9 | LYM160 | 1347 2.2 | G | 0.76 | 1.94E-02 | 39.8 |
| LYM 136 | 13423. 1 | G | 0.83 | 4.81E-03 | 108.8 | LYM136 | 1342 1.7 | G | 0.88 | 2.80E-02 | 61.8 |
| LYM84 | 13403. 3 | G | 0.72 | 4.83E-03 | 81.1 | LYM293 | 1339 1.2 | G | 0.74 | 4.83E-02 | 36. 1 |
| LYM 136 | 13421. 5 | G | 0.62 | 6.93E-03 | 56 | LYM293 | 1339 2.2 | G | 0.88 5 | 4.98E-02 | 62.8 |
| LYM84 | 13403. 2 | G | 0.78 5 | 1.19E-02 | 97.5 | LYM136 | 1342 3.2 | G | 0.79 5 | 8.04E-02 | 46.2 |
| LYM 136 | 13421. 8 | G | 0.87 8 | 1.65E-02 | 120.8 | LYM293 | 1339 1.9 | G | 0.81 3 | 8.66E-02 | 49.4 |
| LYM 136 | 13423. 2 | G | 0.61 | 2.94E-02 | 53.5 | LYM160 | 1347 1.4 | G | 0.72 5 | 9.93E-02 | 33.3 |
| LYM1 | 11604. 4 | G | 0.51 5 | 2.96E-02 | 29.6 | CONTR OL | | G | 0.54 4 | - | 0 |
| CONTR OL | - | G | 0.39 8 | - | 0 | LYM 136 | 1342 1.5 | H | 0.12 2 | 0.00E +00 | 119 |
| LYM 136 | 13423. 1 | H | 0.08 1 | 0.00E +00 | 101.6 | LYM160 | 1347 2.1 | H | 0.11 8 | 0.00E +00 | 110.6 |
| LYM84 | 13403. 1 | H | 0.07 6 | 0.00E +00 | 88.3 | LYM84 | 1340 3.3 | H | 0.15 | 1.00E-06 | 168.9 |
| LYM1 | 11602. 6 | H | 0.07 7 | 1.00E-06 | 91.4 | LYM 136 | 1342 3.4 | H | 0.11 6 | 3.00E-06 | 108.4 |
| LYM1 | 11601. 1 | H | 0.06 8 | 2.00E-06 | 70.4 | LYM293 | 1339 1.4 | H | 0.12 1 | 6.00E-06 | 116.8 |
| LYM84 | 13403. 2 | H | 0.08 3 | 2.00E-06 | 107 | LYM136 | 1342 1.8 | H | 0.10 6 | 7.20E-05 | 90.5 |
| LYM 136 | 13421. 8 | H | 0.08 7 | 4.00E-06 | 116.3 | LYM 160 | 1347 1.1 | H | 0.09 7 | 1.50E-04 | 74.2 |
| LYM 136 | 13421. 6 | H | 0.06 8 | 7.00E-06 | 68.1 | LYM84 | 1340 3.1 | H | 0.08 9 | 8.80E-04 | 59.1 |
| LYM84 | 13403. 3 | H | 0.07 | 9.00E-06 | 74.3 | LYM 136 | 1342 1.7 | H | 0.09 4 | 1.79E-03 | 68.6 |
| LYM84 | 13401. 2 | H | 0.06 5 | 2.60E-05 | 62.6 | LYM84 | 1340 1.2 | H | 0.08 6 | 3.12E-03 | 54.6 |
| LYM 136 | 13421. 5 | H | 0.06 6 | 2.80E-05 | 65 | LYM293 | 1339 2.2 | H | 0.09 3 | 3.73E-03 | 66.4 |
| LYM1 | 11603. 2 | H | 0.06 2 | 3.00E-05 | 54.9 | LYM 160 | 1347 2.2 | H | 0.08 5 | 5.45E-03 | 51.8 |
| LYM84 | 13404. 4 | H | 0.06 | 7.80E-05 | 48.6 | LYM293 | 1339 1.9 | H | 0.08 4 | 2.09E-02 | 50.7 |
| LYM 136 | 13423. 2 | H | 0.06 1 | 1.90E-03 | 51.8 | LYM293 | 1339 1.2 | H | 0.07 8 | 2.83E-02 | 40.1 |
| LYM1 | 11604. 4 | H | 0.05 4 | 4.45E-03 | 35.4 | LYM 136 | 1342 3.2 | H | 0.08 1 | 3.88E-02 | 44.5 |
| LYM1 | 11602. 1 | H | 0.05 3 | 1.86E-02 | 31.5 | LYM 160 | 1347 1.4 | H | 0.07 5 | 6.82E-02 | 34.5 |
| CONTR OL | - | H | 0.04 | - | 0 | CONTR OL | | H | 0.05 6 | - | 0 |
| LYM84 | 13403. 2 | C | 1.02 7 | 1.40E-05 | 80.2 | LYM136 | 1342 1.5 | C | 0.90 8 | 0.00E +00 | 100.7 |
| LYM136 | 13423. 1 | C | 1.02 3 | 1.50E-05 | 79.7 | LYM 136 | 1342 1.8 | C | 0.87 3 | 0.00E +00 | 93.1 |
| LYM 136 | 13421. 8 | C | 1.02 8 | 7.30E-05 | 80.6 | LYM293 | 1339 1.2 | C | 0.79 2 | 0.00E +00 | 75.2 |
| LYM84 | 13403. 1 | C | 0.89 3 | 1.60E-04 | 56.8 | LYM160 | 1347 2.1 | C | 0.72 1 | 1.00E-06 | 59.5 |
| LYM84 | 13403. 3 | C | 0.86 7 | 6.59E-04 | 52.3 | LYM84 | 1340 3.3 | C | 1.08 9 | 1.00E-06 | 140.8 |
| LYM136 | 13421. 6 | C | 0.85 3 | 2.33E-03 | 49.8 | LYM136 | 1342 3.2 | C | 0.83 1 | 3.00E-06 | 83.8 |
| LYM136 | 13423. 2 | C | 0.86 4 | 3.27E-03 | 51.7 | LYM293 | 1339 1.4 | C | 1.07 1 | 3.00E-06 | 136.8 |
| LYM1 | 11603. 2 | C | 0.81 7 | 3.91E-03 | 43.4 | LYM160 | 1347 1.1 | C | 0.77 2 | 3.10E-05 | 70.6 |
| LYM84 | 13401. 2 | C | 0.80 4 | 4.08E-03 | 41.2 | LYM84 | 1340 1.2 | C | 0.69 2 | 3.40E-05 | 52.9 |
| LYM1 | 11602. 6 | C | 0.86 2 | 5.42E-03 | 51.3 | LYM 136 | 1342 3.4 | C | 0.77 7 | 4.90E-05 | 71.7 |
| LYM1 | 11601. 1 | C | 0.78 9 | 1.01E-02 | 38.6 | LYM160 | 1347 2.2 | C | 0.67 2 | 1.41E-04 | 48.7 |
| LYM1 | 11604. 4 | C | 0.82 3 | 1.54E-02 | 44.5 | LYM84 | 1340 3.1 | C | 0.64 6 | 2.2 1E-04 | 42.9 |
| LYM84 | 13404. 4 | C | 0.73 4 | 2.94E-02 | 28.8 | LYM293 | 1339 1.9 | C | 0.76 3 | 2.73E-04 | 68.7 |
| LYM1 | 11602. 1 | C | 0.72 7 | 4.75E-02 | 27.6 | LYM 136 | 1342 1.7 | C | 0.72 7 | 8.57E-04 | 60.7 |
| LYM136 | 13421. 5 | C | 0.71 5 | 7.12E-02 | 25.5 | LYM293 | 1339 2.2 | C | 0.66 8 | 1.97E-02 | 47.8 |
| CONTR OL | - | C | 0.57 | - | 0 | CONTR OL | - | C | 0.45 2 | - | 0 |
| LYM1 | 11604. 4 | E | 0.58 3 | 3.46E-03 | 26.5 | LYM84 | 1340 3.3 | E | 0.55 9 | 5.55E-02 | 23 |
| LYM84 | 13403. 2 | E | 0.57 4 | 1.03E-02 | 24.6 | CONTROL | - | E | 0.45 4 | - | 0 |
| LYM136 | 13421. 8 | E | 0.55 1 | 1.41E-02 | 19.7 | LYM136 | 1342 1.8 | D | 7.68 5 | 9.00E-05 | 87.2 |
| LYM84 | 13403. 3 | E | 0.56 2 | 1.74E-02 | 21.9 | LYM 136 | 1342 1.5 | D | 8.41 8 | 1.06E-04 | 105 |
| LYM1 | 11601. 1 | E | 0.54 9 | 2.97E-02 | 19.1 | LYM160 | 1347 2.1 | D | 6.28 | 5.37E-04 | 52.9 |
| LYM84 | 13401. 2 | E | 0.54 3 | 4.13E-02 | 17.9 | LYM293 | 1339 1.2 | D | 6.82 3 | 2.80E-03 | 66.1 |
| LYM84 | 13403. 1 | E | 0.53 5 | 4.77E-02 | 16.1 | LYM84 | 1340 3.1 | D | 5.88 | 8.55E-03 | 43.2 |
| LYM84 | 13404. 4 | E | 0.52 8 | 5.27E-02 | 14.6 | LYM84 | 1340 1.2 | D | 6.16 5 | 9.88E-03 | 50.1 |
| LYM 136 | 13423. 2 | E | 0.54 3 | 5.95E-02 | 18 | LYM 136 | 1342 3.2 | D | 7.68 5 | 1.61E-02 | 87.2 |
| LYM1 | 11602. 1 | E | 0.52 5 | 6.56E-02 | 13.9 | LYM 160 | 1347 1.1 | D | 6.81 8 | 1.69E-02 | 66 |
| CONTR OL | - | E | 0.46 1 | - | 0 | LYM84 | 1340 3.3 | D | 9.51 8 | 2.04E-02 | 131.8 |
| LYM 136 | 13423. 1 | D | 8.86 3 | 8.70E-05 | 75.8 | LYM 136 | 1342 3.4 | D | 7.01 5 | 2.44E-02 | 70.8 |
| LYM84 | 13403. 1 | D | 7.74 8 | 3.02E-03 | 53.7 | LYM160 | 1347 2.2 | D | 5.75 8 | 2.55E-02 | 40.2 |
| LYM84 | 13403. 2 | D | 8.73 8 | 5.76E-03 | 73.4 | LYM293 | 1339 1.4 | D | 9.49 | 2.56E-02 | 131.1 |
| LYM84 | 13403. 3 | D | 7.59 8 | 8.31E-03 | 50.7 | LYM293 | 1339 1.9 | D | 6.7 | 4.90E-02 | 63.2 |
| LYM84 | 13401. 2 | D | 7.03 8 | 9.19E-03 | 39.6 | LYM 136 | 1342 1.7 | D | 6.53 3 | 5.11E-02 | 59.1 |
| LYM84 | 13404. 4 | D | 6.51 5 | 1.63E-02 | 29.3 | CONTR OL | - | D | 4.10 6 | - | 0 |
| LYM1 | 11603. 2 | D | 7.22 3 | 2.00E-02 | 43.3 | LYM136 | 1342 1.5 | F | 6.76 5 | 1.52E-03 | 21.7 |
| LYM 136 | 13421. 8 | D | 8.99 3 | 2.33E-02 | 78.4 | LYM 136 | 1342 3.2 | F | 6.73 | 2.68E-03 | 21.1 |
| LYM1 | 11601. 1 | D | 6.88 8 | 2.44E-02 | 36.7 | LYM 136 | 1342 1.8 | F | 6.94 3 | 3.01E-03 | 24.9 |
| LYM 136 | 13421. 6 | D | 7.33 5 | 3.72E-02 | 45.5 | LYM84 | 1340 1.2 | F | 6.2 | 9.41E-03 | 11.5 |
| LYM 136 | 13423. 2 | D | 7.40 5 | 4.76E-02 | 46.9 | LYM136 | 1342 1.7 | F | 6.38 | 3.57E-02 | 14.8 |
| LYM1 | 11602. 6 | D | 7.60 5 | 4.83E-02 | 50.9 | LYM293 | 1339 1.9 | F | 6.22 3 | 4.78E-02 | 11.9 |
| LYM1 | 11602. 1 | D | 6.27 8 | 4.96E-02 | 24.6 | LYM84 | 1340 3.3 | F | 6.78 3 | 5.99E-02 | 22 |
| CONTR OL | - | D | 5.04 | - | 0 | LYM293 | 1339 1.2 | F | 6.20 8 | 7.14E-02 | 11.7 |
| LYM 136 | 13421. 8 | F | 7.01 8 | 1.11E-04 | 27.1 | LYM 160 | 1347 1.1 | F | 6.12 5 | 9.99E-02 | 10.2 |
| LYM84 | 13404. 4 | F | 6.47 8 | 3.96E-04 | 17.3 | CONTR OL | - | F | 5.55 9 | - | 0 |
| LYM 136 | 13423.1 | F | 6.81 | 7.54E-04 | 23.3 | | | | | | |
| LYM1 | 11601. 1 | F | 6.77 5 | 2.36E-03 | 22.7 | | | | | | |
| LYM84 | 13403. 3 | F | 6.76 5 | 3.61E-03 | 22.5 | | | | | | |
| LYM84 | 13403. 1 | F | 6.53 | 4.49E-03 | 18.3 | | | | | | |
| LYM84 | 13403. 2 | F | 6.90 3 | 5.58E-03 | 25 | | | | | | |
| LYM1 | 11603. 2 | F | 6.81 3 | 6.13E-03 | 23.4 | | | | | | |
| LYM 136 | 13423. 2 | F | 6.79 | 7.54E-03 | 23 | | | | | | |
| LYM84 | 13401. 2 | F | 6.63 3 | 1.04E-02 | 20.1 | | | | | | |
| LYM1 | 11602. 1 | F | 6.27 5 | 1.24E-02 | 13.7 | | | | | | |
| LYM136 | 13421. 6 | F | 6.11 3 | 1.93E-02 | 10.7 | | | | | | |
| LYM1 | 11604. 4 | F | 6.70 8 | 2.78E-02 | 21.5 | | | | | | |
| CONTR OL | - | F | 5.52 1 | - | 0 | | | | | | |

Table 34. Results of the tissue culture experiments. Provided are the measured values of each tested parameter [parameters (ID.) A-F according to the parameters described in Table 33 above] in plants expressing the indicated polynucleotides. "Ev" = event; "P" = P-value; "Mean " = the average of measured parameter across replicates. % incr. vs. cont. = percentage of increase versus control (as compared to control).

**Table 35**

| ***Measured parameters at tire tissue culture assay (T1 experiment) for transformed agriculture improving trait genes*** | |
|---|---|
| Tested Parameters | ID |
| Dry Weight (gr) | A |
| Fresh Weight (gr) | B |
| RGR Of Root Coverage | C |
| Roots Coverage TP3 (cm²) | D |
| RGR Of Roots Length | E |
| Roots Length TP3 (cm) | F |
| Leaf Area TP3 (cm) | G |
| RGR Of Leaf Area | H |
| Leaf Area TP1 (cm) | I |
| Roots Length TP1 (cm) | J |

Table 35: Provided are the identification (ID) letters of each of the Tested Parameters.
TP3 = Time point 3; RGR - relative growth rate. TP1 = Time point 1.

**Table 36**

| ***Results obtained in a T1 experiment at the tissue culture assay*** | | | | | | | | | |
|---|---|---|---|---|---|---|---|---|---|
| ***Gene name*** | ***ID*** | ***Mean*** | ***P value*** | **% *incr. vs. cont*** | ***Gene name*** | ***ID*** | ***Mean*** | ***P value*** | **% *incr. vs*. *cont*** |
| LYM161 | A | 0.014 | 1.24E-03 | 90.4 | LYM240 | H | 0.078 | 2.23E-02 | 31.2 |
| LYM219 | A | 0.01 | 2.07E-03 | 28.3 | LYM228 | H | 0.077 | 2.59E-02 | 29.8 |
| LYM83 | A | 0.01 | 1.93E-02 | 30.6 | LYM 189 | H | 0.076 | 3.81E-02 | 28 |
| LYM278 | A | 0.009 | 3.67E-02 | 25.9 | LYM184 | H | 0.082 | 4.23E-02 | 37.8 |
| LYM204 | A | 0.01 | 5.84E-02 | 36.3 | LYM248 | H | 0.076 | 6.24E-02 | 27.1 |
| LYM 155 | A | 0.013 | 6.05E-02 | 70.7 | CONTROL | H | 0.06 | - | 0 |
| LYM22 1 | A | 0.009 | 9.70E-02 | 22.9 | LYM117 | A | 0.009 | 5.26E-04 | 65.7 |
| CONTROL | - | 0.007 | - | 0 | LYM146 | A | 0.01 | 1.17E-02 | 81.5 |
| LYM161 | B | 0.282 | 9.40E-05 | 75.5 | LYM144 | A | 0.007 | 1.616-02 | 34.3 |
| LYM83 | B | 0.213 | 3.45E-03 | 32.5 | LYM93 | A | 0.008 | 7.31E-02 | 43.5 |
| LYM219 | B | 0.207 | 2.66E-02 | 28.8 | LYM 123 | A | 0.006 | 7.96E-02 | 19 |
| LYM204 | B | 0.207 | 6.73E-02 | 28.8 | LYM 127 | A | 0.007 | 9.16E-02 | 32.1 |
| LYM155 | B | 0.267 | 9.60E-02 | 66.3 | CONTROL | A | 0.005 | - | 0 |
| LYM278 | B | 0.191 | 9.94E-02 | 18.9 | LYM117 | B | 0.198 | 7.40E-05 | 75.7 |
| CONTROL | - | 0.161 | - | 0 | LYM 127 | B | 0.159 | 8.58E-04 | 41.3 |
| LYM221 | C | 0.468 | 1.90E-03 | 94.9 | LYM146 | B | 0.186 | 8.64E-03 | 65.3 |
| LYM 155 | C | 0.468 | 2.86E-03 | 95.2 | LYM 192 | B | 0.148 | 1.44E-02 | 31.7 |
| LYMI61 | C | 0.484 | 3.4 1E. 03 | 101.5 | LYM144 | B | 0.153 | 4.48E-02 | 35.5 |
| LYM 188 | C | 0.4 | 2.12E-02 | 66.5 | LYM93 | B | 0.172 | 5.30E-02 | 52.3 |
| LYM144 | C | 0.355 | 9.94E-02 | 48.1 | LYM 135 | B | 0.178 | 5.72E-02 | 58 |
| CONTROL | - | 0.24 | - | 0 | LYM123 | B | 0.156 | 5.80E-02 | 38.4 |
| LYM188 | D | 3.425 | 1.59E-03 | 67.6 | LYM200 | B | 0.153 | 6.00E-02 | 35.5 |
| LYM221 | D | 3.964 | 1.79E-03 | 94 | CONTROL | B | 0.113 | - | 0 |
| LYM155 | D | 3.85 | 1.26E-02 | 88.4 | LYM 135 | C | 0.625 | 1.90E-05 | 79.8 |
| LYM 144 | D | 3.038 | 4.84E-02 | 48.6 | LYM 127 | C | 0.614 | 5.30E-05 | 76.4 |
| LYM161 | D | 4.023 | 7.69E-02 | 96.8 | LYM146 | C | 0.553 | 1.54E-04 | 58.8 |
| CONTROL | - | 2.044 | - | 0 | LYM117 | C | 0.537 | 5.03E-04 | 54.2 |
| LYM155 | E | 0.444 | 9.80E-03 | 58.2 | LYM 154 | C | 0.56 | 1.78E-03 | 60.9 |
| LYM221 | E | 0.413 | 2.08E-02 | 47 | LYM 192 | C | 0.492 | 3.37E-03 | 41.5 |
| LYM188 | E | 0.41 | 2.14E-02 | 46.1 | LYM93 | C | 0.477 | 8.26E-03 | 37 |
| LYM161 | E | 0.412 | 2.44E-02 | 46.6 | LYM200 | C | 0.483 | 9.48E-03 | 38.9 |
| LYM144 | E | 0.405 | 2.83E-02 | 44.2 | LYM144 | C | 0.473 | 1.14E-02 | 35.8 |
| CONTROL | - | 0.281 | - | 0 | LYM123 | C | 0.473 | 1.75E-02 | 35.8 |
| LYM188 | F | 4.053 | 6.86E-04 | 41.3 | LYM273 | C | 0.473 | 1.83E-02 | 35.8 |
| LYM144 | F | 3.97 | 2.09E-03 | 38.4 | CONTROL | C | 0.348 | - | 0 |
| LYM221 | F | 4.058 | 3.24E-03 | 41.5 | LYM 192 | D | 4.217 | 1.10E-03 | 42.2 |
| LYM161 | F | 3.914 | 2.30E-02 | 36.5 | LYM93 | D | 4.075 | 1.12E-03 | 37.4 |
| LYM278 | F | 3.493 | 5.26E-02 | 21.8 | LYM144 | D | 4.057 | 4.97E-03 | 36.8 |
| LYM155 | F | 4.084 | 5.40E-02 | 42.4 | LYM117 | D | 4.4 | 7.86E-03 | 48.4 |
| LYM204 | F | 3.68 | 6.02E-02 | 28.3 | LYM146 | D | 4.555 | 9.70E-03 | 53.6 |
| CONTROL | - | 2.868 | - | 0 | LYM200 | D | 4.151 | 3.73E-02 | 40 |
| LYM83 | G | 0.883 | 2.63E-04 | 31.7 | LYM 135 | D | 5.162 | 4.62E-02 | 74 |
| LYM161 | G | 1.129 | 1.06E-03 | 68.3 | LYM127 | D | 5.078 | 5.37E-02 | 71.2 |
| LYM221 | G | 0.856 | 4.40E-03 | 27.7 | LYM 123 | D | 4.062 | 7.29E-02 | 37 |
| LYM278 | G | 0.834 | 9.29E-03 | 24.3 | CONTROL | D | 2.966 | - | 0 |
| LYM155 | G | 1.024 | 2.05E-02 | 52.8 | LYM127 | E | 0.535 | 9.11E-04 | 48.7 |
| LYM144 | G | 0.823 | 3.31E-02 | 22.7 | LYM 135 | E | 0.508 | 2.21E-03 | 41 |
| LYM204 | G | 0.789 | 4.70E-02 | 17.6 | LYM117 | E | 0.463 | 1.53E-02 | 28.7 |
| CONTROL | - | 0.671 | - | 0 | LYM93 | E | 0.448 | 2.20E-02 | 24.5 |
| LYM161 | H | 0.122 | 4.00E-06 | 71.4 | LYM 192 | E | 0.444 | 3.60E-02 | 23.2 |
| LYM 155 | H | 0.112 | 2.07E-04 | 56.9 | LYM154 | E | 0.456 | 4.57E-02 | 26.7 |
| LYM83 | H | 0.096 | 7.84E-03 | 35.1 | CONTROL | E | 0.36 | - | 0 |
| LYM278 | H | 0.09 | 3.91E-02 | 26.5 | LYM93 | F | 4.431 | 8.92E-04 | 21.7 |
| LYM221 | H | 0.089 | 4.80E-02 | 25.3 | LYM192 | F | 4.435 | 9.73E-03 | 21.8 |
| LYM144 | H | 0.088 | 6.23E-02 | 23.8 | LYM144 | F | 4.132 | 6.49E-02 | 13.5 |
| CONTROL | - | 0.071 | - | 0 | LYM 123 | F | 4.108 | 7.42E-02 | 12.8 |
| LYM227 | A | 0.01 | 8.00E-05 | 53.1 | LYM135 | F | 4.683 | 9.16E-02 | 28.6 |
| LYM32 | A | 0.007 | 1.81E-03 | 19.8 | CONTROL | F | 3.641 | - | 0 |
| LYM40 | A | 0.009 | 1.92E-03 | 49.9 | LYM127 | G | 0.72 | 6.12E-04 | 22.6 |
| LYM273 | A | 0.009 | 2.65E-03 | 44.7 | LYM117 | G | 0.904 | 2.69E-03 | 53.9 |
| LYM273 | A | 0.01 | 3.12E-03 | 63.1 | LYM 192 | G | 0.701 | 3.05E-03 | 19.3 |
| LYM200 | A | 0.01 | 8.53E-03 | 57.9 | LYM135 | G | 0.885 | 1.05E-02 | 50.7 |
| LYM118 | A | 0.008 | 2.84E-02 | 24.2 | LYM 146 | G | 0.854 | 1.87E-02 | 45.4 |
| LYM 164 | A | 0.009 | 3.85E-02 | 39.1 | LYM 144 | G | 0.693 | 2.45E-02 | 18 |
| LYM23 | A | 0.008 | 6.86E-02 | 32.3 | LYM93 | G | 0.737 | 9.00E-02 | 25.4 |
| LYM93 | A | 0.01 | 8.67E-02 | 61.9 | CONTROL | G | 0.588 | - | 0 |
| LYM 122 | A | 0.008 | 9.35E-02 | 28.3 | LYM117 | H | 0.095 | 1.50E-05 | 62.9 |
| CONTROL | - | 0.006 | - | 0 | LYM 135 | H | 0.09 | 4.07E-04 | 55.5 |
| LYM227 | B | 0.248 | 4.10E-05 | 47.9 | LYM146 | H | 0.085 | 9.67E-04 | 46.9 |
| LYM40 | B | 0.216 | 149E-04 | 28.6 | LYM93 | H | 0.075 | 2.94E-02 | 29.7 |
| LYM273 | B | 0.231 | 7.82E-03 | 37.8 | LYM127 | H | 0.073 | 7.67E-02 | 25.7 |
| LYM200 | B | 0.251 | 1.45E-02 | 49.8 | CONTROL | H | 0.058 | - | 0 |
| LYM 146 | B | 0.229 | 1.83E-02 | 36.6 | LYM180 | A | 0.006 | 1.39E-04 | 44.3 |
| LYM273 | B | 0.249 | 1.90E-02 | 48.6 | LYM243 | A | 0.006 | 3.56E-03 | 64.4 |
| LYM164 | B | 0.222 | 2.09E-02 | 32.2 | LYM194 | A | 0.006 | 9.85E-03 | 62.5 |
| LYM154 | B | 0.216 | 3.87E-02 | 28.8 | LYM221 | A | 0.005 | 4.06E-02 | 34.6 |
| LYM 122 | B | 0.189 | 6.35E-02 | 12.9 | LYM204 | A | 0.006 | 4.55E-02 | 60.5 |
| LYM 135 | B | 0.197 | 7.69E-02 | 17.4 | LYM 109 | A | 0.005 | 6.49E-02 | 29.4 |
| CONTROL | - | 0.168 | - | 0 | LYM233 | A | 0.007 | 7.12E-02 | 70.9 |
| LYM234 | C | 0.482 | 5.87E-02 | 32.1 | CONTROL | A | 0.004 | - | 0 |
| LYM200 | C | 0.469 | 6.18E-02 | 28.7 | LYM243 | B | 0.148 | 1.67E-02 | 28.8 |
| LYM227 | C | 0.47 | 6.80E-02 | 28.9 | CONTROL | B | 0.115 | - | 0 |
| LYM154 | C | 0.481 | 7.23E-02 | 31.9 | LYM233 | C | 0.383 | 4.28E-04 | 55.4 |
| CONTROL | - | 0.364 | - | 0 | LYM204 | C | 0.377 | 6.67E-04 | 52.8 |
| LYM200 | D | 3.874 | 5.17E-02 | 27.9 | LYM248 | C | 0.377 | 1.32E-03 | 53.1 |
| CONTROL | - | 3.029 | - | 0 | LYM194 | C | 0.366 | 1.66E-03 | 48.4 |
| LYM273 | G | 1.057 | 1.01E-04 | 23 | LYM 186 | C | 0.387 | 1.93E-03 | 57.1 |
| LYM227 | G | 1.033 | 1.50E-04 | 20.2 | LYM243 | C | 0.347 | 4.89E-03 | 40.9 |
| LYM200 | G | 1.012 | 2.71E-04 | 17.8 | LYM83 | C | 0.349 | 5.96E-03 | 41.5 |
| LYM273 | G | 1.027 | 2.79E-04 | 19.6 | LYM 109 | C | 0.34 | 1.16E-02 | 38 |
| LYM 154 | G | 1.022 | 1.12E-02 | 18.9 | LYM228 | C | 0.336 | 1.95E-02 | 36.4 |
| LYM164 | G | 1.027 | 8.89E-02 | 19.5 | LYM115 | C | 0.339 | 1.96E-02 | 37.7 |
| LYM146 | G | 1.063 | 8.89E-02 | 23.7 | LYM252 | C | 0.34 | 2.33E-02 | 38 |
| LYM40 | G | 0.97 | 9.15E-02 | 12.9 | CONTROL | C | 0.246 | - | 0 |
| LYM 135 | G | 1.045 | 9.75E-02 | 21.6 | LYM243 | D | 2.941 | 4.12E-03 | 38.7 |
| CONTROL | - | 0.859 | - | 0 | LYM115 | D | 2.938 | 6.77E-03 | 38.6 |
| LYM93 | H | 0.117 | 2.44E-02 | 29.7 | LYM 194 | D | 3.087 | 8.47E-03 | 45.6 |
| LYM 146 | H | 0.112 | 3.08E-02 | 24.4 | LYM233 | D | 3.284 | 1.36E-02 | 54.9 |
| LYM273 | H | 0.108 | 4.22E-02 | 20.3 | LYM204 | D | 3.176 | 2.25E-02 | 49.8 |
| LYM273 | H | 0.107 | 5.78E-02 | 19 | LYM83 | D | 2.959 | 3.59E-02 | 39.6 |
| LYM 154 | H | 0.108 | 6.06E-02 | 19.8 | LYM 109 | D | 2.886 | 4.28E-02 | 36.1 |
| LYM164 | H | 0.108 | 6.30E-02 | 20 | LYM248 | D | 3.188 | 5.13E-02 | 50.4 |
| LYM135 | H | 0.108 | 7.21E-02 | 19.8 | CONTROL | D | 2.12 | - | 0 |
| LYM227 | H | 0.106 | 7.58E-02 | 17.7 | LYM248 | E | 0.416 | 9.58E-03 | 27.1 |
| LYM200 | H | 0.106 | 7.94E-02 | 17.6 | LYM204 | E | 0.389 | 6.24E-02 | 19.1 |
| LYM234 | H | 0.107 | 8.46E-02 | 18.6 | LYM233 | E | 0.39 | 7.40E-02 | 19.4 |
| CONTROL | - | 0.09 | - | 0 | LYM83 | E | 0.385 | 8.68E-02 | 17.7 |
| LYM131 | A | 0.008 | 2.70E-02 | 60.5 | CONTROL | E | 0.327 | - | 0 |
| LYM 194 | A | 0.007 | 5.17E-02 | 36.9 | LYM115 | F | 3.729 | 2.49E-03 | 13.6 |
| LYM261 | A | 0.006 | 5.62E-02 | 29.7 | LYM248 | F | 4.115 | 5.82E-03 | 25.3 |
| LYM185 | A | 0.01 | 5.77E-02 | 99.7 | LYM228 | F | 3.665 | 2.44E-02 | 11.6 |
| LYM 123 | A | 0.007 | 7.73E-02 | 39 | LYM204 | F | 3.852 | 4.19E-02 | 17.3 |
| LYM 192 | A | 0.006 | 7.80E-02 | 14.4 | LYM83 | F | 3.788 | 6.51E-02 | 15.4 |
| CONTROL | - | 0.005 | - | 0 | LYM233 | F | 3.97 | 8.99E-02 | 20.9 |
| LYM252 | B | 0.165 | 5.43E-03 | 38.4 | CONTROL | F | 3.284 | - | 0 |
| LYM131 | B | 0.181 | 2.17E-02 | 51.5 | LYM233 | G | 0.768 | 5.26E-03 | 41.5 |
| LYM194 | B | 0.164 | 3.02E-02 | 37.5 | LYM194 | G | 0.73 | 1.12E-02 | 34.5 |
| LYM185 | B | 0.225 | 3.55E-02 | 87.9 | LYM228 | G | 0.597 | 4.27E-02 | 9.8 |
| LYM123 | B | 0.154 | 4.39E-02 | 28.9 | LYM243 | G | 0.676 | 4.45E-02 | 24.5 |
| CONTROL | - | 0.12 | - | 0 | LYM215 | G | 0.626 | 6.82E-02 | 15.4 |
| LYM185 | C | 0.527 | 6.00E-06 | 97.4 | LYM204 | G | 0.697 | 8.47E-02 | 28.3 |
| LYM 123 | C | 0.495 | 1.50E-05 | 85.7 | CONTROL | G | 0.543 | - | 0 |
| LYM131 | C | 0.454 | 2.90E-05 | 70.4 | LYM233 | H | 0.078 | 1.07E-04 | 38.3 |
| LYM194 | C | 0.413 | 1.09E-03 | 54.7 | LYM 194 | H | 0.077 | 4.96E-04 | 36.3 |
| LYM243 | C | 0.406 | 3.94E-03 | 52.1 | LYM204 | H | 0.069 | 2.23E-02 | 23.5 |
| LYM252 | C | 0.375 | 1.15E-02 | 40.5 | LYM243 | H | 0.068 | 2.43E-02 | 20.9 |
| LYM233 | C | 0.366 | 1.81E-02 | 37.2 | LYM252 | H | 0.071 | 2.64E-02 | 26.5 |
| LYM215 | C | 0.338 | 7.24E-02 | 26.7 | CONTROL | H | 0.056 | - | 0 |
| CONTROL | - | 0.267 | - | 0 | LYM 189 | A | 0.009 | 3.27E-04 | 32.5 |
| LYM 131 | D | 3.668 | 3.37E-04 | 67.4 | LYM32 | A | 0.01 | 8.84E-03 | 51.3 |
| LYM215 | D | 2.788 | 1.33E-02 | 27.2 | LYM219 | A | 0.01 | 4.76E-02 | 49.4 |
| LYM194 | D | 3.34 | 2.15E-02 | 52.4 | LYM240 | A | 0.009 | 5.15E-02 | 33.2 |
| LYM 123 | D | 4.004 | 3.68E-02 | 82.7 | LYM161 | A | 0.01 | 7.76E-02 | 52 |
| LYM 185 | D | 4.257 | 5.21E-02 | 94.2 | CONTROL | A | 0.007 | - | 0 |
| LYM233 | D | 3.014 | 6.12E-02 | 37.5 | LYM32 | B | 0.19 | 9.39E-04 | 27.5 |
| LYM252 | D | 3.036 | 7.43E-02 | 38.5 | LYM249 | B | 0.225 | 2.56E-03 | 51 |
| CONTROL | - | 2.192 | - | 0 | LYM 189 | B | 0.21 | 3.29E-02 | 40.5 |
| LYM131 | E | 0.469 | 8.00E-06 | 42.8 | LYM 193 | B | 0.193 | 6.69E-02 | 29.1 |
| LYM185 | E | 0.47 | 2.28E- 04 | 43.1 | LYM261 | B | 0.177 | 7.40E-02 | 18.5 |
| LYM123 | E | 0.448 | 3.49E-04 | 36.4 | CONTROL | B | 0.149 | - | 0 |
| LYM 194 | E | 0.411 | 6.53E-03 | 25.3 | LYM234 | C | 0.531 | 0.00E+00 | 123.9 |
| LYM243 | E | 0.407 | 8.35E-03 | 24 | LYM249 | C | 0.539 | 0.00E+00 | 127.6 |
| LYM252 | E | 0.383 | 7.84E-02 | 16.6 | LYM219 | C | 0.517 | 7.00E-06 | 118.2 |
| CONTROL | - | 0.328 | - | 0 | LYM240 | C | 0.415 | 2.30E-05 | 75.1 |
| LYM131 | F | 4.042 | 7.90E-05 | 32 | LYM74 | C | 0.443 | 6.30E-05 | 86.9 |
| LYM243 | F | 3.668 | 1.22E-02 | 19.8 | LYM179 | C | 0.406 | 1.06E-04 | 71.1 |
| LYM215 | F | 3.383 | 3.49E-02 | 10.5 | LYM161 | C | 0.409 | 1.34E-04 | 72.4 |
| LYM194 | F | 3.635 | 4.63E-02 | 18.8 | LYM79 | C | 0.412 | 1.71E-04 | 73.6 |
| LYM123 | F | 3.912 | 5.09E-02 | 27.8 | LYM188 | C | 0.38 | 5.35E-04 | 60.3 |
| LYM185 | F | 4.091 | 7.35E-02 | 33.7 | LYM278 | C | 0.376 | 1.23E-03 | 58.5 |
| CONTROL | - | 3.061 | - | 0 | LYM26 1 | C | 0.369 | 1.75E-03 | 55.6 |
| LYM131 | G | 0.834 | 1.00E-06 | 54 | LYM224 | C | 0.382 | 3.15E-03 | 61.3 |
| LYM123 | G | 0.78 | 2.30E-50 | 44.1 | LYM199 | C | 0.349 | 5.89E-03 | 47.3 |
| LYM233 | G | 0.643 | 8.49E-03 | 18.8 | LYM32 | C | 0.341 | 2.37E-02 | 44 |
| LYM252 | G | 0.713 | 2.16E-02 | 31.8 | LYM 193 | C | 0.322 | 4.86E-02 | 35.6 |
| LYM243 | G | 0.656 | 2.67E-02 | 21.2 | LYM155 | C | 0.302 | 9.72E-02 | 27.2 |
| LYM192 | G | 0.669 | 2.77E-02 | 23.5 | CONTROL | C | 0.237 | | 0 |
| LYM26 1 | G | 0.637 | 3.41E-02 | 17.7 | LYM240 | D | 3.448 | 1.00E-06 | 67.3 |
| LYM194 | G | 0.715 | 5.43E-02 | 32 | LYM188 | D | 3.137 | 6.00E-06 | 52.2 |
| LYM185 | G | 0.825 | 5.58E-02 | 52.4 | LYM179 | D | 3.35 | 9.72E-04 | 62.6 |
| CONTROL | - | 0.541 | - | 0 | LYM189 | D | 2.884 | 1.31E-03 | 39.9 |
| LYM131 | H | 0.087 | 3.00E-06 | 53.4 | LYM234 | D | 4.399 | 4.93E-03 | 113.5 |
| LYM185 | H | 0.092 | 1.70E-05 | 62.1 | LYM249 | D | 4.486 | 8.04E-03 | 117.7 |
| LYM123 | H | 0.083 | 3.10E-05 | 46.2 | LYM74 | D | 3.651 | 9.49E-03 | 77.2 |
| LYM252 | H | 0.077 | 2.13E-03 | 34.4 | LYM261 | D | 3.07 | 2.24E-02 | 49 |
| LYM 194 | H | 0.077 | 3.63E-03 | 34.1 | LYM161 | D | 3.381 | 2.29E-02 | 64.1 |
| LYM 192 | H | 0.073 | 1.17E-02 | 27.3 | LYM278 | D | 3.171 | 2.40E-02 | 53.9 |
| LYM261 | H | 0.069 | 4.77E-02 | 20.8 | LYM79 | D | 3.427 | 3.04E-02 | 66.3 |
| LYM243 | H | 0.068 | 7.19E-02 | 18.9 | LYM219 | D | 4.274 | 7.53E-02 | 107.4 |
| CONTROL | - | 0.057 | - | 0 | LYM155 | D | 2.558 | 8.03E-02 | 24.1 |
| LYM245 | A | 0.008 | 1.20E-02 | 38.7 | CONTROL | D | 2.061 | - | 0 |
| LYM228 | A | 0.008 | 1.47E-02 | 44.3 | LYM249 | E | 0.438 | 5.00E-06 | 53.9 |
| LYM240 | A | 0.008 | 2.80E-02 | 45.2 | LYM234 | E | 0,431 | 3.10E-05 | 51.7 |
| LYM260 | A | 0.007 | 3.74E-02 | 27.4 | LYM219 | E | 0.401 | 1.65E-03 | 41.2 |
| LYM 189 | A | 0.007 | 3.75E-02 | 27 | LYM224 | E | 0.397 | 2.21E-03 | 39.7 |
| LYM 184 | A | 0.009 | 5.19E-02 | 49.1 | LYM 179 | E | 0.378 | 3.93E-03 | 33 |
| CONTROL | - | 0.006 | - | 0 | LYM79 | E | 0.383 | 5.24E-03 | 34.7 |
| LYM245 | B | 0.16 | 1.51E-02 | 41.9 | LYM240 | E | 0.362 | 8.54E-03 | 27.4 |
| LYM260 | B | 0.15 | 2.23E-02 | 33 | LYM74 | E | 0.373 | 9.36E-03 | 31.3 |
| LYM 189 | B | 0.136 | 4.67E-02 | 20.6 | LYM 189 | E | 0.352 | 2.77E-02 | 23.9 |
| LYM240 | B | 0.144 | 5.98E-02 | 27.5 | LYM261 | E | 0.35 | 3.22E-02 | 23 |
| LYM228 | B | 0.148 | 7.33E-02 | 31.1 | LYM 188 | E | 0.352 | 3.95E-02 | 23.9 |
| LYM184 | B | 0.159 | 8.46E-02 | 41.5 | LYM 193 | E | 0.351 | 5.12E-02 | 23.4 |
| CONTROL | - | 0.113 | - | 0 | LYM161 | E | 0.348 | 5.53E-02 | 22.4 |
| LYM184 | C | 0.46 | 1.23E-03 | 98.6 | LYM32 | E | 0.344 | 8.20E-02 | 21 |
| LYM240 | C | 0.363 | 6.06E-03 | 56.9 | LYM251 | E | 0.338 | 9.37E-02 | 18.9 |
| LYM 109 | C | 0.33 | 2.96E-02 | 42.4 | CONTROL | E | 0.284 | - | 0 |
| LYM245 | C | 0.322 | 5.20E-02 | 39.3 | LYM74 | F | 3.635 | 3.70E-05 | 18.8 |
| LYM248 | C | 0.323 | 5.28E-02 | 39.7 | LYM249 | F | 4.232 | 3.54E-03 | 38.3 |
| LYM 189 | C | 0.318 | 6.19E-02 | 37.2 | LYM240 | F | 3.548 | 3.55E-03 | 16 |
| LYM186 | C | 0.315 | 6.33E-02 | 36 | LYM234 | F | 4.189 | 3.00E-02 | 36.9 |
| CONTROL | - | 0.232 | - | 0 | LYM261 | F | 3.464 | 4.23E-02 | 13.2 |
| LYM 109 | D | 2.67 | 9.12E-04 | 41.4 | LYM 179 | F | 3.591 | 9.25E-02 | 17.4 |
| LYM 186 | D | 2.588 | 1.06E-02 | 37 | CONTROL | F | 3.06 | - | 0 |
| LYM240 | D | 2.99 | 1.63E-02 | 58.4 | LYM240 | G | 0.919 | 5.00E-06 | 57.4 |
| LYM189 | D | 2.592 | 1.94E-02 | 37.3 | LYM249 | G | 0.849 | 1.20E-05 | 45.4 |
| LYM245 | D | 2.594 | 4.12E-02 | 37.4 | LYM278 | G | 0.879 | 9.10E-05 | 50.5 |
| LYM115 | D | 2.352 | 4.59E-02 | 24.6 | LYM261 | G | 0.799 | 1.65E-04 | 36.8 |
| LYM248 | D | 2.622 | 7.42E-02 | 38.8 | LYM224 | G | 0.739 | 2.43E-04 | 26.5 |
| LYM180 | D | 2.304 | 7.43E-02 | 22 | LYM 189 | G | 0.853 | 1.11E-03 | 46 |
| CONTROL | - | 1.888 | - | 0 | LYM161 | G | 0.913 | 1.62E-03 | 56.3 |
| LYM240 | E | 0.391 | 1.32E-02 | 29.6 | LYM79 | G | 0.915 | 2.06E-03 | 56.7 |
| LYM248 | E | 0.386 | 2.03E-02 | 28.1 | LYM 188 | G | 0.747 | 1.25E-02 | 27.9 |
| LYM 186 | E | 0.384 | 2.65E-02 | 27.4 | LYM219 | G | 0.841 | 1.69E-02 | 44.1 |
| LYM 184 | E | 0.417 | 5.28E-02 | 38.2 | LYM74 | G | 0.762 | 1.99E-02 | 30.4 |
| LYM180 | E | 0.366 | 6.72E-02 | 21.5 | LYM 179 | G | 0.795 | 4.21E-02 | 36.2 |
| LYM 109 | E | 0.364 | 7.31E-02 | 20.8 | LYM234 | G | 0.815 | 4.73E-02 | 39.5 |
| CONTROL | - | 0.301 | - | 0 | CONTROL | G | 0.584 | - | 0 |
| LYM240 | F | 3.576 | 2.32E-03 | 26.9 | LYM161 | H | 0.097 | 2.10E-05 | 62.6 |
| LYM 109 | F | 3.246 | 1.05E-02 | 15.2 | LYM240 | H | 0.094 | 4.70E-05 | 57.3 |
| LYM248 | F | 3.449 | 1.22E-02 | 22.4 | LYM79 | H | 0.095 | 7.60E-05 | 59.9 |
| LYM 180 | F | 3.271 | 3.14E-02 | 16 | LYM189 | H | 0.091 | 1.67E-04 | 53.1 |
| LYM186 | F | 3.533 | 4.22E-02 | 25.3 | LYM278 | H | 0.088 | 4.71E-04 | 47.7 |
| LYM 189 | F | 3.237 | 6.97E-02 | 14.8 | LYM249 | H | 0.086 | 7.09E-04 | 45.4 |
| LYM115 | F | 3.16 | 7.89E-02 | 12.1 | LYM219 | H | 0.087 | 1.31E-03 | 46 |
| CONTROL | - | 2.819 | - | 0 | LYM234 | H | 0.086 | 2.25E-03 | 44.5 |
| LYM 184 | G | 0.774 | 3.40E-05 | 36 | LYM261 | H | 0.082 | 3.68E-03 | 38.3 |
| LYM228 | G | 0.749 | 1.29E-03 | 31.6 | LYM32 | H | 0.086 | 4.72E-03 | 44.3 |
| LYM 189 | G | 0.726 | 6.50E-03 | 27.5 | LYM 179 | H | 0.081 | 1.11HE-02 | 35.5 |
| LYM240 | G | 0.735 | 2.55E-02 | 29.1 | LYM74 | H | 0.081 | 1.50E-02 | 36.7 |
| LYM 186 | G | 0.653 | 4.33E-02 | 14.7 | LYM188 | H | 0.077 | 2.66E-02 | 29.3 |
| CONTROL | - | 0.569 | - | 0 | LYM224 | H | 0.077 | 4.09E-02 | 30.1 |
| | | | | | CONTROL | H | 0.059 | - | 0 |
| | | | | | LYM38 | B | 0.115 | - | 13.8 |
| | | | | | LYM 126 | B | 0.136 | - | 34.3 |
| | | | | | CONTROL | B | 0.101 | - | 0 |
| LYM36 | A | 0.004 | E-4.75 01 | 6.7 | LYM36 | I | 0.138 | E-014.84 | 7.8 |
| CONTROL | A | 0.004 | - | 0 | CONTROL | I | 0.128 | - | 0 |
| LYM36 | I | 0.09 | E-4.34 01 | 7 | LYM36 | J | 0.693 | E-0 17.20 | 3.3 |
| CONTROL | I | 0.084 | - | 0 | CONTROL | J | 0.671 | - | 0 |

Table 36. Results of the tissue culture experiments. Provided are the measured values of each tested parameter [parameters (ID.) A-F according to the parameters described in Table 35 above] in plants expressing the indicated polynucleotides. "Ev" = event; "P" = P-value; "Mean " = the average of measured parameter across events. % incr.vs.cont. = percentage of increase versus control (as compared to control).

**Table 37**

| ***Measured parameters at the tissueculture assay low nitrogen (T2 experiment) for transformed agriculture improving trait genes*** | |
|---|---|
| ***Tested Parameters*** | ***ID*** |
| Dry Weight (gr) | G |
| Fresh Weight (gr) | H |
| RGR Of Root Coverage | I |
| Roots Coverage TP3 (cm²) | J |
| RGR Of Roots Length | K |
| Roots Length TP3 (cm) | L |
| Leaf Area TP3 (cm) | M |
| RGR Of Leaf Area | N |

Table 37: Provided are the identification (ID) letters of each of the Tested Parameters. TP3 - Time Point 3; RGR- Relative Growth Rate plants were grown on low nitrogen as described above.

**Table 38**

| ***Results obtained in a T2 experiment at the tissue culture assay low nitrogen*** | | | | | | | | | | | |
|---|---|---|---|---|---|---|---|---|---|---|---|
| ***Gene name*** | ***Event*** | ***I D*** | ***Mea n*** | ***P value*** | ***% incr*** .***vs*. *cont*** | ***Gene name*** | ***Event*** | ***I D*** | ***Mea n*** | ***P value*** | ***% incr .vs. cont*** |
| LYM17 8 | 12161 .1 | G | 0.006 | 2.07E-03 | 39.1 | LYM5 | 12432 .1 | G | 0.007 | 1.40E-05 | 69.3 |
| LYM37 | 11801 .2 | G | 0.007 | 3.50E-03 | 60 | LYM268 | 12483 .2 | G | 0.007 | 1.56E-04 | 56.6 |
| LYM34 | 11901 .1 | G | 0.006 | 3.63E-03 | 31.9 | LYM1 | 11602 .6 | G | 0.009 | 2.16E-04 | 107. 8 |
| CONTR OL | - | G | 0.004 | - | 0 | LYM1 78 | 12164 .3 | G | 0.009 | 3.01E-03 | 110. 2 |
| LYM37 | 11801 .2 | I | 1.024 | 1.65E-04 | 56.9 | LYM132 | 12275 .3 | G | 0.006 | 3.52E-03 | 50.6 |
| LYM1 78 | 12161 .1 | I | 1.023 | 5.17E-04 | 56.8 | LYM129 | 12572 .2 | G | 0.006 | 5.24E-03 | 41.6 |
| CONTR OL | - | I | 0.652 | - | 0 | LYM1 | 11604 .4 | G | 0.006 | 5.89E-03 | 39.2 |
| LYM12 | 11873 .4 | L | 6.672 | 5.23E-03 | 23 | LYM129 | 12571 .3 | G | 0.006 | 7.22E-03 | 45.2 |
| LYM37 | 11801 .2 | L | 6.593 | 5.54E-03 | 21.5 | LYM268 | 12483 .4 | G | 0.008 | 7.89E-03 | 95.8 |
| CONTR OL | - | L | 5.425 | - | 0 | LYM1 78 | 12164 .2 | G | 0.006 | 8.14E-03 | 50.6 |
| LYM5 | 12436 .1 | G | 0.008 | 3.89E-04 | 83.5 | LYM1 32 | 12273 .2 | G | 0.005 | 8.21E-03 | 30.7 |
| LYM86 | 12183 .3 | G | 0.007 | 5.67E-04 | 63 | CONTR OL | - | G | 0.004 | - | 0 |
| LYM82 | 12203 .5 | G | 0.011 | 2.31E-03 | 153 | LYM1 | 11602 .6 | H | 0.177 | 1.00E-06 | 103. 6 |
| LYM82 | 12201 .2 | G | 0.008 | 2.64E-03 | 77.8 | LYM5 | 12432 .1 | H | 0.142 | 1.94E-04 | 64 |
| LYM86 | 12182 .3 | G | 0.007 | 2.83E-03 | 49.9 | LYM268 | 12483 .2 | H | 0.154 | 9.59E-04 | 76.8 |
| CONTR OL | - | G | 0.004 | - | 0 | LYM1 29 | 12572 .2 | H | 0.123 | 1.86E-03 | 41.4 |
| LYM268 | 12482 .1 | H | 0.115 | 8.00E-06 | 61.6 | LYM132 | 12275 .3 | H | 0.144 | 2.77E-03 | 66.3 |
| LYM82 | 12203 .5 | H | 0.189 | 2.40E-04 | 166 | LYM1 29 | 12573 .5 | H | 0.13 | 3.59E-03 | 50.2 |
| LYM44 | 11884 .1 | H | 0.12 | 6.61E-04 | 67.8 | LYM6 | 11735 .1 | H | 0.118 | 8.04E-03 | 36 |
| LYM6 | 11735 .2 | H | 0.102 | 6.94E-04 | 43.6 | LYM1 78 | 12164 .2 | H | 0.133 | 9.56E-03 | 53.6 |
| LYM82 | 12201 .2 | H | 0.144 | 1.47E-03 | 101. 5 | CONTR OL | - | H | 0.087 | - | 0 |
| LYM1 29 | 12573 .1 | H | 0.122 | 1.54E-03 | 71.9 | LYM268 | 12483 .4 | I | 1.459 | 0.00E+ 00 | 94.9 |
| LYM86 | 12183 .3 | H | 0.132 | 1.66E-03 | 85.3 | LYM5 | 12435 .1 | I | 1.327 | 5.00E-06 | 77.2 |
| LYM86 | 12182 .3 | H | 0.124 | 2.94E-03 | 73.7 | LYM178 | 12164 .3 | I | 1.39 | 2.60E-05 | 85.7 |
| LYM5 | 12436 .1 | H | 0.155 | 6.44E-03 | 118. 2 | LYM129 | 12571 .3 | I | 1.264 | 3.90E-05 | 68.7 |
| LYM129 | 12572 .2 | H | 0.121 | 7.61E-03 | 69.9 | LYM129 | 12573 .5 | I | 1.215 | 4.60E-05 | 62.3 |
| LYM82 | 12204 .6 | H | 0.091 | 9.35E-03 | 27.4 | LYM268 | 12483 .2 | I | 1.195 | 7.50E-05 | 59.5 |
| LYM5 | 12435.1 | H | 0.104 | 9.36E-03 | 45.6 | LYM268 | 12482 .3 | I | 1.16 | 3.42E-04 | 54.9 |
| LYM8 | 11984.1 | H | 0.15 | 9.54E-03 | 110. 2 | LYM134 | 12314 .2 | I | 1.199 | 4.26E-04 | 60.1 |
| CONTR OL | - | H | 0.071 | - | 0 | LYM1 | 11603 .2 | I | 1.094 | 1.02E-03 | 46.1 |
| LYM268 | 12483 .4 | I | 1.515 | 0.00E+ 00 | 124. 3 | LYM5 | 12432 .2 | I | 1.082 | 2.26E-03 | 44.5 |
| LYM82 | 12203 .2 | I | 1.459 | 0.00E+ 00 | 116 | LYM268 | 12482 .1 | I | 1.23 | 4.74E-03 | 64.3 |
| LYM82 | 12203 .5 | I | 1.419 | 8.00E-06 | 110. 1 | LYM1 | 11602 .6 | I | 1.073 | 6.12E-03 | 43.2 |
| LYM8 | 11984 .1 | I | 1.336 | 1.10E-05 | 97.9 | LYM132 | 12276 .1 | I | 1.134 | 7.20E-03 | 51.5 |
| LYM44 | 11882 .1 | I | 1.135 | 2.40E-05 | 68.1 | CONTR OL | - | I | 0.749 | - | 0 |
| LYM5 | 12436 .1 | I | 1.264 | 3.40E-05 | 87.2 | LYM268 | 12483 .4 | J | 12.19 | 3.00E-05 | 90.2 |
| LYM86 | 12183 .3 | I | 1.056 | 1.39E-04 | 56.3 | LYM5 | 12435.1 .1 | J | 10.94 5 | 1.14E-03 | 70.8 |
| LYM86 | 12182 .3 | I | 1.219 | 1.86E-04 | 80.5 | LYM129 | 12571 .3 | J | 10.37 5 | 1.25E-03 | 61.9 |
| LYM5 | 12436 .2 | I | 1.072 | 2.95E-04 | 58.7 | LYM129 | 12573 .5 | J | 10.09 3 | 2.32E-03 | 57.5 |
| LYM82 | 12201 .2 | I | 0.986 | 3.76E-04 | 46 | LYM268 | 12483 .2 | J | 9.908 | 2.57E-03 | 54.6 |
| LYM44 | 11884 .3 | I | 1.094 | 5.32E-04 | 62 | LYM268 | 12482 .3 | J | 9.59 | 4.95E-03 | 49.6 |
| LYM44 | 11885 .4 | I | 1.249 | 1.21E-03 | 85 | CONTR OL | - | J | 6.409 | - | 0 |
| LYM268 | 12484 .2 | I | 1.147 | 1.40E-03 | 69.9 | LYM129 | 12571 .3 | K | 0.76 | 6.00E-05 | 41.4 |
| LYM8 | 11982 .7 | I | 0.958 | 1.62E-03 | 41.9 | LYM5 | 12435 .1 | K | 0.754 | 6.90E-05 | 40.3 |
| LYM8 | 11983 .1 | I | 1.007 | 1.85E-03 | 49.1 | CONTR OL | - | K | 0.538 | - | 0 |
| LYM129 | 12573 .1 | I | 0.935 | 2.77E-03 | 38.4 | LYM129 | 12571 .3 | L | 7.821 | 3.52E-04 | 23.6 |
| LYM129 | 12573 .3 | I | 0.901 | 2.85E-03 | 33.4 | LYM5 | 12435 .1 | L | 7.76 | 5.05E-04 | 22.6 |
| LYM268 | 12482 .1 | I | 0.992 | 3.37E-03 | 46.9 | LYM268 | 12483 .4 | L | 7.69 | 6.12E-04 | 21.5 |
| LYM6 | 11734 .3 | I | 1.105 | 5.28E-03 | 63.6 | CONTR OL | - | L | 6.328 | - | 0 |
| LYM5 | 12435 .1 | I | 1.047 | 6.27E-03 | 55.1 | LYM5 | 12432 .1 | M | 0.837 | 3.00E-06 | 67.1 |
| LYM 129 | 12572 .2 | I | 0.97 | 7.93E-03 | 43.6 | LYM 129 | 12571 .3 | M | 0.732 | 2.50E-05 | 46.1 |
| CONTR OL | - | I | 0.675 | - | 0 | LYM 132 | 12273 .2 | M | 0.685 | 8.40E-05 | 36.7 |
| LYM82 | 12203 .2 | J | 12.32 8 | 1.84E-03 | 118. 3 | LYM1 | 11604 .4 | M | 0.692 | 1.78E-04 | 38.1 |
| LYM129 | 12573 .3 | J | 7.459 | 3.21E-03 | 32.1 | LYM 129 | 12573 .5 | M | 0.797 | 3.26E-04 | 59.1 |
| LYM268 | 12483 .4 | J | 12.72 6 | 6.83E-03 | 125. 4 | LYM 178 | 12163 .3 | M | 0.668 | 6.70E-04 | 33.4 |
| LYM82 | 12201 .2 | J | 8.641 | 8.55E-03 | 53 | LYMI | 11602 .6 | M | 0.95 | 7.84E-04 | 89.7 |
| CONTR OL | - | J | 5.647 | - | 0 | LYM5 | 12435 .1 | M | 0.756 | 8.12E-04 | 51.1 |
| LYM82 | 12203 .2 | K | 0.636 | 1.22E-03 | 30 | LYM132 | 12275 .3 | M | 0.727 | 1.36E-03 | 45.3 |
| LYM5 | 12435.1 .1 | K | 0.624 | 1.73E-03 | 27.8 | LYMI | 11602 .1 | M | 0.676 | 1.74E-03 | 35.1 |
| LYM86 | 12182 .3 | K | 0.622 | 2.13E-03 | 27.3 | LYM268 | 12483 .4 | M | 0.889 | 2.41E-03 | 77.5 |
| LYM268 | 12483 .4 | K | 0.628 | 3.66E-03 | 28.5 | LYM 178 | 12164 .3 | M | 0.93 | 2.60E-03 | 85.7 |
| LYM44 | 11884 .3 | K | 0.614 | 5.55E-03 | 25.7 | LYM 178 | 12164 .2 | M | 0.732 | 4.37E-03 | 46.3 |
| CONTR OL | - | K | 0.489 | - | 0 | LYM 129 | 12572 .2 | M | 0.7 | 5.37E-03 | 39.9 |
| LYM82 | 12203 .2 | L | 7.098 | 1.50E-05 | 35.2 | LYM 134 | 12314 .2 | M | 0.746 | 5.84E-03 | 48.9 |
| LYM86 | 12182 .3 | L | 6.996 | 2.30E-05 | 33.3 | LYMI | 11603 .2 | M | 0.78 | 9.55E-03 | 55.8 |
| LYM268 | 12483 .4 | L | 7.232 | 2.69E-04 | 37.8 | CONTR OL | - | M | 0.501 | - | 0 |
| LYM5 | 12435 .1 | L | 6.758 | 3.03E-04 | 28.7 | LYM1 | 11602 .6 | N | 0.098 | 0.00E+ 00 | 96 |
| LYM82 | 12203 .5 | L | 7.346 | 4.61E-04 | 39.9 | LYM178 | 12164 .3 | N | 0.094 | 0.00E+ 00 | 87 |
| LYM86 | 12183 .3 | L | 6.532 | 8.44E-04 | 24.4 | LYM268 | 12483 .4 | N | 0.092 | 0.00E+ 00 | 83.5 |
| LYM5 | 12432 .1 | L | 6.265 | 1.21E-03 | 19.3 | LYM5 | 12432 .1 | N | 0.083 | 1.00E-06 | 65.1 |
| LYM5 | 12436 .1 | L | 6.713 | 1.40E-03 | 27.9 | LYM5 | 12435 .1 | N | 0.083 | 6.00E-06 | 65.5 |
| LYM44 | 11884.3 | L | 6.768 | 1.41E-03 | 28.9 | LYM129 | 12573.5 | N | 0.08 | 7.00E-06 | 60.5 |
| LYM268 | 12484 .2 | L | 6.748 | 3.26E-03 | 28.5 | LYM1 | 11603 .2 | N | 0.082 | 1.10E-05 | 64.2 |
| LYM44 | 11885 .4 | L | 6.724 | 3.68E-03 | 28.1 | LYM129 | 12571 .3 | N | 0.076 | 2.30E-05 | 52.5 |
| LYM8 | 11983 .1 | L | 6.614 | 5.58E-03 | 26 | LYM1 78 | 12164 .2 | N | 0.078 | 3.10E-05 | 55.3 |
| LYM5 | 12436 .2 | L | 6.52 | 6.08E-03 | 24.2 | LYM132 | 12275 .3 | N | 0.073 | 7.30E-05 | 45.8 |
| LYM86 | 12181 .3 | L | 6.239 | 6.93E-03 | 18.8 | LYM132 | 12273 .2 | N | 0.071 | 8.20E-05 | 41.5 |
| LYM8 | 11984 .1 | L | 6.951 | 8.05E. 03 | 32.4 | LYM132 | 12276 .1 | N | 0.076 | 1.27E-04 | 52.1 |
| CONTR OL | - | L | 5.25 | - | 0 | LYM1 | 11604 .4 | N | 0.071 | 1.52E-04 | 41 |
| LYM82 | 12203 .2 | M | 0.836 | 8.30E-05 | 110. 7 | LYM5 | 12432 .2 | N | 0.077 | 1.63E-04 | 53.8 |
| LYM129 | 12572 .2 | M | 0.675 | 4.28E-04 | 69.9 | LYM268 | 12482 .1 | N | 0.082 | 1.98E-04 | 63 |
| LYM268 | 12482 .1 | M | 0.63 | 6.12E-04 | 58.6 | LYM129 | 12572 .2 | N | 0.072 | 2.38E-04 | 44.1 |
| LYM82 | 12203 .5 | M | 0.836 | 9.33E-04 | 110. 6 | LYM134 | 12313 .2 | N | 0.084 | 2.41E-04 | 67.5 |
| LYM86 | 12183 .3 | M | 0.684 | 1.14E-03 | 72.2 | LYM134 | 12314 .2 | N | 0.073 | 6.27E-04 | 45.5 |
| LYM82 | 12204 .6 | M | 0.491 | 1.31E-03 | 23.6 | LYM268 | 12483 .2 | N | 0.077 | 1.02E-03 | 52.9 |
| LYM82 | 12201 .2 | M | 0.787 | 1.98E-03 | 98.1 | LYMI | 11602 .1 | N | 0.068 | 1.34E-03 | 36.8 |
| LYM268 | 12483 .4 | M | 0.825 | 2.72E-03 | 107. 9 | LYM132 | 12271 .4 | N | 0.075 | 2.22E-03 | 49.5 |
| LYM5 | 12436 .1 | M | 0.776 | 3.22E-03 | 95.3 | LYM 178 | 12163 .3 | N | 0.064 | 7.09E-03 | 26.9 |
| LYM268 | 12484 .2 | M | 0.743 | 4.16E-03 | 87.2 | LYM1 | 11601 .1 | N | 0.067 | 8.58E-03 | 34.4 |
| LYM8 | 11984.1 .1 | M | 0.716 | 4.66E-03 | 80.3 | CONTR OL | - | N | 0.05 | - | 0 |
| LYM8 | 11982 .7 | M | 0.565 | 8.97E-03 | 42.3 | | | | | | |
| LYM5 | 12435 .1 | M | 0.674 | 9.05E-03 | 69.7 | | | | | | |
| LYM86 | 12182 .3 | M | 0.719 | 9.47E-03 | 81.2 | | | | | | |
| LYM268 | 12481 .1 | M | 0.627 | 9.53E-03 | 58 | | | | | | |
| CONTR OL | - | M | 0.397 | - | 0 | | | | | | |
| LYM268 | 12483 .4 | N | 0.085 | 0.00E+ 00 | 112. 8 | | | | | | |
| LYM82 | 12203 .2 | N | 0.088 | 0.00E+ 00 | 119. 9 | | | | | | |
| LYM82 | 12203.5 | N | 0.087 | 0.00E+ 00 | 116. 8 | | | | | | |
| LYM5 | 12436 .1 | N | 0.078 | 1.00E-06 | 95.5 | | | | | | |
| LYM82 | 12201 .2 | N | 0.074 | 2.00E-06 | 85 | | | | | | |
| LYM8 | 11984 .1 | N | 0.075 | 3.00E-06 | 87 | | | | | | |
| LYM86 | 12183 .3 | N | 0.072 | 3.00E-06 | 80.7 | | | | | | |
| LYM129 | 12573 .1 | N | 0.073 | 1.00E-05 | 82.5 | | | | | | |
| LYM268 | 12484 .2 | N | 0.073 | 1.20E-05 | 82.9 | | | | | | |
| LYM86 | 12182 .3 | N | 0.074 | 2.00E-05 | 85.7 | | | | | | |
| LYM5 | 12435 .1 | N | 0.072 | 2.60E-05 | 80.5 | | | | | | |
| LYM268 | 12482 .1 | N | 0.066 | 6.90E-05 | 63.7 | | | | | | |
| LYM 129 | 12572 .2 | N | 0.068 | 8.00E-05 | 71.2 | | | | | | |
| LYM268 | 12481 .3 | N | 0.066 | 8.40E-05 | 64 | | | | | | |
| LYM44 | 11884 .3 | N | 0.067 | 9.10E-05 | 68 | | | | | | |
| LYM268 | 12481 .1 | N | 0.062 | 4.98E-04 | 54.5 | | | | | | |
| LYM44 | 11885 .4 | N | 0.068 | 5.74E-04 | 70.9 | | | | | | |
| LYM5 | 12436 .2 | N | 0.062 | 9.56E-04 | 55.4 | | | | | | |
| LYM8 | 11982 .7 | N | 0.059 | 1.14E-03 | 47 | | | | | | |
| LYM44 | 11884 .! | N | 0.057 | 2.22E-03 | 42 | | | | | | |
| LYM8 | 11982 .4 | N | 0.057 | 2.75E-03 | 42.3 | | | | | | |
| LYM82 | 12201 .1 | N | 0.056 | 5.28E-03 | 39 | | | | | | |
| LYM86 | 12183 .1 | N | 0.056 | 7.02E-03 | 40.5 | | | | | | |
| LYM44 | 11885 .3 | N | 0.059 | 7.80E-03 | 46.7 | | | | | | |
| LYM44 | 11882 .1 | N | 0.056 | 8.64E-03 | 40.3 | | | | | | |
| CONTR OL | - | N | 0.04 | - | 0 | | | | | | |
| LYM136 | 13423 .2 | H | 0.006 | 2.00E-06 | 107. 3 | LYM136 | 13423 .2 | N | 0.085 | 0.00E+ 00 | 82.6 |
| LYM136 | 13421 .8 | G | 0.005 | 8.66E-04 | 75.6 | LYM 136 | 13421 .8 | N | 0.082 | 0.00E+ 00 | 75.9 |
| LYM84 | 13404 .4 | G | 0.007 | 2.84E-03 | 113. 8 | LYM84 | 13404 .4 | N | 0.092 | 0.00E+ 00 | 96.7 |
| LYM 136 | 13421 .6 | G | 0.006 | 2.89E-03 | 94.3 | LYM84 | 13401 .2 | N | 0.088 | 0.00E+ 00 | 88 |
| LYM1 | 11601 .1 | G | 0.005 | 5.24E-03 | 61 | LYM1 | 11601 .1 | N | 0.081 | 1.00E-06 | 73.2 |
| LYM84 | 13403 .3 | G | 0.006 | 5.30E-03 | 87.8 | LYM84 | 13403 .3 | N | 0.08 | 2.00E-06 | 70.6 |
| LYM84 | 13401 .2 | G | 0.007 | 5.52E-03 | 130. 9 | LYM84 | 13403 .2 | N | 0.073 | 2.00E-06 | 55.9 |
| LYMI | 11603 .2 | G | 0.004 | 9.31E-03 | 42.3 | LYM 136 | 13423 .1 | N | 0.085 | 3.00E-06 | 81.3 |
| LYM84 | 13403 .2 | G | 0.005 | 1.53E-02 | 58.5 | LYM1 | 11602 .6 | N | 0.07 | 1.40E-05 | 49.8 |
| LYM1 | 11602 .6 | G | 0.005 | 2.06E-02 | 50.4 | LYM136 | 13421 .6 | N | 0.075 | 2.50E-05 | 60.5 |
| LYM136 | 13423 .1 | G | 0.007 | 3.33E-02 | 123. 6 | LYM84 | 13403 .1 | N | 0.07 | 7.20E-05 | 50.5 |
| LYM 136 | 13421 .5 | G | 0.005 | 5.81E-02 | 72.4 | LYM 136 | 13421 .5 | N | 0.074 | 1.10E-04 | 57.9 |
| CONTR OL | - | G | 0.003 | - | 0 | LYM1 | 11603 .2 | N | 0.071 | 1.87E-04 | 51.2 |
| LYMI | 11601 .1 | H | 0.103 | 1.93E-04 | 78.3 | LYMI | 11604 .4 | N | 0.058 | 2.56E-02 | 23.7 |
| LYM84 | 13403 .3 | H | 0.11 | 1.55E-03 | 91.3 | LYMI | 11602 .1 | N | 0.056 | 7.26E-02 | 19.1 |
| LYM84 | 13403 .2 | H | 0.113 | 1.81E-03 | 95.7 | CONTR OL | - | N | 0.047 | - | 0 |
| LYM136 | 13423 .2 | H | 0.13 | 4.05E-03 | 126. 1 | LYM136 | 13421 .8 | I | 1.163 | 9.90E-05 | 51.6 |
| LYM1 | 11603 .2 | H | 0.093 | 4.55E-03 | 60.9 | LYM136 | 13423 .1 | I | 1.209 | 8.12E-04 | 57.6 |
| LYM84 | 13404 .4 | H | 0.13 | 6.79E-03 | 126. 1 | LYM1 | 11603 .2 | I | 0.993 | 1.65E-03 | 29.4 |
| LYM1 | 11602 .6 | H | 0.103 | 7.66E-03 | 78.3 | LYM84 | 13404 .4 | I | 1.095 | 2.56E-03 | 42.7 |
| LYM136 | 13423 .1 | H | 0.148 | 9.13E-03 | 156. 5 | LYM84 | 13403 .2 | I | 1.009 | 3.97E-03 | 31.5 |
| LYM136 | 13421 .8 | H | 0.128 | 1.35E-02 | 121. 7 | LYM84 | 13403 .1 | I | 0.997 | 4.57E-03 | 30 |
| LYM84 | 13401 .2 | H | 0.133 | 1.58E-02 | 130. 4 | LYM 136 | 13423 .2 | I | 1.104 | 4.86E-03 | 43.9 |
| LYM 136 | 13421 .5 | H | 0.105 | 2.3 1E-02 | 82.6 | LYM84 | 13403 .3 | I | 1.035 | 8.51E-03 | 34.9 |
| LYM136 | 13421 .6 | H | 0.11 | 2.99E-02 | 91.3 | LYM84 | 13401 .2 | I | 1.049 | 2.01E-02 | 36.8 |
| LYM84 | 13403 .1 | H | 0.095 | 9.17E-02 | 65.2 | LYM136 | 13421 .6 | I | 0.996 | 2.96E-02 | 29.8 |
| CONTR OL | - | H | 0.058 | - | 0 | LYM1 | 11601 .1 | I | 0.899 | 6.99E-02 | 17.1 |
| LYM136 | 13421 .8 | M | 0.805 | 0.00E+ 00 | 84 | LYM1 | 11604 .4 | I | 0.884 | 9.29E-02 | 15.3 |
| LYM84 | 13403.2 | M | 0.683 | 0.00E+00 | 56 | CONTROL | - | I | 0.767 | - | 0 |
| LYM1 | 11602.6 | M | 0.713 | 1.50E-04 | 62.9 | LYM1 | 11603 .2 | J | 8.538 | 1.59E-03 | 30 |
| LYM84 | 13404 .4 | M | 0.893 | 6.76E-04 | 104 | LYM84 | 13403 .1 | J | 8.743 | 1.76E-02 | 33.1 |
| LYM1 | 11601 .1 | M | 0.84 | 7.01E-04 | 92 | LYM136 | 13421 .8 | J | 10.11 5 | 2.04E-02 | 54 |
| LYM 136 | 13423 .2 | M | 0.835 | 7.48E-04 | 90.9 | LYM84 | 13403 .2 | J | 8.468 | 3.82E-02 | 28.9 |
| LYM84 | 13403 .1 | M | 0.675 | 1.60E-03 | 54.3 | LYM84 | 13404 .4 | J | 9.658 | 5.32E-02 | 47 |
| LYM84 | 13403 .3 | M | 0.79 | 2.37E-03 | 80.6 | LYM1 | 11604 .4 | J | 7.745 | 5.47E-02 | 17.9 |
| LYM84 | 13401 .2 | M | 0.86 | 2.92E-03 | 96.6 | LYM136 | 13423 .1 | J | 10.39 8 | 6.40E-02 | 58.3 |
| LYM 136 | 13423 .1 | M | 0.84 | 4.59E-03 | 92 | LYM1 | 11601 .1 | J | 7.858 | 6.86E-02 | 19.6 |
| LYM1 | 11603 .2 | M | 0.693 | 8.94E-03 | 58.3 | CONTR OL | - | J | 6.568 | - | 0 |
| LYM136 | 13421 .6 | M | 0.738 | 9.38E-03 | 68.6 | LYM136 | 13421 .8 | L | 7.173 | 1.89E-03 | 21.6 |
| LYM1 | 11604 .4 | M | 0.54 | 1.15E-02 | 23.4 | LYM84 | 13404 .4 | L | 7.105 | 3.22E-03 | 20.4 |
| LYM 136 | 153421 .5 | M | 0.685 | 1.62E-02 | 56.6 | LYM84 | 13403 .1 | L | 6.823 | 8.12E-03 | 15.7 |
| LYM1 | 11602 .1 | M | 0.538 | 5.30E-02 | 22.9 | LYM 1 | 11603 .2 | L | 6.868 | 9.56E-03 | 16.4 |
| CONTR OL | - | M | 0.438 | - | 0 | LYM 1 | 11601 .1 | L | 6.538 | 2.95E-02 | 10.8 |
| | | | | | | LYM136 | 13423 | L | 6.88 | 3.42E-02 | 16.6 |
| | | | | | | LYM84 | .2 | L | 6.445 | 7.62E-02 | 9.3 |
| | | | | | | CONTR OL | - | L | 5.899 | - | 0 |

Table 38. Results of the tissue culture T2 experiments. Provided are the measured values of each tested parameter [parameters (ID.) G-L according to the parameters described in Table 37 above] in plants expressing the indicated polynucleotides. "Ev" = event; "P" = P-value; "Mean " = the average of measured parameter across events. % incr. vs. cont. = percentage of increase versus control (as compared to control).

These results demonstrate that the polynucleotides are capable of improving yield and additional valuable important agricultural traits such as increase of biomass, abiotic stress tolerance, nitrogen use efficiency, yield, vigor, fiber yield and/or quality. Thus, transformed plants showing improved fresh and dry weight demonstrate the gene capacity to improve biomass a key trait of crops for forage and plant productivity; transformed plants showing improvement of seed yield demonstrate the genes capacity to improve plant productivity; transformed plants showing improvement of plot coverage and rosette diameter demonstrate the genes capacity to improve plant drought resistance as they reduce the loss of soil water by simple evaporation and reduce the competition with weeds; hence reduce the need to use herbicides to control weeds. Transformed plants showing improvement of relative growth rate of various organs (leaf and root) demonstrate the gene capacity to promote plant growth and hence shortening the needed growth period and/or alternatively improving the utilization of available nutrients and water leading to increase of land productivity; Transformed plants showing improvement of organ number as demonstrated by the leaf number parameter exhibit a potential to improve biomass yield important for forage crops and improve the plant productivity; Transformed plants showing increased root length and coverage demonstrate the gene capacity to improve drought resistance and better utilization of fertilizers as the roots can reach larger soil volume; Transformed plants showing improvement of leaf petiole relative area and leaf blade area demonstrate the genes capacity to cope with limited light intensities results from increasing the plant population densities and hence improve land productivity.

## Claims

1. A method of increasing biomass, growth rate, oil content, and/or nitrogen use efficiency of a plant, comprising expressing within the plant an exogenous polynucleotide comprising a nucleic acid sequence encoding a polypeptide at least 80 % identical to SEQ ID NO: 333, thereby increasing the biomass, growth rate, oil content, and/or nitrogen use efficiency of the plant.

2. An isolated polypeptide comprising an amino acid sequence at least 80% homologous to SEQ ID NO: 333, wherein said amino acid sequence is capable of increasing biomass, growth rate, oil content, and/or nitrogen use efficiency of a plant.

3. A plant cell exogenously expressing a nucleic acid construct comprising an isolated polynucleotide comprising a nucleic acid sequence encoding a polypeptide which comprises an amino acid sequence at least 80 % homologous to the amino acid sequence set forth in SEQ ID NO: 333, and a promoter for directing transcription of said nucleic acid sequence in a host cell, wherein said nucleic acid sequence is capable of increasing biomass, growth rate, oil content, and/or nitrogen use efficiency of a plant.

4. A plant cell exogenously expressing the polypeptide of claim 2.

5. The method of claim 1, the isolated polypeptide of claim 2 or the plant cell of claim 3 or 4, wherein said polypeptide comprises an amino acid sequence at least 85 % homologous to the amino acid sequence set forth by SEQ ID NO: 333.

6. The method of claim 1, the isolated polypeptide of claim 2, or the plant cell of claim 3 or 4, wherein said polypeptide comprises an amino acid sequence at least 90 % homologous to the amino acid sequence set forth by SEQ ID NO: 333.

7. The method of claim 1, the isolated polypeptide of claim 2, or the plant cell of claim 3 or 4, wherein said polypeptide comprises an amino acid sequence at least 95 % homologous to the amino acid sequence set forth by SEQ ID NO: 333.

8. The method of claim 1, the isolated polypeptide of claim 2, or the plant cell of claim 3 or 4, wherein said polypeptide is selected from the group consisting of SEQ ID NOs: 333 and 2899.

9. The method of claim 1, or the plant cell of claim 3, wherein said exogenous polynucleotide comprises a nucleic acid sequence at least 80 % identical to SEQ ID NO: 3574 or 96.

10. The method of claim 1, or the plant cell of claim 3, wherein said exogenous polynucleotide comprises the nucleic acid sequence selected from the group consisting of SEQ ID NOs: 3574, 96, and 1392.

11. The plant cell of any one of claims 3-10, wherein said plant cell forms a part of a plant.

12. A method comprising:
(a) transforming one or more cells of a plant with a nucleic acid construct comprising an isolated polynucleotide comprising a nucleic acid sequence encoding a polypeptide which comprises an amino acid sequence at least 80 % homologous to the amino acid sequence set forth in SEQ ID NO: 333, and a promoter for directing transcription of said nucleic acid sequence in a host cell, wherein said nucleic acid sequence is capable of increasing biomass, growth rate, oil content, and/or nitrogen use efficiency of a plant, and
(b) generating a mature plant from said one or more cells of said plant resultant of step (a).

13. The method of claim 12, wherein said amino acid sequence is set forth in SEQ ID NO: 333.

14. A plant obtainable by a method comprising:
(a) transforming one or more cells of a plant with a nucleic acid construct comprising an isolated polynucleotide comprising a nucleic acid sequence encoding a polypeptide which comprises an amino acid sequence at least 80 % homologous to the amino acid sequence set forth in SEQ ID NO: 333, and a promoter for directing transcription of said nucleic acid sequence in a host cell, wherein said nucleic acid sequence is capable of increasing biomass, growth rate, oil content, and/or nitrogen use efficiency of a plant,
(b) generating a mature plant from said one or more cells of said plant resultant of step (a),
(c) cross-breeding regenerated transformed plants expressing said polypeptide and
(d) selecting resultant progeny for superior biomass, oil content and/or nitrogen use efficiency, wherein the plant comprises the nucleic acid acid construct.

15. The plant of claim 14, wherein said amino acid sequence is set forth in SEQ ID NO: 333.

## Patentansprüche

1. Verfahren zum Erhöhen von Biomasse, Wachstumsrate, Ölgehalt und/oder der Wirksamkeit der Stickstoffnutzung einer Pflanze, umfassend ein Exprimieren eines exogenen Polynukleotids in der Pflanze, das eine Nukleinsäuresequenz umfasst, die ein Polypeptid kodiert, das wenigstens zu 80% mit SEQ ID NR: 333 identisch ist, dadurch Erhöhen der Biomasse, Wachstumsrate, Ölgehalt und/oder der Wirksamkeit der Stickstoffnutzung der Pflanze.

2. Isoliertes Polypeptid umfassend eine Aminosäuresequenz, die wenigstens 80% zu SEQ ID NR: 333 homolog ist, wobei die Aminosäuresequenz fähig ist, Biomasse, Wachstumsrate, Ölgehalt und/oder die Wirksamkeit der Stickstoffnutzung einer Pflanze zu erhöhen.

3. Pflanzenzelle, die ein Nukleinsäurekonstrukt exogen exprimiert, das ein isoliertes Polynukleotid umfasst, das eine Nukleinsäuresequenz umfasst, die ein Polypeptid kodiert, das eine Aminosäuresequenz umfasst, die zu wenigstens 80% homolog zu der in SEQ ID NR: 333 aufgeführten Aminosäuresequenz ist, sowie einen Promotor zum Regeln der Transkription der Nukleinsäuresequenz in einer Wirtszelle, wobei die Nukleinsäuresequenz fähig ist, Biomasse, Wachstumsrate, Ölgehalt und/oder die Wirksamkeit der Stickstoffnutzung einer Pflanze zu erhöhen.

4. Pflanzenzelle, die das Polypeptid nach Anspruch 2 exogen exprimiert.

5. Verfahren nach Anspruch 1, isoliertes Polypeptid nach Anspruch 2 oder Pflanzenzelle nach Anspruch 3 oder 4, wobei das Polypeptid eine Aminosäuresequenz umfasst, die wenigstens 85% homolog zu der in SEQ ID NR: 333 aufgeführten Aminosäuresequenz ist.

6. Verfahren nach Anspruch 1, isoliertes Polypeptid nach Anspruch 2 oder Pflanzenzelle nach Anspruch 3 oder 4, wobei das Polypeptid eine Aminosäuresequenz umfasst, die wenigstens 90% homolog zu der in SEQ ID NR: 333 aufgeführten Aminosäuresequenz ist.

7. Verfahren nach Anspruch 1, isoliertes Polypeptid nach Anspruch 2 oder Pflanzenzelle nach Anspruch 3 oder 4, wobei das Polypeptid eine Aminosäuresequenz umfasst, die wenigstens 95% homolog zu der in SEQ ID NR: 333 aufgeführten Aminosäuresequenz ist.

8. Verfahren nach Anspruch 1, isoliertes Polypeptid nach Anspruch 2 oder Pflanzenzelle nach Anspruch 3 oder 4, wobei das Polypeptid ausgewählt ist aus der Gruppe bestehend aus den SEQ ID NR: 333 und 2899.

9. Verfahren nach Anspruch 1 oder Pflanzenzelle nach Anspruch 3, wobei das exogene Polynukleotid eine Nukleinsäuresequenz umfasst, die wenigstens zu 80% identisch mit SEQ ID NR: 3574 oder 96 ist.

10. Verfahren nach Anspruch 1 oder Pflanzenzelle nach Anspruch 3, wobei das exogene Polynukleotid eine Nukleinsäuresequenz umfasst, ausgewählt aus der Gruppe bestehend aus den SEQ ID NR: 3574, 96 und 1392.

11. Pflanzenzelle nach einem der Ansprüche 3-10, wobei die Pflanzenzelle einen Teil einer Pflanze bildet.

12. Verfahren umfassend:
(a) Transformieren einer oder mehrerer Zellen einer Pflanze mit einem Nukleinsäurekonstrukt umfassend ein isoliertes Polynukleotid, das eine Nukleinsäuresequenz umfasst, die ein Polypeptid kodiert, das eine Aminosäuresequenz umfasst, die zu wenigstens zu 80% homolog zu der in SEQ ID NR: 333 aufgeführten Aminosäuresequenz ist, sowie einen Promotor zum Regeln der Transkription der Nukleinsäuresequenz in einer Wirtszelle, wobei die Nukleinsäuresequenz fähig ist, Biomasse, Wachstumsrate, Ölgehalt und/oder die Wirksamkeit der Stickstoffnutzung einer Pflanze zu erhöhen, und
(b) Erzeugen einer reifen Pflanze aus der einen oder den mehreren aus Schritt (a) erhaltenen Zellen der Pflanze.

13. Verfahren nach Anspruch 12, wobei die Aminosäuresequenz so wie in SEQ ID NR: 333 aufgezeigt ist.

14. Pflanze erhältlich durch ein Verfahren umfassend:
(a) Transformieren einer oder mehrerer Zellen einer Pflanze mit einem Nukleinsäurekonstrukt umfassend ein isoliertes Polynukleotid, das eine Nukleinsäuresequenz umfasst, die ein Polypeptid kodiert, das eine Aminosäuresequenz umfasst, die zu wenigstens 80% homolog zu der in SEQ ID NR: 333 aufgeführten Aminosäuresequenz ist, sowie einen Promotor zum Regeln der Transkription der Nukleinsäuresequenz in einer Wirtszelle, wobei die Nukleinsäuresequenz fähig ist, Biomasse, Wachstumsrate, Ölgehalt und/oder die Wirksamkeit der Stickstoffnutzung einer Pflanze zu erhöhen,
(b) Erzeugen einer reifen Pflanze aus der einen oder den mehreren aus Schritt (a) erhaltenen Zellen der Pflanze,
(c) Kreuzungszüchten von regenerierten transformierten Pflanzen, die das Polypeptid exprimieren, und
(d) Auswählen der erzeugten Nachkommen unter Berücksichtigung von höherer Wirksamkeit der Biomasse, Wachstumsrate, Ölgehalt und/oder der Stickstoffnutzung der Pflanze, wobei die Pflanze das Nukleinsäurekonstrukt umfasst.

15. Pflanze nach Anspruch 14, wobei die Aminosäuresequenz so wie in SEQ ID NR: 333 aufgezeigt ist.

## Revendications

1. Procédé pour augmenter la biomasse, le taux de croissance, la teneur en huile, et/ou l'efficacité d'utilisation de l'azote par une plante, qui comprend l'expression dans la plante d'un polynucléotide exogène comprenant une séquence d'acide nucléique codant un polypeptide identique à au moins 80% à la séquence SEQ ID NO : 333, augmentant ainsi la biomasse, le taux de croissance, la teneur en huile, et/ou l'efficacité d'utilisation de l'azote de la plante.

2. Polypeptide isolé comprenant une séquence d'acides aminés homologue à au moins 80% à la séquence SEQ ID NO : 333, dans lequel ladite séquence d'acides aminés est capable d'augmenter la biomasse, le taux de croissance, la teneur en huile, et/ou l'efficacité d'utilisation de l'azote de la plante.

3. Cellule végétale exprimant de manière exogène une construction d'acide nucléique comprenant un polypeptide isolé comprenant une séquence d'acides nucléiques codant un polypeptide qui comprend une séquence d'acides aminés homologue à au moins 80% à la séquence d'acides aminés indiquée par SEQ ID NO : 333, et un promoteur pour réaliser la transcription de ladite séquence d'acides nucléiques dans une cellule hôte, dans laquelle ladite séquence d'acides nucléiques est capable d'augmenter la biomasse, le taux de croissance, la teneur en huile, et/ou l'efficacité d'utilisation de l'azote de la plante.

4. Cellule végétale exprimant de manière exogène le polypeptide selon la revendication 2.

5. Procédé selon la revendication 1, polypeptide isolé selon la revendication 2 ou cellule végétale selon la revendication 3 ou 4, dans lequel ledit polypeptide comprend une séquence d'acides aminés homologue à au moins 85% à la séquence d'acides aminés indiquée par SEQ ID NO : 333.

6. Procédé selon la revendication 1, polypeptide isolé selon la revendication 2 ou cellule végétale selon la revendication 3 ou 4, dans lequel ledit polypeptide comprend une séquence d'acides aminés homologue à au moins 90% à la séquence d'acides aminés indiquée par SEQ ID NO : 333.

7. Procédé selon la revendication 1, polypeptide isolé selon la revendication 2 ou cellule végétale selon la revendication 3 ou 4, dans lequel ledit polypeptide comprend une séquence d'acides aminés homologue à au moins 95% à la séquence d'acides aminés indiquée par SEQ ID NO : 333.

8. Procédé selon la revendication 1, polypeptide isolé selon la revendication 2 ou cellule végétale selon la revendication 3 ou 4, dans lequel ledit polypeptide est choisi dans le groupe constitué par les séquences SEQ ID NO : 333 et 2899.

9. Procédé selon la revendication 1, ou cellule végétale selon la revendication 3, dans lequel ledit polypeptide exogène comprend une séquence d'acides nucléiques identique à au moins 80% de la séquence SEQ ID NO : 3574 ou 96.

10. Procédé selon la revendication 1, ou cellule végétale selon la revendication 3, dans lequel ledit polypeptide exogène comprend la séquence d'acides nucléiques choisie dans le groupe constitué par les séquences SEQ ID NO : 3574, 96 et 1392.

11. Cellule végétale selon l'une quelconque des revendications 3 à 10, dans laquelle ladite cellule végétale forme une partie d'une plante.

12. Procédé comprenant :
(a) la transformation d'une ou de plusieurs cellules d'une plante avec une construction d'acide nucléique comprenant un polynucléotide isolé comprenant une séquence d'acides nucléiques codant un polypeptide qui comprend une séquence d'acides aminés homologue à au moins 80% à la séquence d'acides aminés indiquée par SEQ ID NO : 333, et un promoteur pour réaliser la transcription de ladite séquence d'acides nucléiques dans une cellule hôte, dans laquelle ladite séquence d'acides nucléiques est capable d'augmenter la biomasse, le taux de croissance, la teneur en huile, et/ou l'efficacité d'utilisation de l'azote de la plante, et
(b) la génération d'une plante mature à partir d'une ou plusieurs cellules de ladite plante résultant de l'étape (a).

13. Procédé selon la revendication 12, dans lequel ladite séquence d'acides aminés est indiquée par la séquence SEQ ID NO : 333.

14. Plante pouvant être obtenue par une méthode comprenant :
(a) la transformation d'une ou de plusieurs cellules d'une plante avec une construction d'acide nucléique comprenant un polynucléotide isolé comprenant une séquence d'acides nucléiques codant un polypeptide qui comprend une séquence d'acides aminés homologue à au moins 80% à la séquence d'acides aminés indiquée par SEQ ID NO : 333, et un promoteur pour réaliser la transcription de ladite séquence d'acides nucléiques dans une cellule hôte, dans laquelle ladite séquence d'acides nucléiques est capable d'augmenter la biomasse, le taux de croissance, la teneur en huile, et/ou l'efficacité d'utilisation de l'azote de la plante,
(b) la génération d'une plante mature à partir d'une ou plusieurs cellules de ladite plante résultant de l'étape (a),
(c) le croisement des plantes transformées regénérées exprimant ledit polypeptide et
(d) la sélection résultant de la descendance pour une biomasse, une teneur en huile et/ou une efficacité d'utilisation de l'azote supérieure, dans laquelle la plante comprend la construction d'acide nucléique.

15. Plante selon la revendication 14, dans laquelle ladite séquence d'acides aminés est indiquée par la séquence SEQ ID NO : 333.
